# EUROPEAN PATENT APPLICATION

(11) **EP 4 818 171 A1**
(43) Date of publication of application: **07.10.2026**
(21) Application number: 25738547.6
(22) Date of filing: 10.01.2025
(51) Int. Cl.: C07D 487/14, C07D 487/04, C07D 519/00, A61K 31/4985, A61P 35/00

(54) **NEW PRMT5 INHIBITOR AND USE THEREOF**

(30) Priority: 12.01.2024 CN 202410048857; 07.03.2024 CN 202410263321; 29.04.2024 CN 202410536484; 14.06.2024 CN 202410775545
(71) Applicant: Apeiron Therapeutics (Hong Kong) Ltd., Central, Hong Kong 999077 (HK)
(72) Inventor: YAO, Bing, Shanghai 201210 (CN); WANG, Minghua, Shanghai 201210 (CN); ZHANG, Junqing, Shanghai 201210 (CN)
(74) Representative: Bayramoglu et al.
(86) International application number: PCT/CN2025/071698
(87) International publication number: WO 2025/149020

(57) **Abstract**

The present disclosure describes a novel molecule having protein arginine methyltransferase 5 inhibitory activity, and synthetic and use methods of the compound. Specifically, the present disclosure describes the compound of formula (I) or pharmaceutically acceptable salts, hydrates or solvates thereof and synthetic and use methods of the compound.

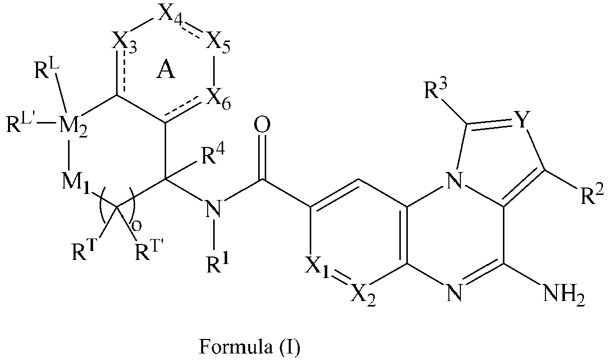

## Description

### TECHNICAL FIELD

The present disclosure belongs to the field of drug synthesis, specifically relating to a PRMT5 inhibitor and use thereof.

### BACKGROUND ART

Epigenetic alterations are key mediators that drive and maintain the malignant phenotype of tumors. Changes in DNA methylation, histone acetylation and methylation, noncoding RNAs, and post-translational modifications are all epigenetic driving forces of cancer development, independent of changes in the DNA sequence. Arginine methylation is an important kind of post-translational modification that affects cell growth and proliferation, ap optosis, angiogenesis and metastasis by regulating transcription and post-transcriptional RNA processing. Three types of methylarginine residues are found in proteins: monomethylarginine (MMA), ω-NG, N'G-asymmetric dimethylarginine (ADMA) and ω-NG, N'G-symmetric dimethylarginine (SDMA). This modification is catalyzed by the protein arginine methyltransferase (PRMT) family, which transfers a methyl group from S-adenosylmethionine (AdoMet) to histone and nonhistone arginine side chains. Nine PRMT genes are annotated in the human genome, classified as Type I (PRMT1, 2, 3, 4, 6 and 8), Type II (PRMT5 and PRMT9), and Type III (PRMT7) based on the type of methylarginine produced. PRMT5 is primarily a type II enzyme that catalyzes the symmetric dimethylation of arginine. PRMT5 was first identified in a two-hybrid assay designed to detect proteins interacting with Janus kinase 2 (JAK2).

PRMT5 is a universal transcriptional repressor that forms complexes with other transcription factors, including BRG1, hBRM, Blimp1, and Snail. PRMT5 participates in different cell biological processes through methylation of substrates in the cytoplasm and nucleus, including histone H4 residue Arg3 (H4R3) and H3 residue Arg8 (H3R8). H4R3 methylation is associated with transcriptional repression, while H3R8 methylation is considered to be associated with both transcriptional activation and transcriptional repression. In addition to direct induction of inhibitory histone labeling by PRMT5, the role of this enzyme in gene silencing is mediated through the formation of multiple inhibitory protein complexes including NuRD fractions, HDACs, MDB proteins and DNA methyltransferase. PRMT5 affects its substrate specificity by interacting with some binding proteins. The core component of this protein complex is MEP50. MEP50 is required for the enzymatic activity of PRMT5. Studies have found that PRMT5 can methylate proteins participating in RNA splicing, such as SmD3, which can be used to track the chemical activity of PRMT5 in cellular organism.

PRMT5 plays an important role in tumorigenesis. Studies have found that PRMT5 expression is up-regulated in a variety of tumors, including lymphoma, lung cancer, breast cancer and colorectal cancer. In addition, PRMT5 expression is increased in the samples of mantle cell lymphoma (MCL) patients, and PRMT5 knockout can inhibit MCL cell proliferation, indicating that PRMT5 plays an important role in MCL. Overexpression of PRMT5 promotes cell proliferation which is inhibited by PRMT5 knockout in the cell lines of melanoma, breast cancer and lung cancer. Therefore, PRMT5 is a potential target for cancer therapy.

Loss of methylthioadenosine phosphorylase (MTAP) makes the cells selectively dependent on PRMT5 and its binding partner WDR77. MTAP is often lost due to its proximity to the tumor suppressor gene CDKN2A which is generally missing. The concentration of methylthioadenosine (MTA, the metabolite cleaved by MTAP) is increased of the cells with MTAP deficiency. MTA has a similar structure to S-adenosylmethionine (SAM). With increase of concentration, MTA acts as an intrinsic selective inhibitor to inhibit the binding of SAM to PRMT5, thus inhibiting the methyltransferase activity of PRMT5.

The most significant biochemical difference between MTAP-deficient cancer cells and MTAP wild-type cancer cells is the PRMT5-MTA complex produced in MTAP-deficient cancer cells due to accumulation of MTA concentration. The inhibitor developed for the PRMT5-MTA complex can selectively target cancer cells with MTAP deficiency, with little effecton normal cells, which greatly improves the therapeutic index.

Thus, the identification and development of small molecules that inhibit PRMT5 activity would be useful as a therapeutic method for treating various PRMT5-related diseases or disorders(e.g., cancer).

### SUMMARY OF THE INVENTION

In order to solve the technical problem of the present disclosure, the present disclosure provides a type of novel structural compounds with excellent inhibitory activity against PRMT5.

Specifically, the present disclosure provides a compound of formula (I), or a pharmaceutically acceptable salt, an ester, a prodrug, a stereoisomer or an isotopic derivative thereof. 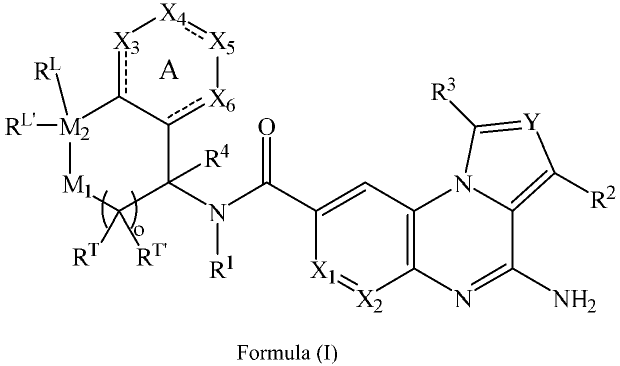
Wherein, the dashed line represents a single bond or double bond;
Wherein, X₁ represents N or CR^{X1};
Wherein, X₂ represents N or CR^{X2};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, Y represents CH or N;
Wherein, R⁴ represents H or D;
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, - S(O)₂R^{a}, -S(O)R^{a}, -S(O)(=NR^{a})R^{b}, -CN, -OC(O)R^{a}, -OCONR^{a}R^{b}, halogen, -OSO₃R^{a}, -NR^{a}R^{b}, -SF₅, -C(O)OR^{a} and - C(O)ONR^{a}R^{b};
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, - C(O)OR^{a}, -C(O)NR^{a}R^{b}, or a group selected from C₃-C₁₀ cycloalkyl, C₆-C₁₀ cycloalkenyl, 4-10-membered saturated or unsaturated heterocyclyl, C6-C10 aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R^{X4} represents -L-R^{X4-1};
Wherein, L represents absent or CR^{a}R^{b}, SiR^{a}R^{b}, O, S, Se, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, said ring A may also be optionally fused with a 5- to 6-membered saturated or unsaturated ring at the chemical bond between X₃ and X₄, which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, - SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, the ring A may also optionally be fused with the chemical bond between X₄ and X₅ to form a 5-6-membered saturated or unsaturated ring which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, - S(O)R^{a}, -S(O)(=NR^{a})R^{b}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R¹ represents hydrogen or a group selected from C₁-C₆ alkyl, C₃-C₁₀ cycloalkyl, 4-10 membered heterocycloalkyl, C₆-C₁₀ aryl, or 5-10 membered heteroaryl optionally substituted with 0-3 substituents selected from: Deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b};
Wherein, R² and R³ each independently represent hydrogen, deuterium, C₁-C₆ alkyl, halogen, hydroxyl, carboxyl, C₁-C₆ haloalkyl, hydroxy (C₁-C₆) alkyl, or mercapto (C₁-C₆) alkyl;
Wherein, M₁ represents CR^{a}R^{b}, -SiR^{a}R^{b}, NR^{a}, O, S or Se;
Wherein, M₂ represents C or Si;
Wherein, R^{L} and R^{L'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
Wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
Wherein, at least one pair among R^{L}/R^{L}' and R^{T}/R^{T}' forms a 3-10-membered saturated or unsaturated ring with the atom connected thereto, and the ring may also optionally contain 0, 1, 2 heteroatoms selected from O, N, S and Se; further, the ring may optionally be substituted with 0, 1, 2 substituents independently selected from deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, - OC(O)R^{a}, -OCONR^{a}R^{b}, halogen, -OSO₃R^{a}, -NR^{a}R^{b} and -SF₅;
wherein, o represents 0, 1 or 2;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

In addition, the present disclosure provides a compound of formula (I-2), or a pharmaceutically acceptable salt, ester, prodrug, stereoisomer or isotopic derivative thereof, 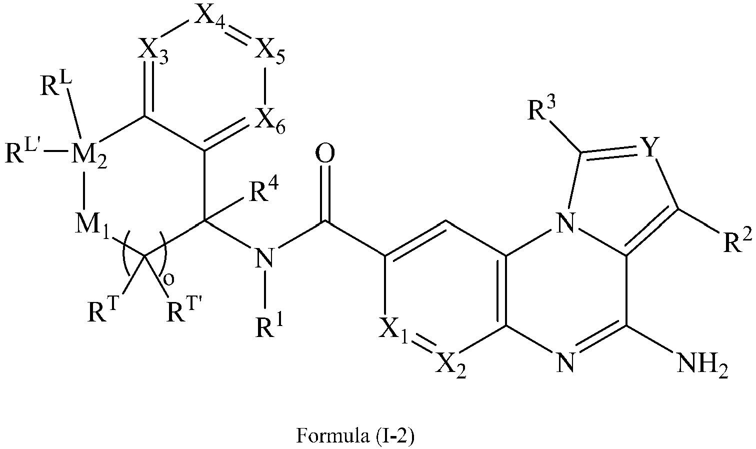
Wherein, X₁ represents N or CR^{X1};
Wherein, X₂ represents N or CR^{X2};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, Y represents CH or N;
Wherein, R⁴ represents H or D;
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, - S(O)₂R^{a}, -S(O)R^{a}, -S(O)(=NR^{a})R^{b}, -CN, -OC(O)R^{a}, -OCONR^{a}R^{b}, halogen, -OSO₃R^{a}, -NR^{a}R^{b}, -SF₅, -C(O)OR^{a} or - C(O)ONR^{a}R^{b};
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, - C(O)OR^{a}, - C(O)NR^{a}R^{b} , or a group selected from C₃-C₁₀ cycloalkyl, C₆-C₁₀ cycloalkenyl, 4-10-membered saturated or unsaturated heterocyclyl, C6-C10 aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R^{X4} represents -L-R^{X4-1};
Wherein, L represents absent or CR^{a}R^{b}, SiR^{a}R^{b}, O, S, Se, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b};
Wherein, R² and R³ each independently represent hydrogen, deuterium, C₁-C₆ alkyl, halogen, hydroxyl, carboxyl, C₁-C₆ haloalkyl, hydroxy (C1-C₆) alkyl, or mercapto (C₁-C₆) alkyl;
Wherein, M₁ represents CR^{a}R^{b}, -SiR^{a}R^{b}, NR^{a}, O, S or Se;
Wherein, M₂ represents C or Si;
Wherein, R^{L} and R^{L'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
Wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
Wherein, at least one pair among R^{L}/R^{L}' and R^{T}/R^{T}' forms a 3-10-membered saturated or unsaturated ring with the atom connected thereto, and the ring may also optionally contain 0, 1, 2 heteroatoms selected from O, N, S and Se; further, the ring may optionally be substituted with 0, 1, 2 substituents independently selected from deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, - OC(O)R^{a}, -OCONR^{a}R^{b}, halogen, -OSO₃R^{a}, -NR^{a}R^{b} and -SF₅;
wherein, o represents 0, 1 or 2;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

In the preferred embodiment shown in the above formula (I) or formula (I-2), X¹ represents N.

In the preferred embodiment shown in the above formula (I) or formula (I-2), X₁ represents CR^{X1}, wherein R^{X1} represents hydrogen, deuterium, halogen, C₁-C₆ alkyl, C₂-C₆ alkenyl, C₂-C₆ alkynyl, C1-C₆ haloalkyl, C₃-C₆ cycloalkyl, hydroxyl, C₁-C₆ alkoxy, C₁-C₆ haloalkoxy, cyano, NH₂, or -N(CH₃)₂.

In the preferred embodiment shown in the above formula (I) or formula (I-2), X² represents CR^{X2}, wherein R^{X2} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X2} represents hydrogen or C₁-C₆ alkyl.

In a preferred embodiment of the above formula (I) or formula (I-2), X³ represents N.

In a preferred embodiment of the above formula (I) or formula (I-2), X³ represents CR^{X3}, wherein R^{X3} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X3} represents hydrogen, halogen, C₁-C₆ alkyl, or C₁-C₆ haloalkyl.

In a preferred embodiment of the above formula (I) or formula (I-2), X⁵ represents N.

In a preferred embodiment of the above formula (I) or formula (I-2), X⁵ represents CR^{X5}, wherein R^{X5} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X5} represents hydrogen, halogen, C₁-C₆ alkyl, or C₁-C₆haloalkyl.

In a preferred embodiment of the above formula (I) or formula (I-2), X⁶ represents N.

In the preferred embodiment shown in the above formula (I) or formula (I-2), X⁶ represents CR^{X6}, wherein R^{X6} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X6} represents hydrogen, halogen, C₁-C₆ alkyl, C₁-C₆ haloalkyl, C₁-C₆ haloalkoxy, or C₁-C₆ haloalkylthio.

In a preferred embodiment of the above formula (I) or formula (I-2), X⁴ represents C-L-R^{X4-1}, wherein L represents absence, and R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -S(O)(=NR^{a})R^{b}, -SF₅, -NR^{a}R^{b}, - C(O)OR^{a}, -OC(O)R^{a}, -OCONR^{a}R^{b}, -NR^{a}COR^{b}, -CONR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0 to 4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, - SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b}.

In the preferred embodiment shown in the above formula (I) or formula (I-2), X⁴ represents C-L-R^{X4-1}, wherein L represents absence, and R^{X4-1} represents hydrogen, deuterium, C₁-C₆ alkyl, halogen, C₁-C₆ haloalkyl, -CN, -NH₂, -NHCH₃, -N(CH₃)₂, -OH, -OCH₃, C₁-C₆ alkoxy, C₁-C₆ haloalkoxy, C₁-C₆ haloalkylthio, -SF₅, or - P(O)(CH₃)₂.

In the preferred embodiment shown in the above formula (I) or formula (I-2), X⁴ represents C-L-R^{X4-1}, wherein L represents absence, and R^{X4-1} represents hydrogen, halogen (preferably F), CN, -CF₃, -CF₂CF₃, -NH₂, -NHCH₃, -N(CH₃)₂, -OCH₃, -OCF₃, -SF₅, -SCF₃, or -P(O)(CH₃)₂.

In a preferred embodiment of the above formula (I) or formula (I-2), X⁴ represents C-L-R^{X4-1}, wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b}.

In a preferred embodiment of the above formula (I) or formula (I-2), X⁴ represents C-L-R^{X4-1}, wherein L represents absence, and R^{X4-1} represents the following groups:

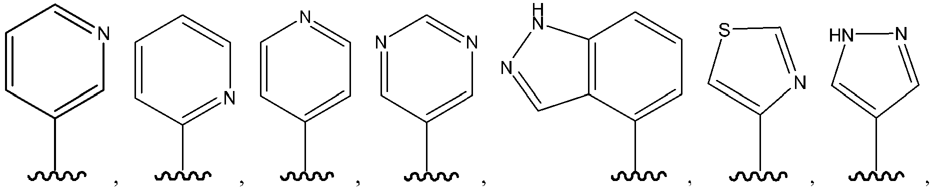

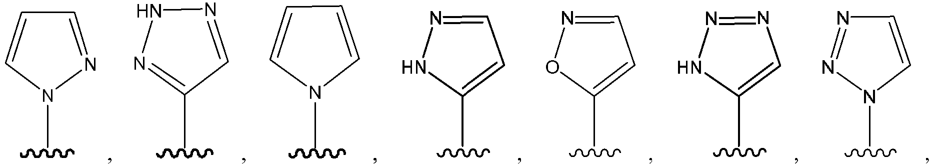

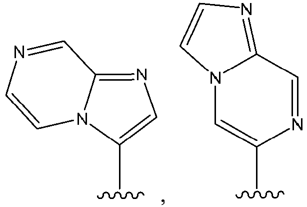

Furthermore, R^{X4} may optionally be substituted with 0, 1 or 2 substituents selected from the group consisting of: halogen, hydroxyl, C₁-C₆ alkyl, C₁-C₆ haloalkyl, and C₁-C₆ haloalkoxy.

In a preferred embodiment of the above formula (I) or formula (I-2), X⁴ represents C-L-R^{X4-1}, wherein L represents absence, and R^{X4-1} represents C₃-C₁₀ cycloalkyl, C₆-C₁₀ cycloalkenyl or 4-10-membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b}.

In a preferred embodiment of the above formula (I) or formula (I-2), X⁴ represents C-L-R^{X4-1}, wherein L represents O, S, NH, or N(CH₃), and R^{X4-1} represents the following groups:

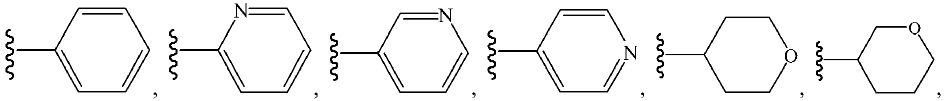

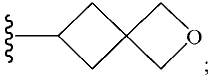

Furthermore, R^{X4-1} may optionally be substituted with 0, 1 or 2 substituents selected from the group consisting of: halogen, hydroxyl, C₁-C₆ alkyl, C₁-C₆ haloalkyl and C₁-C₆ haloalkoxy.

In a preferred embodiment of the above formula (I) or formula (I-2), X⁴ represents C-L-R^{X4-1}, wherein L represents absence, and R^{X4-1} represents the following groups: 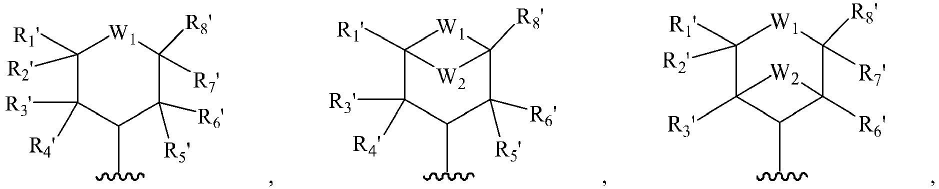 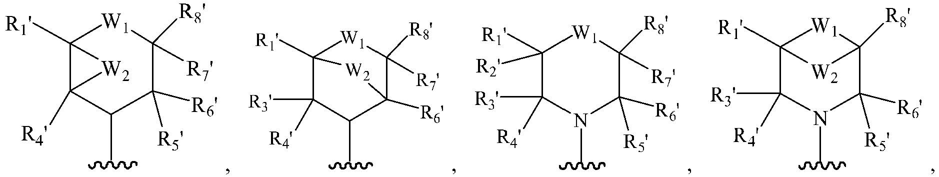 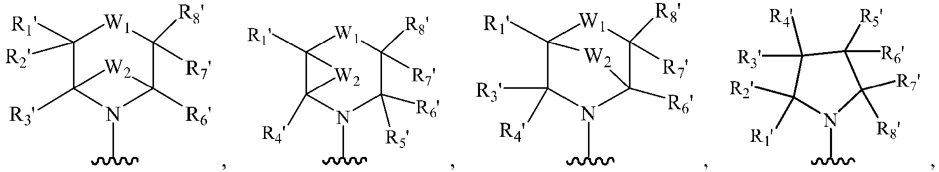 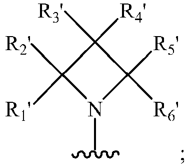
Wherein, W₁ represents CR^{C}R^{D}, NR^{C}, O, S, or SiR^{C}R^{D};
Wherein, W₂ represents -(CR^{M}R^{N})ᵢ-;
Wherein, R₁', R₂', R₃', R₄', R₅', R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
Wherein, R^{C} and R^{D} each independently represent hydrogen, deuterium, halogen, C₁-C₆ alkyl or hydroxyl; or R^{S} and R^{T}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
Wherein, R^{M} and R^{N} each independently represent hydrogen or C₁-C₆ alkyl;
Wherein, i represents an integer of 1 or 2.

In a preferred embodiment of the above formula (I) or formula (I-2), X⁴ represents C-L-R^{X4-1}, wherein L represents absence, and R^{X4-1} represents the following groups:

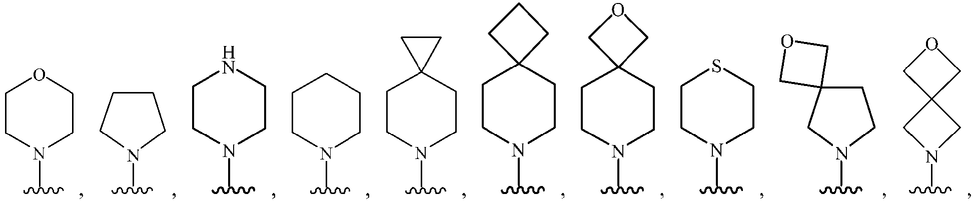

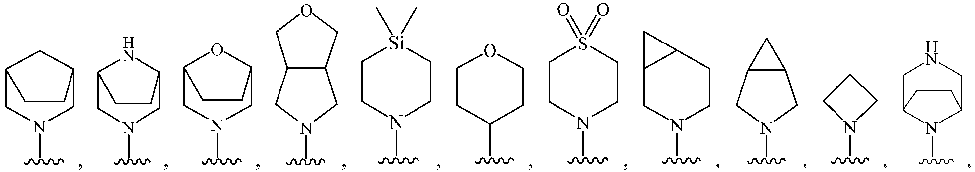

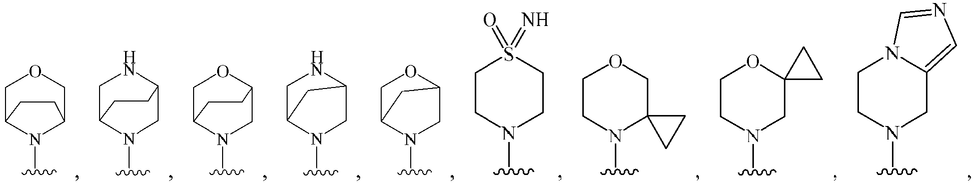

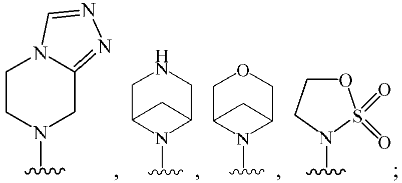

Wherein, the R^{X4-1} may optionally be substituted with 0, 1, 2 substituents independently selected from halogen, C₀-C₆ hydroxyalkyl, C₀-C₆ cyanoalkyl, C₁-C₆ alkyl and C₁-C₆ haloalkyl.

In a preferred embodiment of the above formula (I) or formula (I-2), X⁴ represents C-L-R^{X4-1}, wherein L represents absence, and R^{X4-1} represents the following groups:

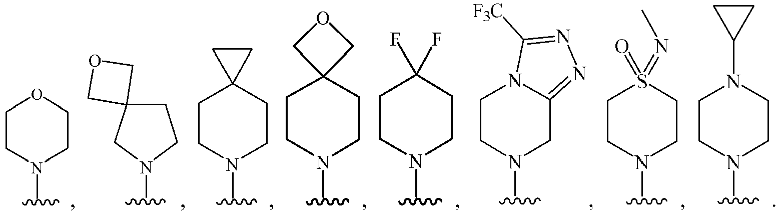

In a preferred embodiment of the above formula (I) or formula (I-2), R^{L} and R^{L'}, together with the atom attached thereto, form a ring of the following structure:

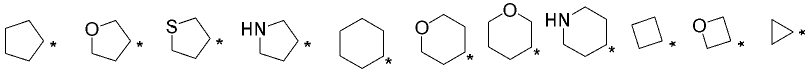

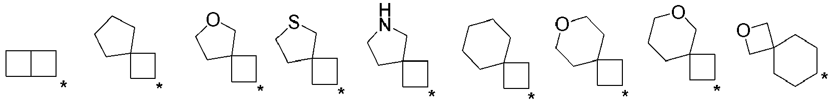

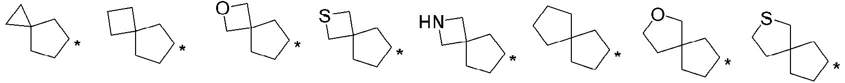

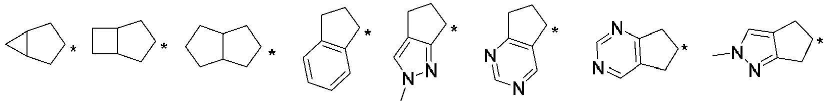

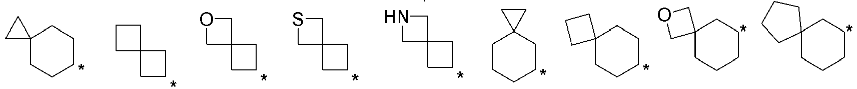

Wherein, * represents an atomic site where R^{L} and R^{L'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, - OCONR^{a}R^{b}, halogen, -OSO₃R^{a}, -NR^{a}R^{b} and -SF₅.

In a preferred embodiment of the above formula (I) or formula (I-2), R^{T} and R^{T'}, together with the atom attached thereto, form a ring of the following structure:

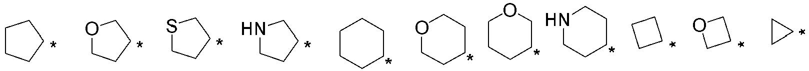

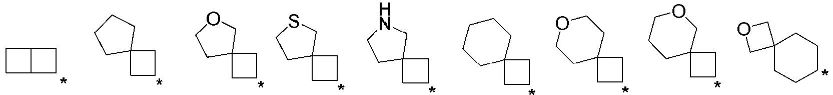

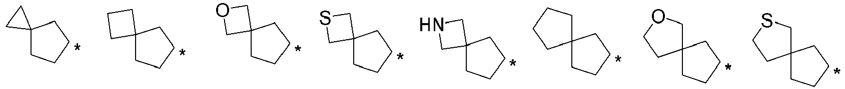

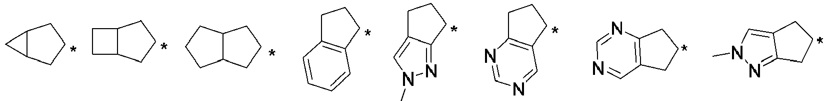

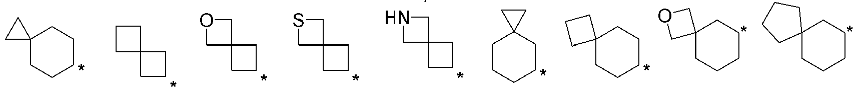

Wherein, * represents an atomic site where R^{T} and R^{T'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, - OCONR^{a}R^{b}, halogen, -OSO₃R^{a}, -NR^{a}R^{b} and -SF₅.

In addition, the present disclosure provides a compound having any of the following structures, or a pharmaceutically acceptable salt, ester, prodrug, stereoisomer, or isotopic derivative thereof, 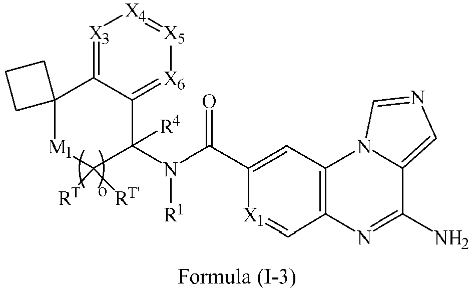 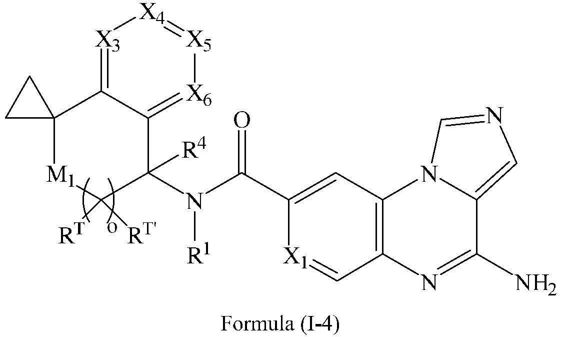 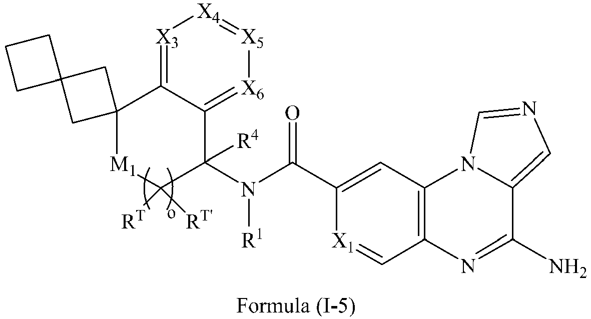 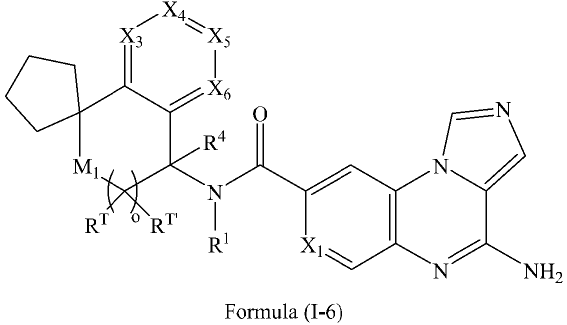 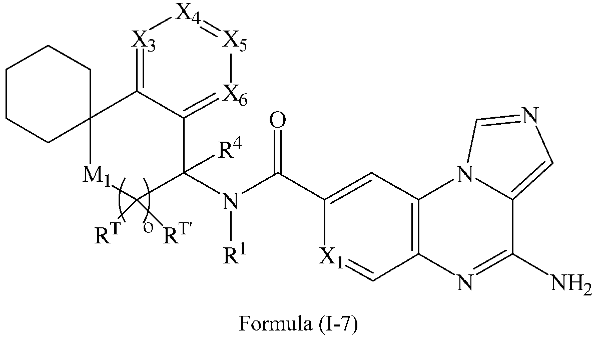 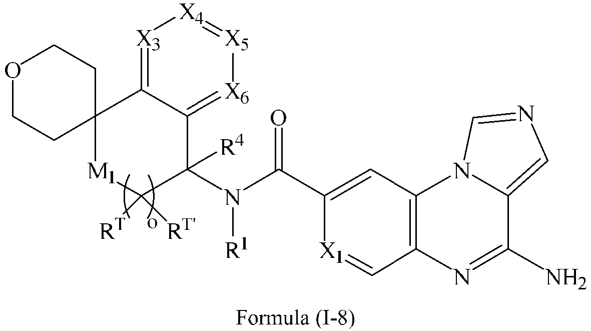 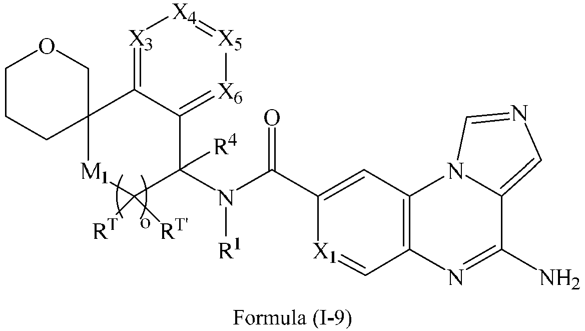 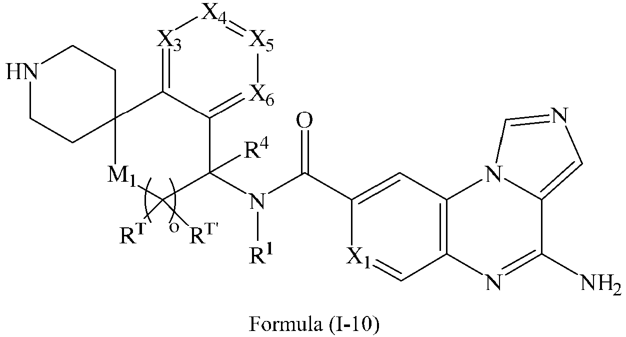 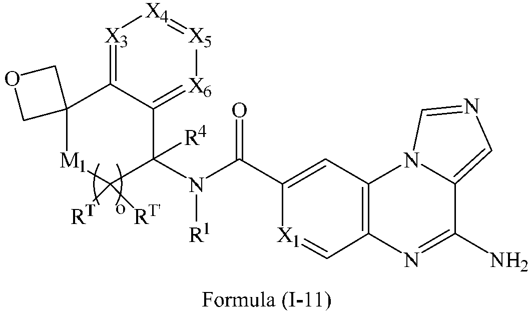 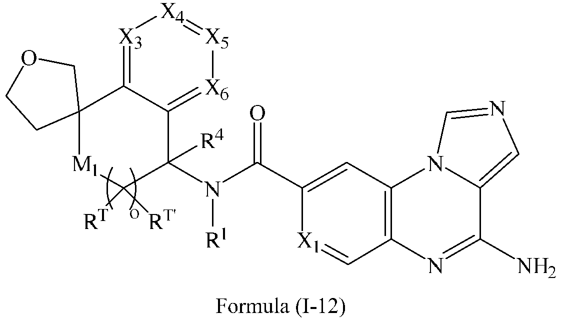 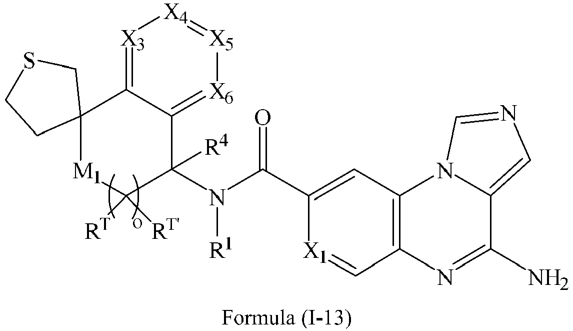
Wherein, X₁ represents N or CR^{X1};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, R⁴ represents H or D;
Wherein, R^{X1} represents hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, - S(O)(=NR^{a})R^{b}, -CN, -OC(O)R^{a}, -OCONR^{a}R^{b}, halogen, -OSO₃R^{a}, -NR^{a}R^{b} or -SF₅;
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R^{X4} represents -L-R^{X4-1};
Wherein, L represents absence or CR^{a}R^{b}, O, S, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
Wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b};
Wherein, M¹ represents CR^{a}R^{b}, NR^{a}, O, S or Se;
   Wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
wherein, o represents 0, 1 or 2;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

In addition, the present disclosure provides a compound having any of the following structures, or a pharmaceutically acceptable salt, ester, prodrug, stereoisomer, or isotopic derivative thereof, 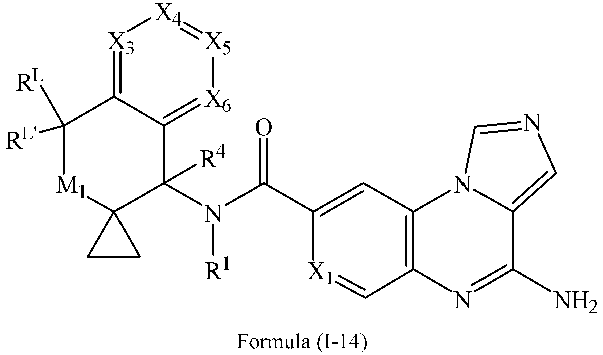 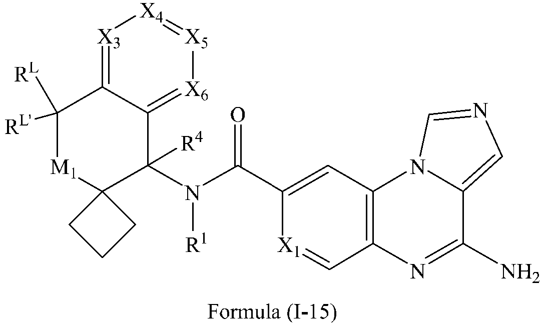 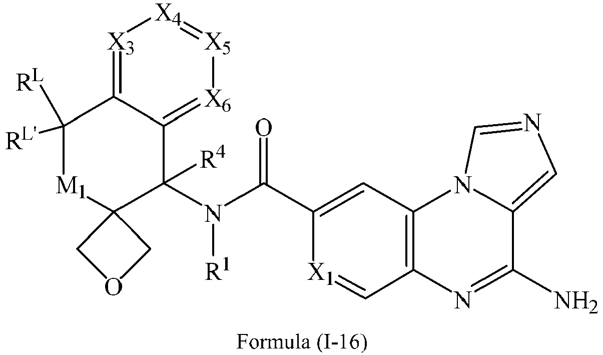 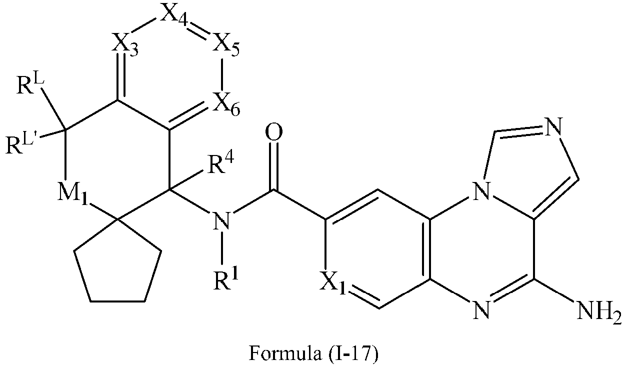
Wherein, X₁ represents N or CR^{X1};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, R⁴ represents H or D;
Wherein, R^{X1} represents hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, - S(O)(=NR^{a})R^{b}, -CN, -OC(O)R^{a}, -OCONR^{a}R^{b}, halogen, -OSO₃R^{a}, -NR^{a}R^{b}, or -SF₅;
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, R^{X4} represents -L-R^{X4-1};
Wherein, L represents absence or CR^{a}R^{b}, O, S, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, - NR^{a}COR^{b} and -CONR^{a}R^{b};
Wherein, M¹ represents CR^{a}R^{b}, NR^{a}, O, S or Se;
Wherein, R^{L} and R^{L'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b}, and - SF₅;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

In a preferred embodiment of formula (I-3) to formula (I-16), X¹ represents N.

In a preferred embodiment of the above formula (I-3) to formula (I-16), X₁ represents CR^{X1}, wherein R^{X1} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂.

In a preferred embodiment of formula (I-3) to formula (I-16), X₃ represents N.

In the preferred embodiment shown in formula (I-3) to formula (I-16), X₃ represents CR^{X3}, wherein R^{X3} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X3} represents hydrogen, halogen, C₁-C₆ alkyl, or C₁-C₆ haloalkyl.

In a preferred embodiment of formula (I-3) to formula (I-16), X⁵ represents N.

In a preferred embodiment of formula (I-3) to formula (I-16), X⁵ represents CR^{X5}, wherein R^{X5} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X5} represents hydrogen, halogen, C₁-C₆ alkyl, or C₁-C₆ haloalkyl.

In a preferred embodiment of formula (I-3) to formula (I-16), X₆ represents N.

In a preferred embodiment of formula (I-3) to formula (I-16), X₆ represents CR^{X6}, wherein R^{X6} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X6} represents hydrogen, halogen, C₁-C₆ alkyl, or C₁-C₆ haloalkyl.

In a preferred embodiment of formula (I-3) to formula (I-16), X⁴ represents C-L-R^{X4-1}, wherein L represents absence, and R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0 to 4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b}.

In a preferred embodiment of formula (I-3) to formula (I-16), X⁴ represents C-L-R^{X4-1}, wherein L represents absence, and R^{X4-1} represents hydrogen, deuterium, C₁-C₆ alkyl, halogen, C₁-C₆ haloalkyl, -CN, -NH₂, -NHCH₃, -N(CH₃)₂, -OH, -OCH₃, C₁-C₆ alkoxy, C₁-C₆ haloalkoxy, -SF₅, or -P(O)(CH₃)₂.

In a preferred embodiment of formula (I-3) to formula (I-16), X⁴ represents C-L-R^{X4-1}, wherein L represents absence, and R^{X4-1} represents hydrogen, halogen (preferably F), -CF₃, -NH₂, -NHCH₃, -N(CH₃)₂, - OCH₃, -OCF₃, -SF₅, or -P(O)(CH₃)₂.

In a preferred embodiment of formula (I-3) to formula (I-16), X⁴ represents C-L-R^{X4-1}, wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b}.

In a preferred embodiment of formula (I-3) to formula (I-16), X⁴ represents C-L-R^{X4-1}, wherein L represents absence, and R^{X4-1} represents the following groups:

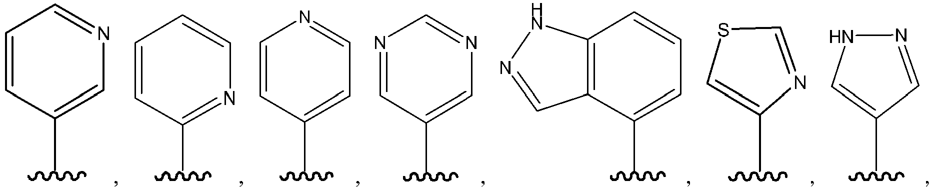

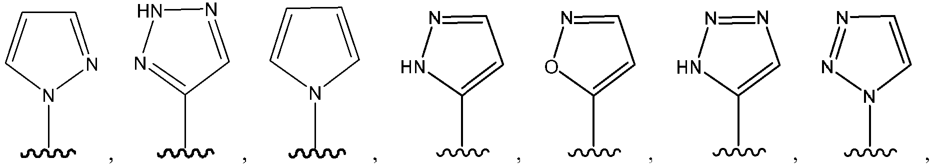

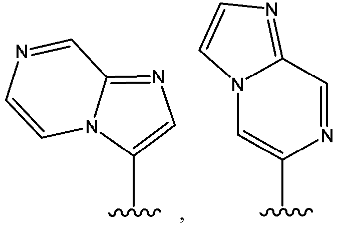

Furthermore, R^{X4} may optionally be substituted with 0, 1 or 2 substituents selected from the group consisting of: halogen, hydroxyl, C₁-C₆ alkyl, C₁-C₆ haloalkyl, and C₁-C₆ haloalkoxy.

In a preferred embodiment of formula (I-3) to formula (I-16), X⁴ represents C-L-R^{X4-1}, wherein L represents absence, and R^{X4-1} represents C₃-C₁₀ cycloalkyl, C₆-C₁₀ cycloalkenyl or 4-10-membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b}.

Wherein X⁴ represents C-L-R^{X4-1}; wherein L represents O, S, NH, or N(CH₃), and R^{X4-1} represents the following groups:

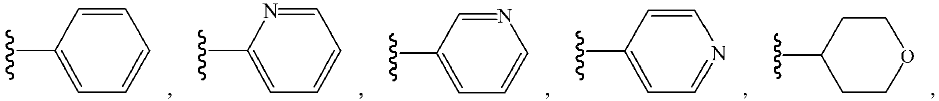

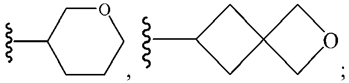

Furthermore, R^{X4-1} may optionally be substituted with 0, 1 or 2 substituents selected from the group consisting of: halogen, hydroxyl, C₁-C₆ alkyl, C₁-C₆ haloalkyl, and C₁-C₆ haloalkoxy.

In a preferred embodiment of formula (I-3) to formula (I-16), X⁴ represents C-L-R^{X4-1}, wherein L represents absence, and R^{X4-1} represents the following groups: 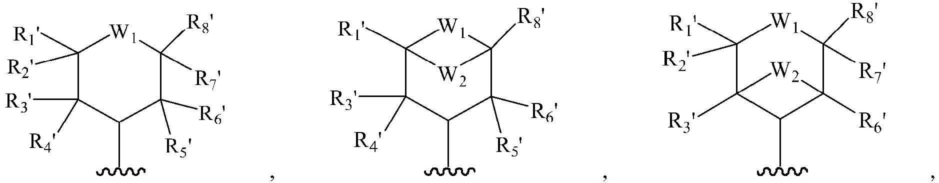 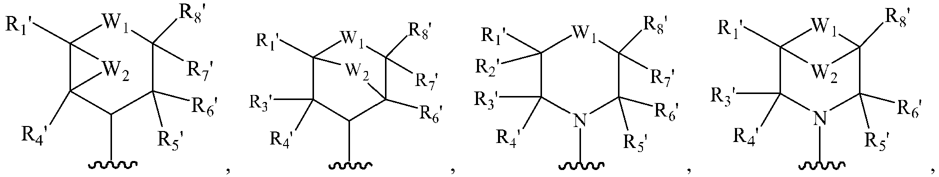 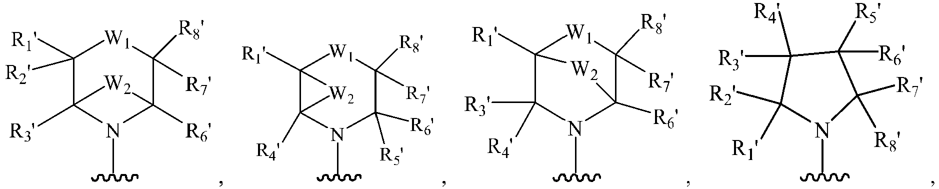 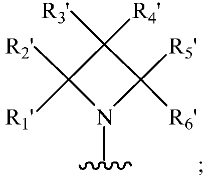
Wherein, W₁ represents CR^{C}R^{D}, NR^{C}, O, or S;
Wherein, W₂ represents -(CR^{M}R^{N})ᵢ-;
Wherein, R₁', R₂', R₃', R₄', R₅', R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
Wherein, R^{C} and R^{D} each independently represent hydrogen, deuterium, halogen, C₁-C₆ alkyl or hydroxyl; or R^{S} and R^{T}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
Wherein, R^{M} and R^{N} each independently represent hydrogen or C₁-C₆ alkyl;
Wherein, i represents an integer of 1 or 2.

In a preferred embodiment of formula (I-3) to formula (I-16), X⁴ represents C-L-R^{X4-1}, wherein L represents absence, and R^{X4-1} represents the following groups:

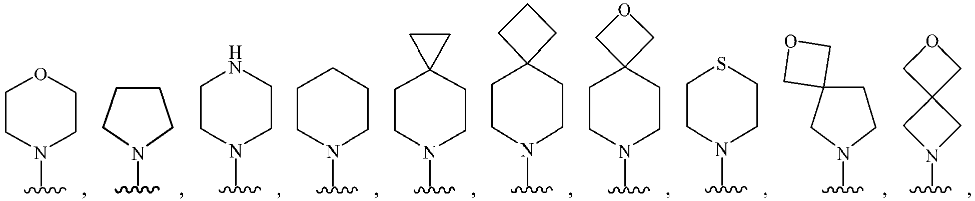

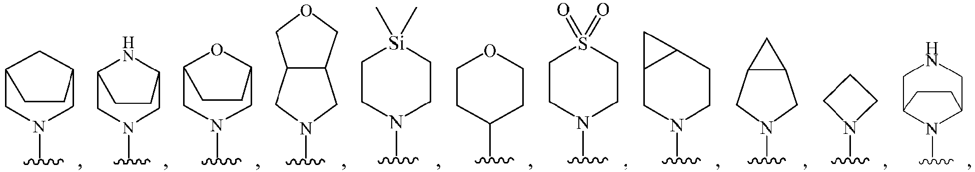

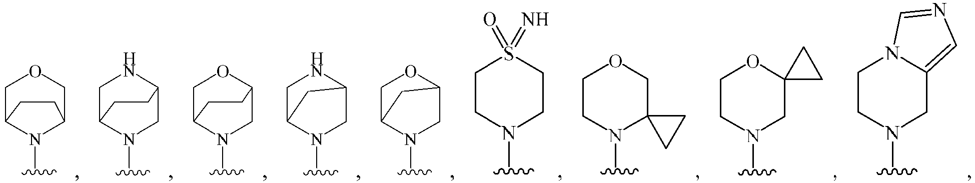

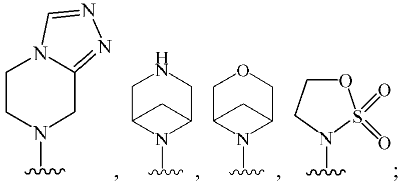

Wherein, the R^{X4-1} may optionally be substituted with 0, 1, or 2 substituents selected from the group consisting of: halogen, hydroxyl, cyano, C₁-C₆ alkyl, and C₁-C₆ haloalkyl.

In a preferred embodiment of formula (I-3) to formula (I-16), X⁴ represents C-L-R^{X4-1}, wherein L represents absence, and R^{X4-1} represents the following groups:

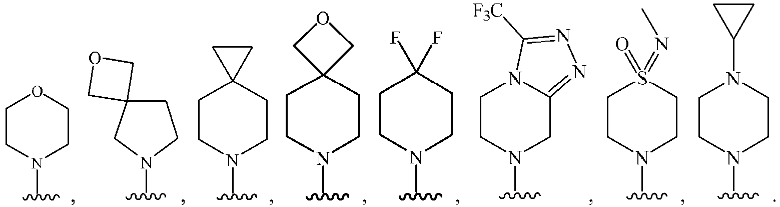

Specifically, the present disclosure provides a compound, or a pharmaceutically acceptable salt, ester, prodrug, stereoisomer or isotopic derivative thereof, having the following structure:

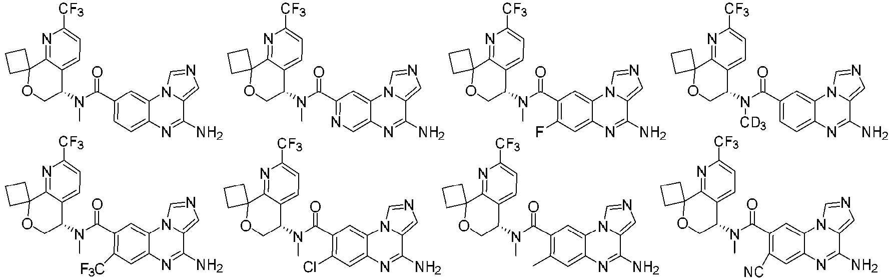

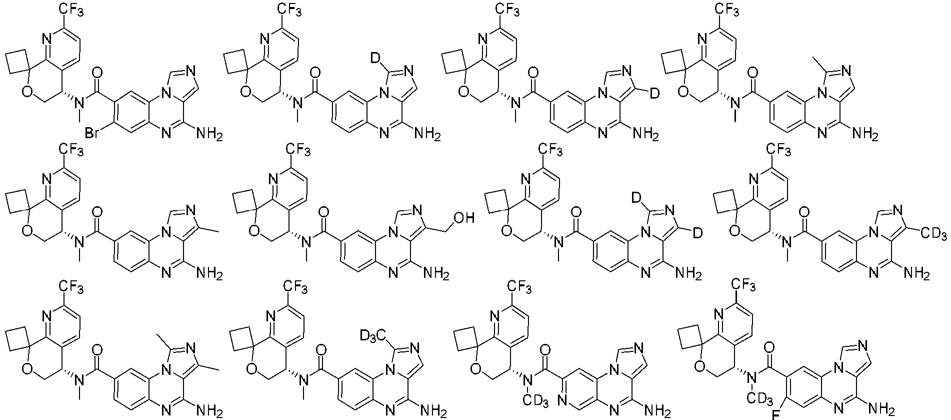

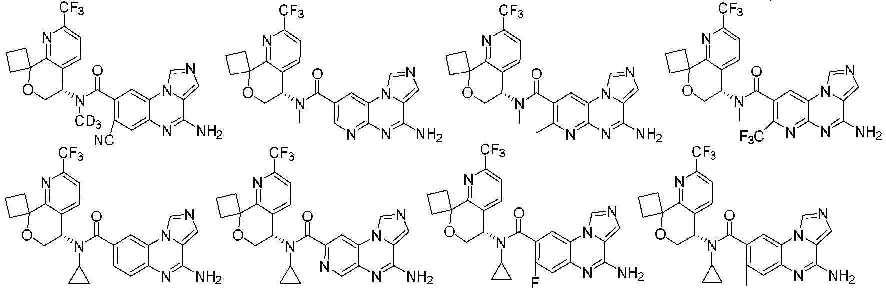

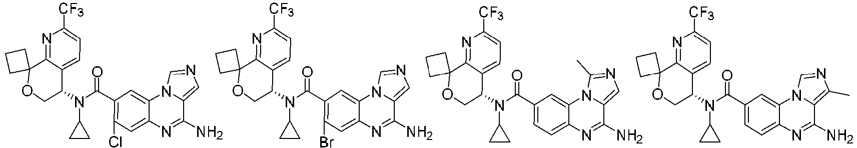

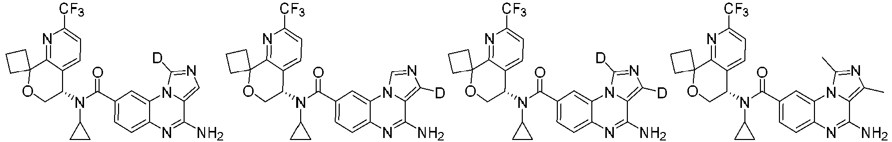

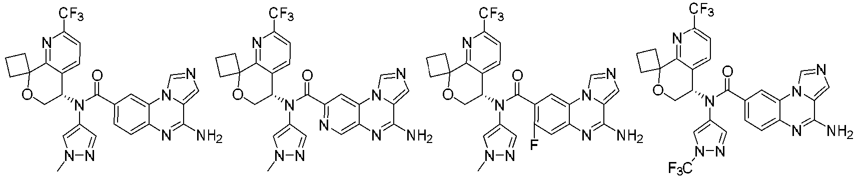

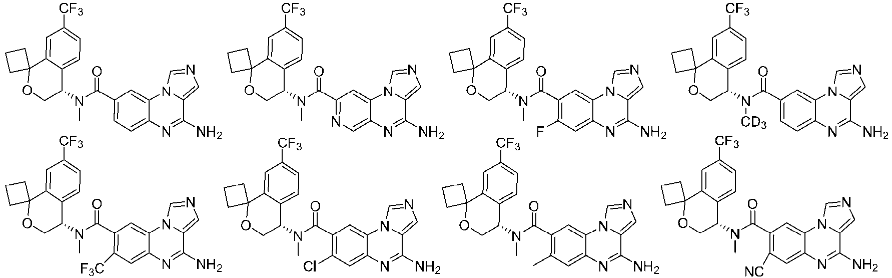

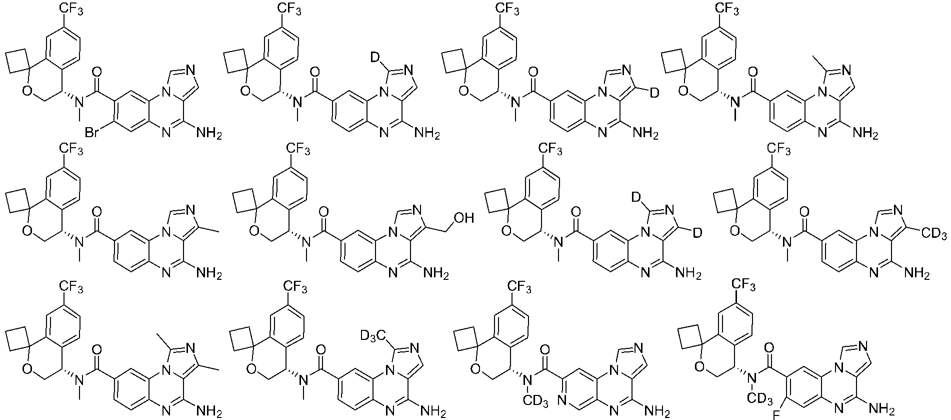

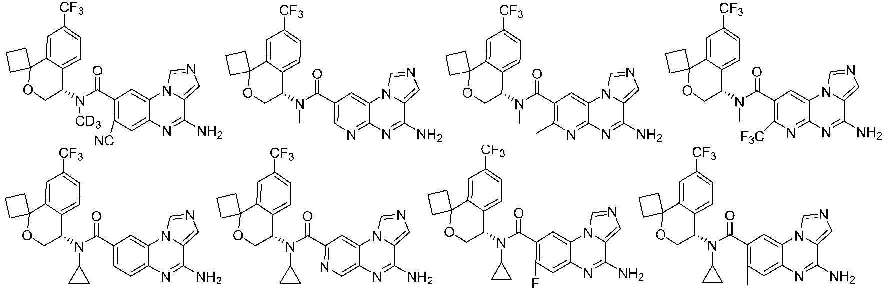

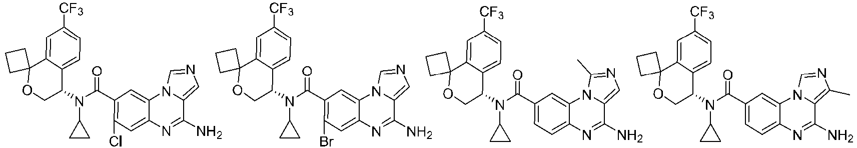

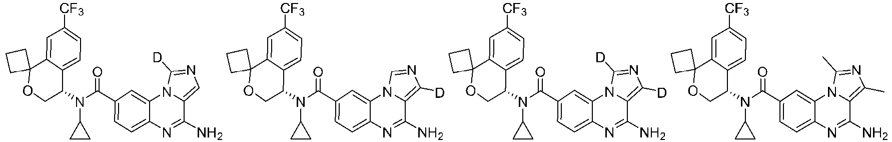

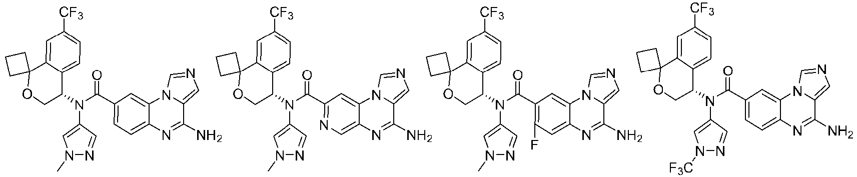

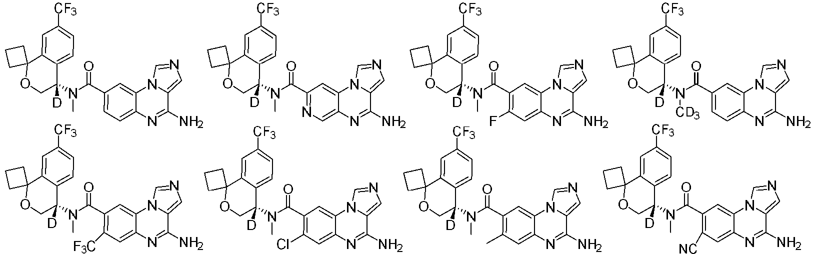

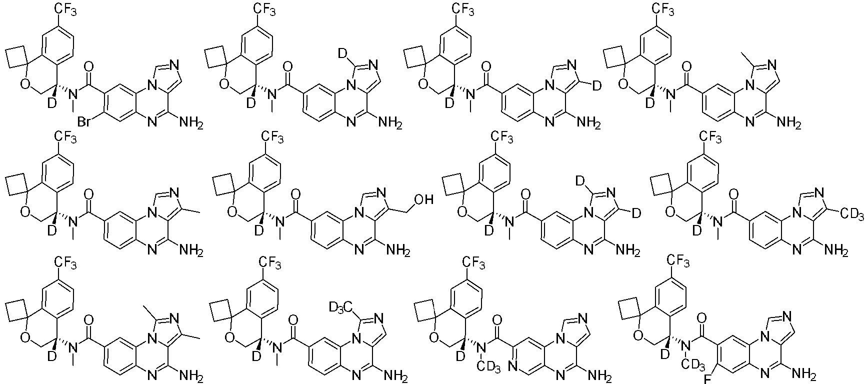

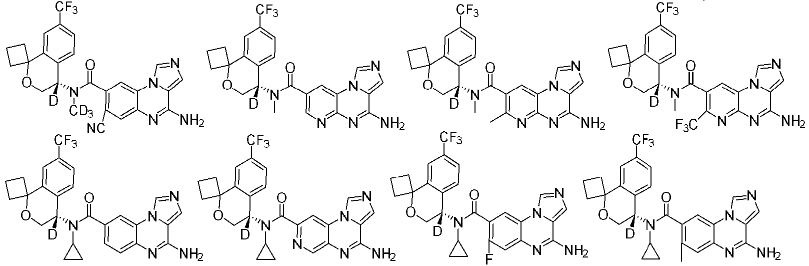

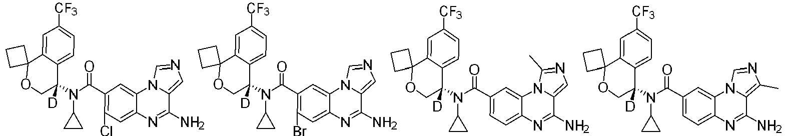

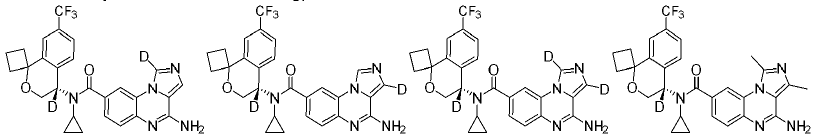

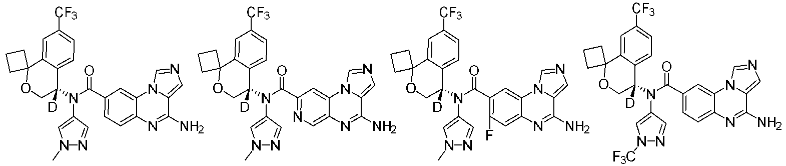

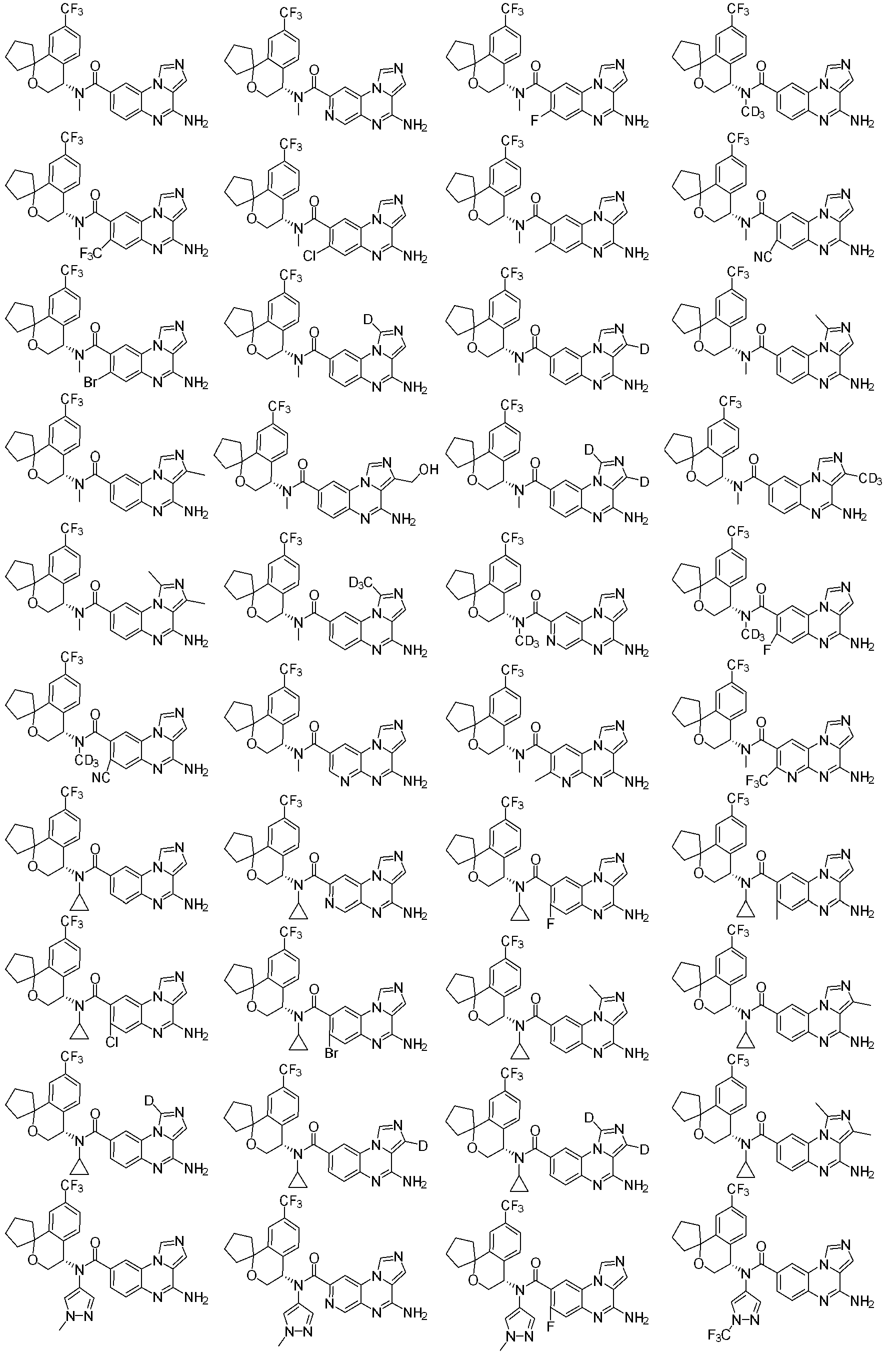

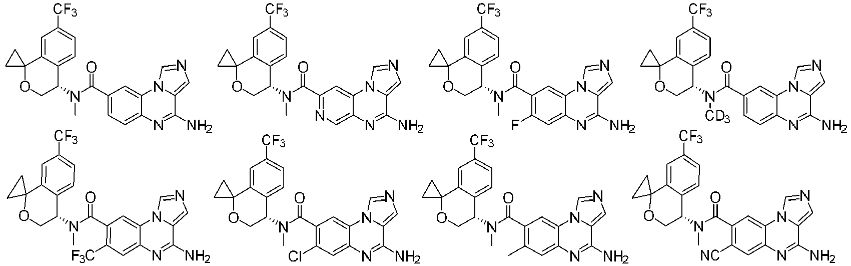

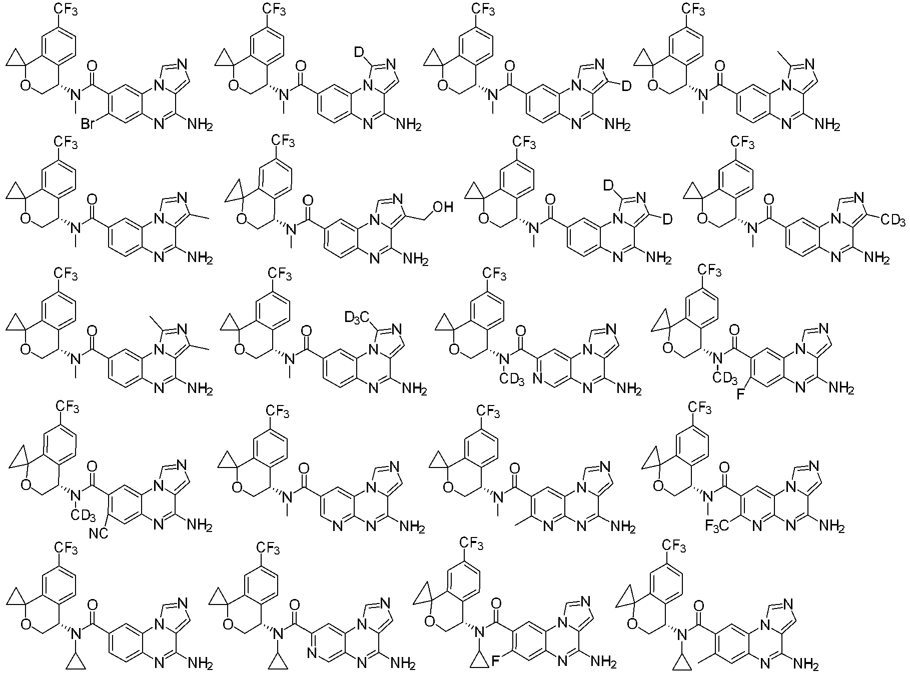

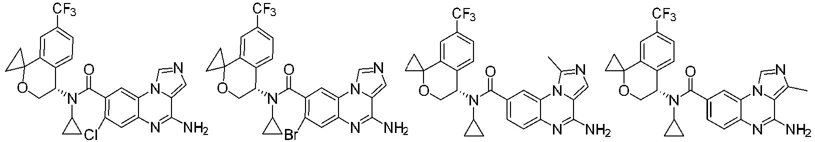

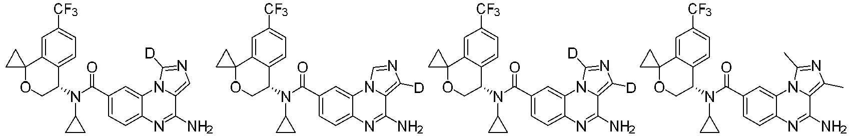

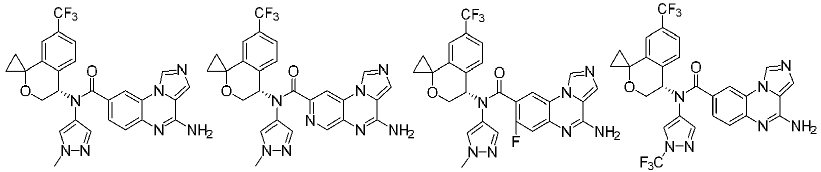

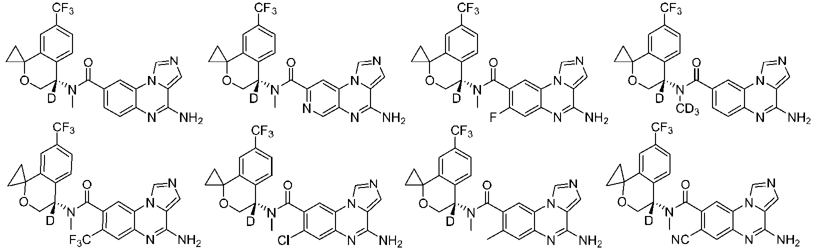

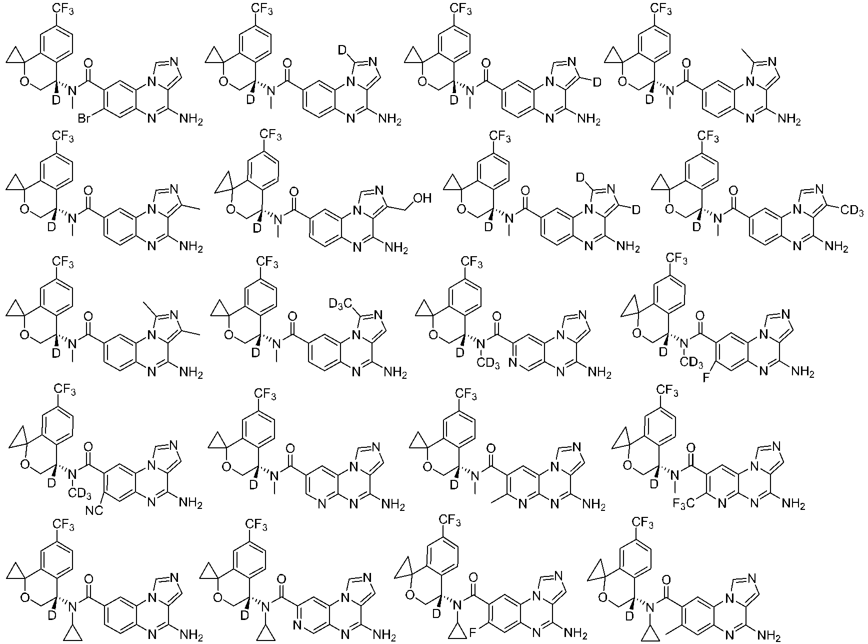

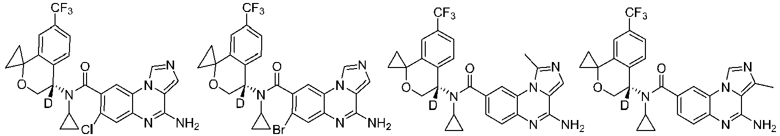

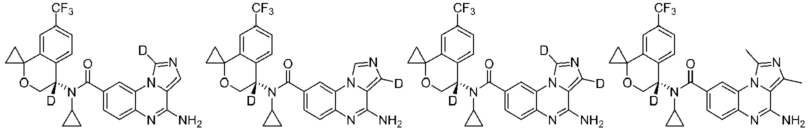

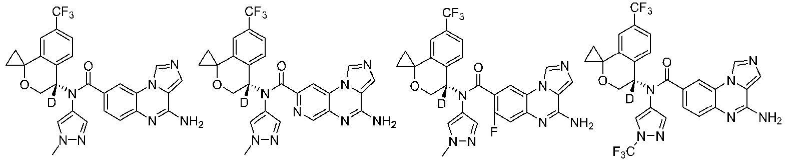

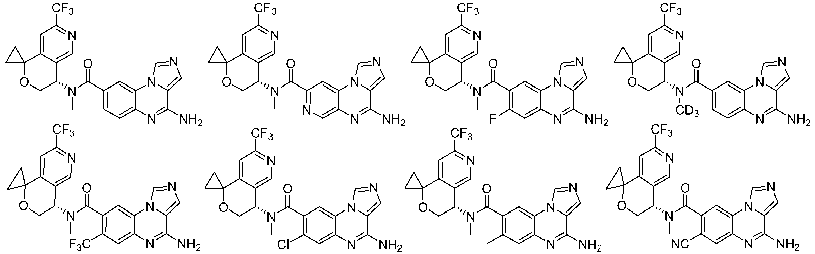

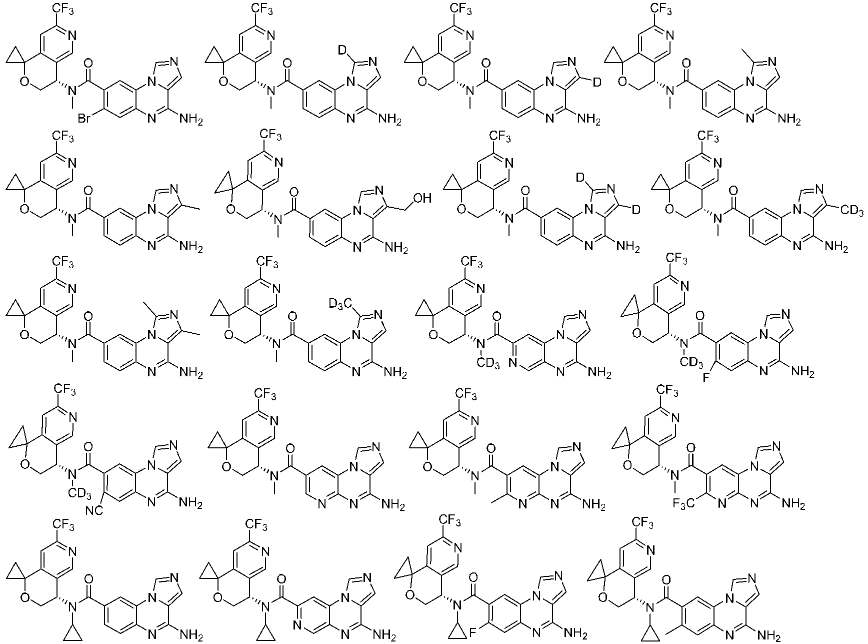

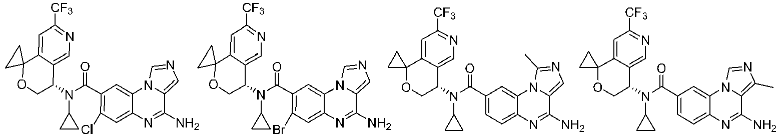

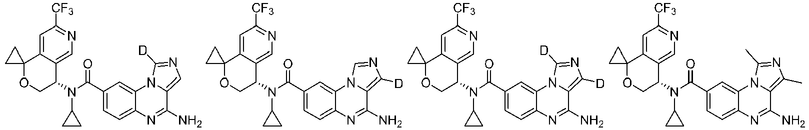

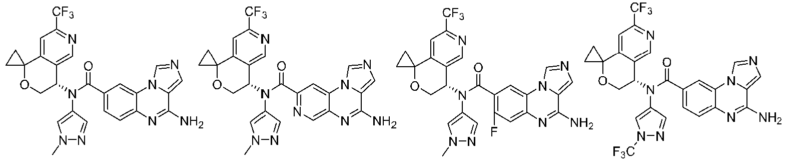

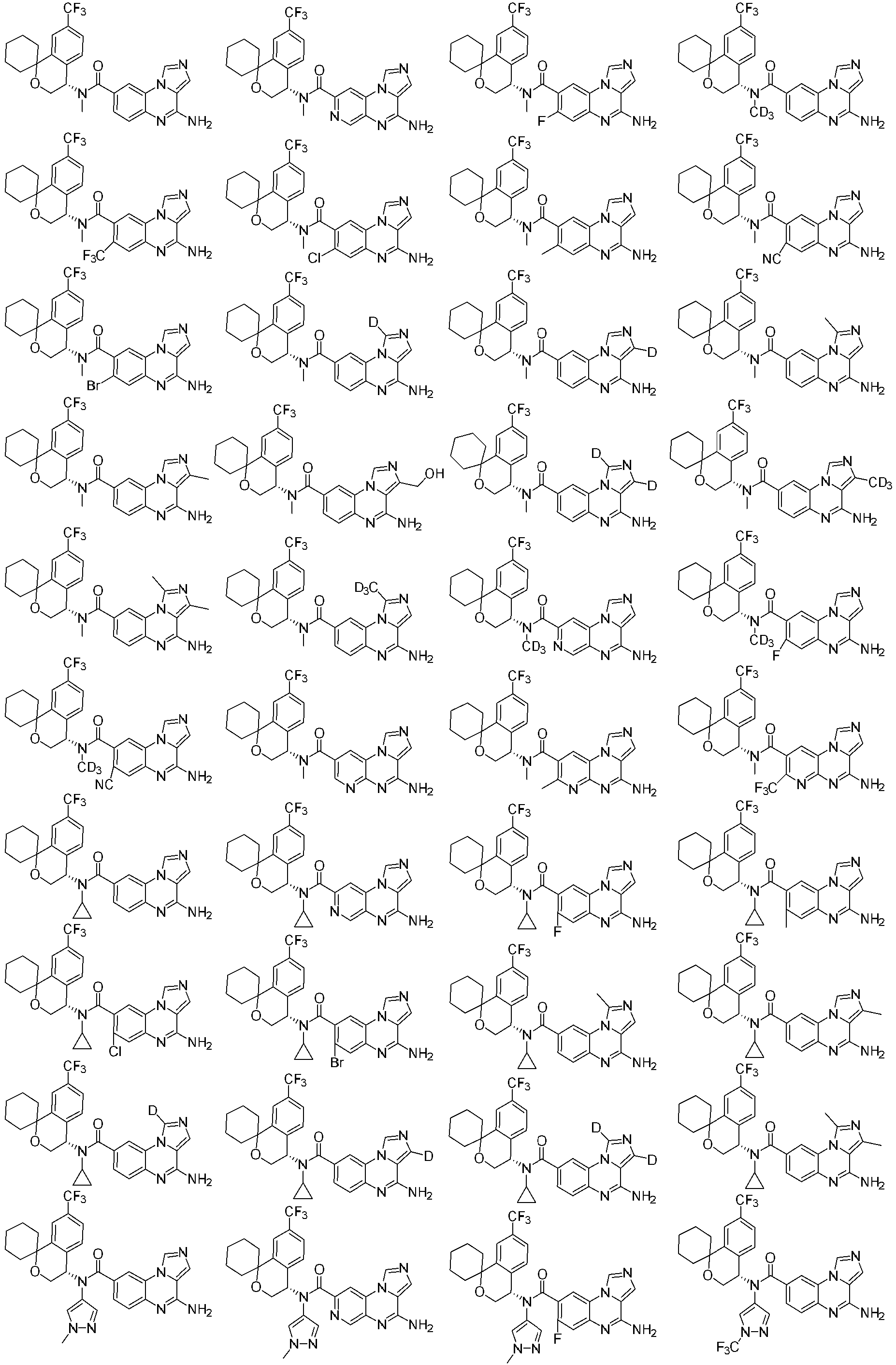

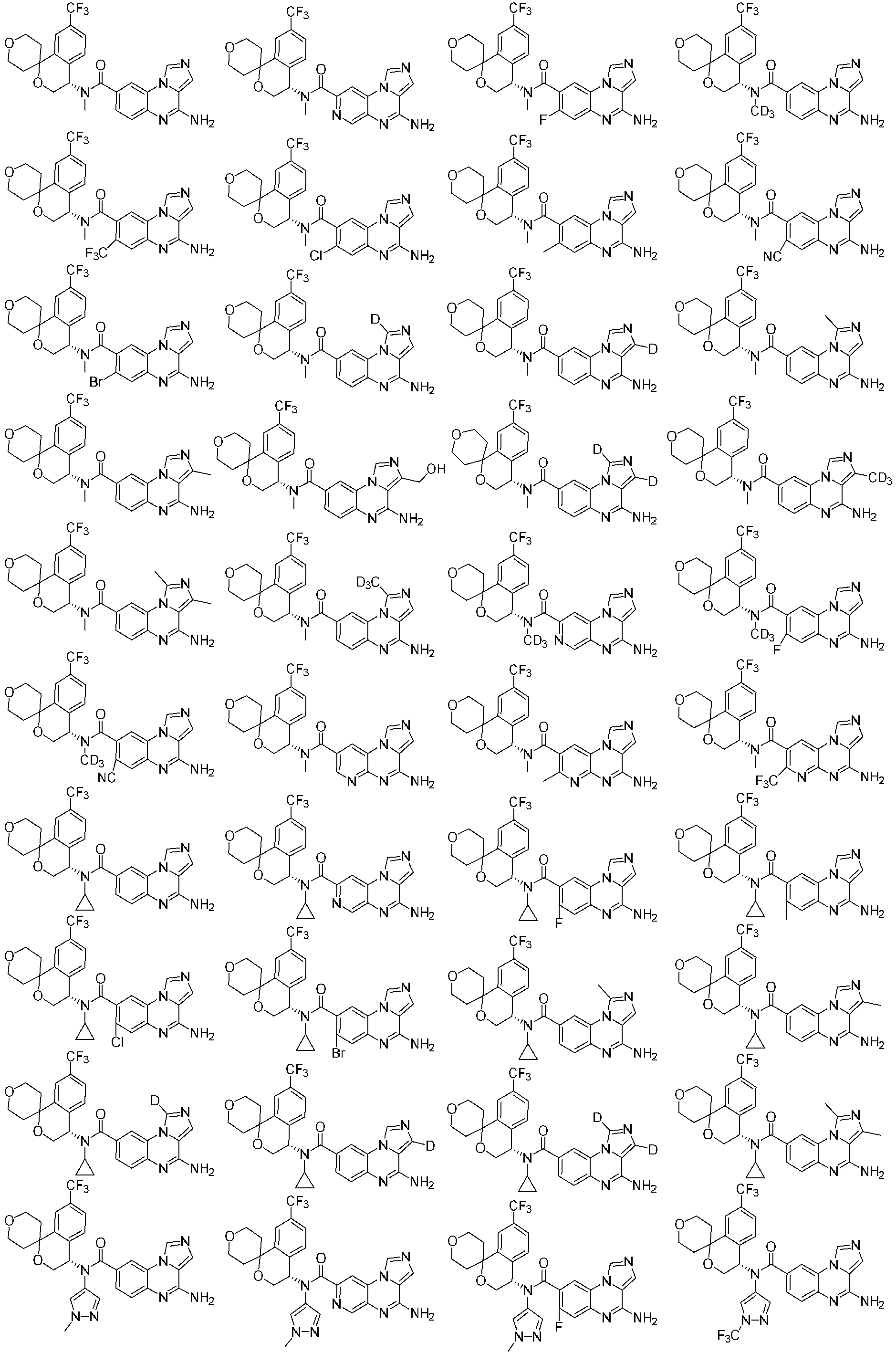

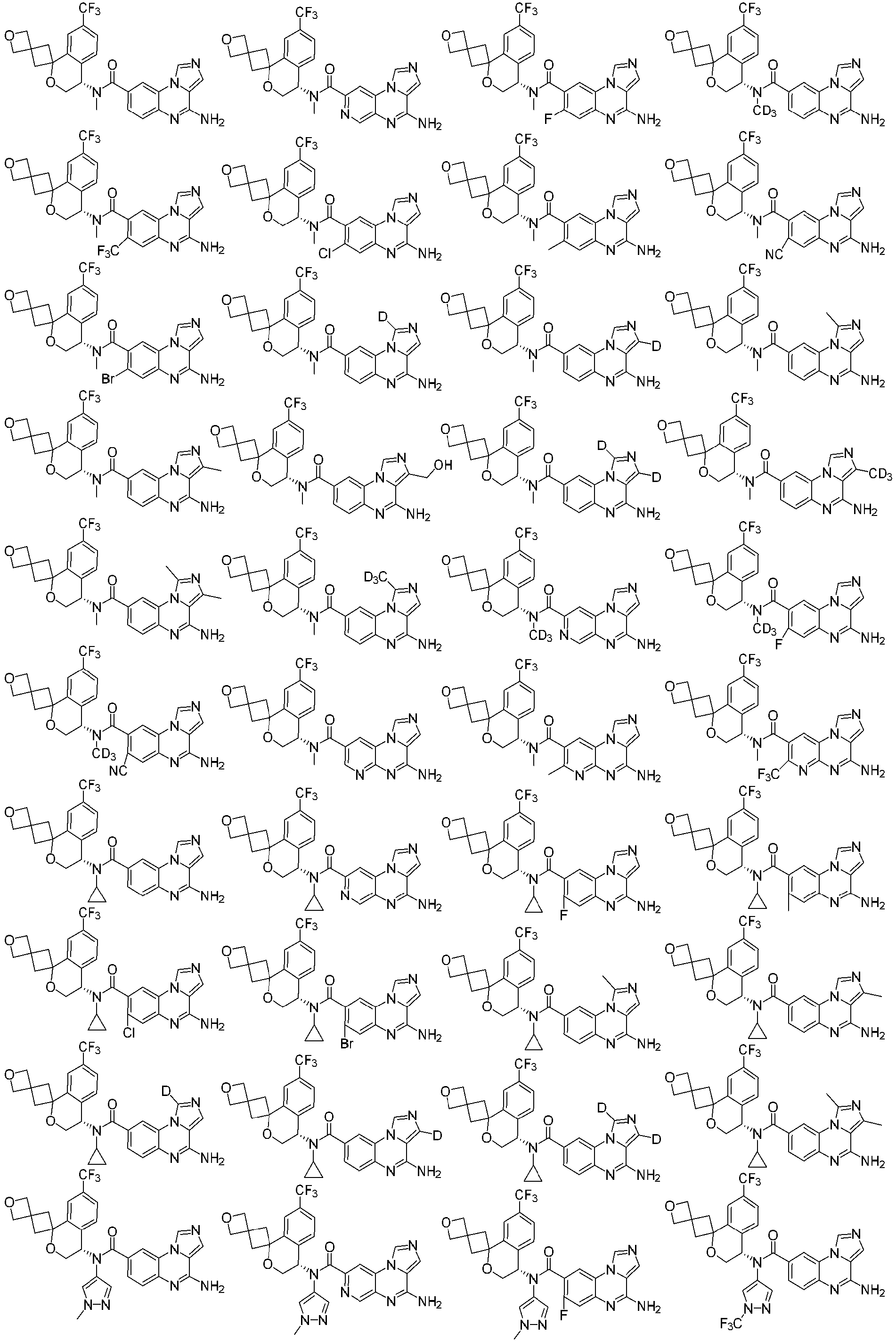

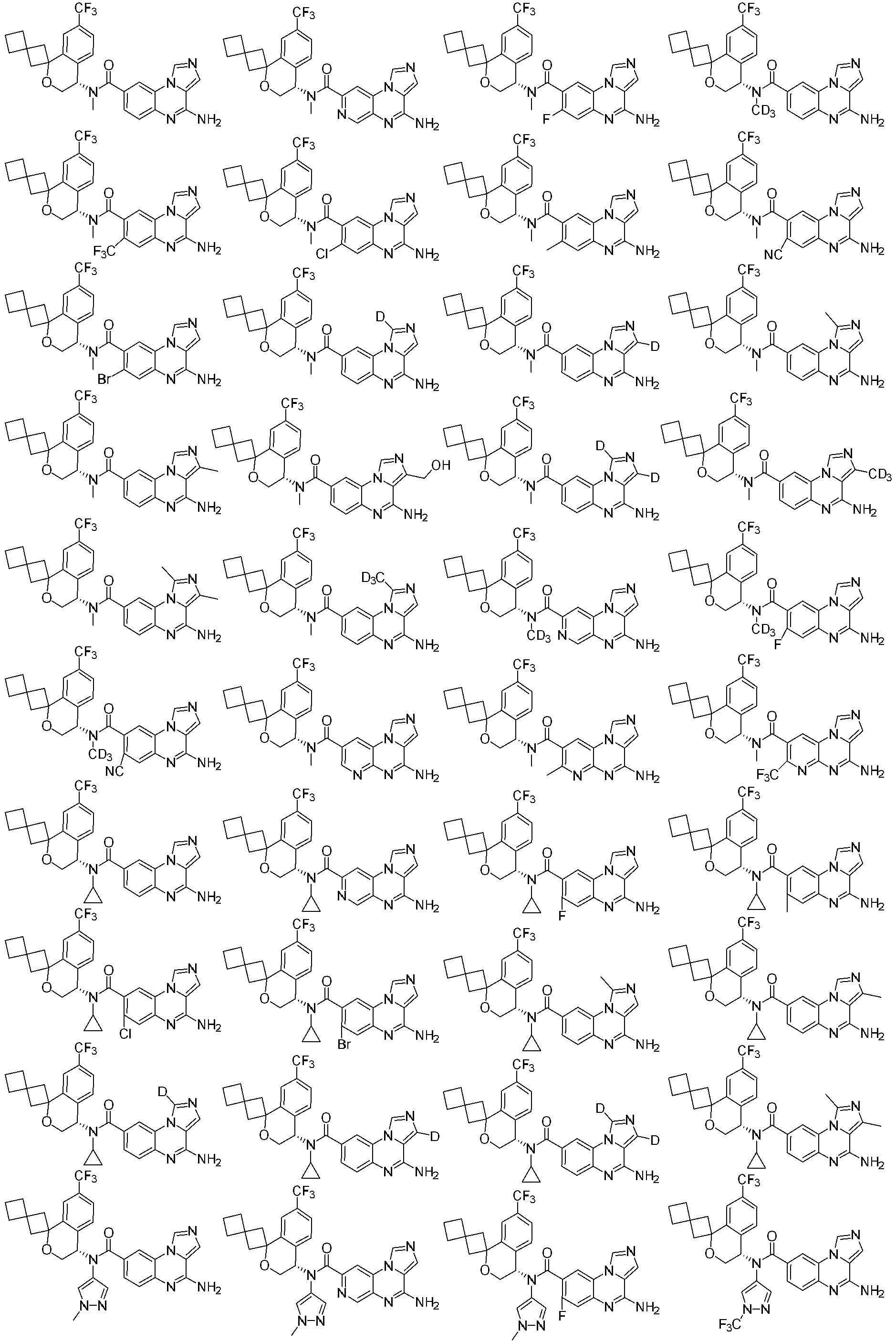

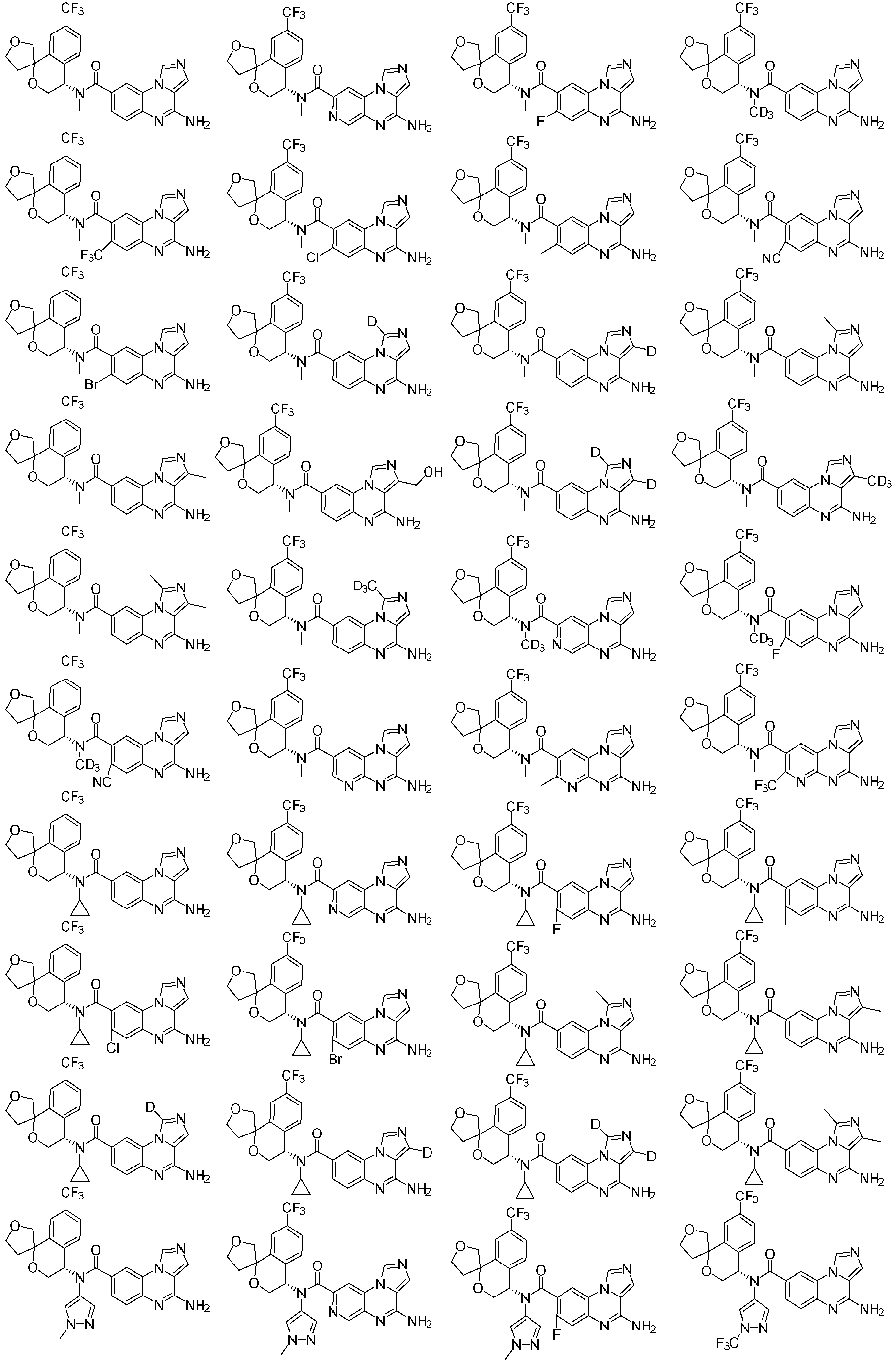

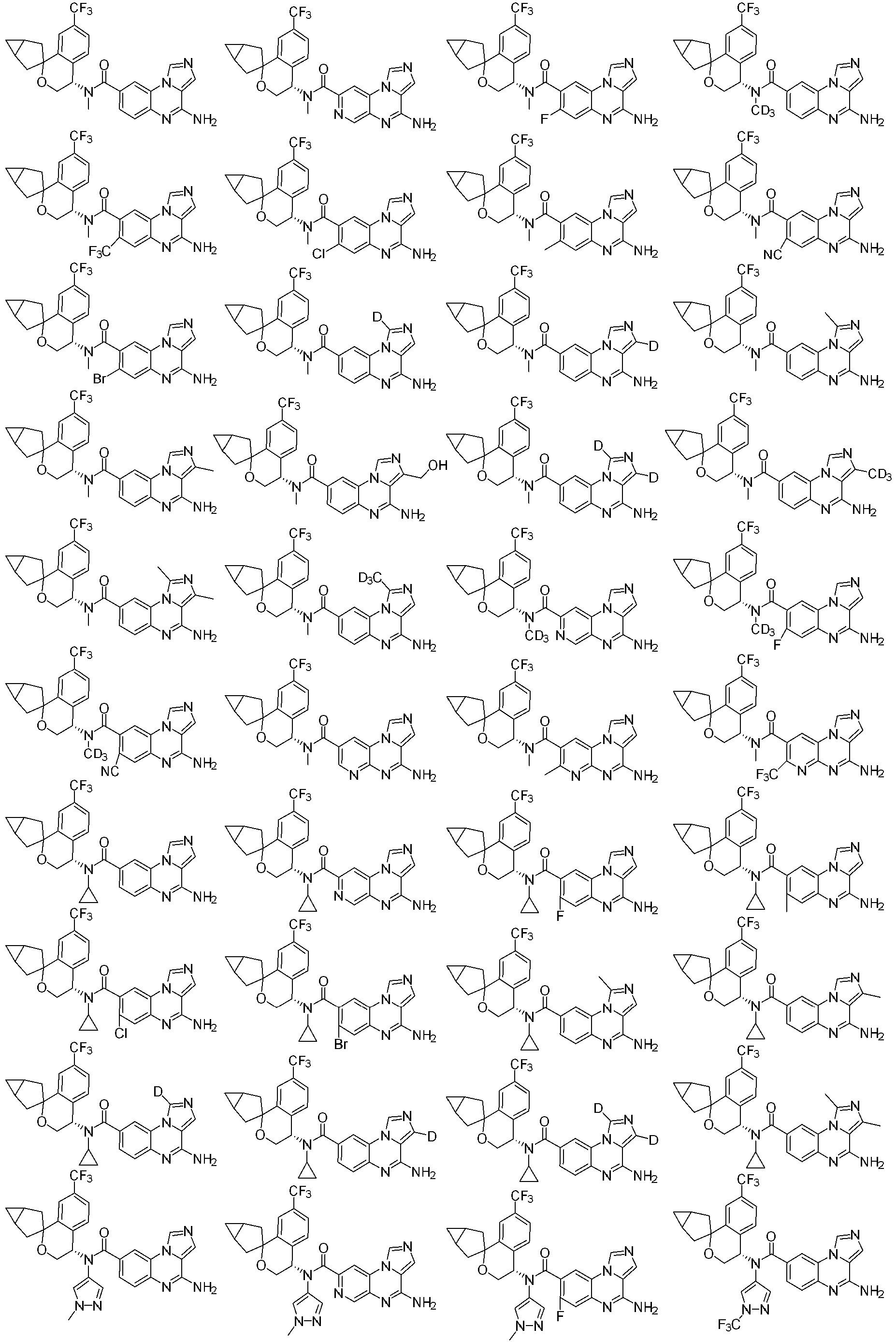

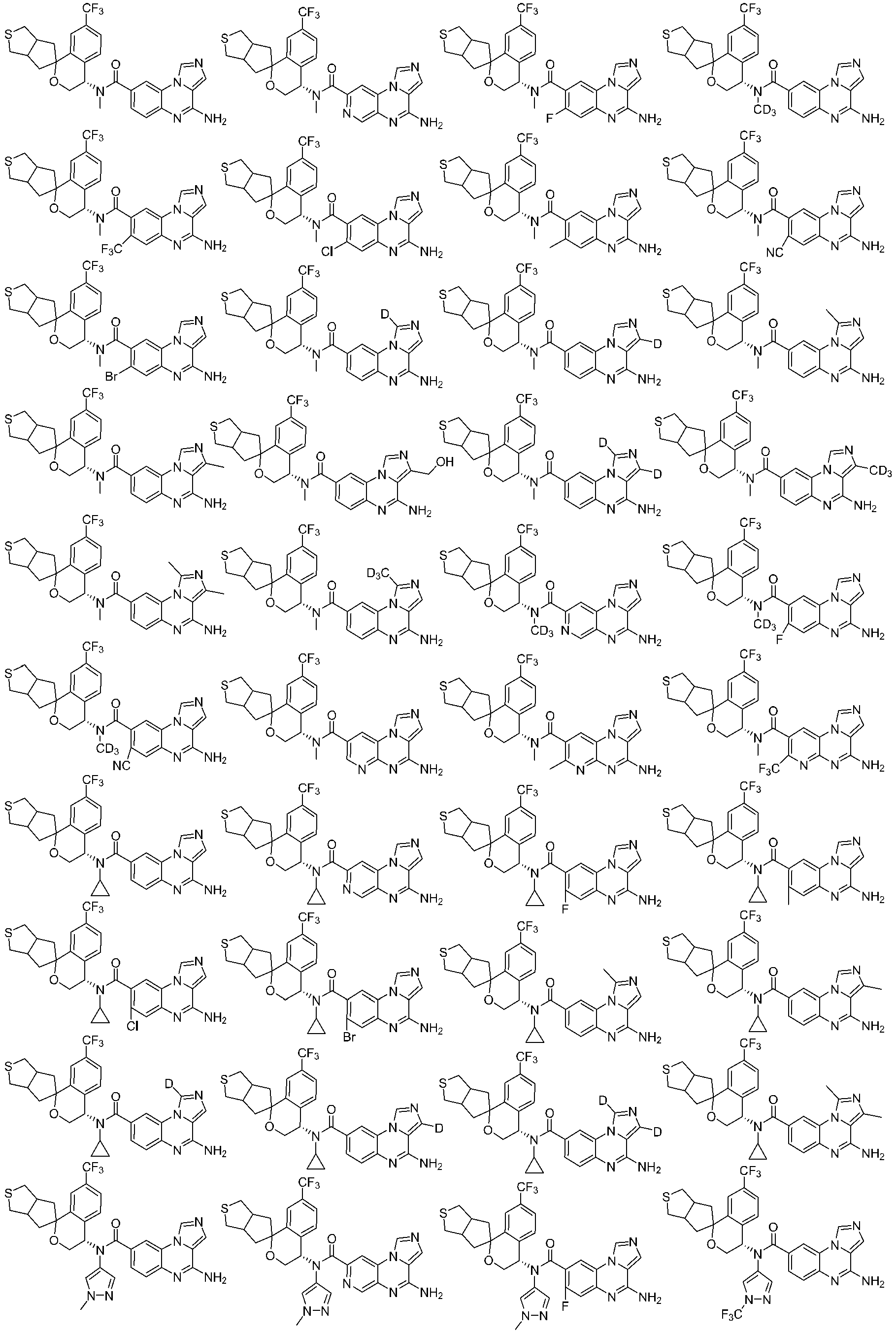

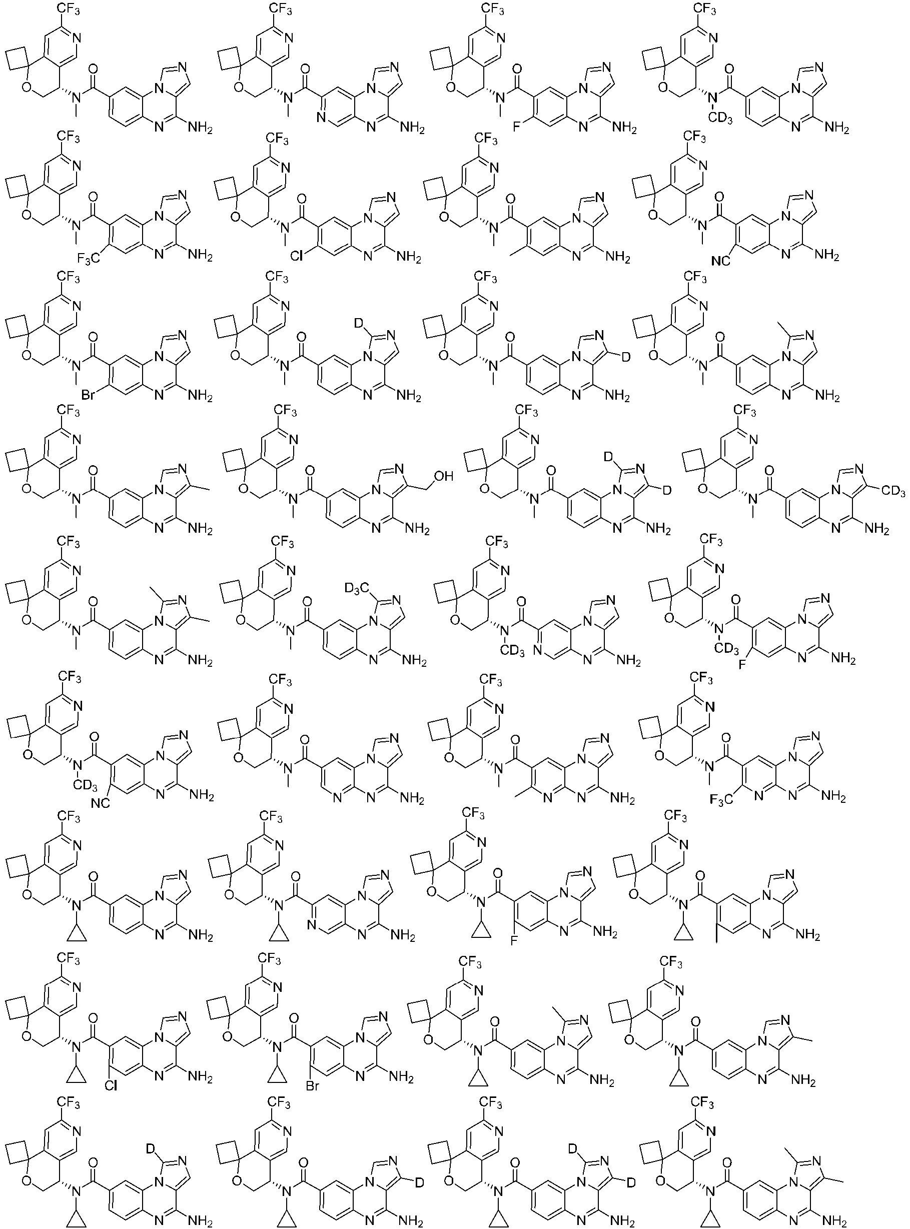

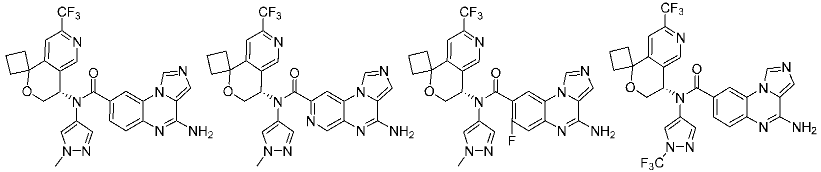

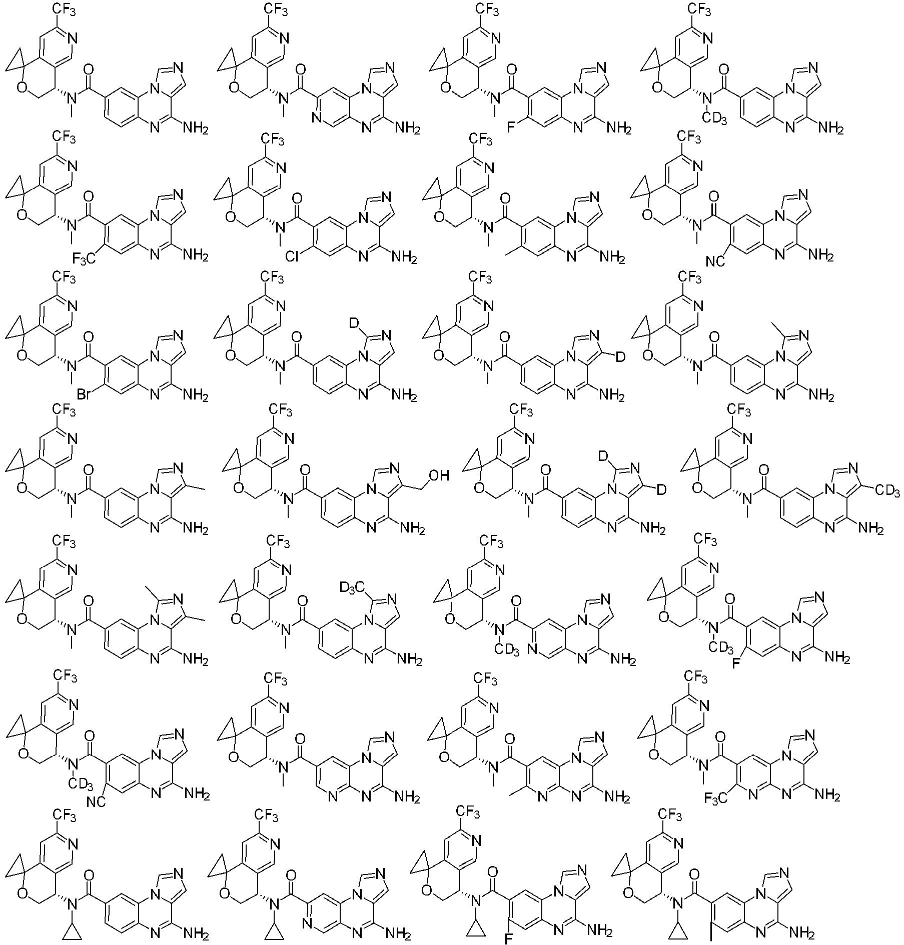

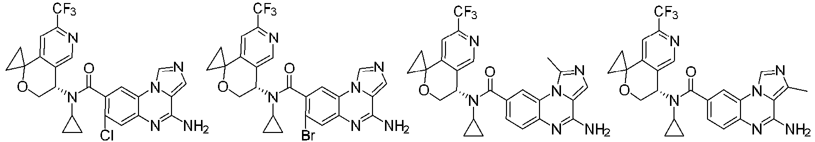

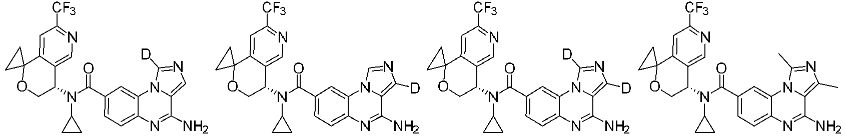

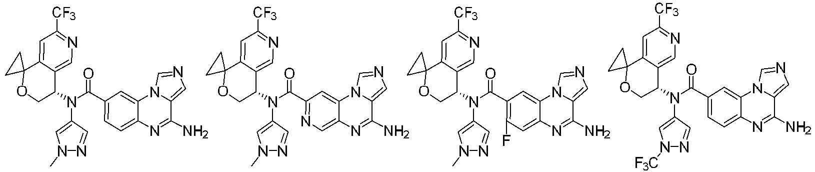

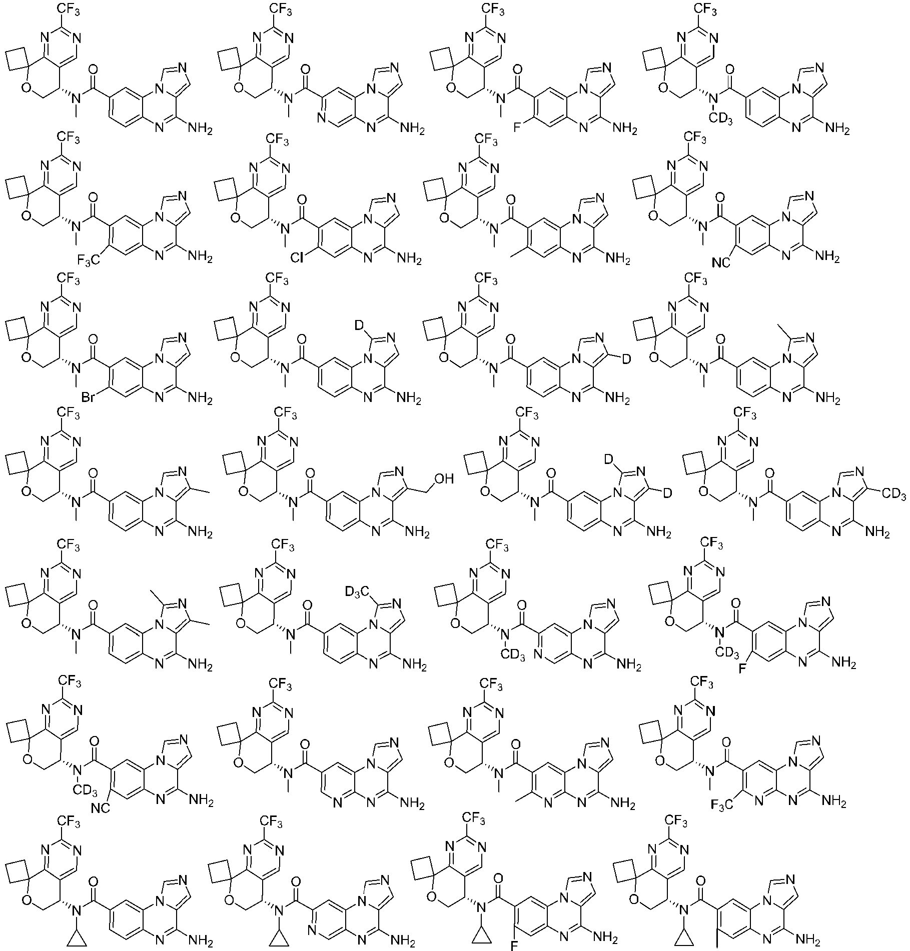

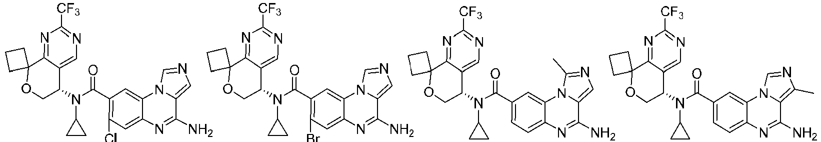

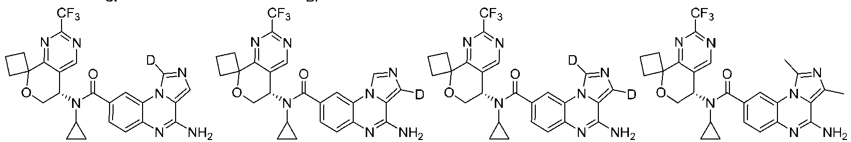

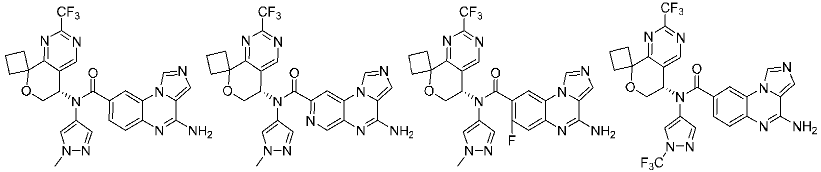

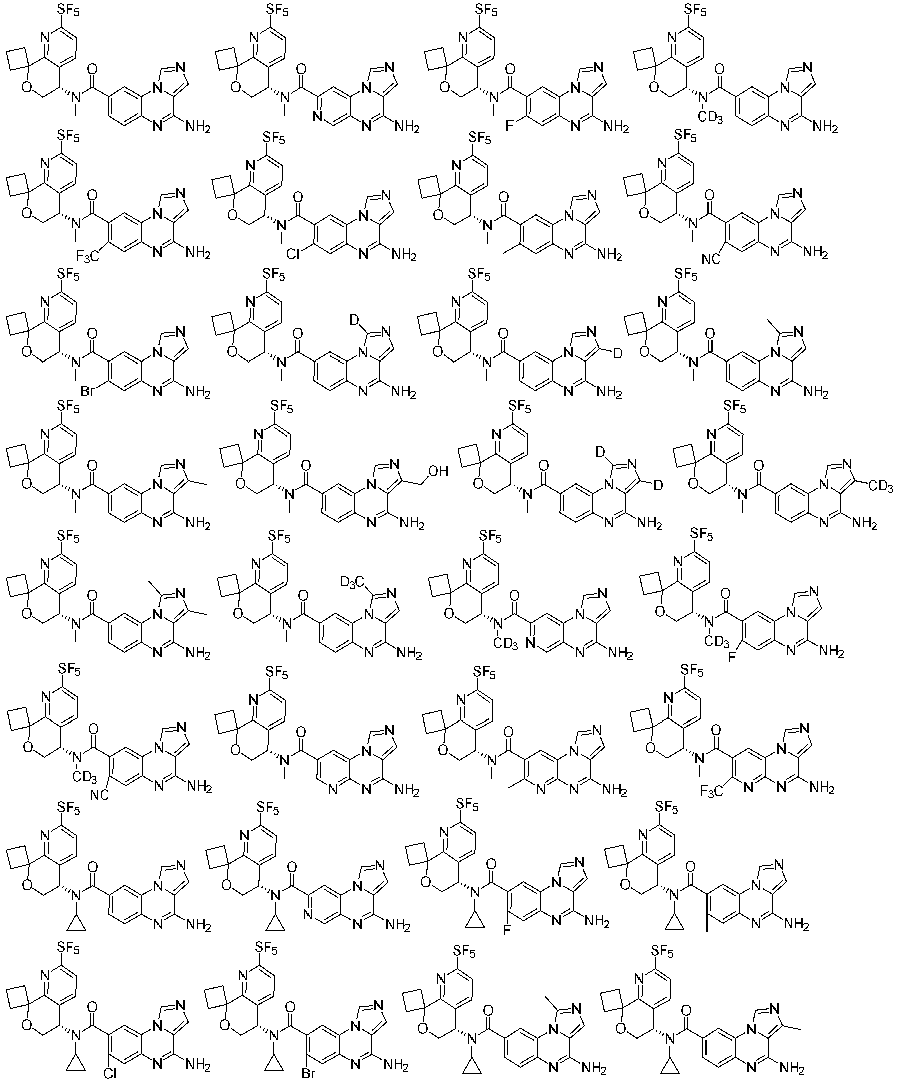

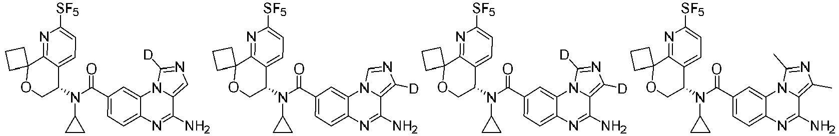

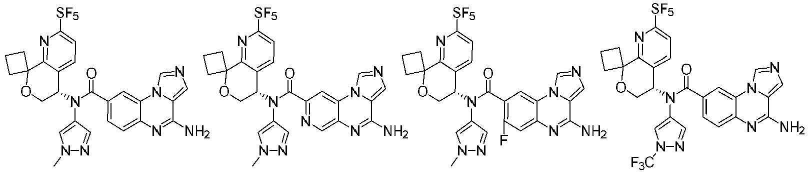

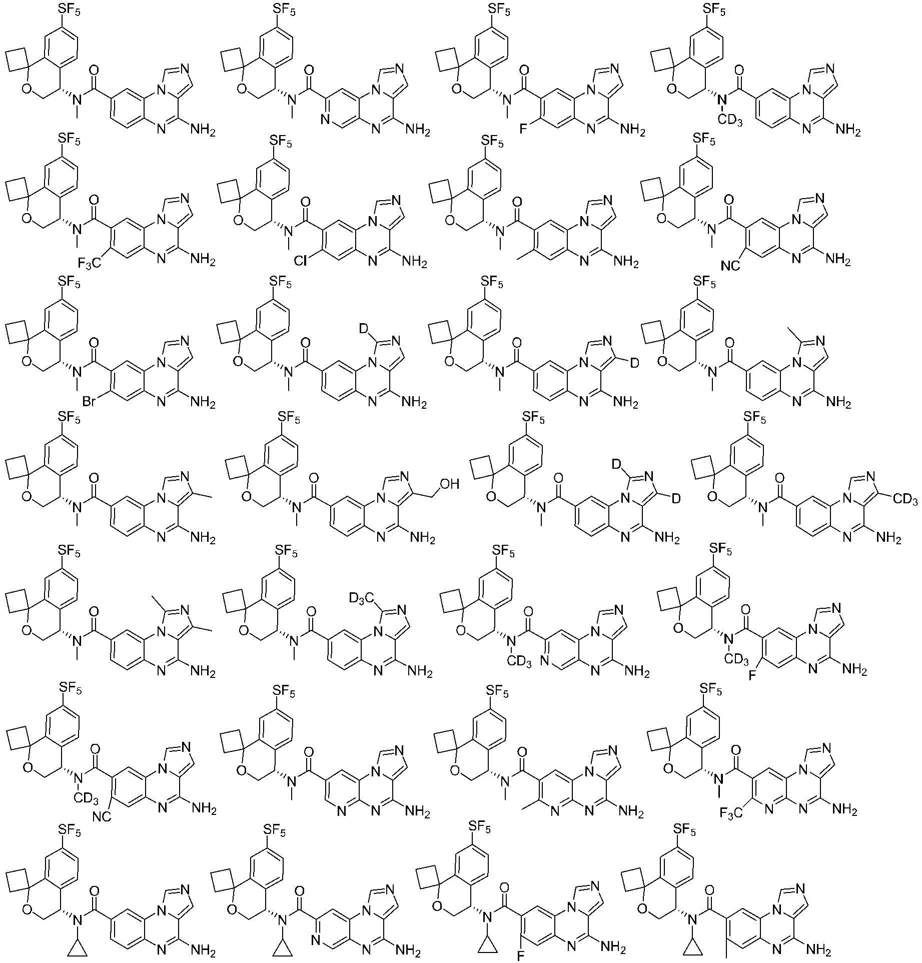

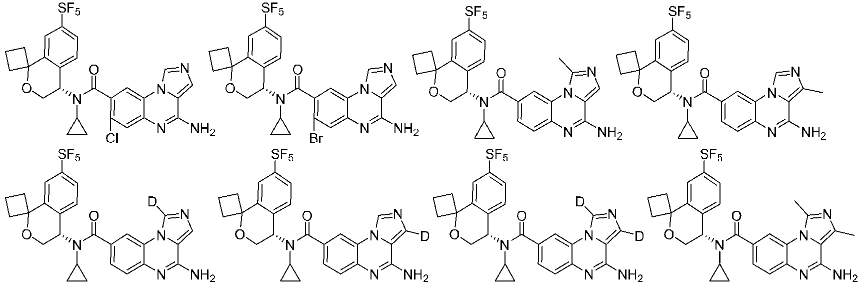

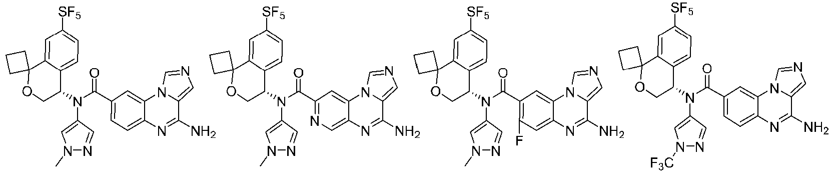

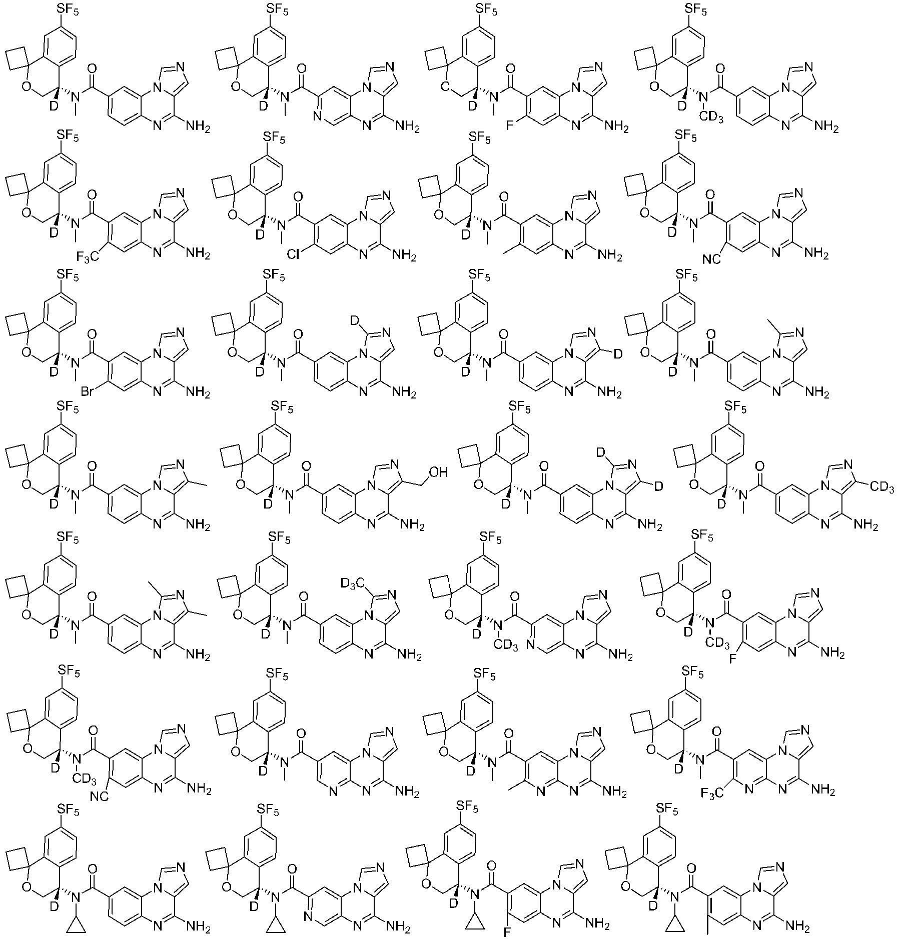

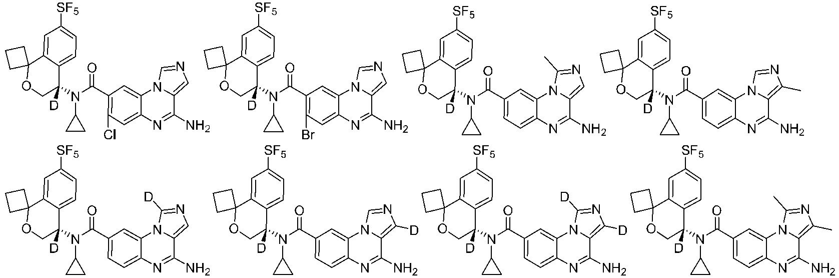

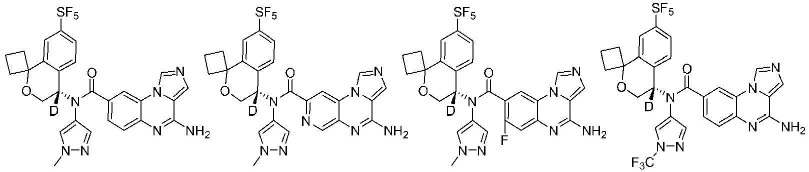

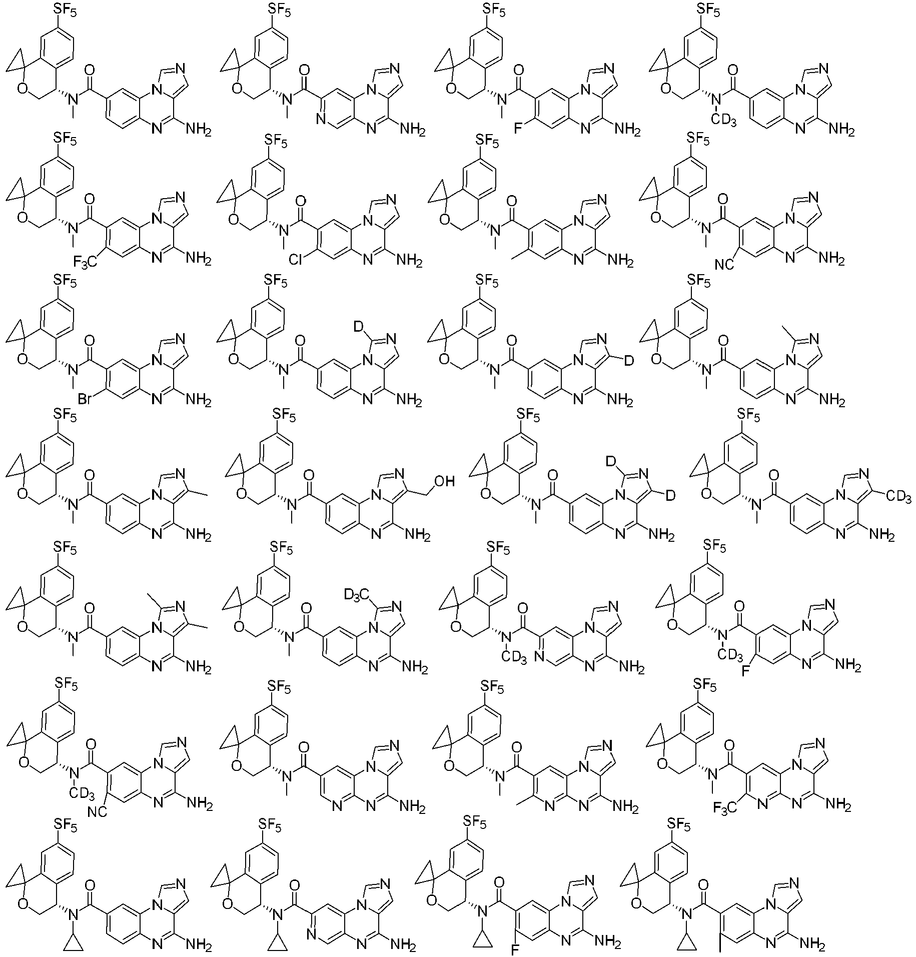

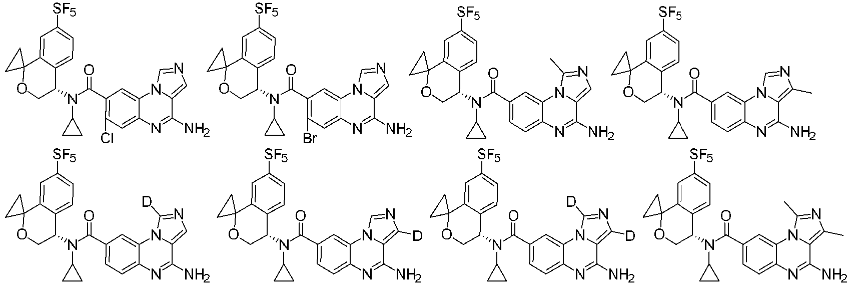

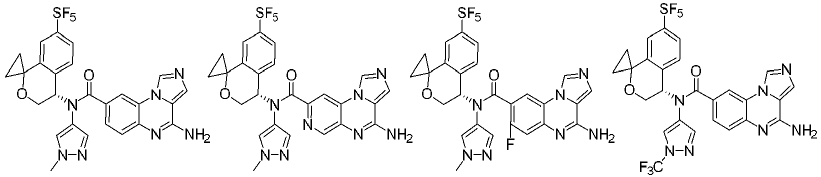

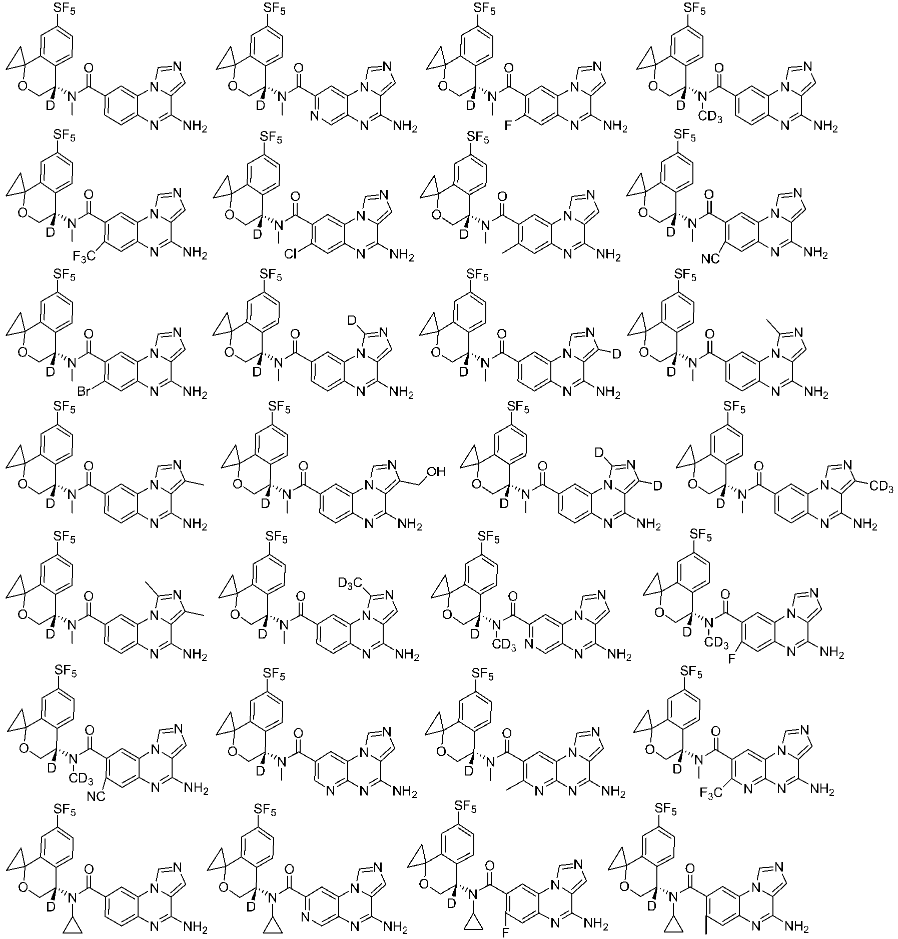

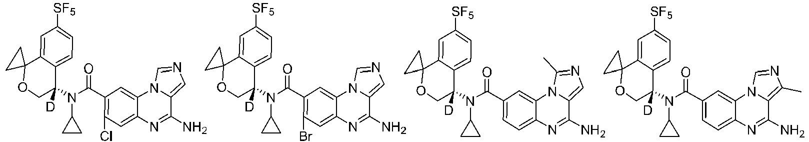

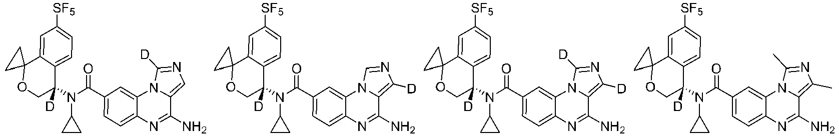

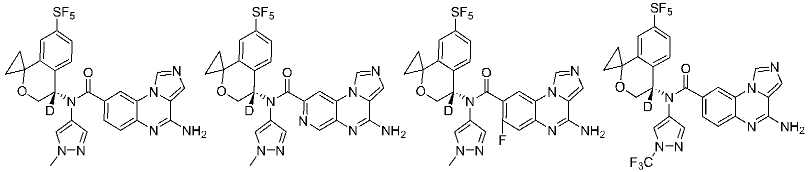

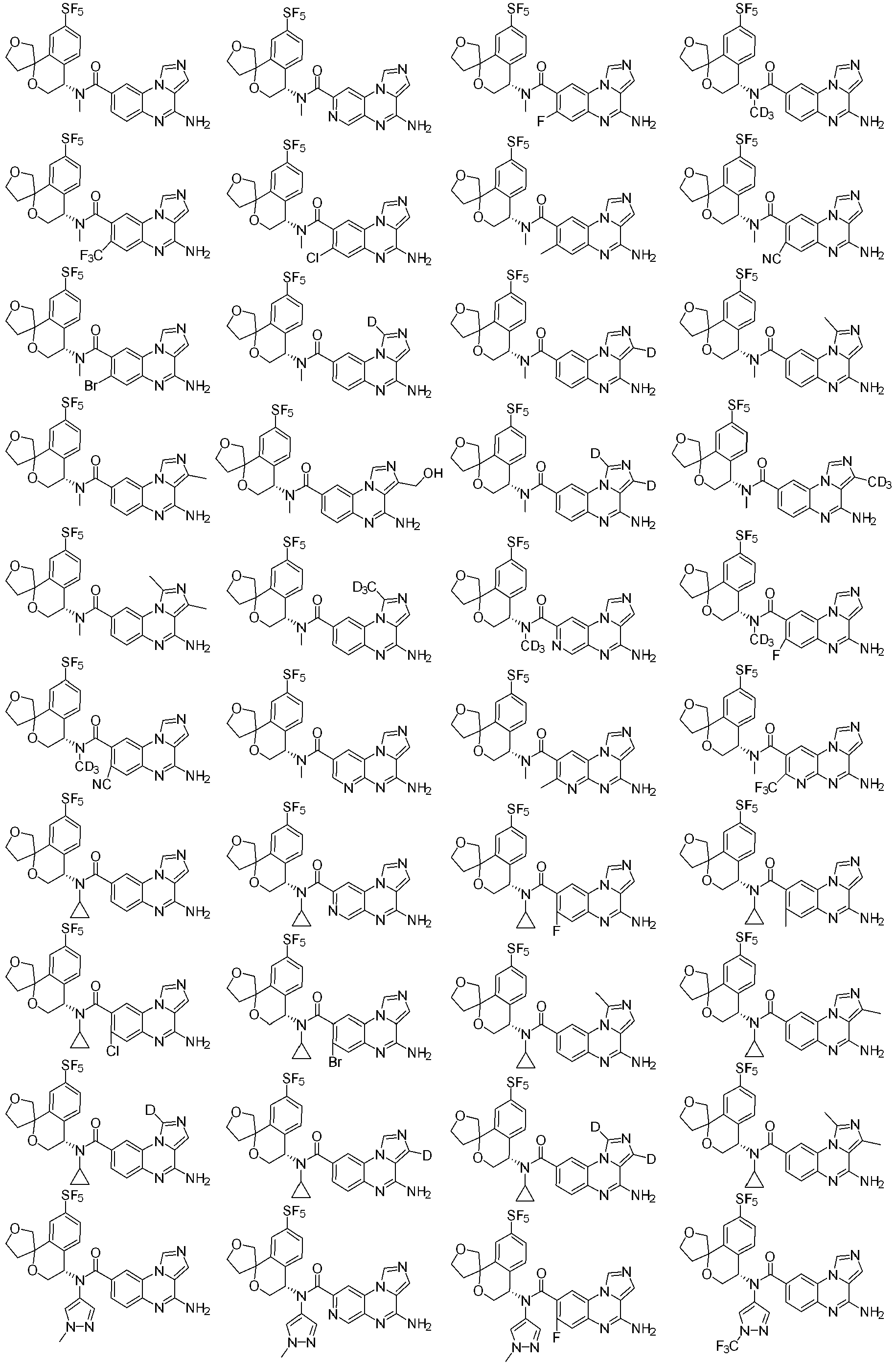

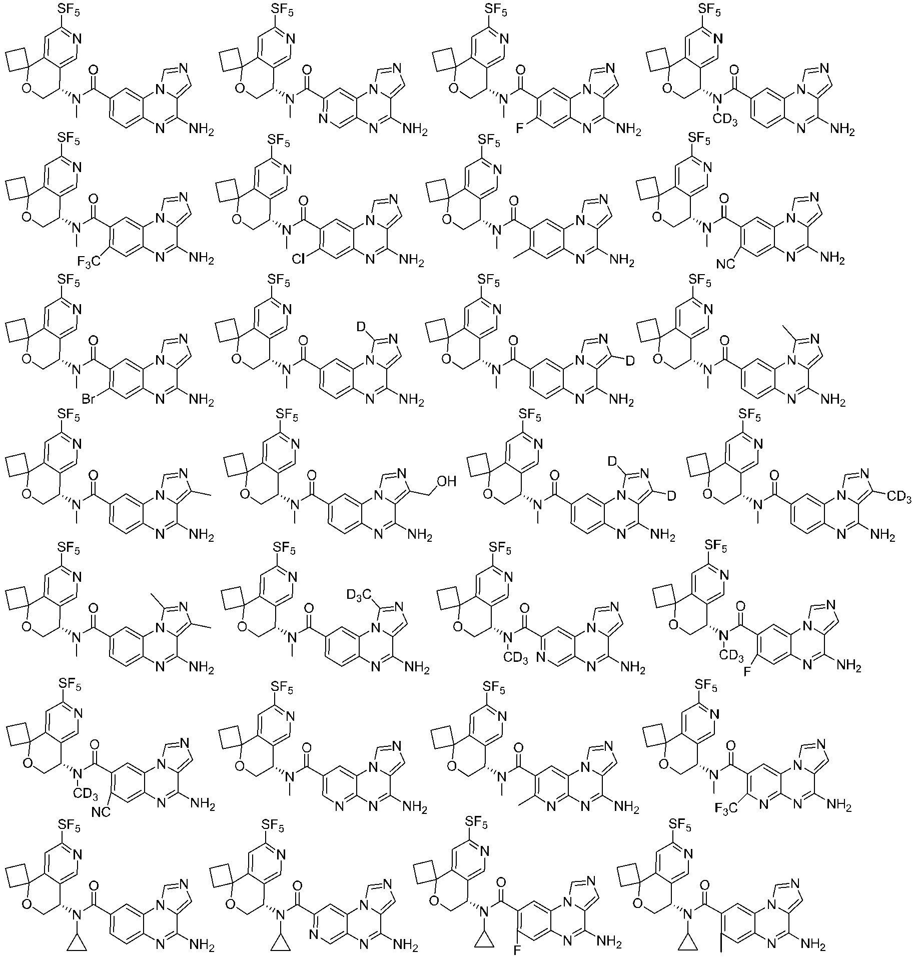

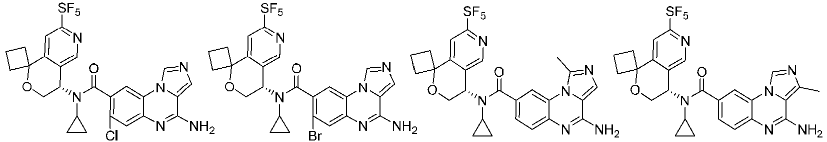

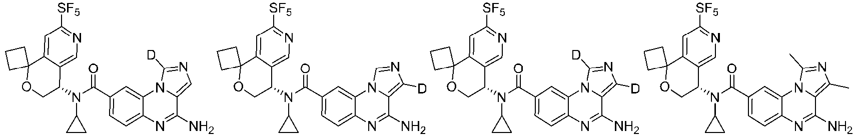

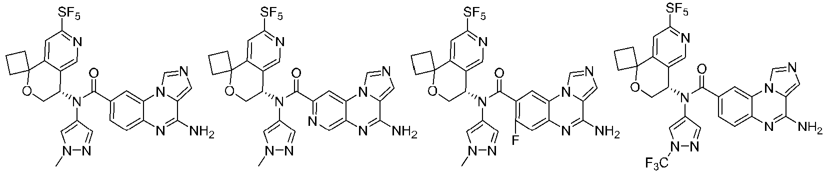

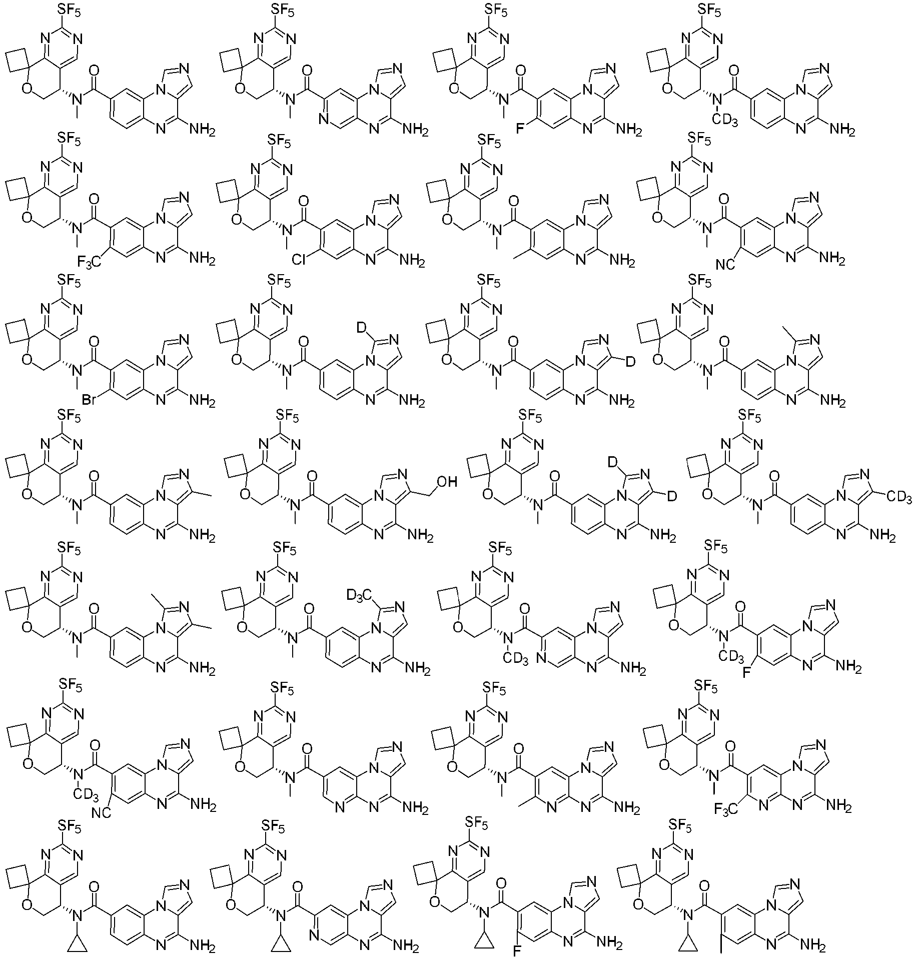

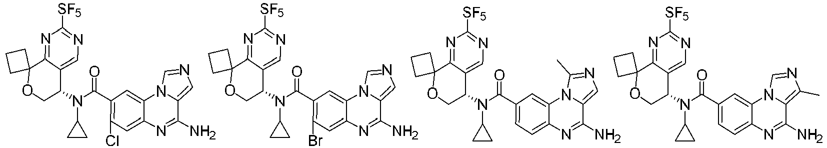

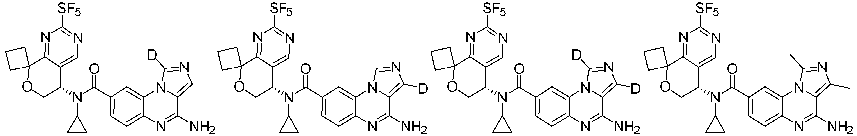

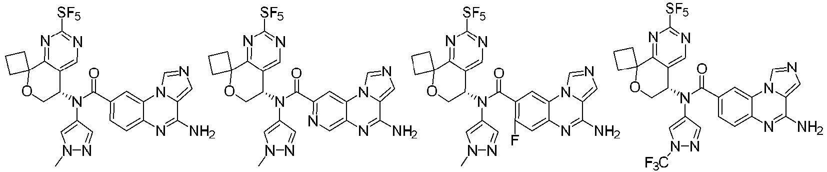

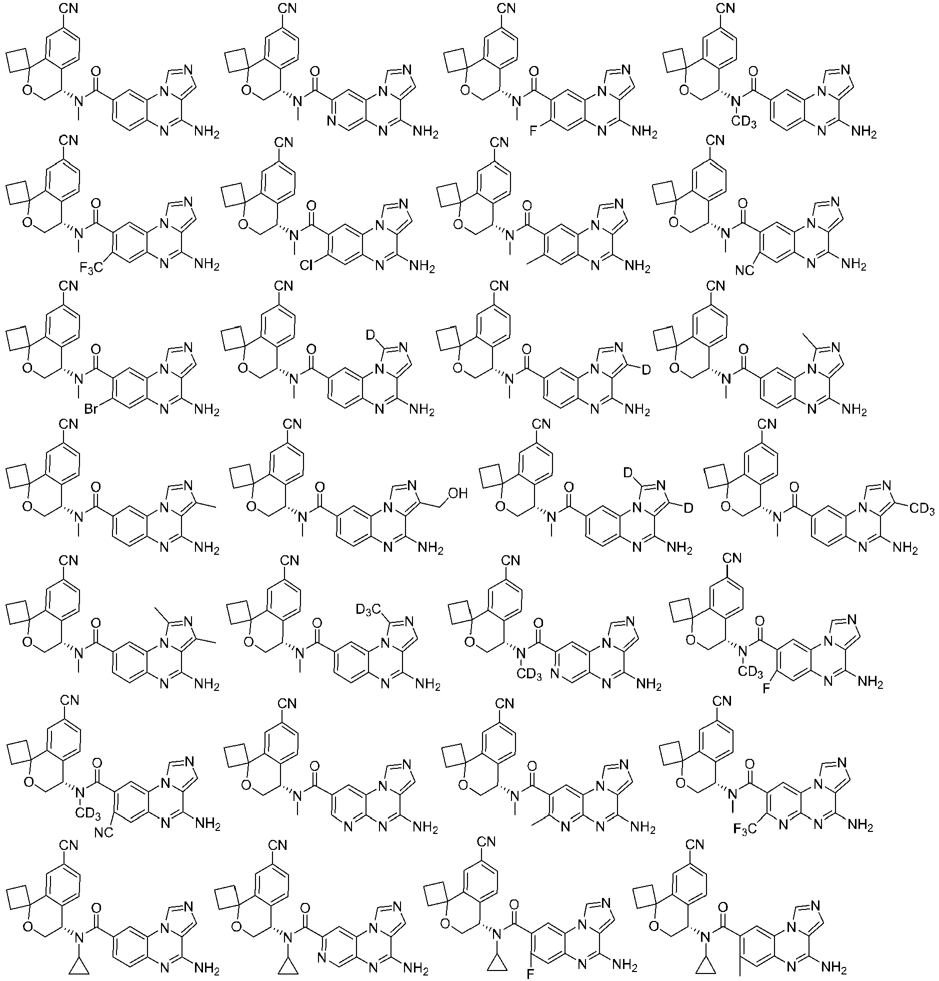

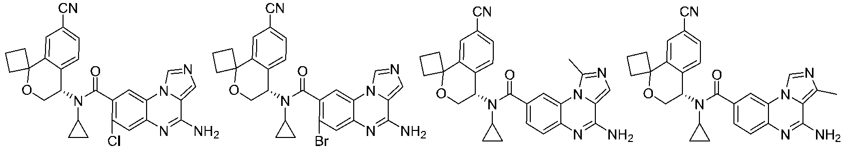

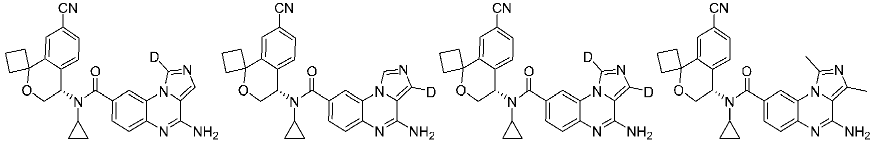

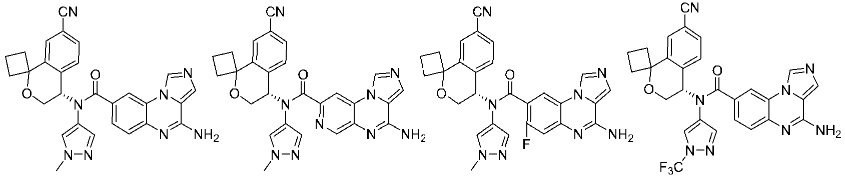

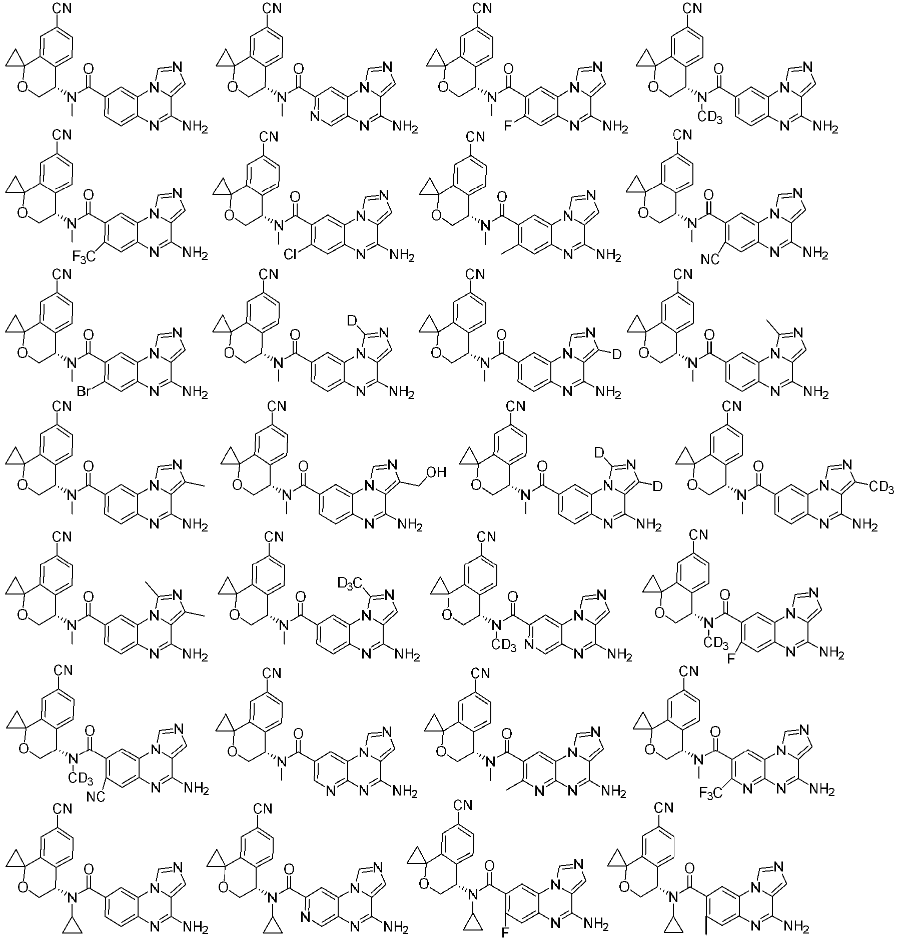

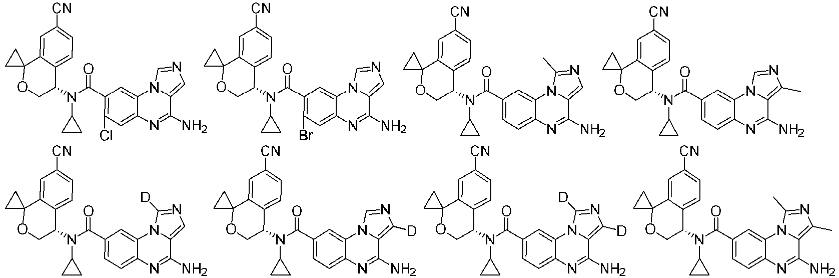

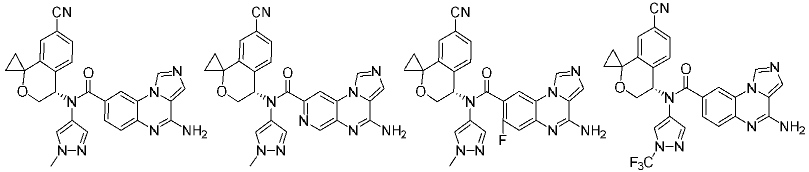

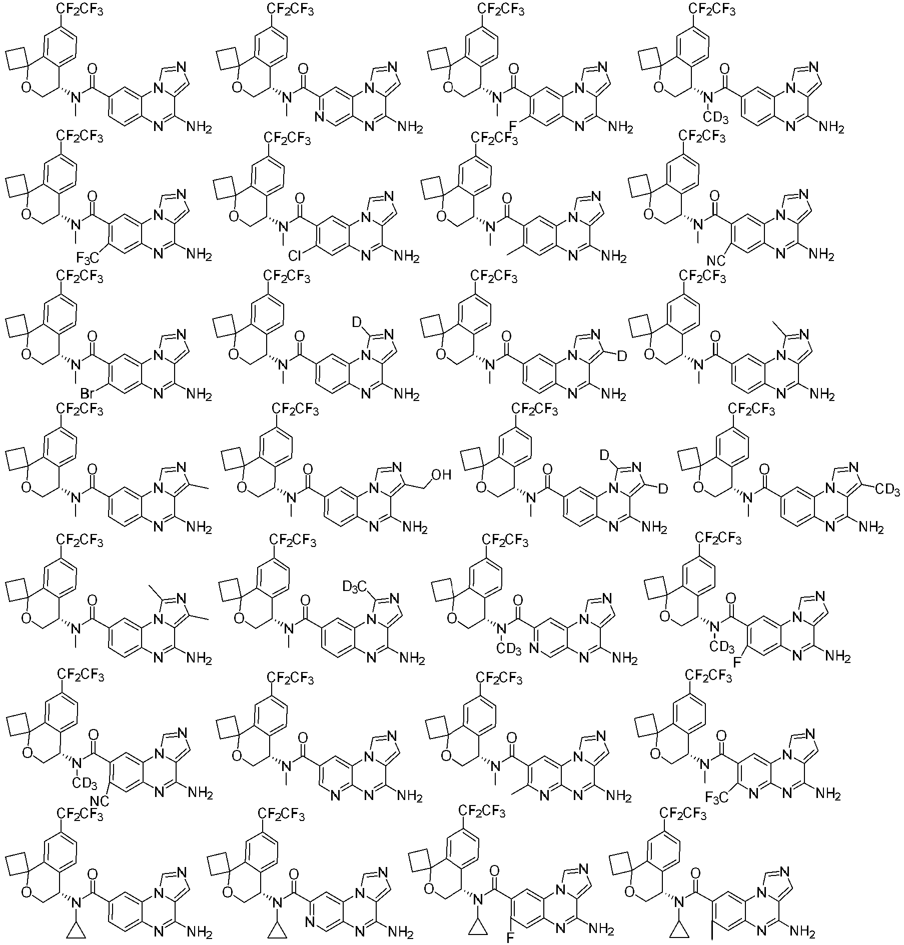

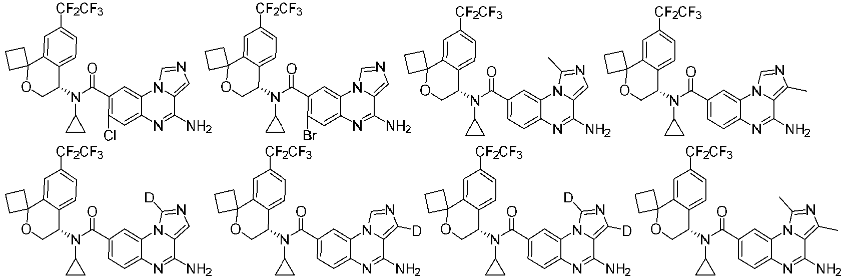

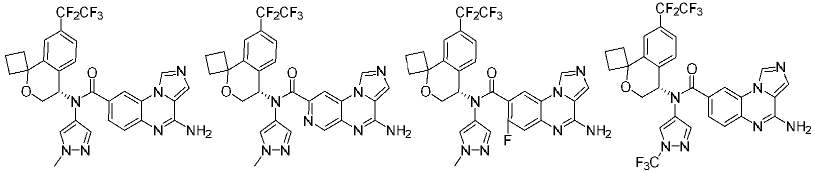

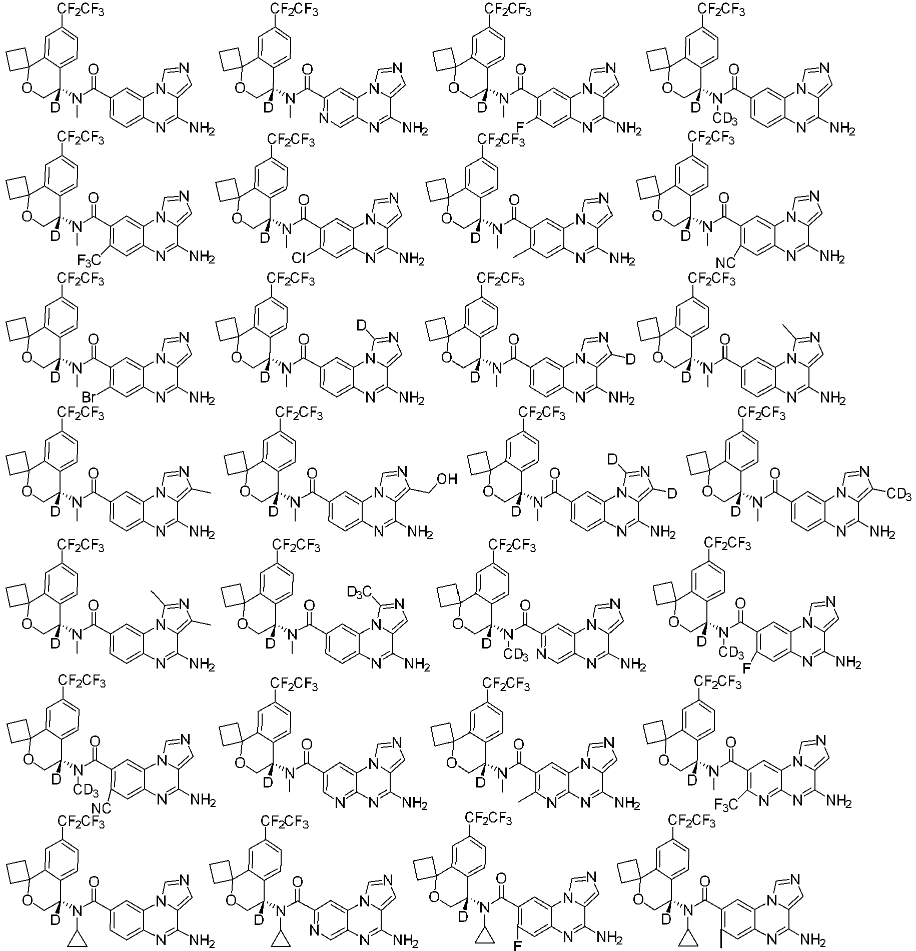

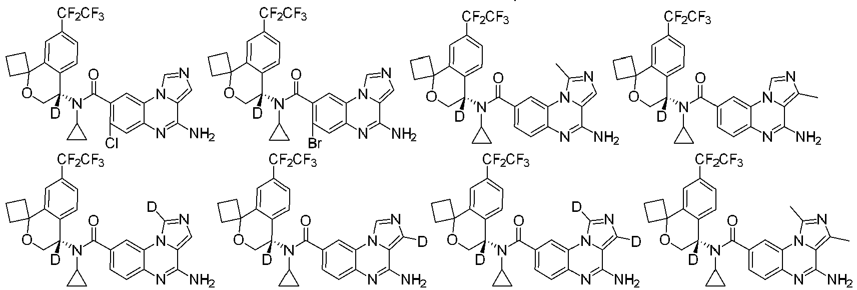

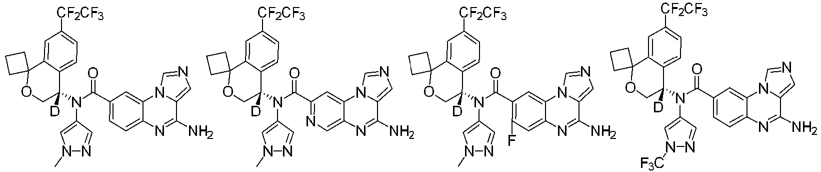

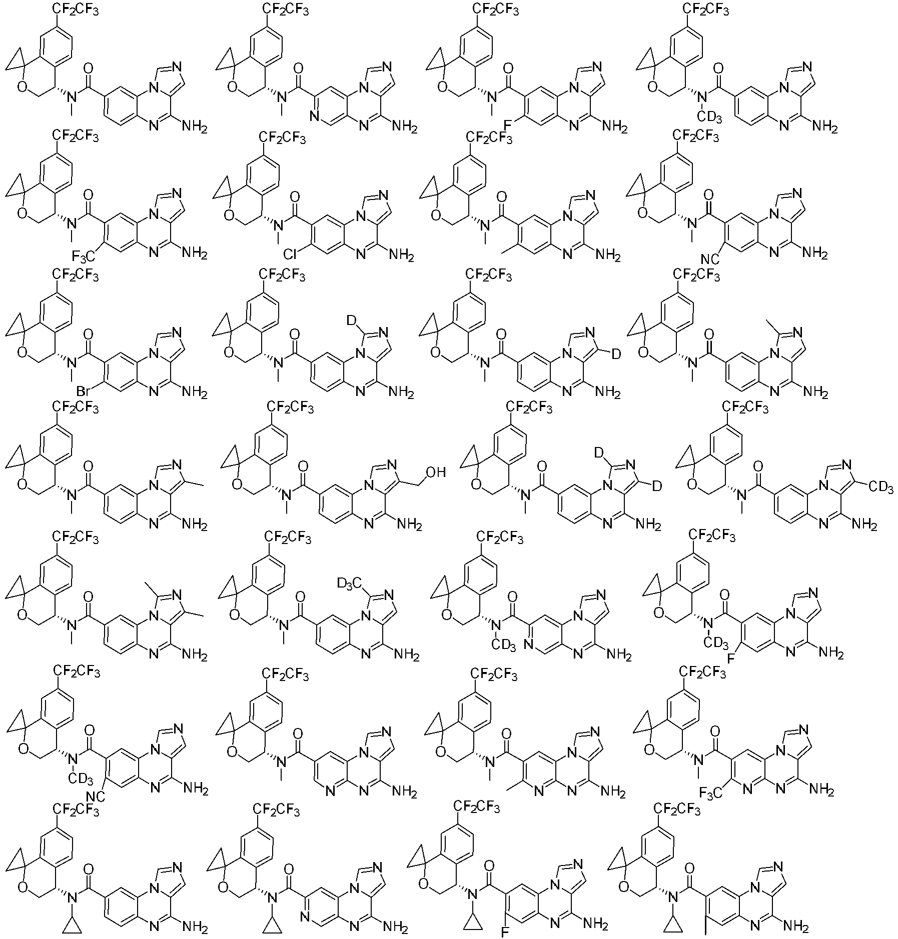

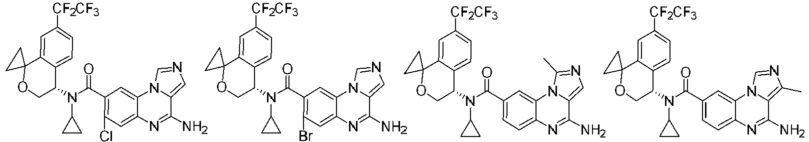

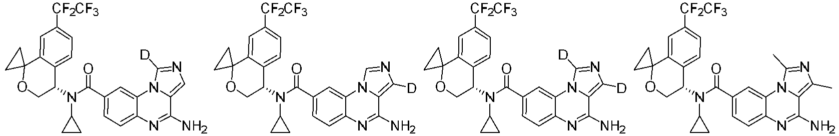

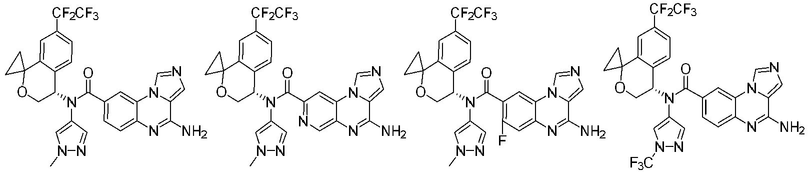

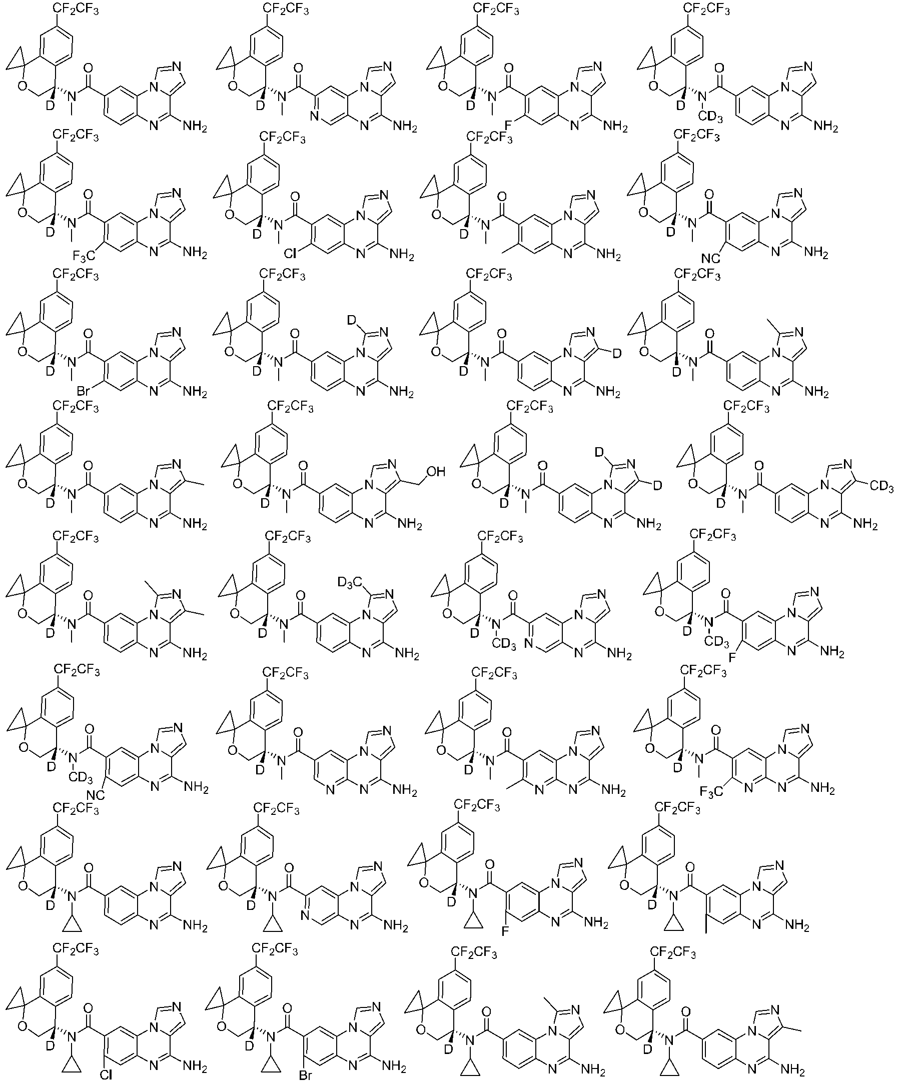

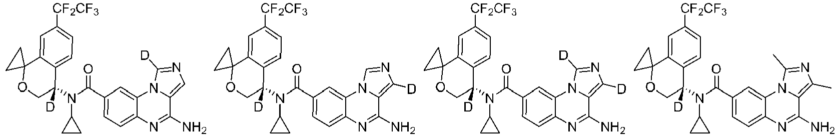

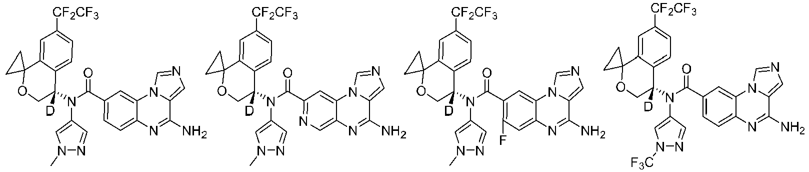

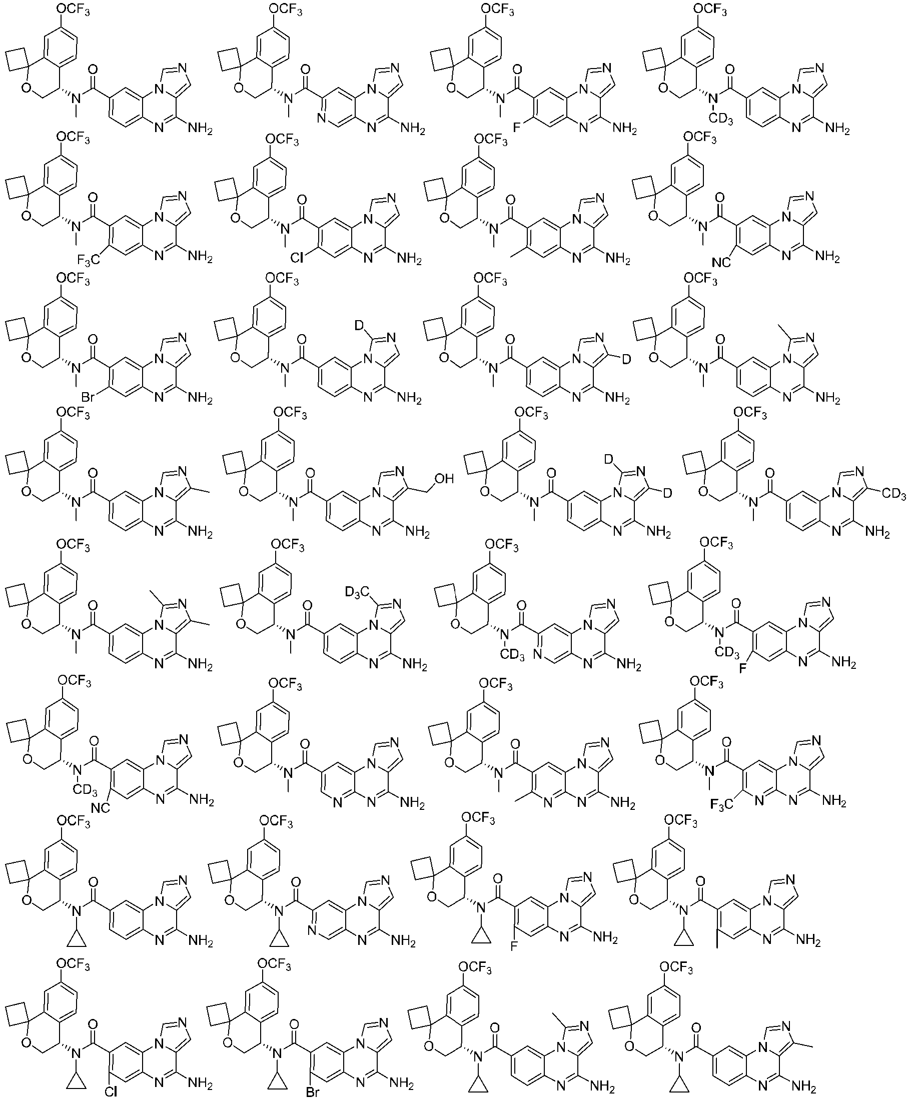

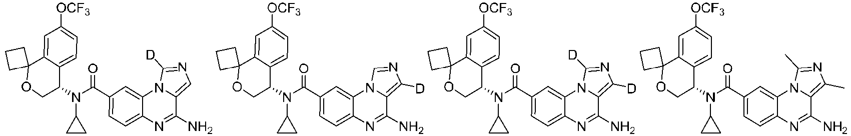

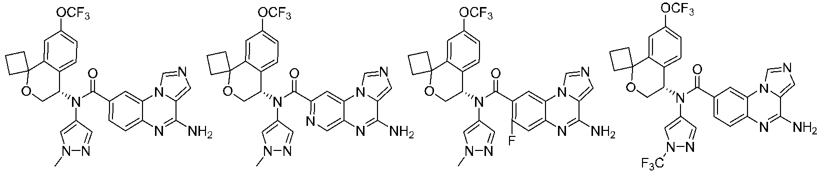

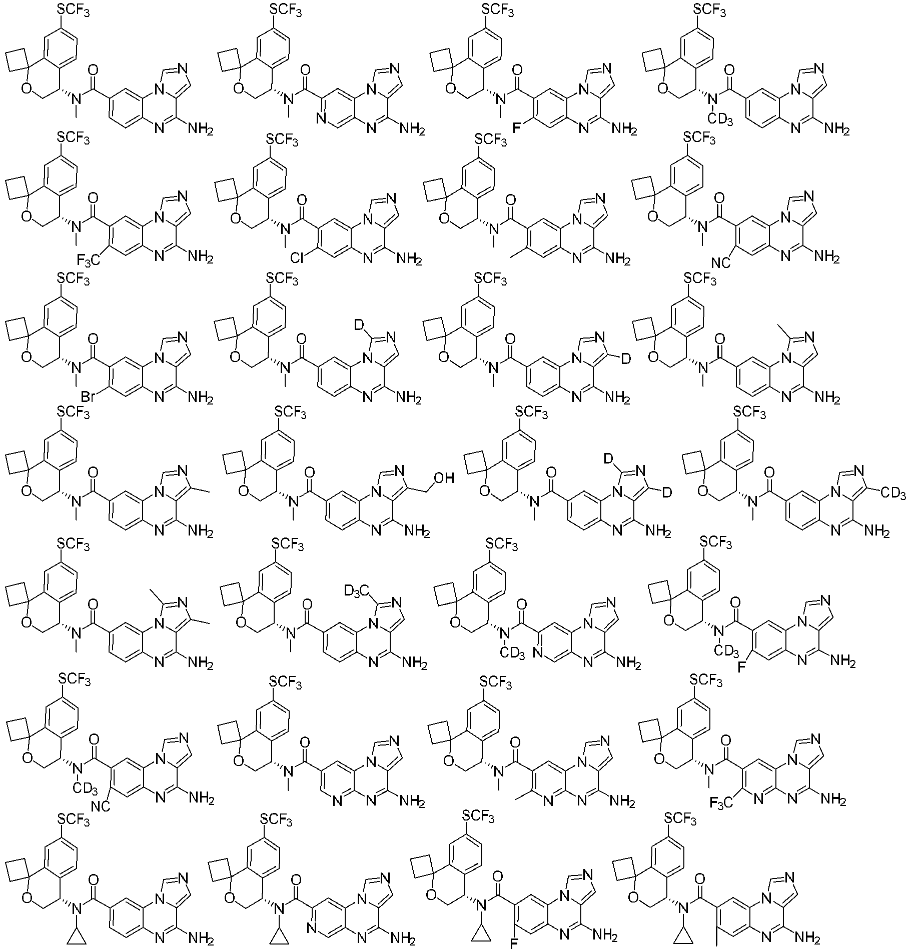

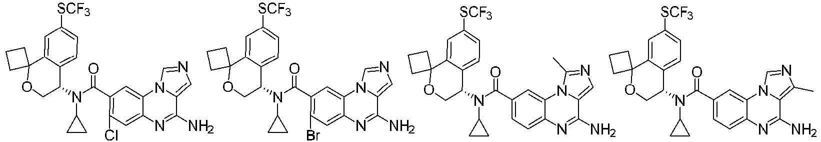

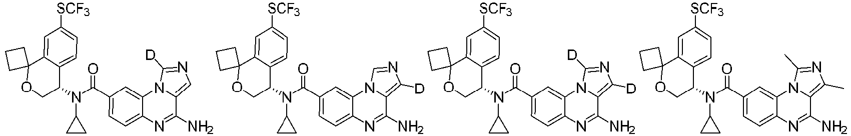

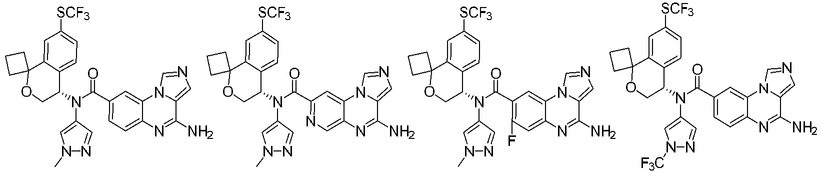

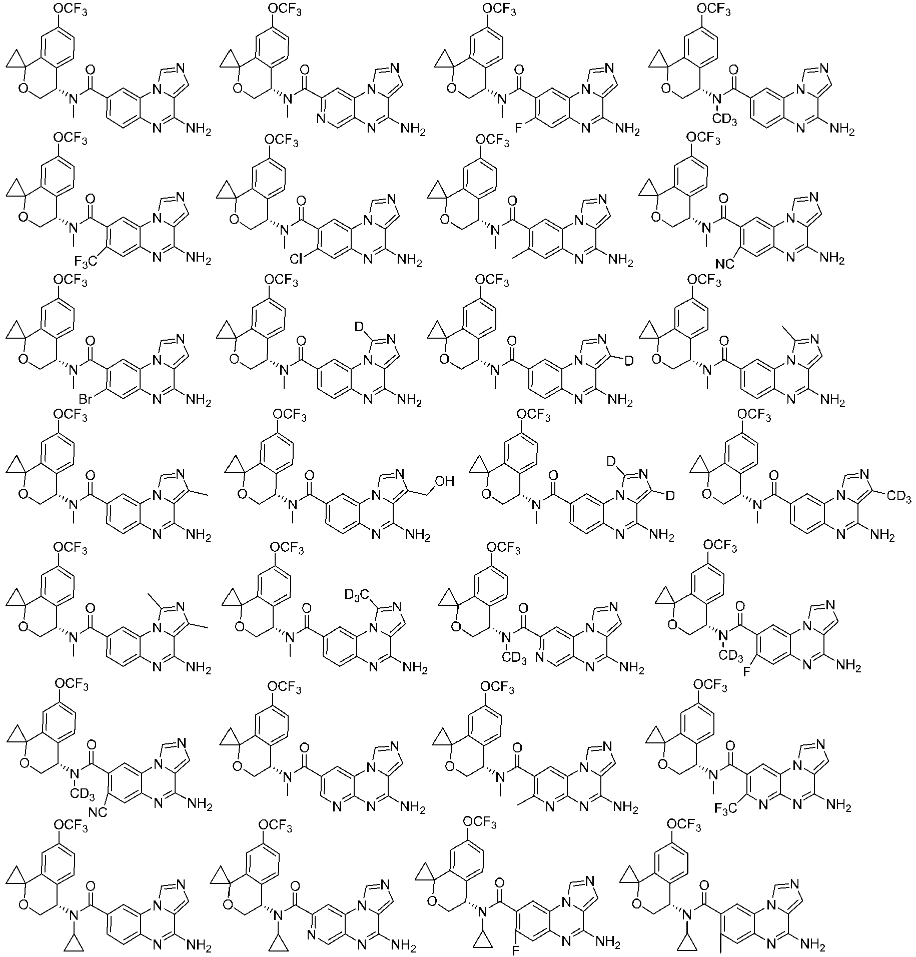

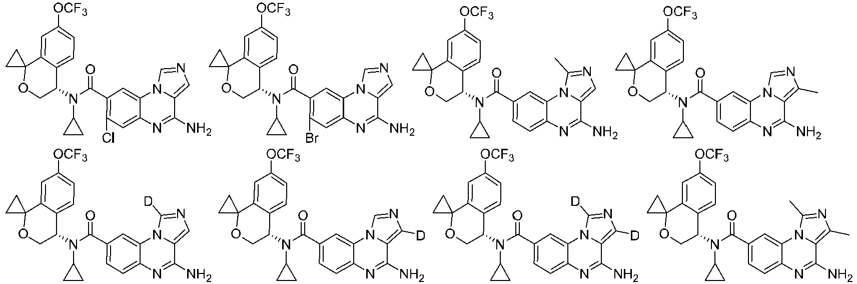

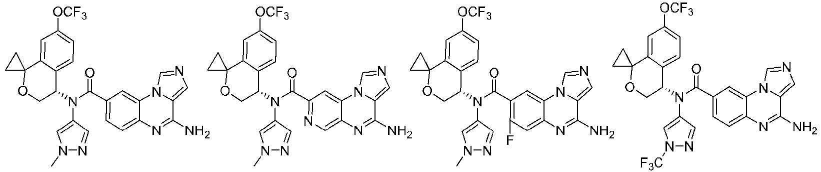

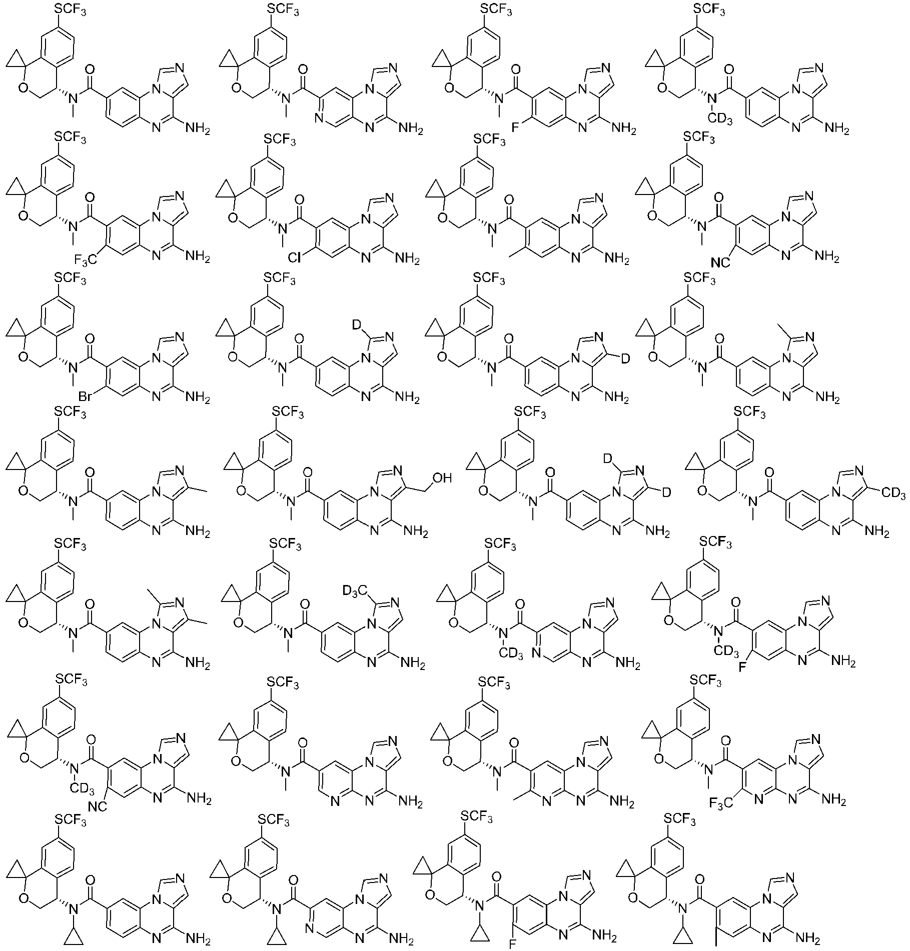

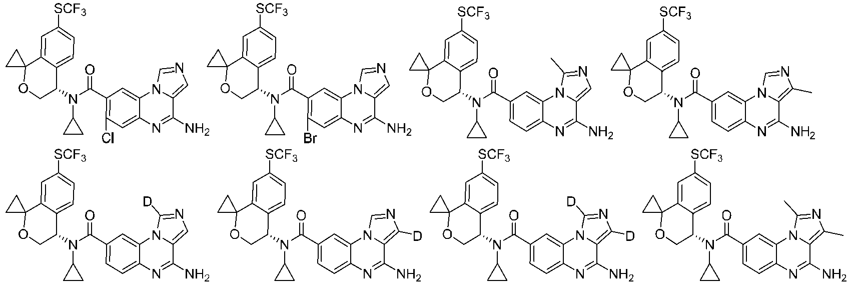

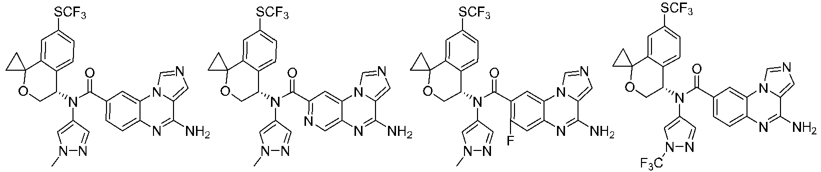

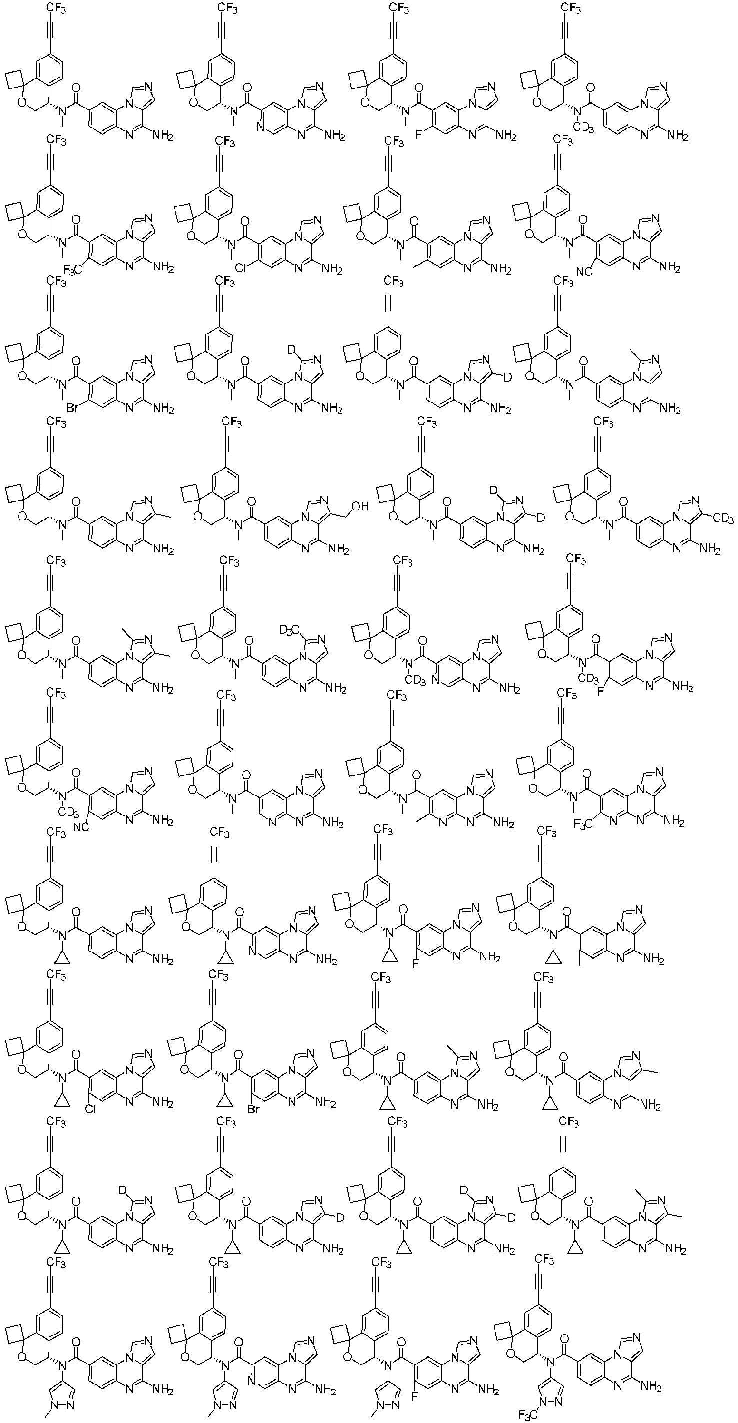

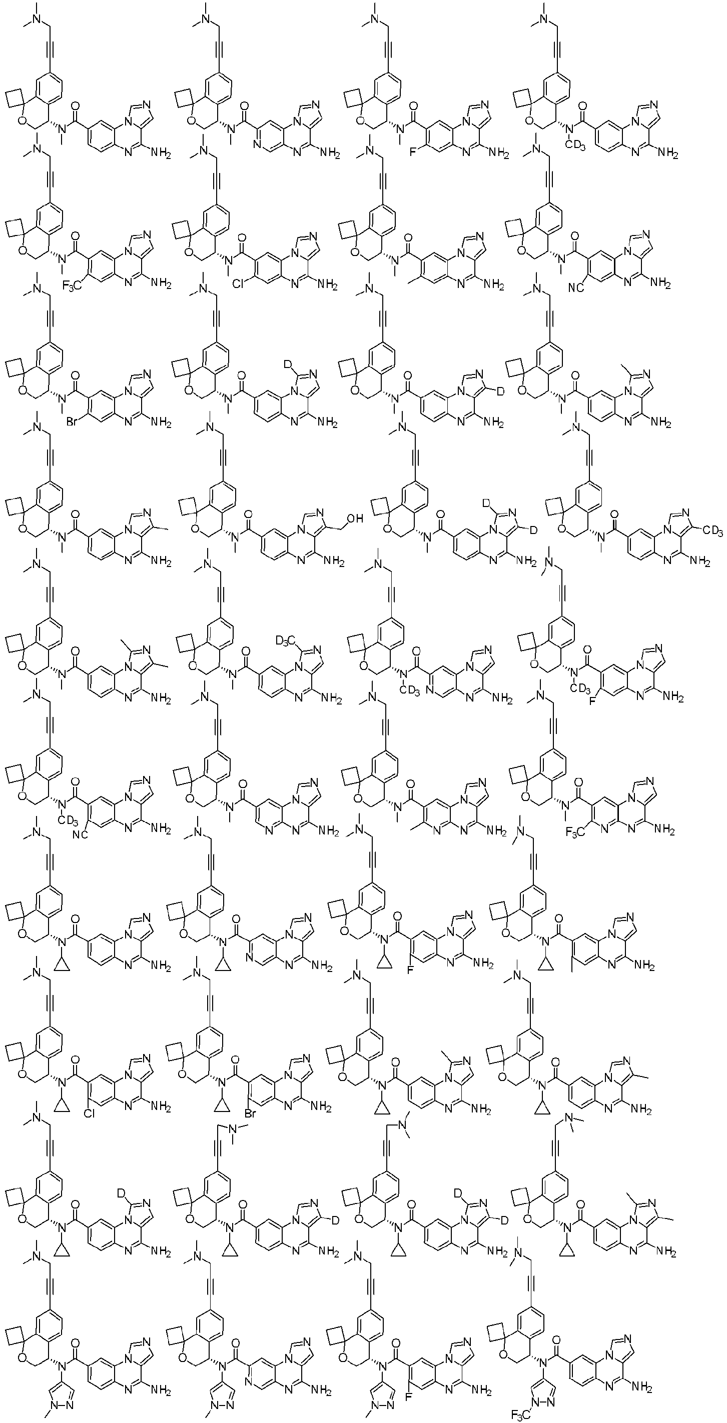

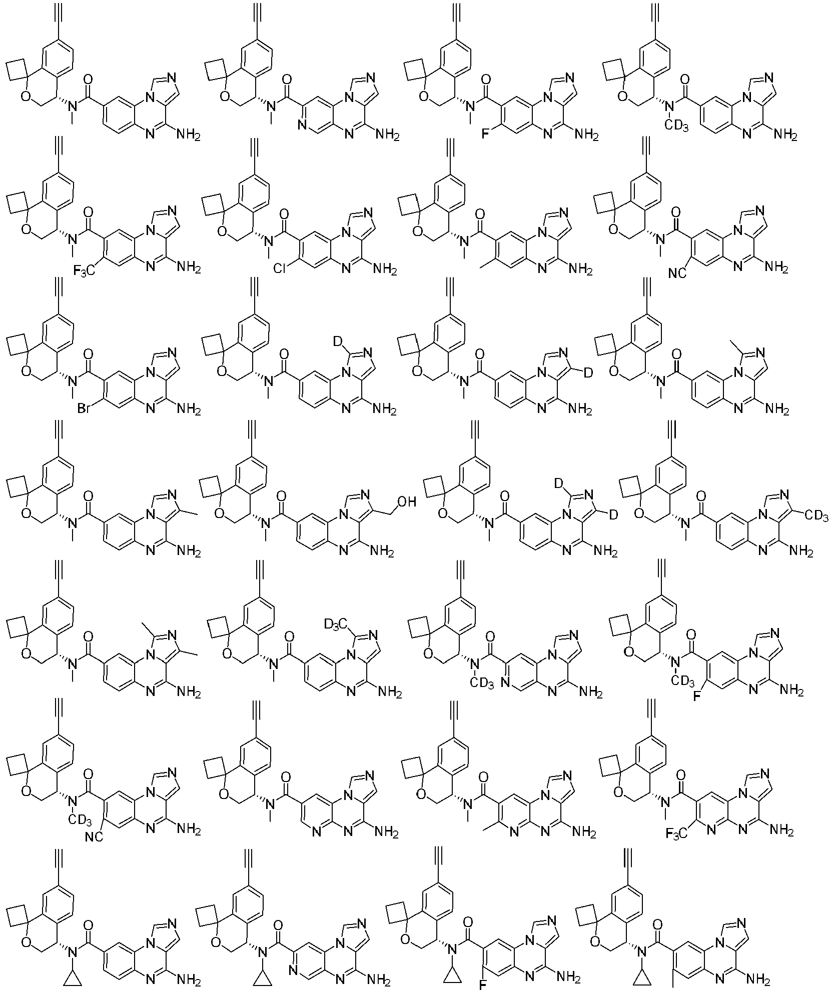

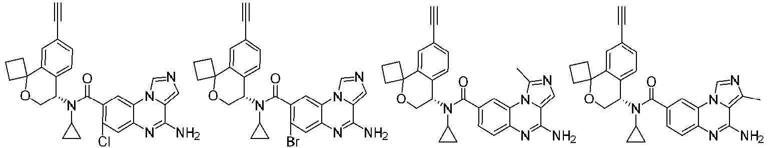

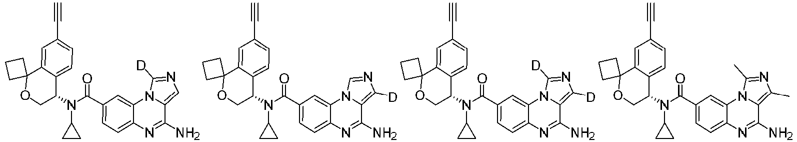

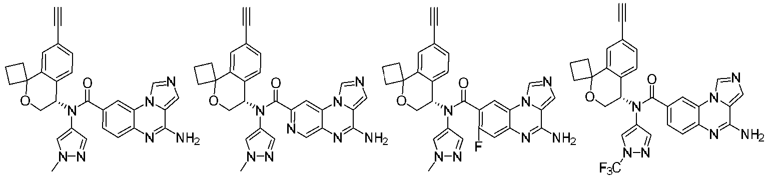

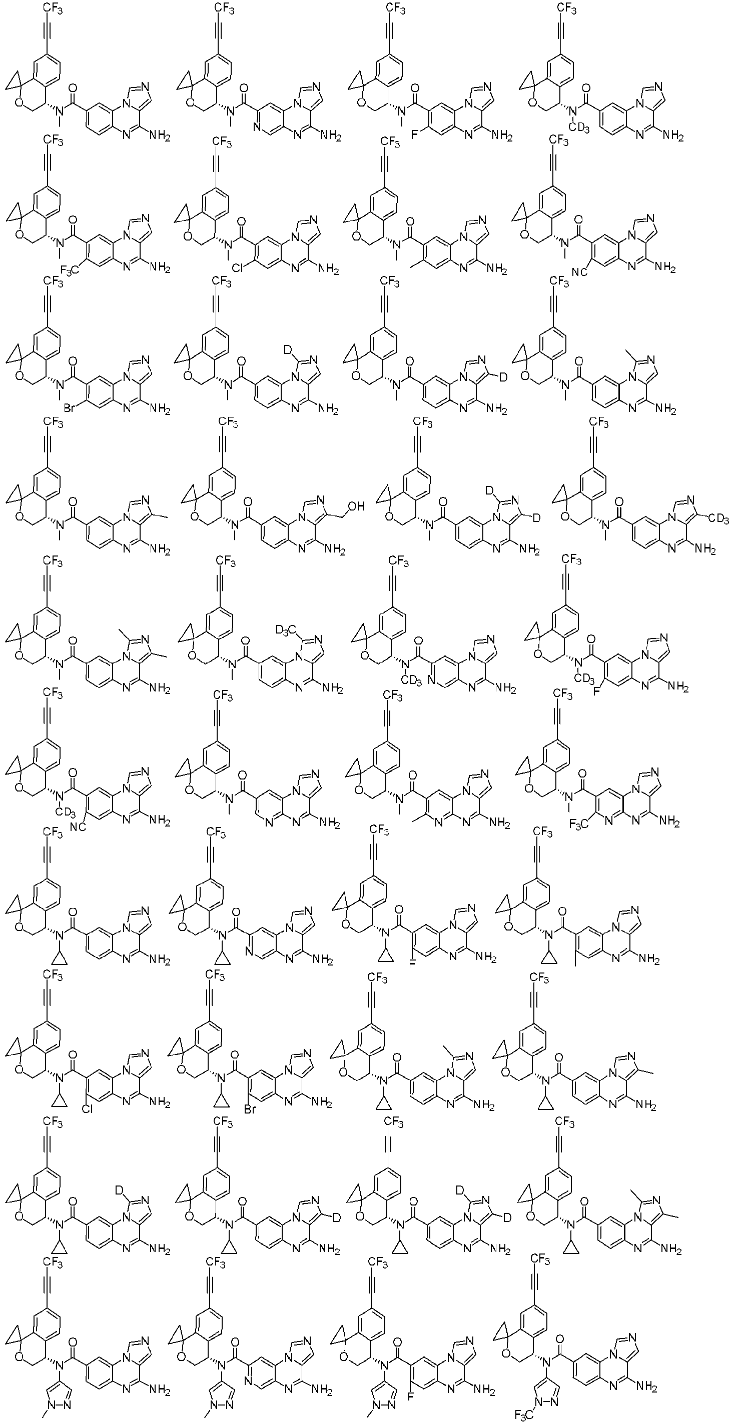

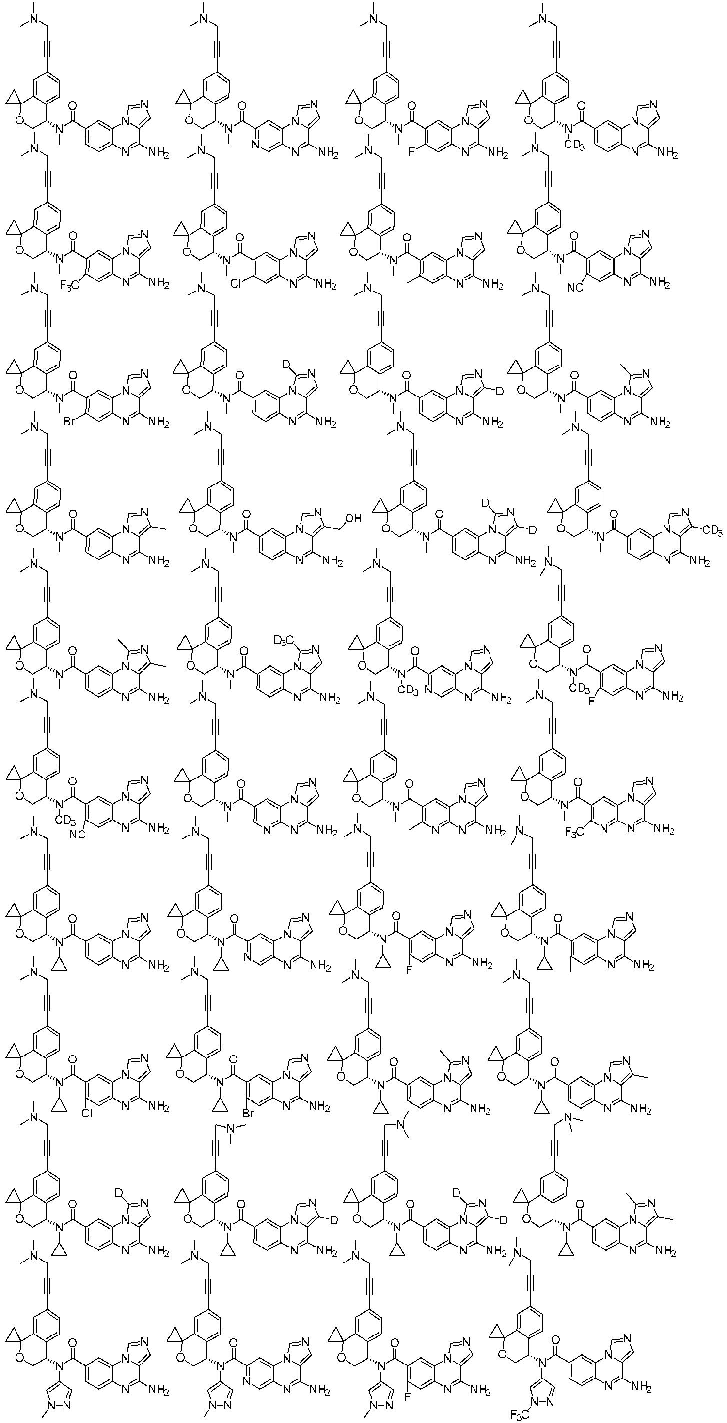

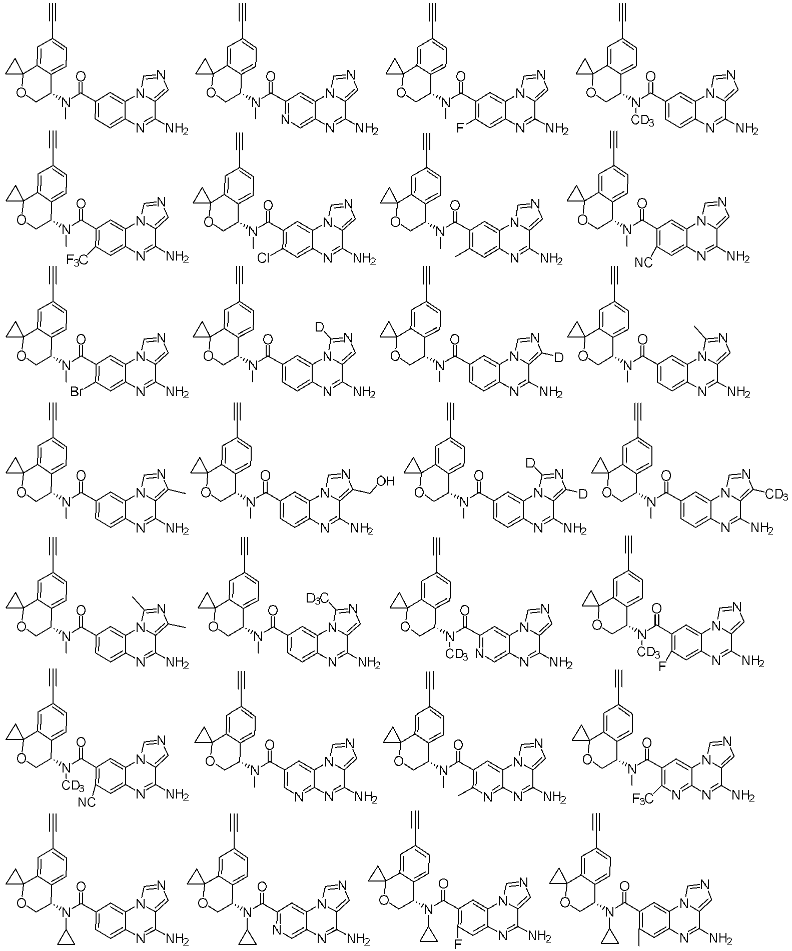

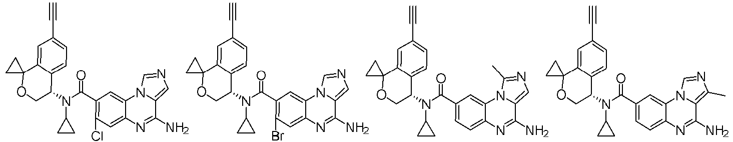

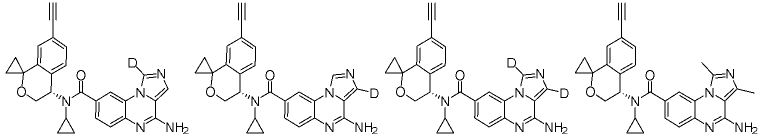

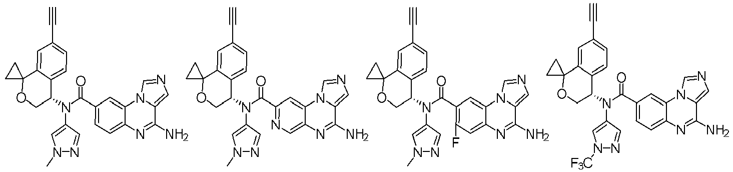

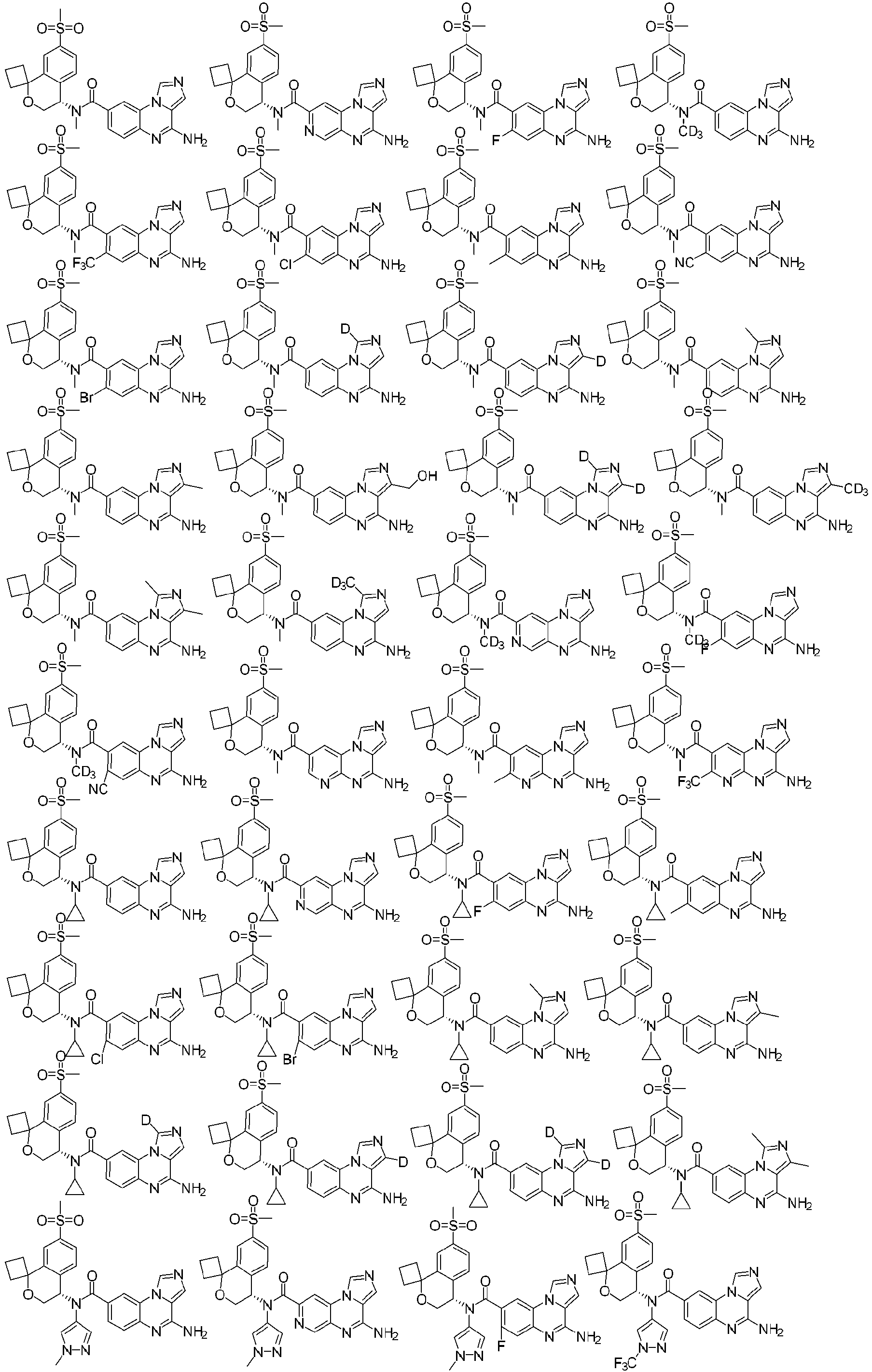

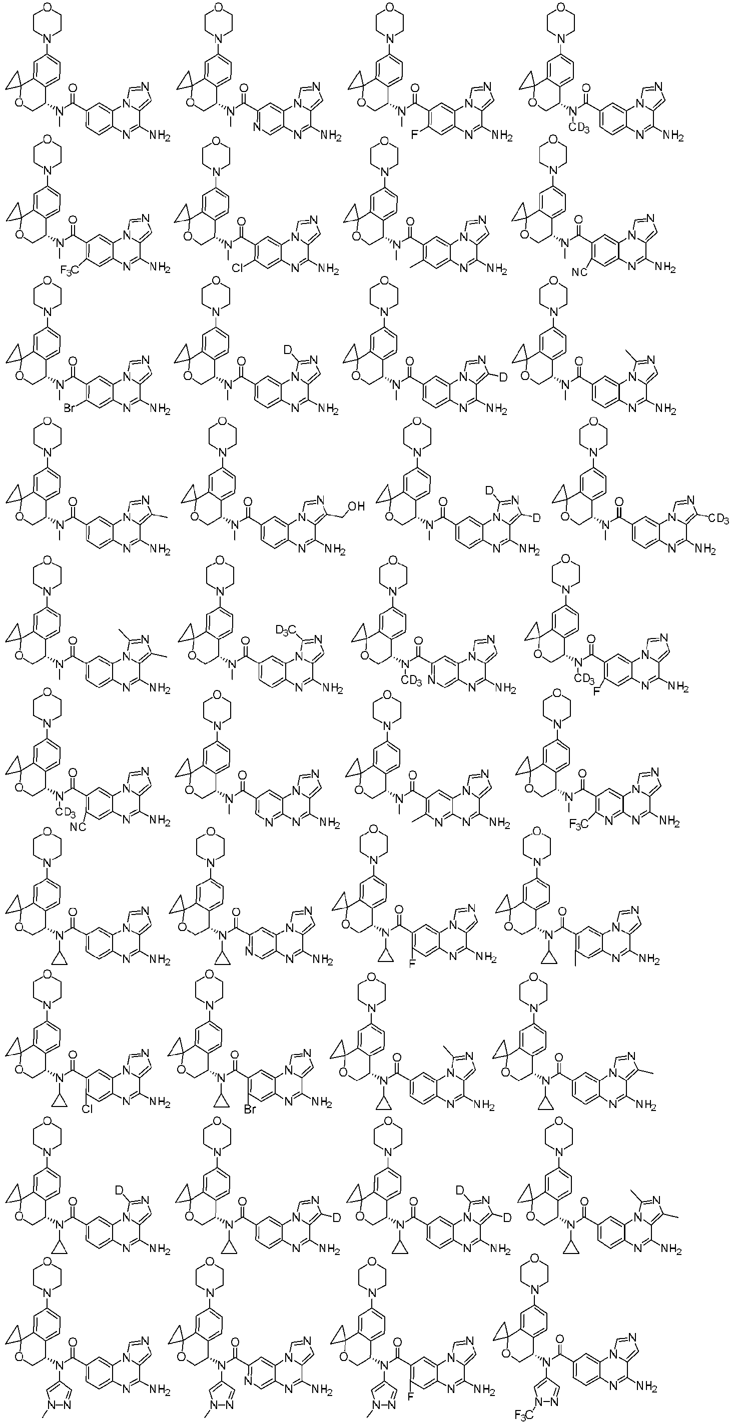

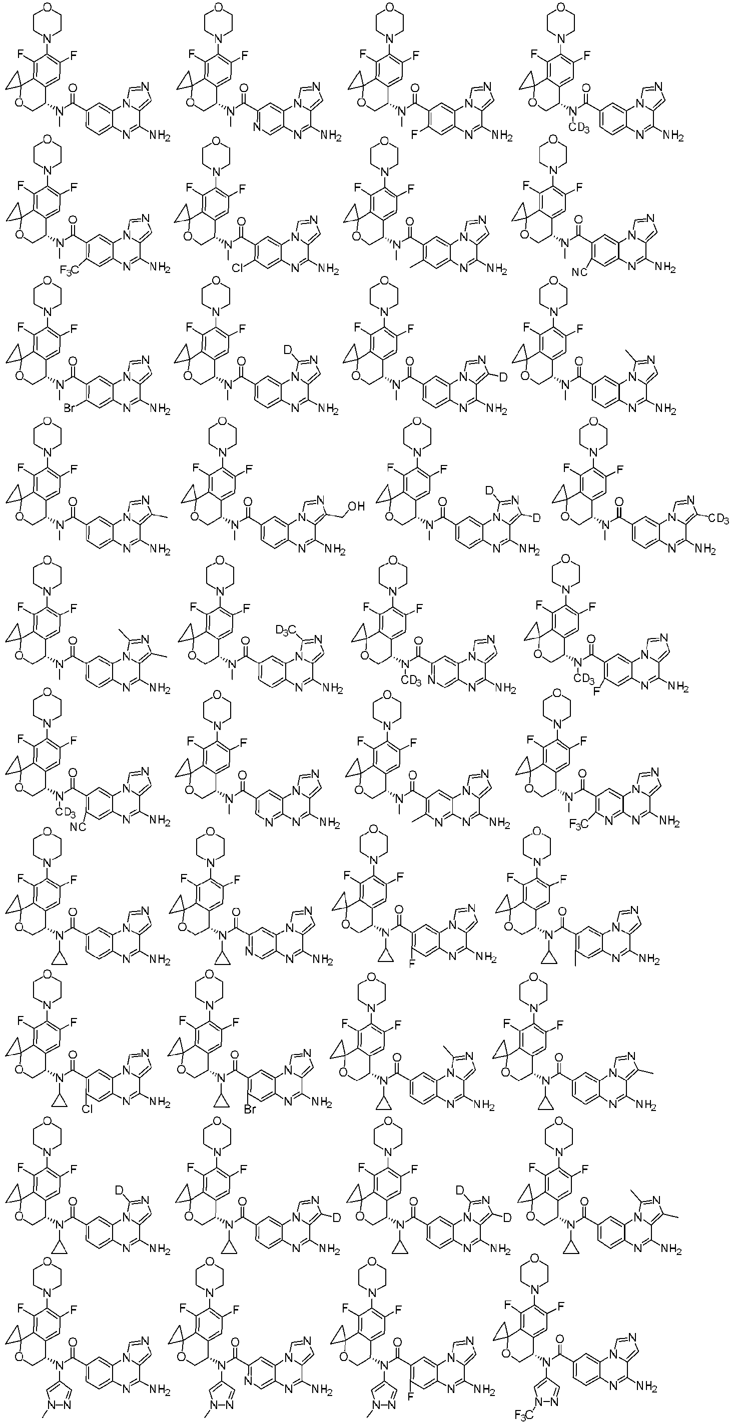

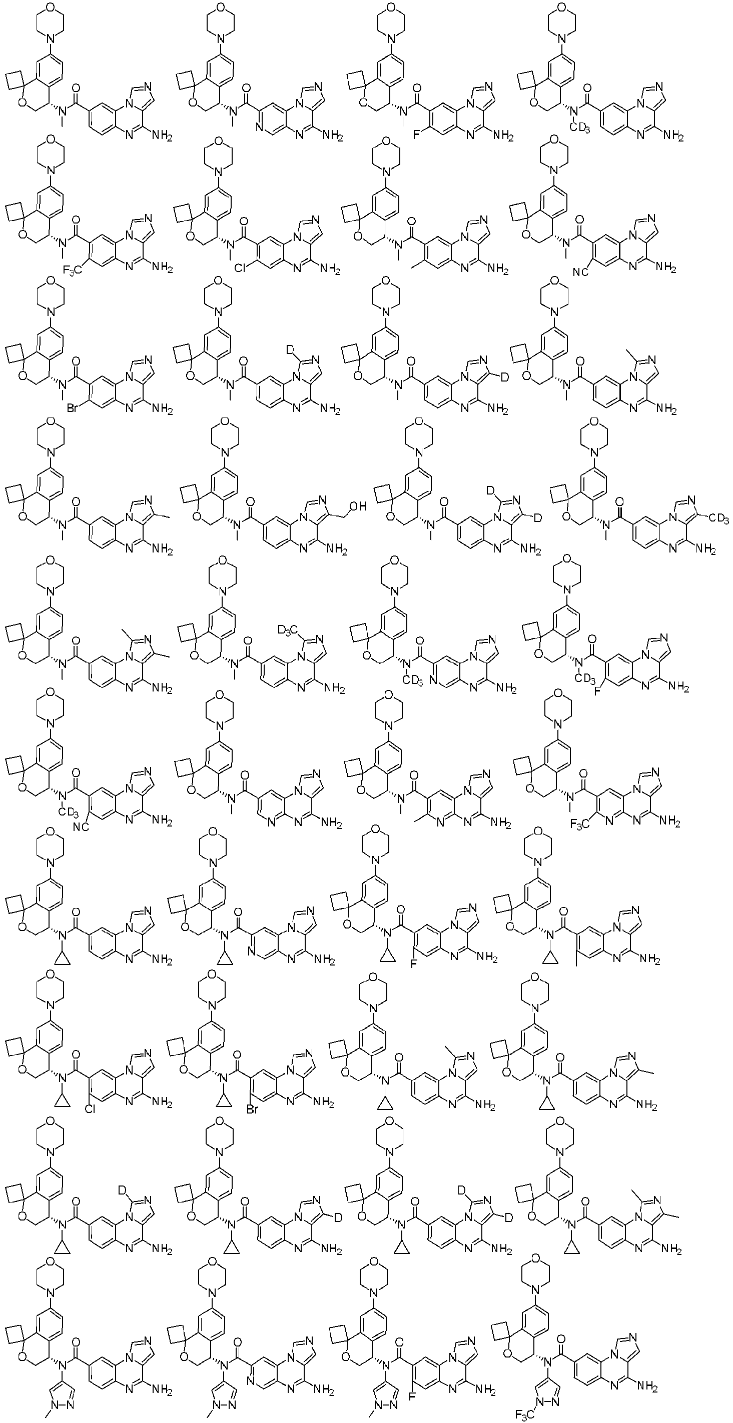

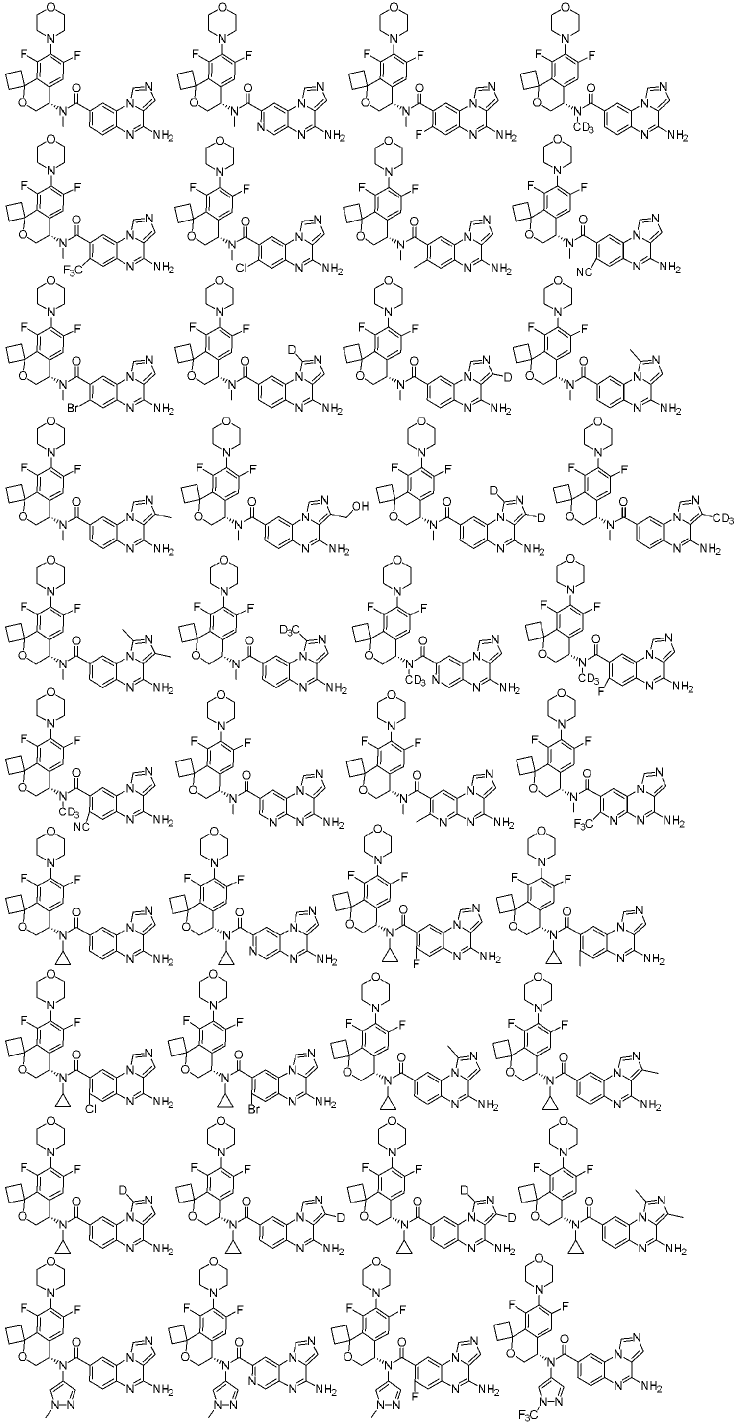

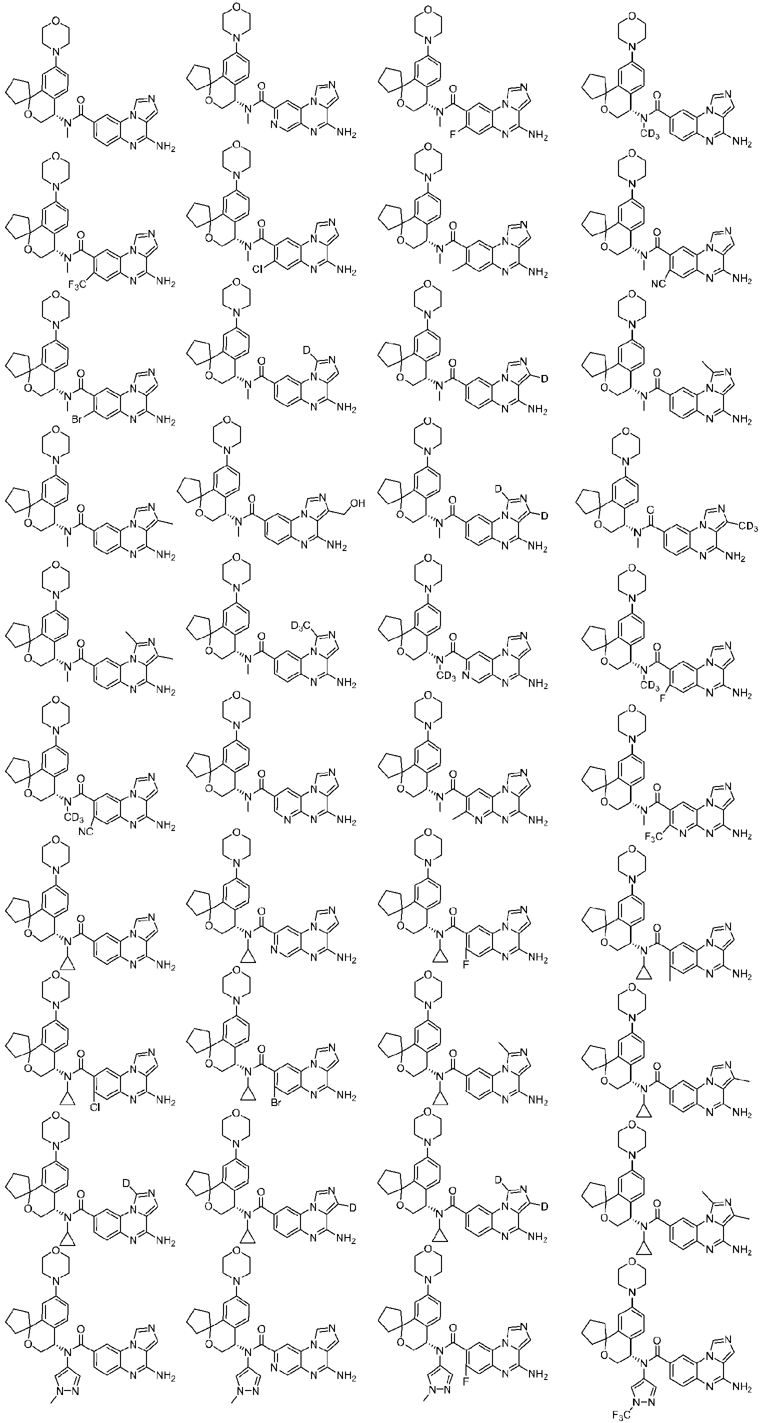

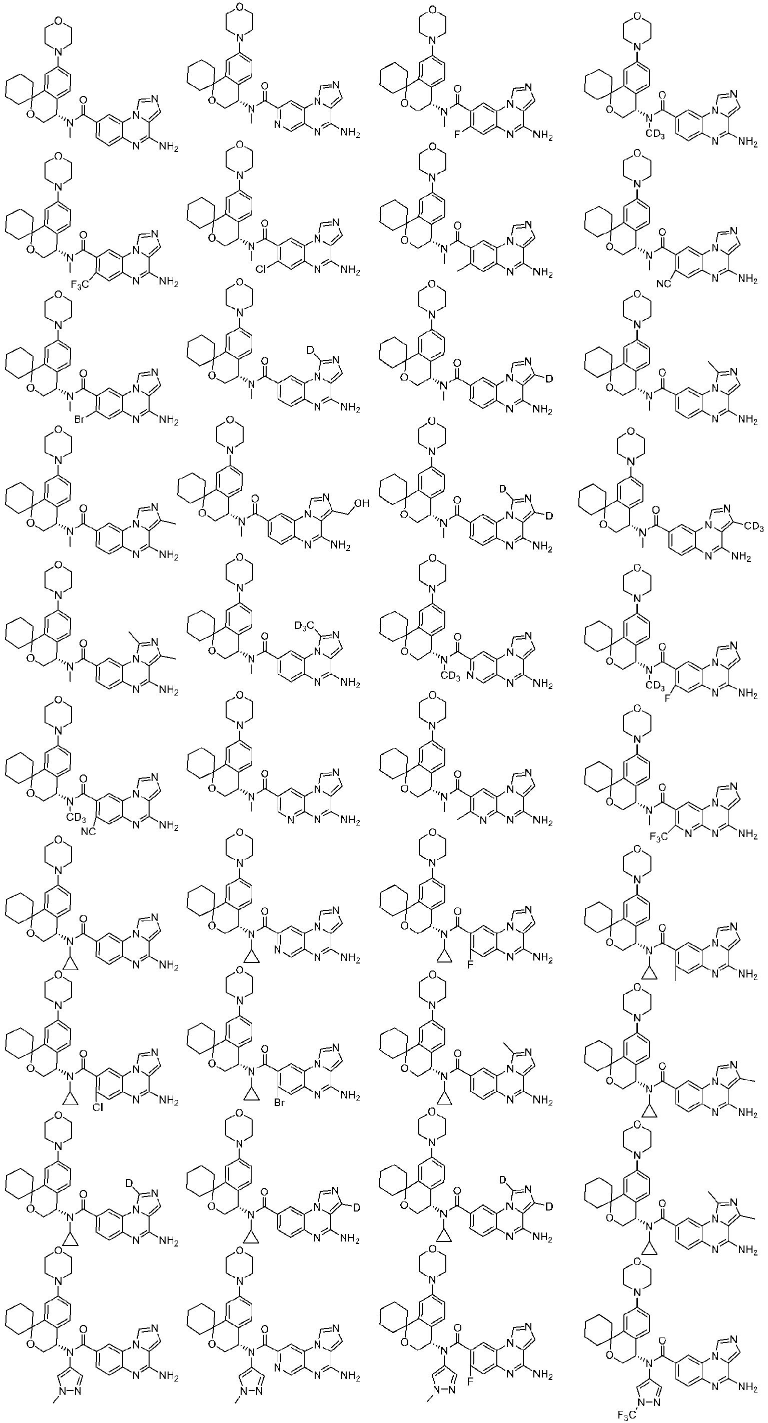

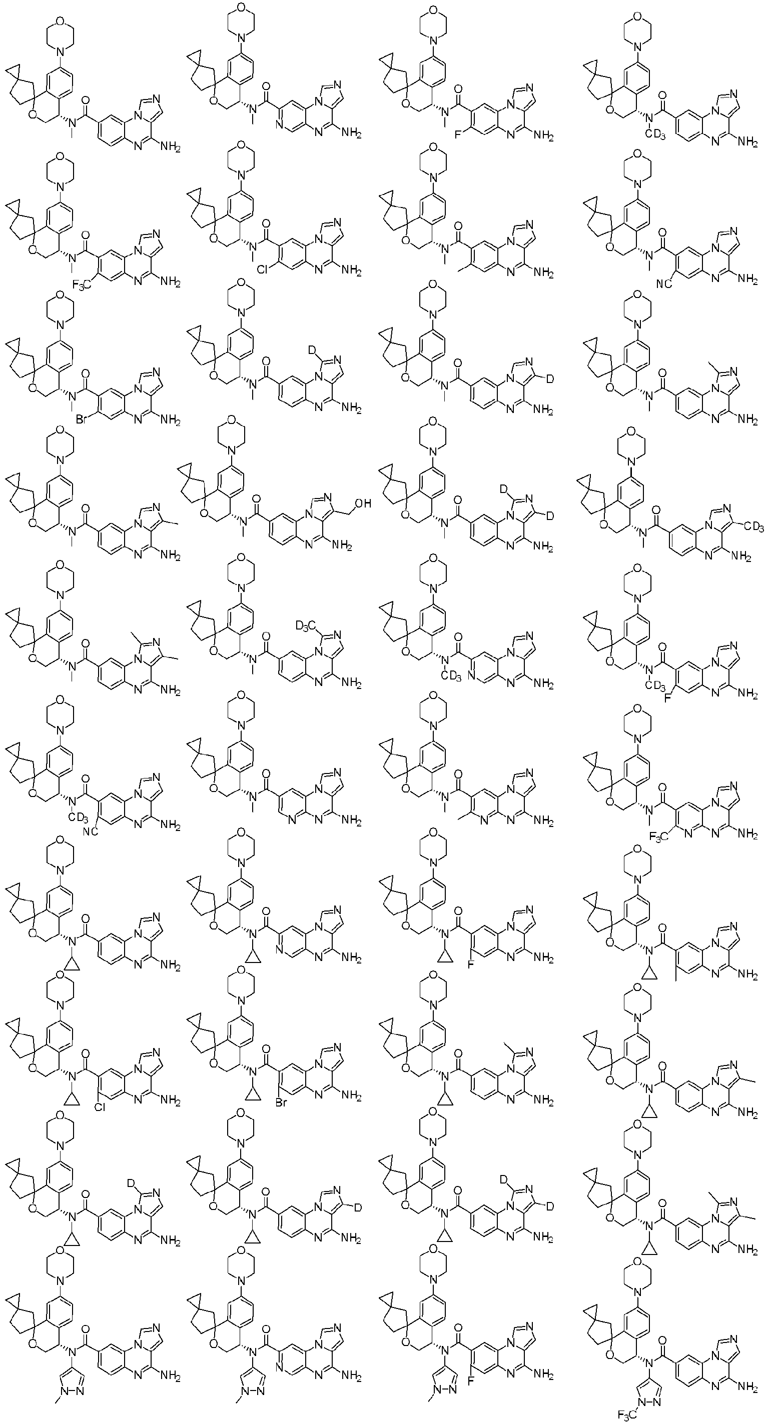

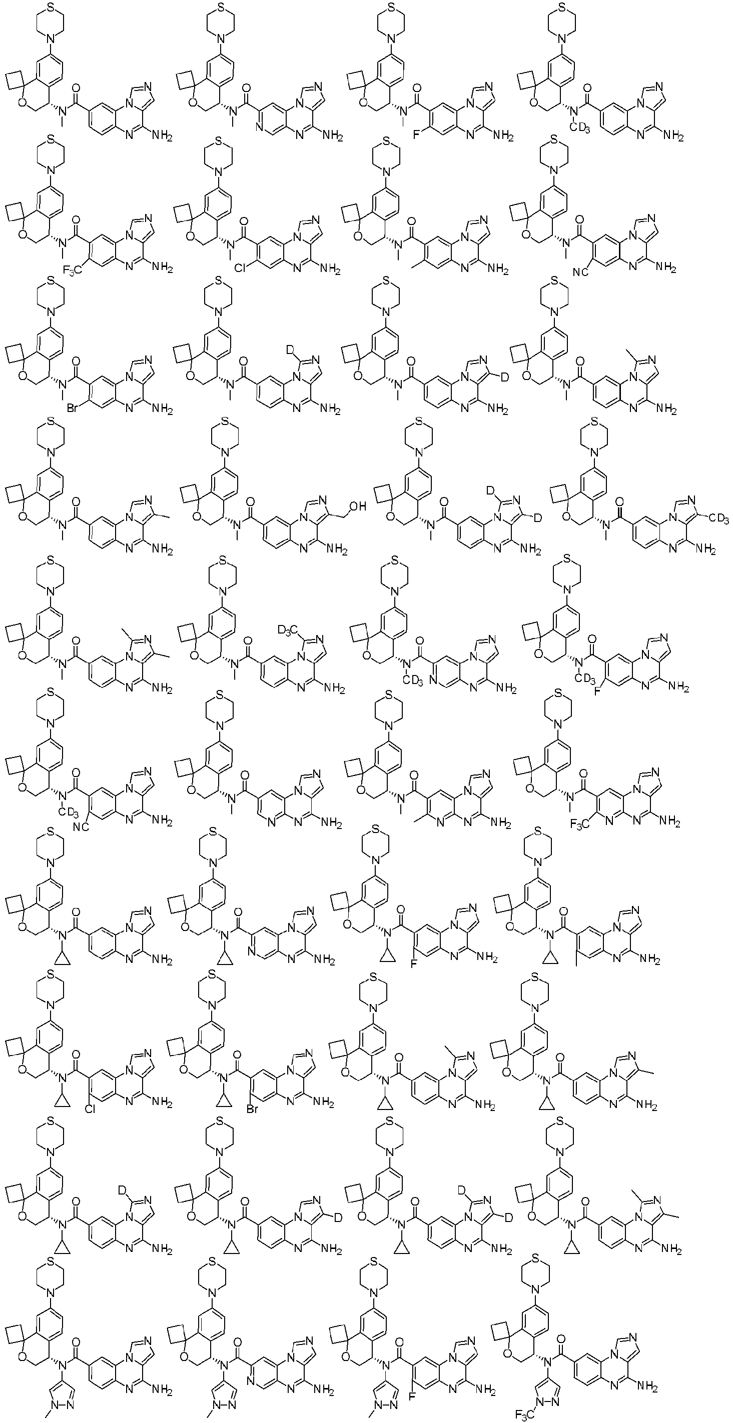

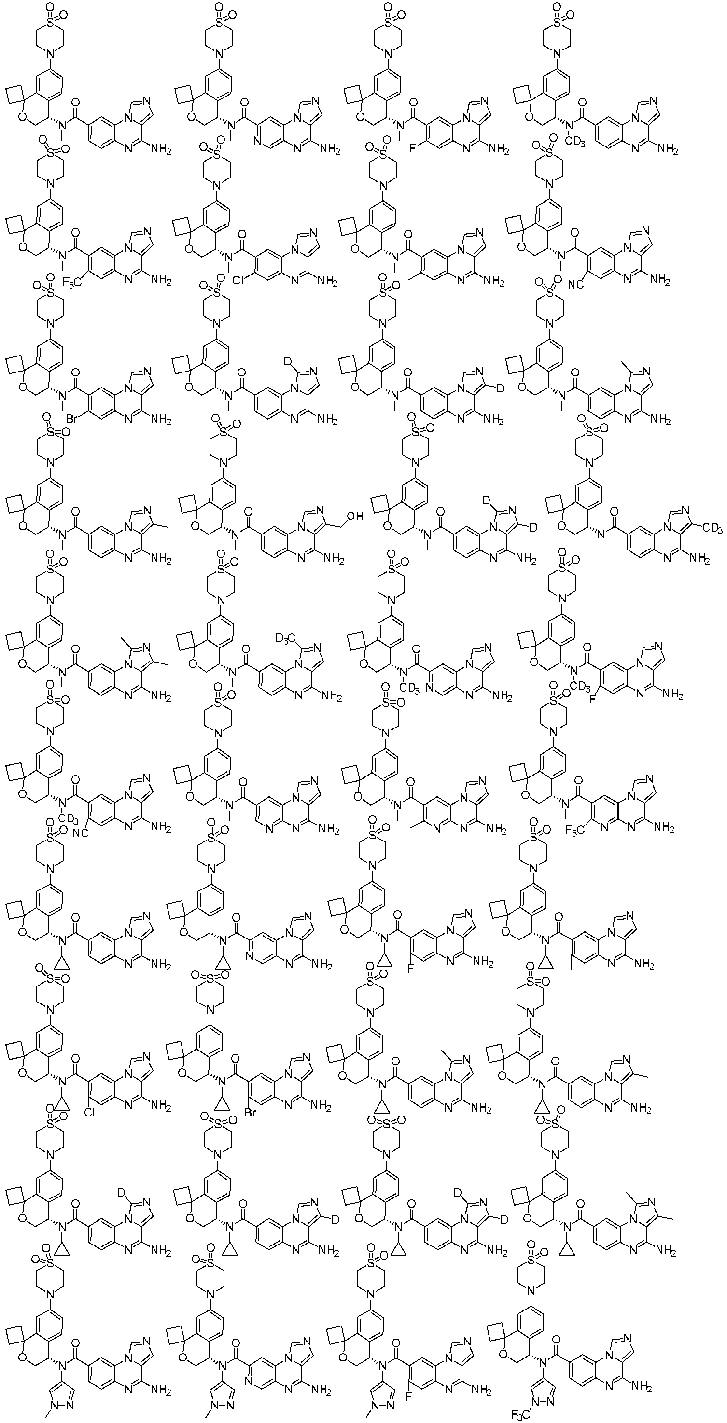

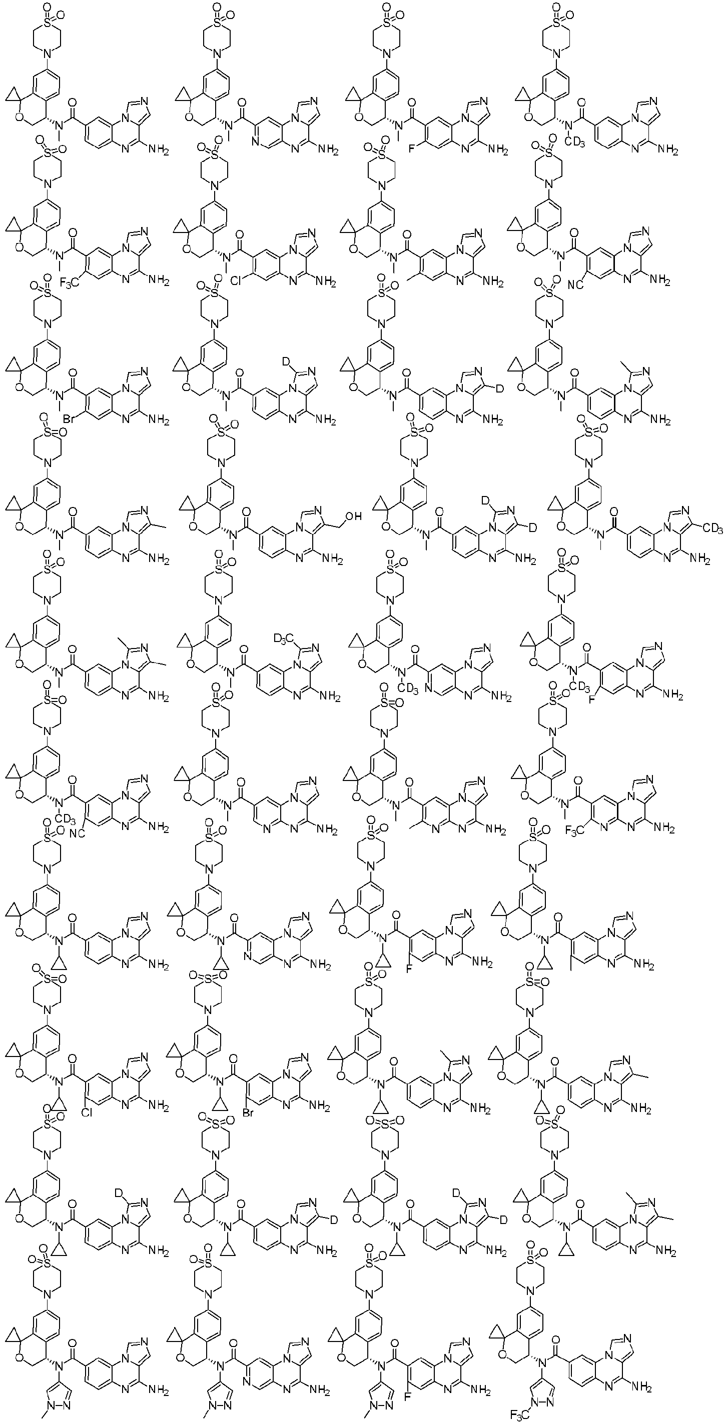

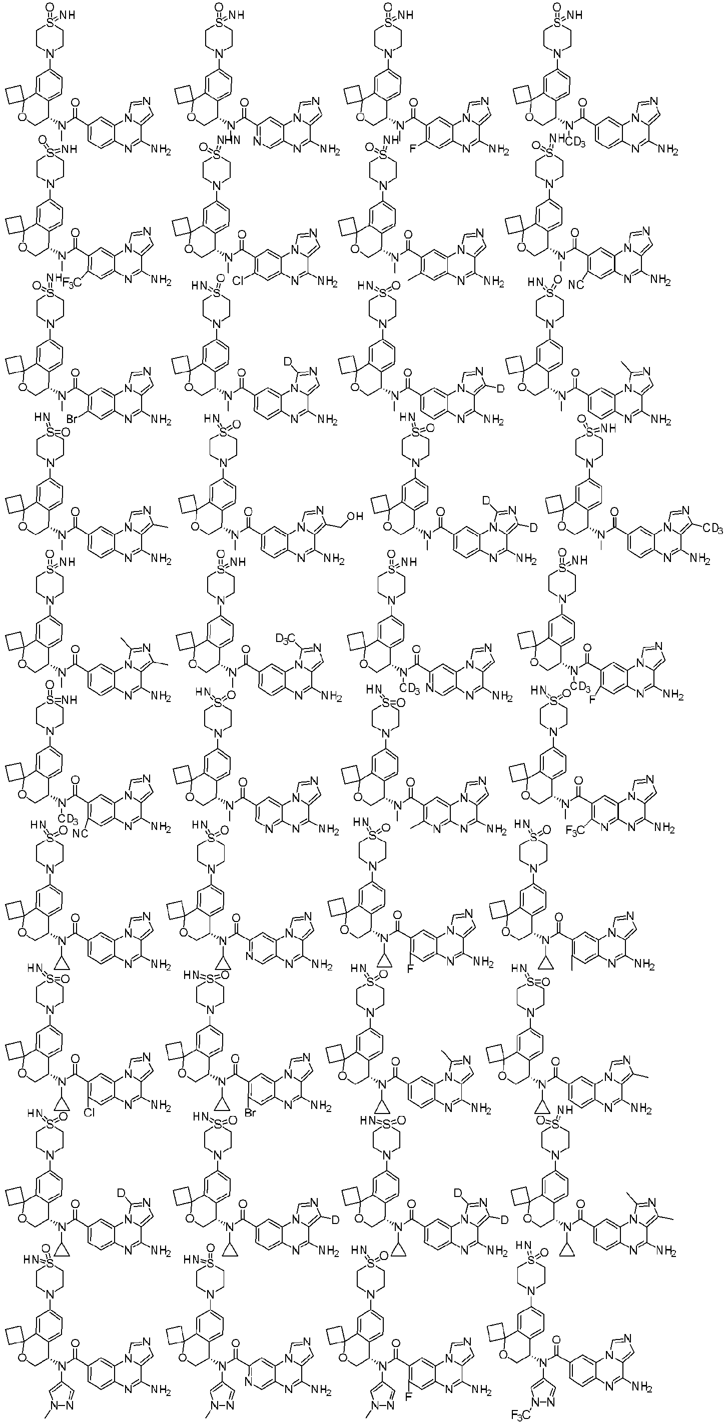

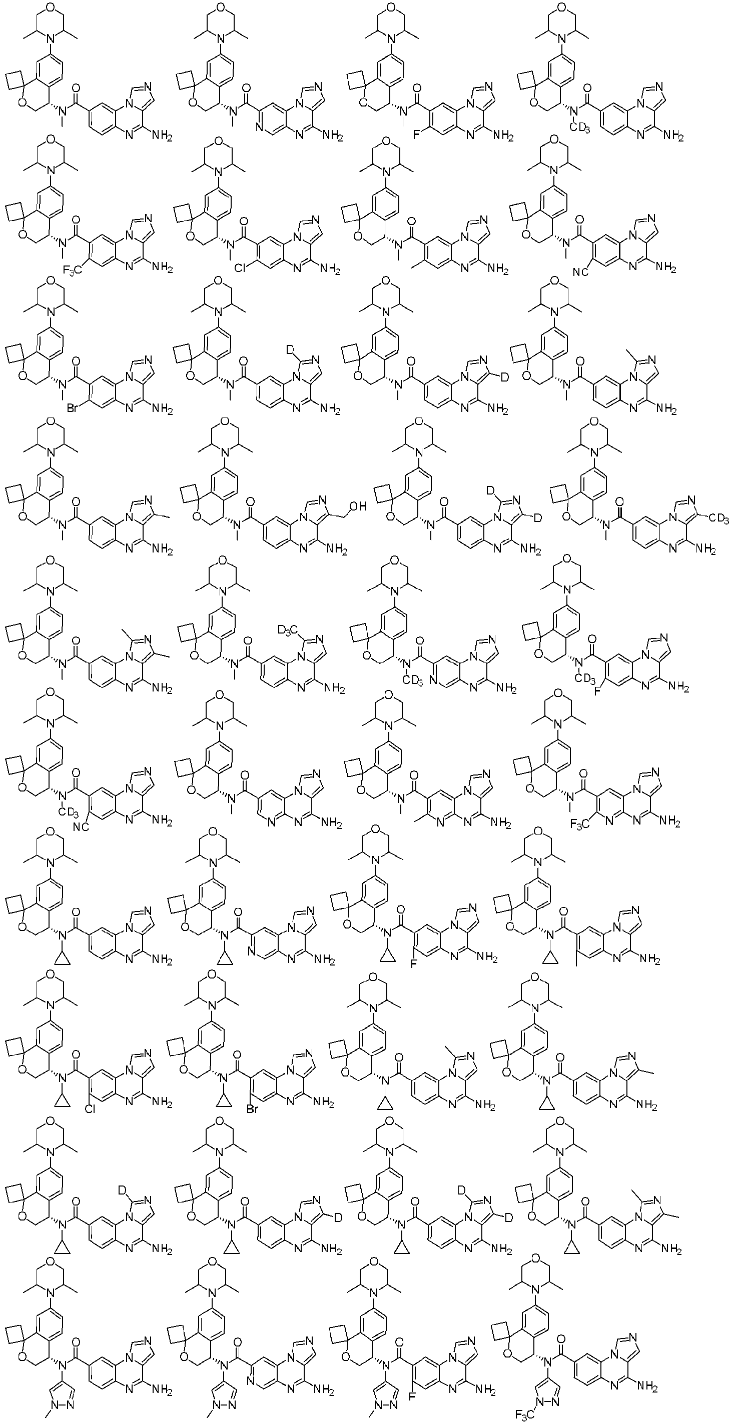

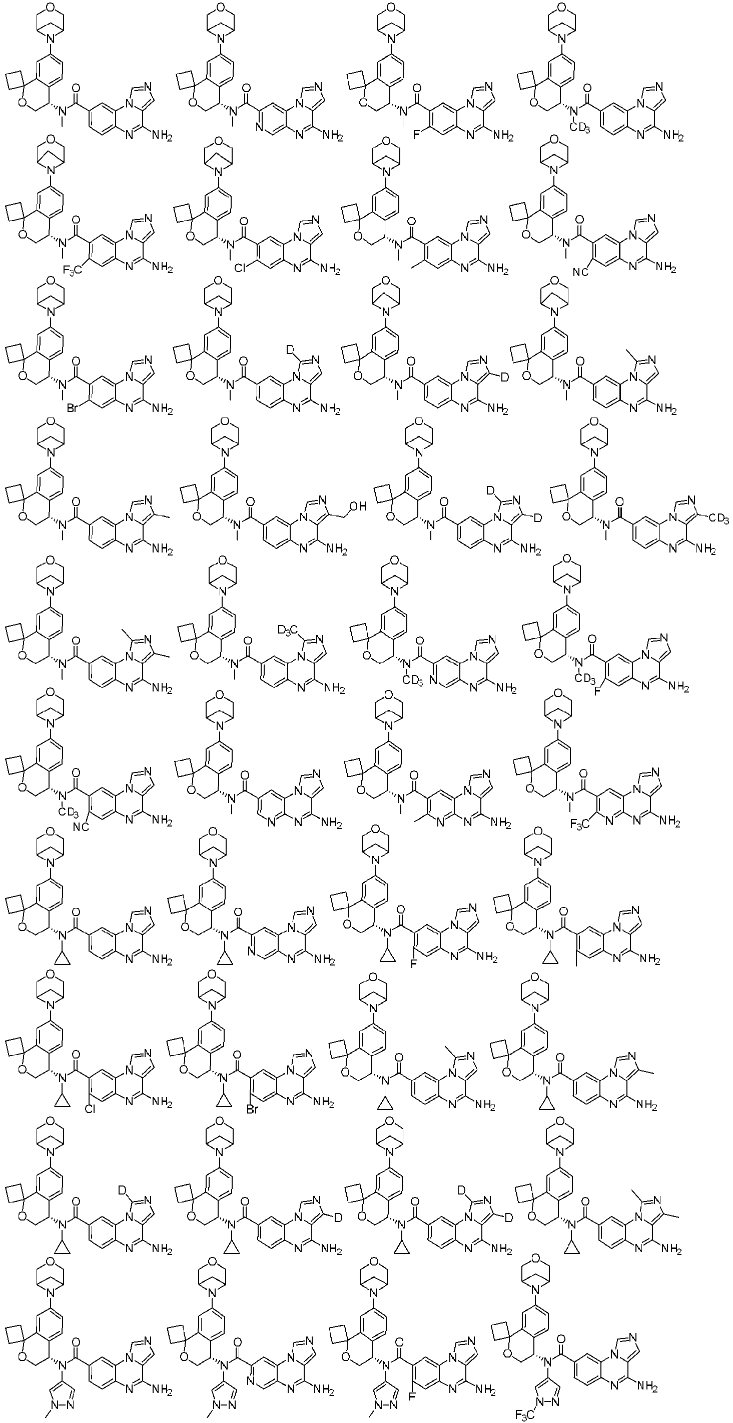

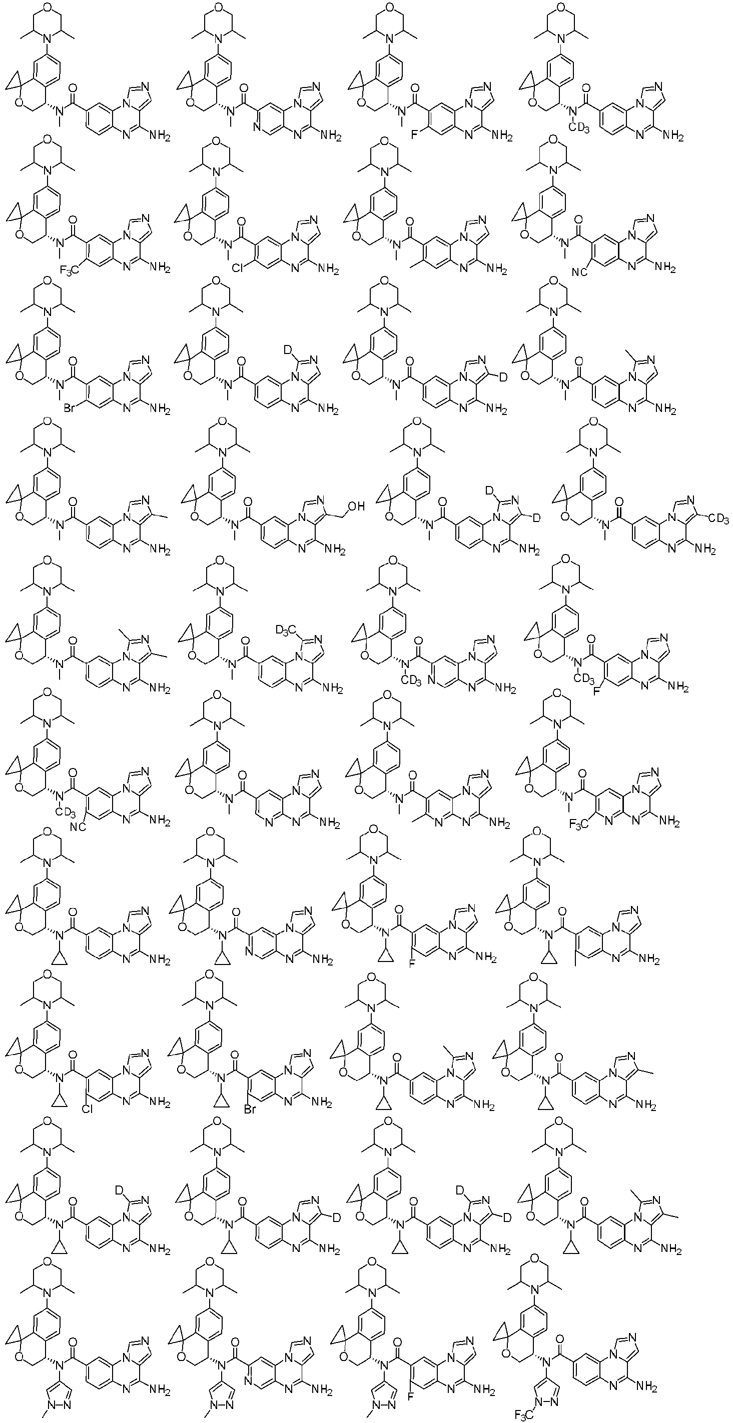

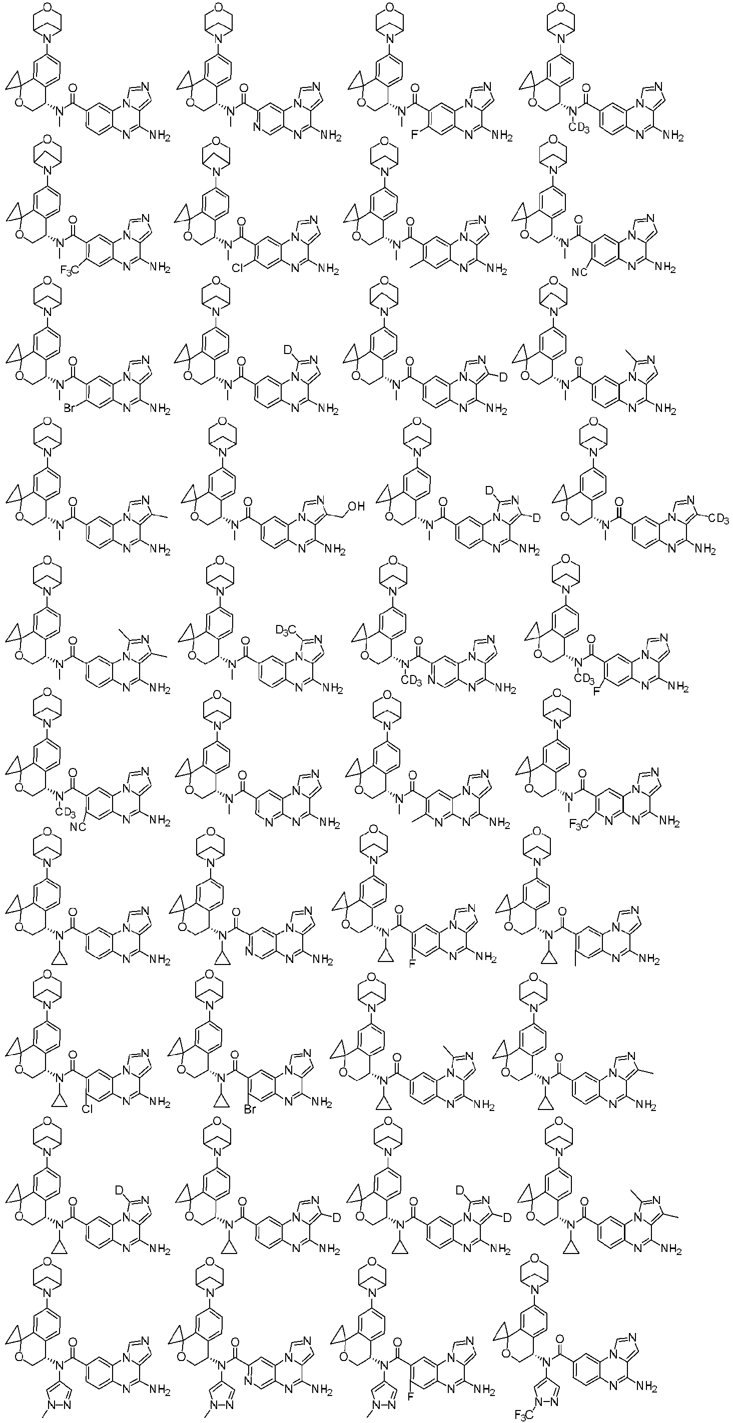

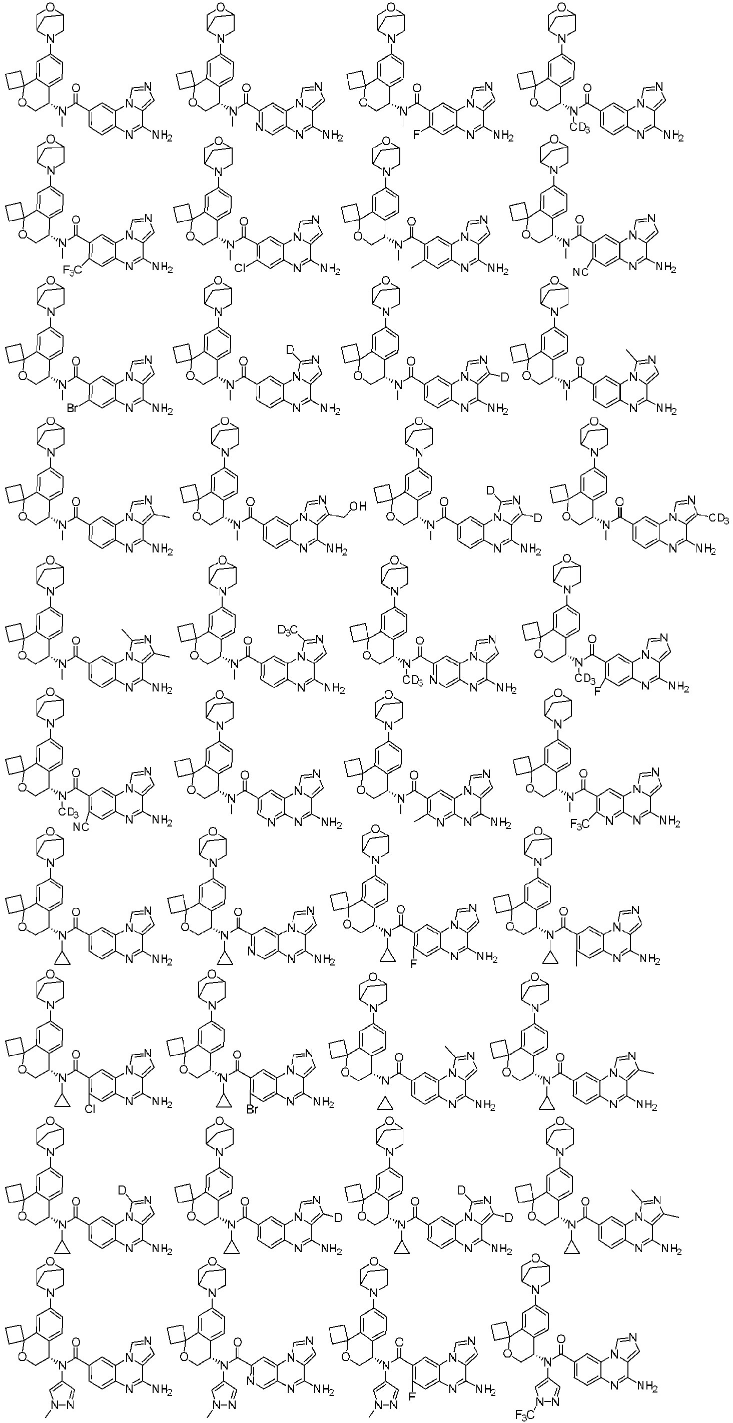

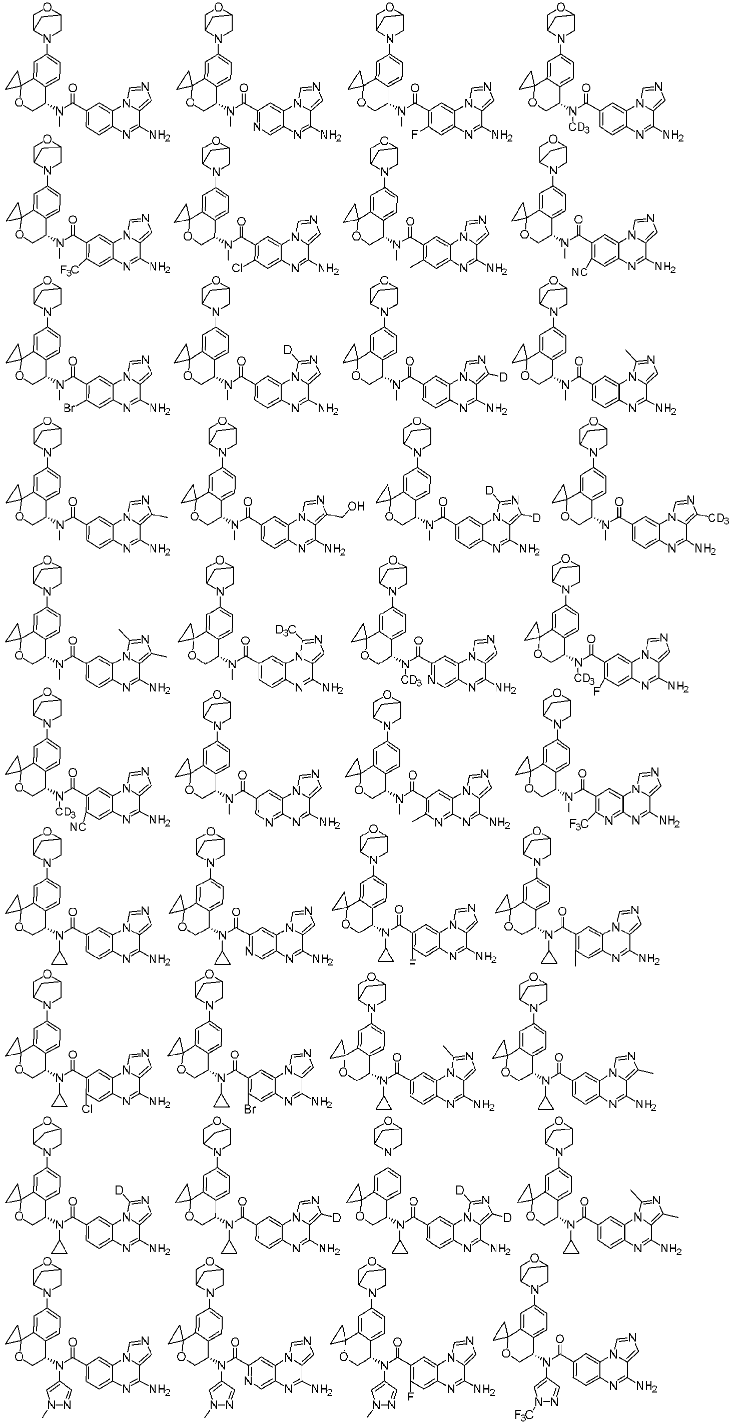

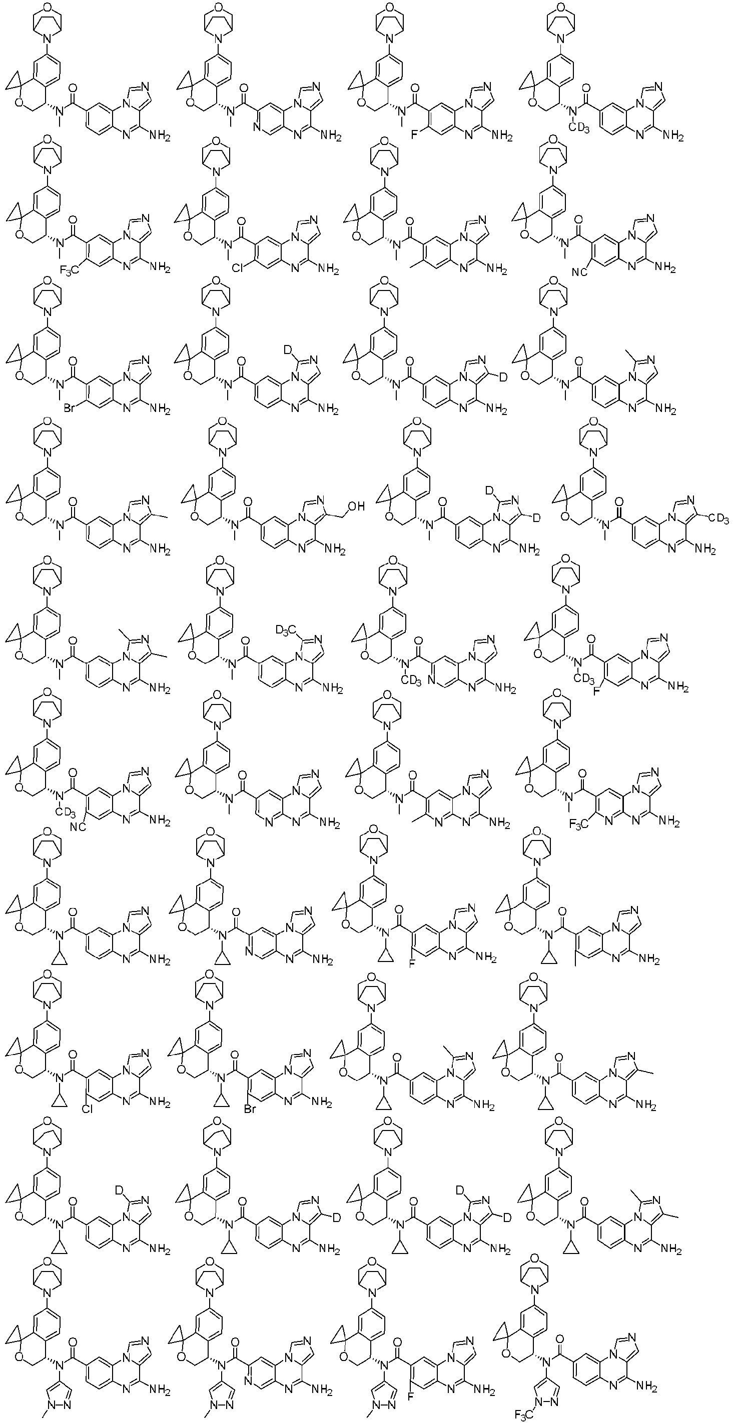

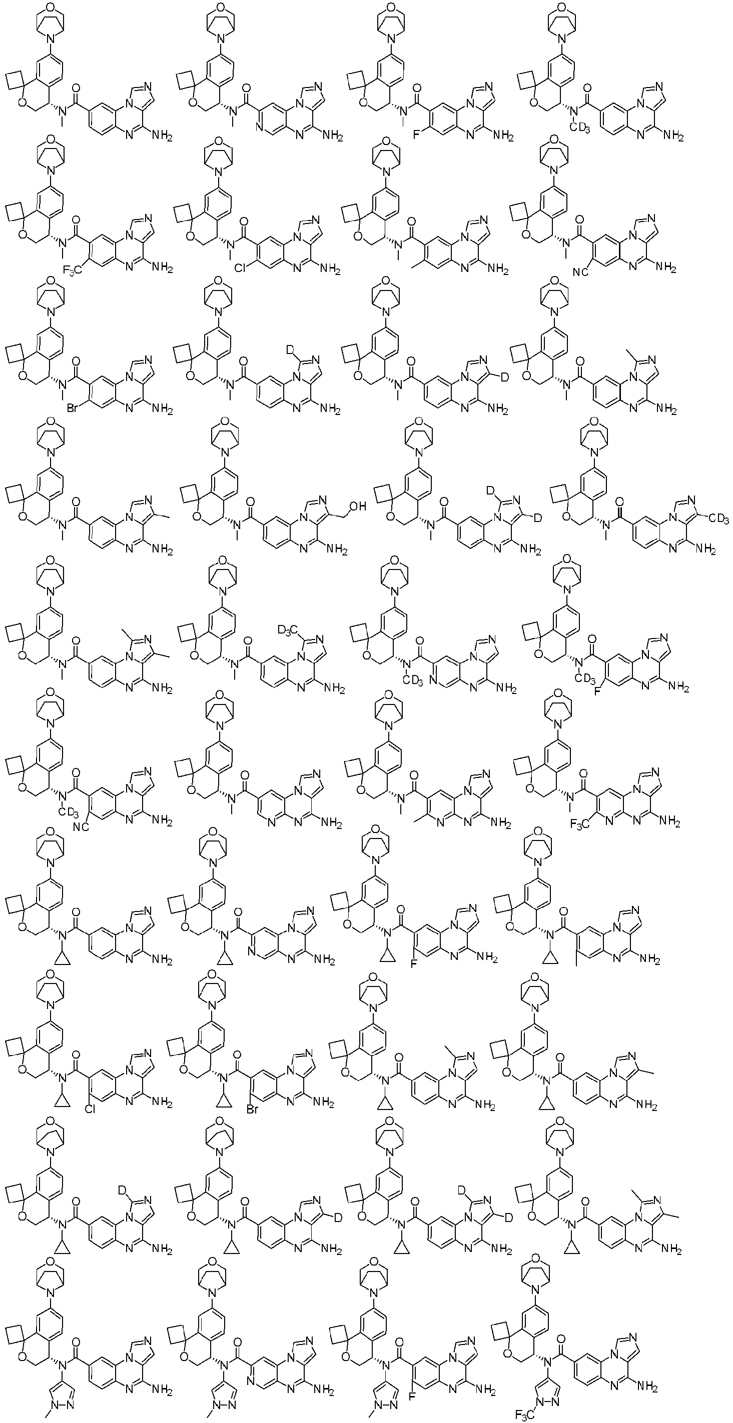

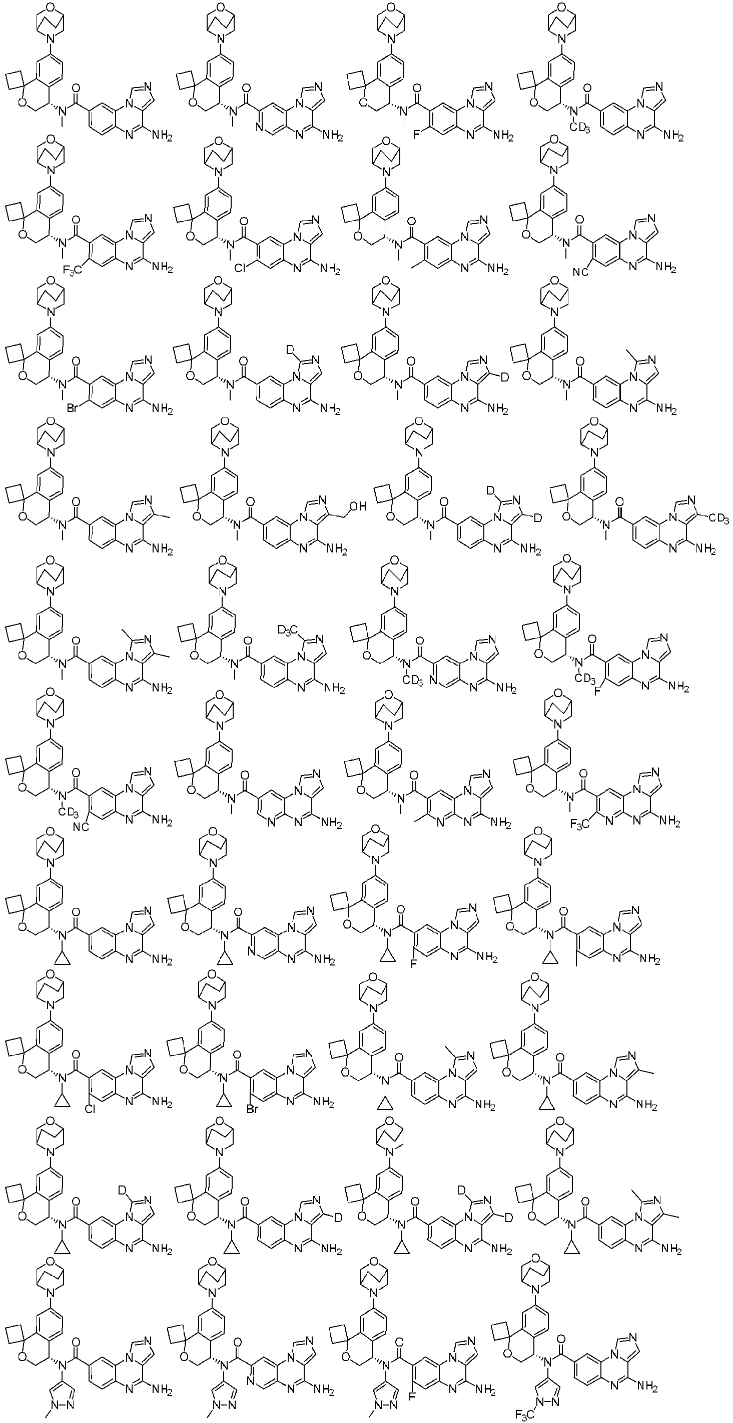

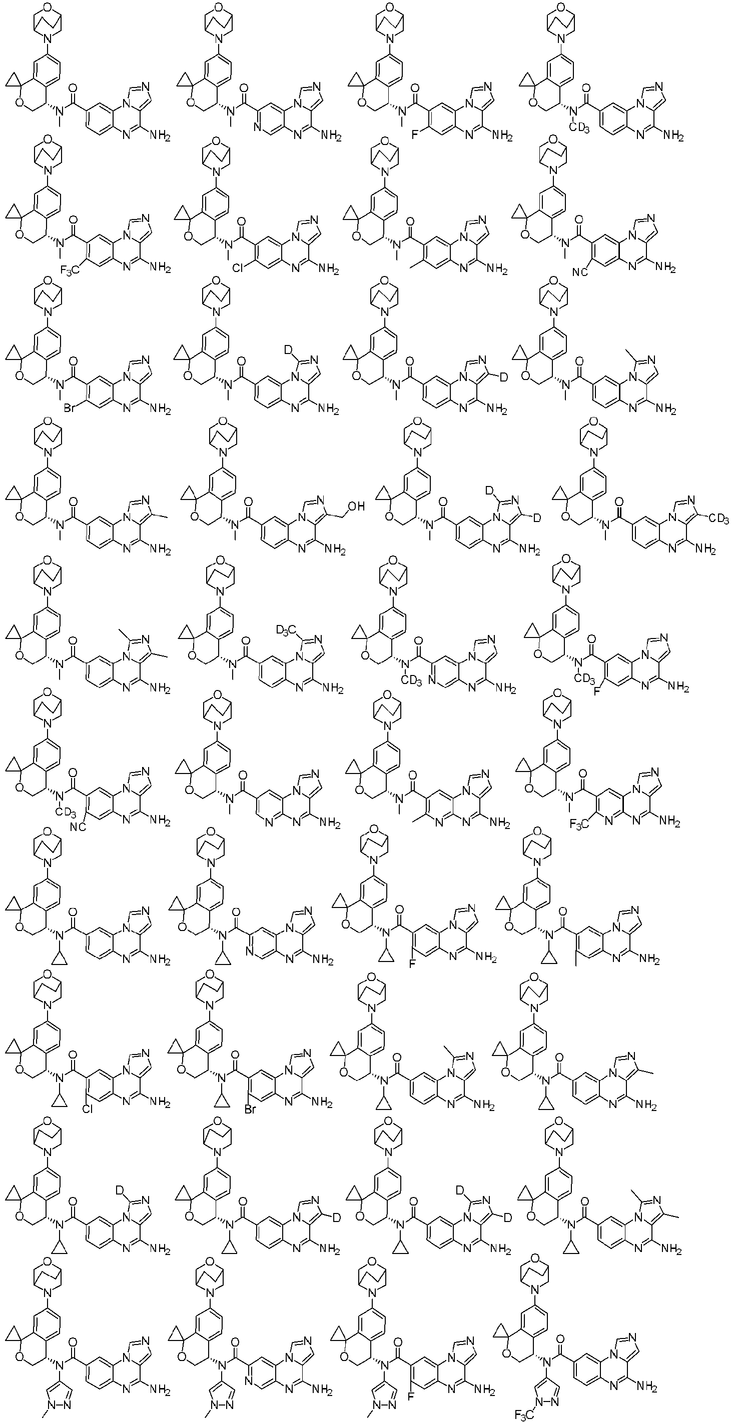

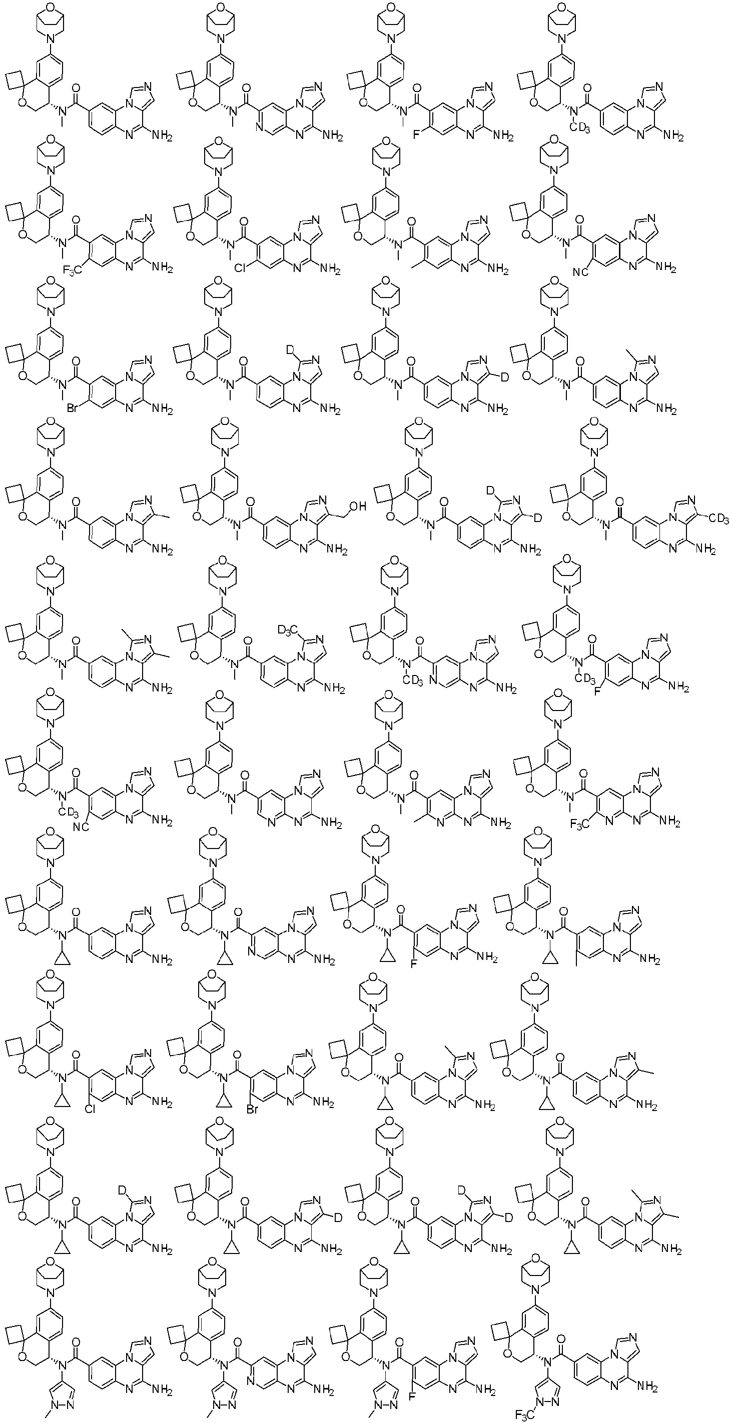

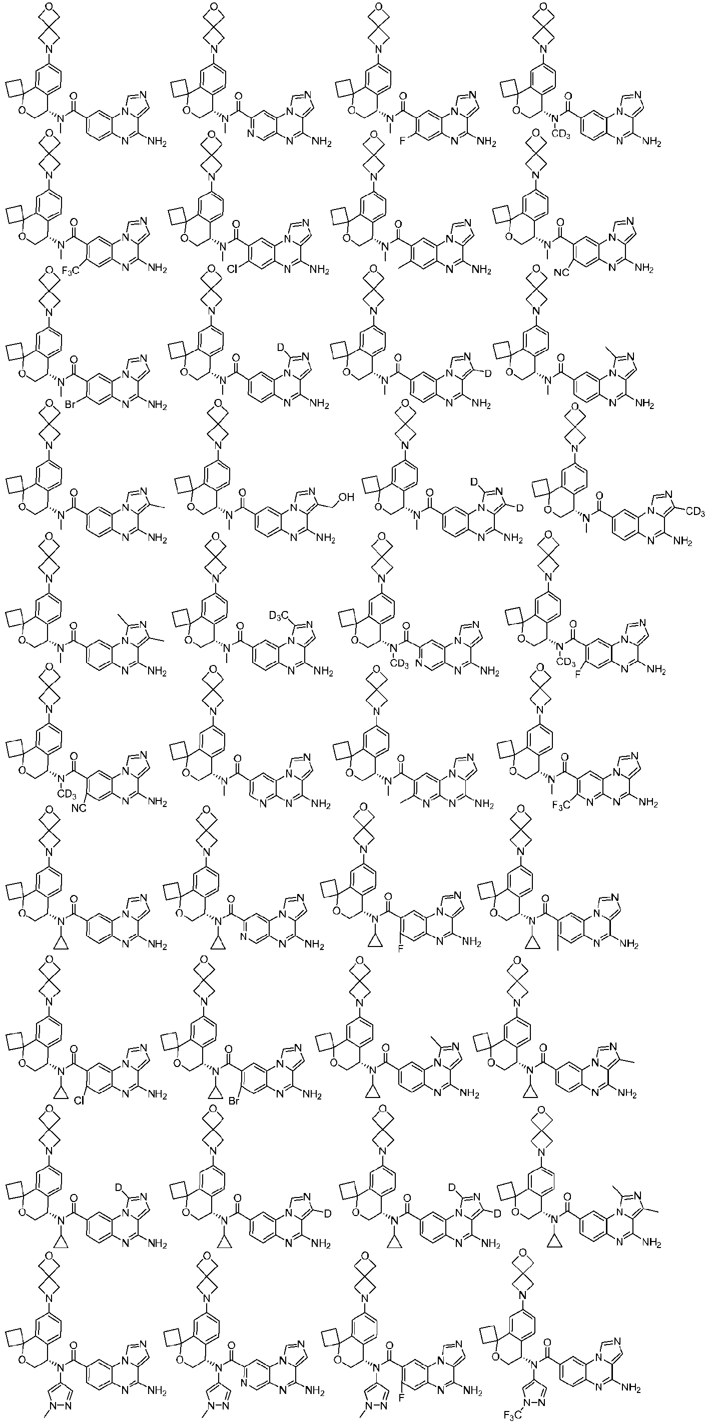

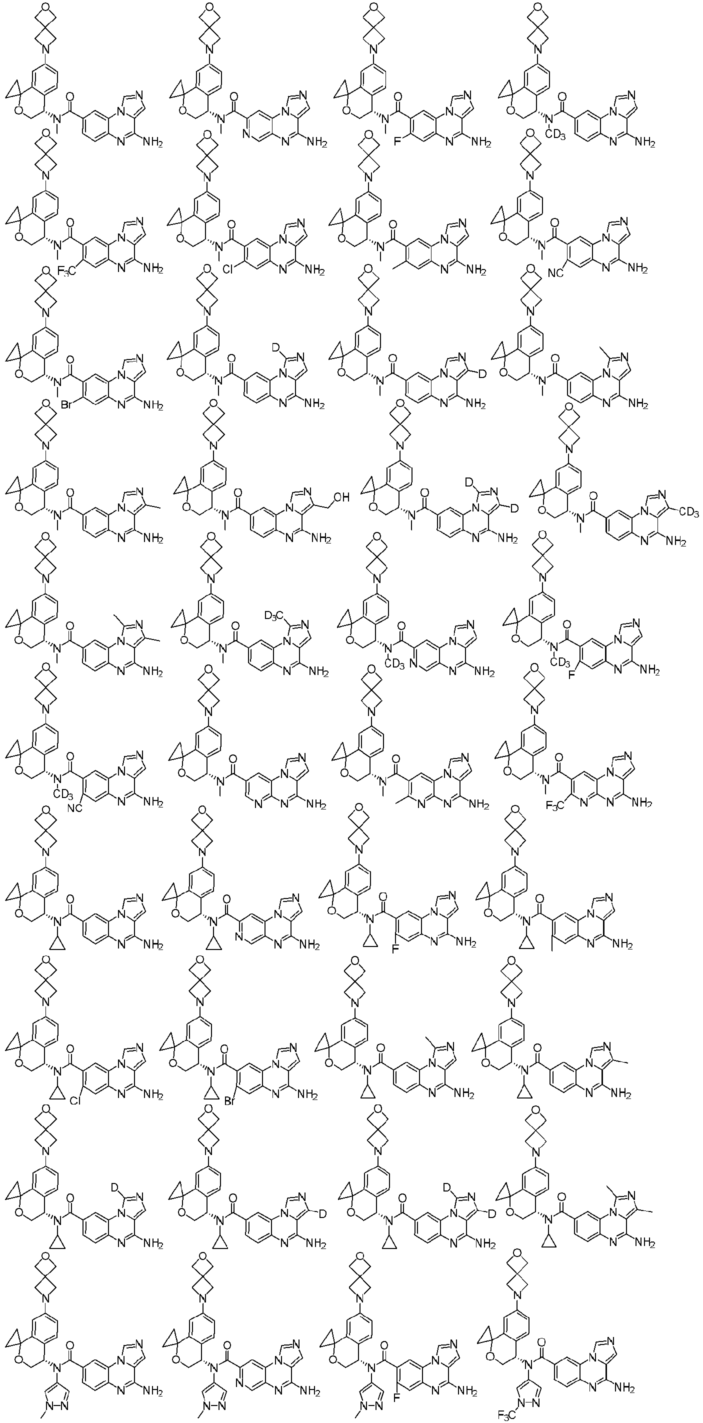

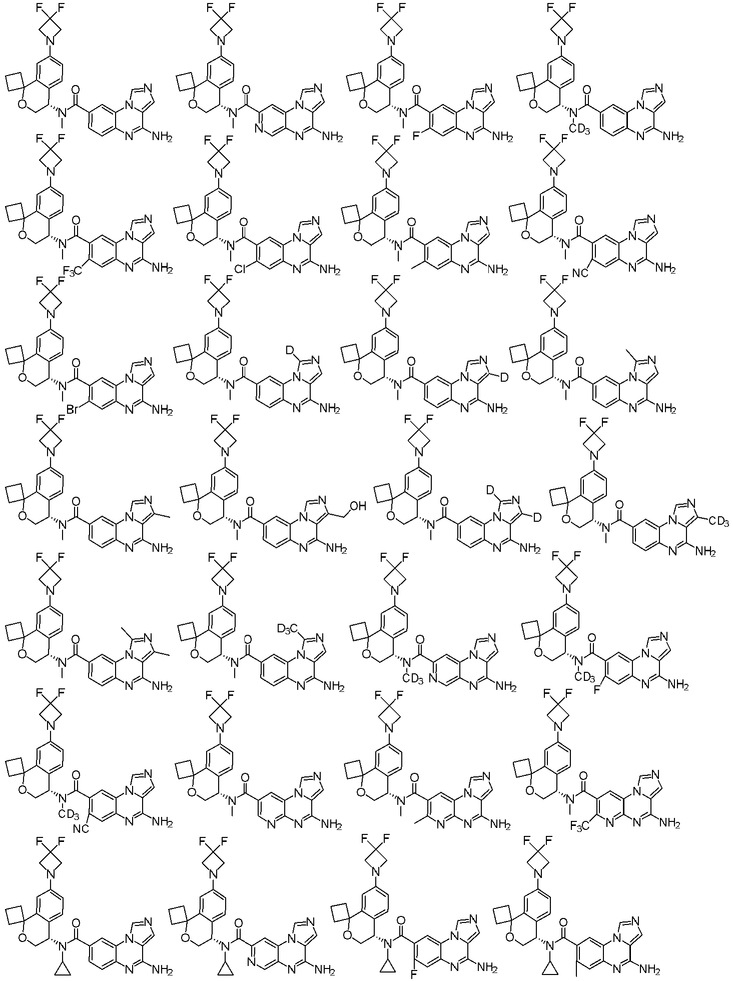

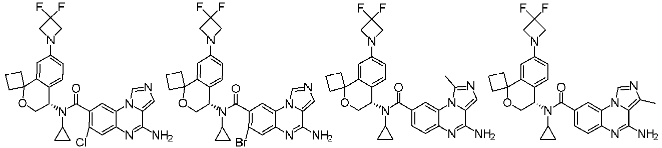

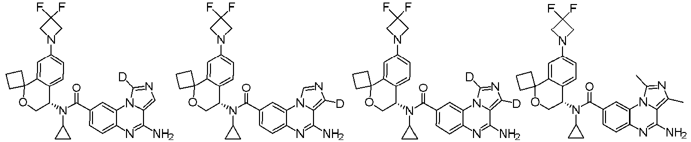

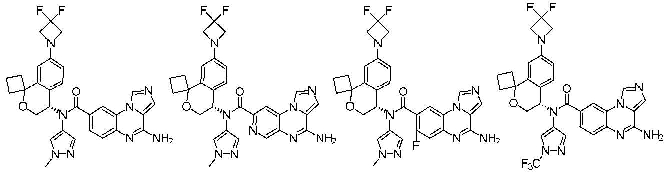

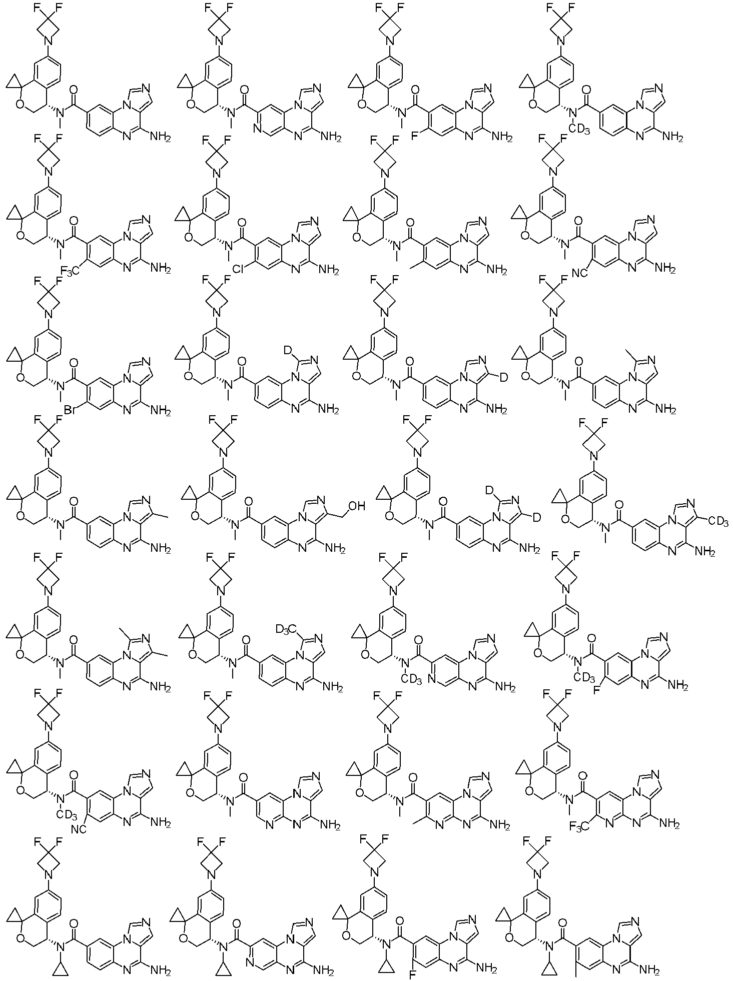

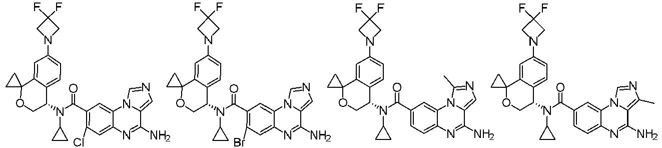

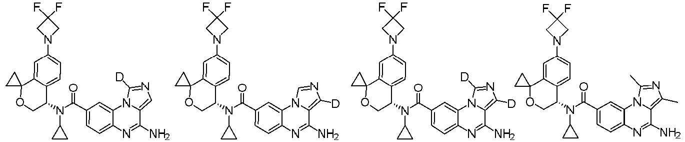

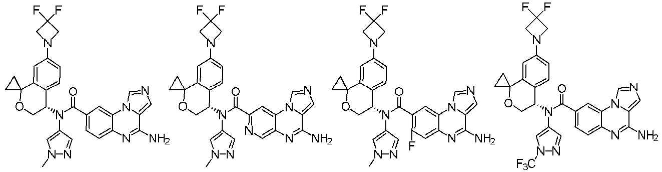

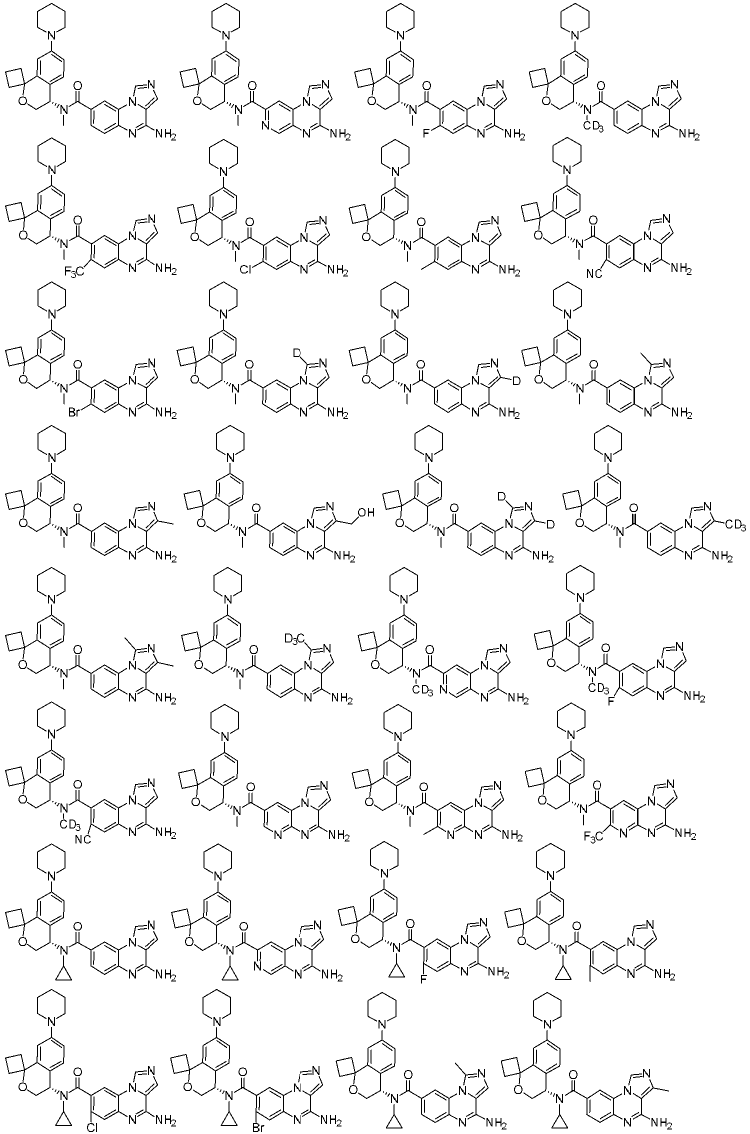

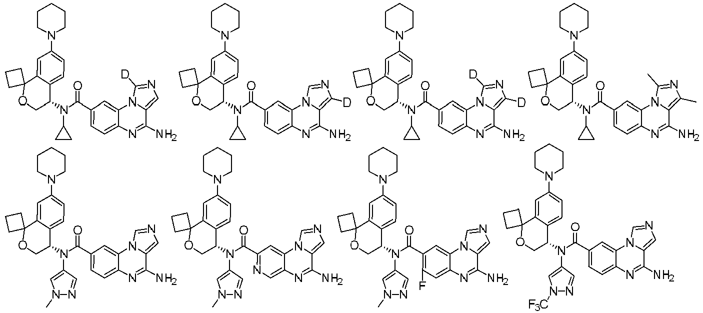

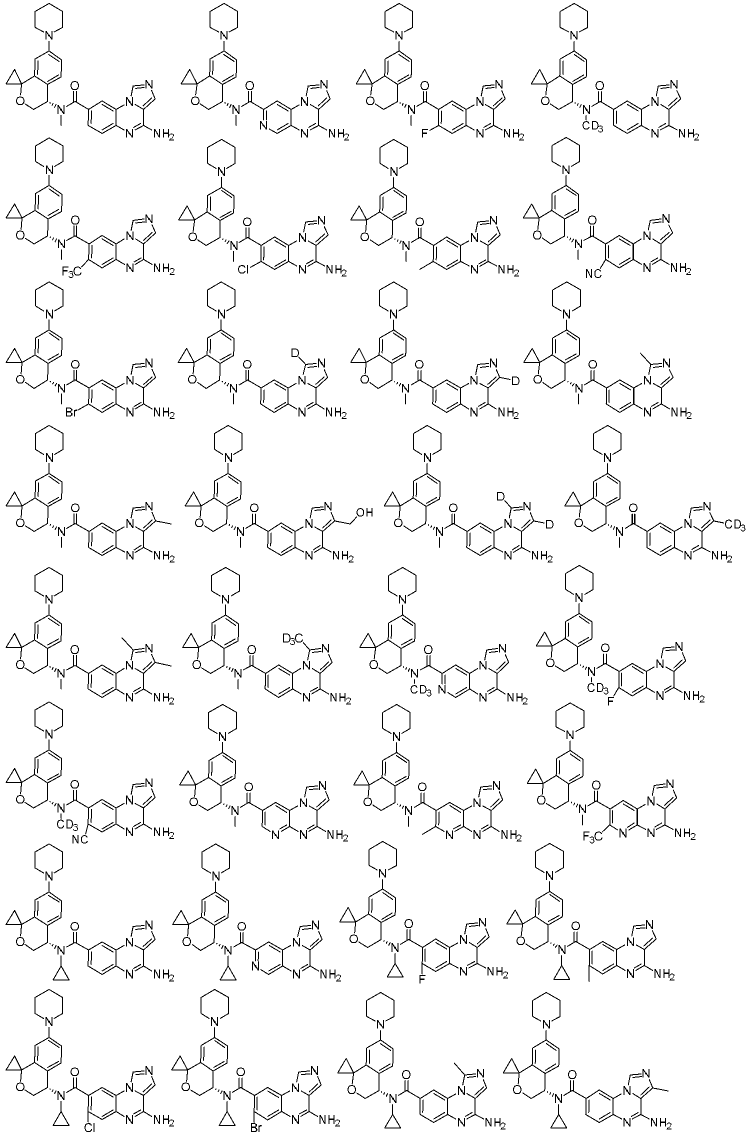

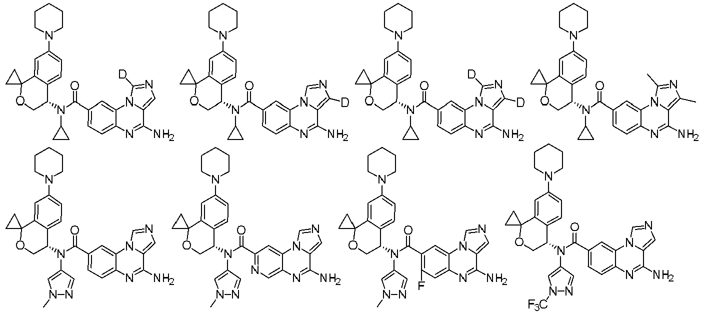

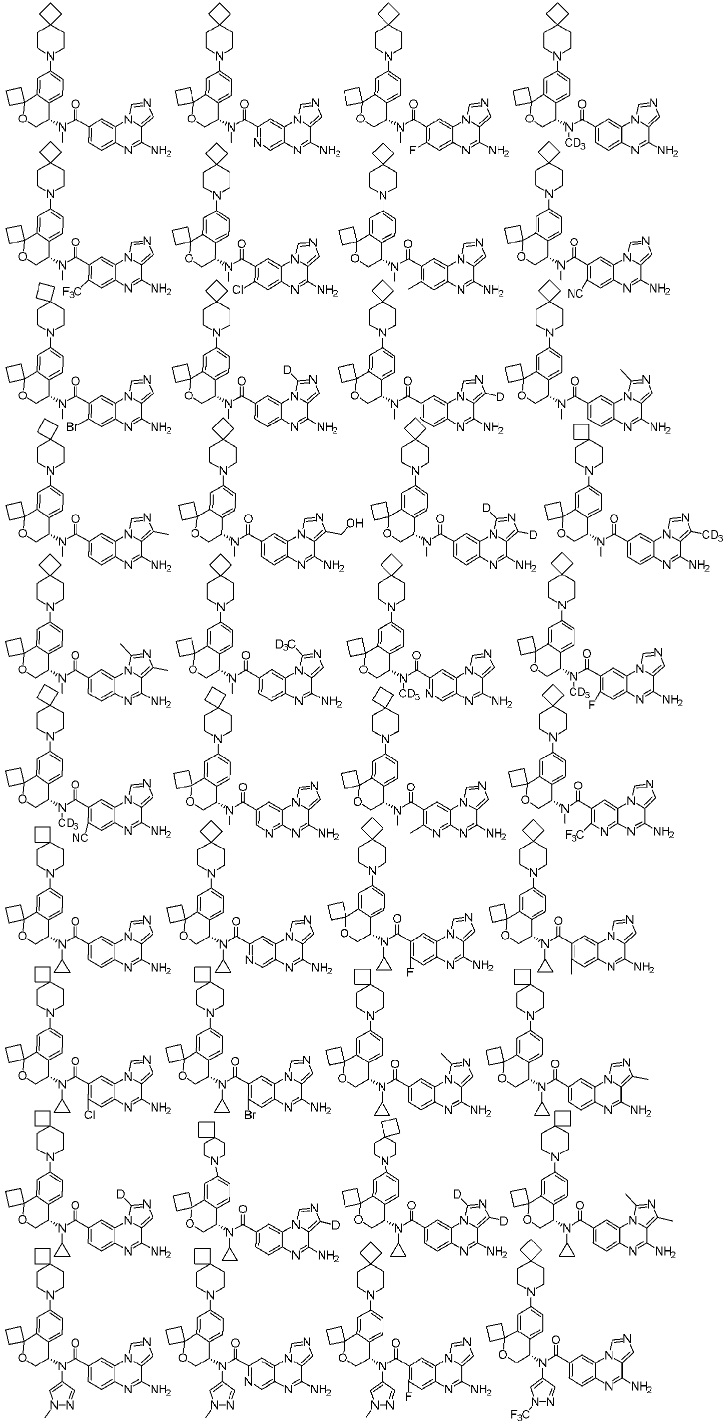

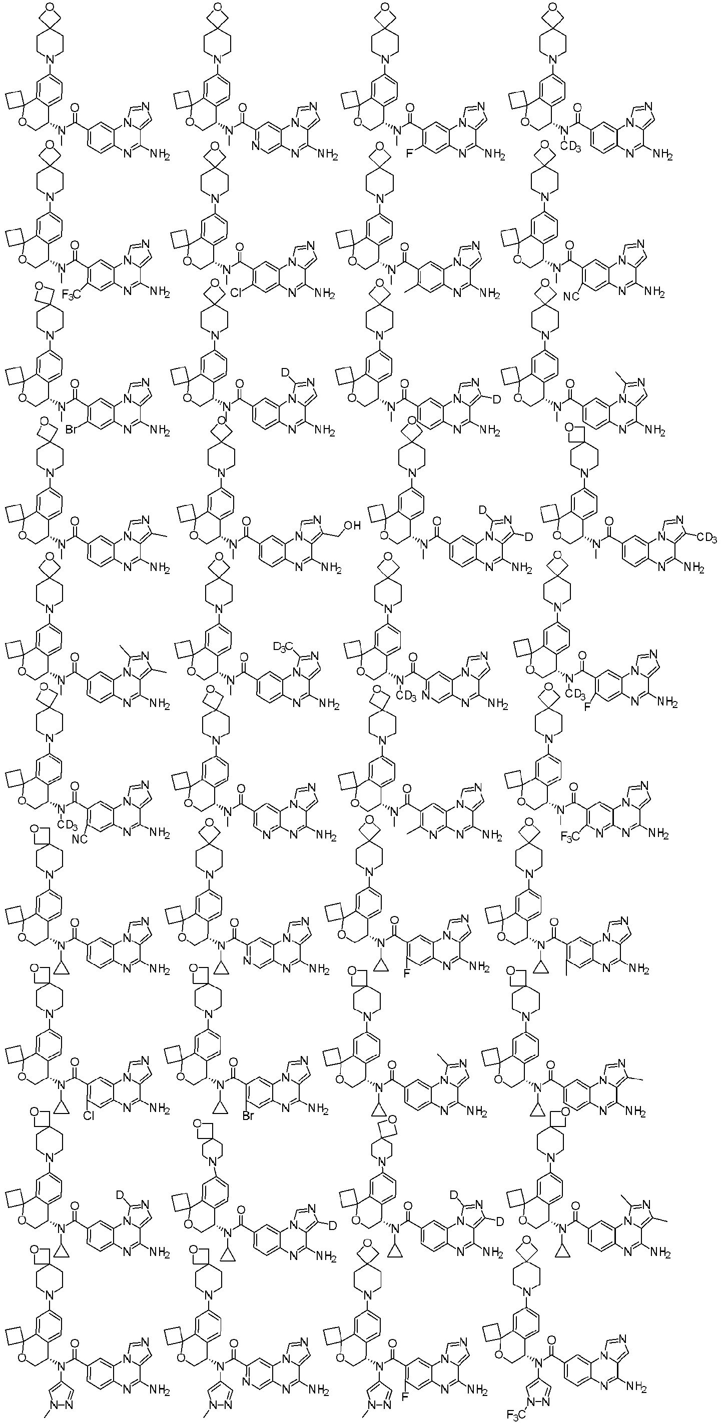

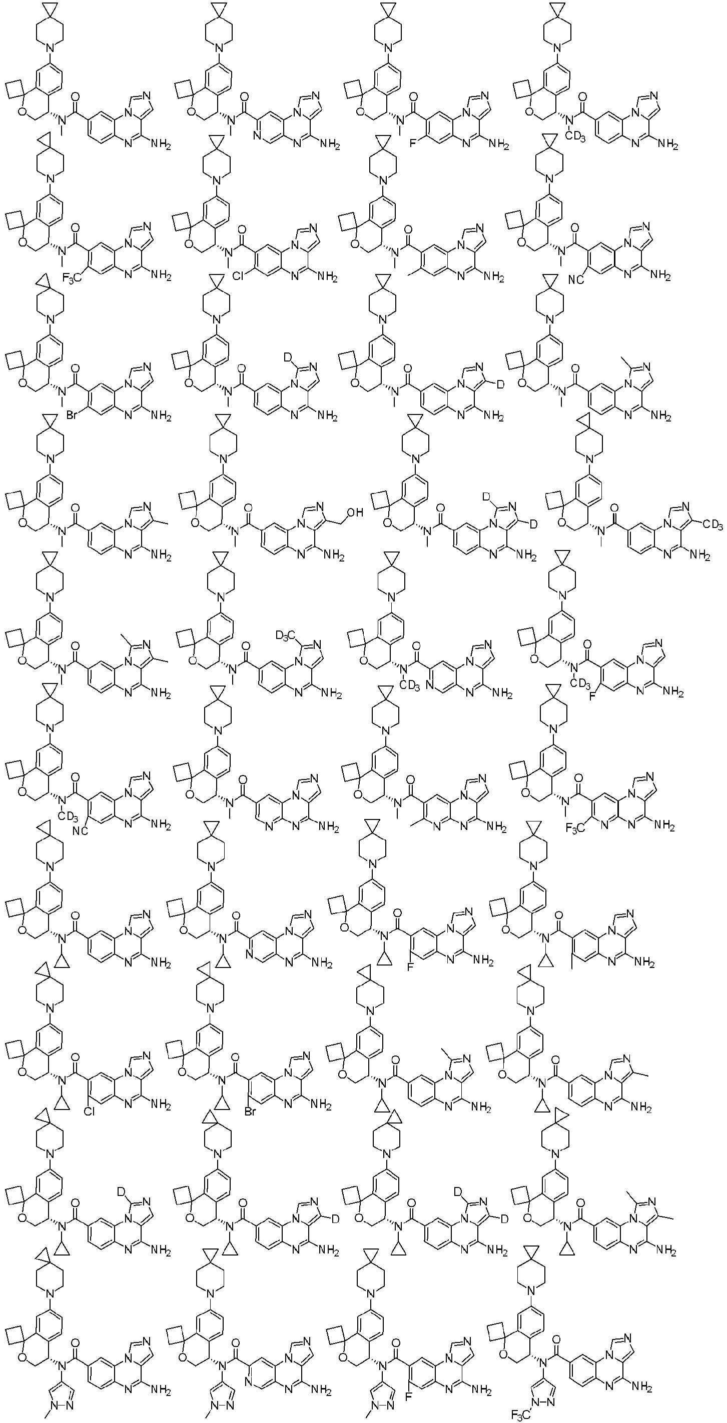

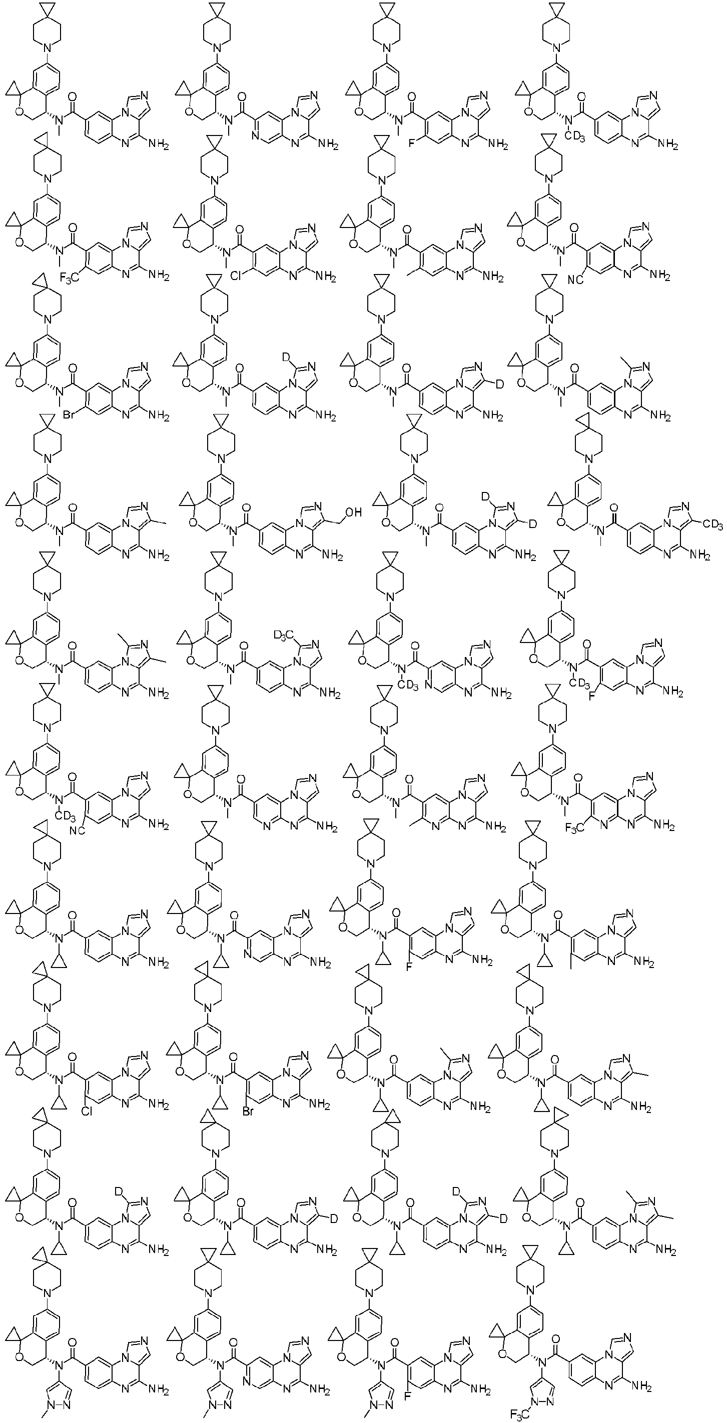

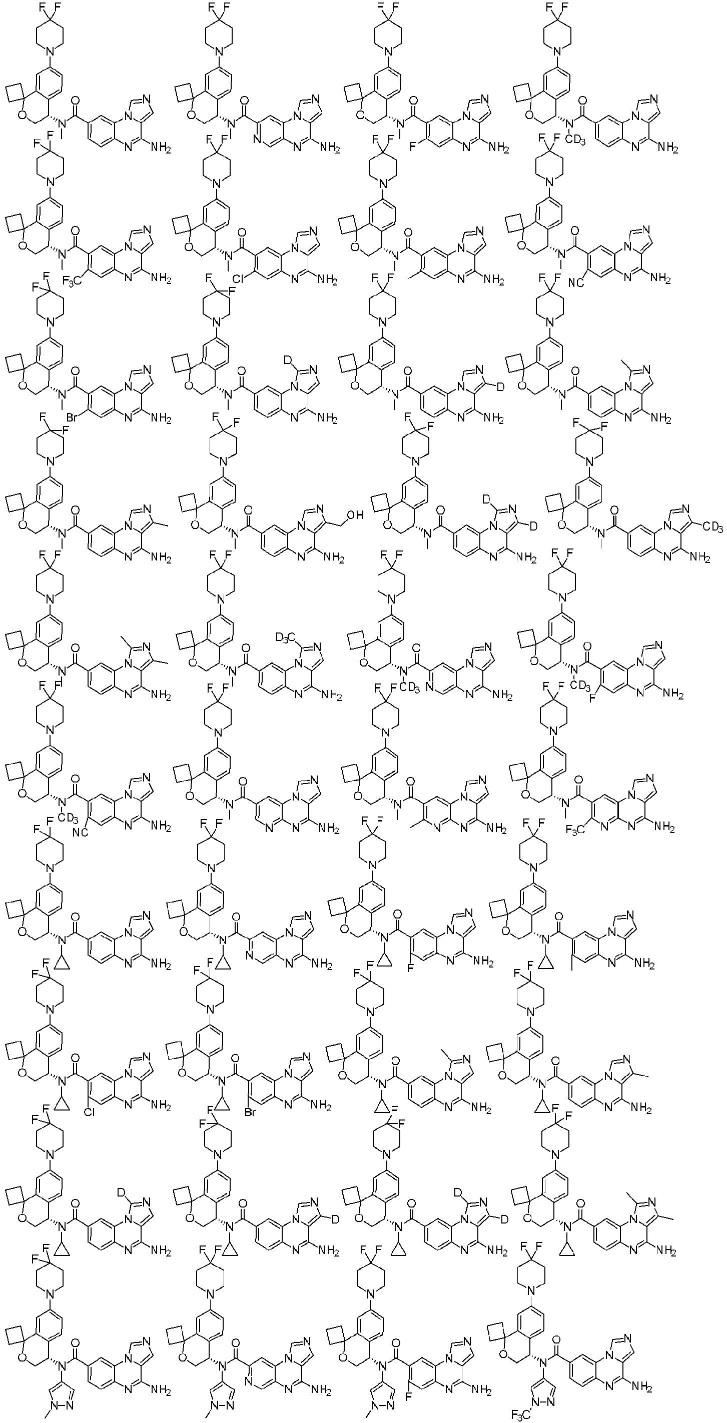

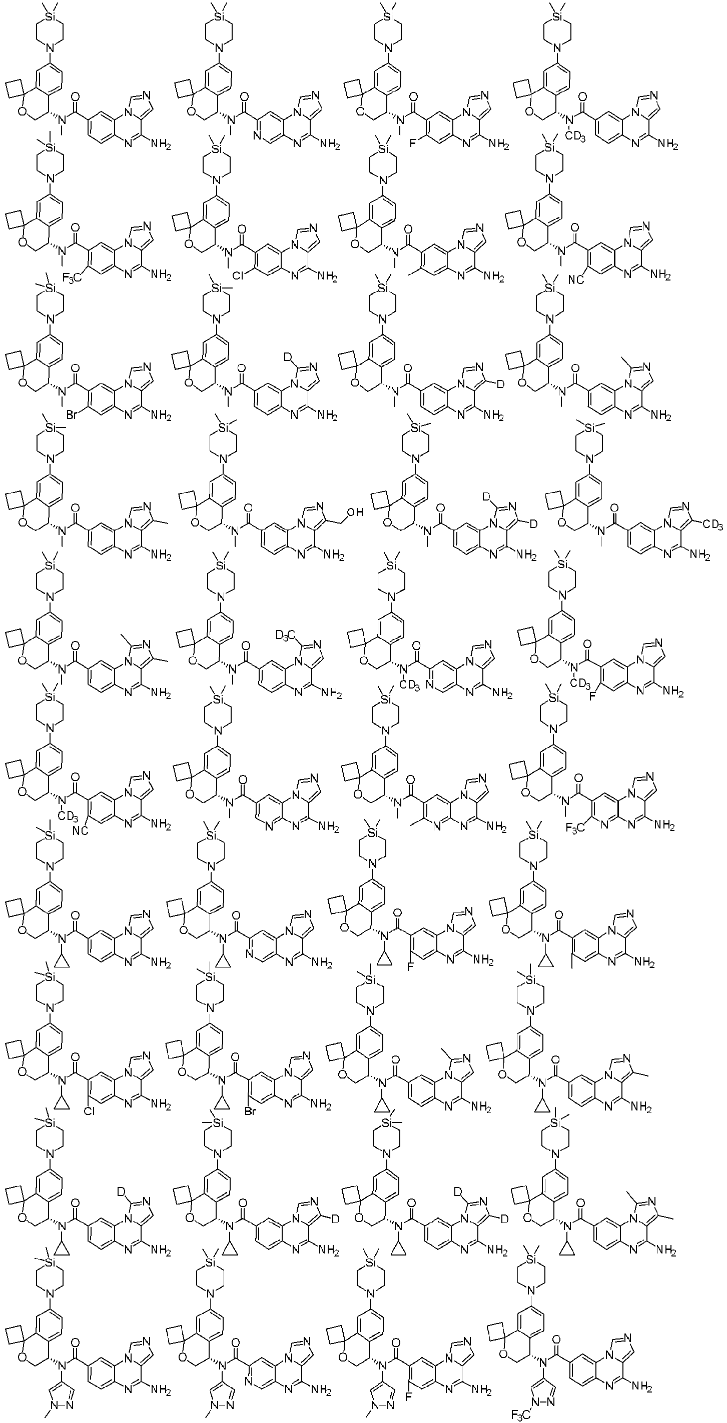

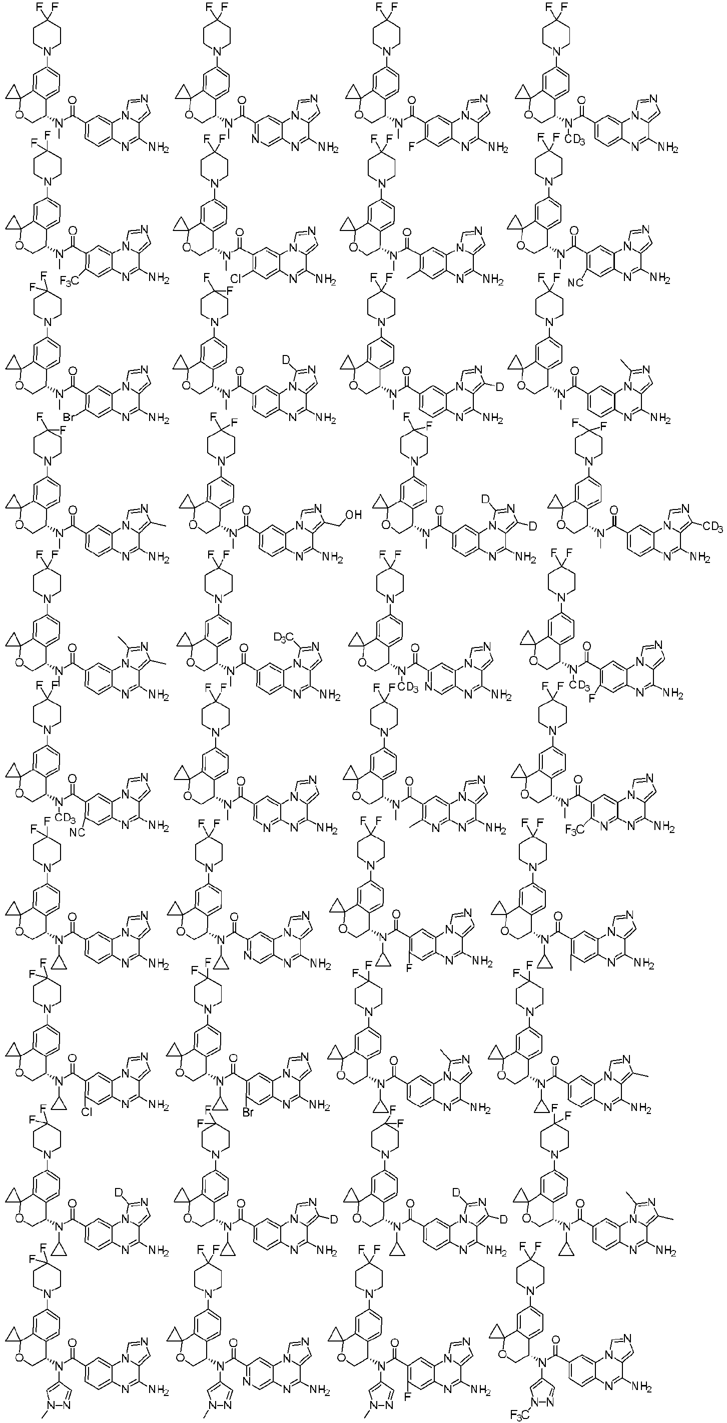

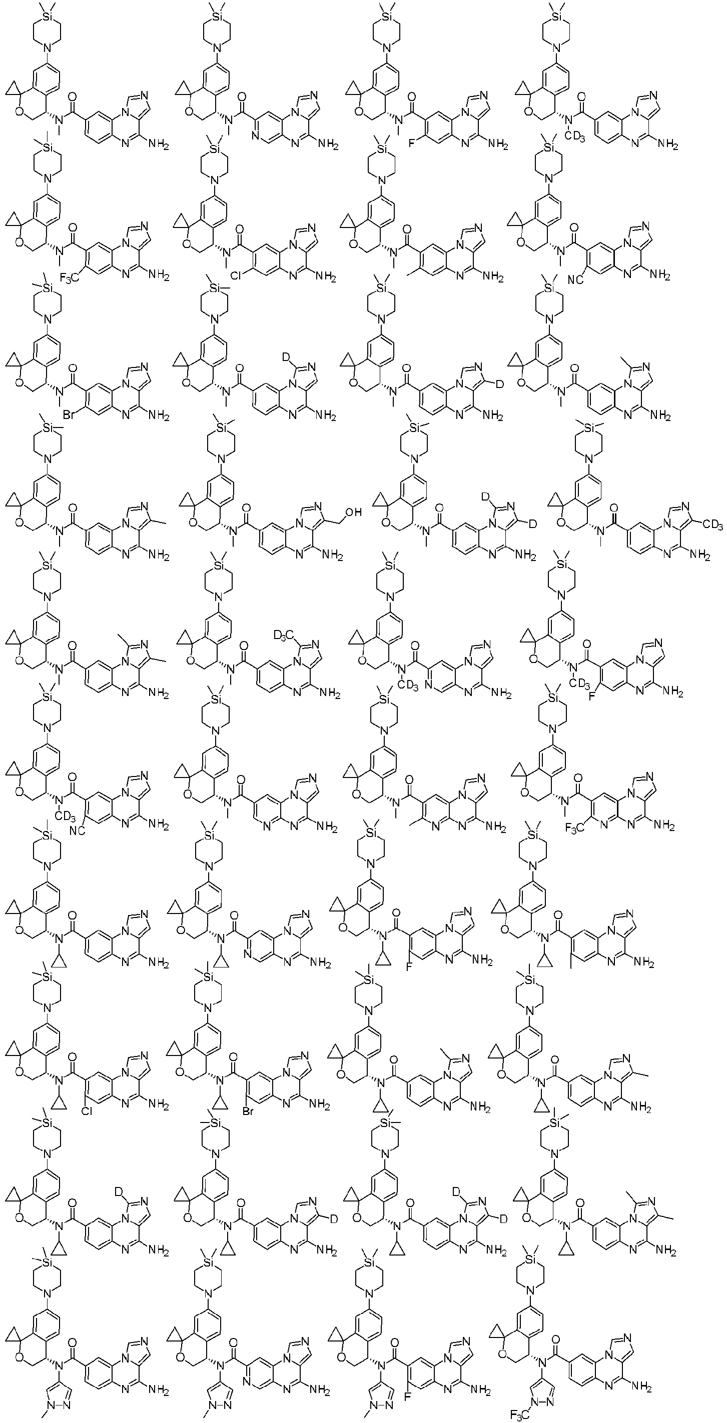

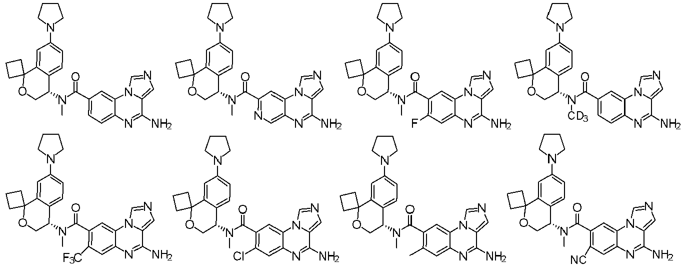

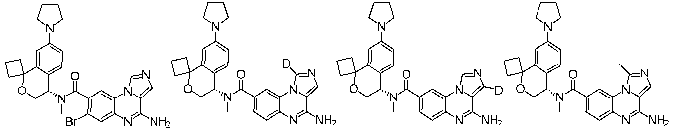

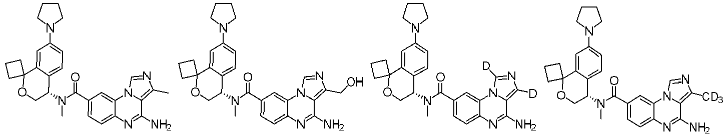

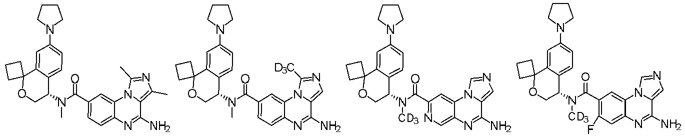

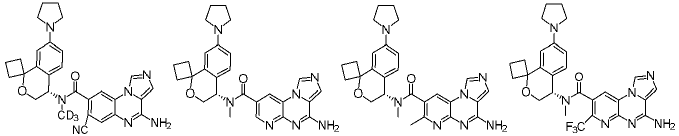

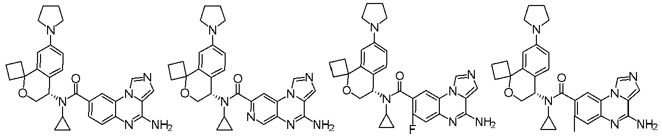

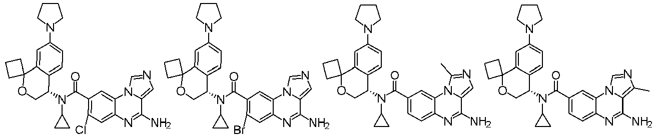

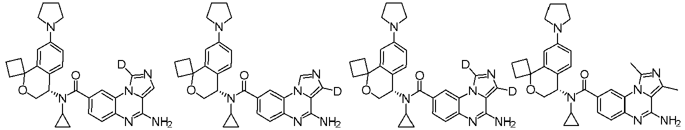

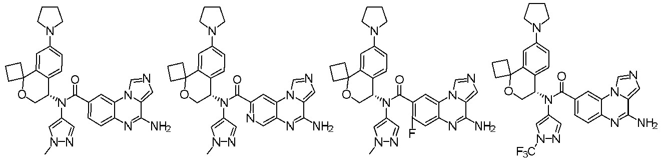

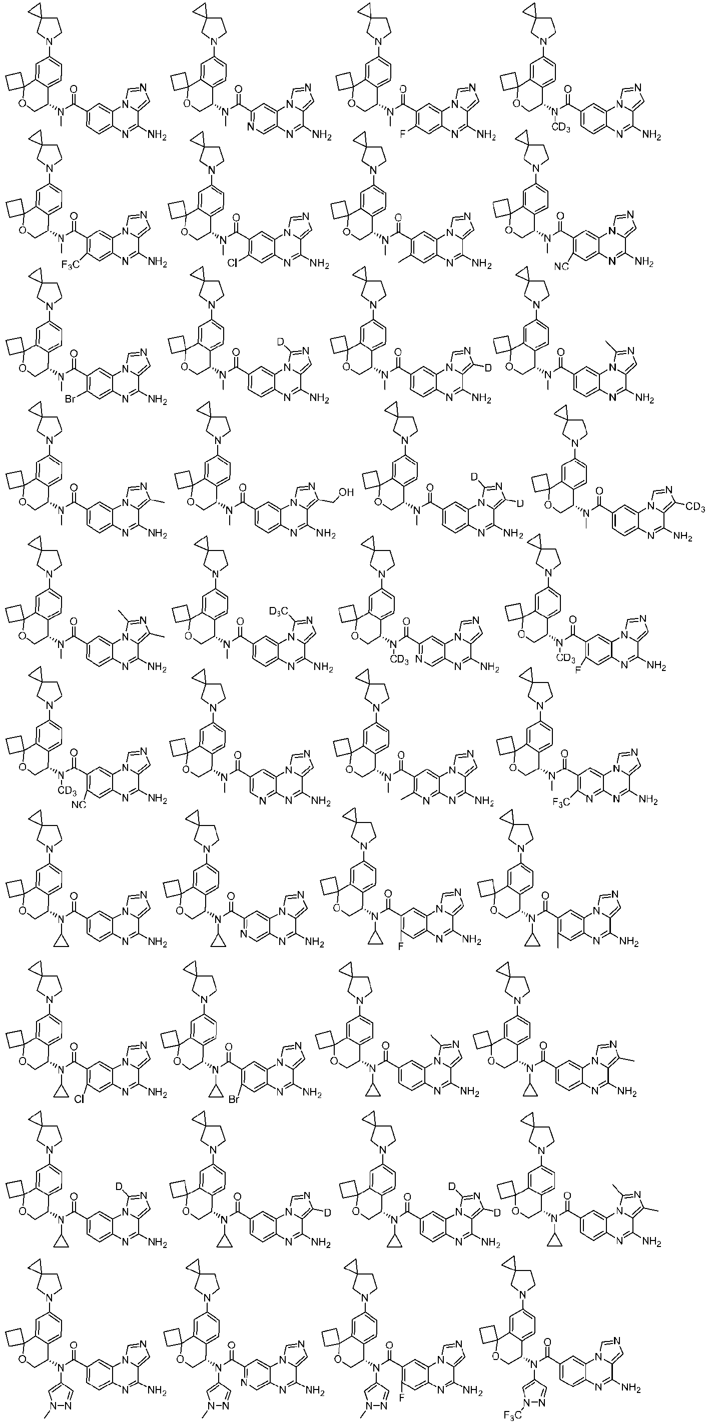

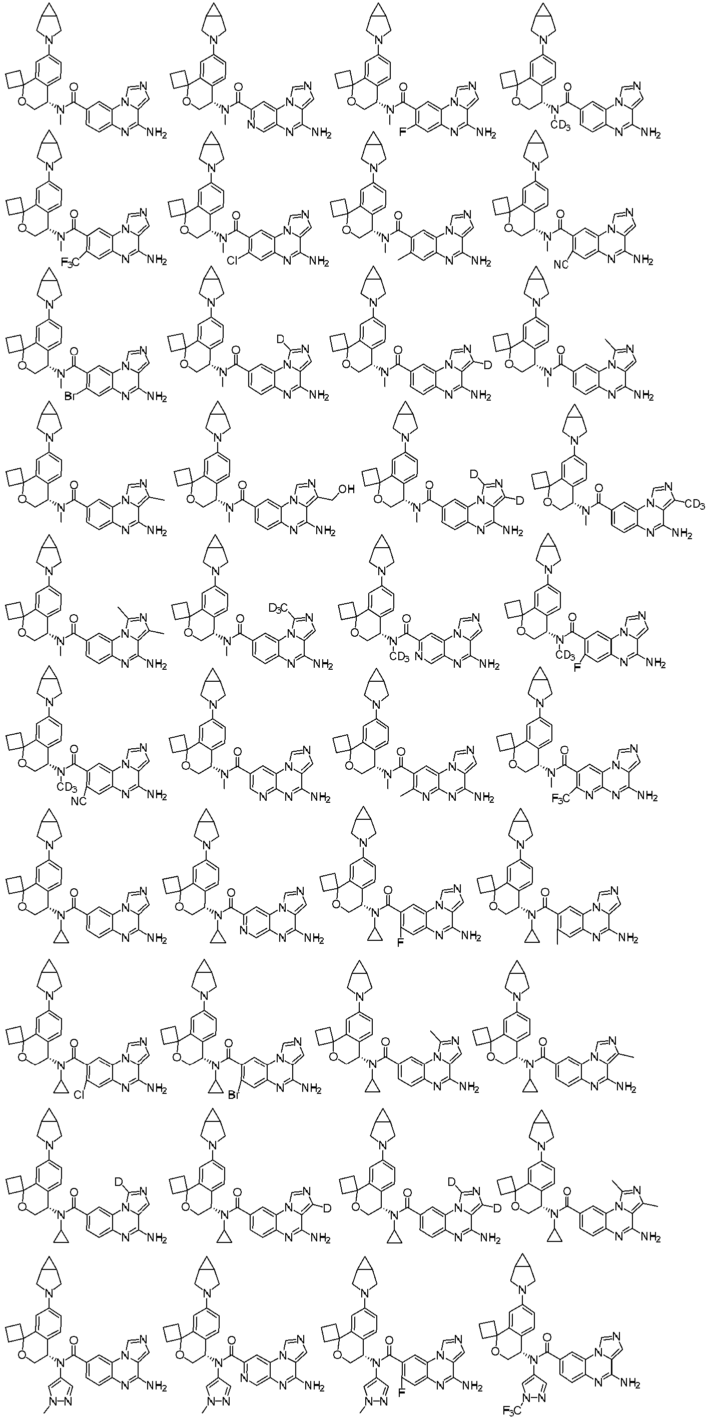

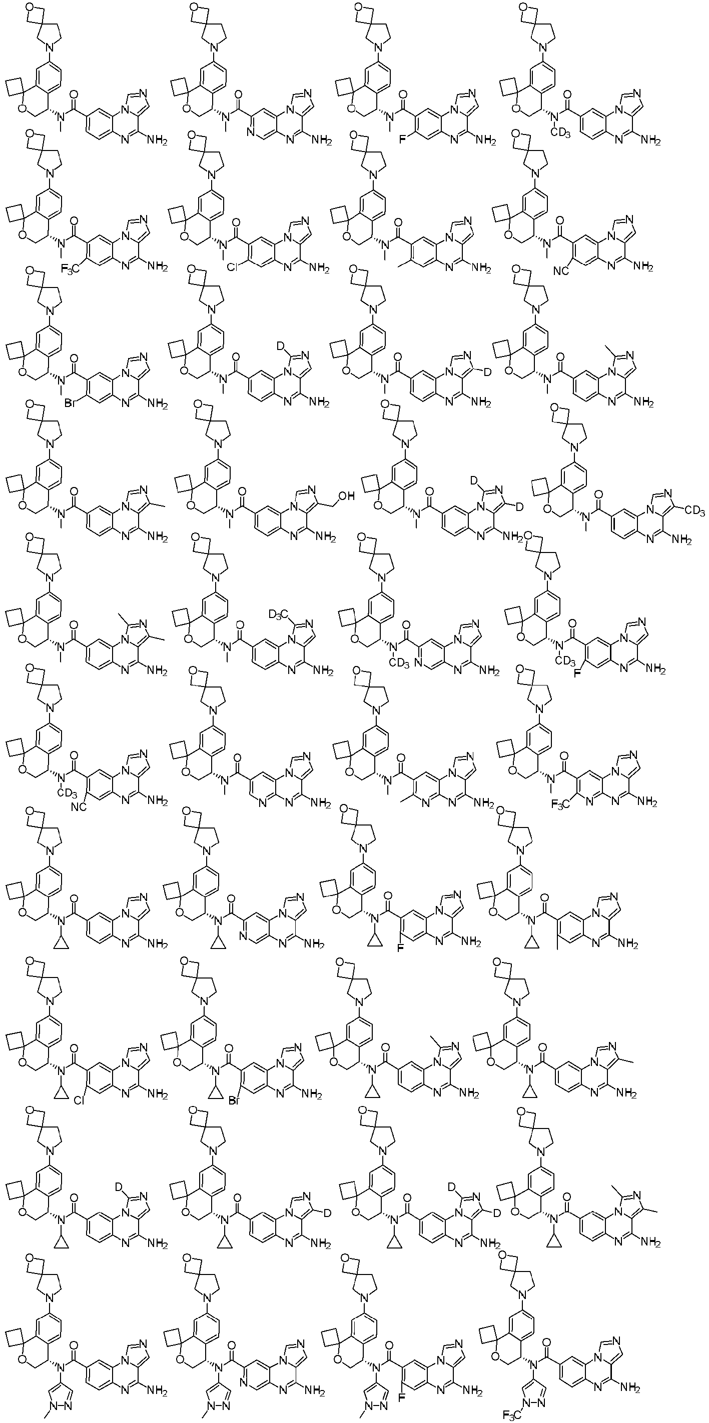

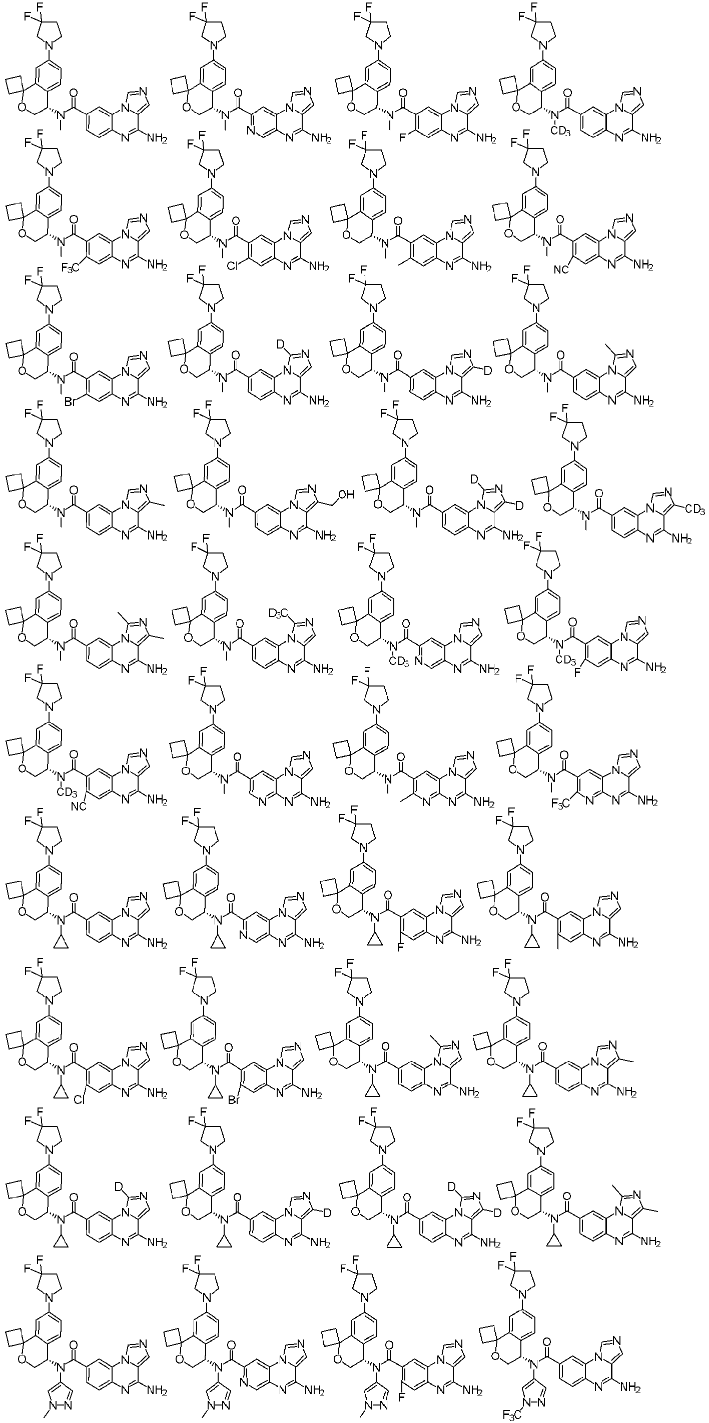

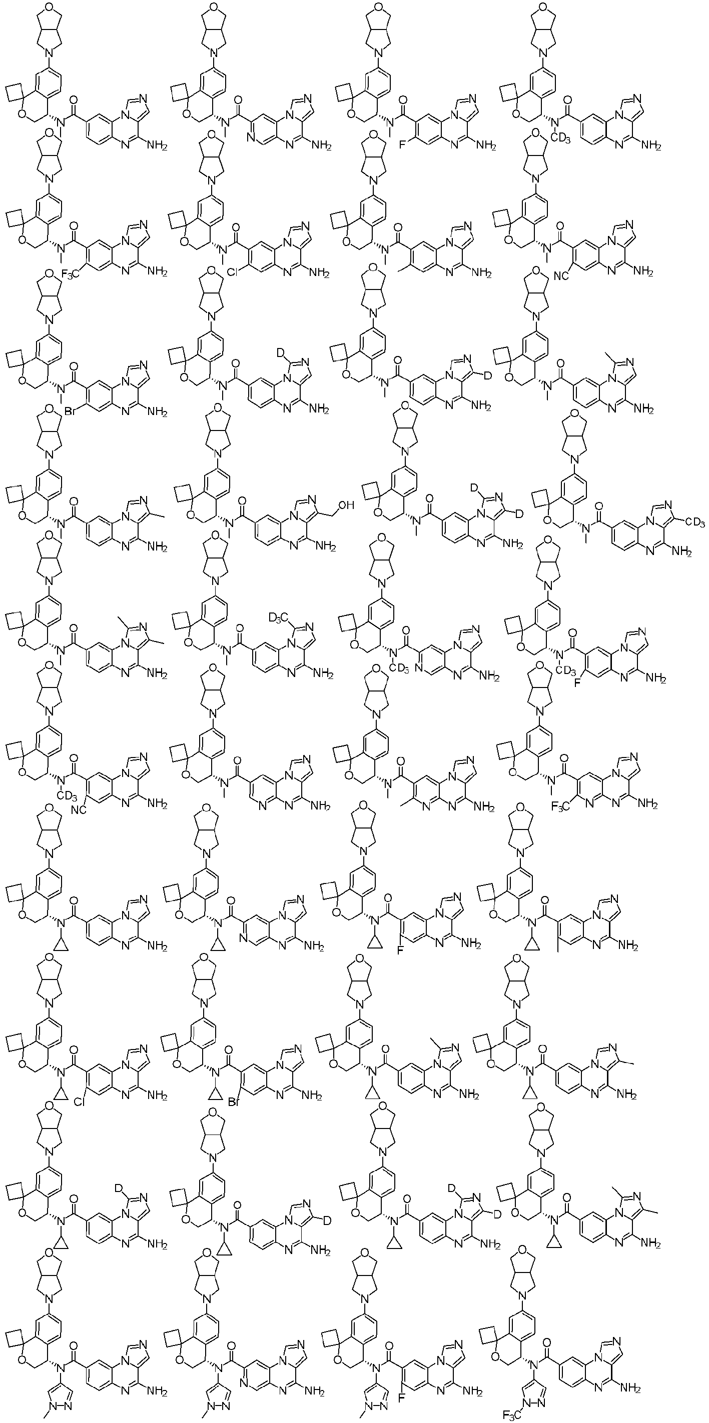

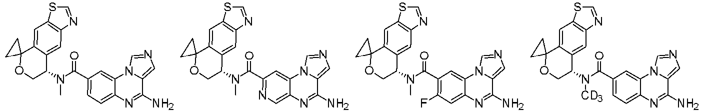

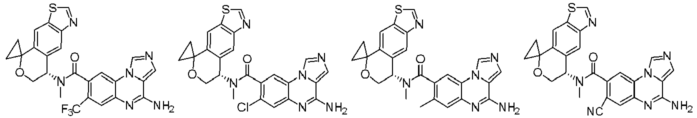

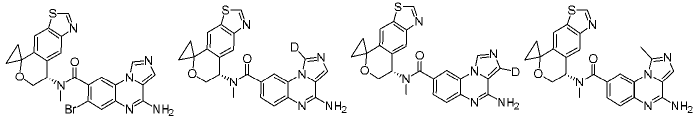

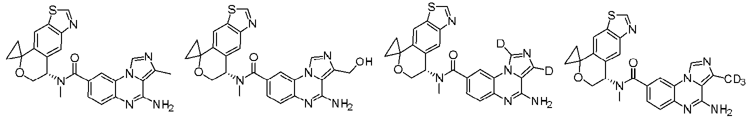

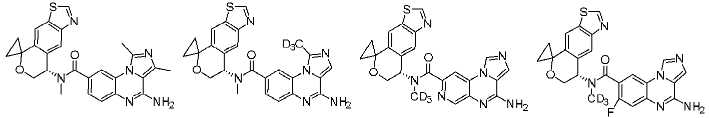

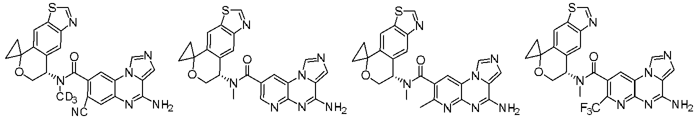

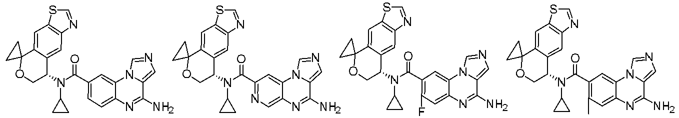

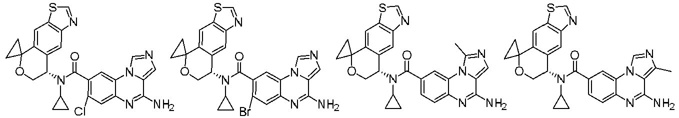

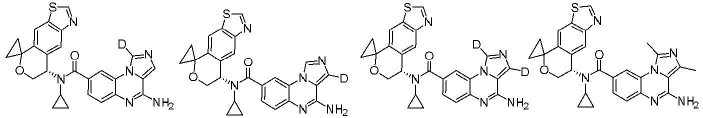

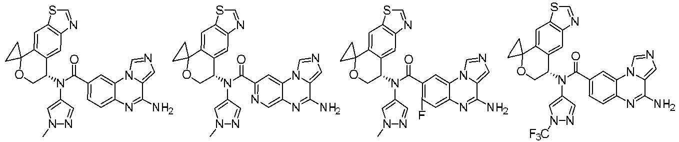

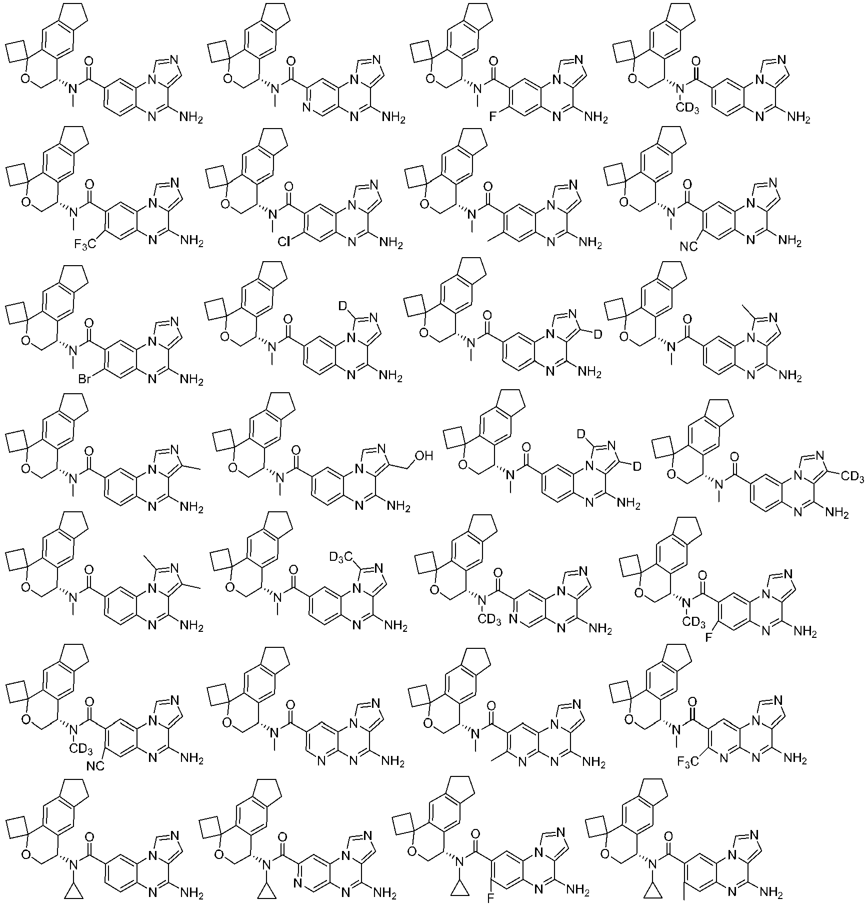

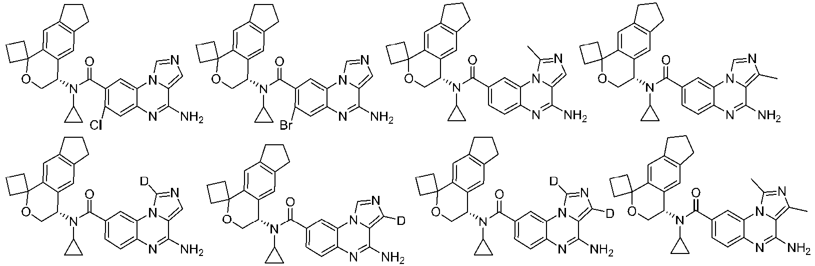

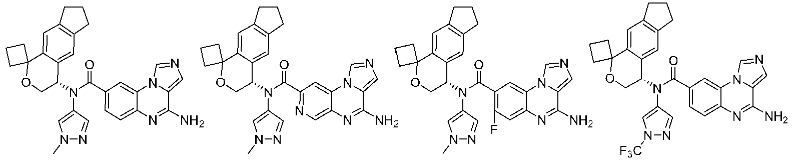

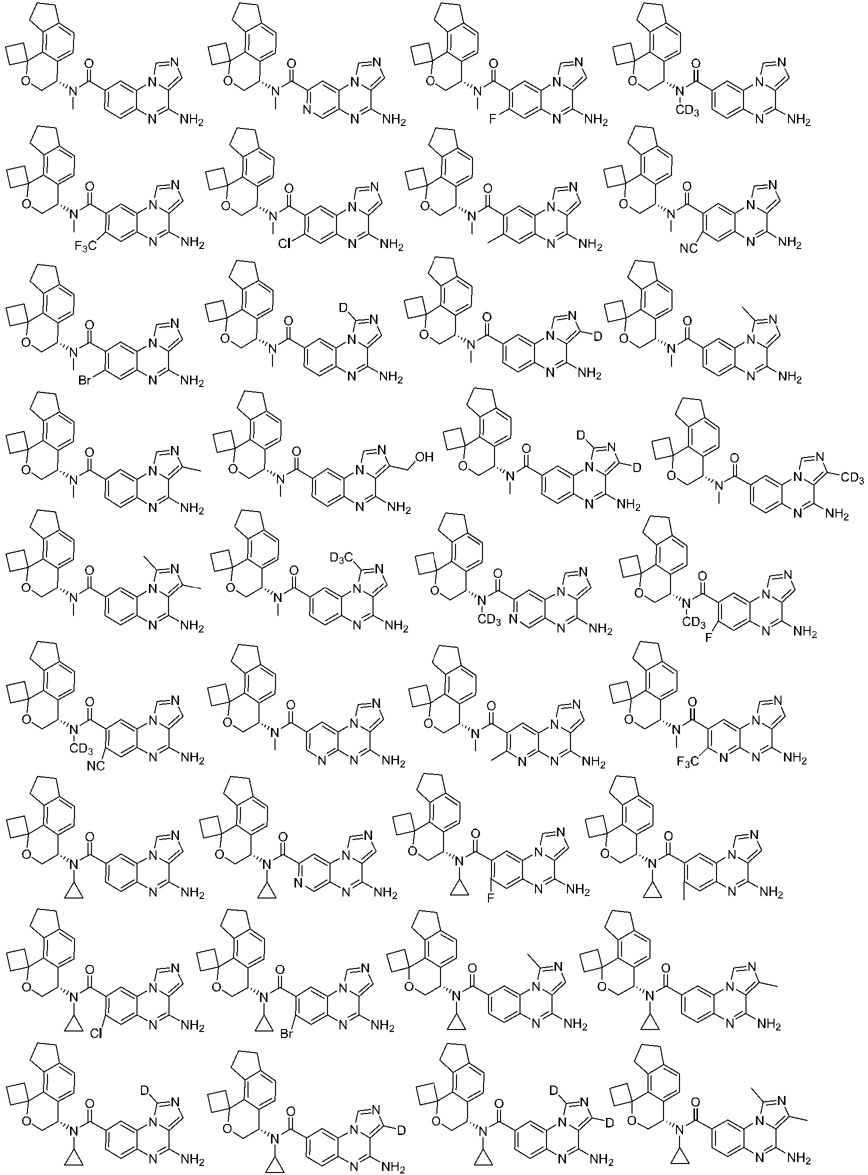

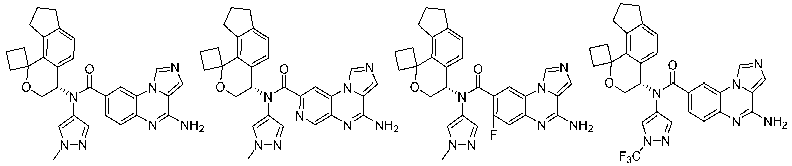

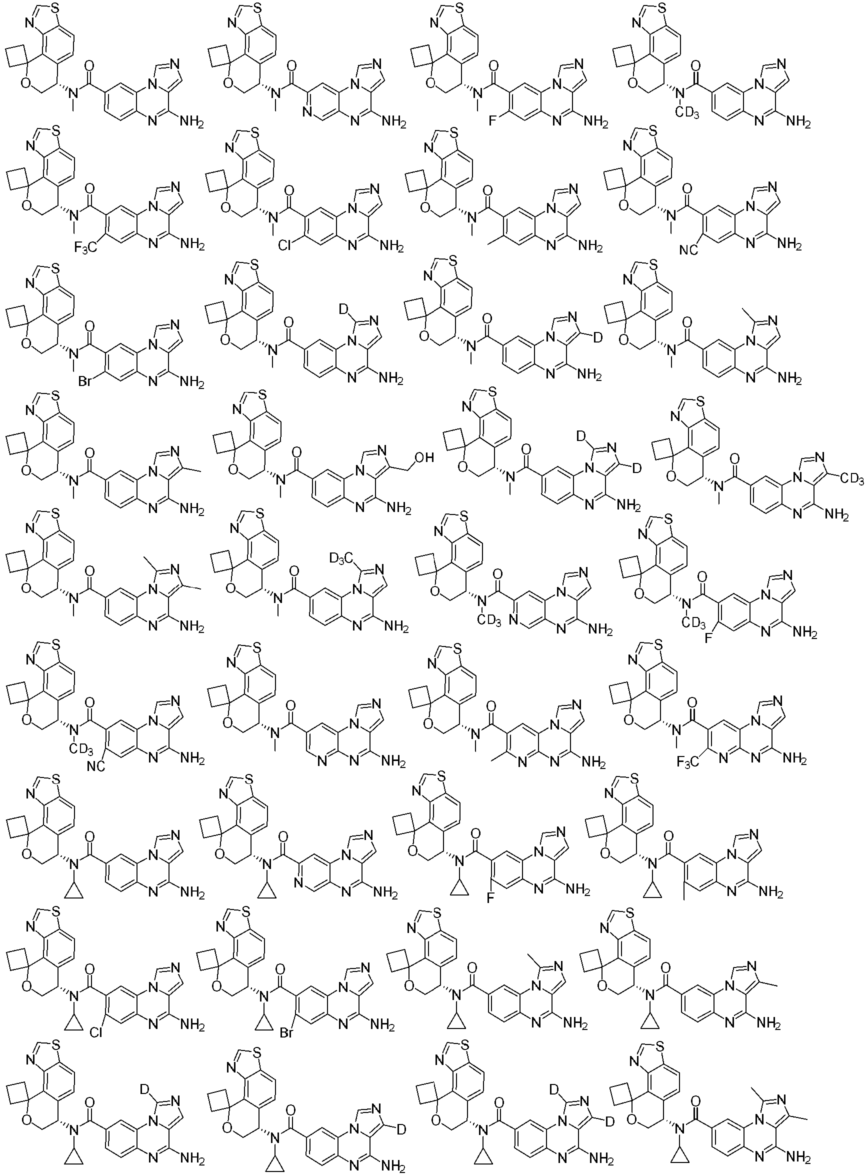

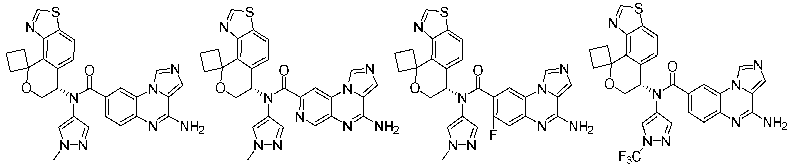

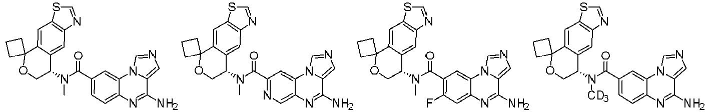

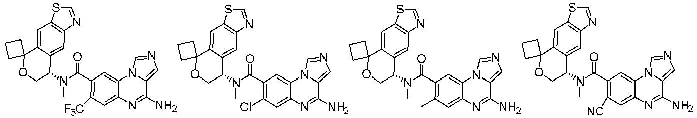

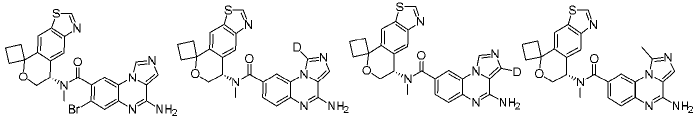

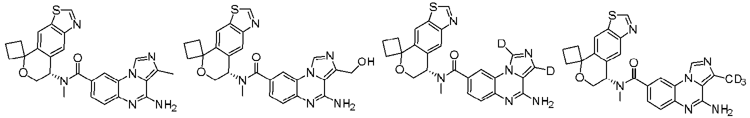

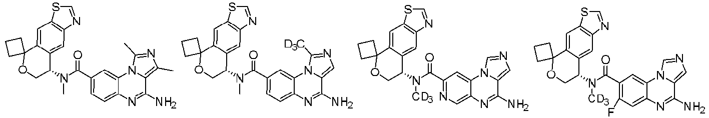

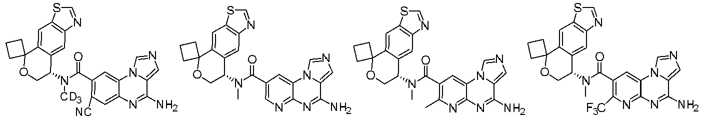

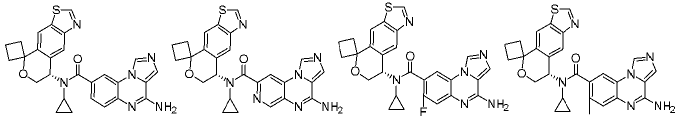

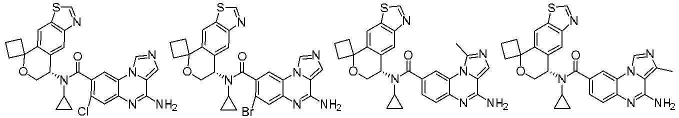

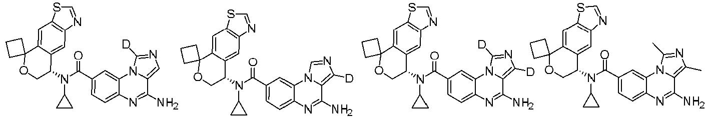

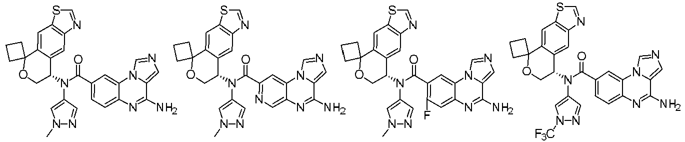

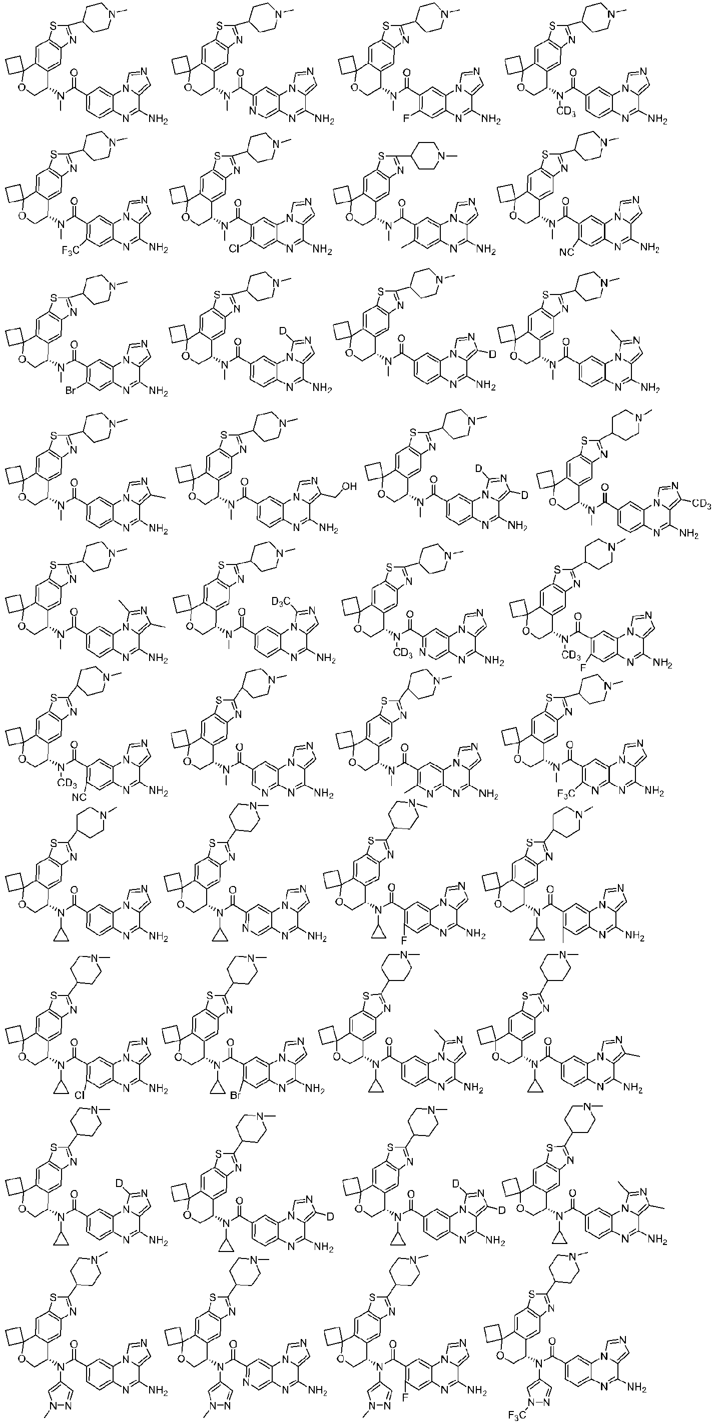

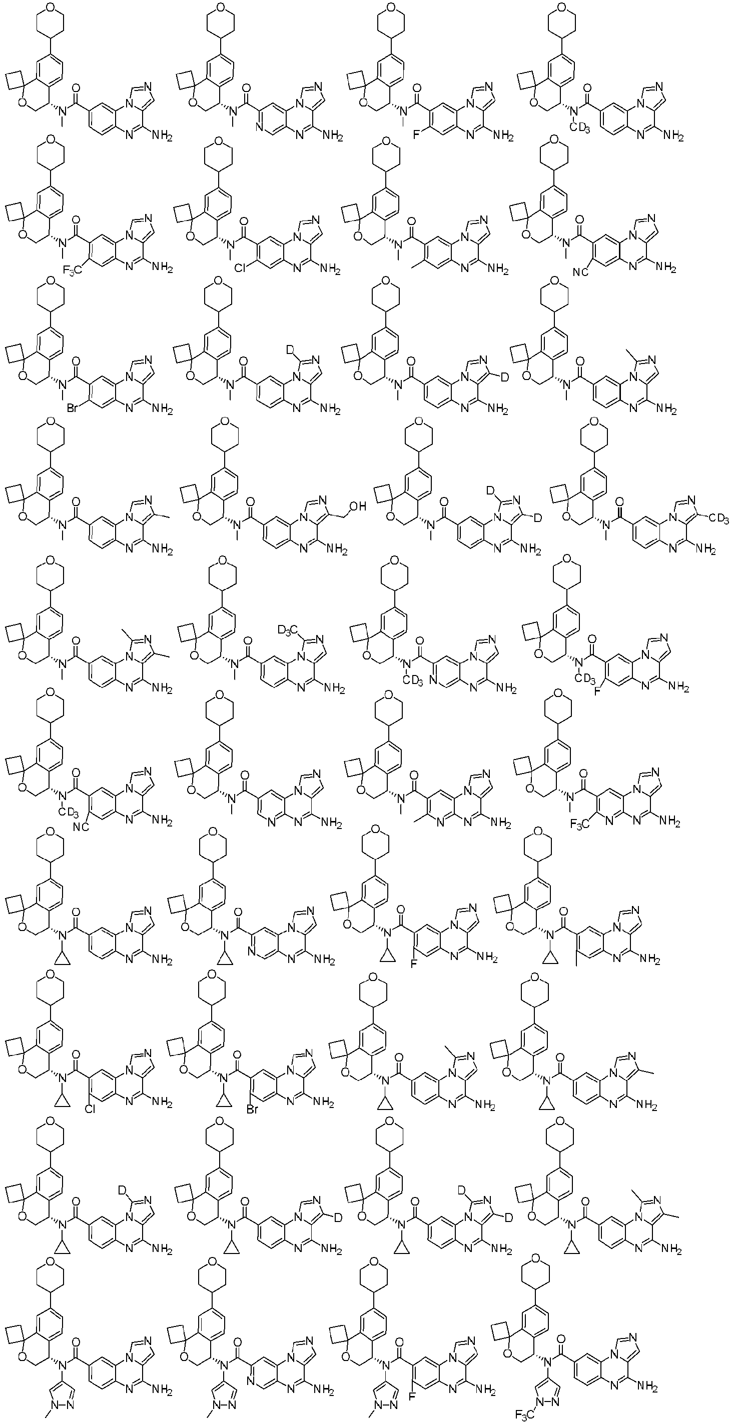

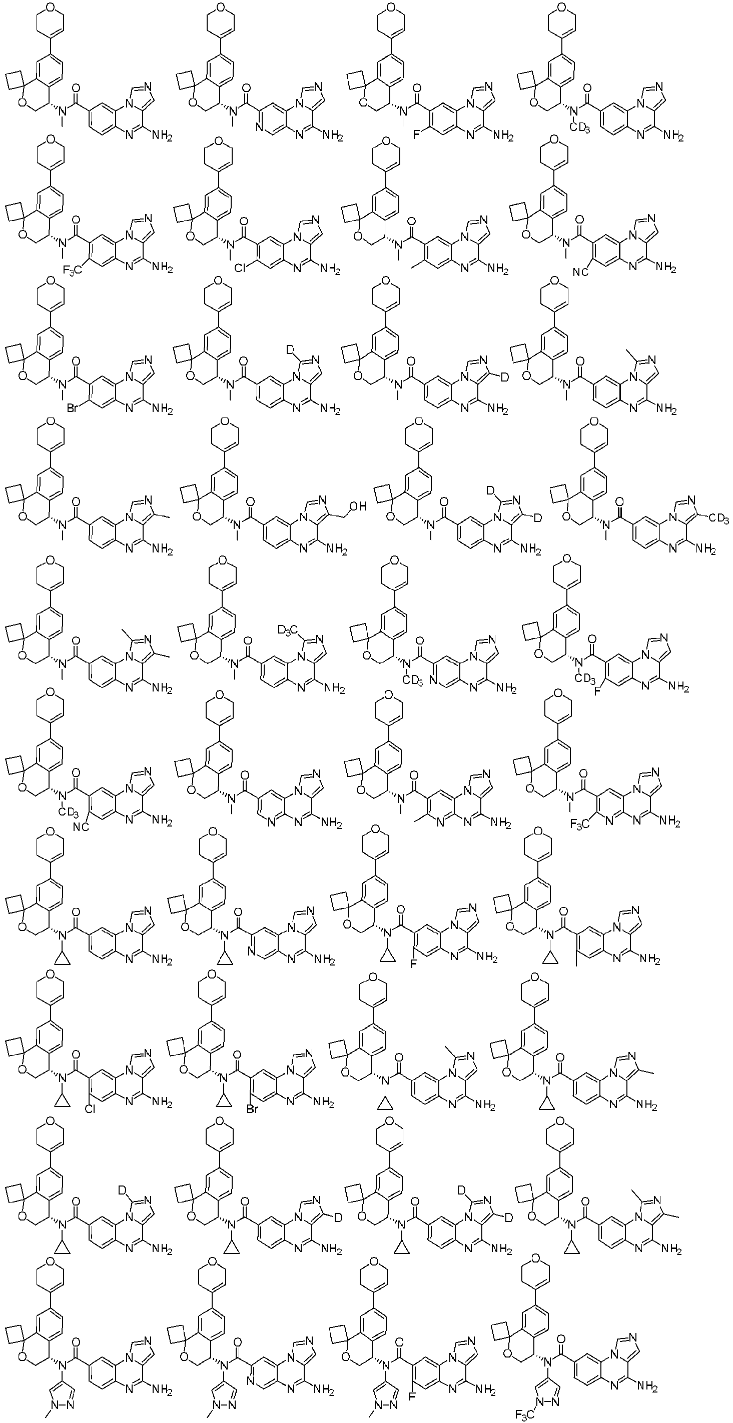

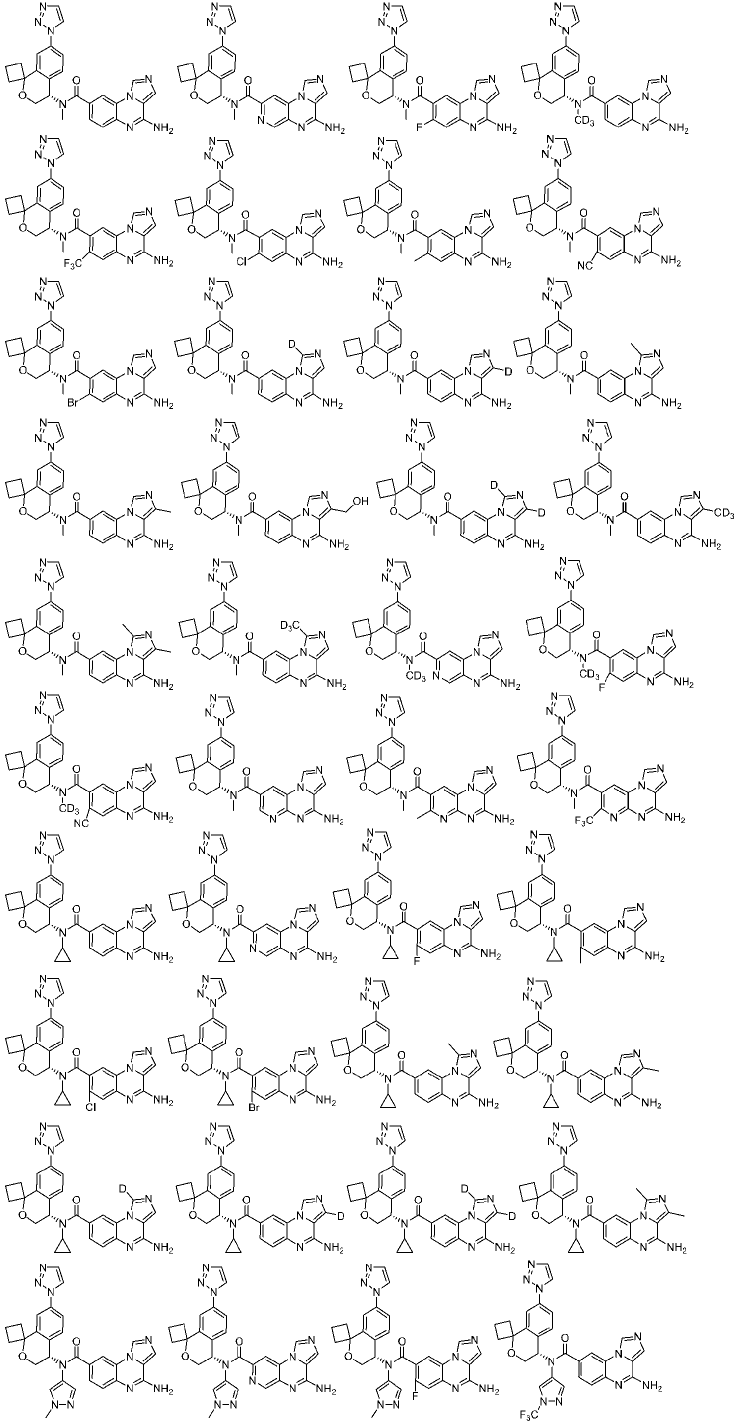

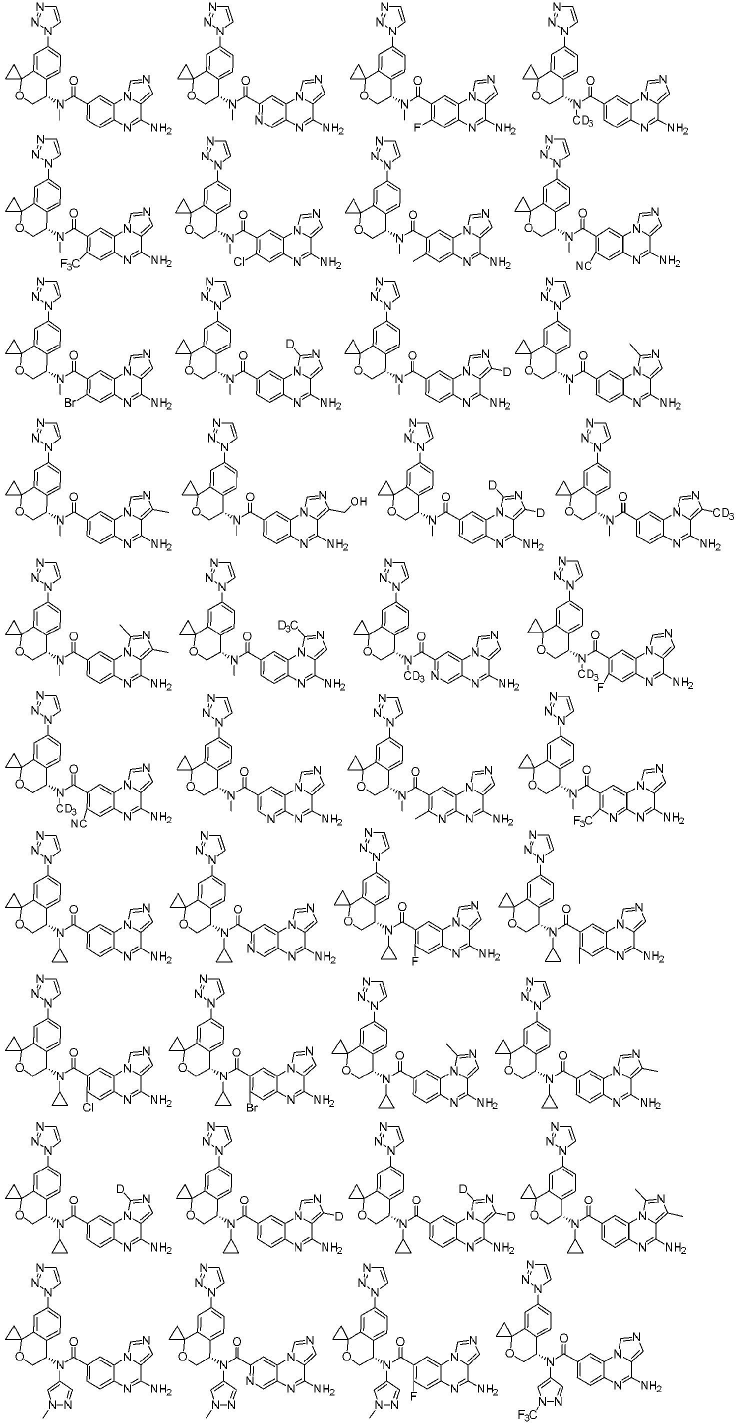

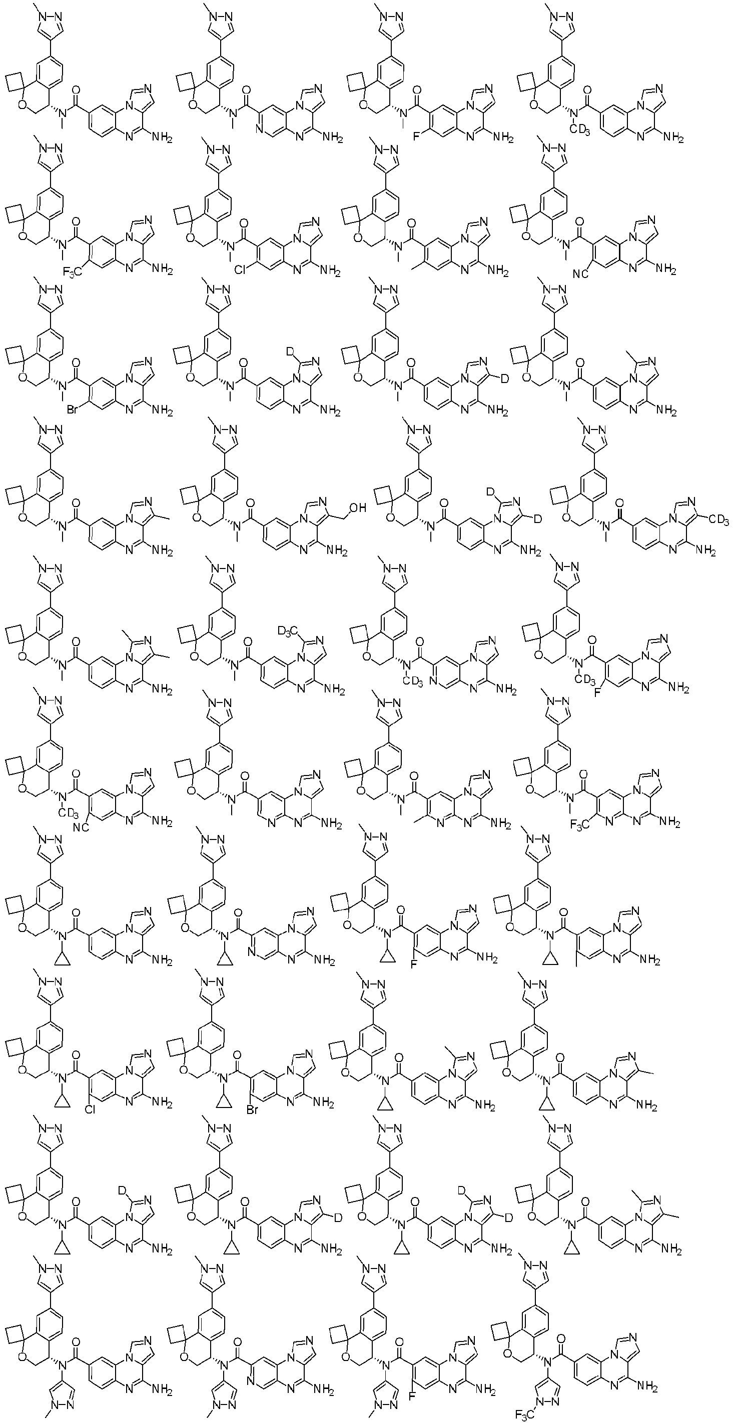

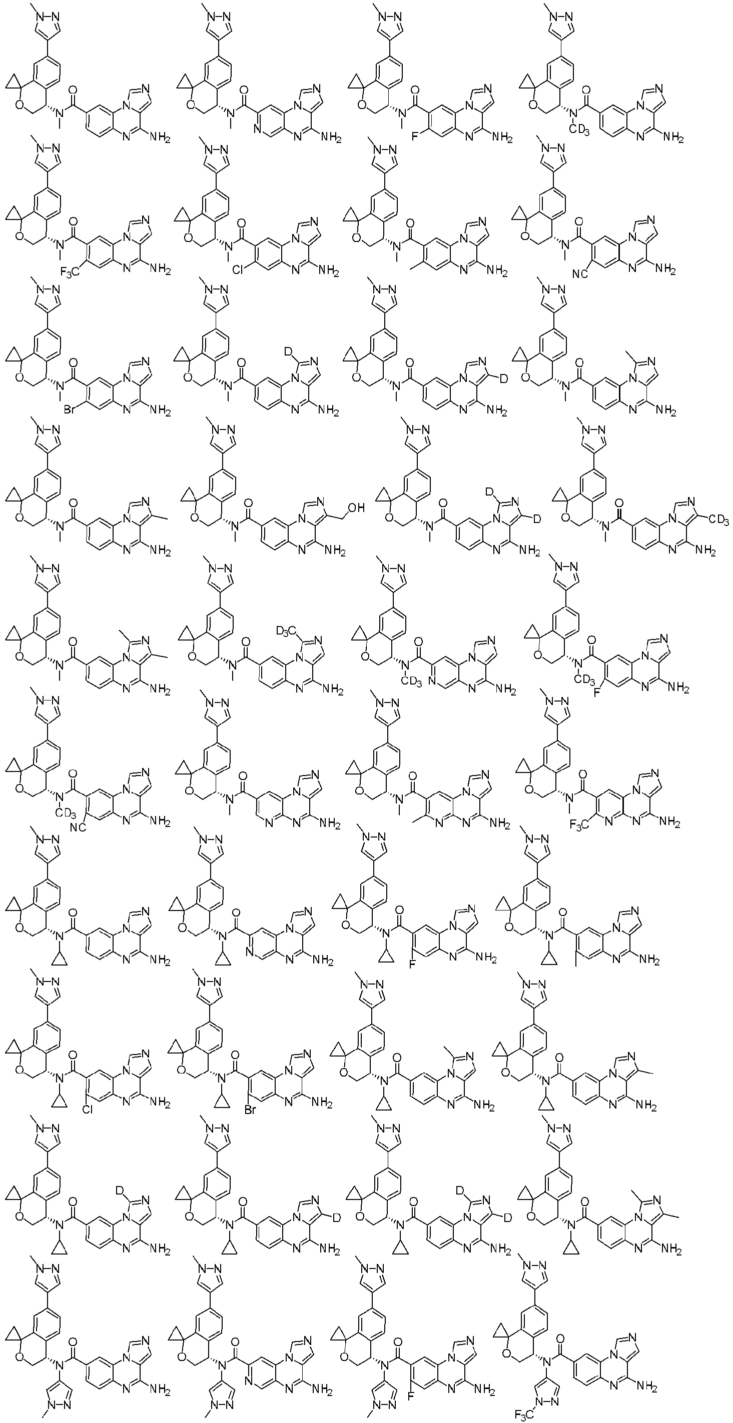

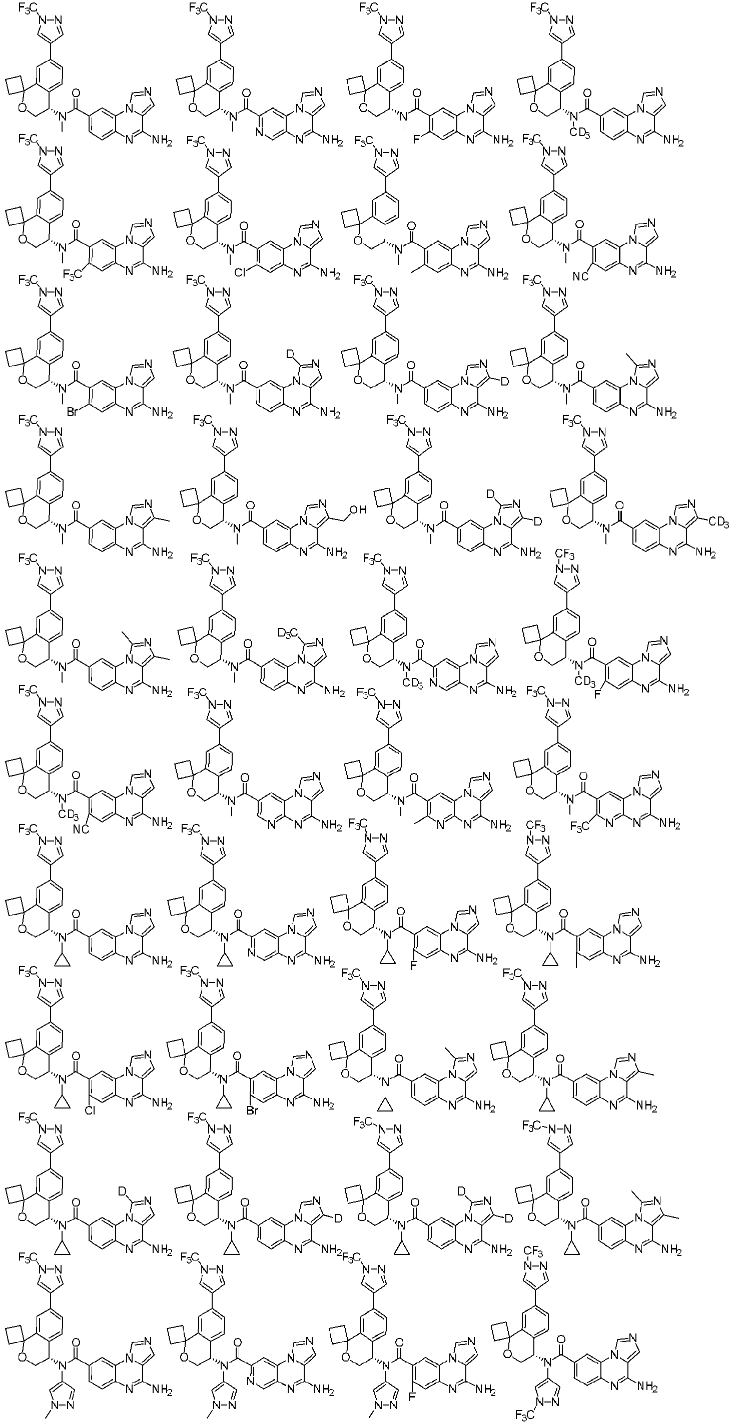

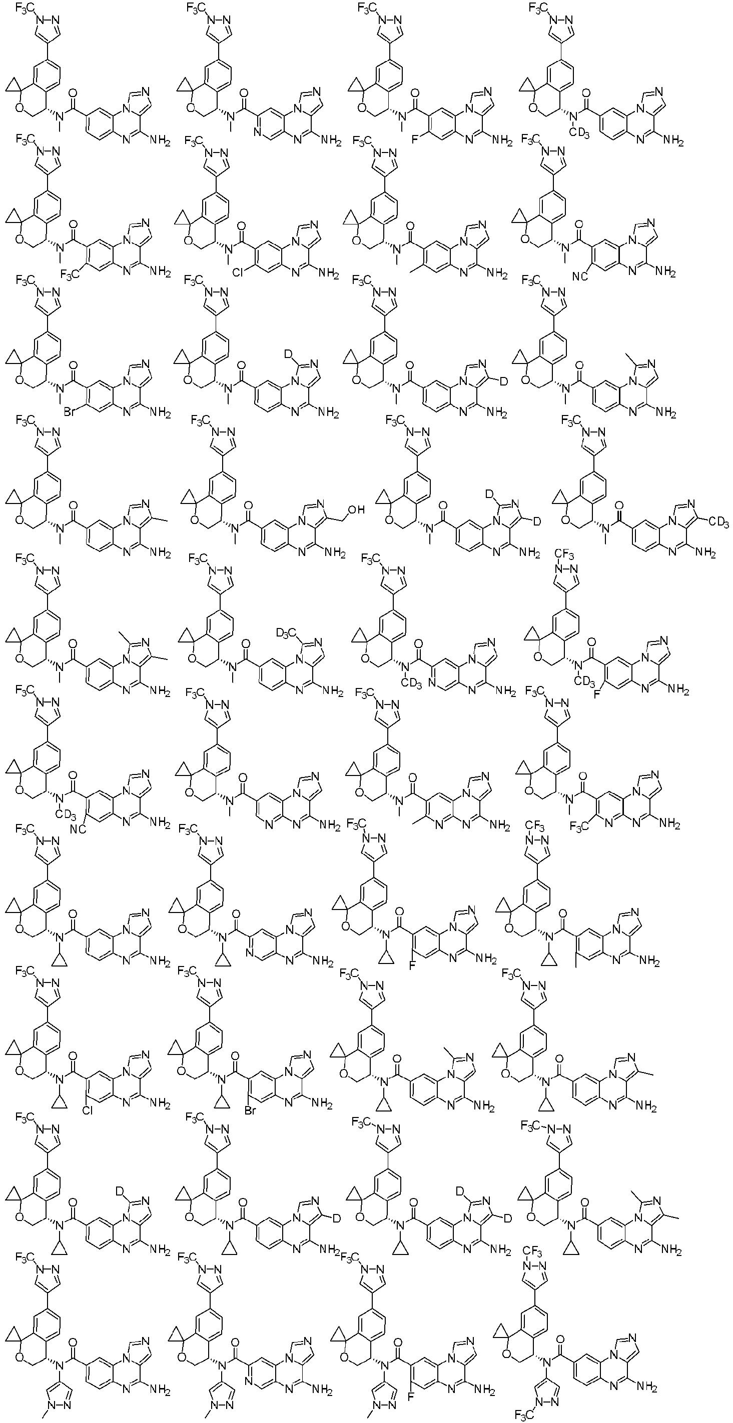

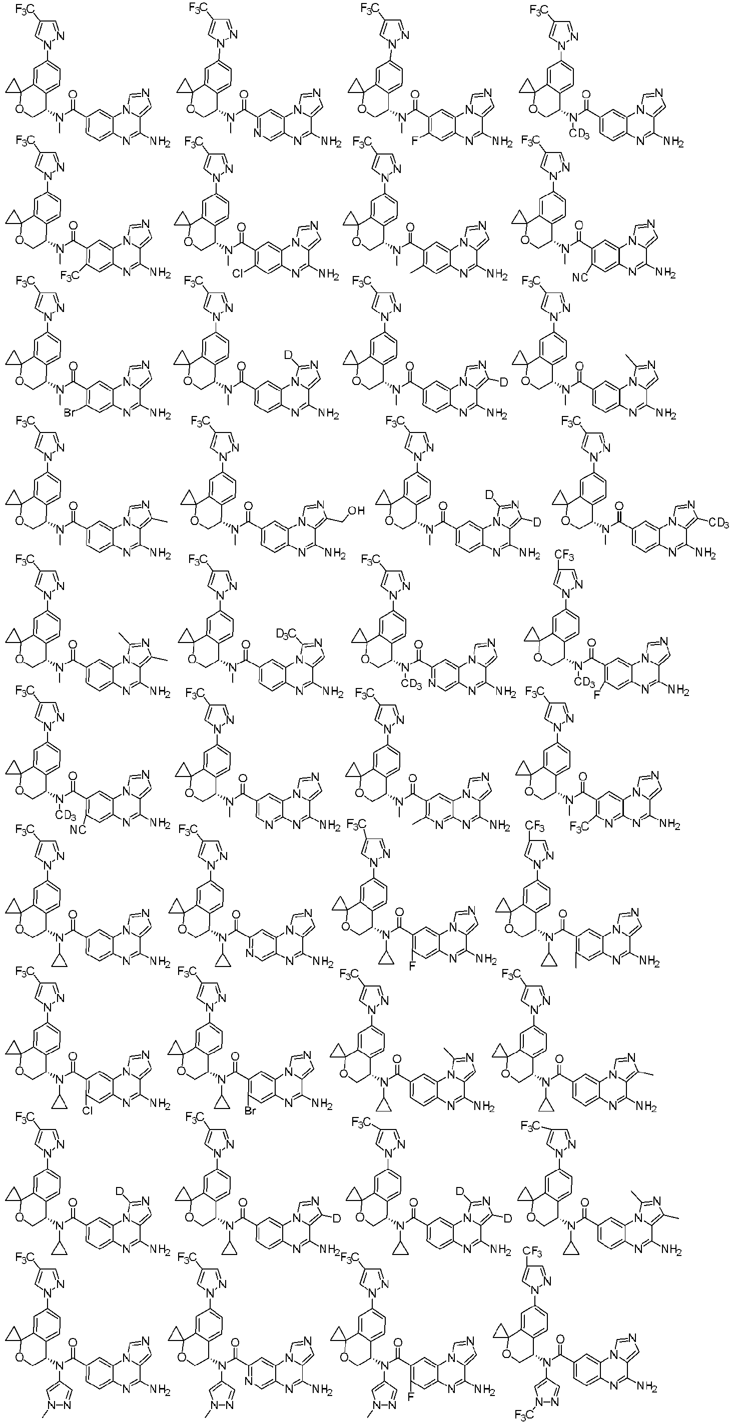

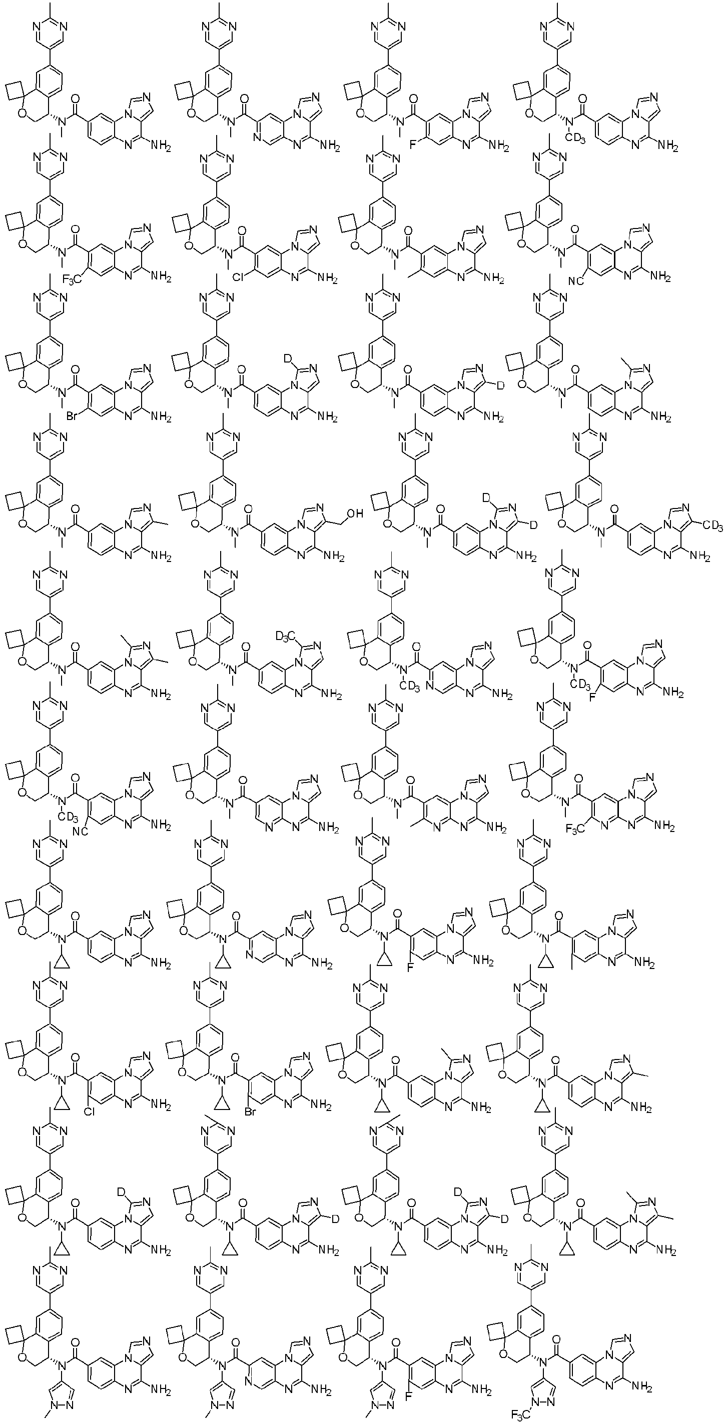

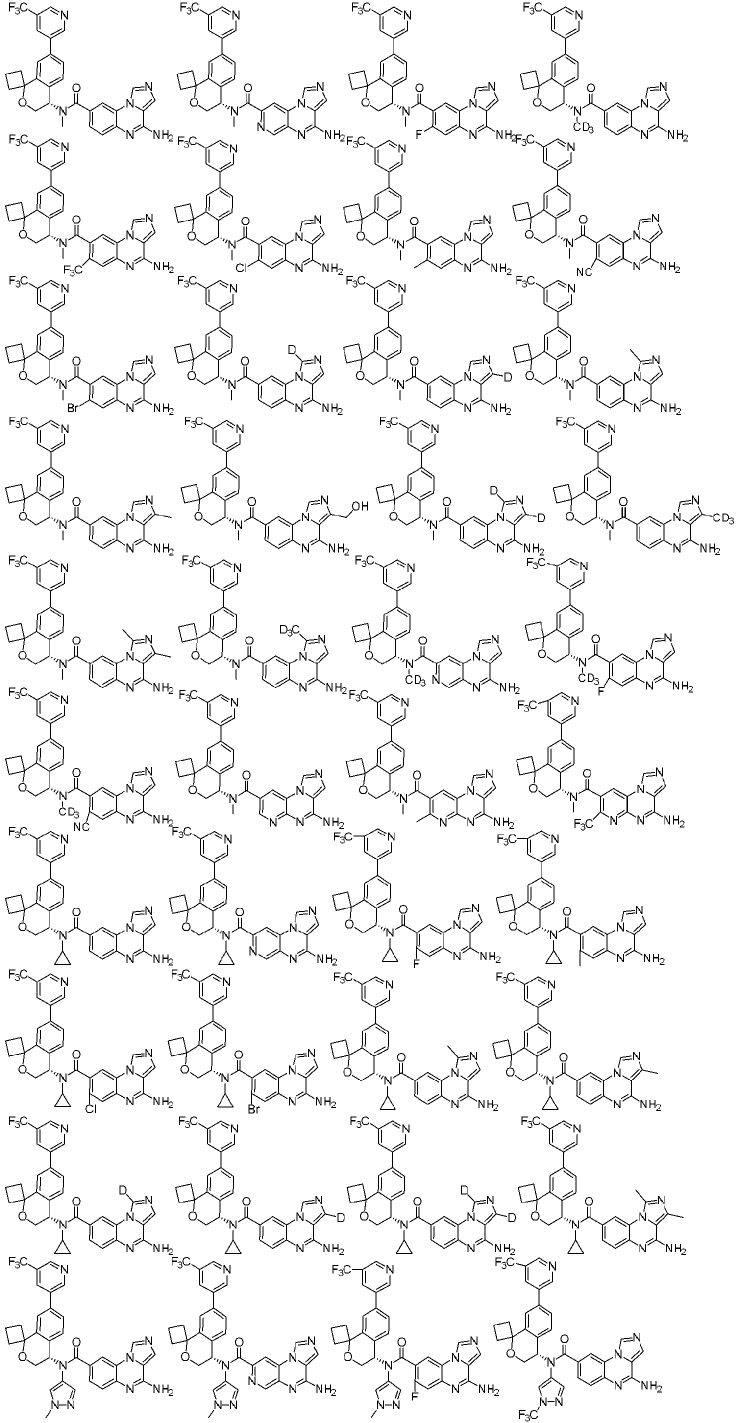

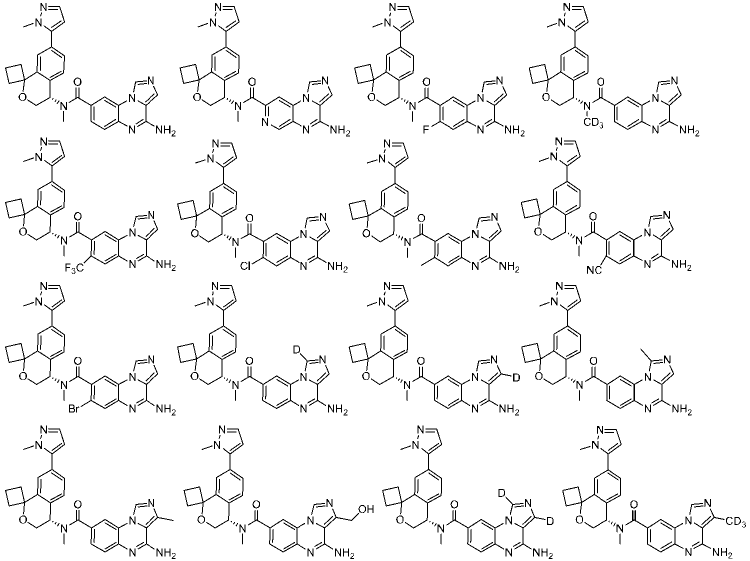

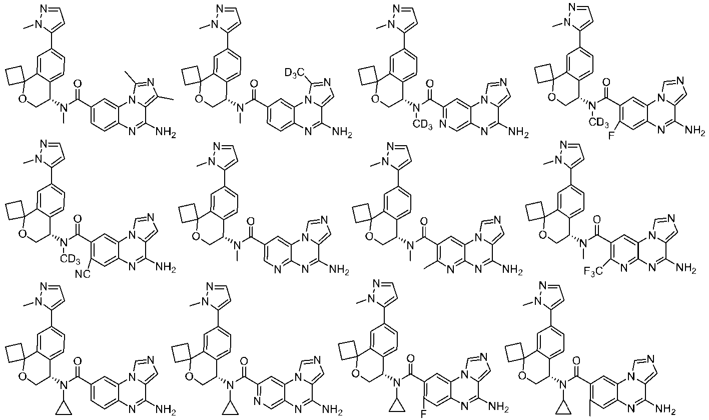

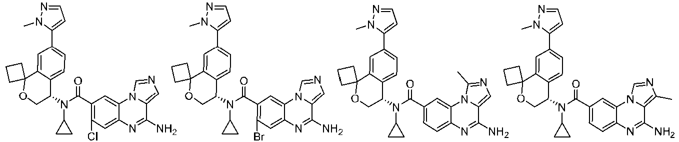

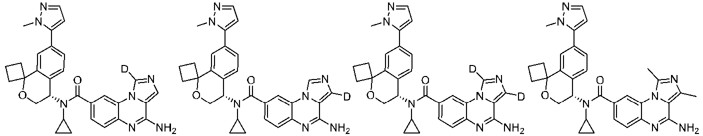

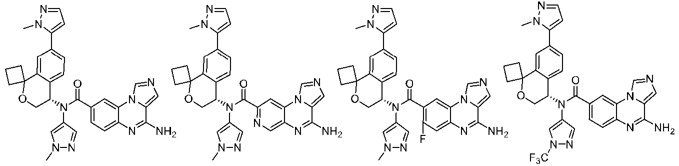

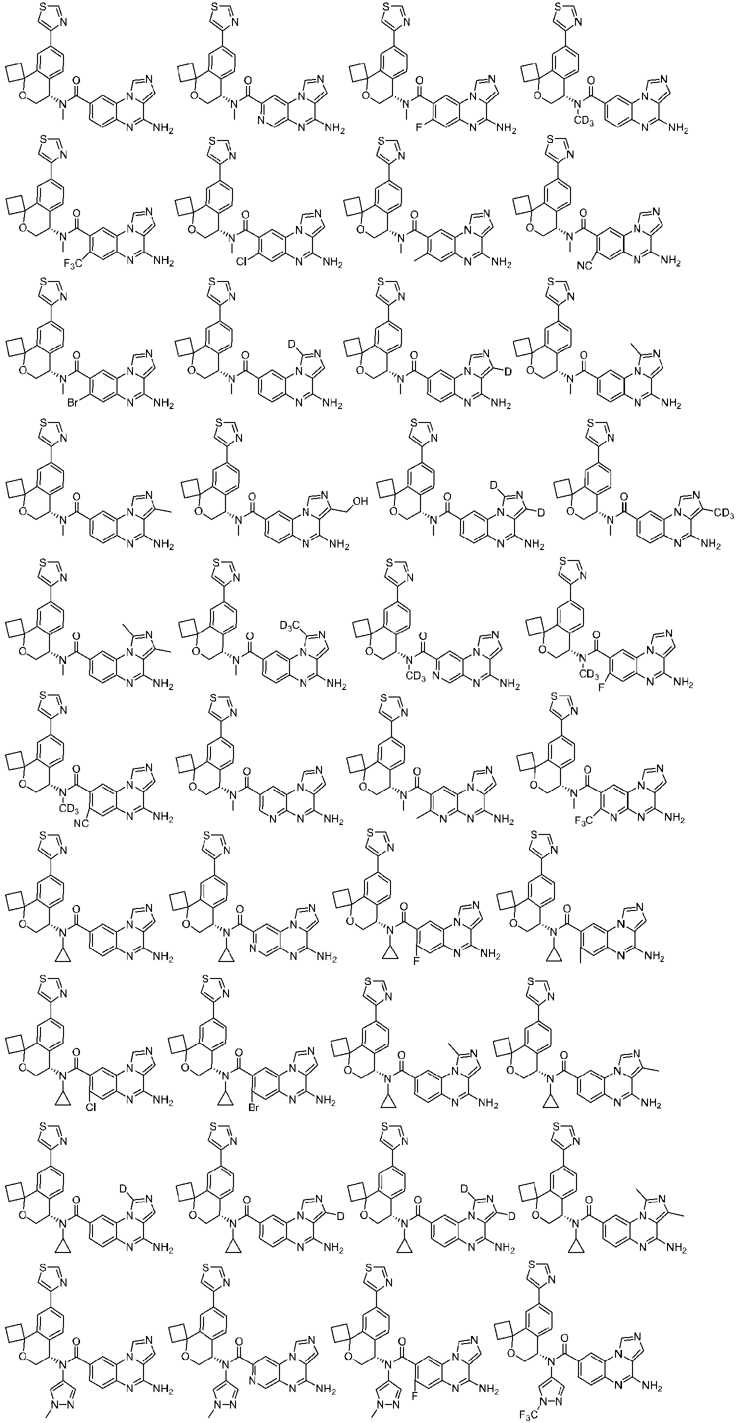

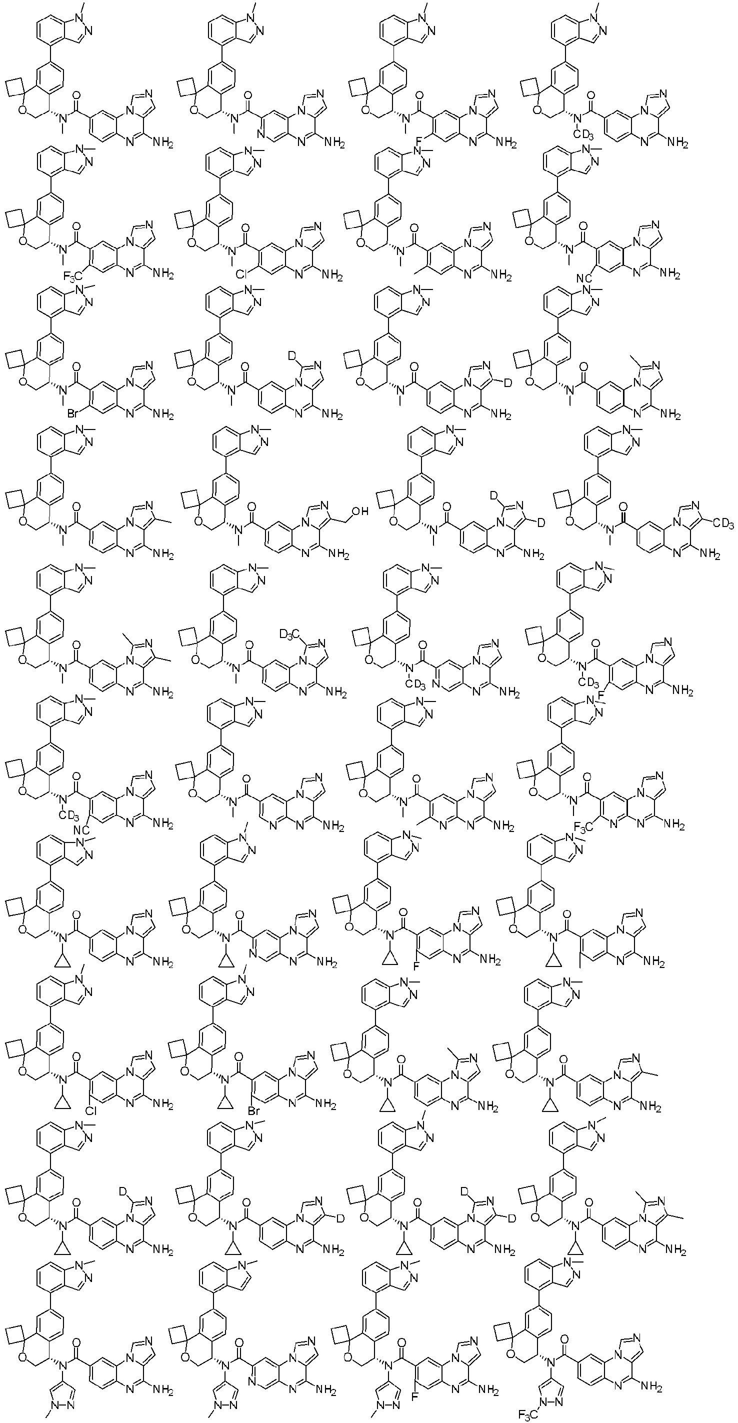

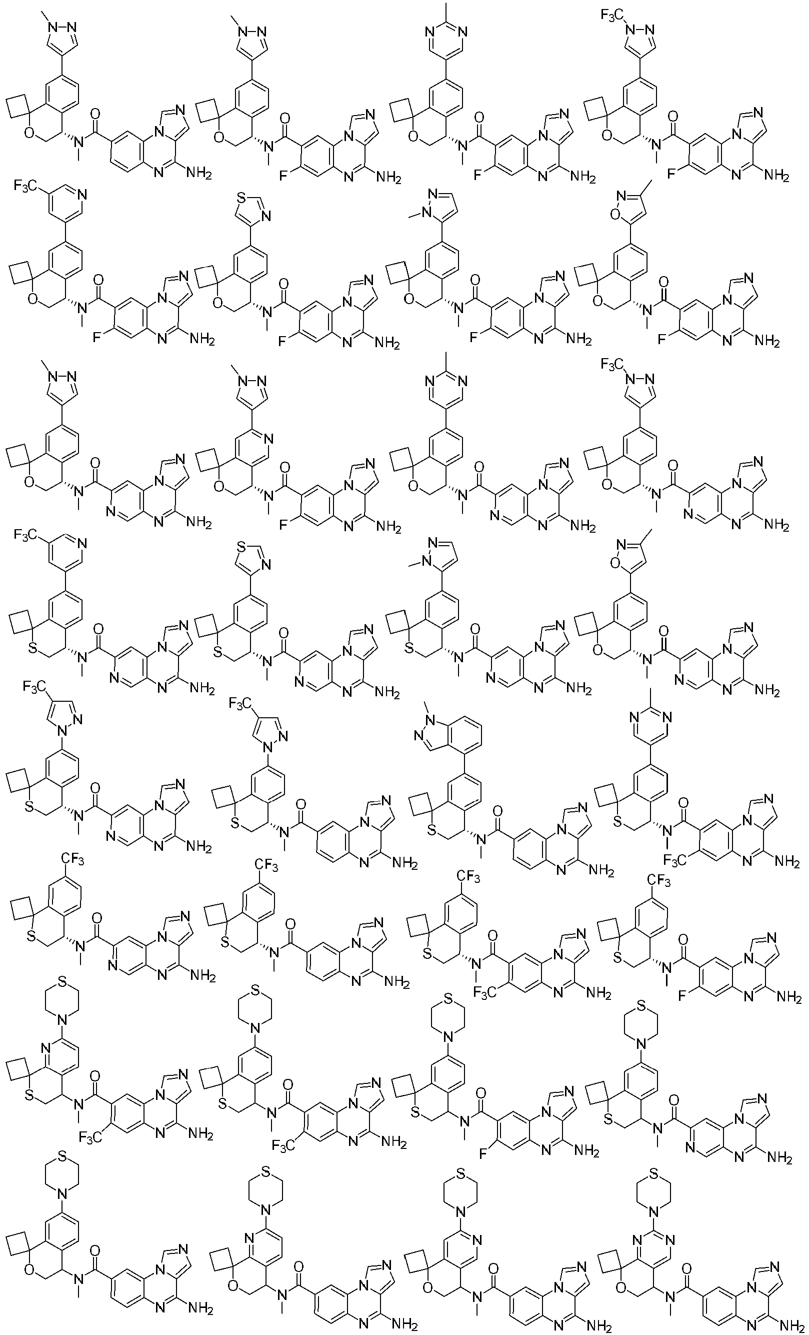

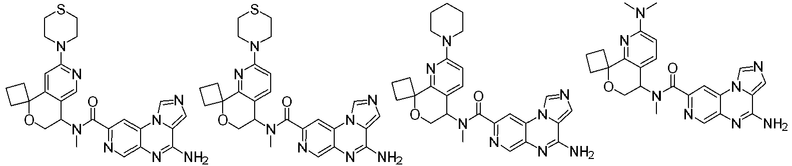

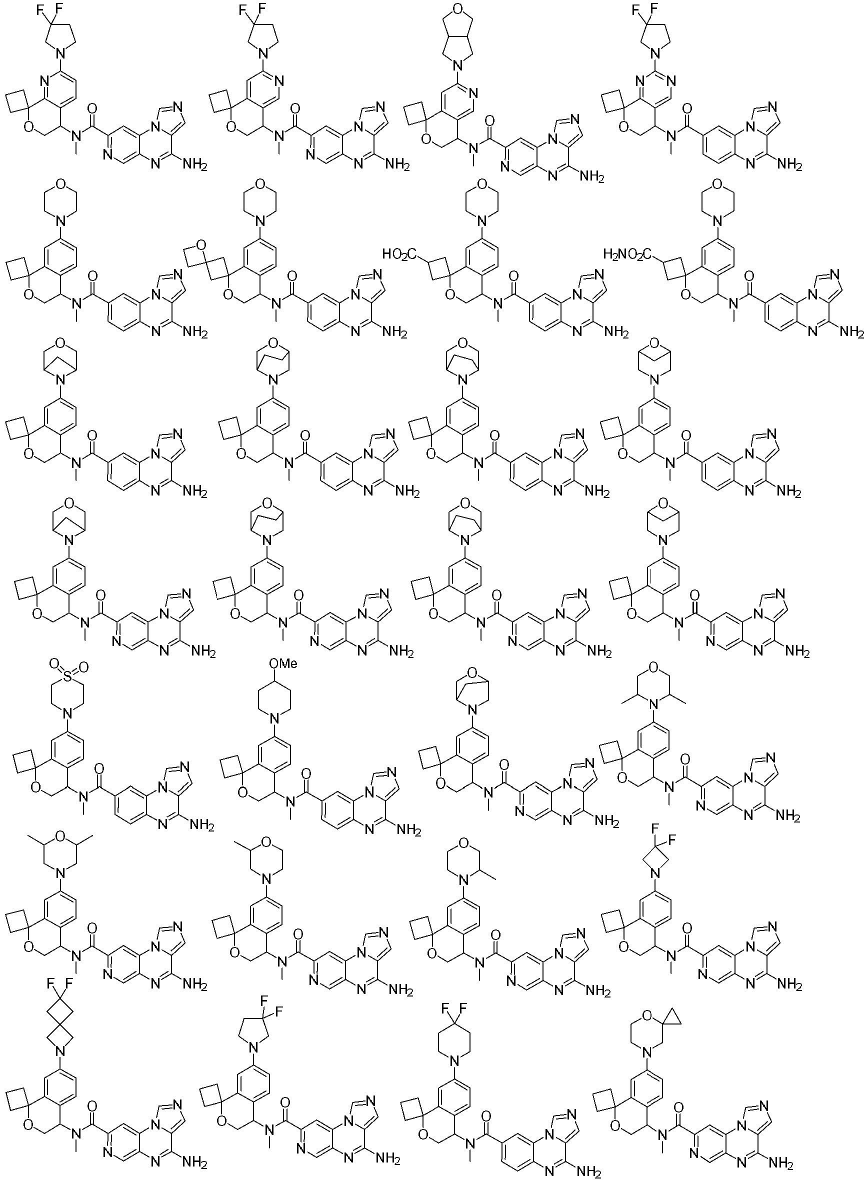

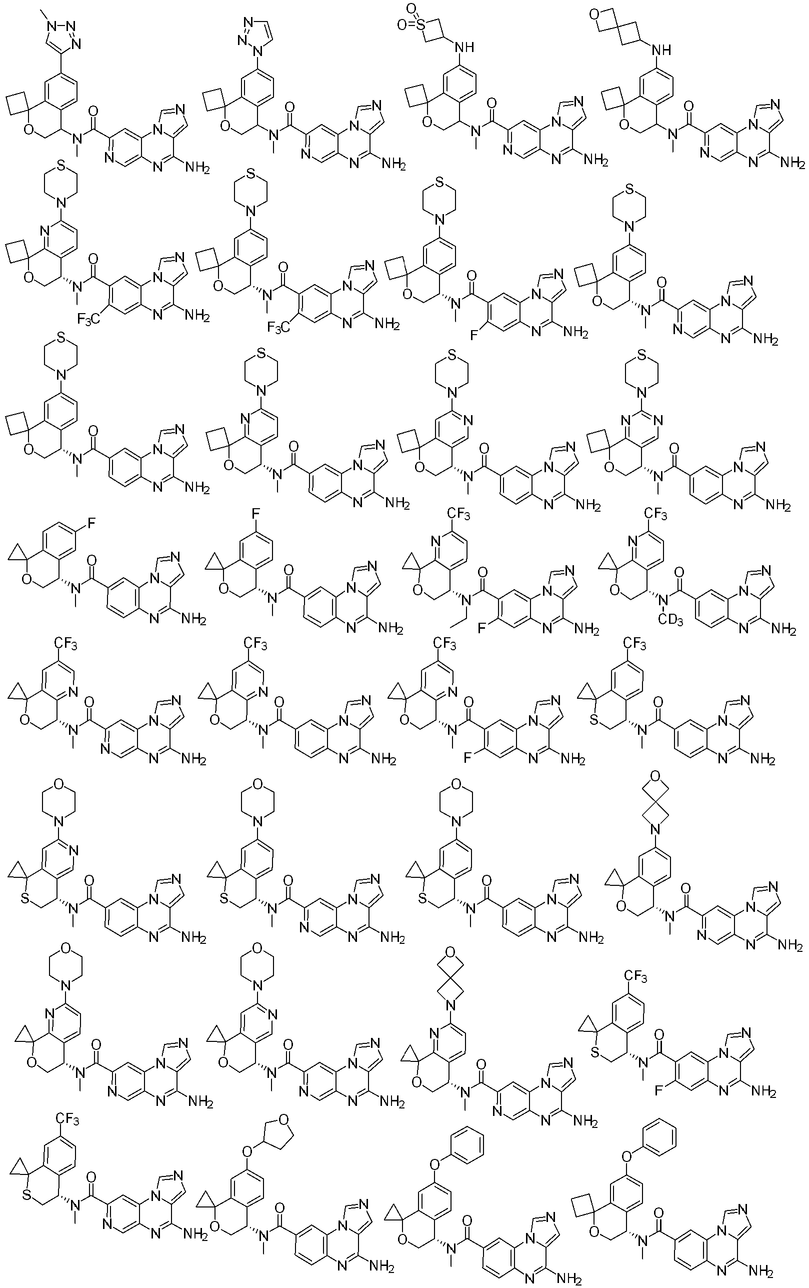

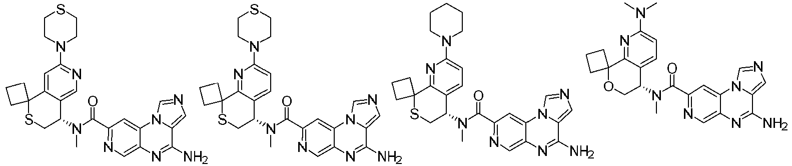

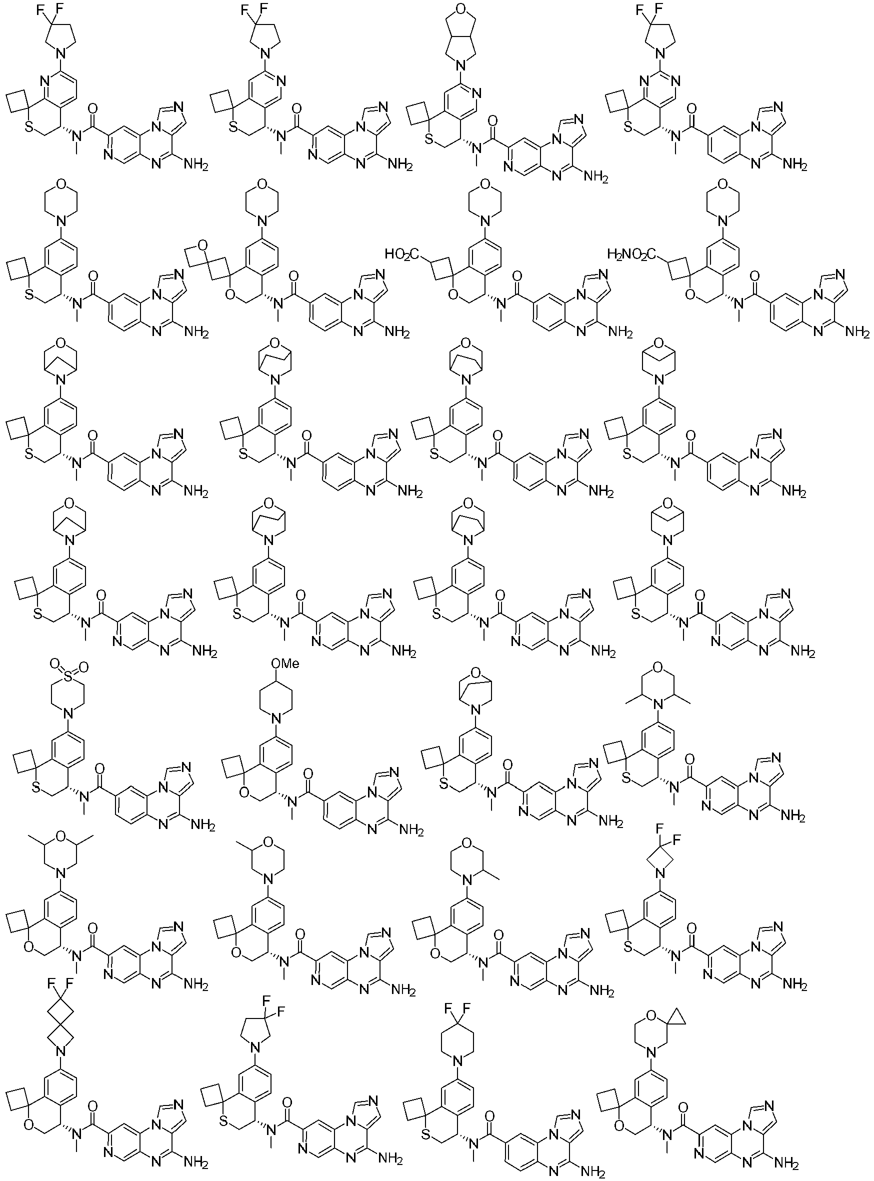

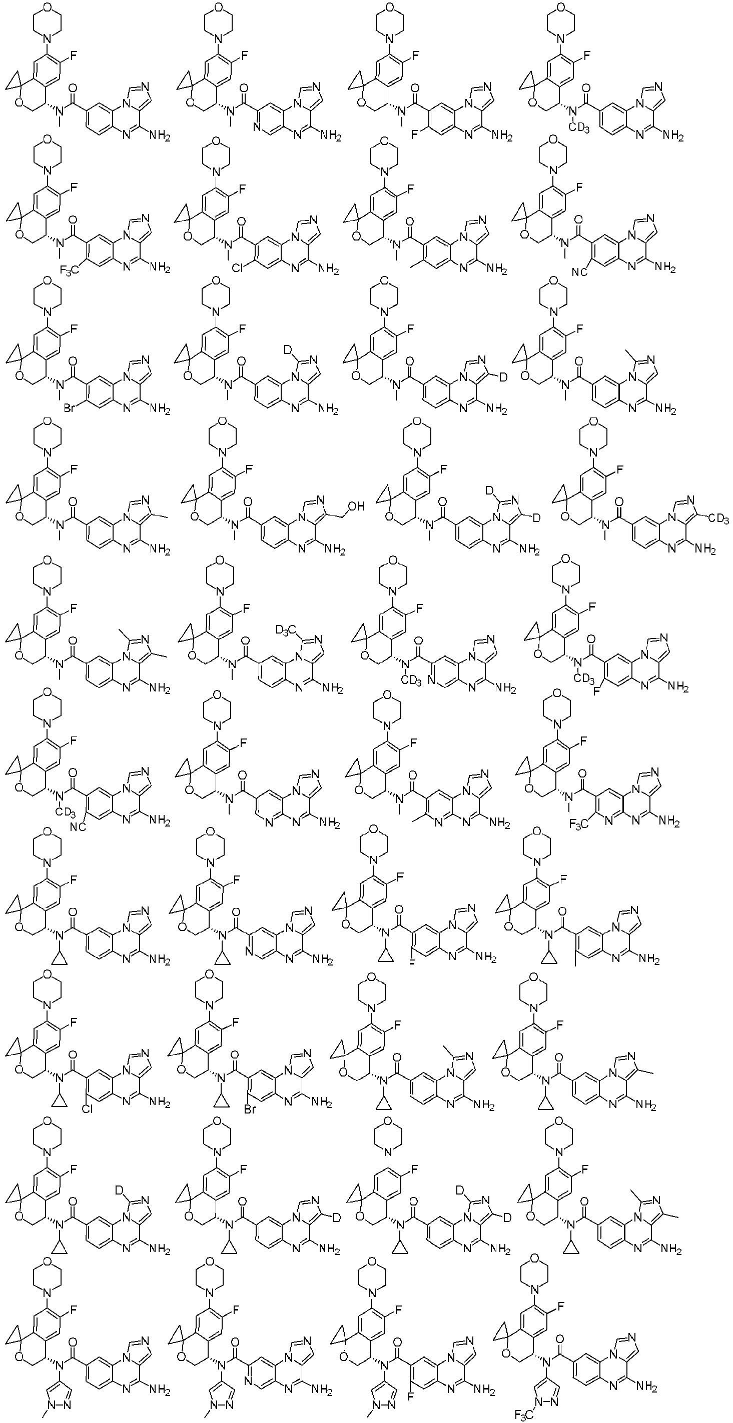

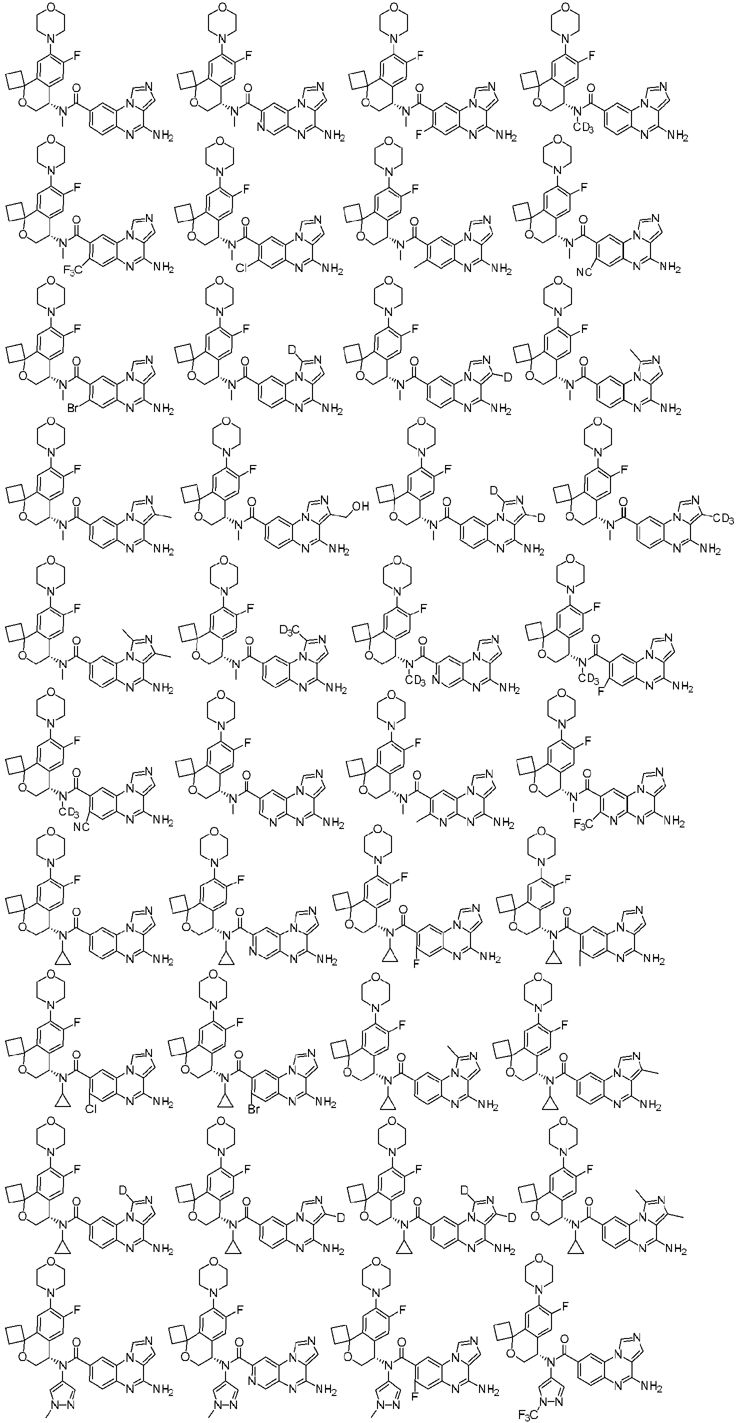

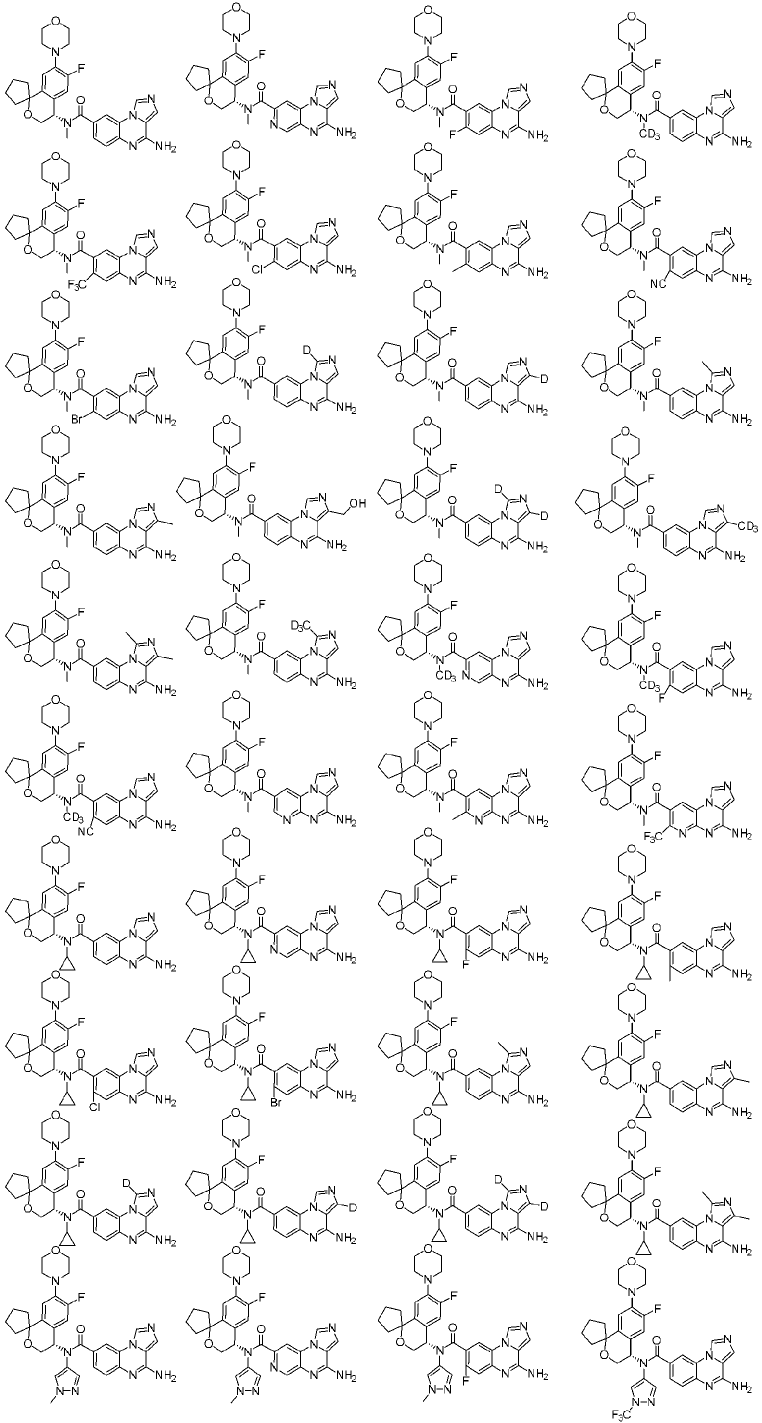

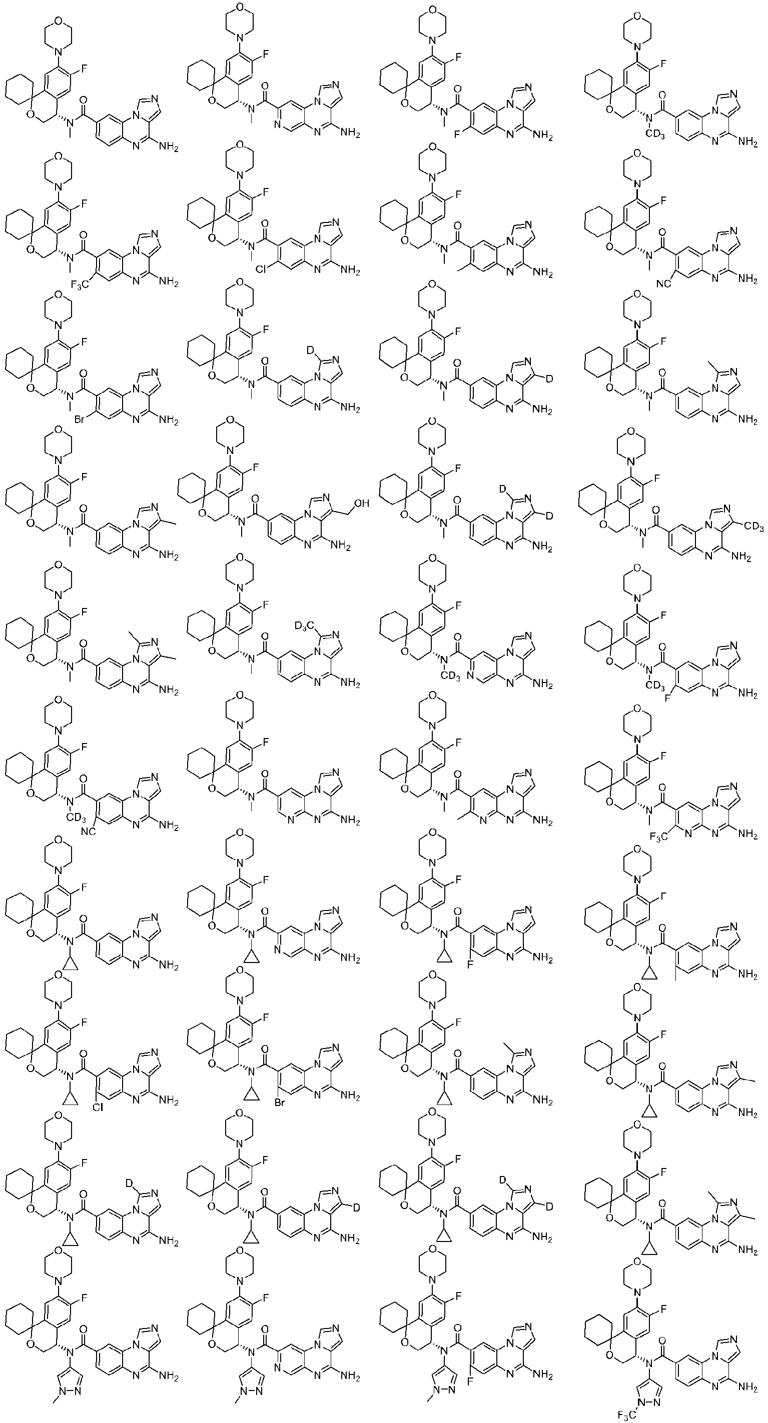

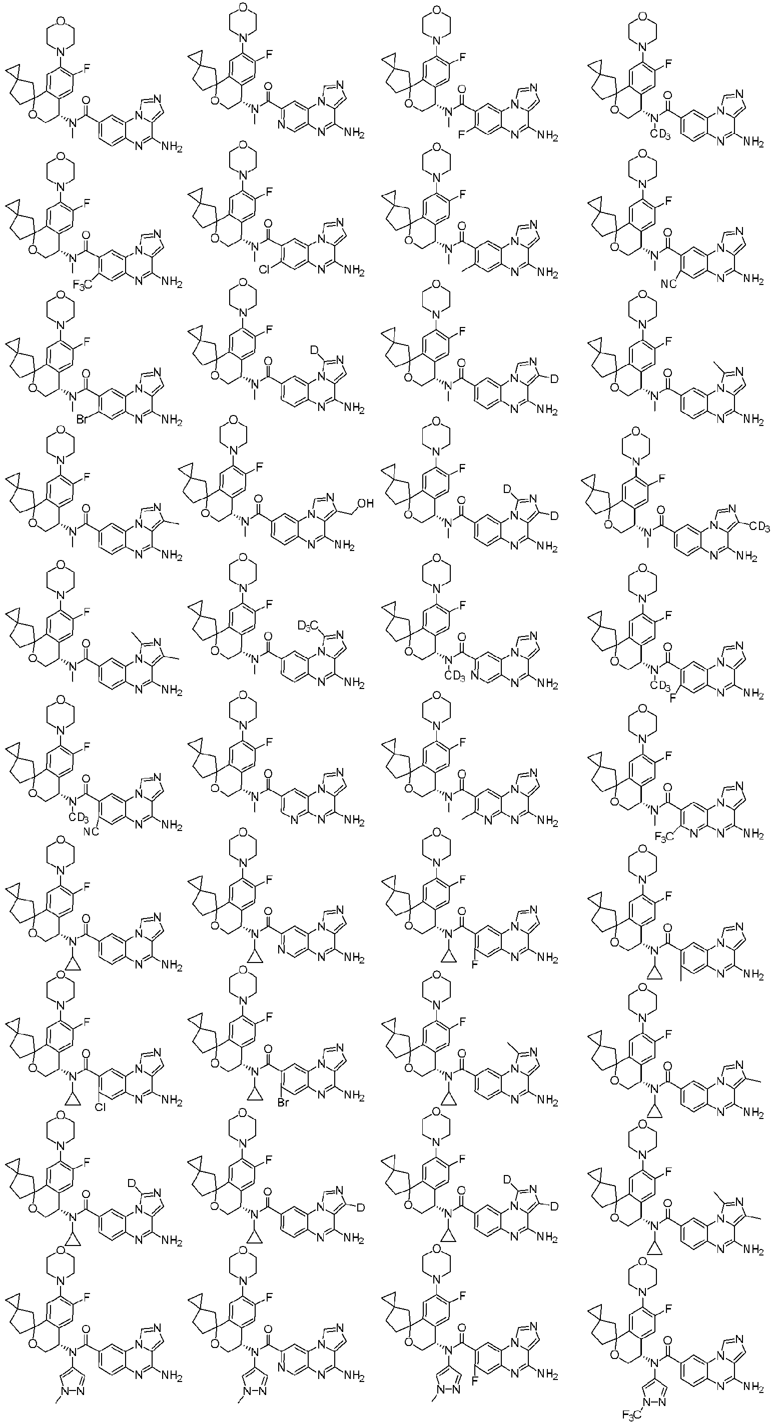

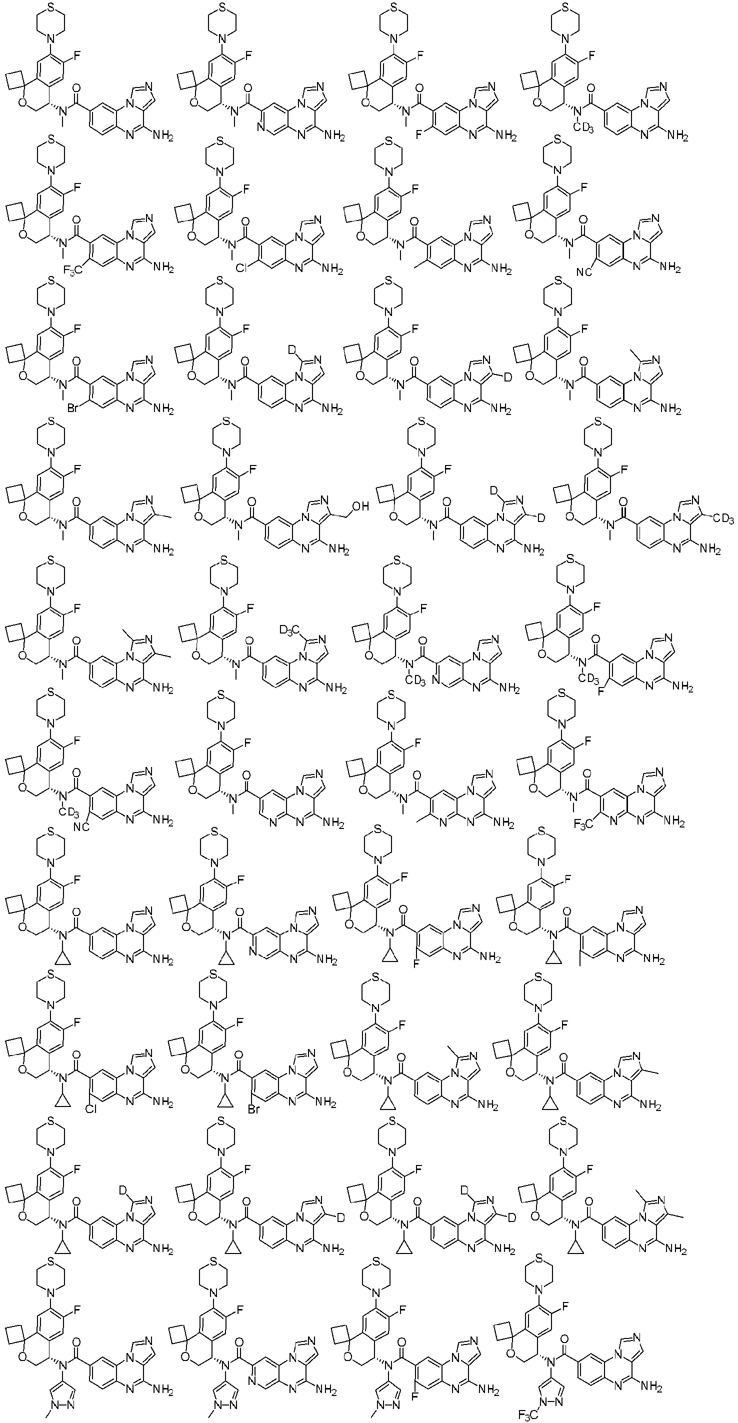

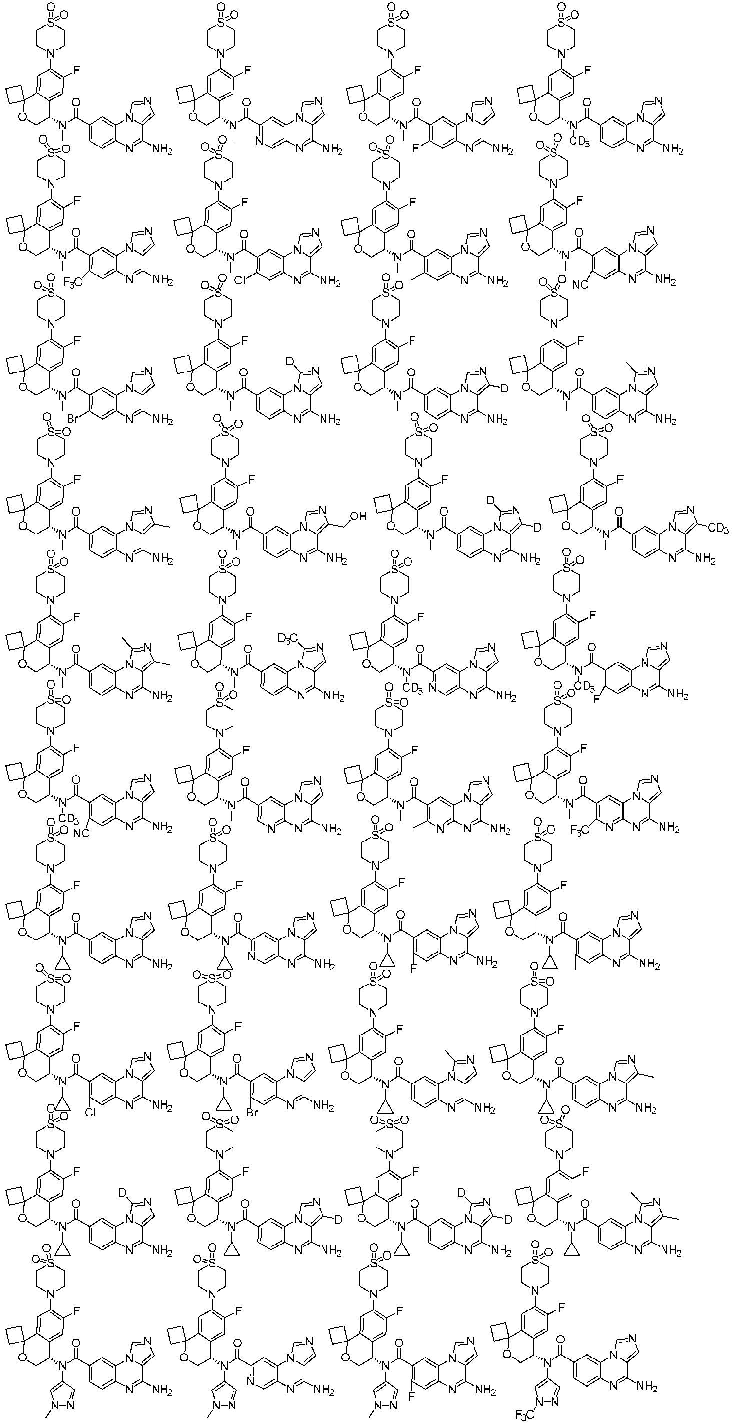

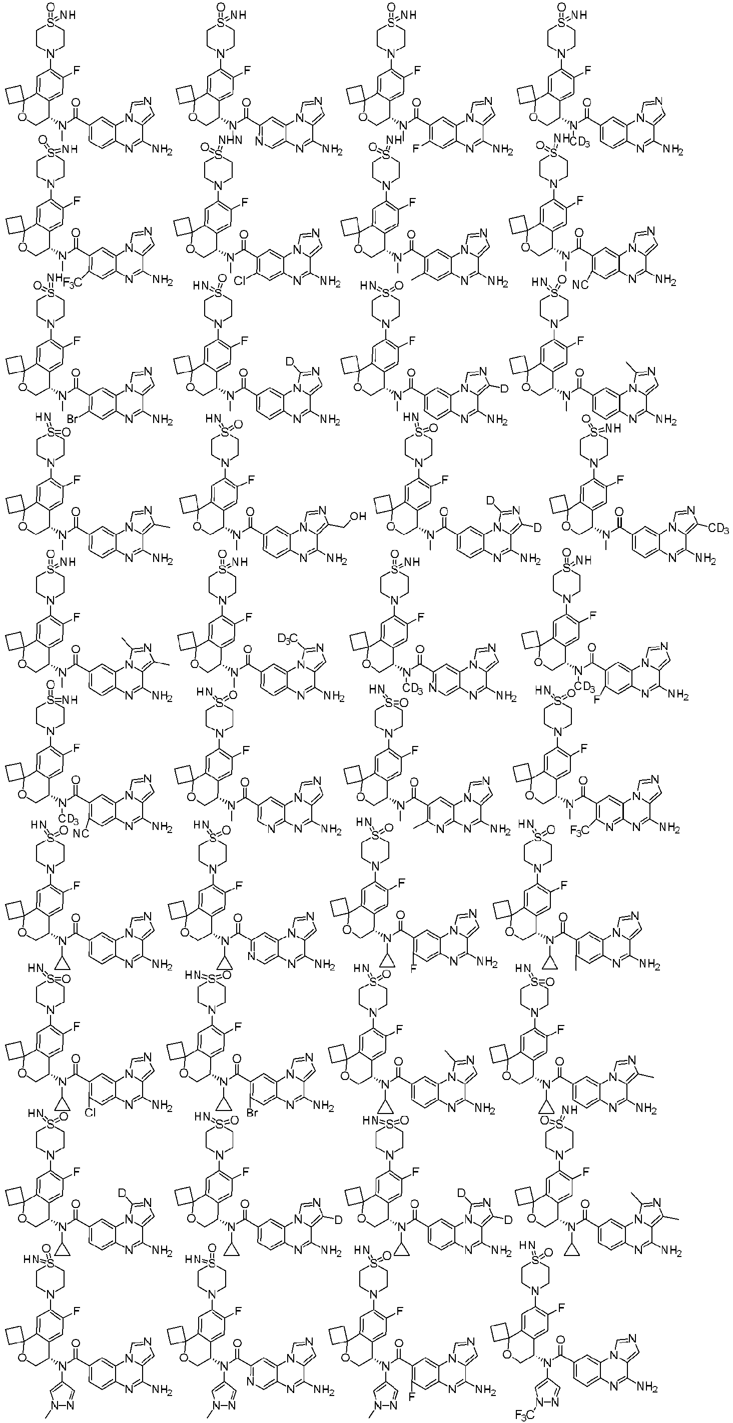

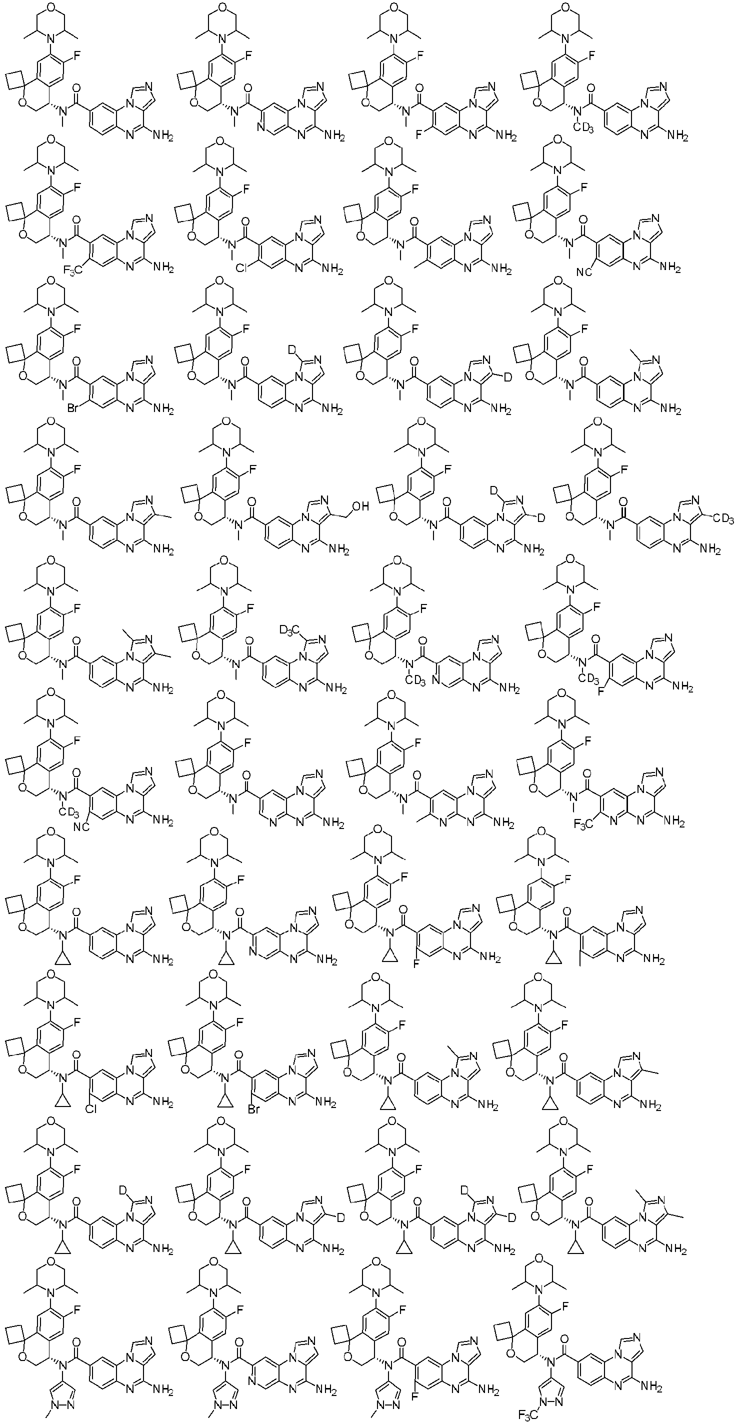

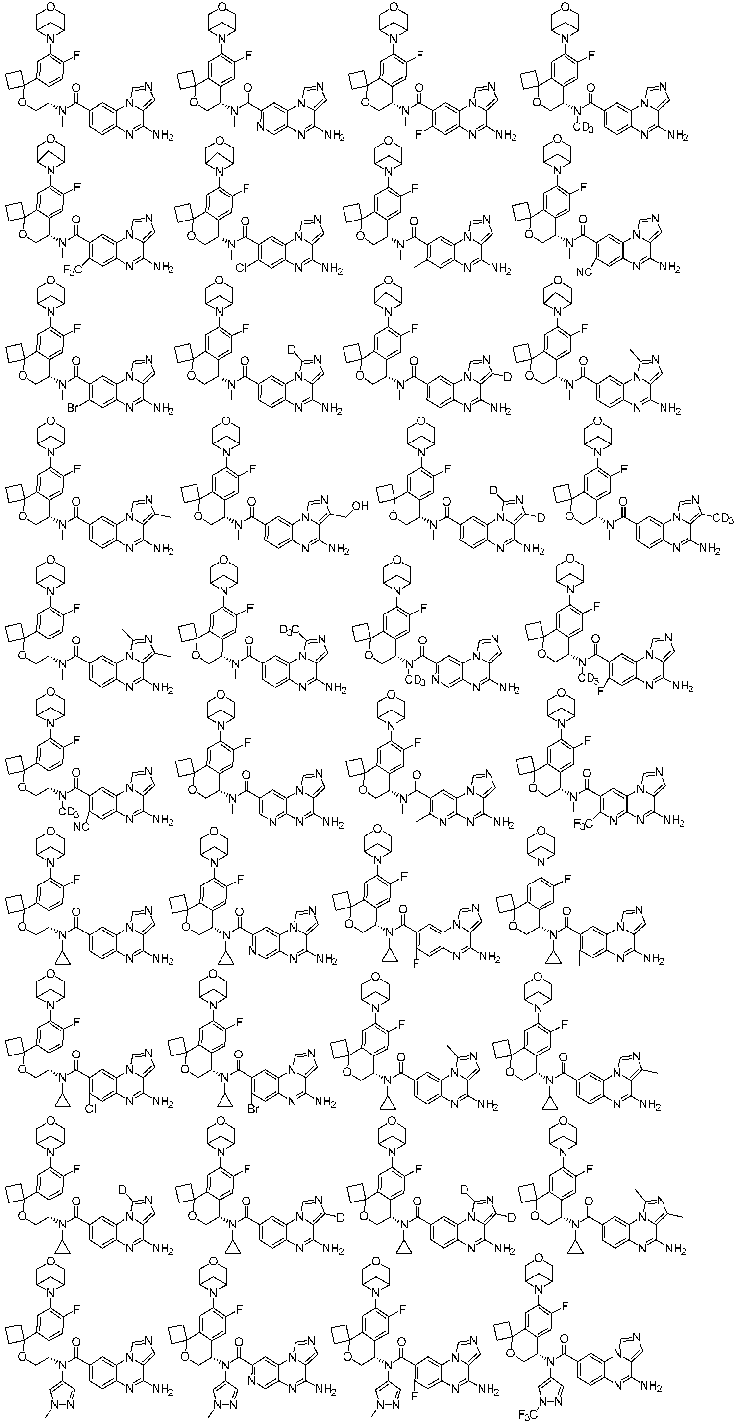

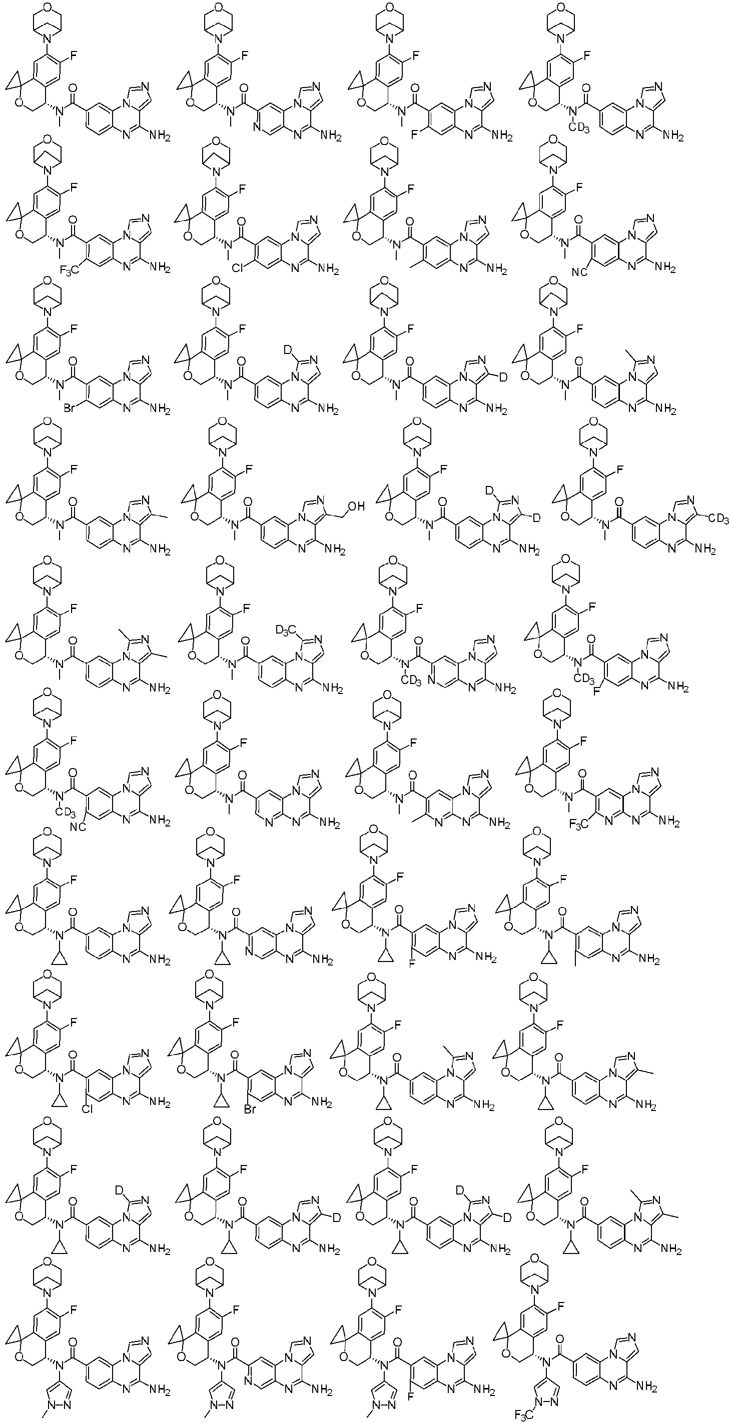

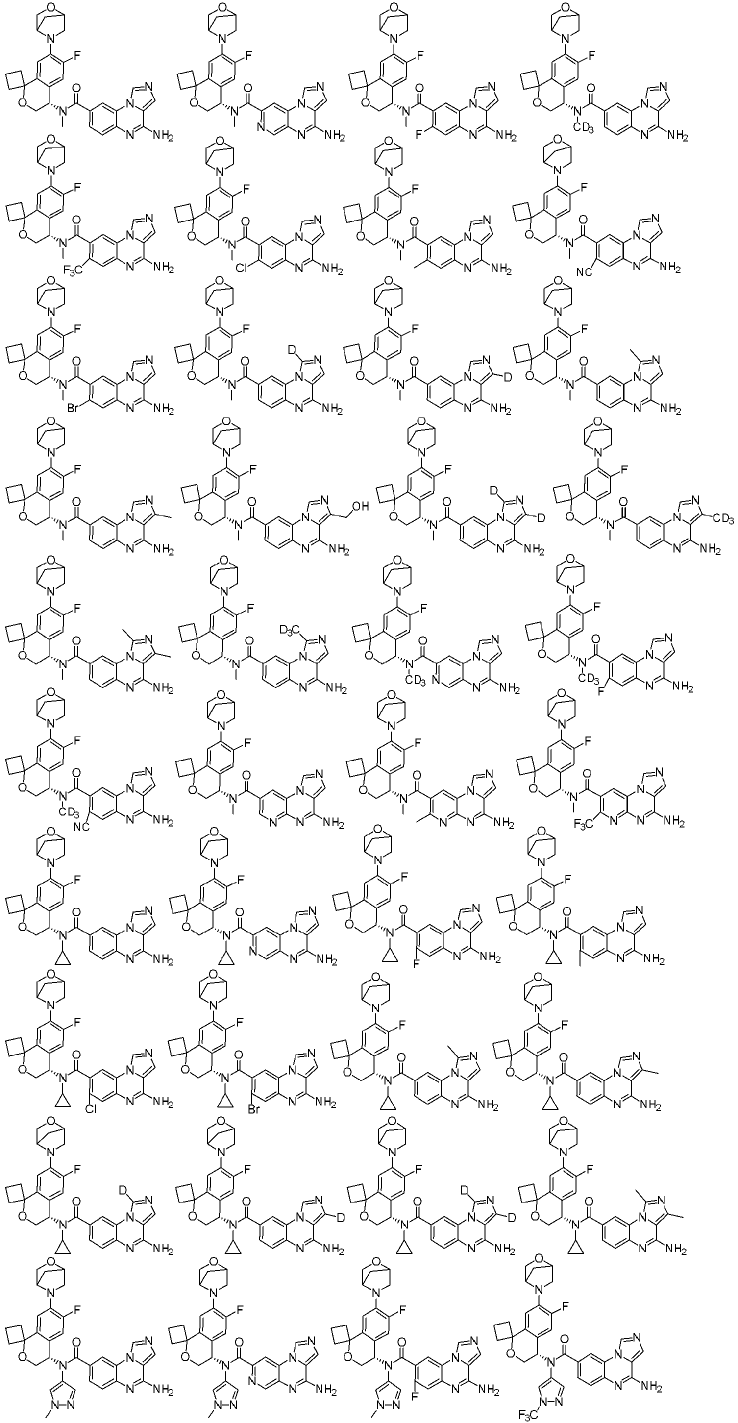

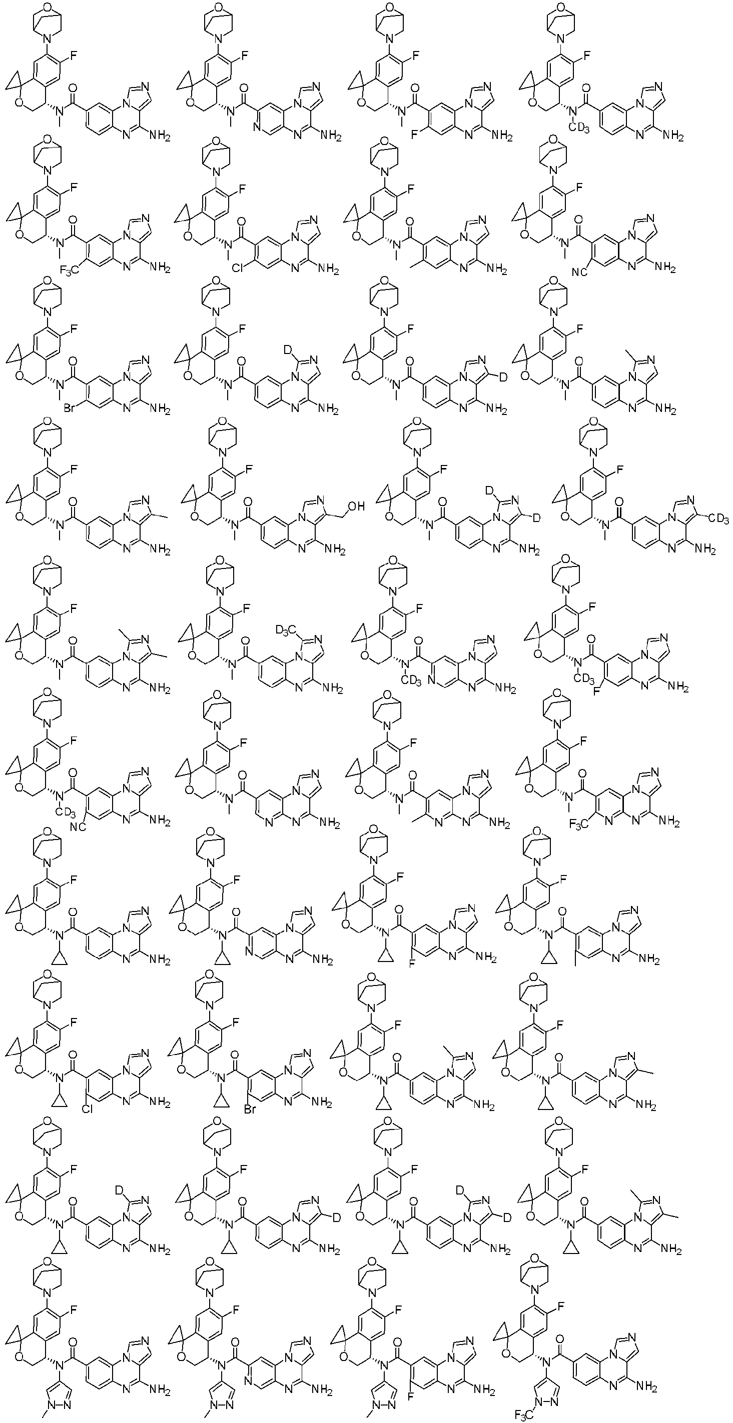

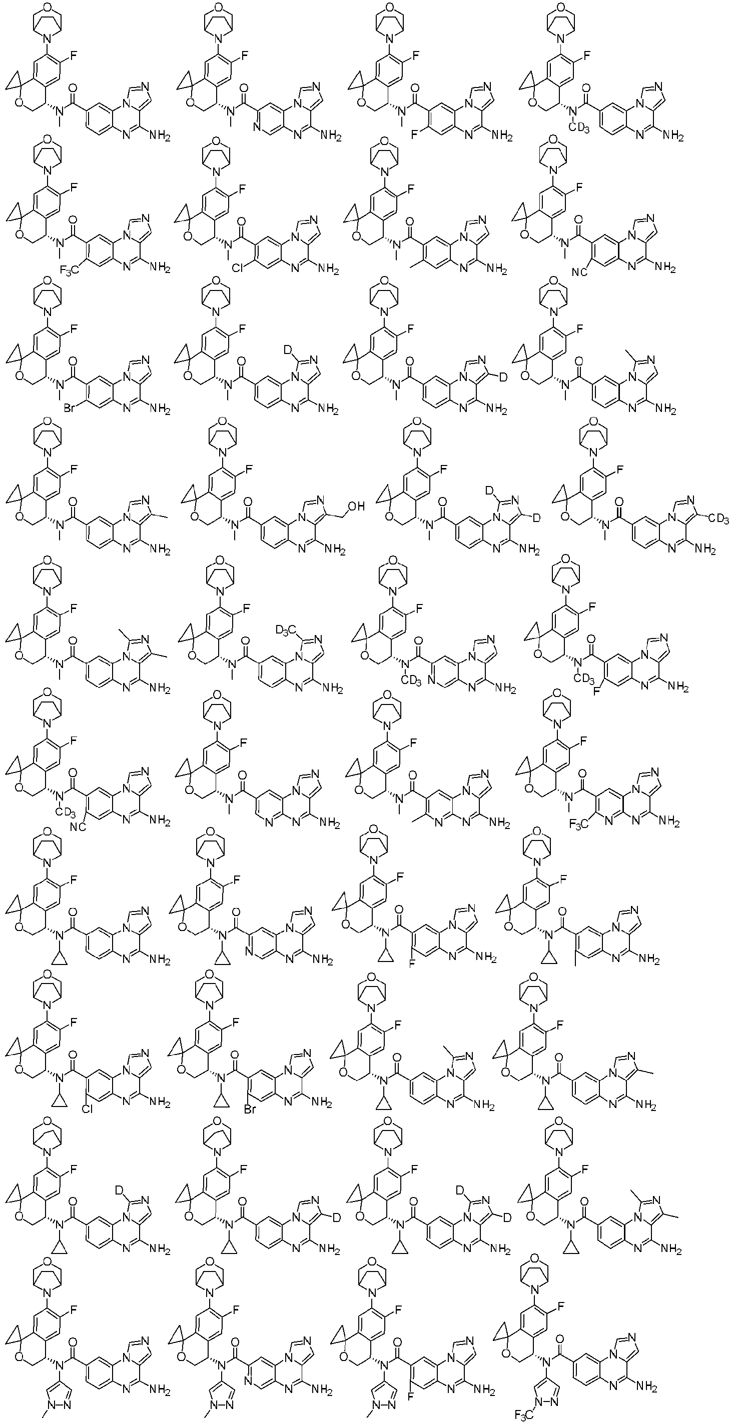

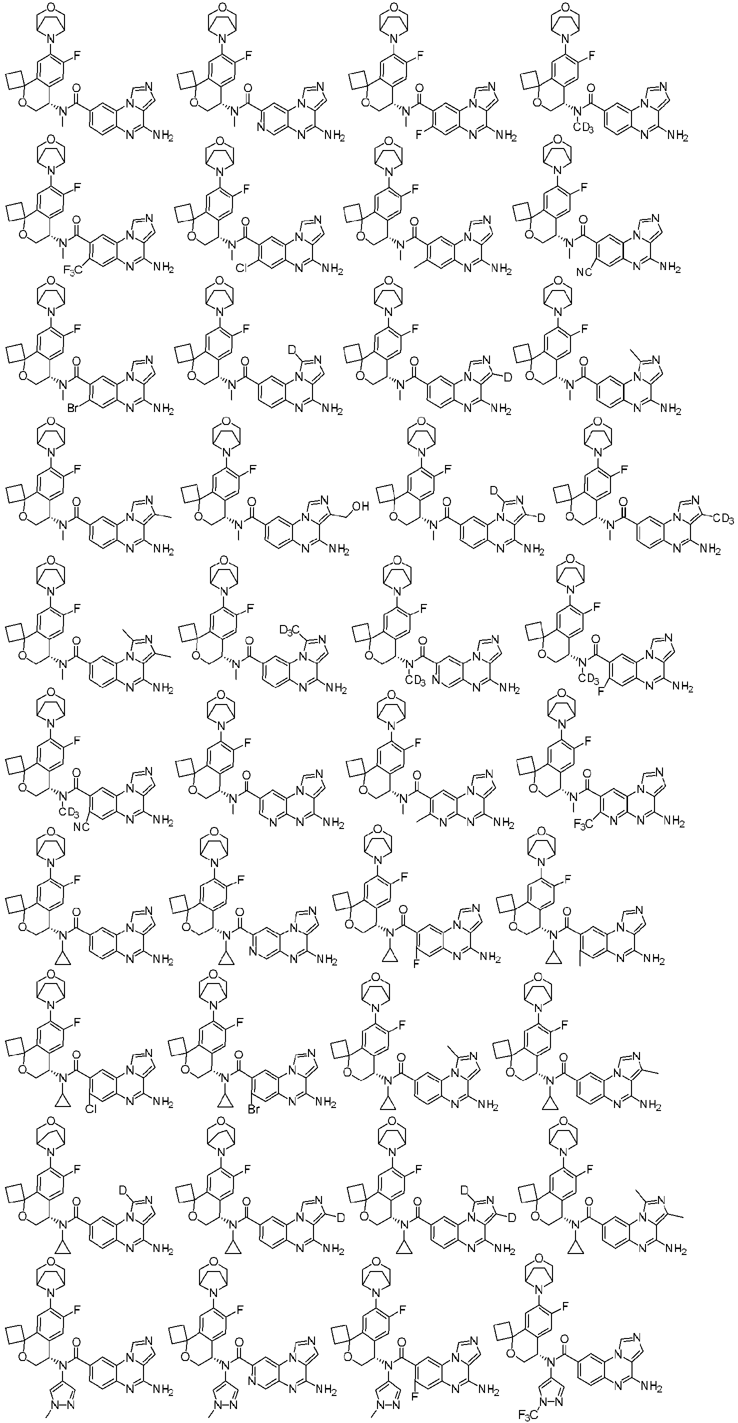

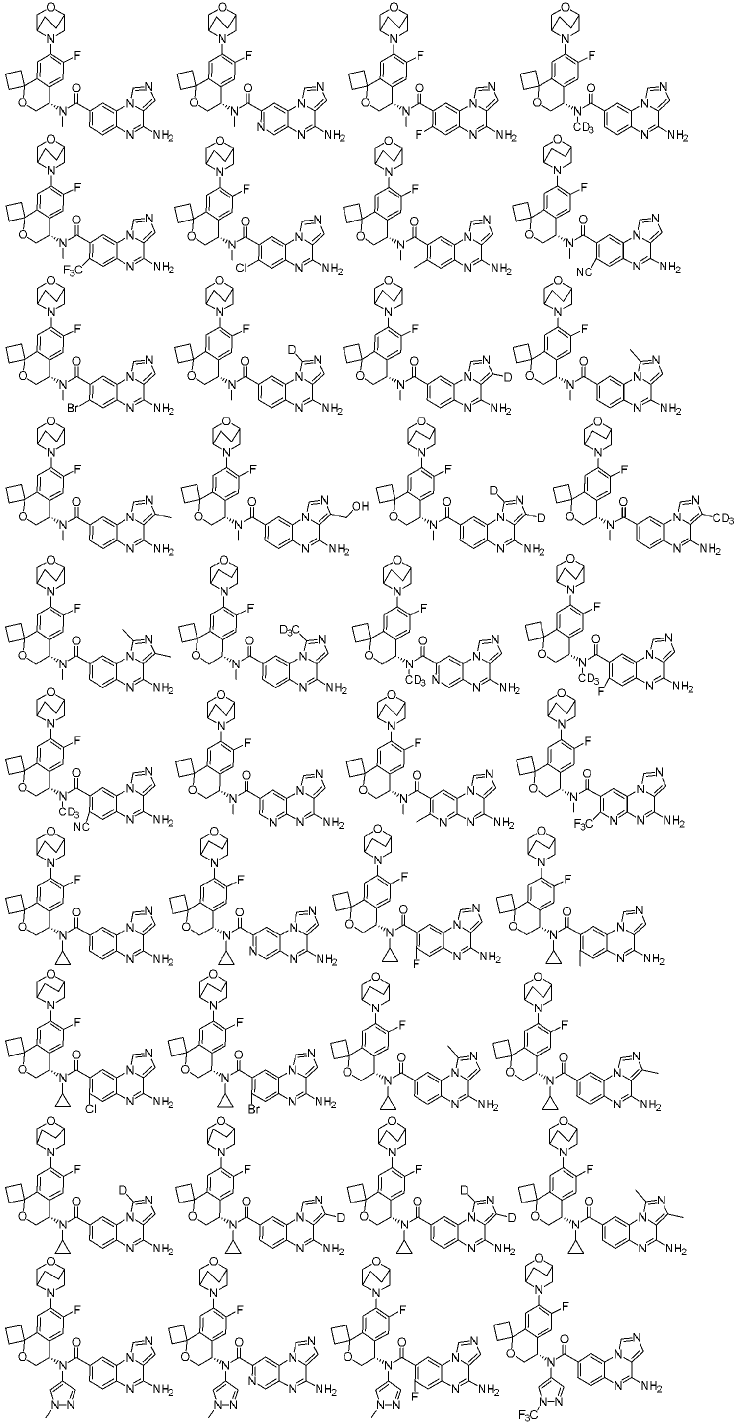

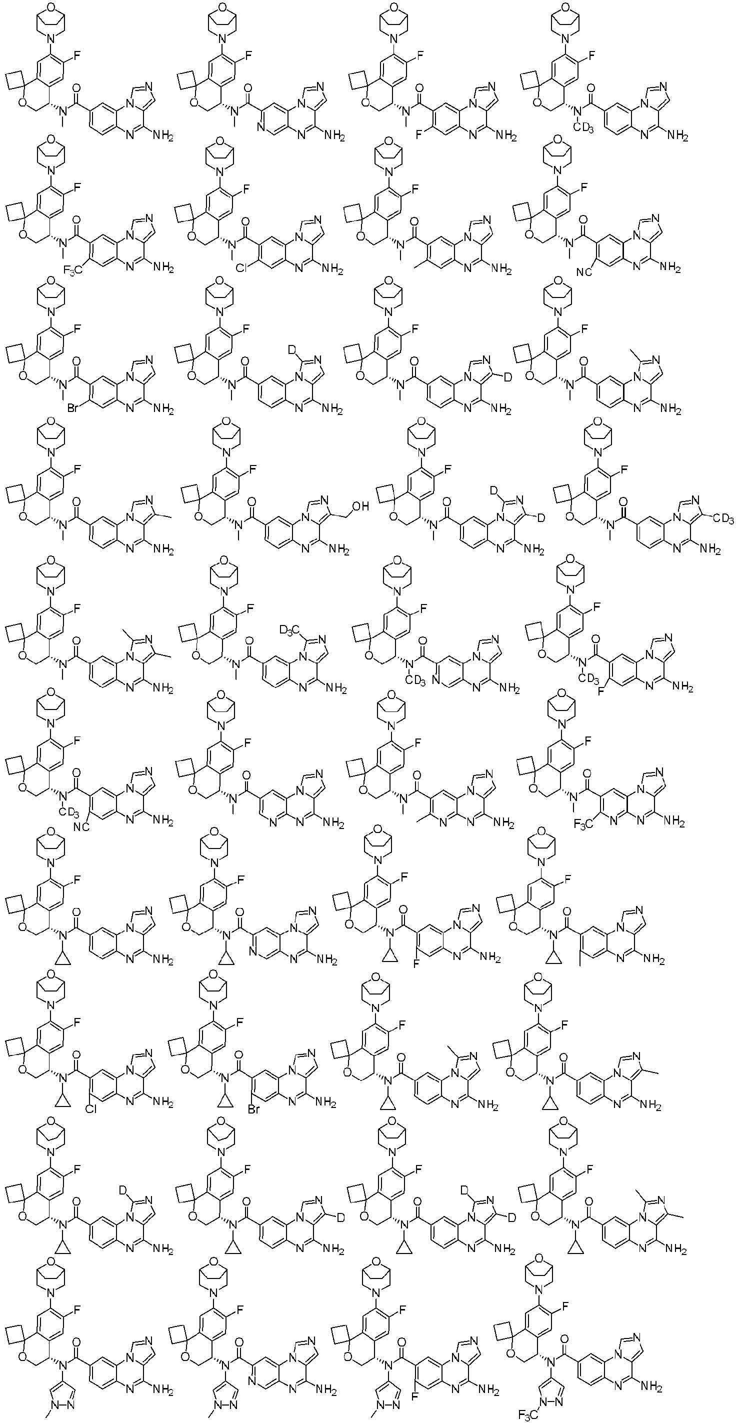

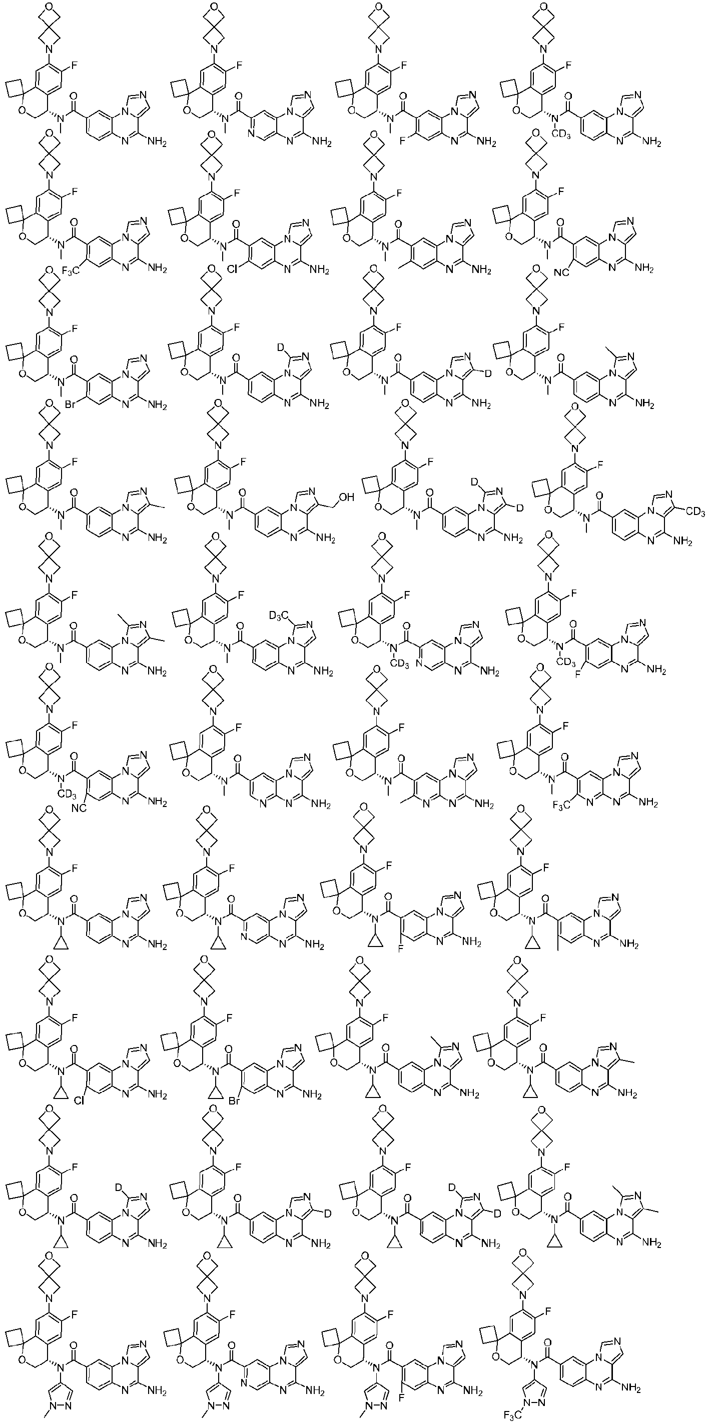

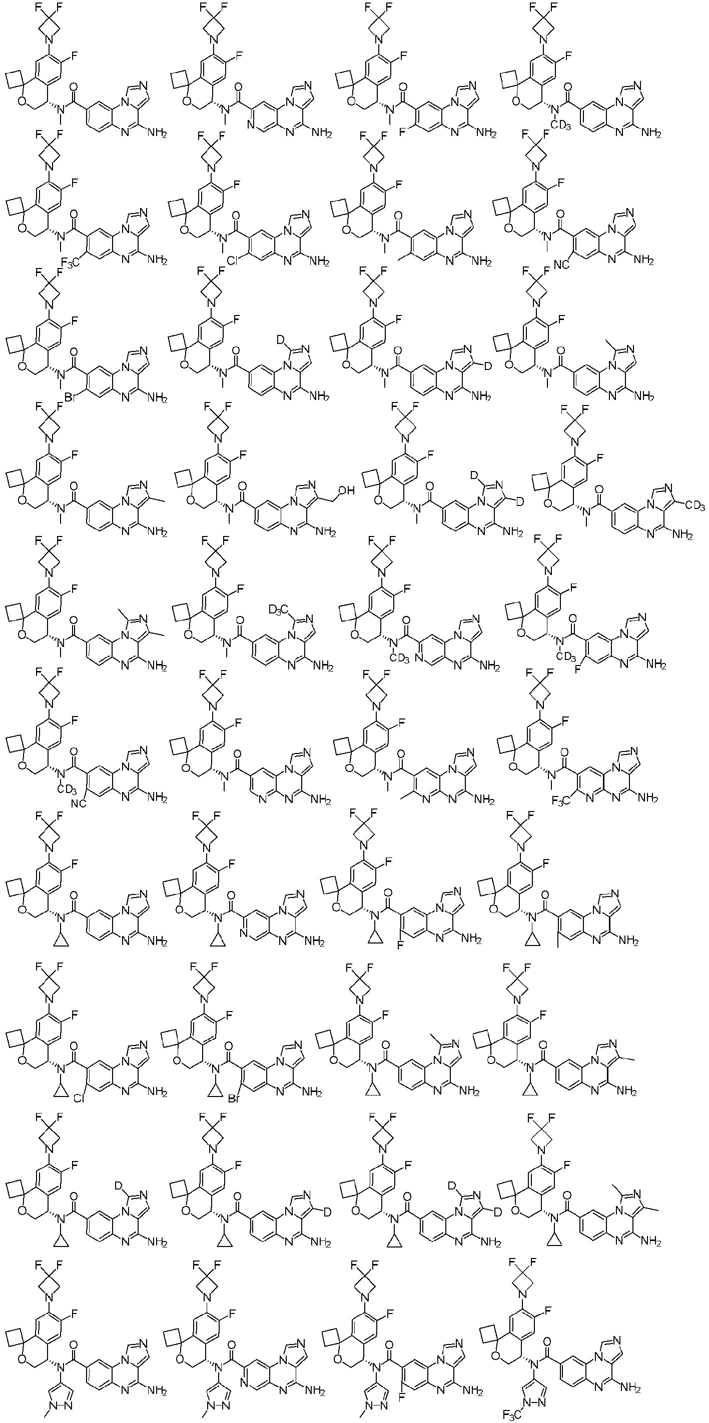

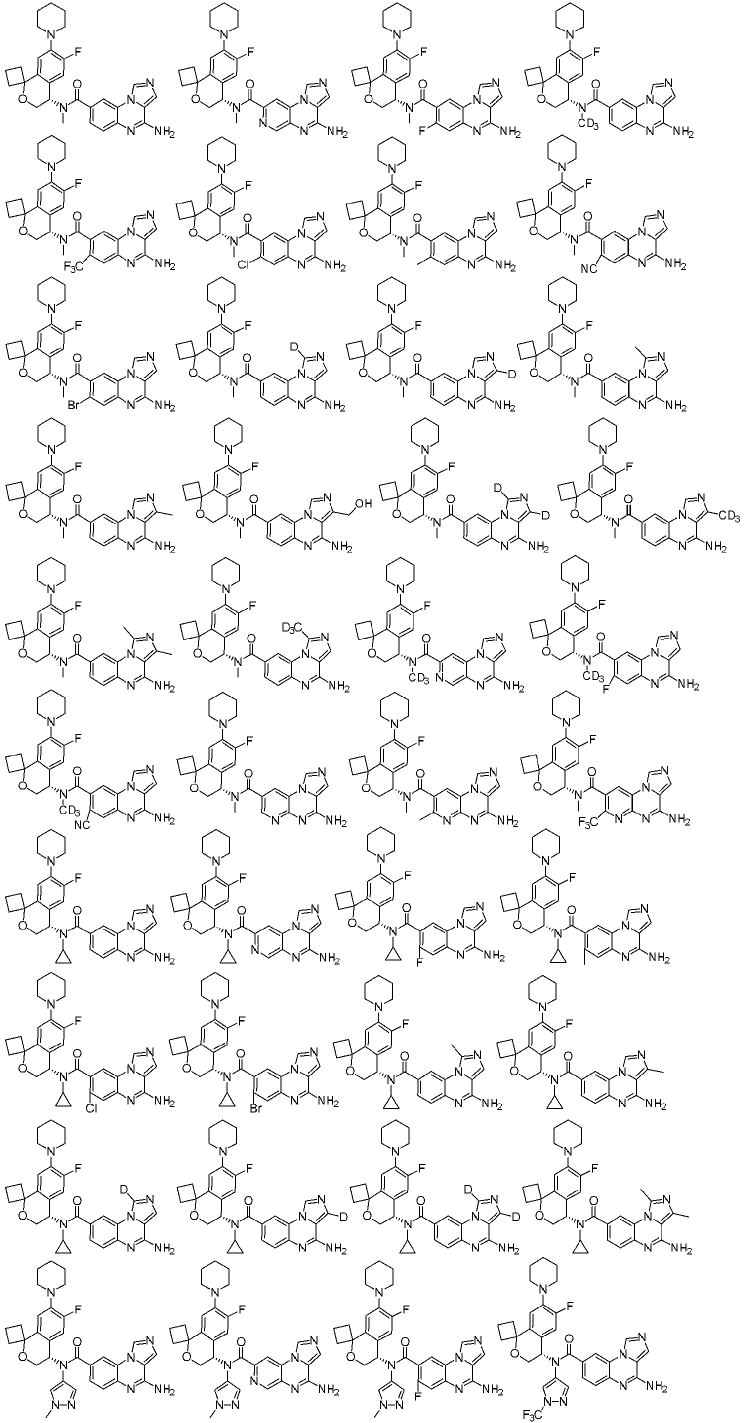

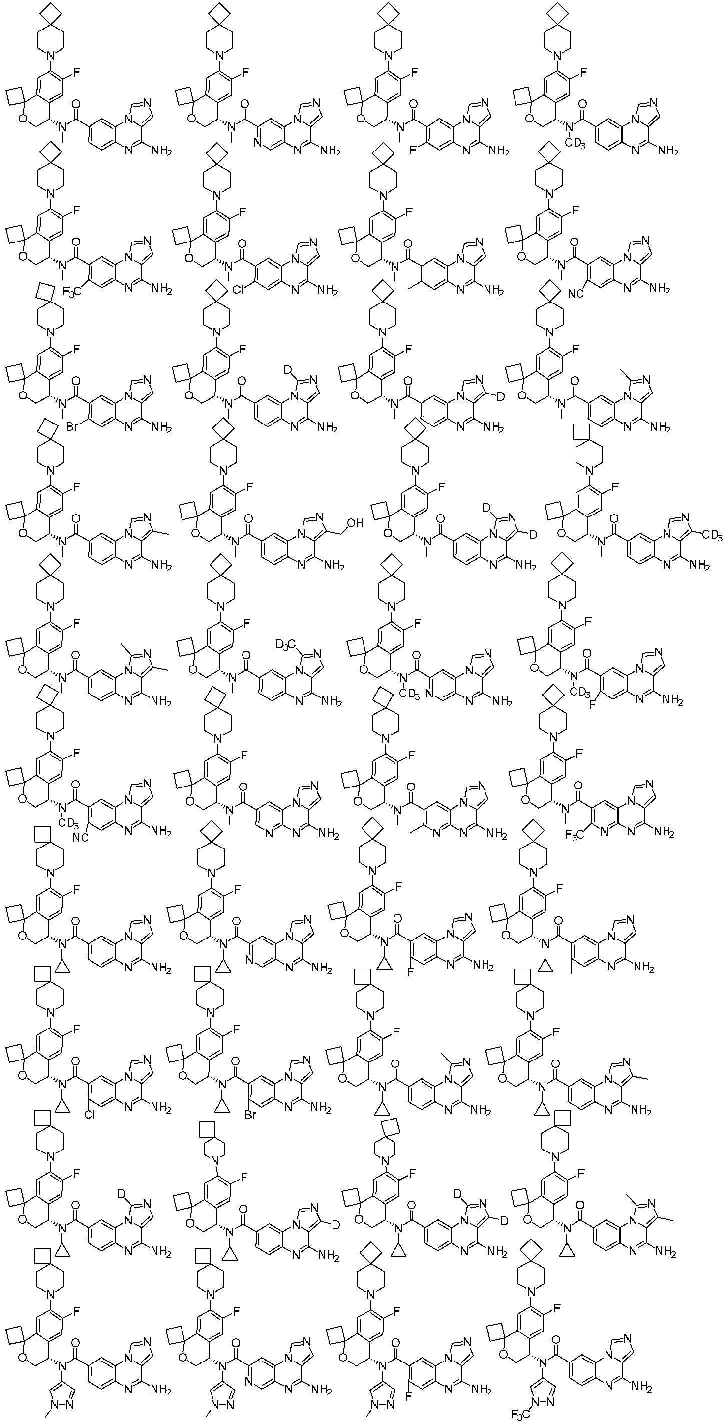

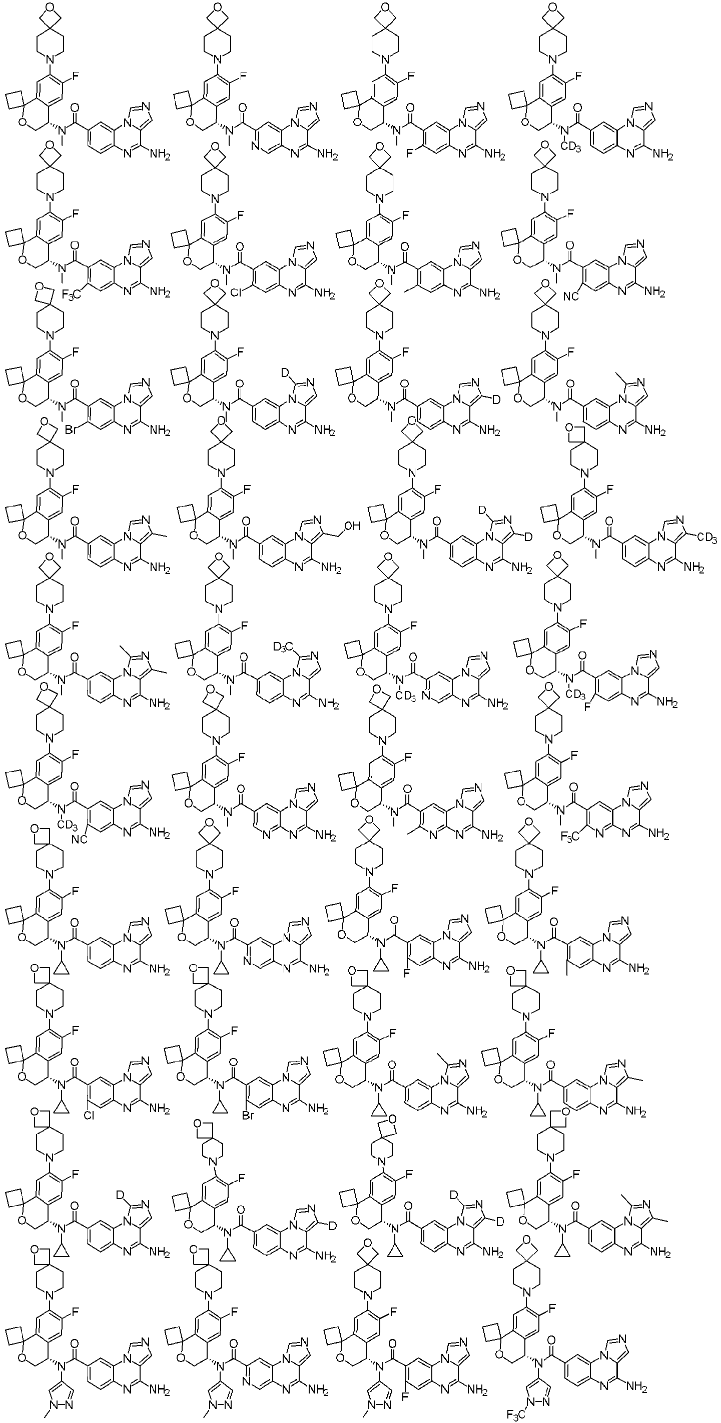

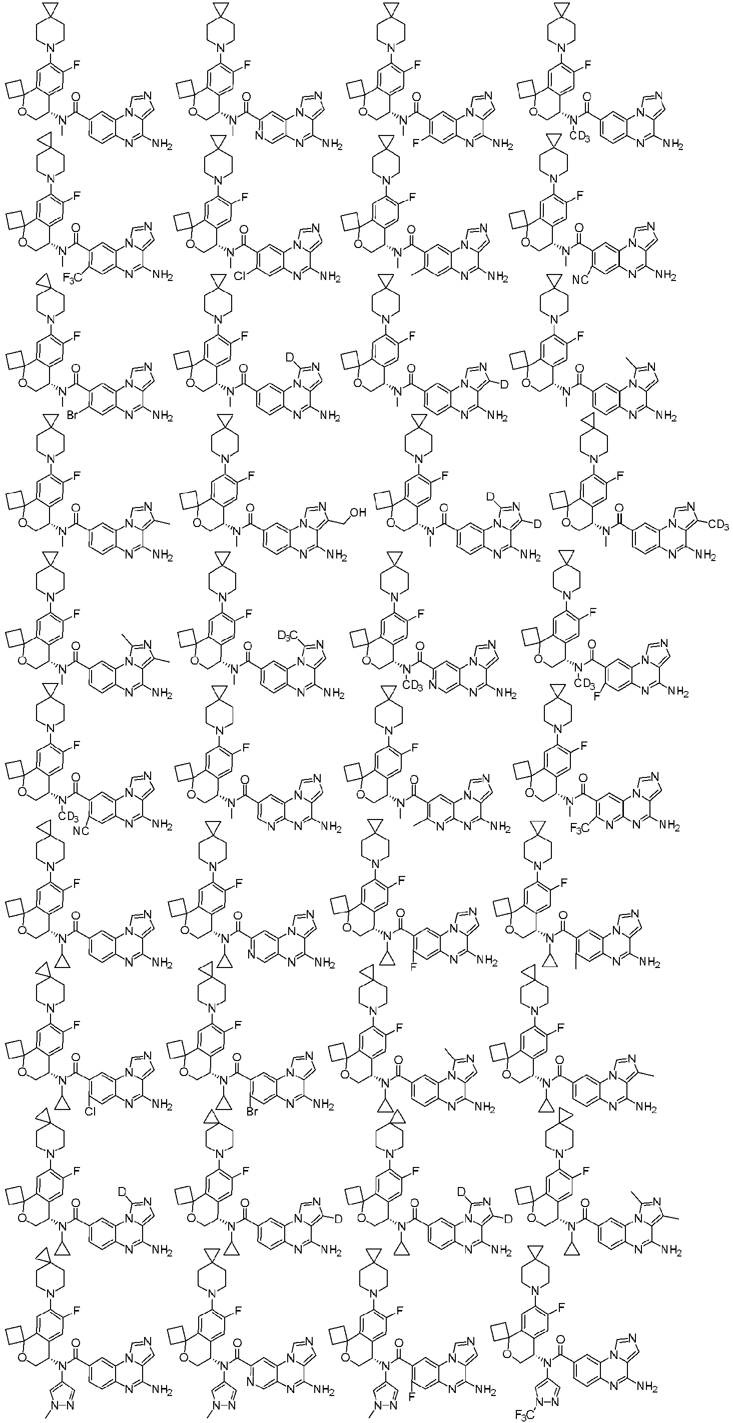

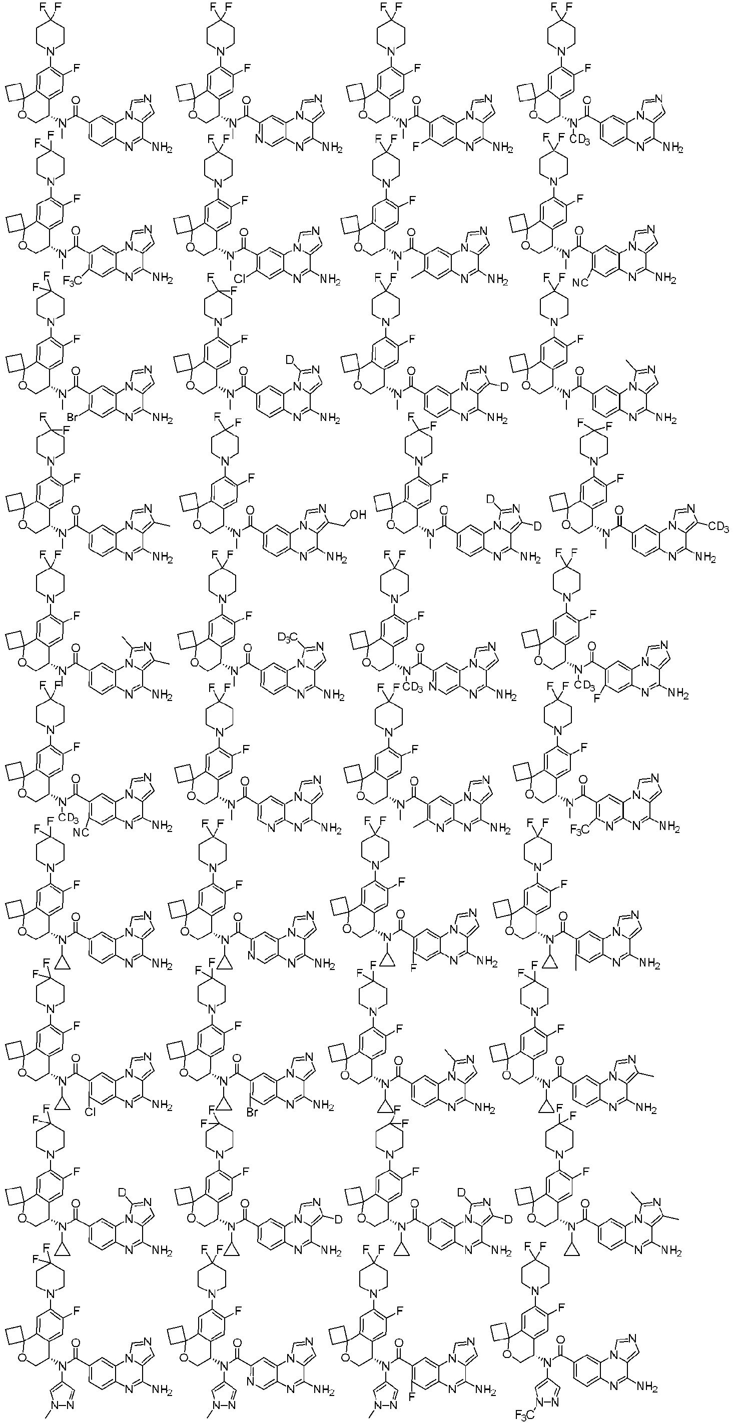

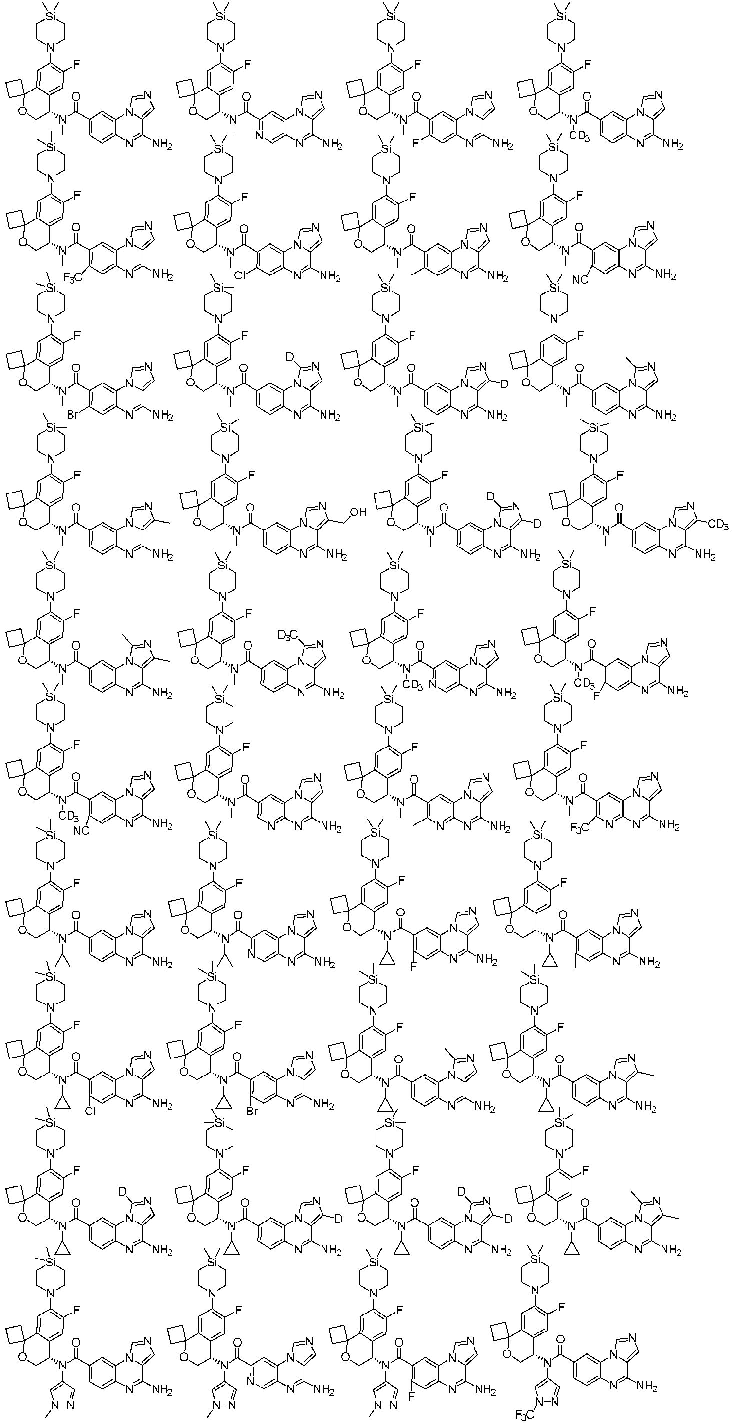

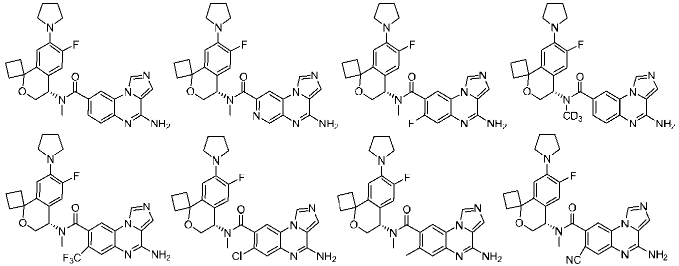

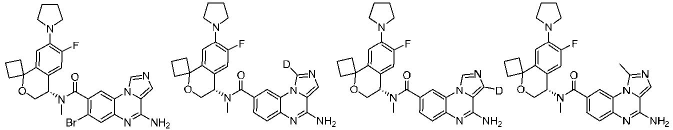

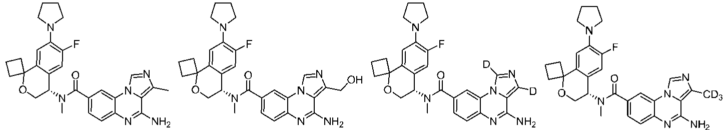

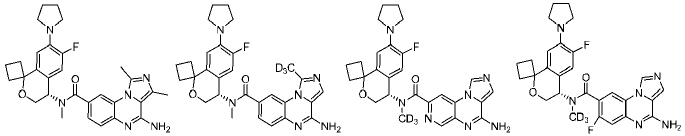

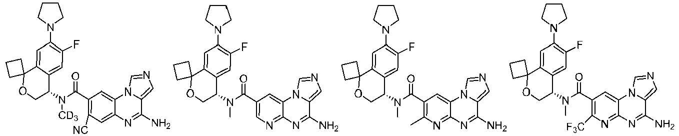

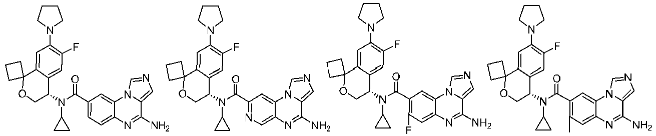

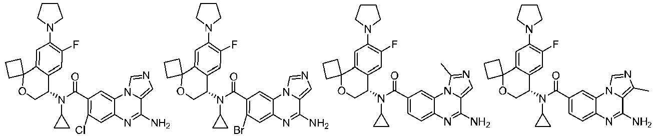

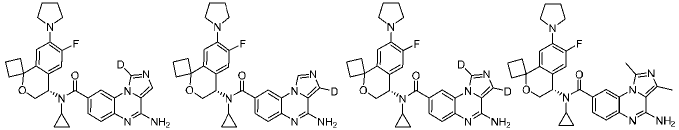

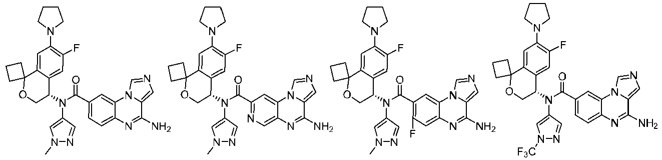

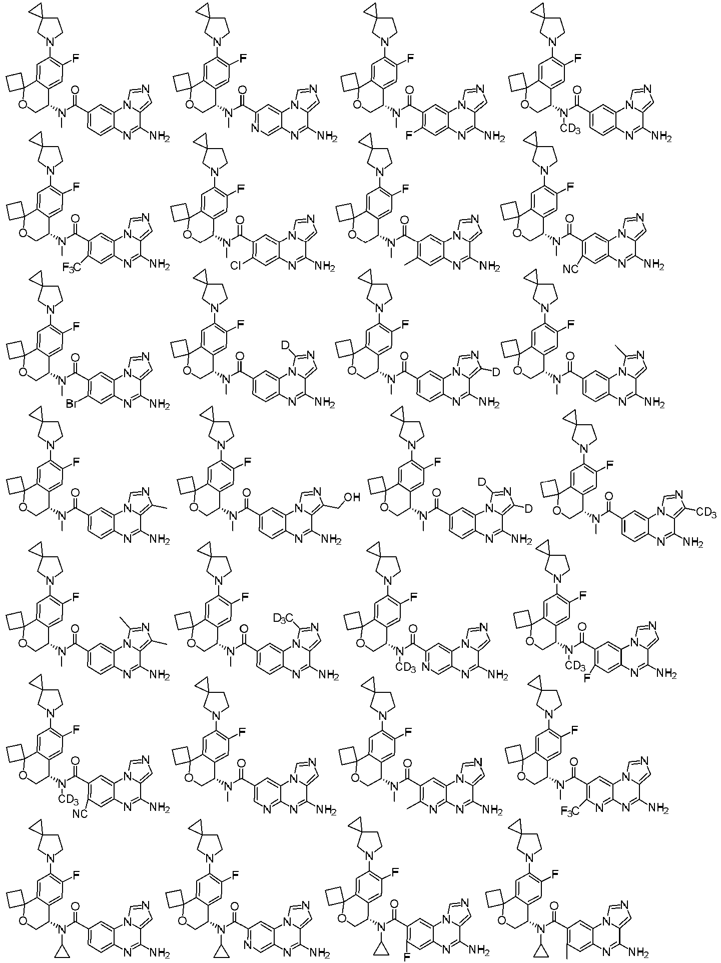

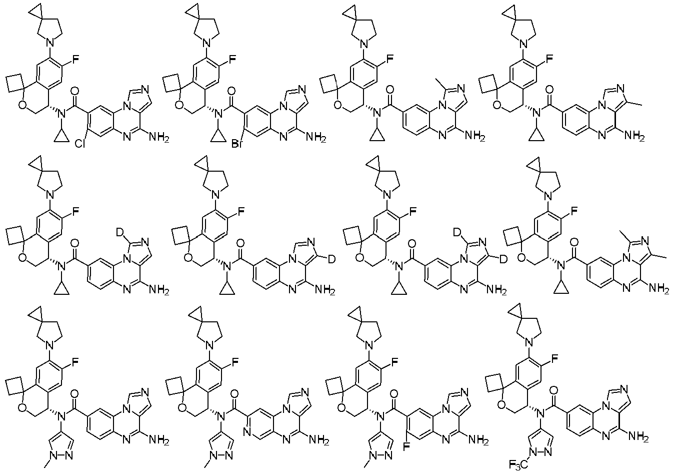

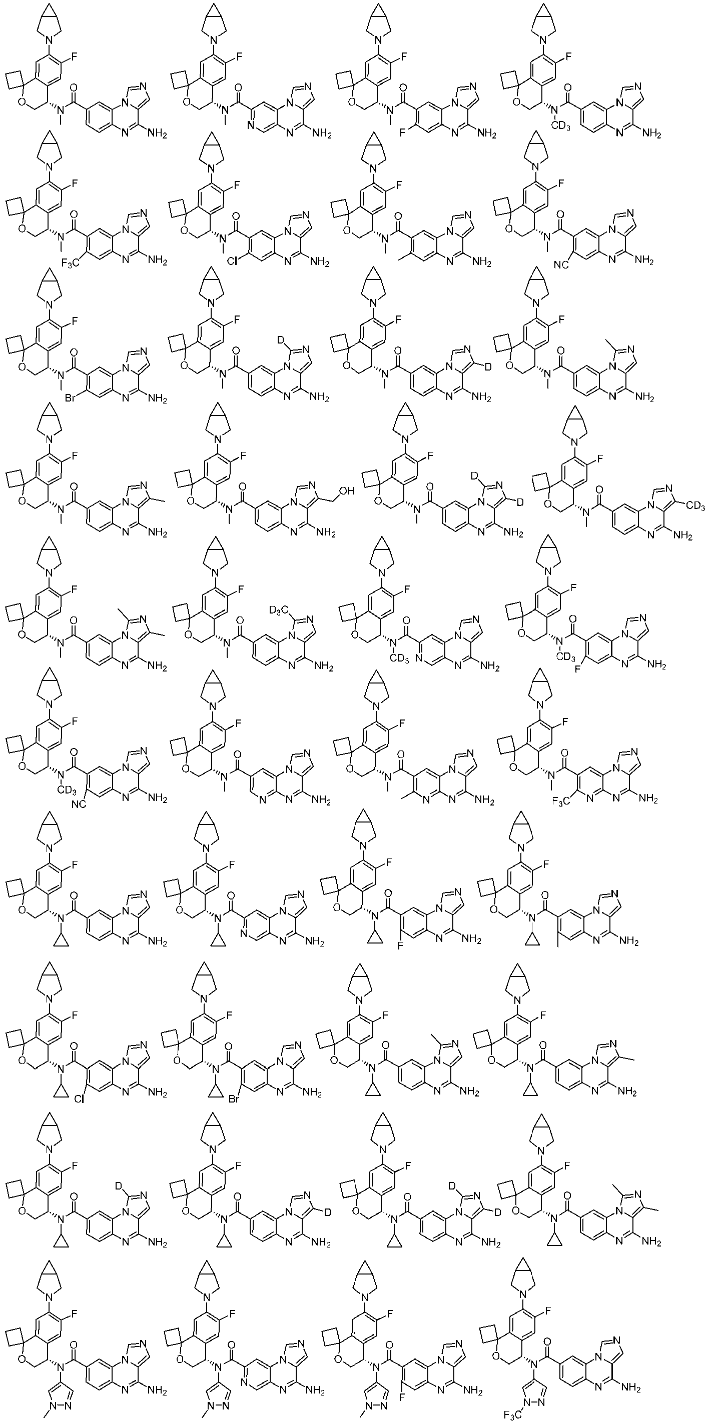

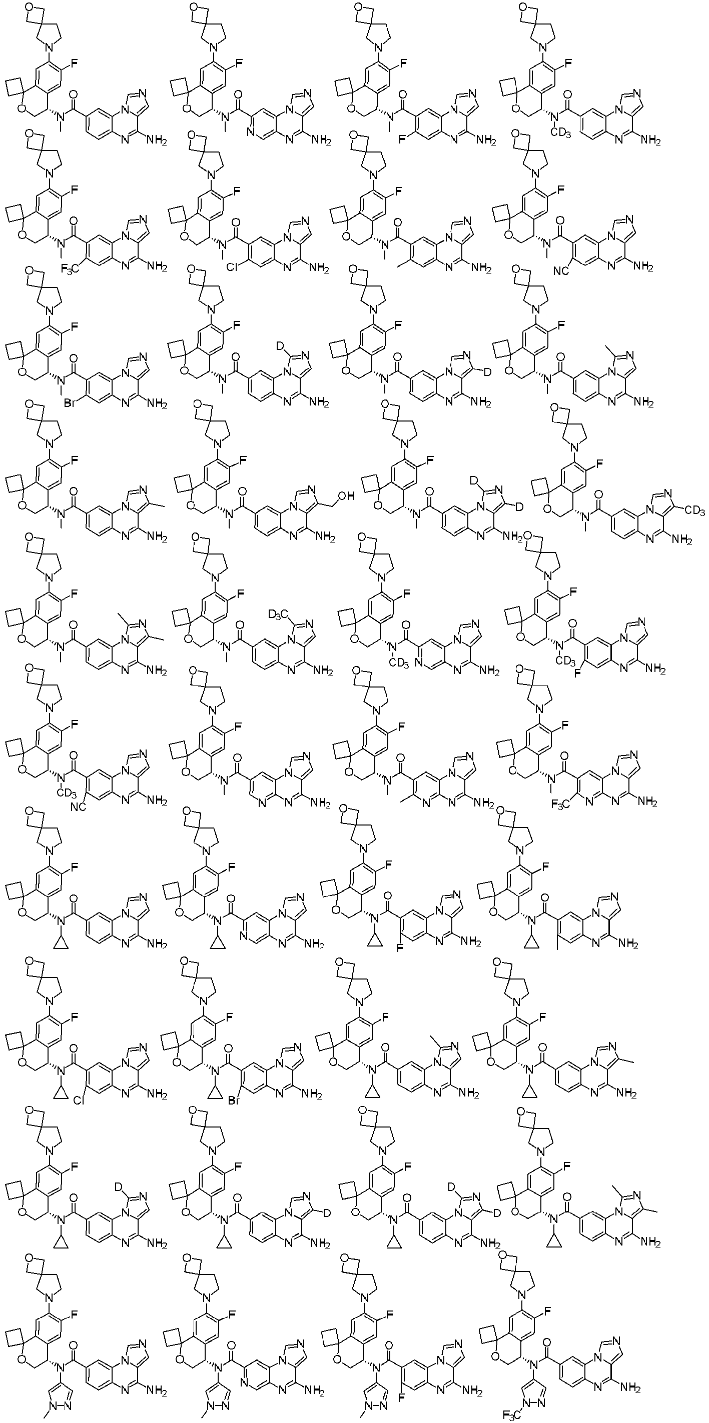

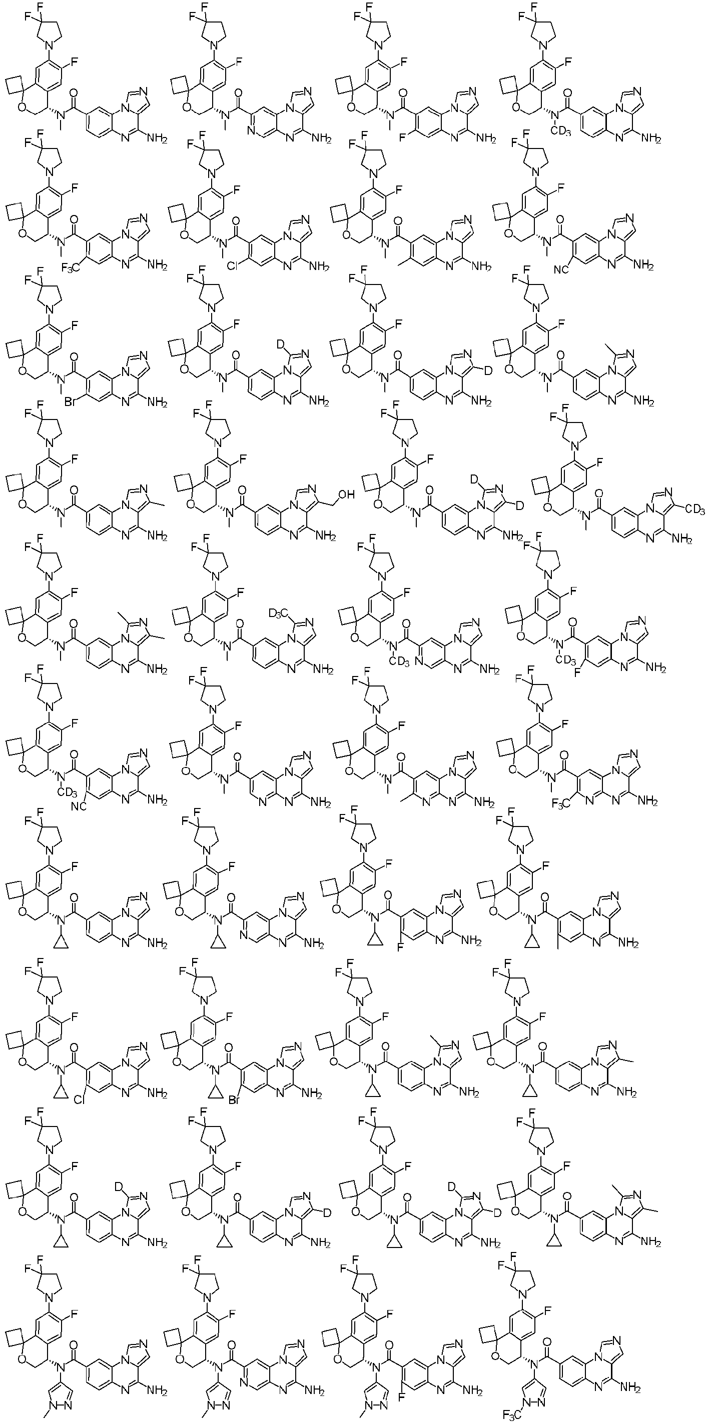

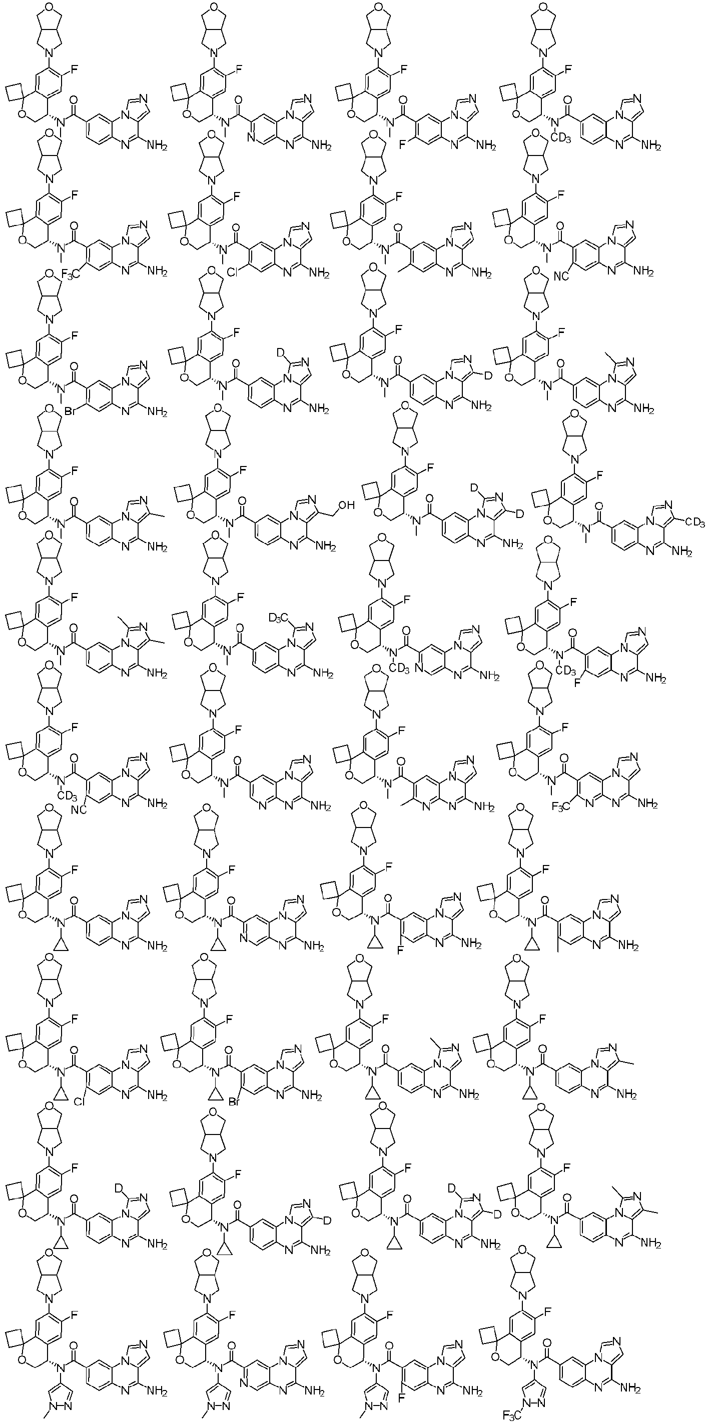

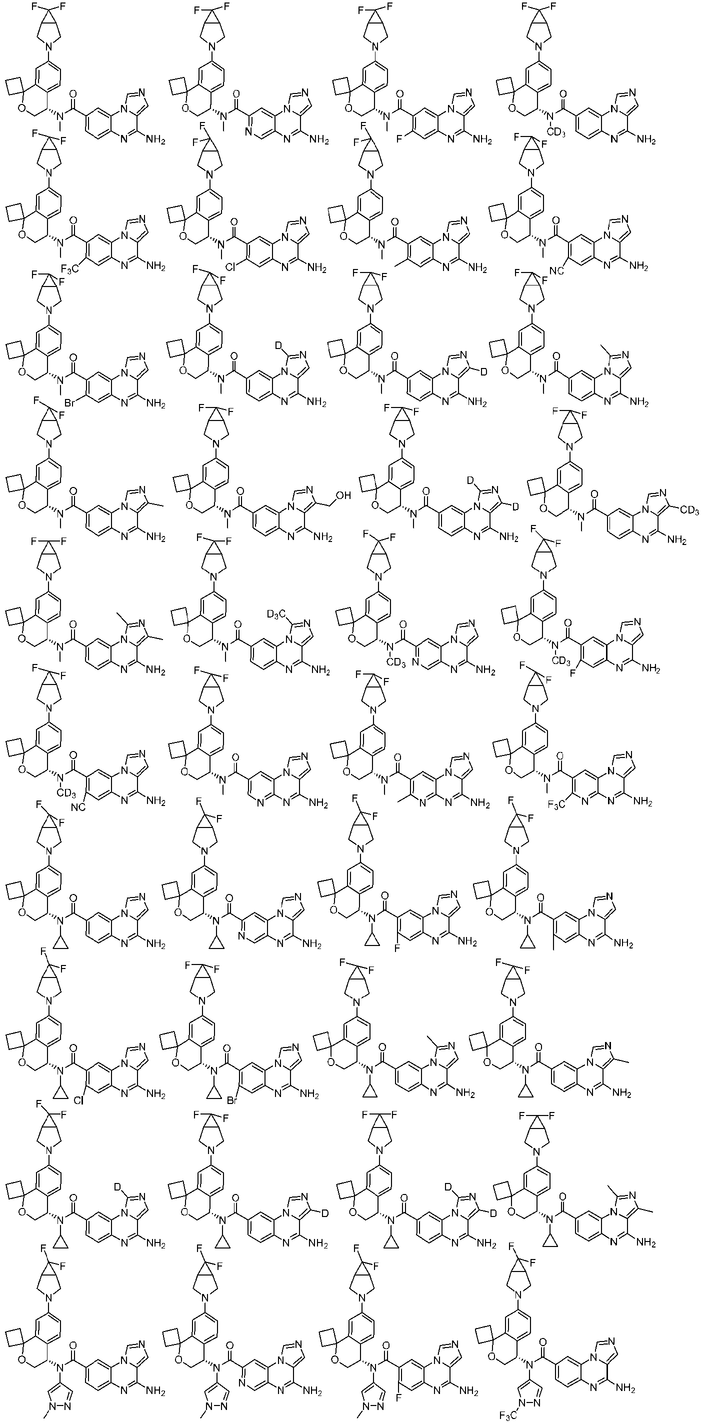

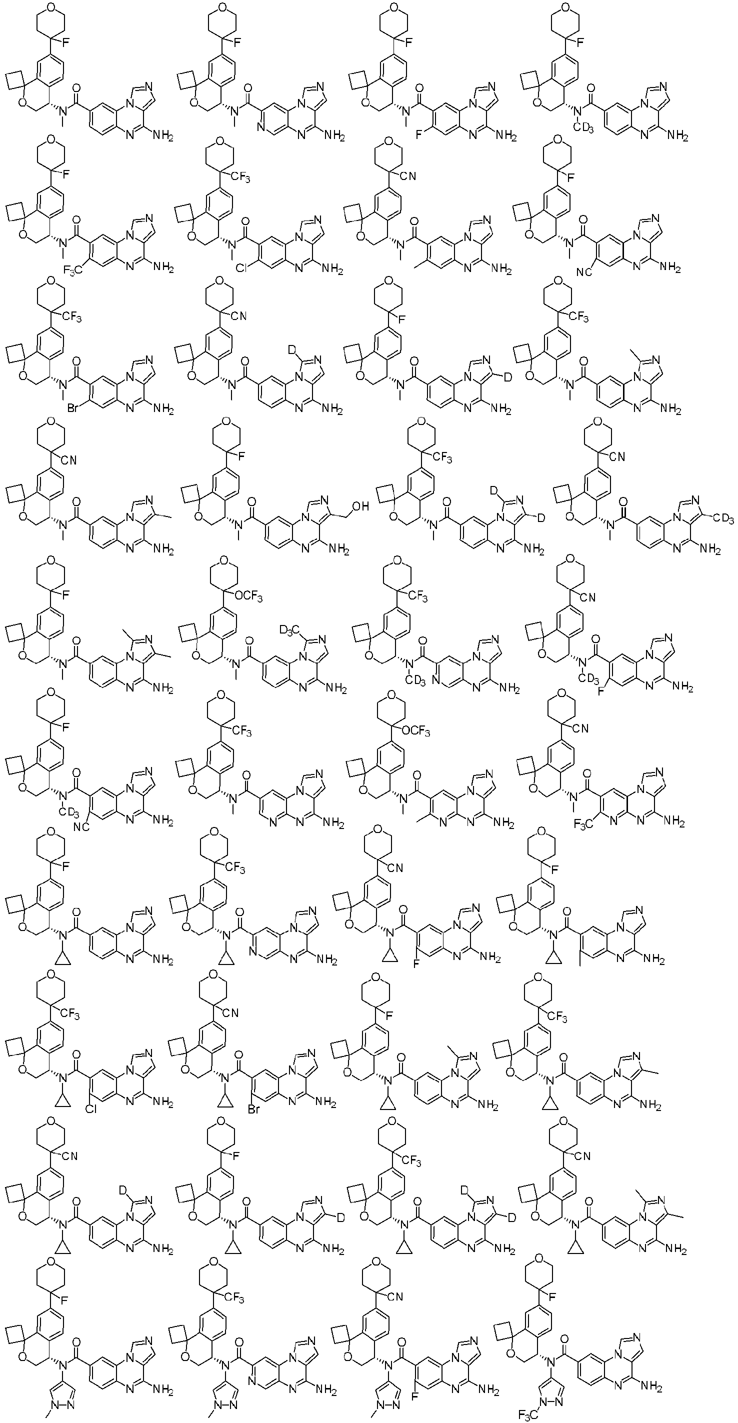

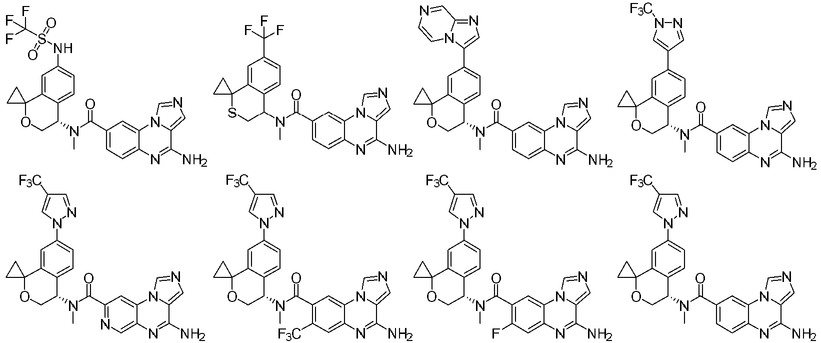

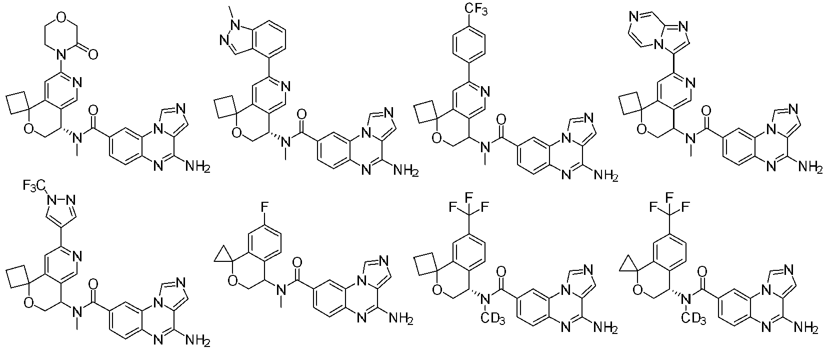

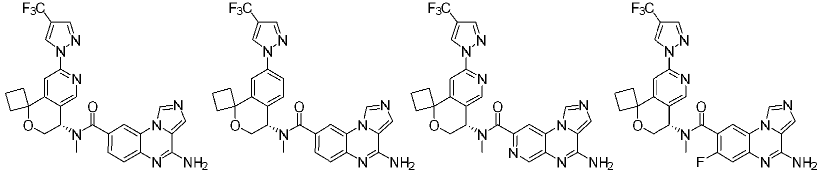

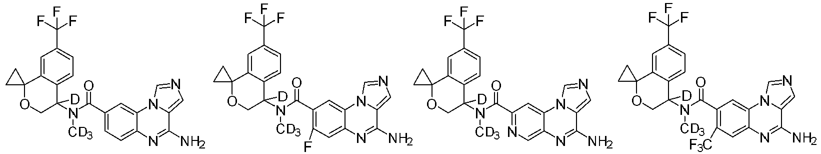

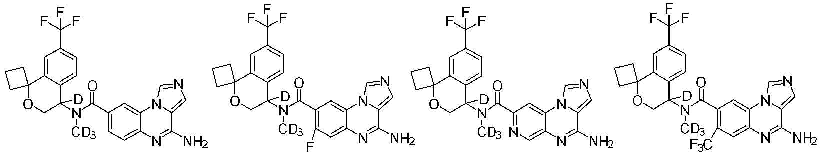

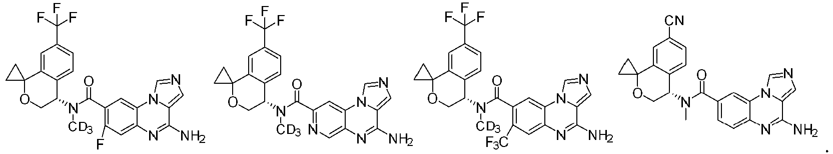

In addition, the present disclosure provides a method for treating and/or preventing a disease associated with abnormal expression of PRMT5, which comprises administering to an individual in need thereof a therapeutically and/or prophylactically effective amount of the compound described herein, tautomers thereof, or pharmaceutically acceptable salts thereof. Specifically, the disease associated with abnormal expression of PRMT5 is a tumor or cancer.

Unless otherwise specified, the compounds of the present disclosure may encompass, in addition to their specific structures, pharmaceutically acceptable salts, stereoisomers, isotope isomers (e.g., deuterates), solvates, hydrates, prodrugs and metabolites thereof. Thus, the pharmaceutically acceptable salts, stereoisomers, isotope isomers, solvates, hydrates, prodrugs, and metabolites of these compounds are also encompassed within the scope of the present disclosure.

Preferably, the pharmaceutical composition of the present disclosure may further comprise a second active substance, wherein the second active substance is an anti-tumor drug selected from one or more of a chemotherapeutic drug, a targeted anti-tumor drug, or an anti-tumor antibody drug.

Furthermore, the present disclosure providesa compound disclosed herein, its pharmaceutically acceptable salts, esters, prodrugs, stereoisomers, or isotopic derivatives, and methods of treating diseases, preferably tumors,by inhibiting PRMT5 activity.

### Definition:

Unless otherwise specified, the term "alkyl" alone or as part of another substituent, refers to linear (i.e. unbranched) or branched, or cyclic hydrocarbyl, or combinations thereof, which may be saturated, mono- or polyunsaturated, and may include divalent or multivalent groups, having a defined number of carbon atoms (i.e., C₁-C₁₀ refers to one to ten carbon atoms). Examples of saturated hydrocarbyl include, but are not limited to, groups such as methyl, ethyl, n-propyl, isopropyl, n-butyl, t-butyl, isobutyl, sec-butyl, cyclohexyl, cyclohexylmethyl, and cyclopropylmethyl, homologs and isomers such as n-pentyl, n-hexyl, n-heptyl, and n-octyl. An unsaturated alkyl has one or more double or triple bonds. Examples of unsaturated alkyl include, but are not limited to, vinyl, 2-propenyl, crotonyl, 2-isopentenyl, 2-(butadienyl), 2,4-pentadienyl, 3-(1,4-pentadienyl), ethynyl, 1- and 3-propynyl, 3-butynyl, as well as higher homologs and isomers. Alkyl groups defined as simple hydrocarbyls are referred to as "homoalkyl".The alkyl is optionally substituted with one or more halogen atoms.

The term "haloalkyl" refers to alkyl as defined above, wherein one or more hydrogen atoms are substituted with halogen atoms.

The term "alkylene" alone or as part of another substituent refers to a divalent group derived from alkyl, such as, but not limited to, -CH₂CH₂CH₂CH₂-, -CH₂CH=CHCH₂-, -CH₂C≡CCH₂- and - CH₂CH₂CH(CH₂CH₂CH₃)CH₂-. The alkyl (or the alkylene) usually has 1-24 carbon atoms, and groups having no more than 10 carbon atomsare preferred in the present disclosure. "Lower alkyl" or "lower alkylene" refers to shorter chain alkyl or alkylene, usually having eight or fewer carbon atoms. The alkylene is optionally substituted with one or more halogen atoms.

The term "alkynyl" refers to a linear or branched hydrocarbon group containing at least one carbon-carbon triple bond, which may be linear or branched, or a combination thereof. Examples of the alkynyl include ethynyl, propargyl, 3-methyl-1-pentynyl and 2-heptynyl. The alkynyl is optionally substituted with one or more halogen atoms.

The term "cycloalkyl" refers to a monocyclic or bicyclic saturated carbocyclic ring, each having 3-10 carbon atoms. The "fused analogues" of cycloalkyl groups refer to a monocyclic ring fused to an aryl or heteroaryl,. with the point of attachment located on the non-aromatic moiety. Examples of cycloalkyl and fused analogues thereof include cyclopropyl, cyclobutyl, cyclopentyl, cyclohexyl, tetrahydronaphthyl, decahydronaphthyl, and dihydroindenyl. The cycloalkyl is optionally substituted with one or more halogen atoms. Further, the term "cycloalkyl" in the present disclosure includes both bridged ring systems and spiro ring systems.

The term "alkoxy" refers to linear or branched alkoxy groups having the specified number of carbon atoms. C₁₋₆ alkoxy, for example, includes methoxy, ethoxy, propoxyand isopropoxy.

Unless otherwise specified, the term "heteroalkyl" alone or in combination with another term refers to a stable linear or branched, or cyclic hydrocarbon consisting of at least one carbon atom and at least one heteroatom selected from O, N, P, Si, S, or a combination thereof, wherein nitrogen atoms, phosphorus atoms or sulfur atoms may optionally be oxidized and the nitrogen atoms may optionally be quaternized. The heteroatoms O, N, P, S and Si may be placed at any position within the heteroalkyl or at positions where the alkyl is linked to the rest of the molecule. Examples include, but are not limited to, -CH₂-CH₂-O-CH₃, -CH₂-CH₂-NH-CH₃, - CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, - Si(CH₃)₃, -CH₂-CH=N-OCH₃, -CH=CH-N(CH₃)-CH₃, -O-CH₃, -O-CH₂-CH₃, and -CN. At most two or three heteroatoms may be contiguous. For instance, -CH₂-NH-OCH₃ and -CH₂-O-Si(CH₃)₃. Similarly, the term "heteroalkylene" itself or combination with another term refers to a divalent group derived from heteroalkyl such as, but not limited to, -CH₂-CH₂-S-CH₂-CH₂- and -CH₂-S-CH₂-CH₂-NH-CH₂-. For heteroalkylene, the heteroatom may be at either or both ends of the chain (e.g., alkyleneoxy, alkylenedioxy, alkyleneamino, alkylenediamino). Furthermore, for alkylene and heteroalkylene linking groups, the direction in the molecular formula of the linking groups does not indicate the orientation of the linking groups. For example, the molecular formula -C(O)OR'- represents -C(O)OR'- and -R'OC(O)-. As mentioned above, the heteroalkyl as used herein includes those groups that are linked to the rest of the molecule via a heteroatom, for example, -C(O)R', - C(O)NR', -NR'R", -OR', -SR' and/or -SO₂R'. Where reference is made to "heteroalkyl" followed by specific heteroalkyl such as -NR'R", it should be understood that the terms heteroalkyl and -NR'R" do not repeat and are not mutually exclusive. Instead, these specific heteroalkyl groups are referenced for greater clarity. Therefore, the term "heteroalkyl" should not be interpreted herein to exclude specific heteroalkyl such as -NR'R".

The term "cycloalkoxyl" refers to cycloalkyl as defined above that is bonded to an oxygen atom, such as cyclopropoxy.

The term "haloalkoxy" refers to alkoxy as defined above in which one or more hydrogen atoms are substituted with halogen.

The term "aryl" refers to a monocyclic or bicyclic aryl consisting exclusively of carbon atoms. The "fused analogues" of aryl are those in which an aryl is fused to a monocyclic cycloalkyl or monocyclic heterocyclyl, with the point of attachmentlocated in the aryl portion. Examples of the aryl and fused ring analogues thereof include phenyl, naphthyl, indanyl, indenyl, tetrahydronaphthyl, 2,3-dihydrobenzofuryl, dihydrobenzopyranyl and 1,4-benzodioxanyl.

The term "heteroaryl" refers to a monocyclic or bicyclic aryl containing at least one heteroatom selected from N, O and S. The "fused analogues" of heteroaryl groups are those in which a heteroaryl is fused to a monocyclic cycloalkyl or monocyclic heterocyclyl, with the point of attachmentlocated in the aryl portion. Examples of the heteroaryl include pyrrolyl, isozolyl, isothiazolyl, pyrazolyl, pyridinyl, oxazolyl, oxadiazolyl, thiadiazolyl, thiazolyl, imidazolyl, triazolyl, tetrazolyl, furyl, triazinyl, thienyl, pyrimidinyl, pyridazinyl, pyrazinyl, benzoxazolyl, benzothiazolyl, benzimidazolyl, benzofuranyl, benzothiopheneyl, furo[2,3-b]pyridinyl, quinolinyl, indolyland isoquinolinyl.

"Substituted or unsubstituted": the defined alkyl, aryl and heteroaryl are unsubstituted or substituted with at least one substituent selected from the following groups: halogen atoms; alkyl having 1 to 6 carbon atoms; alkoxy having 1 to 6 carbon atoms; haloalkyl having 1 to 6 carbon atoms; haloalkoxy having 1 to 6 carbon atoms; -CN; alkynyl having 2 to 6 carbon atoms; alkanoyl having 1 to 6 carbon atoms; cycloalkyl having 3 to 7 ring atoms; heteroaryl; aryl; aralkyloxy having 7 to 10 carbon atoms; arylcarbonyl; aminocarbonyl; alkenyl having 2 to 5 carbon atoms; alkylthio having 1 to 6 carbon atoms; aminosulfinyl; aminosulfonyl; hydroxy; -SF₅; hydroxyalkyl having 1 to 4 carbon atoms; nitro; amino; carboxyl; alkoxycarbonyl having 2 to 5 carbon atoms; alkoxyalkyl having 1 to 4 carbon atoms; alkylsulfonyl having 1 to 4 carbon atoms; alkanoylamino having 1 to 4 carbon atoms; alkanoyl(alkyl)amino having 1 to 6 carbon atoms; alkanoylamidoalkyl having 1 to 6 carbon atoms in each of the alkanoyl and alkyl moieties; alkanoyl(alkyl)aminoalkyl having 1 to 6 carbon atoms in each of the alkanoyl and alkyl portions; alkylsulfonamido having 1 to 4 carbon atoms; monoalkylaminocarbonyl or dialkylaminocarbonyl having 1 to 6 carbon atoms; monoalkylaminosulfinyl or dialkylaminosulfinyl having 1 to 6 carbon atoms; monoalkylaminosulfonyl or dialkylaminosulfonyl having 1 to 6 carbon atoms; aminoalkyl having 1 to 4 carbon atoms; monoalkylamino or dialkylamino having 1 to 6 carbon atoms; monoalkylaminoalkyl or dialkylaminoalkyl having 1 to 6 carbon atoms in each alkyl moiety; aralkyl having 7 to 10 carbon atoms; heteroaralkyl having 1 to 4 carbon atoms in the alkyl portion; heteroarylalkoxy having 1 to 4 carbon atoms in the alkoxy portion; and alkylsulfonamido having 1 to 4 carbon atoms.

As used herein, the term "heterocycle" or "heterocyclic" or "heterocycloalkyl" or "heterocyclyl" refers to a saturated, partially saturated or non-aromatic unsaturated group having a monocyclic or condensed ring, including bridged and spiro systems. The ring system contains1 to 10 carbon atoms and 1 to 4 heteroatoms selected from nitrogen, sulfur, or oxygen, where one or more of the rings may be cycloalkyl, aryl, or heteroaryl, as long as the connection point passes through a nonaromatic ring., In one embodiment, nitrogen atoms and/or sulfur atoms of the heterocyclic group are optionally oxidized to provide N-oxide, sulfinyl and sulfonylmoieties. Examples of "heterocyclyl" and fused analogues thereof include pyrrolidinyl, piperidinyl, piperazinyl, imidazolidinyl, 2,3-dihydrofuro[2,3-b] pyridinyl, benzoxazinyl, tetrahydroquinolinyl, tetrahydroisoquinolinyl, and dihydroindolyl. The term also includes non-aromatic, partially unsaturated monocyclic rings such as 2- or 4-pyridones linked via a nitrogen atom or N-substituted -(1H,3H)-pyrimidine-2,4-diones (N-substituted uracils).

As used herein, the term "substituted heterocyclic" or "substituted heterocycloalkyl" or "substituted heterocyclyl" refers to a heterocyclic group substituted with 1 to 5 (e.g., 1 to 3) substituents, which are the same as those defined for substituted cycloalkyl.

Unless otherwise specified, the term "halo" or "halogen" aolneor as part of another substituent refers to a fluorine, chlorine, bromine or iodine atom. In addition, the term "haloalkyl" includes monohaloalkyl and polyhaloalkyl. For example, the term "(C₁-C₆) haloalkyl"includes, but not limited to, trifluoromethyl, 2,2,2-trifluoroethyl, pentafluoroethyl, 4-chlorobutyland 3-bromopropyl, and the like.

"Prodrug" refers to a substance that is converted into the parent active drug after administration into the body. In some cases, the prodrugs are often used because they are easier to administer than the parent drugs. For example, the prodrugs may may achieve oral bioavailabilitywhile the parent drugs are not. In drug combinations, prodrugs can also exhibit higher solubility than the parent drug. By way of non-limiting example, any compounds of Formula I is administered in the form of an ester (prodrug) to facilitate transport across cell membranes where water solubility is detrimental to migration and which subsequently metabolically hydrolyzes into the active substance (carboxylic acid) once within cells where water solubility is beneficial. Another example of a prodrug can be short peptide (polyamino acid) conjugated with an acid moiety, where the peptide undergoes metabolism to release the active portion.

Optical isomers-diastereomers-geometrical isomers-tautomers:
The compounds of Formula (I) contain one or more asymmetric centers and may therefore exist as racemates and racemic mixtures, single enantiomers, mixtures of diastereomers and single diastereomers. The present disclosure should comprise all these isomeric forms of the compounds of formula (I).

Some of the compounds described herein contain an olefinic double bond, and accordingly includeboth E and Z geometrical isomers unless otherwise specified.

Some compounds of the present disclosure may comprise one or more ring systems, and thus cis- and trans-isomers may be present. The present disclosure encompasses all of these cis- and trans-isomers.

Some compounds described herein may exist in tautomeric forms via proton migration at different active sitesA typical example is keto- enol tautomerism between ketone and enol forms Individual tautomers and their mixtures are included in the compounds of the present disclosure.

The compounds of the present disclosure may be isolated into diastereoisomeric pairs, for example by fractional crystallization from a suitable solvent such as methanol or ethyl acetate or mixtures thereof. A pair of such enantiomers obtained can be separated into individual stereoisomers by conventional methods, for example using optically active amines or acids as resolution reagents or in a chiral HPLC column.

Alternatively, any enantiomers of the compounds of the present disclosure may be obtained by stereospecific synthesis using optically pure starting materials or reagents of known configuration.

Stable isotope-labeled analogues: one or more protons in the compounds of the present disclosure may be replaced bydeuterium atoms, thereby providing deuterated analogues having improved pharmacological activity.

### Salt and Dosage Form

It should be understood that, as used herein, reference to compounds of the present disclosure also includes pharmaceutically acceptable salts.

### Application

The compounds of the present disclosure can be used for treating PRMT5-associated diseases.

The compounds of the present disclosure can be prepared through the following reaction scheme:

### Method A:

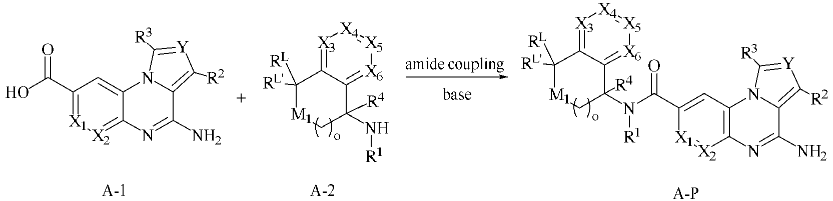

### Method A-SFC

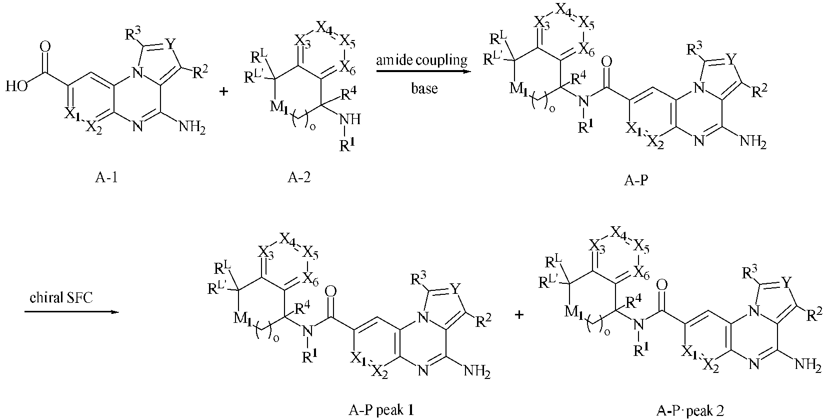

Wherein, X₁, X₂, X₃, X₄, X₅, X₆, Y, R¹, R², R³, M₁, R^{L}, R^{L'}, and o have the definition of the compound in formula (I) above.

Method A: Compounds A-P can be obtained by the amino-acid condensation reaction of carboxylic acid A-1 with amine A-2. The condensing agent may be HATU or PyBrOP, the base may be DIPEA or TEA, and the solvent may be DMF or DMAc. If the amine used is a racemate, it will be resolved by chiral SFC and the stereochemistry of the obtained isomer will be assigned arbitrarily to R or S.

### Analytical HPLC

Equipment: Agilent 1260; column specification: Agilent Poroshell HPH-C18 (3.0×50 mm, 2.7 µm); binary solvent system, mobile phase A: water (0.1% v/v ammonium bicarbonate), mobile phase B: acetonitrile; flow rate: 1 mL/min; gradient: from 10% B to 90% B; duration: 12 min; detector: DAD detector; wavelength: 254/220 nm;

### Preparation of HPLC-MS

HPLC equipment: Waters 2489; column specification: Ultimate µ XB-C18(130A, 5 µm, 30 mm×150 mm); binary solvent system, mobile phase A: water (0.1% v/v ammonium bicarbonate), mobile phase B: acetonitrile; flow rate: 60-100 mL/min; gradient: from 10 % B to 90 % B; detector: DAD detector; wavelength: 254/220 nm;
Mass spectrometer: Agilent G6125B.

The compound of the present disclosure may be prepared by chemical synthesis, examples of which are shown below. It will be understood that the sequence of steps in the process described may vary, those specifically mentioned reagents, solvents and reaction conditions may be substituted, and readily reactable sites may be protected and deprotected if necessary.

The following abbreviations have the meanings shown below. ACN refers to acetonitrile; EA refers to ethyl acetate; CDI refers to N, N'-carbonyldiimidazole; DBU refers to 1,8-diazabicyclo[5.4.0]undec-7-ene; DIBAL-H represents diisobutylaluminium hydride; DIEA refers to diisopropylethylamine; DMAP refers to N,N-dimethylaminopyridine; DME refers to 1,2-dimethoxyethane; DMF refers to N,N-dimethylformamide; DMA and DMAc refers to N,N-dimethylacetamide; DMPE refers to 1,2-bis(dimethylphosphino)ethane; DMSO means dimethylsulfoxide; DPPB means 1,4-bis(diphenylphosphino)butane; dppe refers to 1,2-bis(diphenylphosphino)ethane; dppf means 1,1'-bis(diphenylphosphino)ferrocene; dppm refers to 1,1'-bis(diphenylphosphino)methane; DIAD means diisopropyl azodicarboxylate; EDCI represents 1-(3-dimethylaminopropyl)-3-ethylcarbodiimide; HATU represents 2-(7-aza-1H-benzotriazol-1-yl)-1,1,3,3-tetramethylurea hexafluorophosphate; HMPA refers to hexamethylphosphoramide; IPA refers to isopropyl alcohol; LDA refers to lithium diisopropylamide; LHMDS refers to lithium di(trimethylsilyl)amide; LAH represents lithium aluminium hydride; NCS refers to N-chlorosuccinimide; NaHMDS refers to sodium di(trimethylsilyl)amide; PyBOP refers to benzotriazol-1-yl-oxytripyrrolidinophosphonium hexafluorophosphate; PyBrOP refers to bromo-tris-pyrrolidino-phosphonium hexafluorophosphate; TDA-I refers to tris(2-(2-methoxyethoxy)ethyl)amine; DCM refers to dichloromethane; TEA refers to triethylamine and TFA means trifluoroacetic acid; THF refers to tetrahydrofuran; NCS refers to N-chlorosuccinimide; NMM means N-methylmorpholine; NMP refers to N-methylpyrrolidone; PPh₃ refers to triphenylphosphine; r.t. refers to room temperature; PMB means p-methoxybenzyl; Tosmic means p-Toluenesulfonylmethyl isocyanide; (Boc)₂O refers to di-tert-butyl dicarbonate; PE refers to petroleum ether; o/n refers to overnight reaction.

The following preparations and examples illustrate the present disclosure, but do not limit the present disclosure in any way.

The features and advantages of the subject matter of the present disclosure will become more apparent from a detailed description of selected examples. As will be appreciated, the disclosed and claimed subject matter can be modified in various aspects, and all the modifications will not depart from the scope of the claims. Therefore, the description should be treated as illustrative in nature rather than restrictive. The entire scope of the subject matter of the present disclosure is set forth in the claims.

The present disclosure may be more easily understood with reference to the following examples, which are only used for illustrating the present disclosure instead of limiting the scope of the present disclosure.

### Intermediate 1 Methyl [10-(trifluoromethyl)spiro[cyclopropane-1,1'-isochroman|-7-yl]amine

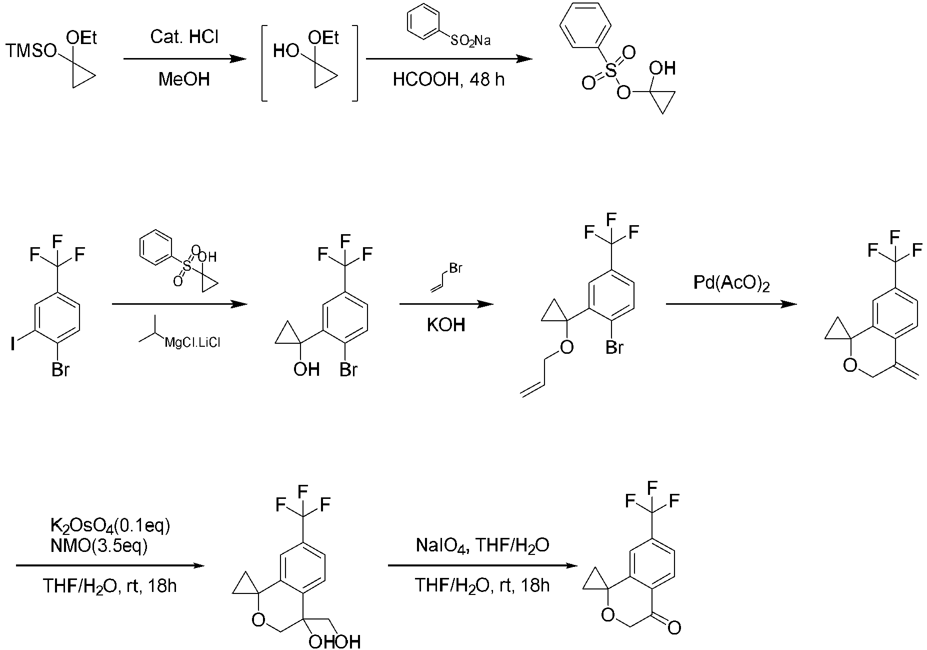

Step 1: A solution of hydrochloric acid in dioxane (0.2 mL, 4 M) was added dropwise to a solution of (1-ethoxycyclopropane)trimethylsilane (20 g, 0.115 mol) in methanol (80 mL), and stirred at room temperature for 15 min. After the reaction was complete, water (160 mL), sodium benzenesulfinate (37.7 g, 0.23 mol), and formic acid (43 mL, 1.15 mol) were directly added to the reaction mixture. The reaction was stirred for 48 h. After the reaction was complete, the reaction mixture was poured into water (300 mL) and extracted with dichloromethane (200 mL ×2). Then, the organic phases were combined, dried over anhydrous sodium sulfate, and concentrated under reduced pressure to obtain the crude solid, which was then triturated with petroleum ether (50 mL) to give 1-hydroxycyclopropanebenzenesulfonic acid (18 g, two-step yield: 73%) as a compound.

LCMS (ESI) m/z: 215.2 [M+H]⁺

Step 2: The solution of isopropylmagnesium chloride-lithium chloride in tetrahydrofuran (39 mL, 50.70 mmol, 1.3 M) was added to the solution of 1-bromo-2-iodo-4-(trifluoromethyl)benzene (17.84 g, 50.85 mmol) in tetrahydrofuran (150 mL) in an ice bath under nitrogen atmosphere. The reaction was allowed to proceed at 0°C for 1 h, then the reaction mixture was cooled to -78°C. The solution of 1-hydroxycyclopropanebenzenesulfonic acid (4.8 g, 24.21 mmol) in tetrahydrofuran (20 mL) was slowly added dropwise into the reaction mixture. After reaction at -78°C for 30 min, the reaction mixture was allowed to warm to room temperature and react for an additional 6 h. Then, it was quenched with a saturated ammonium chloride solution (100 mL) and water (150 mL) and extracted with ethyl acetate (200 mL×3). The organic phases were combined, dried over anhydrous sodium sulfate, and concentrated under reduced pressure to obtain the crude solid, which was purified by column chromatography (petroleum ether: ethyl acetate = 1:0 to 10:1) to give 1-[2-bromo-5-(trifluoromethyl)phenyl]cyclopropane-1-ol (4.1 g, yield: 60%) as a yellow oil

LCMS (ESI) m/z: 263.0 [M-OH]⁺

¹H NMR (400 MHz, CDCl₃) δ 7.71 (d, J= 8.4 Hz, 1H), 7.69 - 7.63 (m, 1H), 7.46 - 7.39 (m, 1H), 1.35 - 1.27 (m, 2H), 1.06 - 0.99 (m, 2H).

Step 3: At room temperature, 1-[2-bromo-5-(trifluoromethyl)phenyl]cyclopropane-1-ol (1.7 g, 6.048 mmol) was dissolved in tetrahydrofuran (20 mL). Potassium hydroxide (0.67 g, 12.09 mmol), tetrabutylammonium hydrogen sulfate (0.4 g, 1.210 mmol), and 3-bromopropene (1.5 g, 12.09 mmol) were added to the mixture and the mixture was stirred at 25 °C for 4 h. After the reaction was complete, the reaction mixture was concentrated under reduced pressure and poured into water (40 mL) and extracted with ethyl acetate (20 mL×3). The organic phases were combined, dried over anhydrous sodium sulfate, and filtered. The filtrate was concentrated under reduced pressure to obtain a crude product, which was purified by column chromatography (petroleum ether: ethyl acetate = 5:1) to give 1-{[2-bromo-5-(trifluoromethyl)phenyl]cyclopropoxy}prop-2-ene (1.8 g, yield: 92.6%) as a compound.

LCMS (ESI) m/z: 322.1 [M+H]⁺

Step 4: To a solution of 1-{[2-bromo-5-(trifluoromethyl)phenyl]cyclopropoxy}prop-2-ene (1.7 g, 5.39 mmol) in *N,N*-dimethylformamide (20 mL) were added *N,N*-diisopropylethylamine (2.1 g, 15.88 mmol), 9,9-dimethyl-4,5-bis(diphenylphosphino)xanthene (0.63 g, 1.059 mmol), and palladium acetate (0.12 g, 0.5294 mmol). The mixture was stirred at 90°C for 16 h under nitrogen atmosphere. The reaction mixture was poured into 60 mL of water and extracted with ethyl acetate (20 mL× 3). The organic phases were combined, dried over anhydrous sodium sulfate, and concentrated under reduced pressure to obtain a crude product, which was purified by column chromatography(petroleum ether: ethyl acetate = 10:1) to give 7-methylene-10-trifluoromethylspiro[cyclopropane-1,1'-isobenzopyrane] (1 g, yield: 78.6%).

LCMS (ESI) m/z: 241 [M+H]⁺

Step 5: At room temperature, 7-methylene-10-trifluoromethylspiro[cyclopropane-1,1'-isobenzopyrane] (1 g, 4.163 mmol) was dissolved in a mixed solvent of acetone (10 mL) and water (2 mL), and *N-*methylmorpholine-*N*-oxide (1.5 g, 12.49 mmol) and potassium osmate (0.16 g, 0.4163 mmol) were added. Under nitrogen atmosphere, the reaction mixture was stirred at 25°C for 16 h. After the reaction was complete, sodium sulfite solid (5 g) was added to the reaction mixture, which was stirred for 10 min, concentrated under reduced pressure to remove a certain amount of acetone, poured into water (20 mL), and extracted with ethyl acetate (20 mL × 3). The organic phases were combined, dried over anhydrous sodium sulfate, and concentrated under reduced pressure to give 7-hydroxymethyl-10-trifluoromethylspiro[cyclopropane-1,1'-isochroman]-7-ol (0.6 g, yield: 52.5%) as a crude product.

LCMS (ESI) m/z: 256 [M-18]⁺

Step 6: At room temperature, 7-hydroxymethyl-10-trifluoromethylspiro[cyclopropane-1,1'-isochroman]-7-ol (1 g, 3.646 mmol) was dissolved in a mixed solvent of tetrahydrofuran (30 mL) and water (3 mL). Sodium metaperiodate (3.183 g, 14.59 mmol) was added to the reaction mixture, which was stirred at 25°C for 4 h under nitrogen atmosphere. After the reaction was complete, the reaction mixture was diluted with ethyl acetate, filtered, and washed. The filtrate was combined, dried over anhydrous sodium sulfate, filtered, and concentrated under reduced pressure to obtain a crude product, which was purified by column chromatography (petroleum ether: ethyl acetate = 3:1) to give 7'-(trifluoromethyl)spiro[cyclopropane-1,1'-isochroman]-4'-one (0.45 g, yield: 51%).

LCMS (ESI) m/z:243.2 [M+H]⁺

Employing the preparation method and steps of intermediate 1, with the sole replacement of the corresponding raw material intermediate, the following intermediates were obtained:

| Intermediate No. | Chemical Structure | Chemical Name | m/z(ESI) : (M+H)⁺ |
|---|---|---|---|
| 2 | 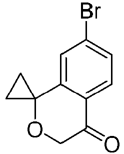 | 7'-bromospiro[cyclopropane-1,1'-isochroman]-4'-one | 254.2 |
| 54 | 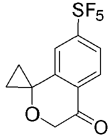 | 7'-(pentafluoro- λ ⁶-sulfanyl)spiro[cyclopropane-1,1'-isochroman]-4'-one | 301.2 |
| 55 | 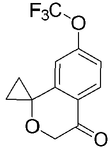 | 7'-(Trifluoromethoxy)spiro[cyclopropane-1,1'-isochroman]-4'-one | 259.2 |
| 56 | 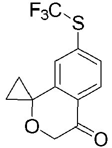 | 7'-(Trifluoromethyl)thio)spiro[cyclopropane-1,1'-isochroman]-4'-one | 275.2 |
| 57 | 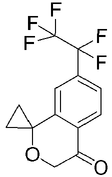 | 7'-(Perfluoroethyl)spiro[cyclopropane-1,1'-isochroman]-4'-one | 293.2 |
| 58 | 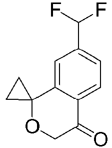 | 7'-(Difluoromethyl)spiro[cyclopropane-1,1'-isochroman]-4'-one | 225.2 |
| 59 | 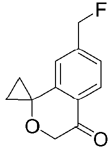 | 7'-(Fluoromethyl)spiro[cyclopropane-1,1'-isochroman]-4'-one | 207.2 |
| 60 | 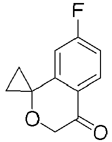 | 7'-Fluorospiro[cyclopropane-1,1'-isochroman]-4'-one | 193.2 |

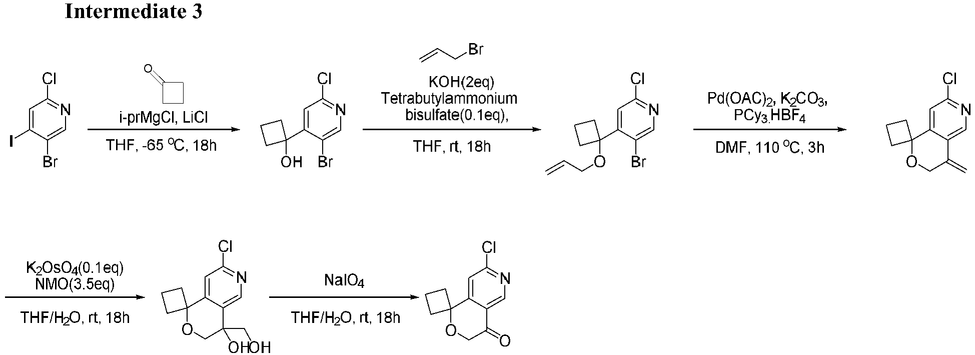

Step 1: At room temperature, 5-bromo-2-chloro-4-iodopyridine (25 g, 78.6 mmol) was dissolved in tetrahydrofuran (250 mL). The mixture was purged with nitrogen three times. At -78 °C, isopropylmagnesium chloride lithium chloride complex (60.4 mL, 1.3 M, 78.5 mmol) was slowly added dropwise to the system. The mixture was stirred for reaction at -78°C for 2 hours. Then, a solution of cyclobutanone (5.5 g, 78.5 mmol) in tetrahydrofuran (30 mL) was added to the reaction mixture. The mixture was slowly warmed to room temperature and reacted for 16 hours. After the reaction was complete, it was quenched with ammonium chloride solution (150 mL). The mixture was poured into water (200 mL) and extracted with ethyl acetate (200 mL×3). The combined organic layer was dried over anhydrous sodium sulfate and filtered. The filtrate was concentrated under reduced pressure to give a crude product. The crude product was purified by column chromatography (petroleum ether: ethyl acetate = 1:0-10:1) to give 1-(5-bromo-2-chloropyridin-4-yl)cyclobutan-1-ol (10.9 g, yield: 53%) as a pale yellow oil. mmol

¹H NMR (400 MHz, DMSO-*d*₆) δ 8.53 (s, 1H), 7.49 (s, 1H), 5.78 (s, 1H), 2.65-2.56 (m, 2H), 2.36-2.27 (m, 2H), 2.03-1.99 (m, 1H), 1.63-1.55 (m, 1H).

Step 2: At room temperature, 1-(5-bromo-2-chloropyridin-4-yl)cyclobutan-1-ol (10.9 g, 41.5 mmol) was dissolved in tetrahydrofuran (100 mL). Potassium hydroxide (4.28 g, 83.0 mmol), tetrabutylammonium hydrogen sulfate (2.59 g, 8.3 mmol) and 3-bromopropene (4.6 g, 41.5mmol) were added. The mixture was reacted at 25°C for 16 hours. After the reaction was complete, the reaction mixture was poured into water (200 mL) and extracted with ethyl acetate (200 mL×3). The combined organic layer was dried over anhydrous sodium sulfate and filtered. The filtrate was concentrated under reduced pressure to give a crude product. The crude product was purified by column chromatography (petroleum ether: ethyl acetate = 10:1) to give 2-[1-(allyloxy)cyclobutyl]-1-bromo-4-(trifluoromethyl)benzene (6.6 g, yield: 57.4%) as a pale yellow oil.

LCMS (ESI): [M+H]⁺ = 301.9/303.9.

Step 3: At room temperature, 1-bromo-2-[1-(allyloxy)cyclobutyl]-4-(trifluoromethyl)benzene (9.5 g, 31.20 mmol) was dissolved in *N,N*-dimethylformamide (30 mL). Potassium carbonate (9.7 g, 70.19 mmol), palladium acetate (II) (0.71 g, 3.11 mmol), and tricyclohexylphosphine tetrafluoroborate (1.14 g, 3.11 mmol) were added to the mixture. The reaction mixture was stirred at 70°C for 16 h under nitrogen atmosphere, poured into 1000 mL of water and extracted with ethyl acetate (800 mL×3). The organic phases were combined, dried over anhydrous sodium sulfate, and concentrated under reduced pressure to obtain a crude product, which was purified by column chromatography(petroleum ether: ethyl acetate = 10:1) to give 7'-chloro-4'-methylene-3',4'-dihydrospiro[cyclobutane-1,1'-pyrano[4,3-c]pyridine] (3.6 g, yield: 43.4%) as a colorless oil.

¹H NMR (400 MHz, DMSO-*d*₆) δ 8.81 (s, 1H), 7.65 (s, 1H), 5.91 (s, 1H), 5.22 (s, 1H), 4.32 (s, 2H), 2.47-2.40 (m, 2H), 2.38-2.29 (m, 2H), 2.07-1.98 (m, 2H).

Step 4: At room temperature, 7'-chloro-4'-methylene-3',4'-dihydrospiro[cyclobutane-1,1'-pyrano[4,3-c]pyridine] (1.5 g, 6.7 mmol) was dissolved in a mixed solvent of tetrahydrofuran (21 mL) and water (7 mL). *N-*methylmorpholine-*N*-oxide (2.76 g, 20.8 mmol) and potassium osmate (0.21 g, 0.67 mmol) were added to the mixture. The reaction mixture was stirred at 25°C for 16 h under nitrogen atmosphere. After the reaction was complete, sodium sulfite solid (1.3 g) was added. The mixture was stirred for 10 min, concentrated under reduced pressure to remove a certain amount of tetrahydrofuran, poured into water (40 mL), and extracted with ethyl acetate (60 mL×4). The organic phases were combined, dried over anhydrous sodium sulfate, and concentrated under reduced pressure to give 7'-chloro-4'-(hydroxymethyl)-3',4'-dihydrospiro[cyclobutane-1,1'-pyrano[4,3-c]pyridine]-4'-ol (1.1 g, crude product).

LCMS (ESI): [M+H]⁺ = 256.12.

Step 5: At room temperature, 7'-chloro-4'-(hydroxymethyl)-3',4'-dihydrospiro[cyclobutane-1,1'-pyrano[4,3-c]pyridine]-4'-ol (1.1 g, 4.3 mmol) was dissolved in a mixed solvent of tetrahydrofuran (40 mL) and water (4 mL). Sodium periodate (2.89 g, 13.36 mmol) was added to the reaction mixture, which was stirred at 25°C for 4 h under nitrogen atmosphere. After the reaction was complete, the reaction mixture was diluted with ethyl acetate, filtered, and washed. The filtrate was combined, dried over anhydrous sodium sulfate, filtered, and concentrated under reduced pressure to obtain a crude product, which was purified by column chromatography (petroleum ether: ethyl acetate = 3:1) to give 7'-chlorospiro[cyclobutane-1,1'-pyrano[4,3-c]pyridine]-4'(3'H)-one (0.7 g, two-step yield: 52%).

¹H NMR (400 MHz, DMSO-*d₆*) δ 8.80 (s, 1H), 7.88 (s, 1H), 4.41 (s, 2H), 2.51-2.47 (m, 4H), 2.05-2.01 (m, 2H).

### Intermediate 4 N-methyl-2'-morpholinyl-5',6'-dihydrospiro[cyclobutane-1,8'-pyrano|[3,4-b]pyridine]-5'-amine

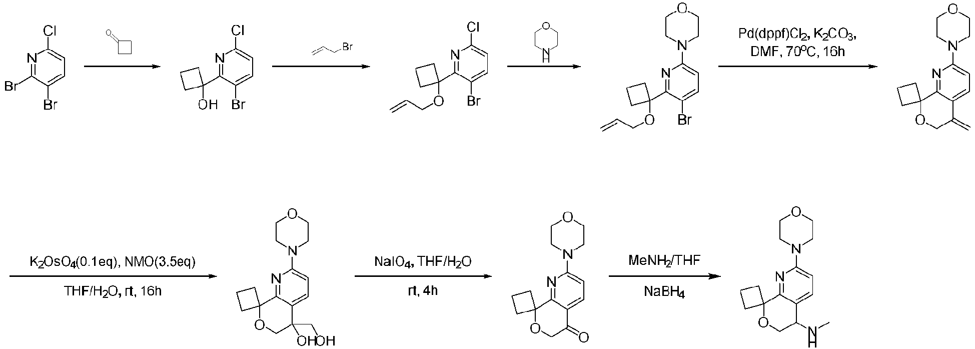

Step 1: At room temperature, 2,3-dibromo-6-chloropyridine (25 g, 92.3 mmol) was dissolved in tetrahydrofuran (250 mL). The mixture was purged with nitrogen three times. Isopropylmagnesium chloride-lithium chloride (70.9 mL, 1.3 M, 92.3 mmol) was slowly added dropwise to the system at -78°C, and the reaction mixture was stirred at -78°C for 2 h. Then, the solution of cyclobutanone (6.5 g, 92.3 mmol) in tetrahydrofuran (30 mL) was added to the above reaction mixture, which was allowed to gradually warm to room temperature and react for 16 h. When the reaction was complete, the reaction mixture was quenched with ammonium chloride solution (150 mL), poured into water (200 mL), and extracted with ethyl acetate (200 mL×3). The organic phases were combined, dried over anhydrous sodium sulfate, and filtered. The filtrate was concentrated under reduced pressure to obtain a crude product, which was purified by column chromatography (petroleum ether: ethyl acetate = 1:0 to 10:1) to give 1-(3-bromo-6-chloropyridin-2-yl)cyclobutane-1-ol (8.6 g, yield: 53%) as a pale yellow oil.

¹H NMR (400 MHz, DMSO-*d*₆) δ 7.84 (d, *J* =7.6Hz, 1H), 7.55 (d, *J* =7.6Hz, 1H), 5.61 (s, 1H), 2.64 - 2.53 (m, 2H), 2.38 - 2.30 (m, 2H), 2.03 - 1.97 (m, 1H), 1.60 - 1.52 (m, 1H).

Step 2: At room temperature, 1-(3-bromo-6-chloropyridin-2-yl)cyclobutane-1-ol (8.6 g, 32.8 mmol) was dissolved in tetrahydrofuran (100 mL). Potassium hydroxide (3.45 g, 65.6 mmol), tetrabutylammonium hydrogen sulfate (2.09 g, 3.3 mmol), and 3-bromopropene (3.7 g, 32.8 mmol) were added to the mixture, which was allowed to react at 25°C for 16 h. After the reaction was complete, the reaction mixture was poured into water (100 mL) and extracted with ethyl acetate (100 mL×3). The organic phases were combined, dried over anhydrous sodium sulfate, and filtered. The filtrate was concentrated under reduced pressure to obtain a crude product, which was purified by column chromatography(petroleum ether: ethyl acetate = 10:1) to give 2-[1-(allyloxy)cyclobutyl]-3-bromo-6-chloropyridinebenzene (6.8 g, yield: 81.6%) as a pale yellow oil.

¹H NMR (400 MHz, DMSO-*d*₆) δ 7.94 (d, *J* =7.6Hz, 1H), 7.59 (d, *J* =7.6Hz, 1H), 5.82 - 5.69 (m, 1H), 5.24 - 5.14 (m, 1H), 5.07 - 4.97 (m, 1H), 3.59 - 3.48 (m, 2H), 2.56 - 2.44 (m, 4H), 2.01 - 1.94 (m, 1H), 1.65 - 1.55 (m, 1H).

Step 3: At room temperature, 2-[1-(allyloxy)cyclobutyl]-3-bromo-6-chloropyridinebenzene (6.8 g, 22.7 mmol) was dissolved in morpholine (12 mL), and the mixture was stirred at 100°C for 16 h. After the reaction was complete, the filtrate was concentrated under reduced pressure to obtain a crude product, which was purified by column chromatography (petroleum ether: ethyl acetate = 3:1) to give 4-(6-(1-(allyloxy)cyclobutyl)-5-bromopyridin-2-yl)morpholine (4.6 g, yield: 60.6%, 1.1 g raw material) as a pale yellow oil.

¹H NMR (400 MHz, DMSO-*d*₆) δ 7.58 (d, *J* =7.6Hz, 1H), 6.78 (d, *J* =7.6Hz, 1H), 5.89 - 5.70 (m, 1H), 5.26 - 5.16 (m, 1H), 5.09 - 4.94 (m, 1H), 3.69 - 3.66 (m, 4H), 3.55 - 3.50 (m, 2H), 3.45 - 3.41 (m, 4H), 2.50 - 2.44 (m, 2H), 2.42 - 2.35 (m, 2H), 1.97 - 1.90 (m, 1H), 1.61 - 1.53 (m, 1H).

Step 4: At room temperature, 4-(6-(1-(allyloxy)cyclobutyl)-5-bromopyridin-2-yl)morpholine (4.6 g, 13.1 mmol) was dissolved in N,N-dimethylformamide (20 mL). Potassium carbonate (5.4 g, 39.3 mmol) and [1,1'-bis(diphenylphosphino)ferrocene]dichloropalladium(II) (0.95 g, 1.3 mmol) were added. The reaction mixture was stirred at 70°C for 16 h under nitrogen atmosphere, cooled, poured into 200 mL of water, and extracted with ethyl acetate (80 mL×3). The organic phases were combined, dried over anhydrous sodium sulfate, and concentrated under reduced pressure to obtain a crude product, which was purified by column chromatography (petroleum ether: ethyl acetate = 3:1) to give 5'-methylene-2'-morpholinyl-5',6'-dihydrospiro[cyclobutane-1,8'-pyrano[3,4-b]pyridine] (2.6 g pure product, yield: 73.4%) as a pale yellow oil.

¹H NMR (400 MHz, DMSO-*d*₆) δ 7.70 (d, *J* =7.6Hz, 1H), 6.85 (d, *J* =7.6Hz, 1H), 6.01 (s, 1H), 5.06 (s, 1H), 4.37 (s, 2H), 3.72 - 3.69 (m, 4H), 3.48 - 3.45 (m, 4H), 2.33 - 2.27 (m, 4H), 1.99-1.94 (m, 1H), 1.94 - 1.87 (m, 1H).

Step 5: At room temperature, 5'-methylene-2'-morpholino-5'-,6'-dihydrospiro[cyclobutane-1,8'-pyrano[3,4-b]pyridine] (2.6 g, 9.6 mmol) was dissolved in a mixed solvent of tetrahydrofuran (30 mL) and water (10 mL). *N*-methylmorpholine-*N*-oxide (3.4 g, 28.8 mmol) and potassium osmate (0.3 g, 1 mmol) were added. Under nitrogen atmosphere, the reaction mixture was stirred at 25°C for 16 h. After the reaction was complete, sodium sulfite solid (1.5 g) was added to the reaction mixture, which was stirred for 10 min, concentrated under reduced pressure to remove a certain amount of acetone, poured into water (60 mL), and extracted with ethyl acetate (80 mL×4). The organic phases were combined, dried over anhydrous sodium sulfate, and concentrated under reduced pressure to give 5'-(hydroxymethyl)-2'-morpholinyl-5',6'-dihydrospiro[cyclobutane-1,8'-pyrano[3,4-b]pyridine]-5'-ol (2.1 g, crude product).

LCMS (ESI): 307.17 [M+H]⁺

Step 6: At room temperature, 5'-(hydroxymethyl)-2'-morpholinyl-5',6'-dihydrospiro[cyclobutane-1,8'-pyrano[3,4-b]pyridine]-5'-alcohol (2.1 g, 6.9 mmol) was dissolved in a mixed solvent of tetrahydrofuran (20 mL) and water (0.8 mL). Sodium periodate (4.5 g, 20.7 mmol) was added to the reaction mixture, which was stirred at 25°C for 4 h under nitrogen atmosphere. After the reaction was complete, the reaction mixture was diluted with ethyl acetate, filtered, and washed. The filtrate was combined, dried over anhydrous sodium sulfate, filtered, and concentrated under reduced pressure to obtain a crude product, which was purified by column chromatography (petroleum ether: ethyl acetate = 2:1) to give 2'-morpholino-spiro[cyclobutane-1,8'-pyrano[3,4-b]pyridine]-5'(6H)-one (970 mg, two-step yield: 53%) as a pale yellow solid.

LCMS (ESI): 275.2 [M+H]⁺

Step 7: At room temperature, 2'-morpholino-spiro[cyclobutane-1,8'-pyrano[3,4-b]pyridine]-5'(6H)-one (200 mg, 0.73 mmol) was dissolved in 1,2-dichloroethane (5 mL), and tetraethoxy titanium (410 mg, 1.8 mmol) and methylamine tetrahydrofuran solution (2 mL, 30%) were added. The mixture was stirred for reaction at room temperature for 16 h, then sodium borohydride (42 mg, 1.1 mmol) was added, and the mixture was further stirred for reaction at room temperature for 8 h. After LCMS indicated the reaction was complete, water (10 mL) was added, and the mixture was extracted with ethyl acetate (20 mL×2). The organic phases were combined, dried over anhydrous sodium sulfate, and filtered. The filtrate was concentrated under reduced pressure and purified by column chromatography (50-100% ethyl acetate/petroleum ether) to give N-methyl-2'-morpholinyl-5',6'-dihydrospiro[cyclobutane-1,8'-pyrano[3,4-b]pyridine]-5'-amine (80 mg, yield: 37.9%).

LCMS (ESI) m/z: 290.2 [M+H]⁺

Employing the preparation method and steps of **intermediate 4,** with the sole replacement of the corresponding raw material intermediate, the following intermediate was obtained:

| Intermediate No. | Chemical Structure | Chemical Name | m/z(ESI): (M+H) ⁺ |
|---|---|---|---|
| 5 | 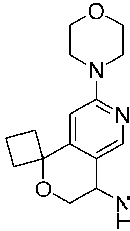 | *N*-methyl-7'-morpholino-3',4'-dihydrospiro[cyclobutane-1,1'-pyrano[4,3-*c*]pyridin]4'-amine | 290.2 |

### Intermediate 6 N,9,9-trimethyl-6,9-dihydro-7H-[1,3]dioxolo[4,5-h]isochroman-6-amine

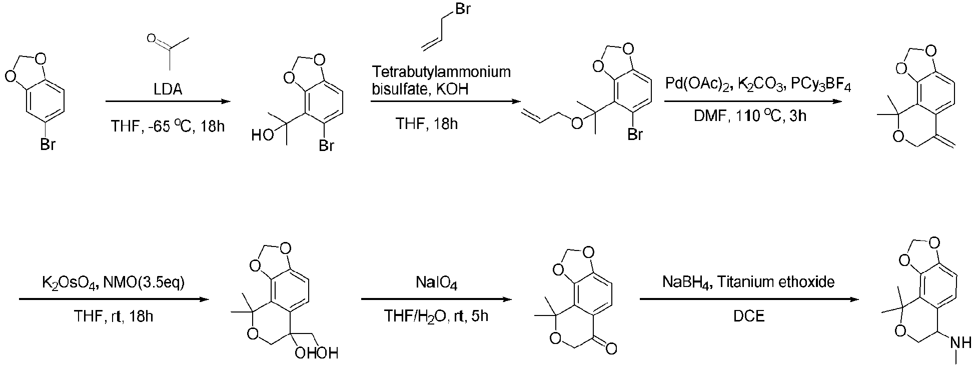

Step 1: At room temperature, 5-bromobenzo[d][1,3]dioxazole (5.0 g, 24.87 mmol) was dissolved in tetrahydrofuran (50 mL). The mixture was purged with nitrogen three times. Lithium diisopropylamide (1.68 mL, 2 M, 27.36 mmol) was slowly added dropwise into the system at -65°C, and the mixture was stirred for 2 h at -65°C. Then, acetone (1.59 g, 27.36 mmol) was slowly dropped into the above reaction mixture, which was stirred at -65°C for 1 h and gradually warmed to room temperature for reaction for 16 h. When the reaction was complete, the reaction mixture was quenched with ammonium chloride solution (50 mL), poured into water (100 mL), and extracted with ethyl acetate (100 mL×3). The organic phases were combined, dried over anhydrous sodium sulfate, and filtered. The filtrate was concentrated under reduced pressure to obtain a crude product, which was purified by column chromatography (petroleum ether: ethyl acetate = 1:0 to 10:1) to give 2-(5-bromobenzo[d][1,3]dihydroxy-4-yl)propan-2-ol (3.8 g, yield: 58.96%).

¹H NMR (400 MHz, CDCl₃) δ 7.09-7.08 (d, 1H, *J* = 7.08), 6.58-6.56 (d, 1H, *J* = 6.57), 5.92 (s, 2H), 1.74 (s, 6H).

Step 2: At room temperature, 2-(5-bromobenzo[d][1,3]dihydroxy-4-yl)propan-2-ol (4 g, 15.44 mmol) was dissolved in tetrahydrofuran (50 mL). Potassium hydroxide (1.73 g, 30.88 mmol), tetrabutylammonium hydrogen sulfate (1.05 g, 3.09 mmol), and 3-bromopropene (2.24 g, 18.53 mmol) were added, and the mixture was stirred at 80°C for 16 h. After the reaction was complete, the reaction mixture was poured into water (100 mL) and extracted with ethyl acetate (60 mL×3). The organic phases were combined, dried over anhydrous sodium sulfate, and filtered. The filtrate was concentrated under reduced pressure to obtain a crude product, which was purified by column chromatography(petroleum ether: ethyl acetate = 10:1) to give 4-(2-(allyloxy)prop-2-yl)-5-bromobenzo[d][1,3]dioxazole (1.5 g, yield: 32.48%).

¹H NMR (400 MHz, CDCl₃) δ 7.15-7.13 (d, 1H, *J* = 7.14), 6.60-6.58 (d, 1H, *J* = 6.59), 6.04-5.96 (m, 1H), 5.91 (s, 2H), 5.29-5.23 (dd, 1H, *J* = 5.26), 5.14-5.10 (dd, 1H, *J* = 5.12), 3.76-3.74 (td, 2H, *J* = 3.75), 1.73 (s, 6H).

Step 3: At room temperature, potassium carbonate (92 mg, 0.67 mmol), tricyclohexylphosphine tetrafluoroborate (12 mg, 0.03 mmol), and palladium acetate (8 mg, 0.03mmol) were added to a solution of 4-(2-(allyloxy)prop-2-yl)-5-bromobenzo[d][1,3]dioxazole (100 mg, 0.33 mmol) in *N,N*-dimethylformamide (3 mL). The mixture was stirred at 110°C for 3 h under nitrogen atmosphere. After the reaction was complete, the reaction mixture was poured into 200 mL of water and extracted with ethyl acetate (100 mL×3). The organic phases were combined, dried over anhydrous sodium sulfate, and concentrated under reduced pressure to give a crude product, which was purified by column chromatography(petroleum ether: ethyl acetate = 10:1) to give 9,9-dimethyl-6-methylene-6,9-dihydro-7H-[1,3]dioxolo[4,5-h]isochroman(35 mg, yield: 47.98%).

¹H NMR (400 MHz, CDCl₃) δ 7.17-7.15 (d, 1H, *J* = 7.16), 6.74-6.72 (d, 1H, *J* = 6.73), 5.96 (s, 2H), 5.42 (s, 1H), 4.92 (s, 1H), 4.33 (s, 2H), 1.59 (s, 6H).

Step 4: At room temperature, 9,9-dimethyl-6-methylene-6,9-dihydro-7H-[1,3]dioxolo[4,5-h]isochroman (0.4 g, 1.83 mmol) was dissolved in a mixed solvent of tetrahydrofuran (6 mL) and water (2 mL), and N-methylmorpholine-*N*-oxide (0.64 g, 5.5 mmol) and potassium osmate (0.057 g, 0.18 mmol) were added. Under nitrogen atmosphere, the reaction was stirred at 25°C for 16 h. After the reaction was complete, sodium sulfite solid (0.3 g) was added to the reaction mixture, which was stirred for 10 min, concentrated under reduced pressure to remove a certain amount of tetrahydrofuran, poured into water (10 mL), and extracted with ethyl acetate (80 mL×4). The organic phases were combined, dried over anhydrous sodium sulfate, and concentrated under reduced pressure to give a crude product 6-(hydroxymethyl)-9,9-dimethyl-6,9-dihydro-7H-[1,3]dioxolo[4,5-h]isochroman-6-ol (0.38 g).

LCMS (ESI): [M-H]⁺ 251.10/251.07.=

Step 5: At room temperature, 6-(hydroxymethyl)-9,9-dimethyl-6,9-dihydro-7H-[1,3]dioxolo[4,5-h]isochroman-6-ol (0.38 g, 1.51 mmol) was dissolved in a mixed solvent of tetrahydrofuran (15 mL) and water (1.5 mL), and sodium periodate (0.97 g, 4.52 mmol) was added. Under nitrogen atmosphere, the reaction was stirred at 25°C for 4 h. After the reaction was complete, the reaction mixture was diluted with ethyl acetate, filtered, and washed. The filtrate was combined, dried over anhydrous sodium sulfate, filtered, and concentrated under reduced pressure to obtain a crude product, which was purified by column chromatography(petroleum ether: ethyl acetate = 2:1) to give 9,9-dimethyl-7H-[1,3]dioxino[4,5-h]isochroman-6(9H)-one (0.3 g, yield: 90.44%).

¹H NMR (400 MHz, CDCl₃) δ 7.71-7.69 (d, 1H, *J* = 7.70), 6.85-6.83 (d, 1H, *J* = 6.84), 6.07 (s, 2H), 4.36 (s, 2H), 1.66 (s, 6H).

Step 6: At room temperature, 9,9-dimethyl-7H-[1,3]dioxino[4,5-h]isochroman-6(9H)-one (0.27 g, 1.22 mmol) and methylamine methanol solution (0.63 g, 6.13 mmol) were dissolved in a mixed solvent of 1,2-dichloroethane (12 mL), and tetraethyl titanate (0.56 g, 2.45 mmol) was added. The mixture was stirred and reacted at 50°C for 2 h. After the reaction was complete, the mixture was cooled to 0°C, and sodium borohydride (0.07 g, 1.84 mmol) was added. The mixture was stirred at 0°C for 0.5 h. After the reaction was complete, saturated ammonium chloride solution (40 mL) was added, and the mixture was extracted with dichloromethane (30 mL×4). The organic phases were combined, dried over anhydrous sodium sulfate, and filtered. The filtrate was concentrated under reduced pressure to obtain a crude product, which was purified by column chromatography(petroleum ether: ethyl acetate = 2:1) to give *N*,9,9-trimethyl-6,9-dihydro-7H-[1,3]dioxolo[4,5-*h*]isochroman-6-amine (0.1 g, yield: 34.67%).

LCMS (ESI): [M-H]⁺ 236.21

Employing the preparation method and steps of **intermediate 6,** with the sole replacement of the corresponding raw material intermediate, the following intermediate was obtained:

| Intermediate No. | Chemical Structure | Chemical Name | m/z(ESI): (M+H)⁺ |
|---|---|---|---|
| 7 | 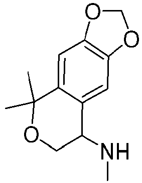 | *N*,5,5-trimethyl-7,8-dihydro-5*H*-[1,3]dioxolo[4,5-g]isochroman-8-amine | 236.21 |

### Intermediate 8 Synthesis of N-methyl-7'-(trifluoromethyl)spiro[cyclobutane-1,1'-isochroman]-4'-amine

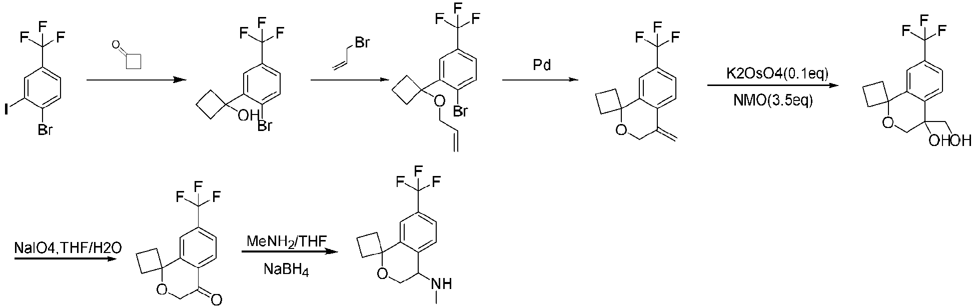

### Step 1: Synthesis of 1-(2-bromo-5-(trifluoromethyl)phenyl)cyclobutan-1-ol

At room temperature, 2-bromo-5-(trifluoromethyl)benzaldehyde (10 g, 39.52 mmol) was dissolved in tetrahydrofuran (100 mL). At 0°C, a 3 M methylmagnesium chloride tetrahydrofuran solution (20 mL, 59.28 mmol) was slowly added dropwise into the system. The mixture was stirred at 0°C for 2 hours. After the reaction was complete, it was quenched with ammonium chloride solution. The reaction mixture was poured into water (200 mL) and extracted with ethyl acetate (100 mL×3). The combined organic layer was dried over anhydrous sodium sulfate and filtered. The filtrate was concentrated under reduced pressure to give 1-(2-bromo-5-(trifluoromethyl)phenyl)cyclobutan-1-ol (10 g, crude), which was used directly in the next step without purification.

LCMS (ESI) m/z:295.1[M-OH]⁺

### Step 2: Synthesis of 2-[1-(allyloxy)cyclobutyl]-1-bromo-4-(trifluoromethyl)benzene

At room temperature, 1-(2-bromo-5-(trifluoromethyl)phenyl)cyclobutan-1-ol (7 g, 23.80 mmol) was dissolved in tetrahydrofuran (100 mL). Potassium hydroxide (2.69 g, 47.60 mmol), tetrabutylammonium hydrogen sulfate (1.67 g, 4.760 mmol) and 3-bromopropene (4.3 g, 35.70mmol) were added. The mixture was stirredat 25°C for 16 hours. After the reaction was complete, the reaction mixture was concentrated under reduced pressure, then poured into water (100 mL), and extracted with ethyl acetate (100 mL×3). The combined organic layer was dried over anhydrous sodium sulfate and filtered. The filtrate was concentrated under reduced pressure to give a crude product. The crude product was purified by column chromatography (petroleum ether: ethyl acetate = 10:1) to give 2-[1-(allyloxy)cyclobutyl]-1-bromo-4-(trifluoromethyl)benzene (4.8 g, yield: 60.3%).

LCMS (ESI) m/z: 335.1 [M+H]⁺

### Step 3: Synthesis of 4'-methylene-7'-(trifluoromethyl)spiro[cyclobutane-1,1'-isochromane]

At room temperature, N,N-diisopropylethylamine (5.57 g, 43.11 mmol), 4,5-bis(diphenylphosphino)-9,9-dimethylxanthene (1.67 g, 2.874 mmol) and palladium (II) acetate (0.325 g, 1.437mmol) were added to a solution of 2-[1-(allyloxy)cyclobutyl]-1-bromo-4-(trifluoromethyl)benzene (4.8 g, 14.37 mmol) in N,N-dimethylformamide (100 mL). The mixture was stirred at 100°C for 16 hours under nitrogen atmosphere. The reaction mixture was poured into water (400 mL) and extracted with ethyl acetate (100 mL×3). The combined organic layer was dried over anhydrous sodium sulfate and concentrated under reduced pressure to give a crude product. The crude product was purified by column chromatography (petroleum ether: ethyl acetate = 10:1) to give 4'-methylene-7'-(trifluoromethyl)spiro[cyclobutane-1,1'-isochromane] (2.7 g, yield: 73.9%).

LCMS (ESI) m/z: 255.1 [M+H]⁺

### Step 4: Synthesis of 4'-(hydroxymethyl)-7'-(trifluoromethyl)spiro[cyclobutane-1,1'-isochroman]-4'-ol

At room temperature, 4'-methylene-7'-(trifluoromethyl)spiro[cyclobutane-1,1'-isochromane] (2.7 g, 10.62 mmol) was dissolved in a mixed solvent of acetone (60 mL) and water (20 mL). *N*-methylmorpholine-*N-*oxide (3.9 g, 31.86 mmol) and potassium osmate (0.41 g, 1.062 mmol) were added. Under nitrogen atmosphere, the mixture was stirred at 25°C for 16 hours. After the reaction was complete, sodium sulfite solid (5 g) was added to the reaction mixture, then stirred for 10 minutes, concentrated under reduced pressure to remove a certain amount of acetone, poured into water (200 mL), and extracted with ethyl acetate (100 mL×3). The combined organic layer was dried over anhydrous sodium sulfate and concentrated under reduced pressure to give 4'-(hydroxymethyl)-7'-(trifluoromethyl)spiro[cyclobutane-1,1'-isochroman]-4'-ol (2.1 g crude, yield: 68.67%).

LCMS (ESI) m/z: 289.0 [M+H]⁺

### Step 5: Synthesis of 7'-(trifluoromethyl)spiro[cyclobutane-1,1'-isochroman]-4'-one

At room temperature, 4'-(hydroxymethyl)-7'-(trifluoromethyl)spiro[cyclobutane-1,1'-isochroman]-4'-ol (2.1 g, 7.29 mmol) was dissolved in a mixed solvent of tetrahydrofuran (50 mL) and water (2 mL). Sodium periodate (6.37 g, 29.16 mmol) was added. Under nitrogen atmosphere, the mixture was stirred at 25°C for 4 hours. After the reaction was complete, the reaction mixture was diluted with ethyl acetate, filtered and washed. The combined filtrate was dried over anhydrous sodium sulfate and filtered. The filtrate was concentrated under reduced pressure to give a crude product. The crude product was purified by column chromatography (petroleum ether: ethyl acetate = 5:1) to give 7'-(trifluoromethyl)spiro[cyclobutane-1,1'-isochroman]-4'-one (0.9 g, yield: 48.23%).

LCMS (ESI) m/z:257.1 [M+H]⁺

### Step 6: Preparation of N-methyl-7'-(trifluoromethyl)spiro[cyclobutane-1,1'-isochroman]-4'-amine

At room temperature, compound 7'-(trifluoromethyl)spiro[cyclobutane-1,1'-isochroman]-4'-one (500 mg, 1.953 mmol) was dissolved in 1,2-dichloroethane (5 mL). Titanium tetraethoxide (1115 mg, 4.88 mmol) and methylamine tetrahydrofuran solution (2 mL, 30%) were added. The mixture was stirred at room temperature for 16 hours, then sodium borohydride (115 mg, 2.928 mmol) was added, and the mixture was further stirred for reaction at room temperature for 8 hours. After LCMS indicated the reaction was complete, water (10 mL) was added, and the mixture was extracted with ethyl acetate (20 mL×2). The combined organic layer was dried over anhydrous sodium sulfate and filtered. The filtrate was concentrated under reduced pressure and purified by column chromatography (50-100% ethyl acetate/petroleum ether) to give N-methyl-7'-(trifluoromethyl)spiro[cyclobutane-1,1'-isochroman]-4'-amine (150 mg, yield: 28.33%).

LCMS (ESI) m/z: 272.1 [M+H]⁺

Employing the preparation method and steps of **intermediate 8,** with the sole replacement of the corresponding raw material intermediate, the following intermediates were obtained:

| Intermediate No. | Chemical Structure | Chemical Name | m/z(E SI): (M+H )⁺ |
|---|---|---|---|
| 9 | 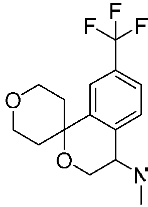 | *N*-methyl-7-trifluoromethyl-2',3',5',6'-tetrahydrospiro[isochroman-1,4'-pyran]-4-amine | 302.1 |
| 10 | 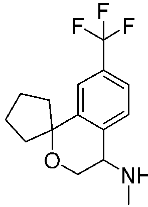 | *N*-methyl-7'-(trifluoromethyl)spiro[cyclopentane-1,1'-isochroman]-4'-amine | 286.1 |
| 11 | 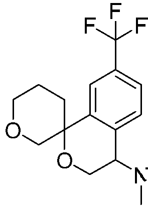 | *N*-methyl-7-(trifluoromethyl)-5',6'-dihydro-2'H,4'H-spiro[isochroman-1,3'-pyran]-4-amine | 302.1 |
| 12 | 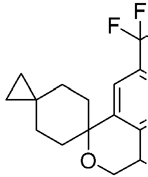 | N-methyl-7"-(trifluoromethyl)dispiro[cyclopropane-1,1'-cyclohexane-4',1"-isochroman]-4"-amine | 326.2 |
| 13 | 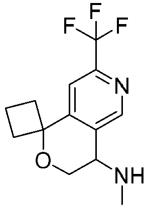 | N-methyl-7'-(trifluoromethyl)-3',4'-dihydrospiro[cyclobutane-1,1'-pyrano[4,3-c]pyridine]-4'-amine | 273.2 |
| 14 | 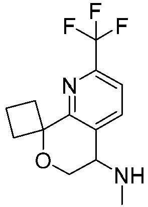 | N-methyl-2'-(trifluoromethyl)-5',6'-dihydrospiro[cyclobutane-1,8'-pyrano[3,4-b]pyridine]-5'-amine | 273.2 |
| 15 | 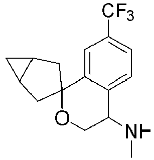 | N-methyl-7'-(trifluoromethyl)spiro[bicyclo[3.1.0]hexane-3,1'-isochroman]-4'-amine | 298.2 |
| 16 | 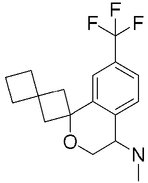 | N-methyl-7"-(trifluoromethyl)dispiro[cyclobutane-1,1'-cyclobutane-3',1"-isochroman]-4"-amine | 312.2 |

### Intermediate 17 N-methyl-7'-(trifluoromethyl)spiro[cyclopropane-1,1'-isochroman]-4'-amine

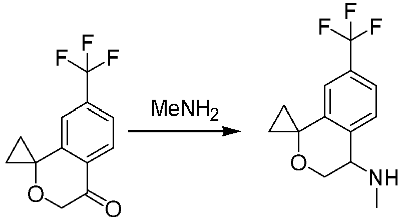

At room temperature, 7-(trifluoromethyl)spiro[cyclopropane-1,1'-isochroman]-4'-one (160 mg, 0.6606 mmol) was dissolved in 1,2-dichloroethane (20 mL), and titanium (IV) ethoxide (0.38 g, 1.65 mmol) and methylamine tetrahydrofuran solution (2 mL) were added. After the reaction mixture was stirred at room temperature for 16 h, sodium borohydride (51 mg, 1.321 mmol) was added, and the mixture was stirred for another 20 min. After the reaction was complete, the reaction mixture was diluted with ethyl acetate, filtered, and washed. The filtrate was combined, dried over anhydrous sodium sulfate, filtered, and concentrated under reduced pressure to obtain a crude product, which was purified by column chromatography (dichloromethane: methanol = 10:1) to give N-methyl-7'-(trifluoromethyl)spiro[cyclopropane-1,1'-isochroman]-4'-amine (60 mg, yield: 35%).

LCMS (ESI) m/z:258.2 [M+H]⁺

Employing the preparation method and steps of **intermediate 17,** with the sole replacement of the corresponding raw material intermediate, the following intermediates were obtained:

| Intermediate No. | Chemical Structure | Chemical Name | m/z(ESI):(M+H)⁺ |
|---|---|---|---|
| 61 | 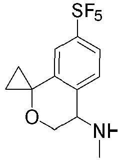 | 7'-(pentafluoro- λ ⁶-sulfanyl)spiro[cyclopropane-1,1'-isochroman]-4'-amine | 236.21 |
| 62 | 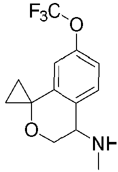 | 7'-(trifluoromethoxy)spiro[cyclopropane-1,1'-isochroman]-4'-amine | 274.2 |
| 63 | 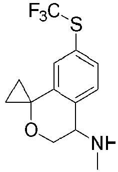 | 7'-(trifluoromethyl)thio)spiro[cyclopropane-1,1'-isochroman]-4'-amine | 290.2 |
| 64 | 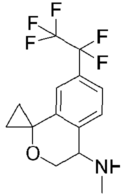 | 7'-(perfluoroethyl)spiro[cyclopropane-1,1'-isochroman]-4'-amine | 308.2 |
| 65 | 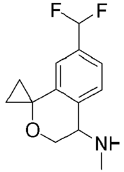 | 7'-(difluoromethyl)spiro[cyclopropane-1,1'-isochroman]-4'-amine | 240.2 |
| 66 | 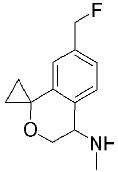 | 7'-(fluoromethyl)spiro[cyclopropane-1,1'-isochroman]-4'-amine | 222.2 |
| 67 | 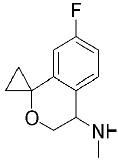 | 7'-fluoro-spiro[cyclopropane-1,1'-isochroman]-4'-amine | 208.2 |
| 68 | 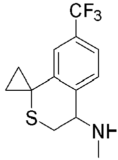 | N-methyl-7'-(trifluoromethyl)spiro[cyclopropane-1,1'-thioisochroman]-4'-amine | 274.2 |
| 84 | 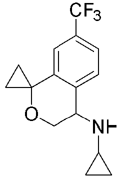 | N-cyclopropyl-7'-(trifluoromethyl)spiro[cyclopropane-1,1'-isochroman]-4'-amine | 284.2 |

### Intermediate 18 Tert-butyl (S)-(7'-chloro-3'-,4'-dihydrospiro[cyclobutane-1,1'-pyrano[4,3-c]pyridine]-4'-yl)(methyl)carbamate

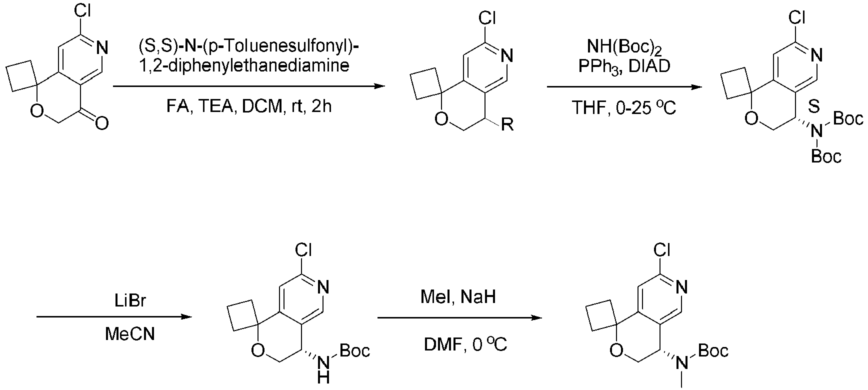

Step 1: At 0°C, formic acid (0.62 g, 13.55 mmol) was added to a solution of triethylamine (1.17 g, 11.56 mmol) in dichloromethane (25 mL), followed by 7'-chlorospiro[cyclobutane-1,1'-pyrano[4,3-c]pyridine]-4'(3'H)-one (1 g, 4.47 mmol) and (S,S)-N-(p-toluenesulfonyl)-1,2-diphenylethanediamine (p-isopropylbenzene)ruthenium chloride (338 mg, 0.65 mmol). The mixture was stirred at 25°C for 2 h. After the reaction was complete, the reaction mixture was poured into 50 mL of water, and extracted with ethyl acetate (50 mL×3). The organic phases were combined, dried over anhydrous sodium sulfate, and concentrated under reduced pressure to give a crude product, which was purified by column chromatography(petroleum ether: ethyl acetate = 3:1) to give (R)-7'-chloro-3'-,4'-dihydrospiro[cyclobutane-1,1'-pyrano[4,3-c]pyridine]-4'-ol (0.9 g, yield: 90%).

LCMS (ESI) m/z: [M+H]⁺ = 226.12.

Step 2: At room temperature, (R)-7'-chloro-3'-,4'-dihydrospiro[cyclobutane-1,1'-pyrano[4,3-c]pyridine]-4'-ol (1 g, 4.4 mmol) was dissolved in tetrahydrofuran (40 mL), and triphenylphosphine (1.4 g, 5.32 mmol) and di-tert-butyl iminodicarboxylate (1. 16 g, 5.36 mmol) were added. The mixture was stirred at 0°C for 10 min under nitrogen atmosphere. Diisopropyl azodicarboxylate (1.07 g, 5.30 mmol) was slowly added dropwise to the reaction system, and the mixture was reacted at 25°C for 16 h. After the reaction was complete, the reaction mixture was poured into water (50 mL) and extracted with ethyl acetate (30 mL×3). The organic phases were combined, dried over anhydrous sodium sulfate, and filtered. The filtrate was concentrated under reduced pressure to obtain a crude product, which was purified by silica gel column chromatography (petroleum ether: ethyl acetate = 4:1) to give tert-butyl (S)-(7'-chloro-3',4'-dihydrospiro[cyclobutane-1,1'-pyrano[4,3-c]pyridine]-4'-yl)carbamate (0.77 g, yield: 40.7%).

LCMS (ESI) m/z: [M+H]⁺ =425.26.

Step 3: At room temperature, tert-butyl (S)-(7'-chloro-3',4'-dihydrospiro[cyclobutane-1,1'-pyrano[4,3-c]pyridine]-4'-yl)carbamate (1 g, 2.23 mmol) was dissolved in acetonitrile (16 mL), and lithium bromide (1 g, 11.56 mmol) was added. The mixture was stirred at 60°C for 16 h. After the reaction was complete, the reaction mixture was concentrated under reduced pressure, poured into water (50 mL), and extracted with ethyl acetate (50 mL×3). The organic phases were combined, dried over anhydrous sodium sulfate, and filtered. The filtrate was concentrated under reduced pressure to obtain the crude product tert-butyl (S)-(7'-chloro-3'-,4'-dihydrospiro[cyclobutane-1,1'-pyrano[4,3-c]pyridine]-4'-yl)carbamate (0.76 g, yield: 95%).

LCMS (ESI) m/z: [M+H]⁺ = 325.22.

Step 4: Under nitrogen atmosphere, tert-butyl (S)-(7'-chloro-3'-,4'-dihydrospiro[cyclobutane-1,1'-pyrano[4,3-c]pyridine]-4'-yl)carbamate (0.76 g, 2.36 mmol) was dissolved in *N,N*-dimethylformamide (30 mL) at room temperature. The mixture was cooled to 0°C, and 60% sodium hydride (190 mg, 4.7 mmol) was added. After stirring for 30 min, the reaction mixture of iodomethane (502 mg, 3.52 mmol) was added and stirred at room temperature for 4 h. After the reaction was complete, the reaction mixture was poured into water (40 mL) and extracted with ethyl acetate (30 mL×3). The organic phases were combined, dried over anhydrous sodium sulfate, and filtered. The filtrate was concentrated under reduced pressure to obtain a crude product, which was subject to column chromatography (petroleum ether: ethyl acetate = 3:1) to give tert-butyl (S)-(7'-chloro-3'-,4'-dihydrospiro[cyclobutane-1,1'-pyrano[4,3-c]pyridine]-4'-yl)(methyl)carbamate (0.5 g, yield: 62.5%).

LCMS (ESI) m/z: [M+H]⁺ = 339.14/339.24.

Employing the preparation method and steps of intermediate **18,** with the sole replacement of the corresponding raw material intermediate, the following intermediates were obtained:

| Intermediate No. | Chemical Structure | Chemical Name | m/z(ES I): (M+H) ⁺ |
|---|---|---|---|
| 19 | 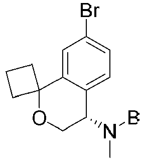 | Tert-butyl (S)-(7'-bromospiro[cyclobutane-1,1'-isochroman]-4'-yl)(methyl)carbamate | 383.2 |
| 20 | 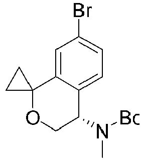 | Tert-butyl (S)-(7'-bromospiro[cyclopropane-1,1'-isochroman]-4'-yl)(methyl)carbamate | 369.2 |
| 21 | 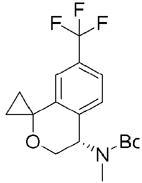 | Tert-butyl (S)-methyl (7'-(trifluoromethyl)spiro[cyclopropane-1,1'-isochroman]-4'-yl)carbamate | 358.2 |
| 69 | 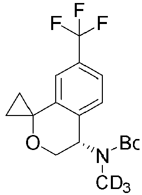 | Tert-butyl (S)-(methyl-*d*₃)(7'-(trifluoromethyl)spiro[cyclopropane-1,1'-isochroman]-4'-yl)carbamate | 361.3 |
| 70 | 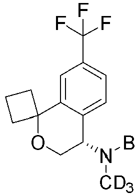 | Tert-butyl (S)-(methyl-*d*₃)(7'-(trifluoromethyl)spiro[cyclobutane-1,1'-isochroman]-4'-yl)carbamate | 375.2 |
| 71 | 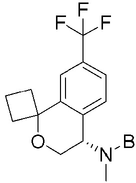 | Tert-butyl (S)-methyl (7'-(trifluoromethyl)spiro[cyclobutane-1,1'-isochroman]-4'-yl)carbamate | 372.2 |

### Intermediate 22 (4'S)-7'-(3-oxa-6-azabicyclo[3.1.1]heptane-6-yl)-N-methyl-3',4'-dihydrospiro[cyclobutane-1,1'-pyrano[4,3-c]pyridine]-4'-amine

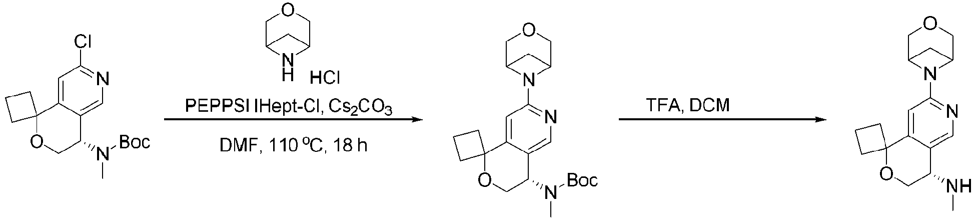

Step 1: Tert-butyl (S)-(7'-chloro-3',4'-dihydrospiro[cyclobutane-1,1'-pyrano[4,3-c]pyridine]-4'-yl)(methyl)carbamate (300 mg, 0.89 mmol) and 3-oxa-6-azabicyclo[3.1.1]heptane hydrochloride (132 mg, 1.33 mmol) were dissolved in *N,N*-dimethylformamide (5.0 mL). Cesium carbonate (865 mg, 2.66 mmol) and (SP-4-1)-[1,3-bis[2,6-bis(1-propylbutyl)phenyl]-4,5-dichloro-1,3-dihydro-2H-imidazol-2-ylidene]dichloro(3-chloropyridine-KN)-palladium (86 mg, 0.09 mmol) were then added, and the mixture was stirred at 110°C for 18 h under nitrogen atmosphere. Monitoring by LCMS showed about 80% of the product. The reaction mixture was cooled to room temperature and filtered. The filtrate was diluted with ethyl acetate (50 mL), washed with water (3×25 mL), washed with saturated brine (25 mL), dried over anhydrous sodium sulfate, filtered, concentrated and purified by column chromatography to give tert-butyl ((4'S)-7'-(3-oxa-6-azabicyclo[3.1.1]heptane-6-yl)-3',4'-dihydrospiro[cyclobutane-1,1'-pyrano[4,3-c]pyridine]-4'-yl)(methyl)carbamate (300 mg, yield: 84%) as a yellow solid.

LCMS (ESI): [M+H]⁺ m/z = 402.40.

Step 2: To a solution of tert-butyl ((4'S)-7'-(3-oxa-6-azabicyclo[3.1.1]heptane-6-yl)-3',4'-dihydrospiro[cyclobutane-1,1'-pyrano[4,3-c]pyridine]-4'-yl)(methyl)carbamate (300 mg, 0.75 mmol) in dichloromethane (12.0 mL) was added trifluoroacetic acid (3.0 mL). After the addition, the mixture was stirred at room temperature for 1 h. Monitoring by LCMS showed about 90% of the product. The reaction mixture was concentrated and purified by reversed phase column (A: 0.5% formic acid in water; B: acetonitrile; gradient: B 5→10% over 10 min) and freeze-dried to give (4'S)-7'-(3-oxa-6-azabicyclo[3.1.1]heptane-6-yl)-N-methyl-3',4'-dihydrospiro[cyclobutane-1,1'-pyrano[4,3-c]pyridine]-4'-amine (200 mg, yield: 89%) as a pale yellow solid.

LCMS (ESI): [M+H-Me NH]⁺ m/z =271.27.

Employing the preparation method and steps of intermediate **22,** with the sole replacement of the corresponding raw material intermediate, the following intermediates were obtained:

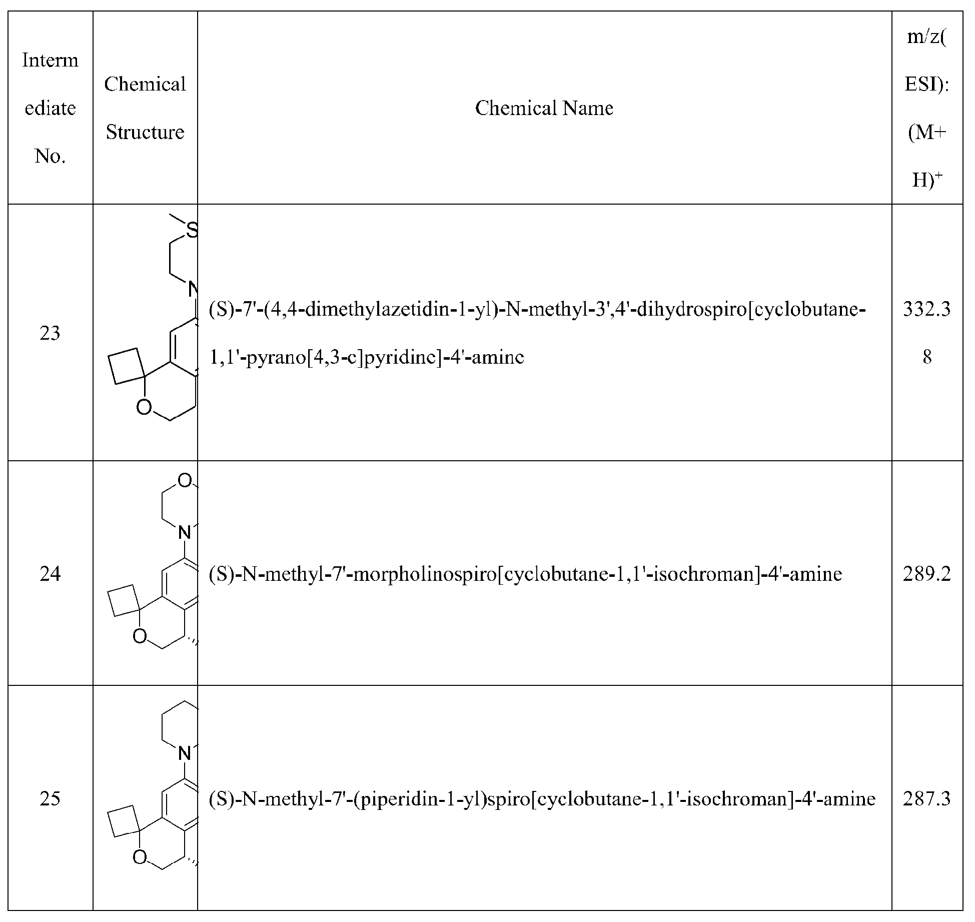

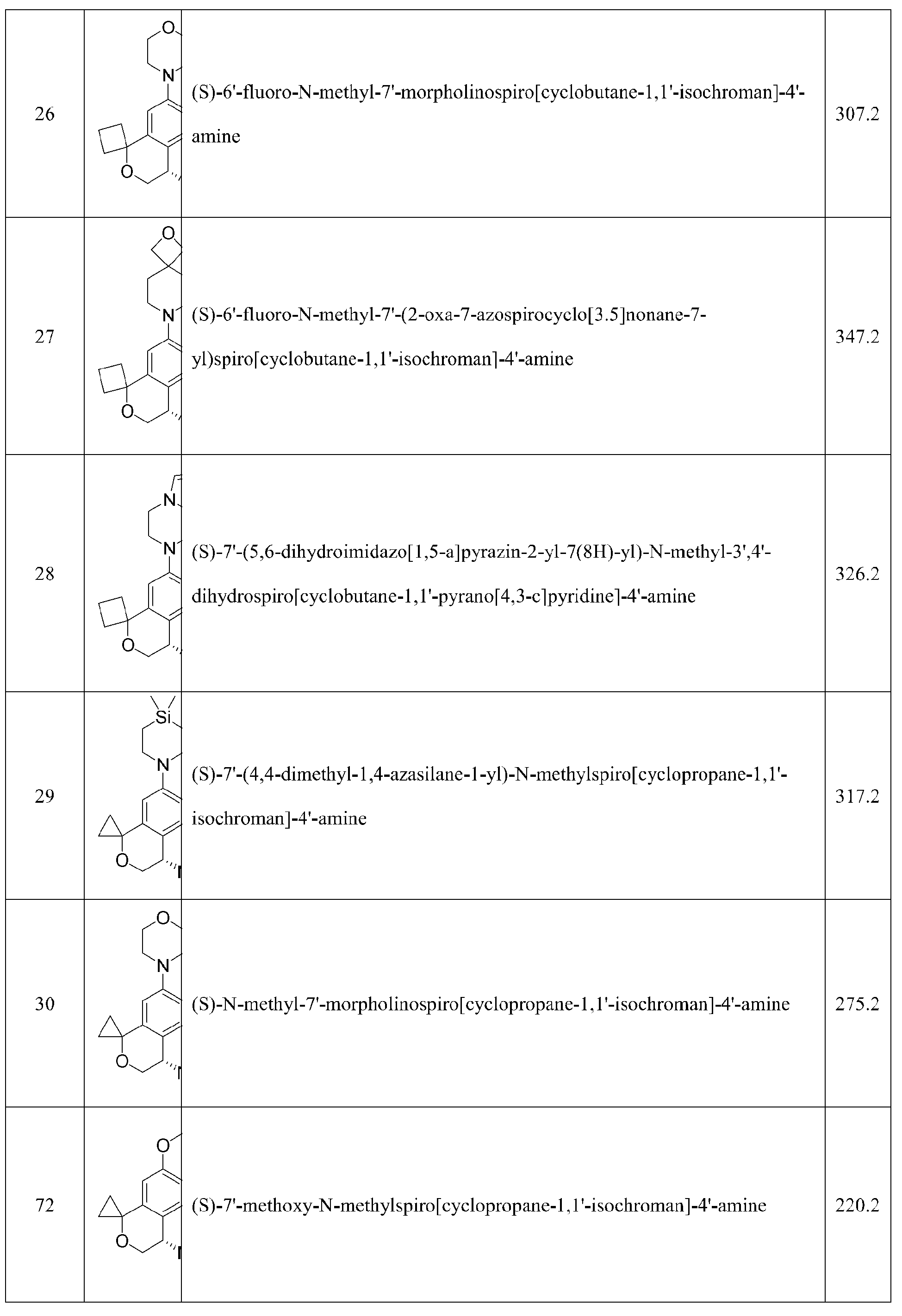

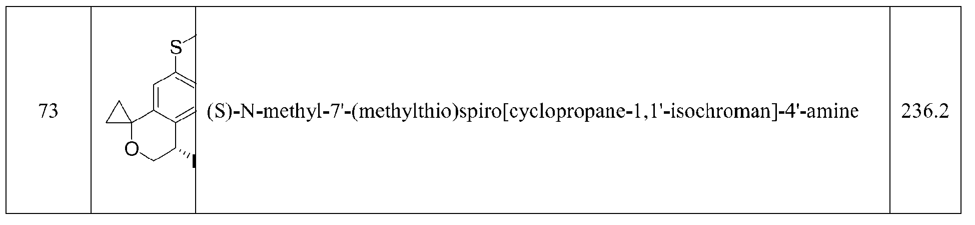

### Intermediate 31 (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate

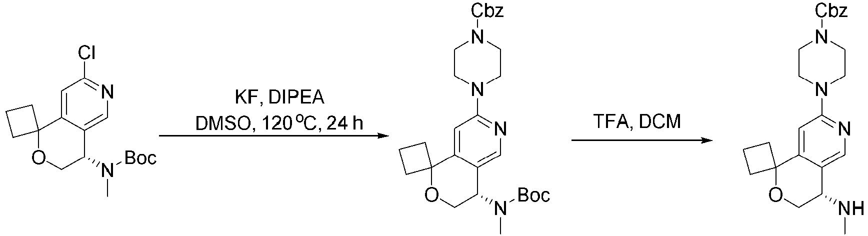

Step 1: Tert-butyl (S)-(7'-chloro-3'-,4'-dihydrospiro[cyclobutane-1,1'-pyrano[4,3-c]pyridine]-4'-yl)(methyl)carbamate (500 mg, 1.48 mmol) was dissolved in dimethyl sulfoxide (1.0 mL). Benzyl piperazine-1-carboxylate (975 mg, 4.43 mmol), potassium fluoride (87 mg, 1.48 mmol), and N,N-diisopropylethylamine (583 mg, 4.43 mmol) were added, and the mixture was stirred at 120°C for 48 h. After the reaction was complete, the reaction mixture was poured into water (40 mL) and extracted with ethyl acetate (60 mL×3). The organic phases were combined, dried over anhydrous sodium sulfate, and filtered. The filtrate was concentrated under reduced pressure to obtain a crude product, which was purified by column chromatography(petroleum ether: ethyl acetate = 3:1) to give benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (0.5 g, yield: 78.4%).

LCMS (ESI) m/z: [M+H]⁺ = 523.33.

Step 2: Benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (500 mg, 0.89 mmol) was dissolved in dichloromethane (3.00 mL). Trifluoroacetic acid (1.00 mL) was added, and the mixture was stirred at room temperature for 1 h. After the reaction was complete, the solvent was removed under vacuum and rotary-evaporated with an oil pump for 10 min. The crude product was directly used in the next step without purification to give benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (0.4 g, yield: 99.0%).

LCMS (ESI) m/z: [M+H]⁺ = 392.35.

Employing the preparation method and steps of intermediate **31,** with the sole replacement of the corresponding raw material intermediate, the following intermediates were obtained:

| Intermediate No. | Chemical Structure | Chemical Name | m/z(ESI): (M+H)⁺ |
|---|---|---|---|
| 32 | 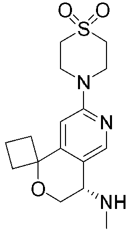 | (S)-4-(4'-(methylamino)-3',4'-dihydrospiro[cyclobutane-1,1'-pyrano[4,3-c]pyridine]-7'-yl)thiomorpholine 1,1-dioxide | 338.2 |
| 33 | 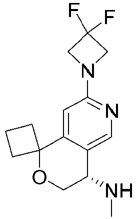 | (S)-1'-(3,3-difluoroazetidin-1-yl)-N-methyl-3',4'-dihydrospiro[cyclobutane-1,1'-pyrano[4,3-c]pyridine]-4'-amine | 296.2 |

### Intermediate 34 (S)-N-methyl-7'-(3,3,3-trifluoropropyl)prop-1-yn-1-yl)spirolcyclopropane-1,1'-isochroman]-4'-amine

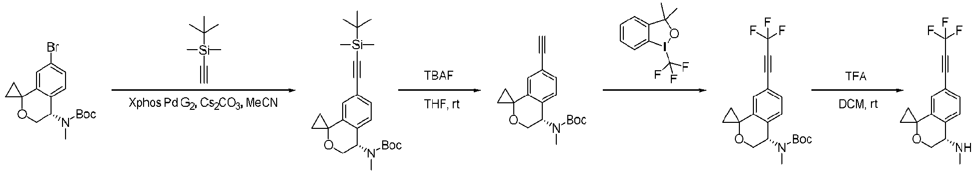

Employing the preparation method and steps of intermediate **34,** with the sole replacement of the corresponding raw material intermediate, the following intermediates were obtained:

| Intermediate No. | Chemical Structure | Chemical Name | m/z(ESI): (M+H)⁺ |
|---|---|---|---|
| 35 | 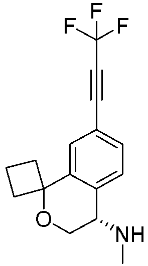 | (S)-N-methyl-7'-(3,3,3-trifluoropropyl)prop-1-yn-1-yl)spiro[cyclobutane-1,1'-isochroman]-4'-amine | 296.2 |
| 36 | 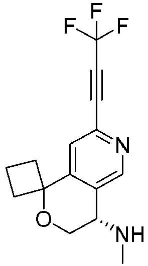 | (S)-N-methyl-7'-(3,3,3-trifluoropropyl)prop-1-yn-1-yl)-3',4'-dihydrospiro[cyclobutane-1,1'-pyrano[4,3-c]pyridine]-4'-amine | 297.2 |
| 37 | 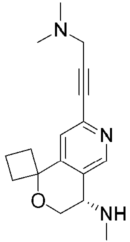 | (S)-7'-(3-(dimethylamino)prop-1-yn-1-yl)-N-methyl-3',4'-dihydrospiro[cyclobutane-1,1'-pyrano[4,3-c]pyridine]-4'-amine | 286.2 |
| 38 | 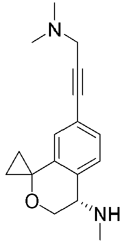 | (S)-7'-(3-(dimethylamino)prop-1-yn-1-yl)-N-methylspiro [cyclopropane-1,1'-isochroman]-4'-amine | 271.2 |
| 39 | 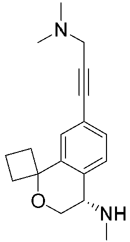 | (S)-7'-(3-(dimethylamino)prop-1-yn-1-yl)-N-methylspiro[cyclobutane-1,1'-isochroman]-4'-amine | 285.2 |

### Intermediate 40 (S)-N-methyl-7'-(4-(trifluoromethyl)-1H-pyrazol-1-yl)-3',4'-dihydrospiro[cyclobutane-1,1'-pyrano[4,3-c]pyridine]-4'-amine

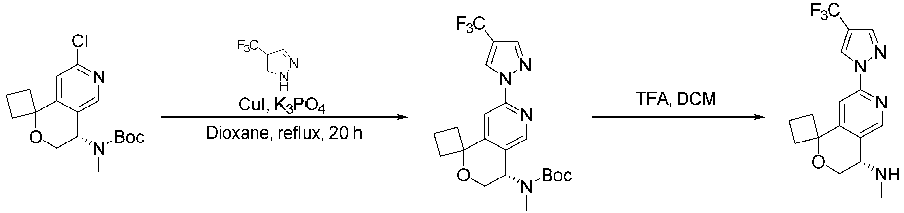

Step 1: Tert-butyl (S)-(7'-chloro-3',4'-dihydrospiro[cyclobutane-1,1'-pyrano[4,3-c]pyridine]-4'-yl)(methyl)carbamate (300 mg, 0.89 mmol) and 4-(trifluoromethyl)-1H-pyrazole (181 mg, 1.33 mmol) were dissolved in 1,4-dioxane (20.0 mL). Potassium phosphate (244 mg, 1.15 mmol), cuprous iodide (219 mg, 1.15 mmol), and trans-N, N-dimethylcyclohexane-1,2-diamine (164 mg, 1.15 mmol) were then added, and the mixture was stirred at reflux temperature under nitrogen atmosphere for 20 h. Monitoring by LCMS showed about 80% of the product. The reaction was cooled to room temperature and filtered. The filtrate was concentrated and purified by column chromatography (ethyl acetate/petroleum ether = 20%) to give (S)-methyl(7'-(4-(trifluoromethyl)-1H-pyrazol-1-yl)-3',4'-dihydrospiro[cyclobutane-1,1'-pyrano[4,3-c]pyridine]-4'-yl)carbamate (350 mg, yield: 90%) as a white solid.

LCMS (ESI): [M+H]⁺ m/z = 439.3

Step 2: To a solution of (S)-methyl(1'-(4-(trifluoromethyl)-1H-pyrazol-1-yl)-3',4'-dihydrospiro[cyclobutane-1,1'-pyrano[4,3-c]pyridine]-4'-yl)carbamate (350 mg, 0.80 mmol) in dichloromethane (12.0 mL) was added trifluoroacetic acid (3.0 mL) dropwise. After the addition, the mixture was reacted at room temperature for 1 h. The reaction mixture was concentrated and purified by reversed phase column (A: 0.5% formic acid in water; B: acetonitrile; gradient: B 5→100% over 10 min) and freeze-dried to give (S)-N-methyl-7'-(4-(trifluoromethyl)-1H-pyrazol-1-yl)-3',4'-dihydrospiro[cyclobutane-1,1'-pyrano[4,3-c]pyridine]-4'-amine (210 mg, yield: 74%) as a pale yellow solid.

LCMS (ESI): [M+H]⁺ m/z =339.2.

Employing the preparation method and steps of intermediate **40,** with the sole replacement of the corresponding raw material intermediate, the following intermediates were obtained:

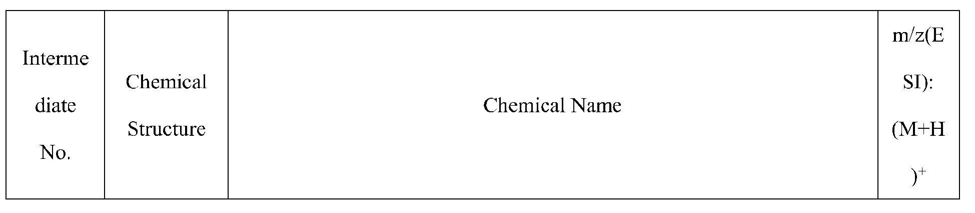

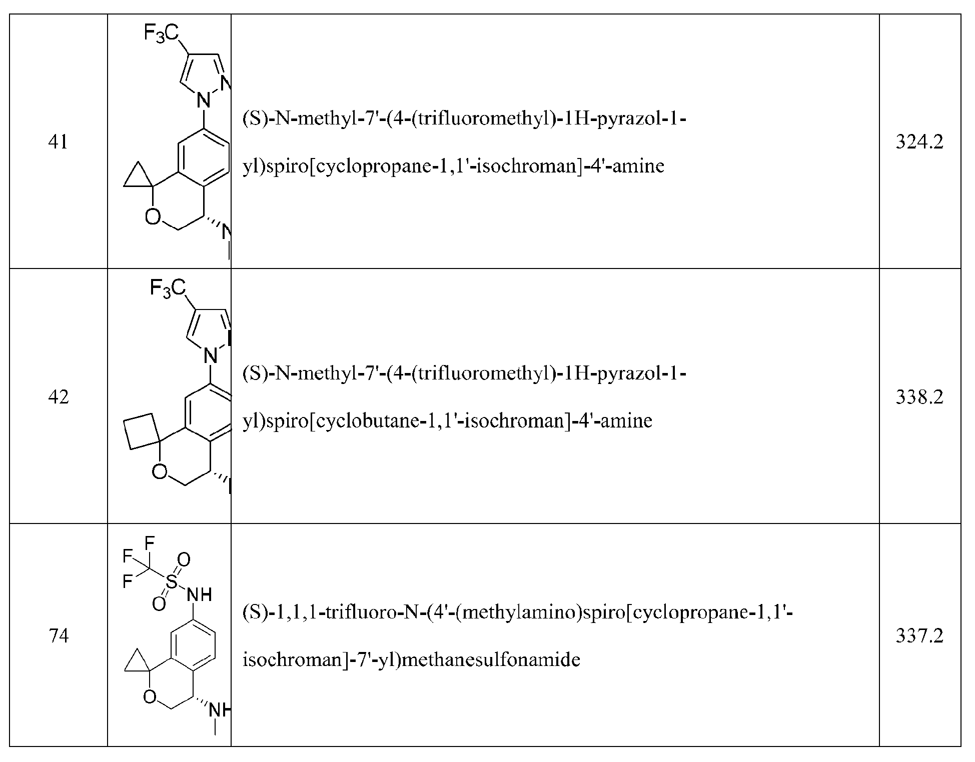

### Intermediate 43 (S)-N-methyl-7'-(1-methyl-1H-pyrazol-4-yl)-3',4'-dihydrospiro[cyclobutane-1,1'-pyrano[4,3-c]pyridine]-4'-amine

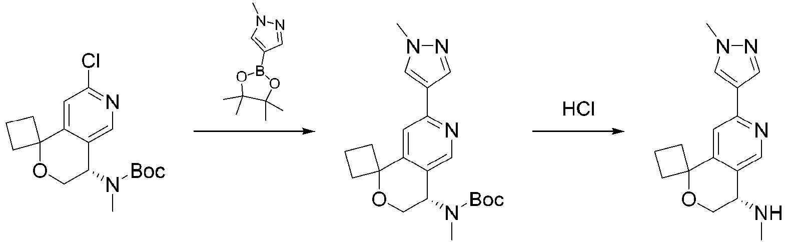

Step 1: Under nitrogen atmosphere, tert-butyl (S)-(7'-chloro-3',4'-dihydrospiro[cyclobutane-1,1'-pyrano[4,3-c]pyridine]-4'-yl)(methyl)carbamate (150 mg, 0.56 mmol) and 1-methyl-4-(4,4,5,5-tetramethyl-1,3,2-dioxaborolan-2-yl)-1H-pyrazole (175 mg, 0.84mmol) were dissolved in a mixed solvent of 1,4-dioxane (5 mL) and water (1 mL). Then, [1,1'-bis(diphenylphosphino)ferrocene]dichloropalladium(II) (40 mg, 0.056 mmol) and potassium carbonate (230 mg, 1.68 mmol) were added to the reaction mixture, which was stirred at 80°C for 16 h, poured into water (15 mL), and extracted with ethyl acetate (15 mL×3). The organic phases were combined, dried over anhydrous sodium sulfate, and filtered to remove the anhydrous sodium sulfate. The filtrate was concentrated under reduced pressure to give a crude product. The crude product was purified by silica gel column chromatography (petroleum ether: ethyl acetate = 1: 1) to give tert-butyl (S)-methyl(7'-(1-methyl-1H-pyrazol-4-yl)-3',4'-dihydrospiro[cyclobutane-1,1'-pyrano[*4,3-c*]pyridine]-4'-yl)carbamate (130 mg, yield: 85%) as a yellow solid.

LCMS (ESI): [M+H]⁺ m/z =385.2.

Step 2: To a solution of tert-butyl (S)-methyl(7'-(1-methyl-1H-pyrazol-4-yl)-3',4'-dihydrospiro[cyclobutane-1,1'-pyrano[4,3-c]pyridine]-4'-yl)carbamate (130 mg) in dichloromethane (5 mL) was added a solution of hydrochloric acid in dioxane (1.0 mL, 4 M) dropwise, and the mixture was reacted at room temperature for 2 h. The reaction mixture was concentrated to give (S)-N-methyl-7'-(1-methyl-1H-pyrazol-4-yl)-3',4'-dihydrospiro[cyclobutane-1,1'-pyrano[*4,3-c*]pyridine]-4'-amine (180 mg, crude product) as a yellow solid. The crude product was directly used in the next step without purification

LCMS (ESI): [M+H]⁺ m/z =285.2.

Employing the preparation method and steps of intermediate **43,** with the sole replacement of the corresponding raw material intermediate, the following intermediates were obtained:

| Intermediate No. | Chemical Structure | Chemical Name | m/z(E SI): (M+H) ⁺ |
|---|---|---|---|
| 44 | 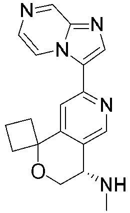 | (S)-7'-(imidazo[1,2-a]pyrazin-2-yl-3-yl)-N-methyl-3',4'-dihydrospiro[cyclobutane-1,1'-pyrano[4,3-c]pyridine]-4'-amine | 322.2 |
| 45 | 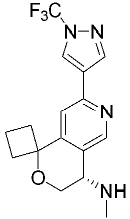 | (S)-N-methyl-7'-(1-(trifluoromethyl)-1H-pyrazol-4-yl)-3',4'-dihydrospiro[cyclobutane-1,1'-pyrano[4,3-c]pyridine]-4'-amine | 339.2 |
| 46 | 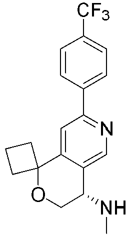 | (S)-N-methyl-7'-(4-(trifluoromethyl)phenyl)-3',4'-dihydrospiro[cyclobutane-1,1'-pyrano[4,3-c]pyridine]-4'-amine | 349.2 |
| 47 | 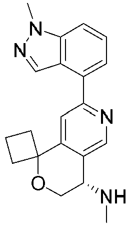 | (S)-N-methyl-7'-(1-methyl-1H-indazol-4-yl)-3',4'-dihydrospiro[cyclobutane-1,1'-pyrano[4,3-c]pyridine]-4'-amine | 335.2 |
| 48 | 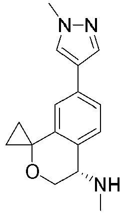 | (S)-N-methyl-7'-(1-methyl-1H-pyrazol-4-yl) spiro[cyclopropane-1,1'-isochroman]-4'-amine | 270.2 |
| 49 | 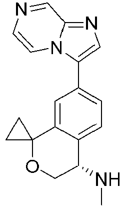 | (S)-7'-(imidazo[1,2-a]pyrazin-2-yl-3-yl)-N-methylspiro[cyclopropane-1,1'-isochroman]-4'-amine | 307.2 |
| 50 | 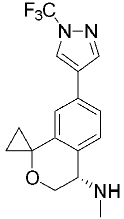 | (S)-N-methyl-7'-(1-(trifluoromethyl)-1H-pyrazol-4-yl)spiro[cyclopropane-1,1'-isochroman]-4'-amine | 324.2 |
| 51 | 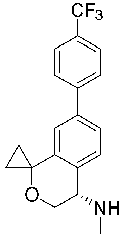 | (S)-N-methyl-7'-(4-(trifluoromethyl)phenyl)spiro[cyclopropane-1,1'-isochroman]-4'-amine | 334.2 |
| 52 | 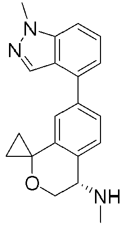 | (S)-N-methyl-7'-(1-methyl-1H-indazol-4-yl) spiro[cyclopropane-1,1'-isochroman]-4'-amine | 320.2 |

### Intermediate 53 1-Methyl-N-(7'-(trifluoromethyl)spiro[cyclobutane-1,1'-isochroman]-4'-yl)-1H-pyrazol-4-amine

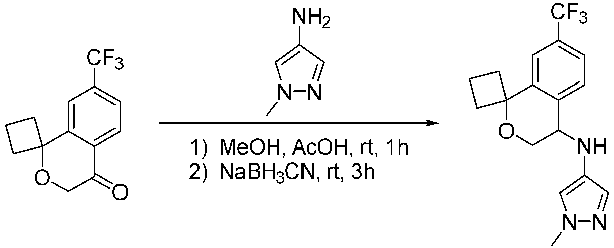

7'-(Trifluoromethyl)spiro[cyclobutane-1,1'-isochroman]-4'-one (500 mg, 1.95 mmol) was dissolved in a solution of methanol (5.0 mL). Then, 1-methyl-1H-pyrazol-4-amine (189 mg, 1.95 mmol) and formic acid (0.10 mL) were added. The mixture was stirred at room temperature for 1 h, added with sodium cyanoborohydride (363 mg, 5.85 mmol), and then stirred at room temperature for 3 h. The target molecular weight was detected by LCMS, and the reaction mixture was directly mixed with silica gel and eluted with a 50%-100% ethyl acetate/petroleum ether gradient to give 1-methyl-N-(7'-(trifluoromethyl)spiro[cyclobutane-1,1'-isochroman]-4'-yl)-1H-pyrazol-4-amine (480.00 mg, yield: 73%) as a light red oil.

LCMS (ESI): [M+H]⁺ = 338.26.

¹H NMR (400 MHz, Chloroform-d) δ 7.66 (s, 1H), 7.52 - 7.44 (m, 2H), 7.17 (d, *J* = 1.0 Hz, 1H), 6.98 (s, 1H), 4.05 (t, *J* = 3.4 Hz, 1H), 3.97 (dd, *J* = 11.8, 3.4 Hz, 1H), 3.87 (dd, *J* = 11.8, 3.2 Hz, 1H), 3.81 (s, 3H), 2.58 - 2.45 (m, 3H), 2.39 - 2.31 (m, 1H), 2.20 (m, 1H), 2.03 (m, 1H).

### Intermediate 75 (S)-N-methyl-7'-(trifluoromethyl)spiro[cyclopropane-1,1'-isochroman]-4'-amine

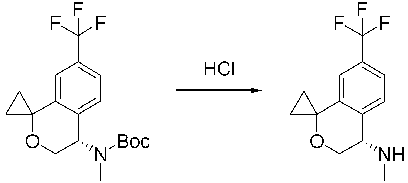

To a solution of tert-butyl (S)-methyl (7'-(trifluoromethyl)spiro[cyclopropane-1,1'-isochroman]-4'-yl)(methyl)carbamate (150 mg) in dichloromethane (5 mL) was added with a solution of hydrochloric acid in dioxane (1.0 mL, 4 M) dropwise, and the mixture was stirred at room temperature for 2 h. The reaction mixture was concentrated to give (S)-N-methyl-7'-(trifluoromethyl)spiro[cyclopropane-1,1'-isochroman]-4'-amine (190 mg, crude product) as a yellow solid. The crude product was directly used in the next step without purification

LCMS (ESI): [M+H]⁺ m/z =258.2.

Employing the preparation method and steps of intermediate **76,** with the sole replacement of the corresponding raw material intermediate, the following intermediates were obtained:

| Intermediate No. | Chemical Structure | Chemical Name | m/z(ESI): (M+H)⁺ |
|---|---|---|---|
| 76 | 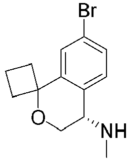 | (S)-7'-bromo-N-methylspiro[cyclobutane-1,1'-isochroman]-4'-amine | 283.2 |
| 77 | 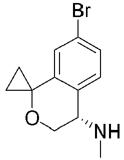 | (S)-7'-bromo-N-methylspiro[cyclopropane-1,1'-isochroman]-4'-amine | 269.2 |
| 78 | 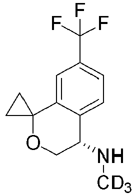 | (S)-N-(methyl-*d₃*)-7'-(trifluoromethyl)spiro[cyclopropane-1,1'-isochroman]-4'-amine | 261.2 |
| 79 | 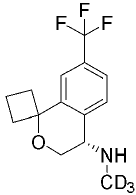 | (S)-N-(methyl-*d*₃)-7'-(trifluoromethyl)spiro[cyclobutane-1,1'-isochroman]-4'-amine | 275.2 |
| 80 | 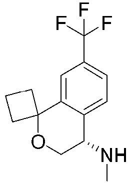 | (S)-N-methyl-7'-(trifluoromethyl)spiro[cyclobutane-1,1'-isochroman]-4'-amine | 272.2 |
| 81 | 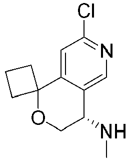 | (S)-7'-chloro-N-methyl-3',4'-dihydrospiro[cyclobutane-1,1'-pyrano[4,3-c]pyridine]-4'-amine | 239.2 |

### Intermediate 82 (S)-4'-(methylamino)spiro[cyclopropane-1,1'-isochroman]-7'-formonitrile

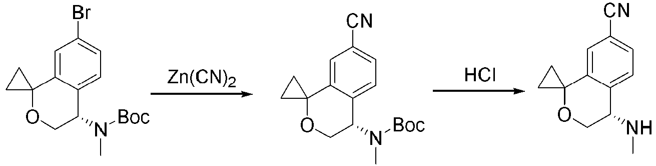

Step 1: Under nitrogen atmosphere, tetrakis(triphenylphosphine)palladium (157 mg, 0.136 mmol) was added to a solution of tert-butyl (S)-(7'-bromospiro[cyclopropane-1,1'-isochroman]-4'-yl)(methyl)carbamate (500 mg, 1.36 mmol) and zinc cyanide (95 mg, 0.82 mmol) in N,N-dimethylformamide (5 mL). The mixture was purged with nitrogen three times and stirred at 90°C for 16 h. After the reaction was complete, the mixture was poured into water (20 mL) and extracted with ethyl acetate (15 mL×3). The organic phases were combined, dried over anhydrous sodium sulfate, and filtered to remove the anhydrous sodium sulfate. The filtrate was concentrated under reduced pressure to give a crude product. The crude product was purified by silica gel column chromatography (petroleum ether: ethyl acetate = 5:1) to give tert-butyl (S)-(7'-cyanospiro[cyclopropane-1,1'-isochroman]-4'-yl)(methyl)carbamate (210mg, yield: 49%) as a yellow solid.

LCMS (ESI): [M+H]⁺ m/z =315.2.

Step 2: To a solution of (S)-(7'-cyanospiro[cyclopropane-1,1'-isochroman]-4'-yl)(methyl)carbamate (210 mg) in dichloromethane (5 mL) was added with a solution of hydrochloric acid in dioxane (1.0 mL, 4 M), and the mixture was reacted at room temperature for 2 h. The reaction mixture was concentrated to give (S)-4'-(methylamino)spiro[cyclopropane-1,1'-isochroman]-7'-formonitrile (250 mg, crude product) as a yellow solid. The crude product was directly used in the next step without purification

LCMS (ESI): [M+H]⁺ m/z =215.2.

### Intermediate 83 (S)-4'-(methylamino)spiro[cyclopropane-1,1'-isochroman]-7'-carboxamide

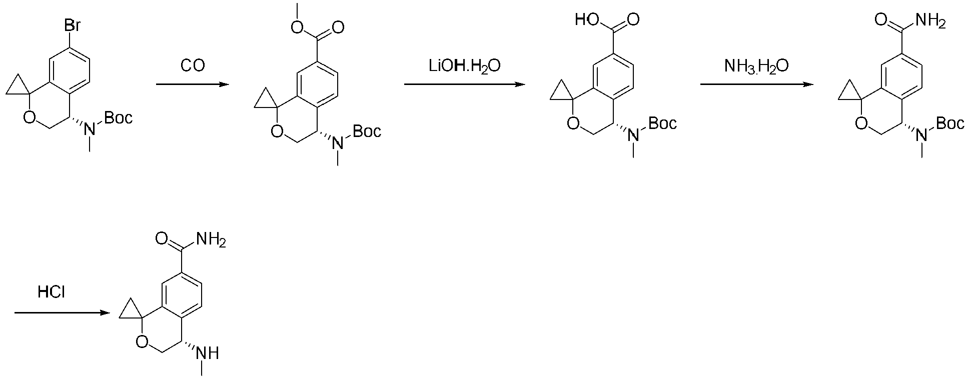

### Intermediate 85 N-(methyl-d₃)-7'-(trifluoromethyl)spiro[cyclopropane-1,1'-isochroman]-4'-d-4'-amine

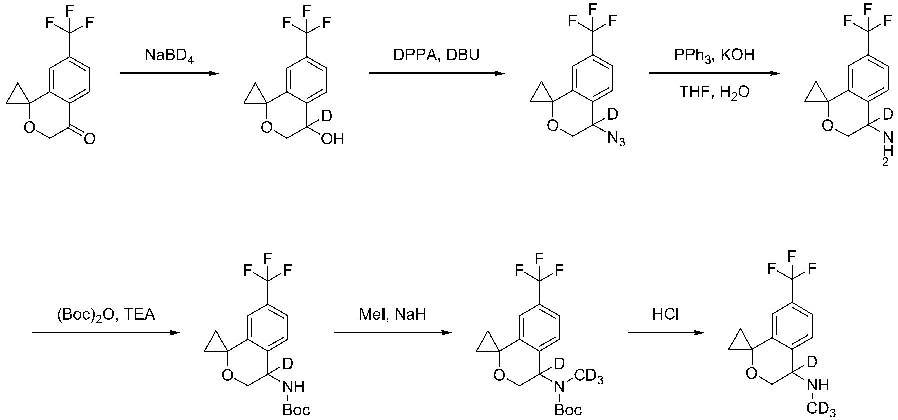

Step 1: At 0°C, sodium borodeuteride (186 mg, 4.54 mmol) was added to a solution of 7'-(trifluoromethyl)spiro[cyclopropane-1,1'-isochroman]-4'-one (1 g, 4.13 mmol) in methanol (15 mL), and the reaction mixture was stirred at room temperature for 1 h. After the reaction was complete, the reaction mixture was directly concentrated to obtain a crude product, which was separated and purified by column chromatography (petroleum ether: ethyl acetate = 3:1) to give 7'-(trifluoromethyl)spiro[cyclopropane-1,1'-isochroman]-4'-d-4'-ol (510 mg, yield: 50%) as a yellow solid.

LCMS (ESI) m/z: 246.2 [M+H]⁺

Step 2: Under nitrogen atmosphere, a solution of diphenylphosphoryl azide (2.47 g, 8.98 mmol) in toluene (10 mL) was slowly added dropwise to a solution of 7'-(trifluoromethyl)spiro[cyclopropane-1,1'-isochroman]-4'-d-4'-ol (2 g, 8.16 mmol) and 1,8-diazabicyclo[5.4.0]undec-7-ene (1.36 g, 8.98 mmol) in toluene (30 mL). The reaction mixture was stirred at room temperature for 16 h. After the reaction was complete, water (100 mL) was added to the reaction mixture, which was extracted with ethyl acetate (100 mL×2). The organic phases were combined, dried over anhydrous sodium sulfate, and concentrated under reduced pressure to obtain a crude product, which was separated and purified by column chromatography (petroleum ether: ethyl acetate = 1:0 to 3:1) to give 4'-azido-7'-(trifluoromethyl)spiro[cyclopropane-1,1'-isochroman]-4'-d (1.3 g, yield: 59%) as a yellow solid.

Step 3: Under nitrogen atmosphere, triphenylphosphine (1.89 g, 7.22 mmol) was added to a solution of 4'-azido-7'-(trifluoromethyl)spiro[cyclopropane-1,1'-isochroman]-4'-d (1.3 g, 4.81 mmol) in tetrahydrofuran (15 mL), and the mixture was stirred at room temperature for 1 h. Then, a solution of potassium hydroxide (670 mg, 12 mmol) in water (5 mL) was added to the reaction mixture, which was stirred at room temperature for 16 h. After the reaction was complete, water (50 mL) was added to the reaction mixture and extracted with ethyl acetate (50 mL×3). The organic phases were combined, dried over anhydrous sodium sulfate, and concentrated under reduced pressure to obtain a crude product, which was separated and purified by column chromatography (petroleum ether: ethyl acetate = 0:1 to 1:0) to give 7'-(trifluoromethyl)spiro[cyclopropane-1,1'-isochroman]-4'-d-4'-amine (700 mg, yield: 60%) as a yellow solid

LCMS (ESI) m/z: 245.2 [M+H]⁺

Step 4: Di-tert-butyl dicarbonate (940 mg, 4.3 mmol) was added to a solution of 7'-(trifluoromethyl)spiro[cyclopropane-1,1'-isochroman]-4'-d-4'-amine (700 mg, 2.87 mmol) and triethylamine (870 mg, 8.61 mmol) in dichloromethane (15 mL), and the reaction mixture was stirred at room temperature for 3 h. After the reaction was complete, water (30 mL) was added to the reaction mixture, which was extracted with dichloromethane (30 mL×3). The organic phases were combined, dried over anhydrous sodium sulfate, and concentrated under reduced pressure to obtain a crude product, which was separated and purified by column chromatography (petroleum ether: ethyl acetate = 1:0 to 5:1) to give tert-butyl (7'-(trifluoromethyl)spiro[cyclopropane-1,1'-isochroman]-4'-yl-4'-d)carbamate (890 mg, yield: 90%) as a yellow solid

LCMS (ESI) m/z: 345.2 [M+H]⁺

Step 5: At 0°C, sodium hydride (155 mg, 60% purity, 3.88 mmol) was added to a solution of tert-butyl (7'-(trifluoromethyl)spiro[cyclopropane-1,1'-isochroman]-4'-yl-4'-d)carbamate (890 mg, 2.58 mmol) in tetrahydrofuran (15 mL), and the mixture was stirred for 30 min at 0°C. Then, iodomethane-d₃ (560 mg, 3.88 mmol) was added to the reaction mixture, which was stirred at room temperature for 2 h. After the reaction was complete, water (30 mL) was added to the reaction mixture, which was extracted with ethyl acetate (30 mL×3). The organic phases were combined, dried over anhydrous sodium sulfate, and concentrated under reduced pressure to obtain a crude product, which was separated and purified by column chromatography (petroleum ether: ethyl acetate = 1:0 to 5:1) to give tert-butyl (methyl-*d*₃) (7'-(trifluoromethyl) spiro[cyclopropane-1,1'-isochroman]-4'-yl-4'-d) carbamate (650 mg, yield: 70%) as a yellow solid

LCMS (ESI) m/z: 362.2 [M+H]⁺

Step 6: To a solution of tert-butyl(methyl-*d*₃)(7'-(trifluoromethyl)spiro[cyclopropane-1,1'-isochroman]-4'-yl-4'-d)carbamate (650 mg) in dichloromethane (15 mL) was added with a solution of hydrochloric acid in dioxane (3.0 mL, 4 M) dropwise, and the mixture was stirred at room temperature for 5 h. The reaction mixture was concentrated to give N-(methyl-*d*₃)(7'-(trifluoromethyl)spiro[cyclopropane-1,1'-isochroman]-4'-d-4'-amine (720 mg, crude product) as a yellow solid. The crude product was directly used in the next step without purification

LCMS (ESI): [M+H]⁺ m/z =262.2

Employing the preparation method and steps of intermediate **86,** with the sole replacement of the corresponding raw material intermediate, the following intermediate was obtained:

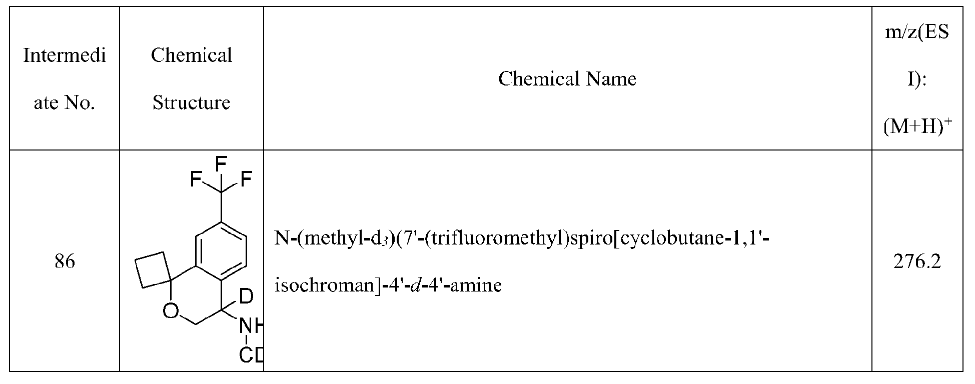

### Example 1 4-Amino-N-methyl-N-(7'-(trifluoromethyl)spiro[cyclobutane-1,1'-isochroman]-4¹-yl)imidazo[1,5-a]quinoxaline-8-carboxamide

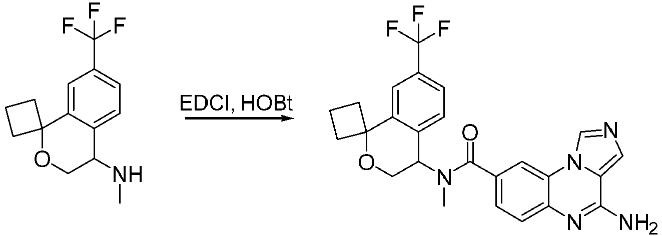

At room temperature, compound 4-amino-1-methylpyrazolo[4,5-c]quinoline-8-carboxylic acid (100 mg, 0.442 mmol) was dissolved in *N,N*-dimethylformamide (3 mL). 1-(3-Dimethylaminopropyl)-3-ethylcarbodiimide hydrochloride (85 mg, 0.442 mmol) and 1-hydroxybenzotriazole (60 mg, 0.442 mmol) were added, then *N,N*-diisopropylethylamine (87 mg, 0.66 mmol) was added and the mixture was stirred for reaction at room temperature for 0.5 hours. Then, N-methyl-7'-(trifluoromethyl)spiro[cyclobutane-1,1'-isochroman]-4'-amine (60 mg, 0.221 mmol) was added to the mixture, which was stirred at room temperature for 16 h. After LCMS indicated the reaction was complete, the mixture was poured into water (40 mL) and extracted with ethyl acetate (30 mL). The organic phases were washed with saturated saline (20 mL), dried over anhydrous sodium sulfate, and filtered. The filtrate was concentrated under reduced pressure to obtain a crude product, which was separated and purified by column chromatography (0-5% methanol/dichloromethane) to give 4-amino-N-methyl-N-(7'-(trifluoromethyl)spiro[cyclobutane-1,1'-isochroman]-4'-yl)imidazo[*1,5-a*]quinoxaline-8-carboxamide (25 mg, yield: 13.1%) as a white solid.

LCMS (ESI) m/z: 496.2 [M+H]⁺

¹H NMR (400 MHz, DMSO-*d*₆) δ 9.19 (d, *J* = 18.9 Hz, 1H), 8.36 (d, 1H), 7.89 (d, *J* = 15.1 Hz, 2H),7.68 (d, 1H), 7.63 - 7.35 (m, 5H), 5.31 (d, *J* = 341.0 Hz, 1H), 4.07 (d, 2H), 2.73 (s, 3H), 2.48 - 2.29 (m,3H), 2.26 - 1.93 (m, 3H).

### The following compounds are prepared using the method or part of the method used in Example 1, only replacing the corresponding materials:

| Exampl e No. | Chemical Structure | Chemical Name | ¹HNMR& ¹⁹F NMR& LCMS |
|---|---|---|---|
| 2 | 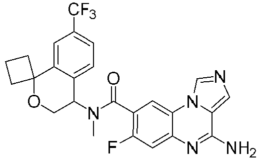 | 4-Amino-7-fluoro-N-methyl-N-(7'-(trifluoromethyl)sp iro[cyclobutane-1,1'-isochroman]-4'-yl)imidazo[1,5-a]quinoxaline-8-carboxamide | ¹H NMR (400 MHz, DMSO-*d₆*) δ 9.14 (d, *J* = 9.7 Hz, 1H), 8.36 (d, *J* = 6.6 Hz, 1H), 7.97 - 7.81 (m, 2H), 7.80 - 7.43 (m, 4H), 7.26 (dd, *J* = 11.0, 9.6 Hz, 1H), 5.79 - 4.75 (m, 1H), 4.07 (ddd, *J* = 35.2, 12.3, 5.0 Hz, 2H), 2.79 - 2.60 (m, 3H), 2.50 - 1.90 (m, 6H). |
| | | | LCMS (ESI) m/z: 500.2 [M+H]⁺ |
| 5 | 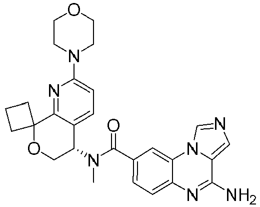 | (S)-4-amino-N-methyl-N-(2'-morpholinyl-5',6'-dihydrospiro[cyclo butane-1,8'-pyrano[3,4-b]pyridine]-5'-yl)imidazo[1,5-a]quinoxaline-8-carboxamide | ¹H NMR (400 MHz, DMSO-d*₆*) δ 9.25 (d, *J* = 18.8 Hz, 1H), 8.37 (d, *J* = 26.9 Hz, 1H), 8.12 (s, 2H), 7.88-7.27 (m, 4H), 6.91 - 6.76 (m, 1H), 5.45 - 4.70 (m, 1H), 4.07 (d, *J* = 5.7 Hz, 2H), 3.72 (t, *J* = 4.9 Hz,4H), 3.45 (d, *J* = 5.0 Hz, 4H), 2.69 (s, 3H), 2.43 - 2.27 (m, 3H), 2.24 - 2.15 (m, 1H), 2.06 - 1.83 (m, 2H). |
| | | | LCMS (ESI) m/z: 500.2 [M+H]⁺ |
| 6 | 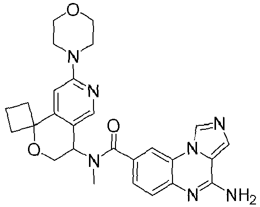 | 4-Amino-N-methyl-N-(7'-morpholinyl-3',4'-dihydrospiro[cyclo butane-1,1'-pyrano[4,3-c]pyridine]-4'-yl)imidazo[1,5-a]quinoxaline-8-carboxamide | ¹H NMR (400 MHz, DMSO-*d₆*) δ 9.20 (d, *J* = 13.9 Hz, 1H), 8.34 (s, 1H), 8.20 - 7.95 (m, 1H), 7.91 (s, 1H), 7.53 - 7.35 (m, 4H), 6.88 (t, *J* = 22.0 Hz, 1H), 5.17 (d, *J* = 334.2 Hz, 1H), 3.97 (d, *J* = 29.3 Hz, 2H), 3.76 - 3.67 (m, 4H), 3.50 (d, *J* = 6.6 Hz, 4H), 2.72 (s, 3H), 2.36 - 2.28 (m, 2H), 2.02 (s, 2H), 1.28 - 1.08 (m, 2H). |
| | | | LCMS (ESI) m/z: 500.2 [M+H]⁺ |
| 7 | 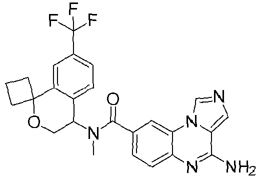 | 4-Amino-N-methyl-N-(7'-(trifluoromethyl)sp iro[cyclobutane-1,1'-isochroman]-4'-yl)imidazo[1,5-a]quinoxaline-8-carboxamide | ¹H NMR (400 MHz, DMSO-*d₆*) δ 9.19 (d, *J* = 18.9 Hz, 1H), 8.36 (d, 1H), 7.89 (d, *J* = 15.1 Hz, 2H), 7.68 (d, 1H), 7.63 - 7.35 (m, 5H), 5.31 (d, *J* = 341.0 Hz, 1H), 4.07 (d, 2H), 2.73 (s, 3H), 2.48 - 2.29 (m, 3H), 2.26 - 1.93 (m, 3H). |
| | | | LCMS (ESI) m/z: 482.2 [M+H]⁺ |
| 8 | 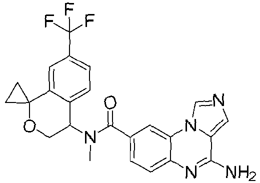 | 4-Amino-N-methyl-N-(7'-(trifluoromethyl)sp iro[cyclopropane-1,1'-isochroman]-4'-yl)imidazo[1,5-a]quinoxaline-8-carboxamide | ¹H NMR (400 MHz, DMSO-d*₆*) δ 9.19 (d, J = 20.3 Hz, 1H), 8.37 (s, 1H), 7.91 (s, 1H), 7.63 (s, 2H), 7.57 - 7.39 (m, 4H), 7.14(d, J = 24.3 Hz, 1H), 5.47 (d, J = 340.3 Hz, 1H), 4.19 (d, J = 5.1 Hz, 2H), 2.79 (s, 3H), 1.36 - 1.05 (m, 4H). |
| | | | LCMS (ESI) m/z: 468.2 [M+H]⁺ |
| 9 | 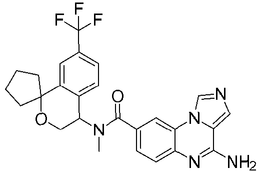 | 4-Amino-N-methyl-N-(7'-(trifluoromethyl)sp iro[cyclopentane-1,1'-isochroman]-4'-yl)imidazo[1,5-a]quinoxaline-8-carboxamide | ¹H NMR (400 MHz, DMSO-*d₆*) δ 9.21 (d, *J* = 20.6 Hz, 1H), 8.37 (s, 1H), 7.93 (s, 1H), 7.73 - 7.39 (m, 7H), 5.75 (d, *J* = 13.3 Hz, 1H), 4.06 (d, *J* = 12.0 Hz, 2H), 2.77 (d, *J* = 8.0 Hz, 3H), 2.02 (d, *J* = 138.5 Hz, 8H). |
| | | | LCMS (ESI) m/z: 496.2 [M+H]⁺ |
| 10 | 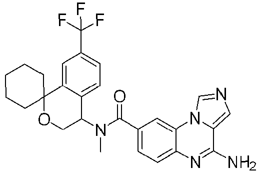 | 4-Amino-N-methyl-N-(7'-(trifluoromethyl)sp iro[cyclohexane-1,1'-isochroman]-4'-yl)imidazo[1,5-a]quinoxaline-8-carboxamide | LCMS (ESI) m/z: 410.2 [M+H]⁺ |
| 11 | 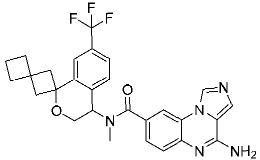 | 4-Amino-N-methyl-N-(7'-(trifluoromethyl)sp iro[cyclobutane-1,1'-cyclobutane-3',1"-isochroman]-4'-yl)imidazo[1,5-a]quinoxaline-8-carboxamide | LCMS (ESI) m/z: 522.2 [M+H]⁺ |
| 12 | 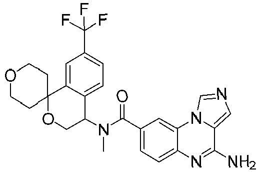 | 4-Amino-N-methyl-N-(7'-(trifluoromethyl)-2',3',5',6'-tetrahydrospiro[iso chroman-1,4'-pyran]-4-yl)imidazo[1,5-a]quinoxaline-8-carboxamide | ¹H NMR (400 MHz, DMSO-*d₆*) δ 9.20 (d, *J* = 15.8 Hz, 1H), 8.37 (s, 1H), 7.91 (s, 1H), 7.80 - 7.37 (m, 7H), 5.75 (d, *J* = 5.1 Hz, 1H), 4.18 (d, *J* = 19.3 Hz, 2H), 3.82 - 3.53 (m, 4H), 2.77 (d, *J* = 11.0 Hz, 3H), 2.27 - 1.71 (m, 4H). |
| | | | LCMS (ESI) m/z: 512.2 [M+H]⁺ |
| 13 | 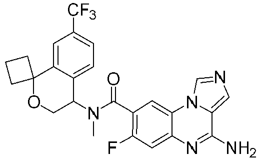 | 4-Amino-7-fluoro-N-methyl-N-(7'-(trifluoromethyl)sp iro[cyclobutane-1,1'-isochroman]-4'-yl)imidazo[1,5-a]quinoxaline-8-carboxamide | ¹H NMR (400 MHz, DMSO-*d₆*) δ 9.14 (d, *J* = 9.7 Hz, 1H), 8.36 (d,*J* = 6.6 Hz, 1H), 7.97 - 7.81 (m, 2H), 7.80 - 7.43 (m, 4H), 7.26 (dd, *J* = 11.0, 9.6 Hz, 1H), 5.79 - 4.75 (m, 1H), 4.07 (ddd, *J* = 35.2, 12.3, 5.0 Hz, 2H), 2.79 - 2.60 (m, 3H), 2.50 - 1.90 (m, 6H). |
| 16 | 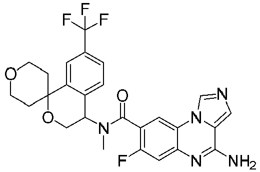 | 4-Amino-7-fluoro-N-methyl-N-(7-(trifluoromethyl)-2',3',5',6'-tetrahydrospiro[iso chroman-1,4'-pyran]-4-yl)imidazo[1,5-a]quinoxaline-8-carboxamide | ¹H NMR (400 MHz, DMSO-*d₆*) δ 9.15 (d, *J* = 8.1 Hz, 1H), 8.38 (d, *J* = 6.6 Hz, 1H), 7.94 (d, *J* = 1.9 Hz, 1H), 7.82 -7.70 (m, 2H), 7.62 (d, *J* = 2.6 Hz, 2H), 7.52 (d, *J* = 8.1 Hz, 1H), 7.32 -7.22 (m, 1H), 5.78 (d, *J* = 4.7 Hz, 1H), 4.16 (ddd, *J* = 44.1, 12.4, 4.1 Hz, 2H), 3.85 -3.54 (m, 4H), 2.86 -2.65 (m, 3H), 2.30 -.66 (m, 4H). |
| | | | LCMS (ESI) m/z: 530.2[M+H]⁺ |
| 22 | 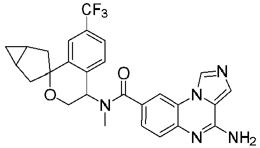 | 4-Amino-N-methyl-N-(7'-(trifluoromethyl) spiro[bicyclo[3.1.0] hexane-3,1'-isochroman-4'-yl)imidazo[1,5-a]quinoxaline-8-carboxamide | LCMS (ESI) m/z: 508.2[M+H]⁺ |
| 23 | 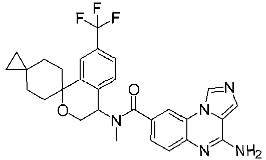 | 4-Amino-N-methyl-N-(7"-(trifluoromethyl) spiro[cyclopropane-1,1'-cyclohexane-4',1"-isochroman-4"-yl)imidazo[1,5-a]quinoxaline-8-carboxamide | ¹H NMR (400 MHz, DMSO-*d₆*) *δ* 9.20 (d, J = 16.2 Hz, 1H), 8.45 - 8.28 (m, 1H), 7.91 (s, 1H), 7.72 - 7.58 (m, 3H), 7.53 - 7.41 (m, 4H), 5.31 (d, J = 348.1 Hz, 1H), 4.15 (d, J = 24.2 Hz, 2H), 2.78 (d, J = 12.2 Hz, 3H), 2.16 - 1.82 (m, 6H), 0.72 (d, J = 12.5 Hz, 2H), 0.33 (s, 4H) |
| | | | LCMS (ESI) m/z: 536.2[M+H]⁺ |
| 25 | 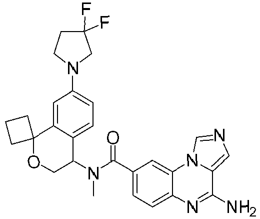 | 4-Amino-N-(7'-(3,3-difluoropyrrole-1-yl)spiro[cyclobutane -1,1'-isochroman]-4'-yl)-N-methylimidazo[1,5 -a]quinoxaline-8-carboxamide | LCMS (ESI) m/z: 519.2[M+H]⁺ |
| 26 | 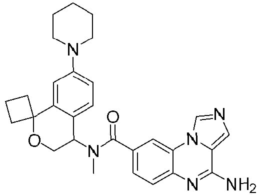 | 4-Amino-N-methyl-N-(7'-(piperidin-1-yl)spiro[cyclobuta ne-1,1'-isochroman]-4'-yl)imidazo[1,5-a]quinoxaline-8-carboxamide | LCMS (ESI) m/z: 497.26 [M+H]⁺ |
| 27 | 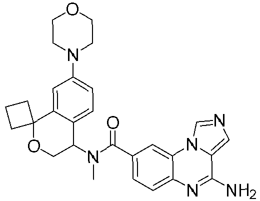 | 4-Amino-N-methyl-N-(7'-morpholinyl-spiro[cyclobutane-1,1'-isochroman]-4'-yl)imidazo[1,5-a]quinoxaline-8-carboxamide | LCMS (ESI) m/z: 499.24 [M+H]⁺ |
| 28 | 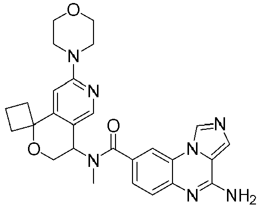 | 4-Amino-N-methyl-N-(7'-morpholinyl-3',4'-dihydrospiro[cyclo butane-1,1'-pyrano[4,3-c]pyridine]-4'-yl)imidazo[1,5-a]quinoxaline-8-carboxamide | ¹H NMR (400 MHz, DMSO-d*₆*) δ 9.20 (d, J = 13.9 Hz, 1H), 8.34 (s, 1H), 8.20 - 7.95 (m, 1H), 7.91 (s, 1H), 7.53 - 7.35 (m, 4H), 6.88 (t, J = 22.0 Hz, 1H), 5.17 (d, J = 334.2 Hz, 1H), 3.97 (d, J = 29.3 Hz, 2H), 3.76 - 3.67 (m, 4H), 3.50 (d, J = 6.6 Hz, 4H), 2.72 (s, 3H), 2.36 - 2.28 (m, 2H), 2.02 (s, 2H), 1.28 - 1.08 (m, 2H) |
| | | | LCMS (ESI) m/z: 500.23 [M+H]⁺ |
| 29 | 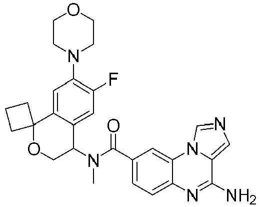 | 4-Amino-N-(6'-fluoro-7'-morpholinospiro[c yclobutane-1,1'-isochroman]-4'-yl)-N-methylimidazo[1,5 -a]quinoxaline-8-carboxamide | LCMS (ESI) m/z: 517.23 [M+H]⁺ |
| 30 | 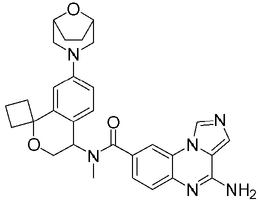 | N-(7'-((1R,5S)-8-oxa-3-azabicyclo[3.2.1]o ctan-3-yl)spiro[cyclobuta ne-1,1'-isochroman]-4'-yl)-4-amino-N-methylimidazo[1,5 -a]quinoxaline-8-carboxamide | LCMS (ESI) m/z: 525.25 [M+H]⁺ |
| 31 | 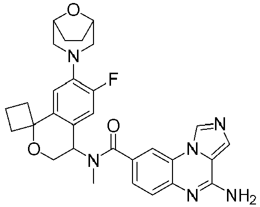 | N-(7'-((1R,5S)-8-oxa-3-azabicyclo[3.2.1]o ctan-3-yl)-6'-fluoro-spiro[cyclobutane-1,1'-isochroman]-4'-yl)-4-amino-N-methylimidazo[1,5 -a]quinoxaline-8-carboxamide | LCMS (ESI) m/z: 543.24 [M+H]⁺ |
| 32 | 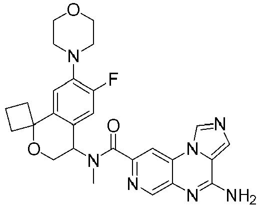 | 4-Amino-N-(6'-fluoro-7'-morpholinospiro[c yclobutane-1,1'-isochroman]-4'-yl)-N-methylimidazo[1,5 -a]pyrido[3,4-e]pyrazine-8-carboxamide | LCMS (ESI) m/z: 518.22 [M+H]⁺ |
| 33 | 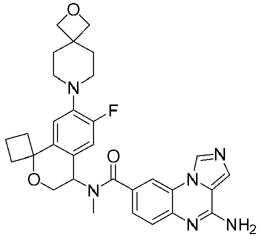 | 4-Amino-N-(6'-fluoro-7'-(2-oxa-7-azaspiro[3.5]nonan e-7-yl)spiro[cyclobuta ne-1,1'-isochroman]-4'-yl)-N-methylimidazo[1,5 -a]quinoxaline-8-carboxamide | LCMS (ESI) m/z: 557.26 [M+H]⁺ |
| 35 | 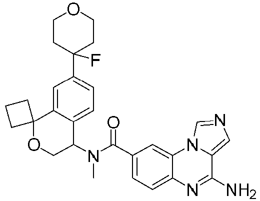 | 4-Amino-N-(7'-(4-fluorotetrahydro-2H-pyran-4-yl)spiro[cyclobuta ne-1,1'-isochroman]-4'-yl)-N-methylimidazo[1,5 -a]quinoxaline-8-carboxamide | LCMS (ESI) m/z: 516.23 [M+H]⁺ |
| 36 | 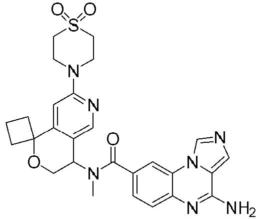 | 4-Amino-N-(7'-(1,1-dioxythiomorpholi nyl)-3',4'-dihydrospiro[cyclo butane-1,1'-pyrano[4,3-c]pyridine]-4'-yl)-N-methylimidazo[1,5 -a]quinoxaline-8-carboxamide | LCMS (ESI) m/z: 548.2 [M+H]⁺ |
| 37 | 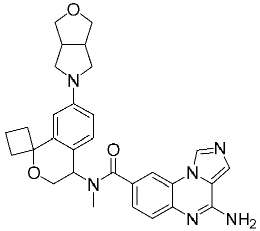 | 4-Amino-N-methyl-N-(7'-(tetrahydro-1H-furano[3,4-c]pyrrole-5(3H)-yl)spiro[cyclobuta ne-1,1'-isochroman]-4'-yl)imidazo[1,5-a]quinoxaline-8-carboxamide | LCMS (ESI) m/z: 525.25 [M+H]⁺ |
| 38 | 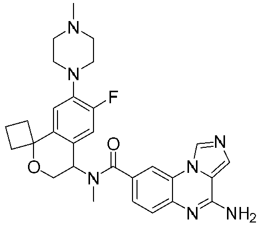 | 4-Amino-N-(6'-fluoro-7'-(4-methylpiperazine-1-yl)spiro[cyclobuta ne-1,1'-isochroman]-4'-yl)-N-methylimidazo[1,5 -a]quinoxaline-8-carboxamide | LCMS (ESI) m/z: 530.26 [M+H]⁺ |
| 39 | 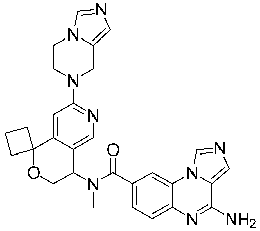 | 4-Amino-N-(7'-(5,6-dihydroimidazo[1, 5-a]pyrazine-7(8H)-yl)-3',4'-dihydrospiro[cyclo butane-1,1'-pyrano[4,3-c]pyridine]-4'-yl)-N-methylimidazo[1,5 -a]quinoxaline-8-carboxamide | LCMS (ESI) m/z: 536.24 [M+H]⁺ |
| 40 | 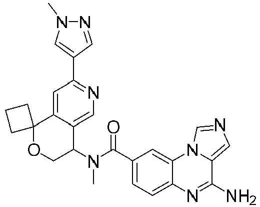 | 4-Amino-N-methyl-N-(7'-(1-methyl-1H-pyrazol-4-yl)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 495.22 [M+H]⁺ |
| 41 | 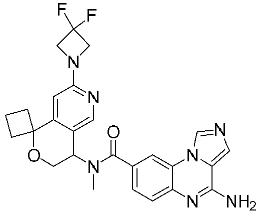 | 4-Amino-N-(7'-(3,3-difluoroazetidin-1-yl)-3',4'-dihydrospiro[cyclo butane-1,1'-pyrano[4,3-c]pyridine]-4'-yl)-N-methylimidazo[1,5 -a]quinoxaline-8-carboxamide | LCMS (ESI) m/z: 506.2 [M+H]⁺ |
| 42 | 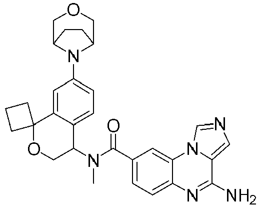 | N-(7'-((1R,5S)-3-oxa-8-azabicyclo[3.2.1] octan-8-yl)spiro[cyclobuta ne-1,1'-isochroman]-4'-yl)-4-amino-N-methylimidazo[1,5 -a]quinoxaline-8-carboxamide | LCMS (ESI) m/z: 525.25 [M+H]⁺ |
| 43 | 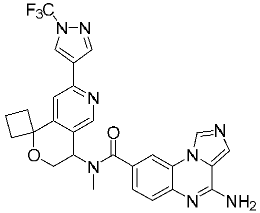 | 4-Amino-N-methyl-N-(7'-(1-(trifluoromethyl)-1H-pyrazol-4-yl)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 549.19 [M+H]⁺ |
| 44 | 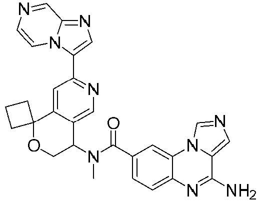 | 4-Amino-N-(7'-(imidazo[1,2-a]pyrazine-3-yl)-3',4'-dihydrospiro[cyclo butane-1,1'-pyrano[4,3-c]pyridine]-4'-yl)-N-methylimidazo[1,5 -a]quinoxaline-8-carboxamide | LCMS (ESI) m/z: 532.21 [M+H]⁺ |
| 46 | 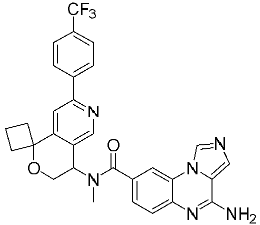 | 4-Amino-N-methyl-N-(7'-(4-(trifluoromethyl) phenyl)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 559.19 [M+H]⁺ |
| 47 | 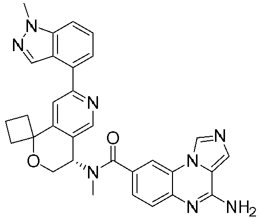 | (S)-4-amino-N-methyl-N-(7'-(1-methyl-1H-indazol-4-yl)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 545.23 [M+H]⁺ |
| 49 | 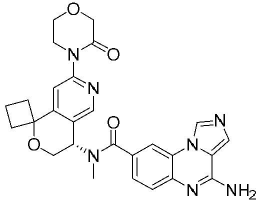 | (S)-4-amino-N-methyl-N-(7'-(morpholin-3-one)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 514.23 [M+H]⁺ |
| 55 | 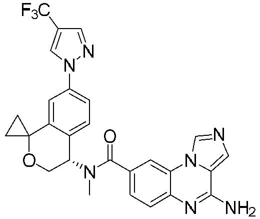 | (S)-4-amino-N-methyl-N-(7'-(4-(trifluoromethyl)-1H-pyrazol-1-yl)spiro[cyclopropane -1,1'-isochroman]-4'-yl)imidazo[1,5-a]quinoxaline-8-carboxamide | LCMS (ESI) m/z: 534.2 [M+H]⁺ |
| 56 | 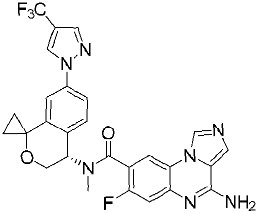 | (S)-4-amino-7-fluoro-N-methyl-N-(7'-(4-(trifluoromethyl)-1H-pyrazol-1-yl)spiro[cyclopropane -1,1'-isochroman]-4'-yl)imidazo[1,5-a]quinoxaline-8-carboxamide | LCMS (ESI) m/z: 552.2 [M+H]⁺ |
| 57 | 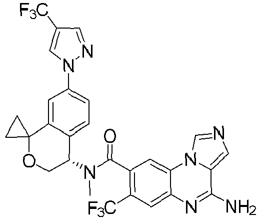 | (S)-4-amino-N-methyl-7-(trifluoromethyl)-N-(7'-(4-(trifluoromethyl)-1H-pyrazol-1-yl)spiro[cyclopropane -1,1'-isochroman]-4'-yl)imidazo[1,5-a]quinoxaline-8-carboxamide | LCMS (ESI) m/z: 602.2 [M+H]⁺ |
| 58 | 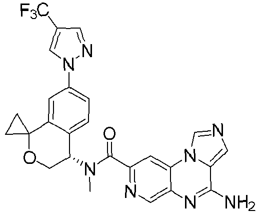 | (S)-4-amino-N-methyl-N-(7'-(4-(trifluoromethyl)-1H-pyrazol-1-yl)spiro[cycloprop ane-1,1'-isochroman]-4'-yl)imidazo[1,5-a]pyrido[3,4-e]pyrazine-8-carboxamide | LCMS (ESI) m/z: 535.2 [M+H]⁺ |
| 59 | 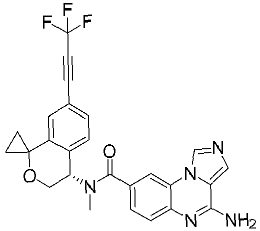 | (S)-4-amino-N-methyl-N-(7'-(3,3,3-trifluoropropyl)prop -1-yn-1-yl)spiro[cycloprop ane-1,1'-isochroman]-4'-yl)imidazo[1,5-a]quinoxaline-8-carboxamide | LCMS (ESI) m/z: 492.2 [M+H]⁺ |
| 60 | 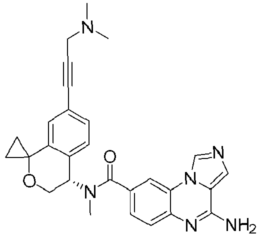 | (S)-4-amino-N-(7'-(3-(dimethylamino) prop-1-yn-1-yl)spiro[cyclopropane -1,1'-isochroman]-4'-yl)-N-methylimidazo[1,5 -a]quinoxaline-8-carboxamide | LCMS (ESI) m/z: 481.2 [M+H]⁺ |
| 61 | 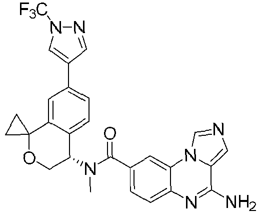 | (S)-4-amino-N-methyl-N-(7'-(1-(trifluoromethyl)-1H-pyrazol-4-yl)spiro[cycloprop ane-1,1'-isochroman]-4'-yl)imidazo[1,5-a]pyrido[3,4-e]pyrazine-8-carboxamide | LCMS (ESI) m/z: 434.2 [M+H]⁺ |
| 62 | 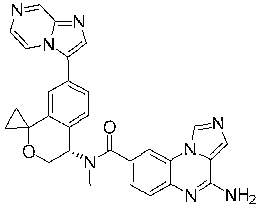 | (S)-4-amino-N(7'-(imidazo[1,2-a]pyrazin-2-yl)spiro[cycloprop ane-1,1'-isochroman]-4'-yl)imidazo[1,5-a]pyrido[3,4-e]pyrazine-8-carboxamide | LCMS (ESI) m/z: 517.2 [M+H]⁺ |
| 63 | 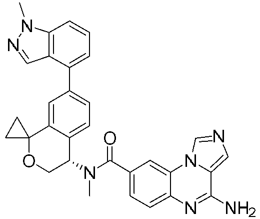 | (S)-4-amino-N-methyl-N-(7'-(1-methyl-1H-indazol-4-yl)spiro[cycloprop ane-1,1'-isochroman]-4'-yl)imidazo[1,5-a]pyrido[3,4-e]pyrazine-8-carboxamide | LCMS (ESI) m/z: 530.2 [M+H]⁺ |
| 64 | 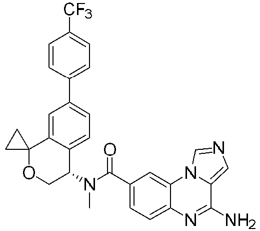 | (S)-4-amino-N-methyl-N-(7'-(4-(trifluoromethyl)p henyl)spiro[cyclop ropane-1,1'-isochroman]-4'-yl)imidazo[1,5-a]pyrido[3,4-e]pyrazine-8-carboxamide | LCMS (ESI) m/z: 544.2 [M+H]⁺ |
| 65 | 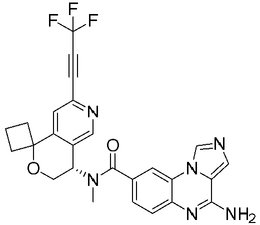 | (S)-4-amino-N-methyl-N-(7'-(3,3,3-trifluoropropyl-1-propyn-1-yl)-3',4'-dihydrospiro[cyclo butane-1,1'-pyrano[4,3-c]pyridine]-4'-yl)imidazo[1,5-a]quinoxaline-8-carboxamide | LCMS (ESI) m/z: 507.2 [M+H]⁺ |
| 66 | 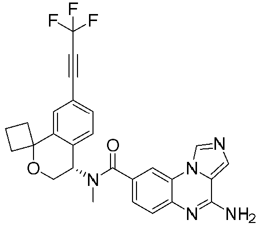 | (S)-4-amino-N-methyl-N-(7'-(3,3,3-trifluoropropyl)prop -1-yn-1-yl)spiro[cyclobuta ne-1,1'-isochroman]-4'-yl)imidazo[1,5-a]quinoxaline-8-carboxamide | LCMS (ESI) m/z: 506.2 [M+H]⁺ |
| 67 | 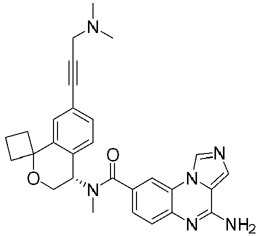 | (S)-4-amino-N-(7'-(3-(dimethylamino)prop -1-yn-1-yl)spiro[cyclobuta ne-1,1'-isochroman]-4'-yl)-N-methylimidazo[1,5 -a]quinoxaline-8-carboxamide | LCMS (ESI) m/z: 495.2 [M+H]⁺ |
| 68 | 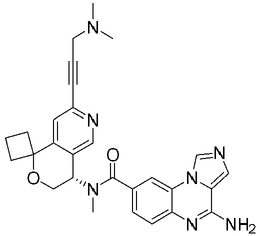 | (S)-4-amino-N-(7'-(3-(dimethylamino)prop -1-yn-1-yl)-3',4'-dihydrospiro[cyclo butane-1,1'-pyrano[4,3-c]pyridine]-4'-yl)-N-methylimidazo[1,5 -a]quinoxaline-8-carboxamide | LCMS (ESI) m/z: 496.2 [M+H]⁺ |
| 69 | 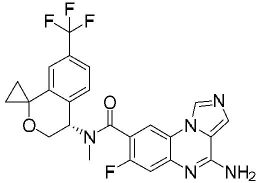 | (S)-4-amino-7-fluoro-N-methyl-N-(7'-(trifluoromethyl)sp iro[cyclopropane-1,1'-isochroman]-4'-yl)imidazo[1,5-a]quinoxaline-8-carboxamide | LCMS (ESI) m/z: 486.2 [M+H]⁺ |
| 70 | 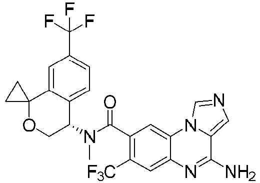 | (S)-4-amino-N-methyl-7-(trifluoromethyl)-N-(7'-(trifluoromethyl) spiro[cyclopropane-1,1'-isochroman]-4'-yl)imidazo[1,5-a]quinoxaline-8-carboxamide | LCMS (ESI) m/z: 536.2 [M+H]⁺ |
| 71 | 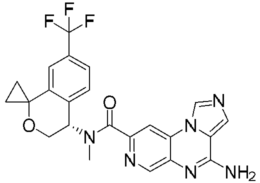 | (S)-4-amino-N-methyl-N-(7'-(trifluoromethyl) spiro[cyclopropane-1,1'-isochroman]-4'-yl)imidazo[1,5-a]pyrido[3,4-e]pyrazine-8-carboxamide | LCMS (ESI) m/z: 536.2 [M+H]⁺ |
| 72 | 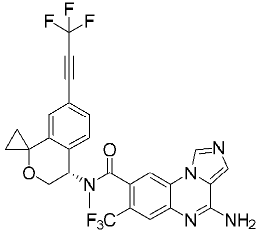 | (S)-4-amino-N-methyl-7-(trifluoromethyl)-N-(7'-(3,3,3-trifluoroprop-1-yn-1-yl)spiro[cyclopropane -1,1'-isochroman]-4'-yl)imidazo[*1,5-a*]quinoxaline-8-carboxamide | LCMS (ESI) m/z: 560.2 [M+H]⁺ |
| 73 | 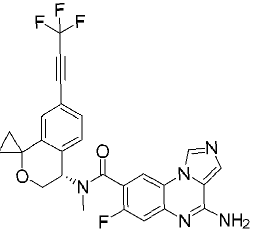 | (S)-4-amino-7-fluoro-N-methyl-N-(7'-(3,3,3-trifluoroprop-1-yn-1-yl)spiro[cycloprop ane-1,1'-isochroman]-4'-yl)imidazo[1,5-a]quinoxaline-8-carboxamide | LCMS (ESI) m/z: 510.2 [M+H]⁺ |
| 74 | 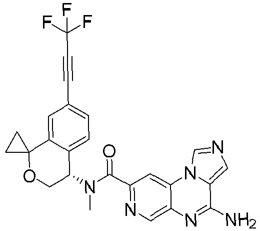 | (S)-4-amino-N-methyl-N-(7'-(3,3,3-trifluoroprop-1-yn-1-yl)spiro[cycloprop ane-1,1'-isochroman]-4'-yl)imidazo[1,5-a]pyrido[3,4-e]pyrazine-8-carboxamide | LCMS (ESI) m/z: 493.2 [M+H]⁺ |
| 75 | 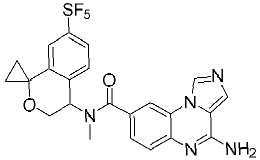 | 4-Amino-N-methyl-N-(7'-(pentafluoro-λ⁶-hydrogenthio)spiro [cyclopropane-1,1'-isochroman]-4'-yl)imidazo[1,5-a]quinoxaline-8-carboxamide | LCMS (ESI) m/z:526.2 [M+H]⁺ |
| 76 | 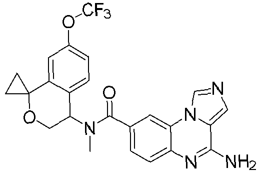 | 4-Amino-N-methyl-N-(7'-((trifluoromethoxy )thio)spiro[cyclopr opane-1,1'-isochroman]-4'-yl)imidazo[1,5-a]quinoxaline-8-carboxamide | LCMS (ESI) m/z:484.2 [M+H]⁺ |
| 77 | 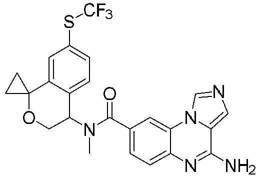 | 4-Amino-N-methyl-N-(7'-(trifluoromethoxy) spiro[cyclopropane -1,1'-isochroman]-4'-yl)imidazo[1,5-a]quinoxaline-8-carboxamide | LCMS (ESI) m/z:500.2 [M+H]⁺ |
| 78 | 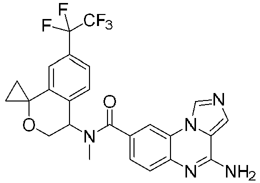 | 4-Amino-N-methyl-N-(7'-(perfluoroethyl)spi ro[cyclopropane-1,1'-isochroman]-4'-yl)imidazo[1,5-a]quinoxaline-8-carboxamide | LCMS (ESI) m/z:518.2 [M+H]⁺ |
| 79 | 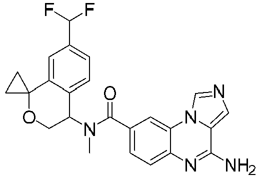 | 4-Amino-N-(7'-(difluoromethyl)sp iro[cyclopropane-1,1'-isochroman]-4'-yl)imidazo[1,5-a]quinoxaline-8-carboxamide | LCMS (ESI) m/z:450.2 [M+H]⁺ |
| 80 | 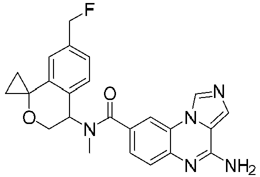 | 4-Amino-N-(7'-(fluoromethyl)spir o[cyclopropane-1,1'-isochroman]-4'-yl)imidazo[1,5-a]quinoxaline-8-carboxamide | LCMS (ESI) m/z:432.2 [M+H]⁺ |
| 81 | 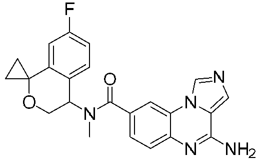 | 4-Amino-N-(7'-fluoro-spiro[cyclopropane -1,1'-isochroman]-4'-yl)imidazo[1,5-a]quinoxaline-8-carboxamide | LCMS (ESI) m/z:418.2 [M+H]⁺ |
| 82 | 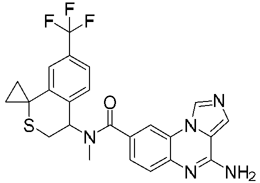 | 4-Amino-N-methyl-N-(7'-(trifluoromethyl) spiro[cyclopropane-1,1'-thioisochroman]-4'-yl)imidazo[1,5-a]quinoxaline-8-carboxamide | LCMS (ESI) m/z:484.2 [M+H]⁺ |
| 83 | 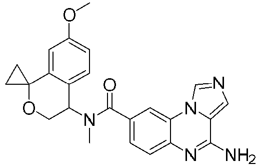 | 4-Amino-N-methyl-N-(7'-methoxyspiro[cycl opropane-1,1'-isochroman]-4'-yl)imidazo[1,5-a]quinoxaline-8-carboxamide | LCMS (ESI) m/z:430.2 [M+H]⁺ |
| 84 | 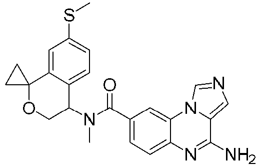 | 4-Amino-N-methyl-N-(7'-(methylthio)spiro[ cyclopropane-1,1'-isochroman]-4'-yl)imidazo[1,5-a]quinoxaline-8-carboxamide | LCMS (ESI) m/z:446.2 [M+H]⁺ |
| 85 | 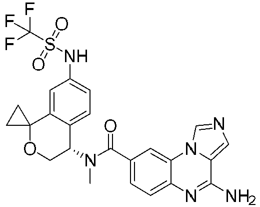 | (S)-4-amino-N-methyl-N-(7'-((trifluoromethyl)s ulfonamide)spiro[c yclopropane-1,1'-isochroman]-4'-yl)imidazo[1,5-a]quinoxaline-8-carboxamide | LCMS (ESI) m/z:547.2 [M+H]⁺ |
| 86 | 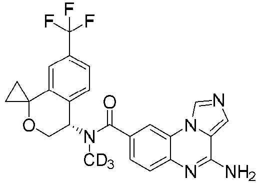 | (S)-4-amino-N-(methyl-*d*₃)-N-(7'-(trifluoromethyl)sp iro[cyclopropane-1,1'-isochroman]-4'-yl)imidazo[1,5-a]quinoxaline-8-carboxamide | LCMS (ESI) m/z:471.2 [M+H]⁺ |
| 87 | 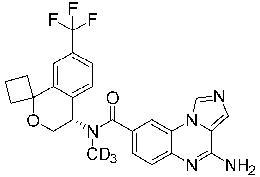 | (S)-4-amino-N-(methyl-*d*₃)-N-(7'-(trifluoromethyl)sp iro[cyclobutane-1,1'-isochroman]-4'-yl)imidazo[1,5-a]quinoxaline-8-carboxamide | LCMS (ESI) m/z:485.2 [M+H]⁺ |
| 88 | 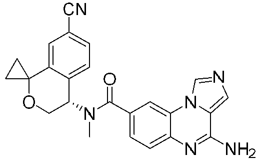 | (S)-4-amino-N-(7'-cyanospiro[cyclopr opane-1,1'-isochroman]-4'-yl)-N-methylimidazo[1,5 -a]quinoxaline-8-carboxamide | LCMS (ESI) m/z:425.2 [M+H]⁺ |
| 89 | 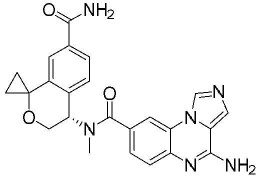 | (S)-4-amino-N-(7'-carbamoylspiro[cy clopropane-1,1'-isochroman]-4'-yl)-N-methylimidazo[*1,5* -*a*]quinoxaline-8-carboxamide | LCMS (ESI) m/z:443.2 [M+H]⁺ |
| 90 | 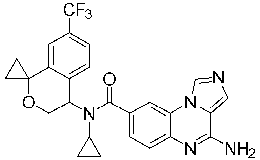 | 4-Amino-N-cyclopropyl-N-(7'-(trifluoromethyl)sp iro[cyclopropane-1,1'-isochroman]-4'-yl)imidazo[1,5-a]quinoxaline-8-carboxamide | LCMS (ESI) m/z:494.2 [M+H]⁺ |
| 91 | 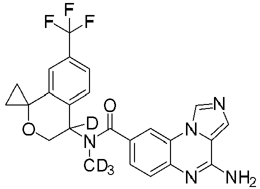 | 4-Amino-N-(methyl-*d*₃)-N-(7'-(trifluoromethyl)sp iro[cyclopropane-1,1'-isochroman]-4'-yl-4'-d)imidazo[1,5-a]quinoxaline-8-carboxamide | LCMS (ESI) m/z:472.2 [M+H]⁺ |
| 92 | 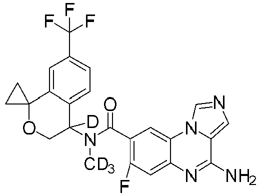 | 4-Amino-7-fluoro-N-(methyl-*d*₃)-N-(7'-(trifluoromethyl)sp iro[cyclopropane-1,1'-isochroman]-4'-yl-4'-d)imidazo[1,5-a]quinoxaline-8-carboxamide | LCMS (ESI) m/z:490.2 [M+H]⁺ |
| 93 | 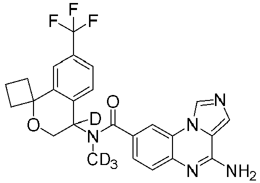 | 4-Amino-N-(methyl-*d*₃)-N-(7'-(trifluoromethyl)sp iro[cyclobutane-1,1'-isochroman]-4'-yl-4'-d)imidazo[1,5-a]quinoxaline-8-carboxamide | LCMS (ESI) m/z:486.2 [M+H]⁺ |
| 94 | 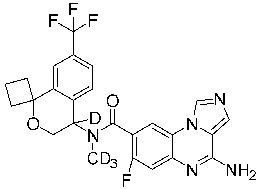 | 4-Amino-7-fluoro-N-(methyl-*d*₃)-N-(7'-(trifluoromethyl)sp iro[cyclobutane-1,1'-isochroman]-4'-yl-4'-d)imidazo[1,5-a]quinoxaline-8-carboxamide | LCMS (ESI) m/z:504.2 [M+H]⁺ |
| 95 | 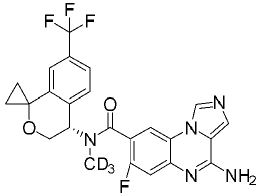 | (S)-4-amino-7-fluoro-N-(methyl-*d*₃)-N-(7'-(trifluoromethyl)sp iro[cyclopropane-1,1'-isochroman]-4'-yl)imidazo[1,5-a]quinoxaline-8-carboxamide | LCMS (ESI) m/z: 489.2 [M+H]⁺ |
| 96 | 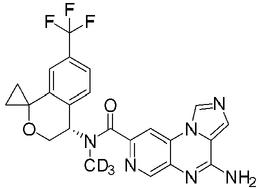 | (S)-4-amino-N-(methyl-*d*₃)-N-(7'-(trifluoromethyl)sp iro[cyclopropane-1,1'-isochroman]-4'-yl)imidazo[1,5-a]pyrido[3,4-e]pyrazine-8-carboxamide | LCMS (ESI) m/z: 472.2 [M+H]⁺ |
| 97 | 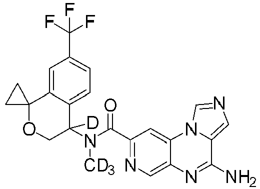 | (S)-4-amino-N-(methyl-*d*₃)-N-(7'-(trifluoromethyl)sp iro[cyclopropane-1,1'-isochroman]-4'-yl-4'-d)imidazo[1,5-a]pyrido[3,4-e]pyrazine-8-carboxamide | LCMS (ESI) m/z: 473.2 [M+H]⁺ |
| 98 | 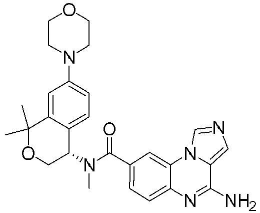 | (S)-4-amine-N-(1,1-dimethyl-7-morpholinoisochro man-4-yl)-N-methylimidazo[1,5 -a]quinoxaline-8-carboxamide | ¹H NMR (400 MHz, DMSO) δ 9.31 - 9.09 (m, 1H), 8.43 - 8.27 (m, 1H), 7.91 (s, 1H), 7.55 - 7.33 (m, 4H), 7.22 - 7.02 (m, 1H), 6.96 - 6.70 (m, 2H), 5.56 (s, 0.5H), 4.69 (s, 0.5H), 4.17 - 3.88 (m, 2H), 3.74 (s, 4H), 3.23 - 3.02 (m, 4H), 2.86 - 2.67 (m, 3H), 1.61 - 1.27 (m, 6H). |
| | | | LCMS (ESI) m/z: 487.2 [M+H]⁺ |
| 99 | 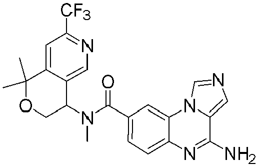 | 4-Amino-N-(1,1-dimethyl-7-(trifluoromethyl)-3,4-dihydro-1H-pyrano[4,3-c]pyridin-4-yl)-N-methylimidazo[1,5 -a]quinoxaline-8-carboxamide | ¹H NMR (400 MHz, DMSO) δ 9.21 (d, J = 14.5 Hz, 1H), 8.75 (d, J = 26.6 Hz, 1H), 8.40 (s, 1H), 7.95 (d, J = 24.2 Hz, 2H), 7.62 - 7.33 (m, 4H), 5.34 (d, J = 311.1 Hz, 1H), 4.35 - 4.03 (m, 2H), 2.84 (s, 3H), 1.58 (d, J = 20.6 Hz, 6H) |
| | | | LCMS (ESI) m/z: 471.1 [M+H]⁺ |

### Example 17 4-Amino-N-(1-methyl-1H-pyrazol-4-yl)-N-(7'-(trifluoromethyl)spiro[cyclobutane-1,1'-isochroman]-4'-yl)imidazo[1,5-a]quinoxaline-8-carboxamide

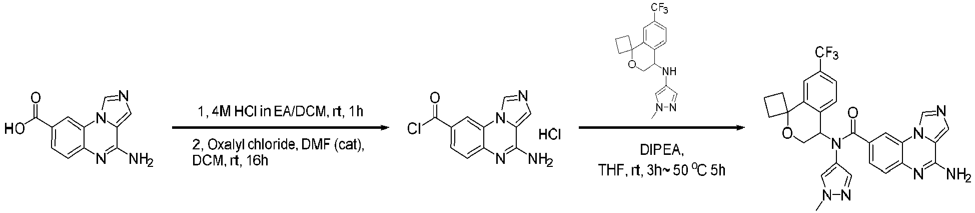

Step 1: 4-Aminoimidazo[1,5-a]quinoxaline-8-carboxylic acid (200 mg, 0.88 mmol) was dissolved in dichloromethane (2.0 mL), and a solution of 4 M ethyl acetate hydrochloride (0.3 mL) was added. The reaction mixture was stirred at 20°C for 30 min. The reaction mixture was concentrated and dissolved in dichloromethane (1.0 mL), and oxalyl chloride (566 mg, 4.38 mmol) and *N,N*-dimethylformamide (6.41 mg, 0.09 mmol) were added at 0°C for catalysis. The reactant was stirred at room temperature for 16 h. The reaction was monitored by LCMS (methanol), which showed 80% methyl ester product. The reaction mixture was directly concentrated to give 4-aminoimidazo[1,5-a]quinoxaline-8-formylchloride hydrochloride (210 mg, yield: 97%) as a gray solid crude.

LCMS (ESI): [M+H]⁺ = 243.14.

Step 2: 1-Methyl-N-(7'-(trifluoromethyl)spiro[cyclobutane-1,1'-isochroman]-4'-yl)-1H-pyrazol-4-amine (120 mg, 0.36 mmol) was dissolved in tetrahydrofuran (10.0 mL), and N,N-diisopropylethylamine (230 mg, 1.78 mmol) and 4-aminoimidazo[1,5-a]quinoxaline-8-formyl chloride hydrochloride (105 mg, 0.43 mmol) were added. The reaction mixture was stirred at 20°C for 16 h. The reaction was monitored by LCMS (methanol), which showed 35% product. The reaction mixture was concentrated, purified by silica gel column, and eluted with 10-15% methanol/dichloromethane for 20 min to give a product with a purity of 70%. After the product was dissolved in 3 mL of *N,N*-dimethylformamide, it was further purified by HPLC (0.5% aqueous ammonium bicarbonate solution/acetonitrile, 60%-70%), and freeze-dried to give 4-amino-N-(1-methyl-1H-pyrazol-4-yl)-N-(7'-(trifluoromethyl)spiro[cyclobutane-1,1'-isochroman]-4'-yl)imidazo[1,5-a]quinoxaline-8-carboxamide (83.27 mg, yield: 52%) as a white solid.

LCMS (ESI): [M+H]⁺ = 548.3.

¹H NMR (400 MHz, DMSO-*d₆*) δ ppm 9.08 (s, 1H), 8.20 (s, 1H), 7.88 (s, 1H), 7.75-7.65 (m, 3H), 7.41.7.32 (m, 3H), 7.29-7.23 (m, 2H), 6.95 (s, 1H), 5.86 (s, 1H), 4.16-4.12 (m, 1H), 3.99-3.97 (m, 1H), 3.50 (s, 3H), 2.51-2.04 (m, 6H).

### The following compounds are prepared using the method or part of the method used in Example 17, only replacing the corresponding materials:

| Example No. | Chemical Structure | Chemical Name | ¹HNMR& ¹⁹F NMR& LCMS |
|---|---|---|---|
| 18 | 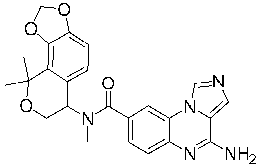 | 4-Amino-N-(9,9-dimethyl-6,9-dihydro-7H-[1,3]dioxino[4,5-h]isochroman-6-yl)-N-methylimidazo[1,5 -a]quinoxaline-8-carboxamide | ¹H NMR (400 MHz, DMSO-*d₆*) δ 9.21 (s, 1H), 8.34 (d, *J* = 11.9 Hz, 1H), 7.93 (s, 1H), 7.44 (d, *J* = 10.7 Hz, 4H), 7.12-6.51 (m, 2H), 6.04 (d, *J* = 7.2 Hz, 2H), 5.66-5.17 (m, 1H), 4.35-3.84 (m, 2H), 2.77 (s, 3H), 1.56 (s, 3H), 1.23 (s, 3H). |
| | | | LCMS (ESI): [M+H]⁺ = 446.3 |

### Example 19 4-Amino-7-fluoro-N,1-dimethyl-N-(7-(trifluoromethyl)-2',3',5',6'-tetrahydrospiro[isochroman-1,4'-pyran]-4-yl)imidazo[1,5-a]quinoxaline-8-carboxamide

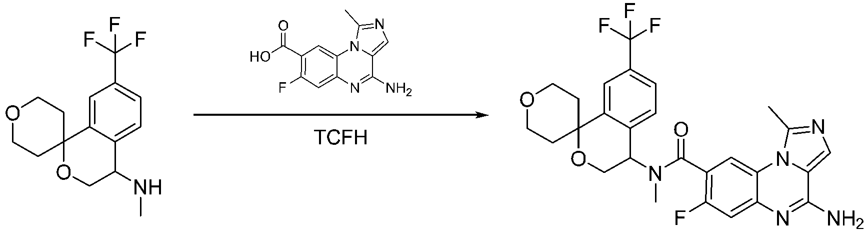

Step 1: N-methyl-7-(trifluoromethyl)-2',3',5',6'-tetrahydrospiro[isochroman-1,4'-pyran]-4-amine (300 mg, 0.99 mmol) was dissolved in a solution of N,N-dimethylacetamide (8.0 mL), and 4-amino-7-fluoro-1-methylimidazo[1,5-a]quinoxaline-8-carboxylic acid (311 mg, 1.19 mmol), *N,N,N*'*,*N'-tetramethylchloroformamidine hexafluorophosphate (335 mg, 1.19 mmol), and N-methylimidazole (409 mg, 4.98 mmol) were added. The reaction mixture was stirred at 50°C for 16 h, added with water (50 mL), extracted with ethyl acetate (30 mL× 3), dried over anhydrous sodium sulfate, filtered and concentrated, purified by HPLC (0.5% aqueous formic acid solution/acetonitrile, 40%-50%), and freeze-dried to give 4-amino-7-fluoro-N,1-dimethyl-N-(7-(trifluoromethyl)-2',3',5',6'-tetrahydrospiro[isochroman-1,4'-pyran]-4-yl)imidazo[1,5-a]quinoxaline-8-carboxamide (220 mg, yield: 41%) as a white solid.

LCMS (ESI): [M+H]⁺ = 544.3.

¹H NMR (400 MHz, DMSO-*d₆*) δ 8.17-8.14 (m, 1H), 7.96 (s, 1.75H), 7.78-7.65 (m, 3.37H), 7.55 (d, *J* = 8.2Hz, 1H), 7.33-7.30 (m, 1.3H), 5.75 (s, 0.5H), 4.81 (s, 0.5H), 4.23-4.01 (m, 2H), 3.77-3.65 (m, 4H), 3.99-3.97 (m, 3H), 2.75 (d, *J* = 2.74, 3H), 2.34-1.89 (m, 4H).

**The following compounds are prepared using the method or part of the method used in Example 19, only replacing the corresponding materials:**

| Exampl e No. | Chemical Structure | Chemical Name | ¹HNMR& ¹⁹F NMR& LCMS |
|---|---|---|---|
| 20 | 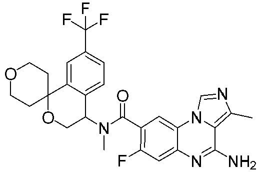 | 4-Amino-7-fluoro-N,3-dimethyl-N-(7-(trifluoromethyl)-2',3',5',6'-tetrahydrospiro[iso chroman-1,4'-pyran]-4-yl)imidazo[1,5-a]quinoxaline-8-carboxamide | ¹H NMR (400 MHz, DMSO-*d₆*) δ ppm 8.92 (d, *J*=8.2Hz, 1H), 8.20 (d, *J* = 6.6 Hz, 0.65H), 7.69 (s, 0.5H), 7.62 (d, *J* = 9.4 Hz, 1.6H), 7.41 (d, *J* = 8.2 Hz, 0.7H), 7.11 (t, *J* = 11.5 Hz, 1.2H) 6.98 (s, 1.85H), 5.67 (s, 0.6H), 4.67 (s, 0.45H), 4.09-3.98 (m, 2H), 3.67-3.46 (m, 4H), 2.64 (d, *J* = 30.6 Hz, 3H), 2.53 (d, *J* = 2.6 Hz, 3H) 2.01-1.69 (m, 4H, *J* = 2.74). |
| | | | LCMS (ESI): [M+H]⁺ = 544.3 |
| 24 | 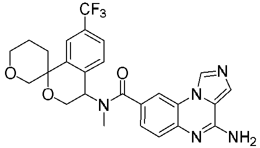 | 4-Amino-N-methyl-N-(7-(trifluoromethyl)-5',6'-dihydro-2'H,4'H-spiro[isochroman-1,3'-pyran]-4-yl)imidazo[1,5-a]quinoxaline-8-carboxamide | ¹H NMR (400 MHz, DMSO-*d*₆) δ 9.20 (d, *J* = 18.0 Hz, 1H), 8.37 (s, 1H), 8.13 (s, 0.4H), 7.93 (s, 1H), 7.83 (d, *J* = 22.0 Hz, 1H), 7.72 - 7.44 (m, 6H), 5.76 - 4.83 (m, 1H), 4.18 (d, *J* = 40.0 Hz, 2H), 3.84 (s, 2H), 3.58 (s, 2H), 2.77 (s, 3H), 2.12 - 1.86 (m, 3H), 1.46 (d, *J* = 12.0 Hz, 1H). |
| | | | LCMS (ESI) m/z: 512.3[M+H]⁺ |
| 34 | 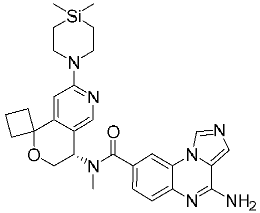 | (S)-4-amino-N-(7'-(4,4-dimethylazetidin-1-yl)-3',4'-dihydrospiro[cyclo butane-1,1'-pyrano[4,3-c]pyridine]-4'-yl)-N-methylimidazo[1,5 -a]quinoxaline-8-carboxamide | ¹H NMR (400 MHz, DMSO-d*₆*) δ 9.20 (s, 1H), 8.33 (s, 1H), 8.01 (d, J = 46.0 Hz, 1H), 7.91 (s, 1H), 7.46 (s, 2H), 7.40 (s, 2H), 6.72 (d, J = 21.6 Hz, 1H), 5.13 (d, J = 329.6 Hz, 1H), 4.02 - 3.74 (m, 6H), 2.72 (s, 3H), 2.55 (s, 1H), 2.31 (q, J = 10.9, 9.8 Hz, 2H), 1.98 (s, 2H), 0.72 (s, 4H), 0.07 (s, 6H). |
| | | | LCMS (ESI): [M+H]⁺ = 542.4 |
| 45 | 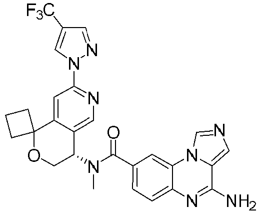 | (S)-4-amino-N-methyl-N-(7'-(4-(trifluoromethyl)-1H-pyrazol-1-yl)-3',4'-dihydrospiro[cyclo butane-1,1'-pyrano[4,3-c]pyridine]-4'-yl)imidazo[1,5-a]quinoxaline-8-carboxamide | ¹H NMR (400 MHz, DMSO-*d*₆) δ 9.19 (s, 2H), 8.59 - 8.37 (m, 2H), 8.34 (s, 1H), 8.08 (s, 1H), 7.92 (s, 1H), 7.57 - 7.45 (m, 2H), 7.43 (s, 2H), 5.35 (d, *J* = 315.2 Hz, 1H), 4.12 (d, *J =* 6.5 Hz, 2H), 2.79 (s, 3H), 2.61 (s, 1H), 2.49 - 2.32 (m, 3H), 2.09 (d, *J* = 32.5 Hz, 2H). F¹⁹NMR, δ -55.26 ppm. |
| | | | LCMS (ESI): [M+H]⁺ = 549.3 |
| 48 | 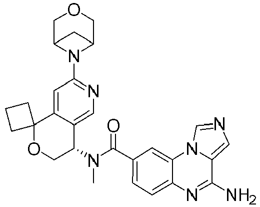 | N-((4'S)-7'-(3-oxa-6-azabicyclo[3.1.1]h eptane-6-yl)-3',4'-dihydrospiro[cyclo butane-1,1'-pyrano[4,3-c]pyridine]-4'-yl)-4-amino-N-methylimidazo[1,5 -a]quinoxaline-8-carboxamide | ¹H NMR (400 MHz, DMSO-*d*₆) δ 9.21 (s, 1H), 8.34 (s, 1H), 8.06 (d, *J* = 45.5 Hz, 1H), 7.91 (s, 1H), 7.46 (s, 2H), 7.41 (s, 2H), 6.62 (d, *J* = 19.9 Hz, 1H), 5.17 (d, *J* = 332.9 Hz, 1H), 4.39 (s, 2H), 4.19 (d, *J* = 14.2 Hz, 2H), 3.97 (d, *J* = 29.1 Hz, 2H), 3.68 (s, 2H), 2.73 (s, 3H), 2.68 (d, *J* = 6.8 Hz, 1H), 2.39 - 2.29 (m, 2H), 2.22 (s, 1H), 2.01 (s, 2H), 1.82 (d, *J* = 8.0 Hz, 1H). |
| | | | LCMS (ESI): [M+H]⁺ = 512.3 |
| 50 | 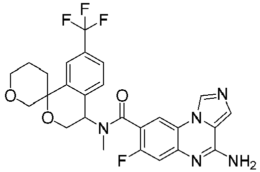 | 4-Amino-7-fluoro-N-methyl-N-(7-(trifluoromethyl)-5',6'-dihydro-2'H,4'H-spiro[isochroman-1,3'-pyran]-4-yl)imidazo[1,5-a]quinoxaline-8-carboxamide | ¹H NMR (400 MHz, DMSO-*d*₆) δ 9.15 -9.12 (m, 1H), 8.37 (m, *J* = 6.6 Hz, 1H), 7.93 (d, *J* = 2.2 Hz, 1H), 7.84 (d, *J* = 25.4 Hz, 1H), 7.73 (d, *J* = 8.1 Hz, 1H), 7.60 (s, 2H), 7.54 - 7.40 (m, 1H), 7.31 - 7.20 (m, 1H), 5.83 - 4.69 (m, 1H), 4.30 - 4.00 (m, 2H), 3.96 - 3.70 (m, 2H), 3.67 - 3.50 (m, 2H), 2.81 - 2.65 (m, 3H), 2.26 - 1.73 (m, 3H), 1.40 (dd, *J* = 45.0, 12.6 Hz, 1H). |
| | | | LCMS (ESI): [M+H]⁺ = 530.3 |
| 51 | 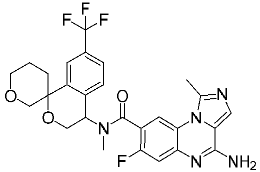 | 4-Amino-7-fluoro-N,1-dimethyl-N-(7-(trifluoromethyl)-5',6'-dihydro-2'H,4'H-pyran[isochroman-1,3'-pyran]-4-yl)imidazo[1,5-a]quinoxaline-8-carboxamide | ¹H NMR (400 MHz, DMSO-*d₆*) δ ppm 8.10 (dd, *J* = 22.7, 6.4 Hz, 1H), 7.91 - 7.78 (m, 2H), 7.72 (dq, *J* = 7.5, 4.8, 3.8 Hz, 1H), 7.59 - 7.42 (m, 3H), 7.31 - 7.17 (m, 1H), 5.75 (m, 0.5H), 4.92 - 4.71 (m, 0.5H), 4.33 - 4.00 (m, 2H), 4.01 - 3.70 (m, 2H), 3.70 - 3.48 (m, 2H), 2.96 (d, *J* = 5.9 Hz, 3H), 2.78 - 2.70 (m, 3H), 2.33 - 1.65 (m, 3H), 1.40 (m, 1H). LCMS (ESI): m/z [M+H]⁺ = 544.3 |
| 52 | 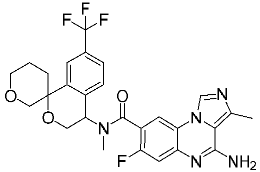 | 4-Amino-7-fluoro-N,3-dimethyl-N-(7-(trifluoromethyl)-5',6'-dihydro-2'H,4'H-pyran[isochroman-1,3'-pyran]-4-yl)imidazo[1,5-a]quinoxaline-8-carboxamide | ¹H NMR (400 MHz, DMSO-*d₆*) δ ppm 9.01 (dd, *J* = 9.6, 3.8 Hz, 1H), 8.60 - 8.17 (m, 1H), 8.02 - 7.63 (m, 2H), 7.61 - 7.34 (m, 1H), 7.32 - 7.09 (m, 1H), 7.05 (s, 2H), 5.77 (dt, *J* = 8.9, 4.7 Hz, 0.5H), 4.94 - 4.59 (m, 0.5H), 4.33 - 4.01 (m, 2H), 3.85-3.73 (m, 2H), 3.67-3.49 (m, 2H), 2.80 - 2.67 (m, 3H), 2.63 (d, *J* = 2.9 Hz, 3H), 2.32 - 1.77 (m, 3H), 1.40 (dd, *J* = 44.4, 12.5 Hz, 1H). |
| | | | LCMS (ESI): m/z [M+H]⁺ = 544.3 |

### Example 21 4-Amino-N-(1-methyl-1H-pyrazol-4-yl)-N-(7'-(trifluoromethyl)spiro[cyclobutane-1,1'-isochroman]-4'-yl)imidazo[1,5-a]quinoxaline-8-carboxamide

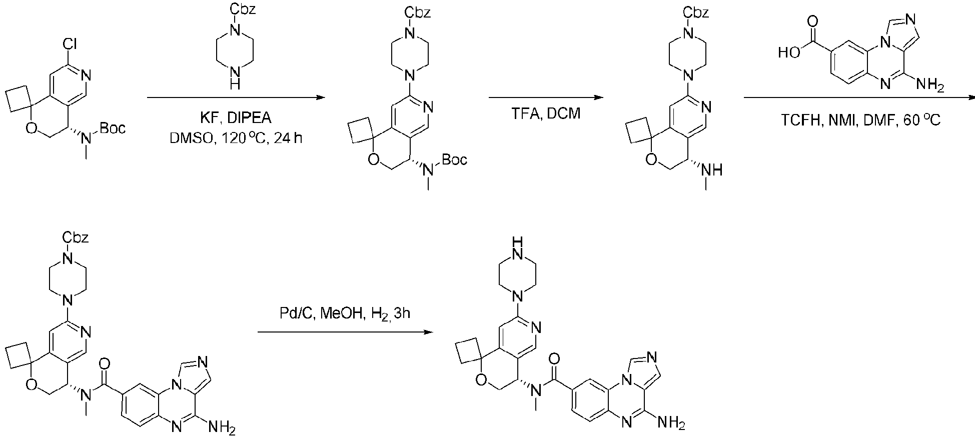

Step 1: Tert-butyl (S)-(7'-chloro-3'-,4'-dihydrospiro[cyclobutane-1,1'-pyrano[4,3-c]pyridine]-4'-yl)(methyl)carbamate (500 mg, 1.48 mmol) was dissolved in dimethyl sulfoxide (1.0 mL). Benzyl piperazine-1-carboxylate (975 mg, 4.43 mmol), potassium fluoride (87 mg, 1.48 mmol), and N, N-diisopropylethylamine (583 mg, 4.43 mmol) were added, and the mixture was stirred at 120°C for 48 h. After the reaction was complete, the reaction mixture was poured into water (40 mL) and extracted with ethyl acetate (60 mL×3). The organic phases were combined, dried over anhydrous sodium sulfate, and filtered. The filtrate was concentrated under reduced pressure to obtain a crude product, which was purified by column chromatography(petroleum ether: ethyl acetate = 3:1) to give benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (0.5 g, yield: 78.4%).

LCMS (ESI) m/z: [M+H]⁺ = 523.33.

Step 2: Benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (500 mg, 0.89 mmol) was dissolved in dichloromethane (3.00 mL). Trifluoroacetic acid (1.00 mL) was added, and the mixture was stirred at room temperature for 1 h. After the reaction was complete, the solvent was removed under vacuum and rotary-evaporated with an oil pump for 10 min. The crude product was directly used in the next step without purification to give benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (0.4 g, yield: 99.0%).

LCMS (ESI) m/z: [M+H]⁺ = 392.35.

Step 3: 4-Amino-10-hydroimidazo[1,5-a]quinoxaline-8-carboxylic acid (259 mg, 1.14 mmol) was dissolved in N,N-dimethylformamide (5.00 mL), and chloro-N,N,N',N'-tetramethylformamidinium hexafluorophosphate (325 mg, 1.14 mmol), N-methylimidazole (392 mg, 4.73 mmol), and N,N-diisopropylethylamine (374 mg, 2.84 mmol) were added, respectively. The mixture was stirred at room temperature for 20 min, added with benzyl (S)-4-(4'-(tert-butoxycarbonyl)(methyl)amino)-3'-dihydrospiro[cyclobutane-1,1'-pyrano[4,3-c]pyridin]-7'-yl)piperazine-1-carboxylate (400 mg, 0.95 mmol), and stirred at 60°C for 16 h. After the reaction was complete, the reaction mixture was poured into water (40 mL) and extracted with ethyl acetate (30 mL×3). The organic phases were combined, dried over anhydrous sodium sulfate, and filtered. The filtrate was concentrated under reduced pressure to obtain a crude product, which was purified by column chromatography(dichloromethane: methanol = 15:1) to give benzyl (S)-4-(4'-(4-amino-N-methylimidazo[1,5-a]quinoxaline-8-carboxamido)-3'-dihydrospiro[cyclobutane-1,1'-pyrano[4,3-c]pyridine]-7'-yl)piperazine-1-carboxylate (245 mg, yield: 41.0%).

LCMS (ESI) m/z: [M+H]⁺ = 633.35.

Step 4: Benzyl (S)-4-(4'-(4-amino-N-methylimidazo[1,5-a]quinoxaline-8-carboxamido)-3'-dihydrospiro[cyclobutane-1,1'-pyrano[4,3-c]pyridine]-7'-yl)piperazine-1-carboxylate (20 mg, 0.03 mmol) was dissolved in trifluoroacetic acid (1.00 mL), and the reaction mixture was stirred at 50°C for 16 h. After the reaction was complete, the solvent was removed under vacuum. The residue was purified by preparative high performance liquid chromatography, eluting with 10%-90% acetonitrile/water (containing 0.1% ammonium bicarbonate), and freeze-dried to give (S)-4-amino-N-methyl-N-(7'-(piperazin-1-yl)-3'-,4'-dihydrospiro[cyclobutane-1,1'-pyrano[4,3-c]pyridine]-4'-yl)imidazo[1,5-a]quinoxaline-8-carboxamide (3.37 mg, yield: 20.0%) as a white solid.

LCMS (ESI) m/z: [M+H]⁺ = 499.30.

¹H NMR (400 MHz, DMSO-*d*₆) δ 9.21 (s, 1H), 8.33 (s, 1H), 8.04 (d, *J* = 47.4 Hz, 1H), 7.90 (s, 1H), 7.43 (d, *J* = 17.8 Hz, 4H), 6.79 (d, *J* = 22.6 Hz, 1H), 5.60-4.69 (m, 1H), 3.95 (d, *J* = 30.0 Hz, 2H), 3.45 (s, 4H), 2.79 (s, 4H), 2.71 (s, 3H), 2.36-2.27 (m, 3H), 2.06-1.93 (m, 3H), 1.45-0.85 (m, 1H).

After chiral SFC resolution, the compounds in the following table are obtained:

| Example No. | Chemical Structure | Chemical Name | SFC condition |
|---|---|---|---|
| 3 | 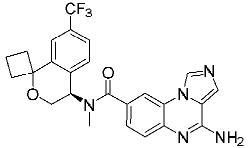 | (R)-4-amino-N-methyl-N-(7'-(trifluoromethyl)sp iro[cyclobutane-1,1'-isochroman]-4'-yl)imidazo[1,5-a]quinoxaline-8-carboxamide | Prepeak: SFC (column: Daicel ChiralCel OD, 40 mm I.D. × 250 mm, 10 µm), conditions: A for carbon dioxide and B for methanol (0.1% aqueous ammonia), 25%/25%, flow rate: 140 mL/min) |
| 4 | 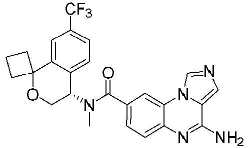 | (R)-4-amino-N-methyl-N-(7'-(trifluoromethyl)sp iro[cyclobutane-1,1'-isochroman]-4'-yl)imidazo[1,5-a]quinoxaline-8-carboxamide | Postpeak: SFC (column: Daicel ChiralCel OD, 40 mm I.D. × 250 mm, 10 µm), conditions: A for carbon dioxide and B for methanol (0.1% aqueous ammonia), 25%/25%, flow rate: 140 mL/min) |
| 14 | 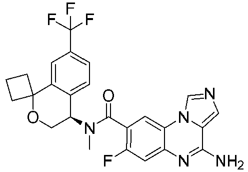 | (R)-4-amino-7-fluoro-N-methyl-N-(7'-(trifluoromethyl)sp iro[cyclobutane-1,1'-isochroman]-4'-yl)imidazo[1,5-a]quinoxaline-8-carboxamide | Prepeak: SFC (column: Daicel ChiralPak IC, 40 mm I.D. × 250 mm, 10 µm), conditions: A for carbon dioxide and B for methanol (0.1% aqueous ammonia), 40%/40%, flow rate: 140 mL/min) |
| 15 | 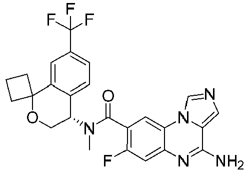 | (S)-4-amino-7-fluoro-N-methyl-N-(7'-(trifluoromethyl)sp iro[cyclobutane-1,1'-isochroman]-4'-yl)imidazo[1,5-a]quinoxaline-8-carboxamide | Postpeak: SFC (column: Daicel ChiralPak IC, 40 mm I.D. × 250 mm, 10 µm), conditions: A for carbon dioxide and B for methanol (0.1% aqueous ammonia), 40%/40%, flow rate: 140 mL/min) |
| 53 | 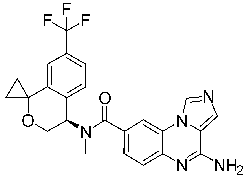 | (R)-4-amino-N-methyl-N-(7'-(trifluoromethyl)sp iro[cyclopropane-1,1'-isochroman]-4'-yl)imidazo[1,5-a]quinoxaline-8-carboxamide | Prepeak: SFC (column: Daicel chiralPak IBN (44 mm*250 mm, 10 µm), conditions: A for carbon dioxide and B for methanol (0.1% aqueous ammonia), 35%/35%, flow rate: 140 mL/min) |
| 54 | 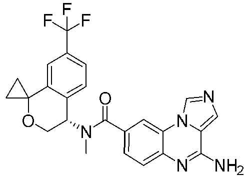 | (S)-4-amino-N-methyl-N-(7'-(trifluoromethyl)sp iro[cyclopropane-1,1'-isochroman]-4'-yl)imidazo[1,5-a]quinoxaline-8-carboxamide | Postpeak: SFC (column: Daicel chiralPak IBN (44 mm*250 mm, 10 µm), conditions: A for carbon dioxide and B for methanol (0.1% aqueous ammonia), 35%/35%, flow rate: 140 mL/min) |
| 100 | 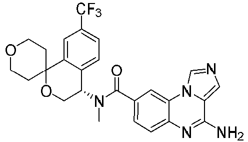 | (S)-4-amino-N-methyl-N-(7'-(trifluoromethyl)-2',3',5',6'-tetrahydrospiro[iso chroman-1,4'-pyran]-4-yl)imidazo[1,5-a]quinoxaline-8-carboxamide | Prepeak: SFC (column: ChiralPak AD, 250×40 mm I.D., 10 µm), conditions: A for hexane and B for ethanol, 40%/40%, flow rate: 80 mL/min) |
| 101 | 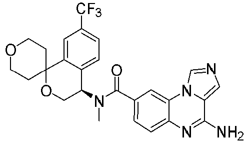 | (R)-4-amino-N-methyl-N-(7'-(trifluoromethyl)-2',3',5',6'-tetrahydrospiro[iso chroman-1,4'-pyran]-4-yl)imidazo[1,5-a]quinoxaline-8-carboxamide | Postpeak: SFC (column: ChiralPak AD, 250×40 mm I.D., 10 µm), conditions: A for hexane and B for ethanol, 40%/40%, flow rate: 80 mL/min) |

### Biochemical Evaluation

### I. Inhibitory Activity Test of the Compound on Tumor Cell Proliferation

### Test example 1: Inhibitory activity test of compounds on the proliferation of HCT-116 MTAP(- /-)deleted cells

Materials and cells: HCT-116 MTAP(-/-)deleted cells were purchased from Kyinno Biotechnology (China); RPMI-1640 medium, fetal bovine serum and penicillin-streptomycin were purchased from Thermo Fisher (United States); 384-well plates were purchased from PerkinElmer (United States); Cell-Titer Glo kit was purchased from Promega (United States).

Cell culture: HCT116 MTAP(-/-)deleted cells were cultured with RPMI 1640 medium containing 10% fetal bovine serum +1% penicillin-streptomycin at 37°C in 5% carbon dioxide. Only cells in the logarithmic growth phase could be used for the test.
Test of inhibitory activity on cell proliferation: The Cell-Titer Glo kit was used to test the proliferation inhibitory activity of the compounds on HCT-116 MTAP(-/-)deleted cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-10,000 nM (starting concentration 10,000 nM, 3-fold dilution, 10 points), and the cell plate with 0.1% DMSO content was incubated at 37°C and 5% carbon dioxide for 8 d. 40 µL of Cell-Titer Glo reagent was added to test cell viability. The test results are shown in Table 1.

### Test example 2: Inhibitory activity test of compounds on HCT-116 wild-type cells proliferation

Materials and cells: HCT-116 wild-type cells were purchased from Kyinno Biotechnology (China); RPMI-1640 medium, fetal bovine serum and penicillin-streptomycin were purchased from Thermo Fisher (United States); 384-well plates were purchased from PerkinElmer (United States); Cell-Titer Glo kit was purchased from Promega (United States).

Cell culture: HCT-116 wild-type cells were cultured with a RPMI-1640 medium containing 10% fetal bovine serum+1% penicillin-streptomycin at 37°C in 5% carbon dioxide. Only cells in the logarithmic growth phase could be used for the test.

Test of cell proliferation activity: Cell-Titer Glo kit was used to test the proliferation inhibitory activity of the compound on HCT-116 wild-type cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-10,000 nM (starting concentration 10,000 nM, 2-fold dilution, 10 points), and the cell plate with 0.1% DMSO content was incubated at 37°C and 5% carbon dioxide for 8 d. 40 µL of Cell-Titer Glo reagent was added to test cell viability. The test results are shown in Table 1.

Table 1 below shows the inhibitory activities of the compounds of the Examples against the proliferation of HCT116 MTAP(-/-)deleted cells and HCT116 wild-type cells.

**Table 1**

| Examples | IC₅₀ (nM) | |
|---|---|---|
| | HCT-116 MTAP(-/-)deleted | HCT-116 wild-type |
| 1 | 10 | 959.2 |
| 2 | 5.3 | 597.4 |
| 3 | 5.1 | 636 |
| 6 | 109.7 | 10000 |
| 7 | 5.1 | 636 |
| 8 | 9 | 3931.7 |
| 9 | 6.5 | 2223.7 |
| 12 | 7.64 | 1727 |
| 13 | 5.3 | 597.4 |
| 16 | 5.9 | 1612.9 |
| 17 | 5.17 | 324 |
| 19 | 21.7 | 2707 |
| 20 | 9.46 | 920 |
| 21 | >1000 | >20000 |
| 23 | 51.5 | 4521 |
| 28 | 110 | >10000 |
| 34 | 40 | 5248 |
| 45 | 34.1 | 7047 |
| 48 | 144 | >20000 |
| 51 | 8.02 | 1830 |
| 52 | 8.06 | 602 |
| 53 | 419 | 7586 |
| 54 | 5.8 | 1038 |
| 98 | 47.9 | >10000 |
| 99 | NA | NA |
| 100 | 5.2 | 902.5 |
| 101 | 402.7 | 9486.2 |

| | | |
|---|---|---|
| NA indicates not tested. | | |

Table 1 shows that the compounds in the examples described herein potently inhibit the proliferation of MTAP-deficient HCT116 cells and exhibit good selectivity over MTAP wild-type HCT116 cells.

### Test example 3: Inhibitory activity assay of compounds on proliferation of LU99 MTAP(-/-)deleted cells

Materials and cells: LU99 MTAP deleted cell line was purchased from Kyinno Biotechnology (China); cell culture medium and fetal bovine serum were purchased from Thermo Fisher Scientific (USA); 384-well plates were purchased from PerkinElmer (USA); Cell-Titer Glo kits were purchased from Promega (USA). Cell culture: LU99 MTAP(-/-)deleted cells were cultured with medium containing 10% fetal bovine serum at 37°C in 5% carbon dioxide. Only cells in the logarithmic growth phase could be used for the test.

Test of inhibitory activity on cell proliferation: The Cell-Titer Glo kit was used to test the proliferation inhibitory activity of the compounds on LU99 MTAP(-/-)deleted cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-1,000 nM (starting concentration 1,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 2.

### Test example 4: Inhibitory activity assay of compounds on proliferation of LU99 MTAP overexpressing cells

Materials and cells: LU99 MTAP overexpressing cell line was purchased from Kyinno Biotechnology (China); cell culture medium and fetal bovine serum were purchased from Thermo Fisher Scientific (USA); 384-well plates were purchased from PerkinElmer (USA); Cell-Titer Glo kits were purchased from Promega (USA).

Cell culture: LU99 MTAP overexpressing cells were cultured with medium containing 10% fetal bovine serum at 37°C in 5% carbon dioxide. Only cells in the logarithmic growth phase could be used for the test.

Test of inhibitory activity on cell proliferation: The Cell-Titer Glo kit was used to test the proliferation inhibitory activity of the compounds on LU99 MTAP overexpressing cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-10,000 nM (starting concentration 10,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 2.

**Table 2**

| Examples | IC₅₀ (nM) | |
|---|---|---|
| | Lu99 MTAP(-/-)Deleted | Lu99 MTAP Overexpressing |
| 1 | 13.2 | 651.7 |
| 2 | 7.8 | 773.1 |
| 3 | 8.3 | 623.6 |
| 4 | 1103.5 | 741.8 |
| 5 | 961.1 | 2359.6 |
| 6 | 88.5 | 3882.3 |
| 7 | 8.3 | 623.6 |
| 8 | 13.4 | 2313.4 |
| 9 | 10.9 | 1979.6 |
| 12 | 8.8 | 1636.4 |
| 13 | 7.8 | 773.1 |
| 15 | 7.1 | 424.9 |
| 16 | 10.8 | 1293.5 |
| 17 | 4.5 | 104.5 |
| 18 | 99.6 | 755.8 |
| 19 | 31.3 | 1393 |
| 20 | 20.5 | 1065.3 |
| 21 | >1000 | 12481 |
| 23 | 66.1 | 3968 |
| 24 | 9.54 | 1467 |
| 28 | 88.5 | 3882 |
| 34 | 41.5 | 1427 |
| 45 | 30.2 | 4709 |
| 48 | 150 | 10639 |
| 50 | 7.13 | 591 |
| 51 | 14 | 1322 |
| 52 | 14.9 | 539 |
| 53 | 682 | 1753 |
| 54 | 8.11 | 1103 |
| 98 | 57.5 | 8101.9 |
| 99 | 152.5 | 2971.3 |
| 100 | 8.5 | 938.1 |
| 101 | >1000 | 5644 |

Table 2 shows that the compounds in the examples described herein potently inhibit the proliferation of MTAP-deficient LU99 cells and exhibit good selectivity over MTAP-overexpressing LU99 cells.

### II. Pharmacokinetic Test in Mice

### 1. Test Compounds

The test compounds are compounds according to examples of the present disclosure

### 2. Test Animals

ICR male mice N=3/group; original source: Zhejiang Vital River Laboratory Animal Technology Co., Ltd.

### 3. Drug Preparation and Administration

Single oral administration (PO) to ICR mice: The compound was weighed and dissolved with DMSO, a certain volume of polyethylene glycol 400 and water for injection (WFI) were added, and then a small volume of 1 mol/L hydrochloric acid solution was added to adjust to a clear solution. A total of 3 mice were intragastrically administered after fasting overnight at a dose of 10 mg/kg.

Single intravenous (IV) administration to ICR mice: The compound was weighed and dissolved with DMSO, a certain volume of polyethylene glycol 400 and water for injection (WFI) were added, and then a small volume of 1 mol/L hydrochloric acid solution was added to adjust to a clear solution. The administration via tail vein injection was conducted for a total of 3 mice after fasting overnight at a dose of 3 mg/kg.

### 4. Sampling

Blood was collected from the dorsalis pedis vein at approximately 30 µL/time point, anticoagulated with dipotassium ethylenediaminetetraacetate and kept on ice until plasma was separated by centrifugation (4000 g/min; 5 min; 4°C) within 1 h. The blood sampling time points were 0.0833 (intravenous injection), 0.25, 0.5, 1, 2, 4, 6, 8, and 24 h. The samples were stored in a refrigerator at -20°C.

A total of 30 µL of plasma sample (10 µL sample + 20 µL blank plasma sample) was added with 200 µL ice-cold ACN containing internal standard, vortexed for 30 s, and centrifuged at 4000 g/min for 20 min. A total of 100 µL of the supernatant was transferred to a 96-well plate, 200 µL of ultrapure water was added, vortexed for 30 s, and 5 µL or 10 µL was injected into LC-MS/MS for analysis.

**Table 2: Pharmacokinetic Data**

| Example No. | Mode of Administration (Dose) | AUCₗₐₛₜ (hr*ng/ml) | T_{1/2}(h) | Bioavailability (F) (%) |
|---|---|---|---|---|
| | IV(3mg/kg) | 25405 | 2.37 | |
| **54** | PO(30mg/kg) | 219857 | 1.47 | 86.5 |
| | PO(100mg/kg) | 408057 | 2.06 | 48.2 |

Although preferred examples have been described hereinabove, it will be apparent to those skilled in the art that modifications may be made without departing from the present disclosure. Such modifications are considered to be possible variants encompassed within the scope of the present disclosure.

## Claims

1. A compound of formula (I), or a pharmaceutically acceptable salt, ester, prodrug, stereoisomer, or isotopic derivative thereof, 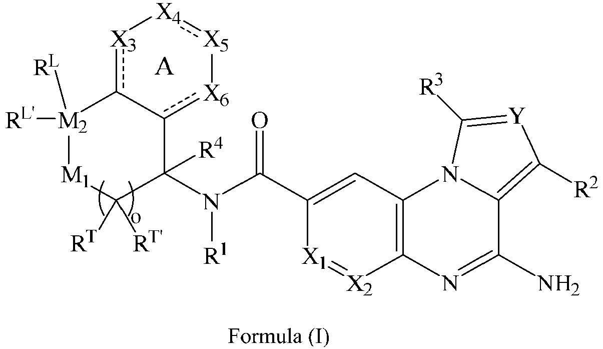
wherein, the dashed line represents a single bond or double bond;
wherein, X₁ represents N or CR^{X1};
wherein, X₂ represents N or CR^{X2};
wherein, X₃ represents N or CR^{X3};
wherein, X₄ represents N or CR^{X4};
wherein, X₅ represents N or CR^{X5};
wherein, X₆ represents N or CR^{X6};
wherein, Y represents CH or N;
wherein, R⁴ represents H or D;
wherein, R^{X1} and R^{X2} each independently represent hydrogen, deuterium, C₁-C₆ alkyl, C₁-C₆ deuterated alkyl, C₁-C₆ haloalkyl, C₁-C₆haloalkoxy, hydroxy (C₁-C₆) alkyl, C₂-C₆ alkenyl, C₂-C₆ alkynyl, -OR^{a}, -SR^{a}, -S(O)₂R^{a}, - S(O)R^{a}, -S(O)(=NR^{a})R^{b}, -CN, -OC(O)R^{a}, -OCONR^{a}R^{b}, halogen, -OSO₃R^{a}, -NR^{a}R^{b}, -SF₅, -C(O)OR^{a} and - C(O)ONR^{a}R^{b};
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, - CO₂NR^{a}R^{b}, or a group selected from C₃-C₁₀ cycloalkyl, C₆-C₁₀ cycloalkenyl, 4-10-membered saturated or unsaturated heterocyclyl, C6-C10 aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}C(O)R^{b} or-C(O)NR^{a}R^{b};
wherein, R^{X4} represents -L-R^{X4-1};
wherein, L represents absent or CR^{a}R^{b}, SiR^{a}R^{b}, O, S, Se, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} or -C(O)NR^{a}R^{b};
wherein, said ring A may also be optionally fused with a 5- to 6-membered saturated or unsaturated ring at the chemical bond between X₃ and X₄, which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, - SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, the ring A may also optionally be fused with the chemical bond between X₄ and X₅ to form a 5-6-membered saturated or unsaturated ring which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, - S(O)(=NR^{a})R^{b}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, - SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, -NR^{a}COR^{b} and - C(O)NR^{a}R^{b};
wherein, R² and R³ each independently represent hydrogen, deuterium, C₁-C₆ alkyl, halogen, hydroxyl, carboxyl, C₁-C₆ haloalkyl, hydroxy (C₁-C₆) alkyl or mercapto (C₁-C₆) alkyl;
wherein, M₁ represents CR^{a}R^{b}, -SiR^{a}R^{b}, NR^{a}, O, S or Se;
wherein, M₂ represents C or Si;
wherein, R^{L} and R^{L'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom which they are attached , form a 3-10-membered saturated or unsaturated ring which may optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom which they are attached , form a 3-10-membered saturated or unsaturated ring which may also optionally contain 0, 1 or 2 heteroatoms selected from O, N, S and Se; further, the ring may optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SFs;
wherein, at least one pair among R^{L}/R^{L'} and R^{T}/R^{T'} forms a 3-10-membered saturated or unsaturated ring with the atom which they are attached , and the ring may also optionally contain 0, 1, 2 heteroatoms selected from O, N, S and Se; further, the ring may optionally be substituted with 0, 1, 2 substituents independently selected from deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, - OC(O)R^{a}, -OCONR^{a}R^{b}, halogen, -OSO₃R^{a}, -NR^{a}R^{b} and -SF₅;
wherein, o represents 0, 1 or 2;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom which they are attached , form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

2. A compound of formula (I-2), or a pharmaceutically acceptable salt, ester, prodrug, stereoisomer or isotopic derivative thereof, 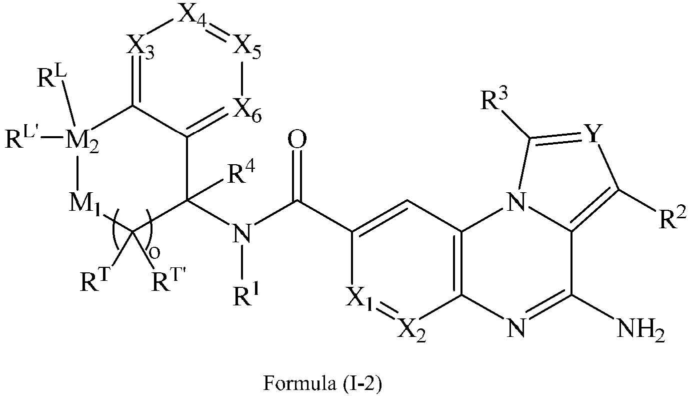
wherein, X₁ represents N or CR^{X1};
wherein, X₂ represents N or CR^{X2};
wherein, X₃ represents N or CR^{X3};
wherein, X₄ represents N or CR^{X4};
wherein, X₅ represents N or CR^{X5};
wherein, X₆ represents N or CR^{X6};
wherein, Y represents CH or N;
wherein, R⁴ represents H or D;
wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, - S(O)R^{a}, -S(O)(=NR^{a})R^{b}, -CN, -OC(O)R^{a}, -OCONR^{a}R^{b}, halogen, -OSO₃R^{a}, -NR^{a}R^{b}, -SF₅, -C(O)OR^{a} and - C(O)ONR^{a}R^{b};
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, - CO₂NR^{a}R^{b}, or a group selected from C₃-C₁₀ cycloalkyl, C₆-C₁₀ cycloalkenyl, 4-10-membered saturated or unsaturated heterocyclyl, C6-C10 aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, R^{X4} represents -L-R^{X4-1};
wherein, L represents absent or CR^{a}R^{b}, SiR^{a}R^{b}, O, S, Se, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, -NR^{a}COR^{b} and - C(O)NR^{a}R^{b};
wherein, R² and R³ each independently represent hydrogen, deuterium, C₁-C₆ alkyl, halogen, hydroxyl, carboxyl, C₁-C₆ haloalkyl, hydroxy (C₁-C₆) alkyl, or mercapto (C₁-C₆) alkyl;
wherein, M₁ represents CR^{a}R^{b}, -SiR^{a}R^{b}, NR^{a}, O, S or Se;
wherein, M₂ represents C or Si;
wherein, R^{L} and R^{L'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
wherein, at least one pair among R^{L}/R^{L}' and R^{T}/R^{T}' forms a 3-10-membered saturated or unsaturated ring with the atom connected thereto, and the ring may optionally contain 0, 1, 2 heteroatoms selected from O, N, S and Se; further, the ring may optionally be substituted with 0, 1, 2 substituents independently selected from deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, - OCONR^{a}R^{b}, halogen, -OSO₃R^{a}, -NR^{a}R^{b} and -SF₅;
wherein, o represents 0, 1 or 2;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

3. The compound of formula (I) according to claim 1 or formula (I-2) according to claim 2, or a pharmaceutically acceptable salt, ester, prodrug, stereoisomer or isotopic derivative thereof, wherein X¹ represents N.

4. The compound of formula (I) according to claim 1 or formula (I-2) according to claim 2, or a pharmaceutically acceptable salt, ester, prodrug, stereoisomer or isotopic derivative thereof, wherein X₁ represents CR^{X1}; wherein R^{X1} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂ or - N(CH₃)₂.

5. The compound of formula (I) according to claim 1 or formula (I-2) according to claim 2, or a pharmaceutically acceptable salt, ester, prodrug, stereoisomer or isotopic derivative thereof, wherein X² represents CR^{X2}; wherein R^{X2} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂ or - N(CH₃)₂; preferably, R^{X2} represents hydrogen or C₁-C₆ alkyl.

6. The compound of formula (I) according to claim 1 or formula (I-2) according to claim 2, or a pharmaceutically acceptable salt, ester, prodrug, stereoisomer or isotopic derivative thereof, wherein X³ represents N.

7. The compound of formula (I) according to claim 1 or formula (I-2) according to claim 2, or a pharmaceutically acceptable salt, ester, prodrug, stereoisomer or isotopic derivative thereof, wherein X³ represents CR^{X3}; wherein R^{X3} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or - N(CH₃)₂; preferably, R^{X3} represents hydrogen, halogen, C₁-C₆ alkyl or C₁-C₆ haloalkyl.

8. The compound of formula (I) according to claim 1 or formula (I-2) according to claim 2, or a pharmaceutically acceptable salt, ester, prodrug, stereoisomer or isotopic derivative thereof, wherein X⁵ represents N.

9. The compound of formula (I) according to claim 1 or formula (I-2) according to claim 2, or a pharmaceutically acceptable salt, ester, prodrug, stereoisomer or isotopic derivative thereof, wherein X⁵ represents CR^{X5}; wherein R^{X5} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or - N(CH₃)₂; preferably, R^{X5} represents hydrogen, halogen, C₁-C₆ alkyl or C₁-C₆ haloalkyl.

10. The compound of formula (I) according to claim 1 or formula (I-2) according to claim 2, or a pharmaceutically acceptable salt, ester, prodrug, stereoisomer or isotopic derivative thereof, wherein X⁶ represents N.

11. The compound of formula (I) according to claim 1 or formula (I-2) according to claim 2, or a pharmaceutically acceptable salt, ester, prodrug, stereoisomer or isotopic derivative thereof, wherein X⁶ represents CR^{X6}; wherein R^{X6} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or - N(CH₃)₂; preferably, R^{X6} represents hydrogen, halogen, C₁-C₆ alkyl, C₁-C₆ haloalkyl, C₁-C₆ haloalkoxy or C₁-C₆ haloalkylthio.

12. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, -C(O)OR^{a}, -OC(O)R^{a}, -OCONR^{a}R^{b}, -NR^{a}COR^{b}, -C(O)NR^{a}R^{b}, C₁-C₆ haloalkyl, C₁-C₆ haloalkoxy, C₁-C₆ haloalkylthio, hydroxy (C₁-C₆) alkyl or C₃-C₁₀ cycloalkyl, C₆-C₁₀ cycloalkenyl, 4-10-membered saturated or unsaturated heterocyclyl, C₆-C₁₀ aryl, or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b,} and -C(O)NR^{a}R^{b}.

13. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents hydrogen, deuterium, C₁-C₆ alkyl, halogen, C₁-C₆ haloalkyl, -CN, -NH₂, -NHCH₃, -N(CH₃)₂, -OH, -OCH₃, C₁-C₆ alkoxy, C₁-C₆ haloalkoxy, C₁-C₆ haloalkylthio, -SF₅, or -P(O)(CH₃)₂.

14. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents hydrogen, halogen (preferably F), CN, -CF₃, -CF₂CF₃, -NH₂, -NHCH₃, -N(CH₃)₂, -OCH₃, -OCF₃, -SF₅, -SCF₃, or -P(O)(CH₃)₂.

15. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b}.

16. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 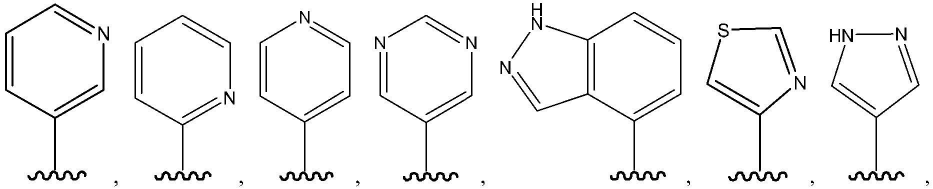 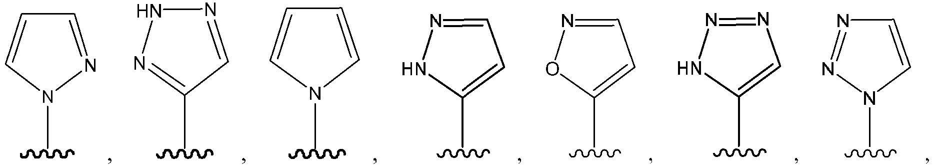 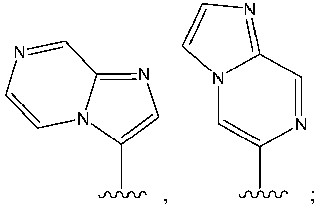 furthermore, R^{X4} may optionally be substituted with 0, 1 or 2 substituents selected from the group consisting of: halogen, hydroxyl, C₁-C₆ alkyl, C₁-C₆ haloalkyl and C₁-C₆ haloalkoxy.

17. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents C₃-C₁₀ cycloalkyl, C₆-C₁₀ cycloalkenyl, or 4-10 membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b}.

18. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents O, S, NH, or N(CH₃), and R^{X4-1} represents the following groups: 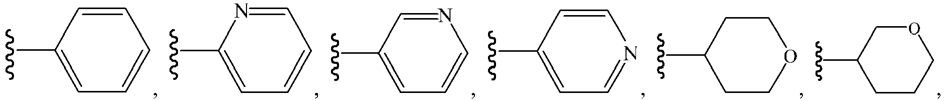 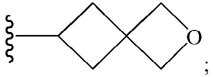 furthermore, R^{X4-1} may optionally be substituted with 0, 1 or 2 substituents selected from the group consisting of: halogen, hydroxyl, C₁-C₆ alkyl, C₁-C₆ haloalkyl and C₁-C₆ haloalkoxy.

19. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 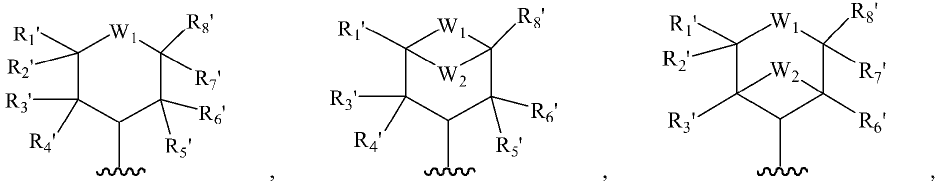 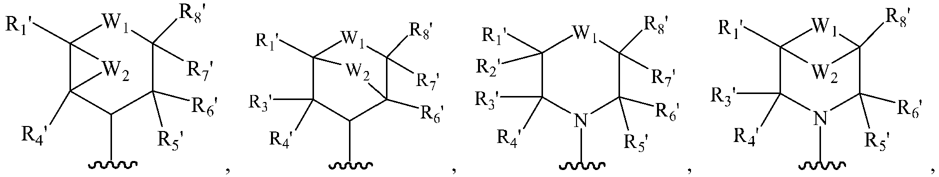 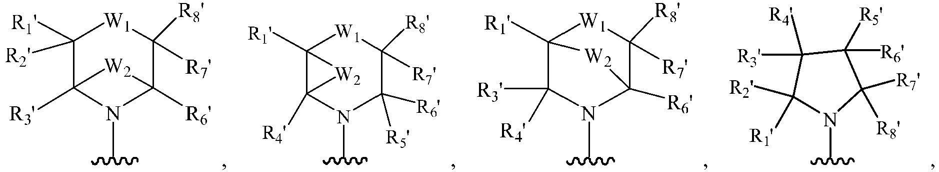 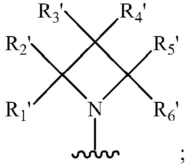
wherein, W₁ represents CR^{C}R^{D}, NR^{C}, O, S, or SiR^{C}R^{D};
wherein, W₂ represents -(CR^{M}R^{N})ᵢ;
wherein, R₁', R₂', R₃', R₄', R₅', R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
wherein, R^{C} and R^{D} each independently represent hydrogen, deuterium, halogen, C₁-C₆ alkyl or hydroxyl; or R^{S} and R^{T}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
wherein, R^{M} and R^{N} each independently represent hydrogen or C ₁-C₆ alkyl;
wherein, i represents an integer of 1 or 2.

20. The compound of formula (I) according to claim 1 or formula (I-2) according to claim 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 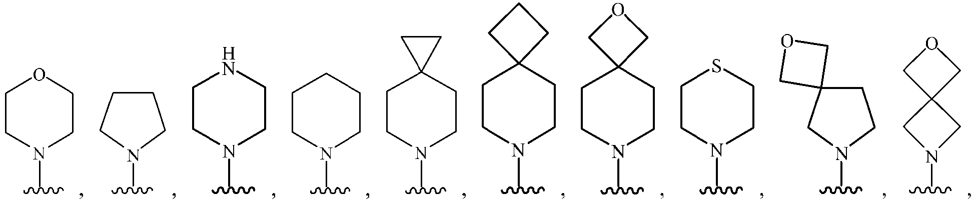 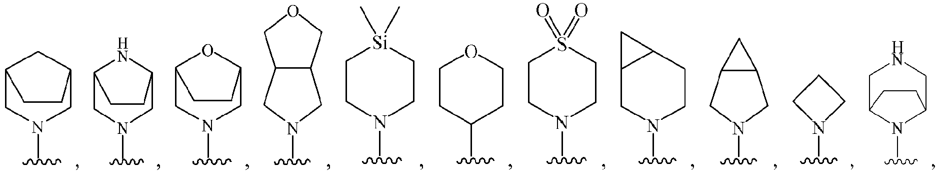 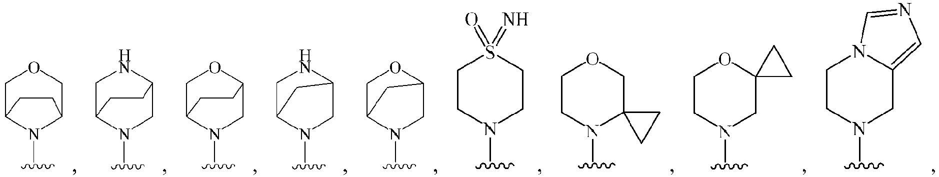 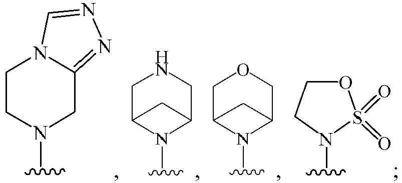 wherein, the R^{X4-1} may optionally be substituted with 0, 1, 2 substituents independently selected from halogen, C₀-C₆ hydroxyalkyl, C₀-C₆ cyanoalkyl, C₁-C₆ alkyl and C₁-C₆ haloalkyl.

21. The compound of formula (I) according to claim 1, or a pharmaceutically acceptable salt, ester, prodrug, stereoisomer, or isotopic derivative thereof, wherein X⁴ represents C-L-R^{X4-1}; wherein L represents absence, and R^{X4-1} represents the following groups:

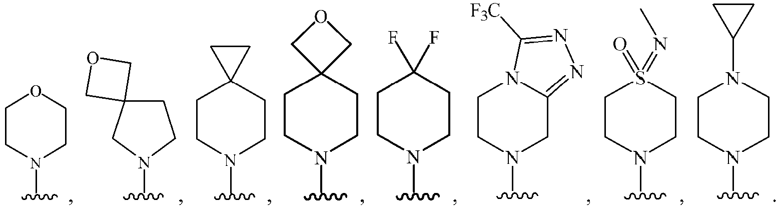

22. The compound of formula (I) according to claim 1 or formula (I-2) according to claim 2, or a pharmaceutically acceptable salt, ester, prodrug, stereoisomer or isotopic derivative thereof, wherein R^{L} and R^{L'}, together with the atom attached thereto, form a ring of the following structures: 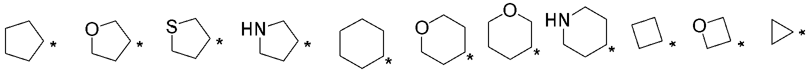 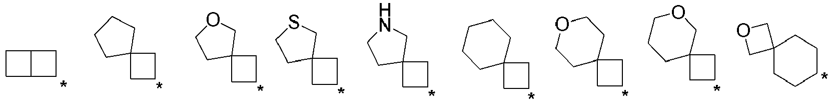 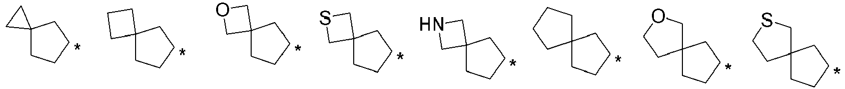 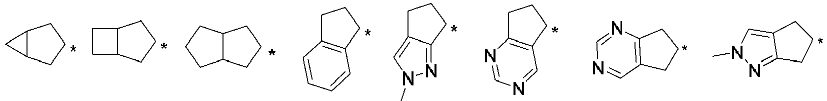 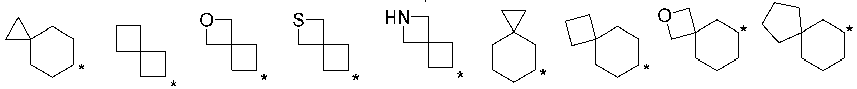 wherein, * represents an atomic site where R^{L} and R^{L'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and -SF₅.

23. The compound of formula (I) according to claim 1 or formula (I-2) according to claim 2, or a pharmaceutically acceptable salt, ester, prodrug, stereoisomer or isotopic derivative thereof, wherein R^{T} and R^{T}', together with the atom attached thereto, form a ring of the following structures: 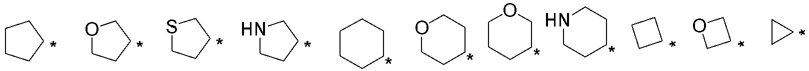 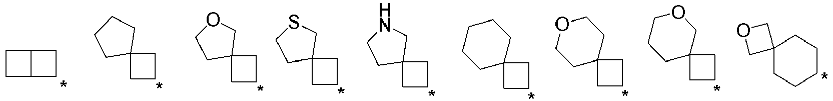 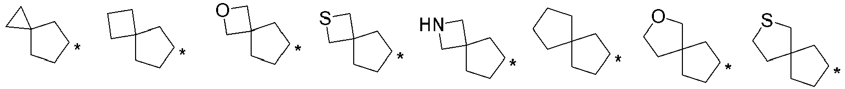 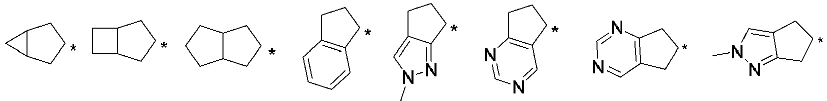 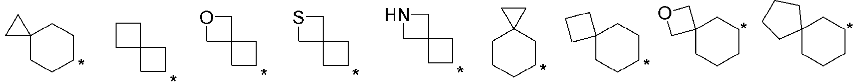 wherein, * represents an atomic site where R^{T} and R^{T'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b},

24. A compound having any one of the following structures, or a pharmaceutically acceptable salt, ester, prodrug, stereoisomer or isotopic derivative thereof, 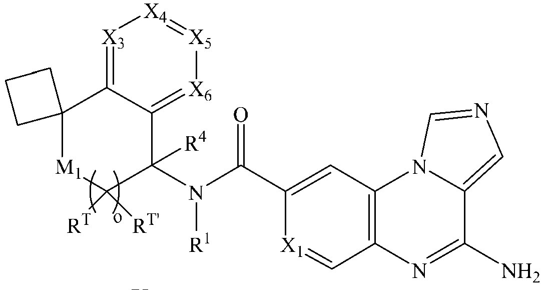 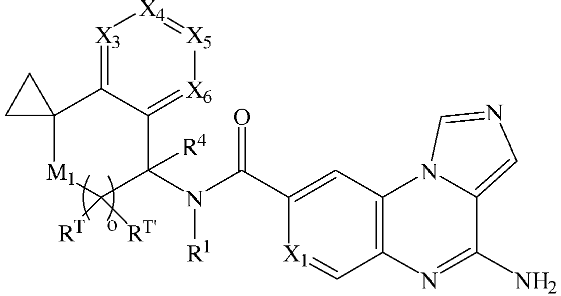 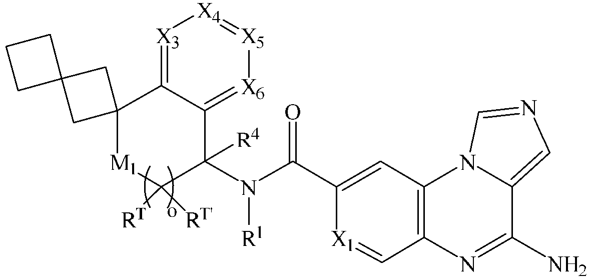 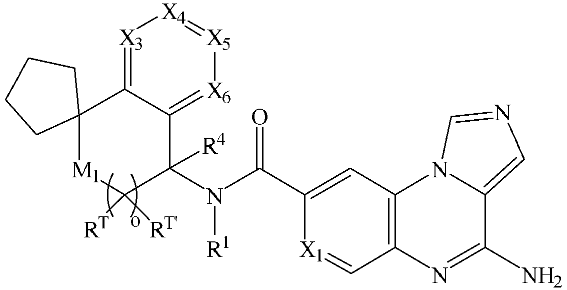 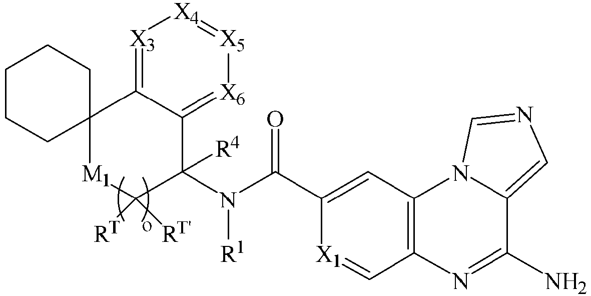 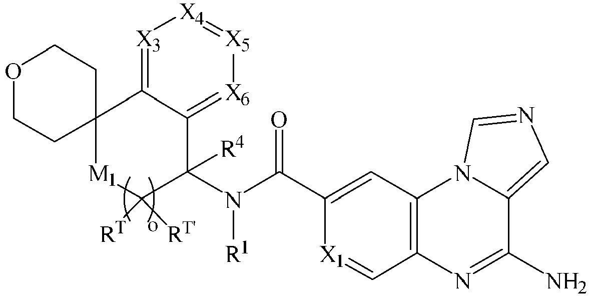 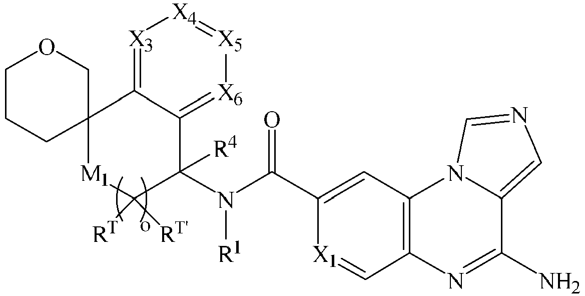 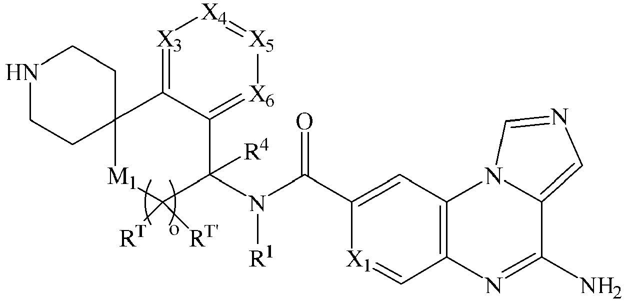 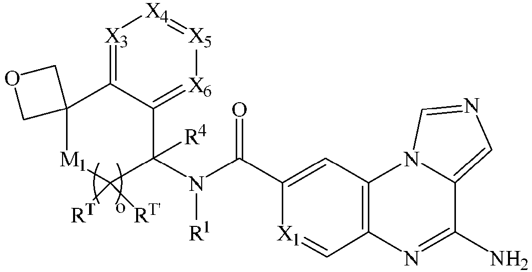 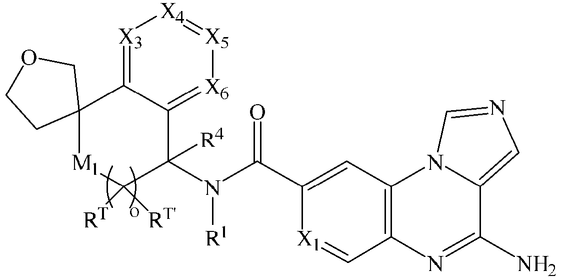 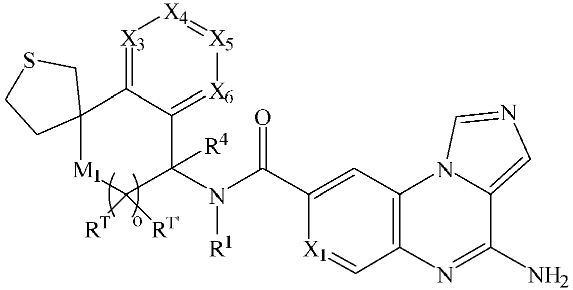
wherein, X₁ represents N or CR^{X1};
wherein, X₃ represents N or CR^{X3};
wherein, X₄ represents N or CR^{X4};
wherein, X₅ represents N or CR^{X5};
wherein, X₆ represents N or CR^{X6};
wherein, R⁴ represents H or D;
wherein, R^{X1} represents hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -S(O)(=NR^{a})R^{b}, -CN, -OC(O)R^{a}, -OCONR^{a}R^{b}, halogen, -OSO₃R^{a}, -NR^{a}R^{b} or-SF₅;
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, **4-10-membered** saturated or unsaturated heterocyclyl, C₆-C₁₀ aryl **or** 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b} - NR^{a}COR^{b} and -C(O)NR^{a}R^{b};
wherein, R^{X4} represents -L-R^{X4-1};
wherein, L represents absence or CR^{a}R^{b}, O, S, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, -NR^{a}COR^{b} and - C(O)NR^{a}R^{b};
wherein, M¹ represents CR^{a}R^{b}, NR^{a}, O, S or Se;
wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom which they are attached, form a 3-10-membered saturated or unsaturated ring which may optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SFs;
wherein, o represents 0, 1 or 2;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

25. A compound having any one of the following structures, or a pharmaceutically acceptable salt, ester, prodrug, stereoisomer, or isotopic derivative thereof, 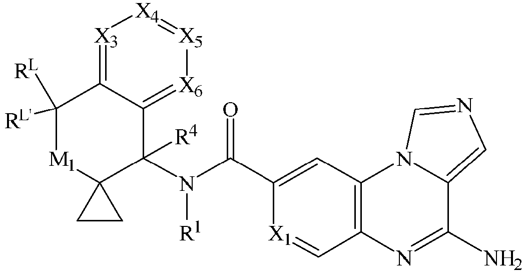 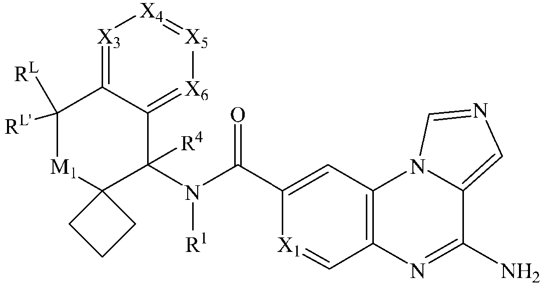 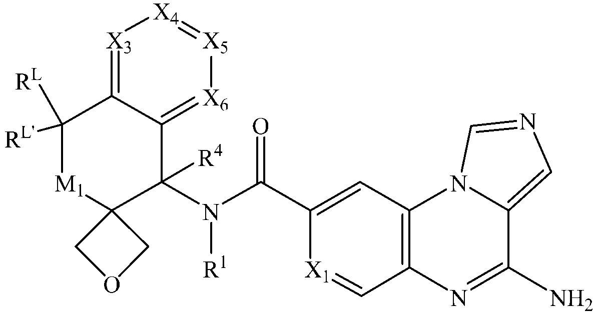 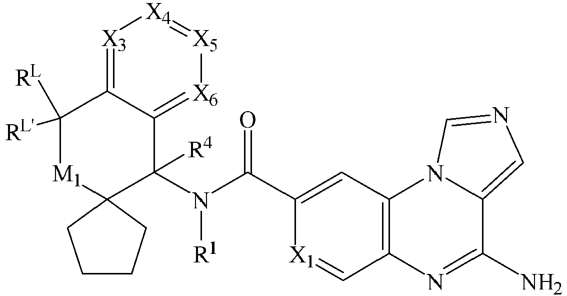
wherein, X₁ represents N or CR^{X1};
wherein, X₃ represents N or CR^{X3};
wherein, X₄ represents N or CR^{X4};
wherein, X₅ represents N or CR^{X5};
wherein, X₆ represents N or CR^{X6};
wherein, R⁴ represents H or D;
wherein, R^{X1} represents hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -S(O)(=NR^{a})R^{b}, -CN, -OC(O)R^{a}, -OCONR^{a}R^{b}, halogen, -OSO₃R^{a}, -NR^{a}R^{b} or -SF₅;
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b};
wherein, R^{X4} represents -L-R^{X4-1};
wherein, L represents absence or CR^{a}R^{b}, O, S, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b};
wherein, R¹ represents hydrogen or a group selected from C₁-C₆ alkyl, C₃-C₁₀ cycloalkyl, 4-10 membered heterocycloalkyl, C₆-C₁₀ aryl or 5-10 membered heteroaryl optionally substituted with 0-3 substituents selected from the group consisting of: deuterium, halogen, C₁-C₆ alkyl, C₂-C₆ alkenyl, C₂-C₆ alkynyl, C₃-C₁₀ cycloalkyl (e.g., C₃-C₆ cycloalkyl), substituted or unsubstituted 3-10-membered saturated or unsaturated heterocyclyl (e.g., 3-6-membered saturated or unsaturated heterocyclyl), substituted or unsubstituted C₆-C₁₀ aryl, substituted or unsubstituted 5-10-membered heteroaryl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -S(O) (=NR^{a})R^{b}, -SF₅, -C(O)R^{a}, -C(O)OR^{a}, -OC(O)R^{a}, - OC(O)NR^{a}R^{b}, -NR^{a}COR^{b} and -C(O)NR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -S(O)₂R^{a}, -S(O)R^{a}, -S(O)(=NR^{a})R^{b}, -SF₅, -C(O)R^{a}, -C(O)OR^{a}, -OC(O)R^{a}, -OC(O)NR^{a}R^{b}, -NR^{a}COR^{b} and - C(O)NR^{a}R^{b};
wherein, M¹ represents CR^{a}R^{b}, NR^{a}, O, S or Se;
wherein, R^{L} and R^{L'}each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{L} and R^{L'}, together with the atom which they are attached, form a 3-10-membered saturated or unsaturated ring which may optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -S(O)(=NR^{a})R^{b}, -CN, -C(O)OR^{a}, -OC(O)R^{a}, -OCONR^{a}R^{b}, halogen, -OSO₃R^{a}, -NR^{a}R^{b} and - SF₅;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

26. The compound according to claim 24 or 25, pharmaceutically acceptable salts, esters, prodrugs, stereoisomers or isotopic derivatives thereof, wherein X¹ represents N.

27. The compound according to claim 24 or 25, or a pharmaceutically acceptable salt, ester, prodrug, stereoisomer, or isotopic derivative thereof, wherein X₁ represents CR^{X1}; wherein R^{X1} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂.

28. The compound according to claim 24 or 25, or a pharmaceutically acceptable salt, ester, prodrug, stereoisomer, or isotopic derivative thereof, wherein X³ represents N.

29. The compound according to claim 24 or 25, or a pharmaceutically acceptable salt, ester, prodrug, stereoisomer, or isotopic derivative thereof, wherein X³ represents CR^{X3}; wherein R^{X3} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X3} represents hydrogen, halogen, C₁-C₆ alkyl , or C₁-C₆ haloalkyl.

30. The compound according to claim 24 or 25, or a pharmaceutically acceptable salt, ester, prodrug, stereoisomer, or isotopic derivative thereof, wherein X⁵ represents N.

31. The compound according to claim 24 or 25, or a pharmaceutically acceptable salt, ester, prodrug, stereoisomer, or isotopic derivative thereof, wherein X⁵ represents CR^{X5}; wherein R^{X5} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X5} represents hydrogen, halogen, C₁-C₆ alkyl, or C₁-C₆ haloalkyl.

32. The compound according to claim 24 or 25, or a pharmaceutically acceptable salt, ester, prodrug, stereoisomer, or isotopic derivative thereof, wherein X⁶ represents N.

33. The compound according to claim 24 or 25, or a pharmaceutically acceptable salt, ester, prodrug, stereoisomer, or isotopic derivative thereof, wherein X⁶ represents CR^{X6}; wherein R^{X6} represents hydrogen, deuterium, halogen, C₁-C₆ alkyl, C₂-C₆ alkenyl, C₂-C₆ alkynyl, C₁-C₆ haloalkyl, C₃-C₆ cycloalkyl, hydroxyl, C₁-C₆ alkoxy, C₁-C₆ haloalkoxy, cyano, NH₂, or -N(CH₃)₂; preferably, R^{X6} represents hydrogen, halogen, C₁-C₆ alkyl, or C₁-C₆ haloalkyl.

34. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -S(O)(=NR^{a})R^{b}, -SF₅, -NR^{a}R^{b}, C₁-C₆ haloalkyl, C₁-C₆ haloalkoxy, C₁-C₆ haloalkylthio, hydroxy (C₁-C₆) alkyl, or C₃-C₁₀ cycloalkyl, C₆-C₁₀ cycloalkenyl, 4-10-membered saturated or unsaturated heterocyclyl, C₆-C₁₀ aryl, or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -C(O)NR^{a}R^{b}.

35. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents hydrogen, deuterium, C₁-C₆ alkyl, halogen, C₁-C₆ haloalkyl, -CN, -NH₂, -NHCH₃, - N(CH₃)₂, -OH, -OCH₃, C₁-C₆ alkoxy, C₁-C₆ haloalkoxy, -SF₅, or -P(O)(CH₃)₂.

36. The compound according to claim 24, or a pharmaceutically acceptable salt, ester, prodrug, stereoisomer, or isotopic derivative thereof, wherein X⁴ represents C-L-R^{X4-1}; wherein L represents absence, and R^{X4-1} represents hydrogen, halogen (preferably F), -CF₃, -NH₂, -NHCH₃, -N(CH₃)₂, -OCH₃, -OCF₃, -SF₅, or - P(O)(CH₃)₂.

37. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b}.

38. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents the following groups: 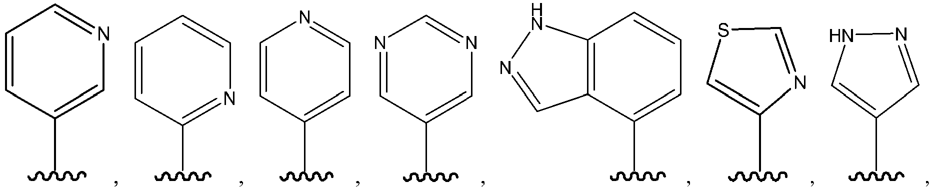 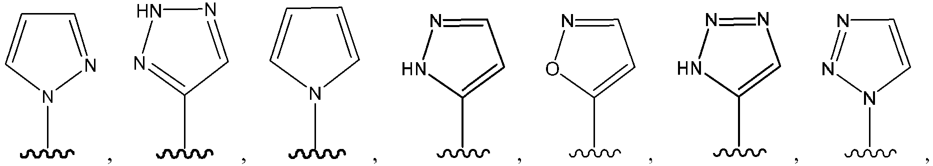 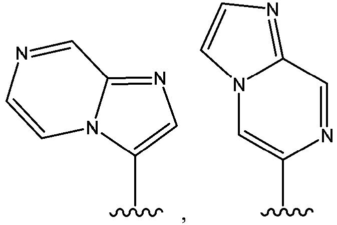 furthermore, R^{X4} may optionally be substituted with 0, 1 or 2 substituents selected from the group consisting of: halogen, hydroxyl, -CN, C₁-C₆ alkyl, C₁-C₆ haloalkyl and C₁-C₆ haloalkoxy.

39. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents C₃-C₁₀ cycloalkyl, C₆-C₁₀ cycloalkenyl or 4-10-membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, - CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -C(O)NR^{a}R^{b}.

40. The compound according to claim 24 or 25, or a pharmaceutically acceptable salt, ester, prodrug, stereoisomer, or isotopic derivative thereof, wherein X⁴ represents C-L-R^{X4-1}; wherein L represents O, S, NH and N(CH₃), and R^{X4-1} represents the following groups: 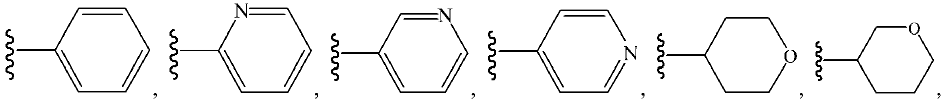 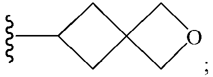 furthermore, R^{X4-1} may optionally be substituted with 0, 1 or 2 substituents selected from the group consisting of: halogen, hydroxyl, -CN, C₁-C₆ alkyl, C₁-C₆ haloalkyl and C₁-C₆ haloalkoxy.

41. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents the following groups: 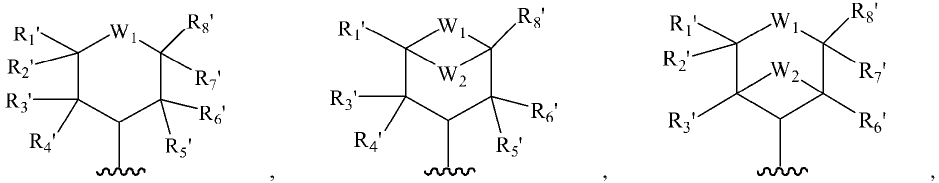 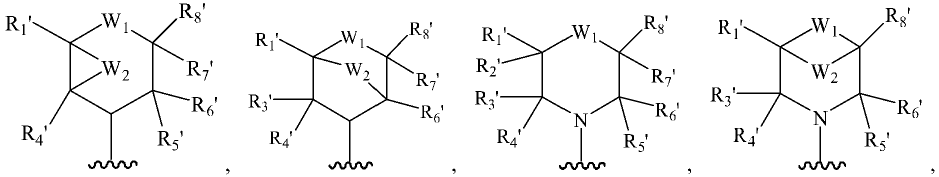 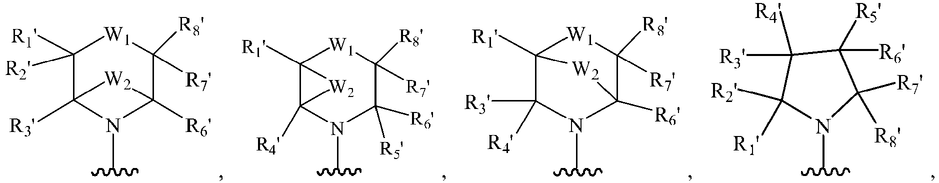 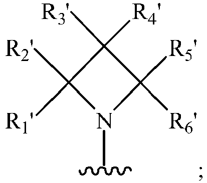
wherein, W₁ represents CR^{C}R^{D}, NR^{C}, O, or S;
wherein, W₂ represents -(CR^{M}R^{N})ᵢ-;
wherein, R₁', R₂', R₃', R₄', R₅'^{,} R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl and hydroxyl;
wherein, R^{C} and R^{D} each independently represent hydrogen, deuterium, halogen, C₁-C₆ alkyl or hydroxyl; or R^{S} and R^{T}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl,
C₁-C₆ haloalkyl and hydroxyl;
wherein, R^{M} and R^{N} each independently represent hydrogen or C₁-C₆ alkyl;
wherein, i represents an integer of 1 or 2.

42. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents the following groups: 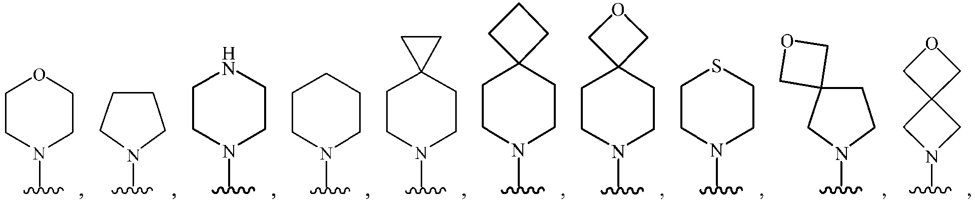 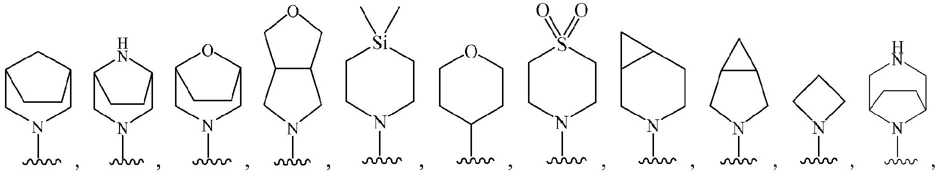 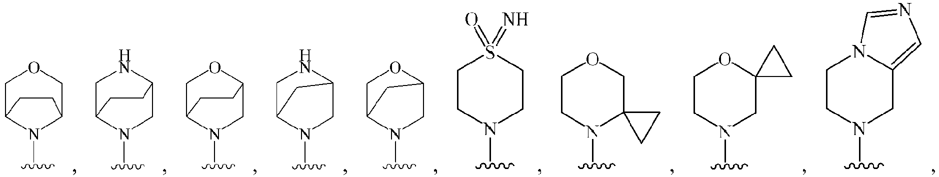 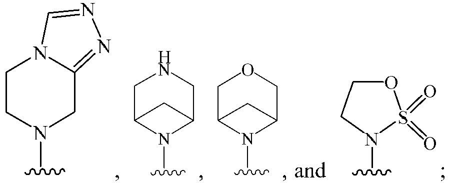 wherein R^{X4-1} may optionally be substituted with 0, 1, or 2 substituents selected from the group consisting of: halogen, hydroxyl, cyano, C₁-C₆ alkyl and C₁-C₆ haloalkyl.

43. The compound according to claim 24 or 25, or a pharmaceutically acceptable salt, ester, prodrug, stereoisomer, or isotopic derivative thereof, wherein X⁴ represents C-L-R^{X4-1}; wherein L represents absence, and R^{X4-1} represents the following groups:

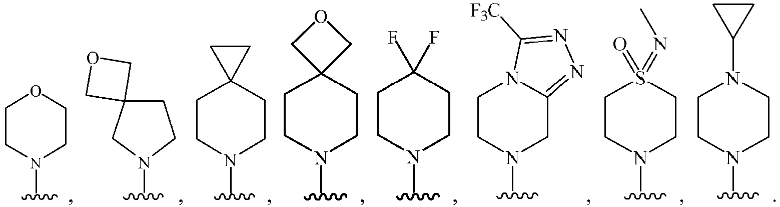

44. A compound, or a pharmaceutically acceptable salt, ester, prodrug, stereoisomer or isotopic derivative thereof, having the following structures:

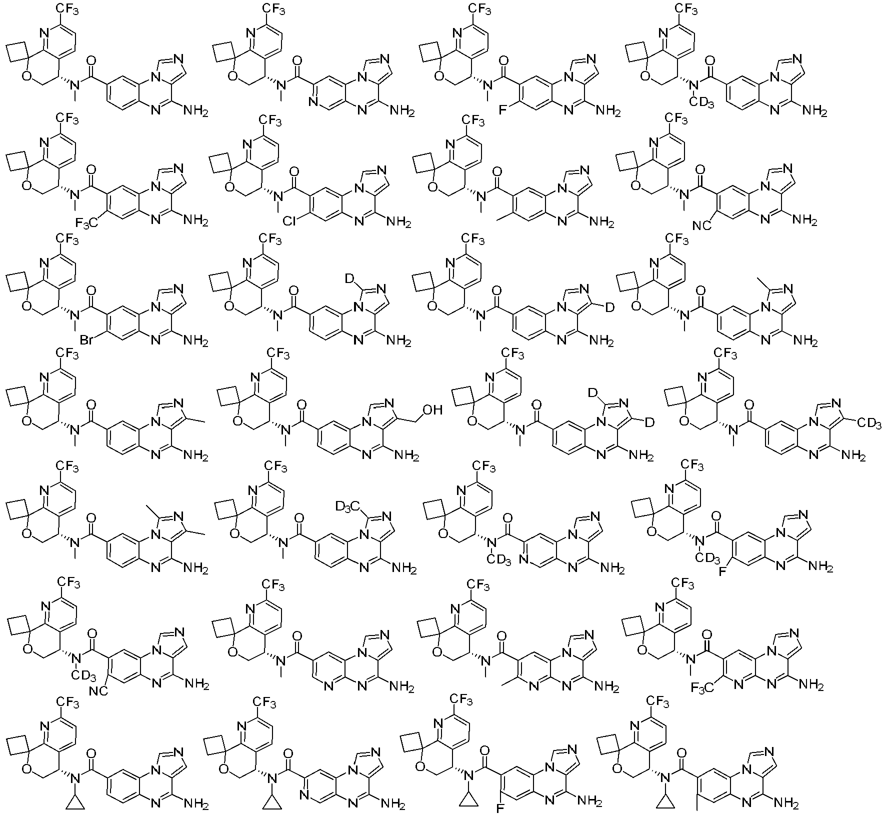

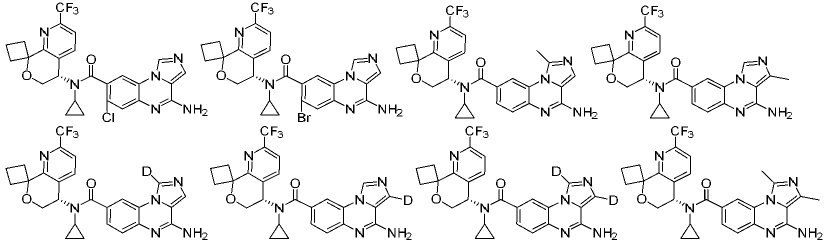

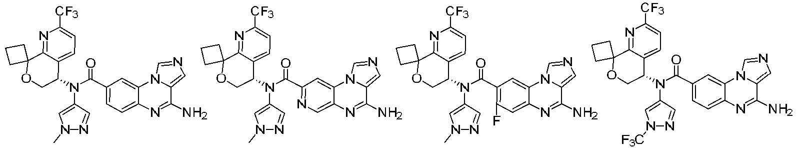

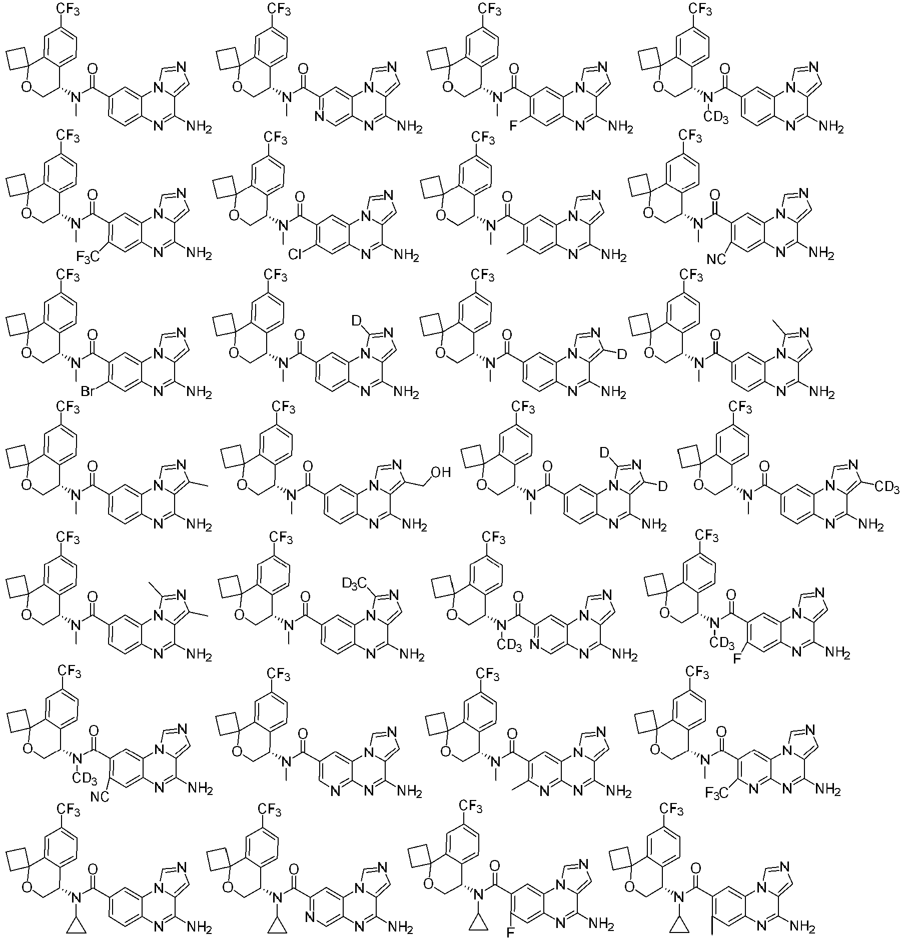

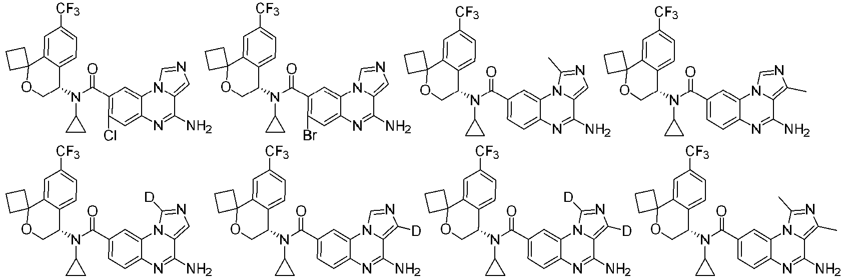

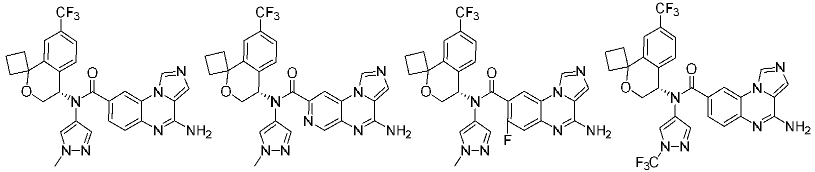

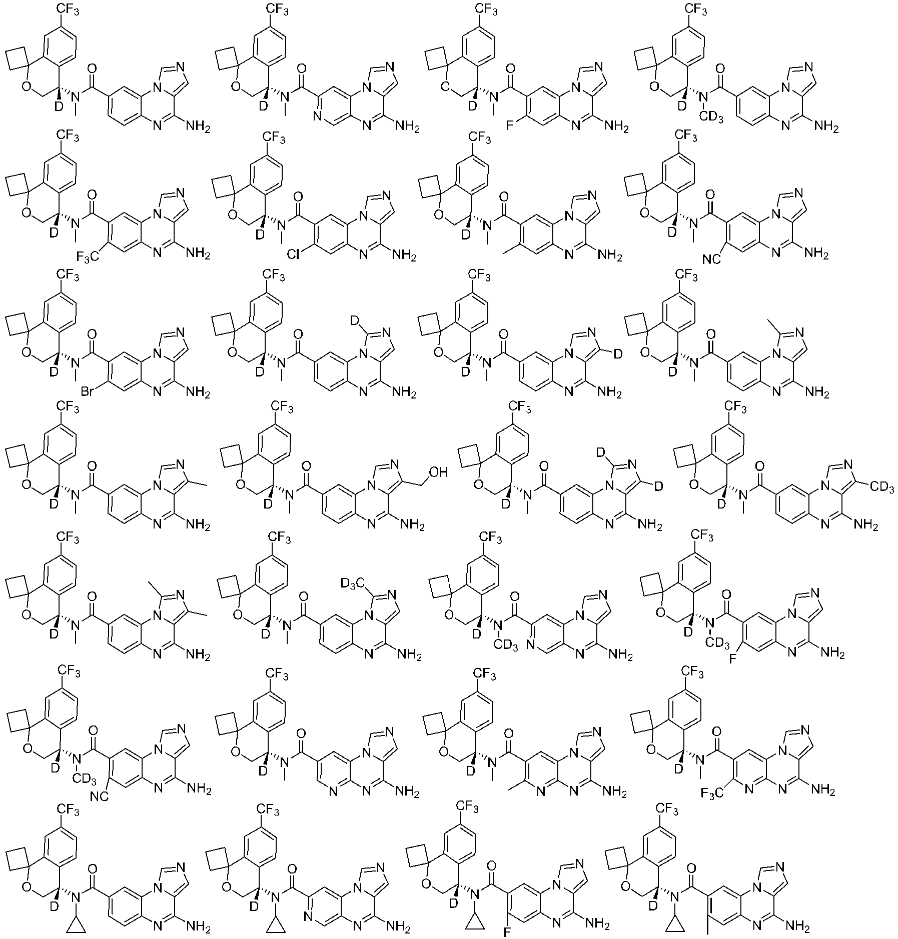

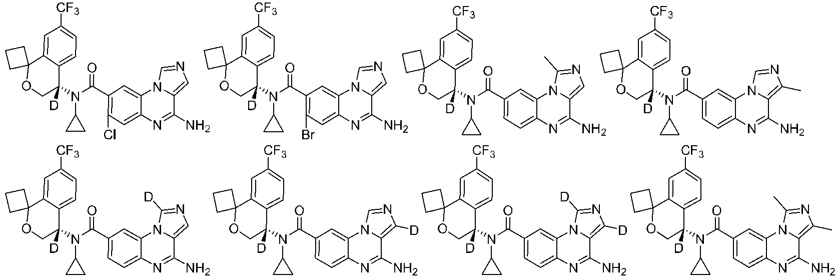

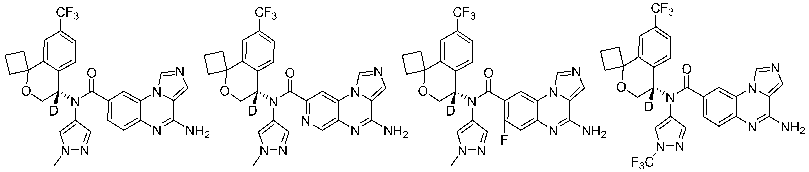

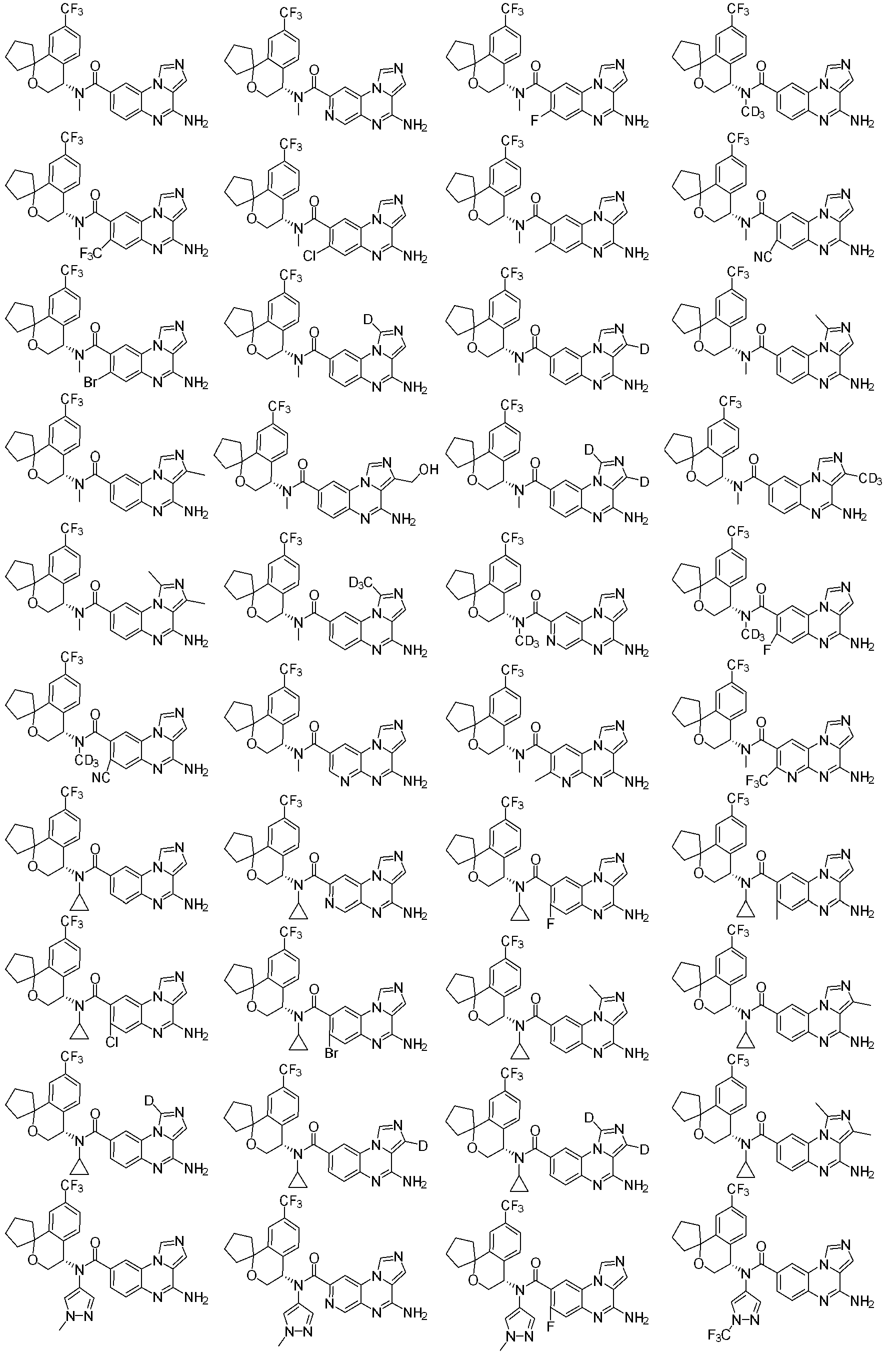

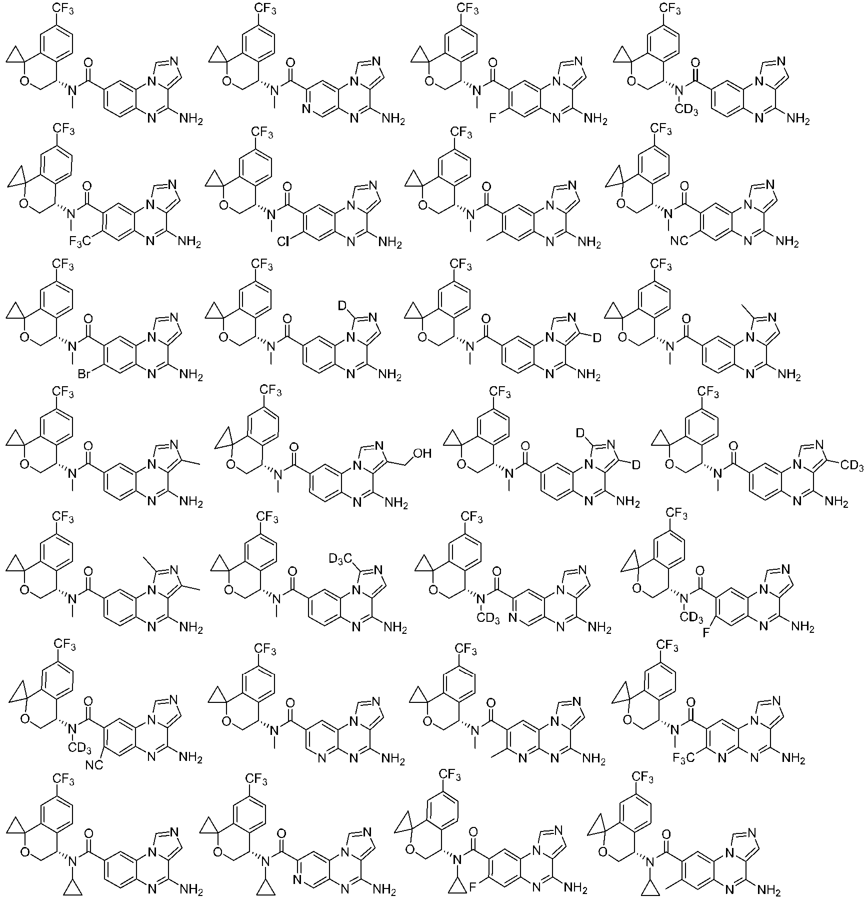

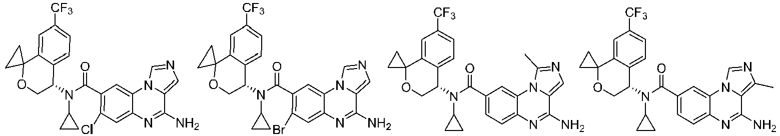

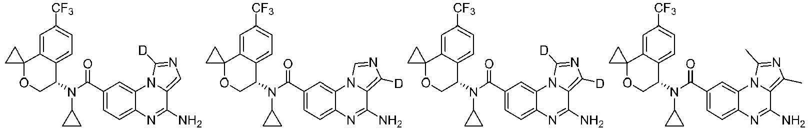

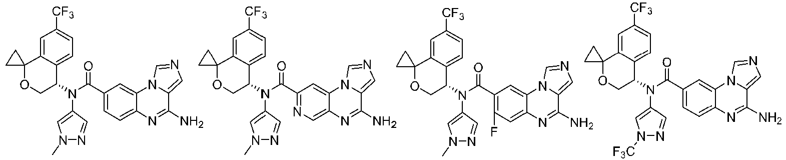

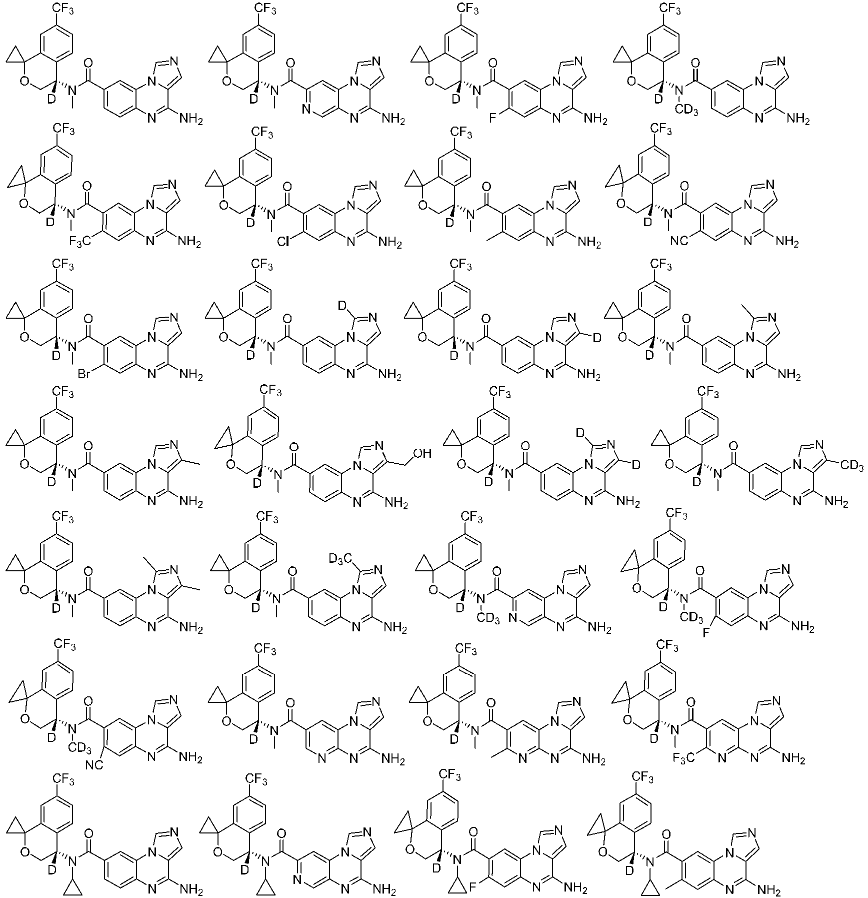

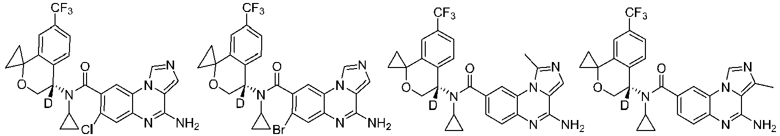

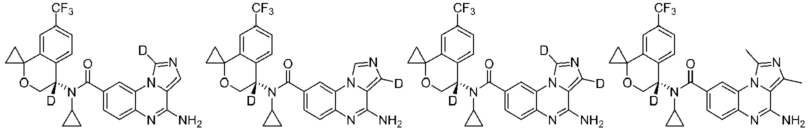

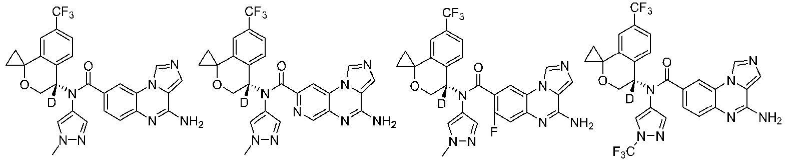

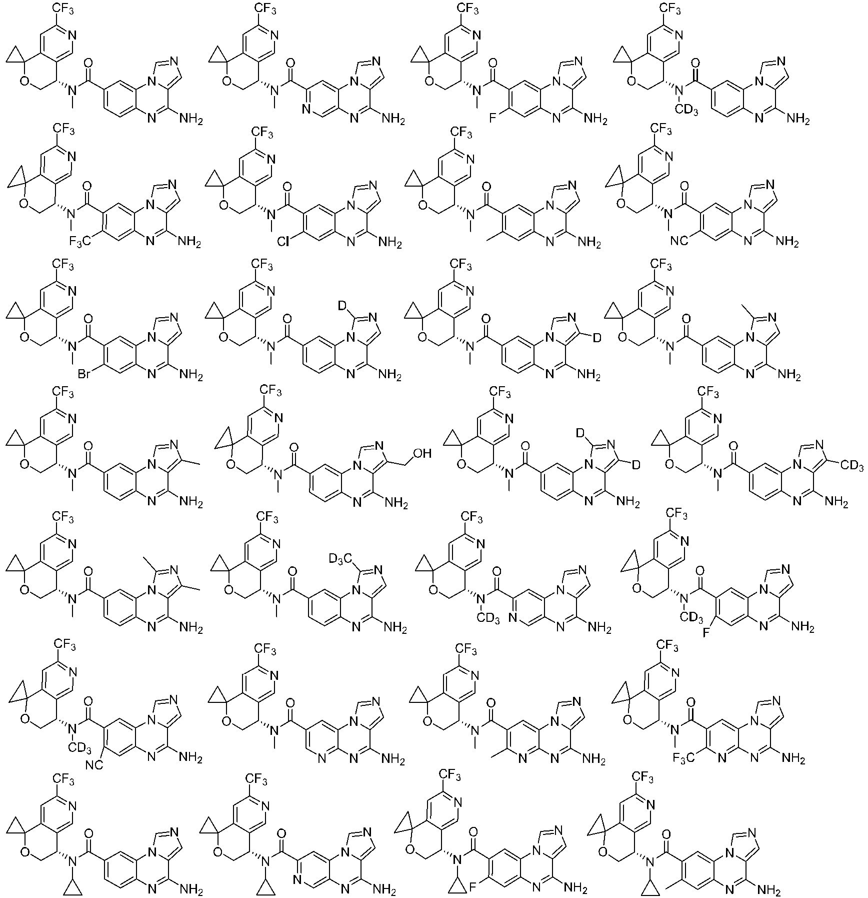

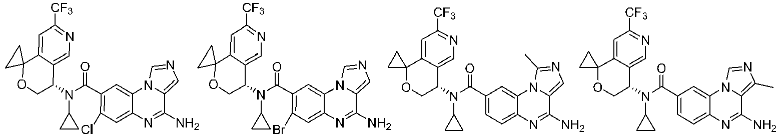

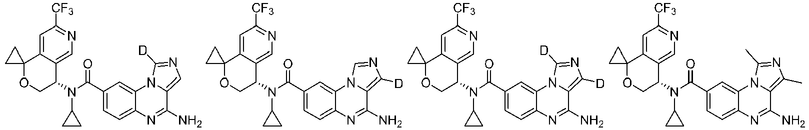

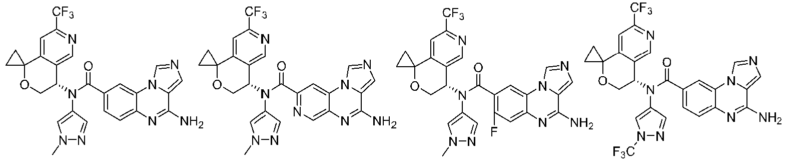

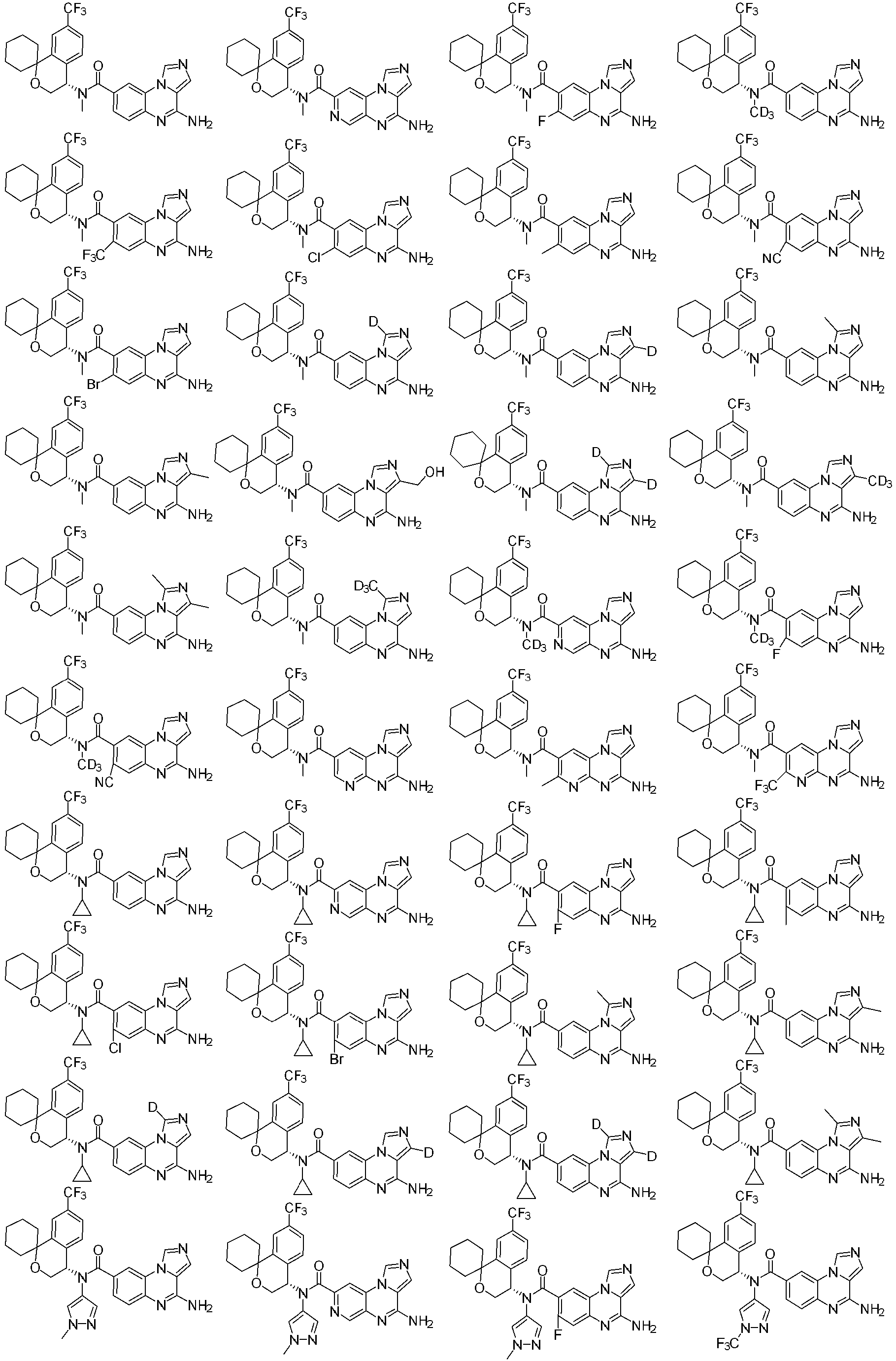

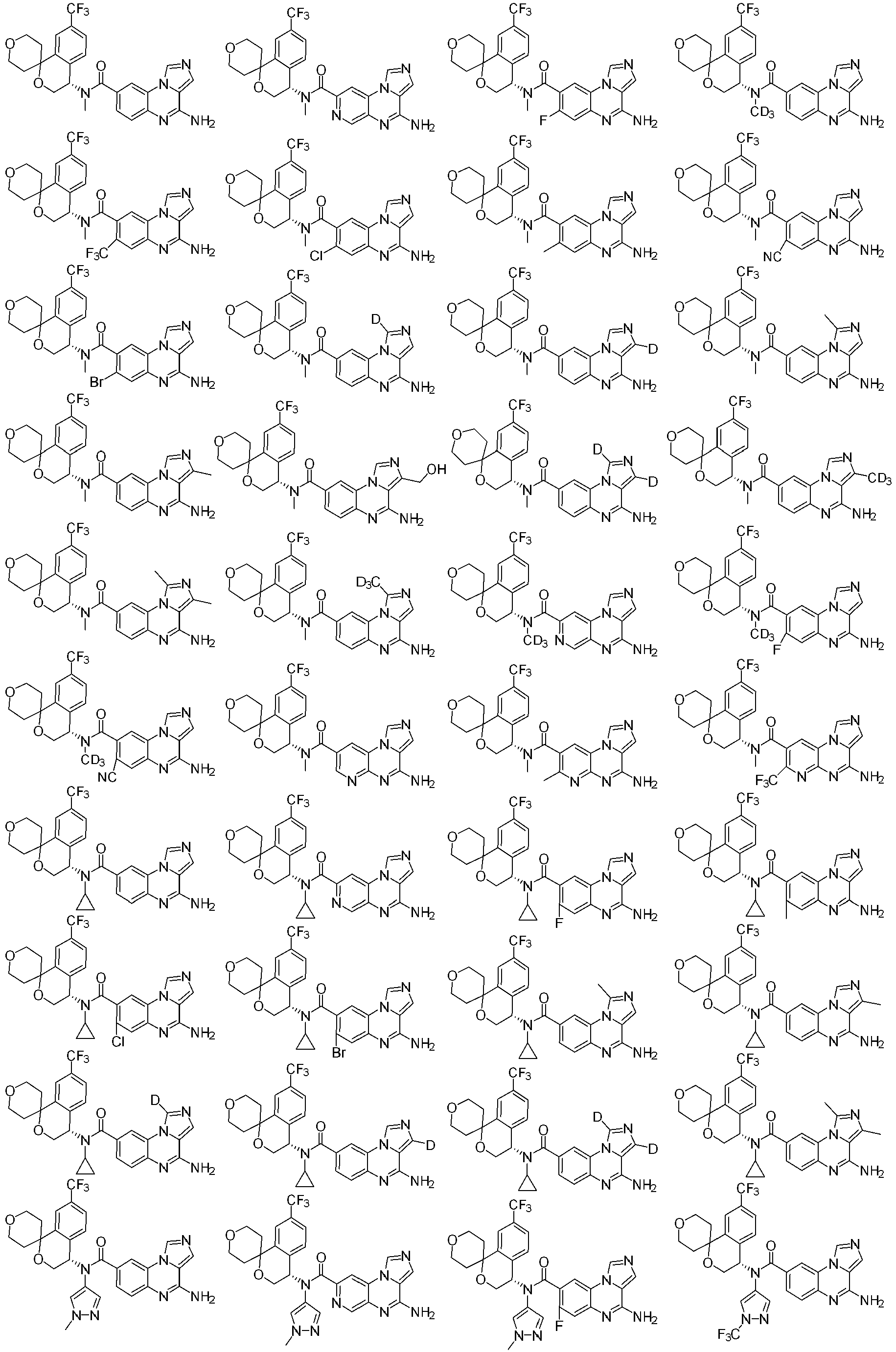

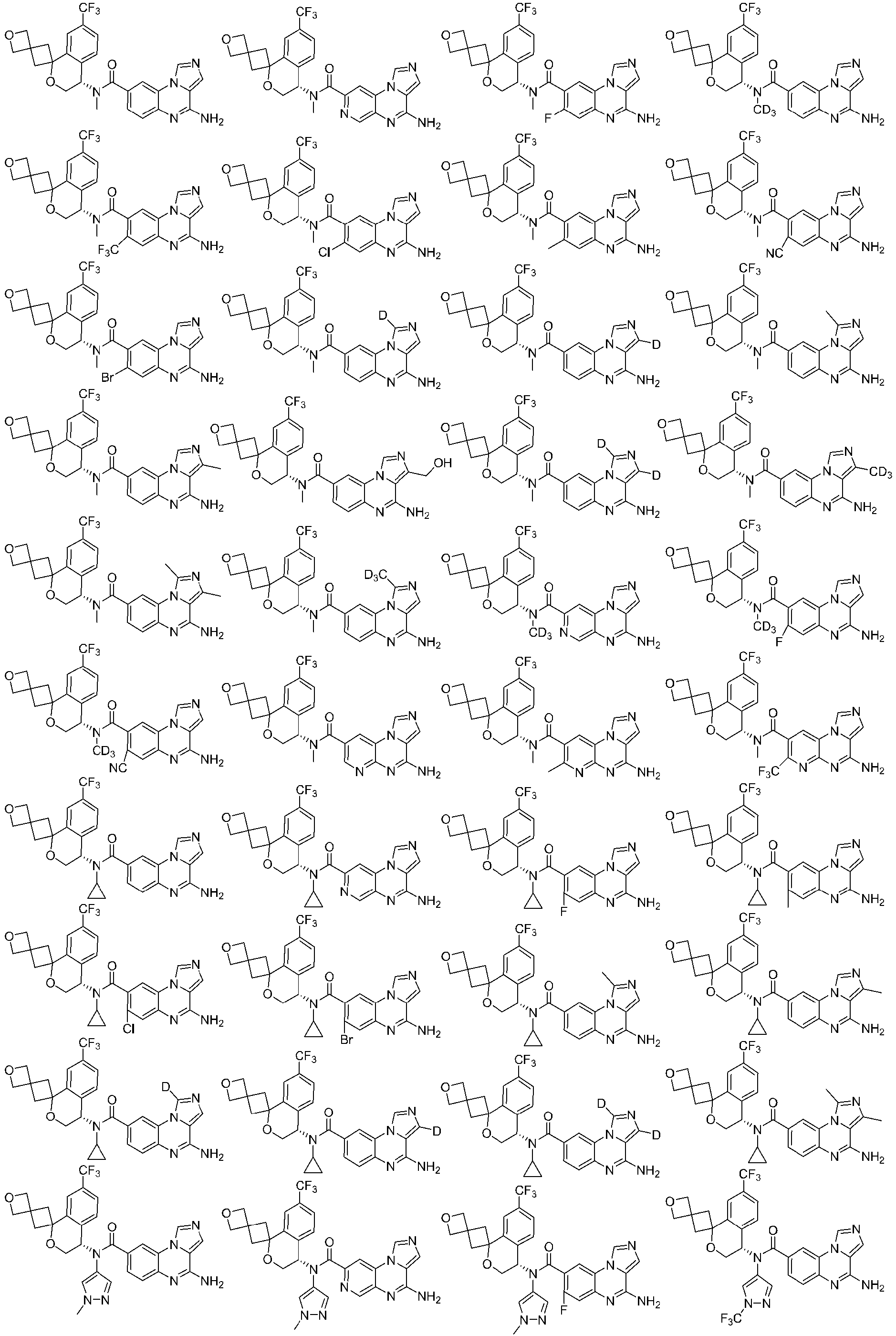

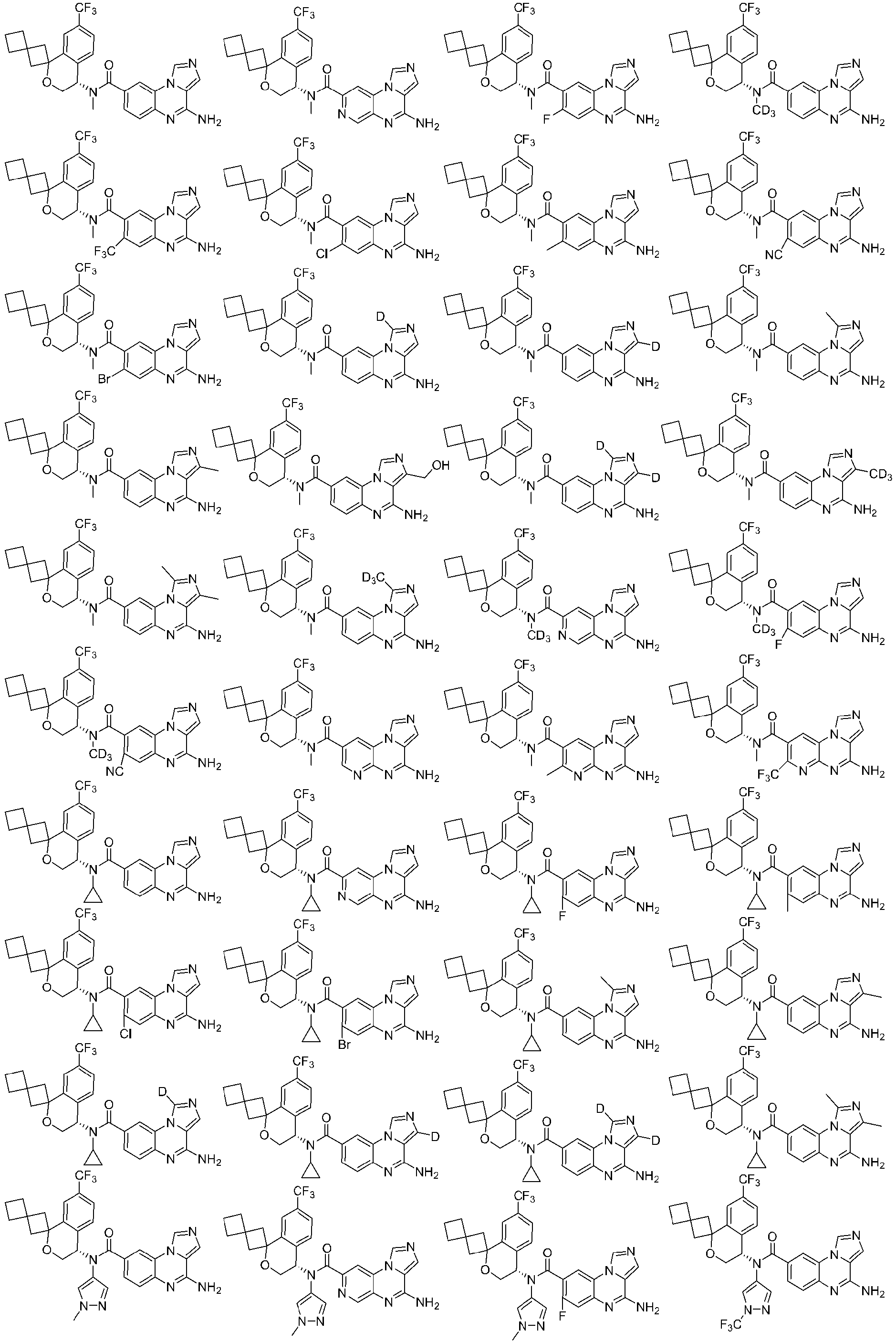

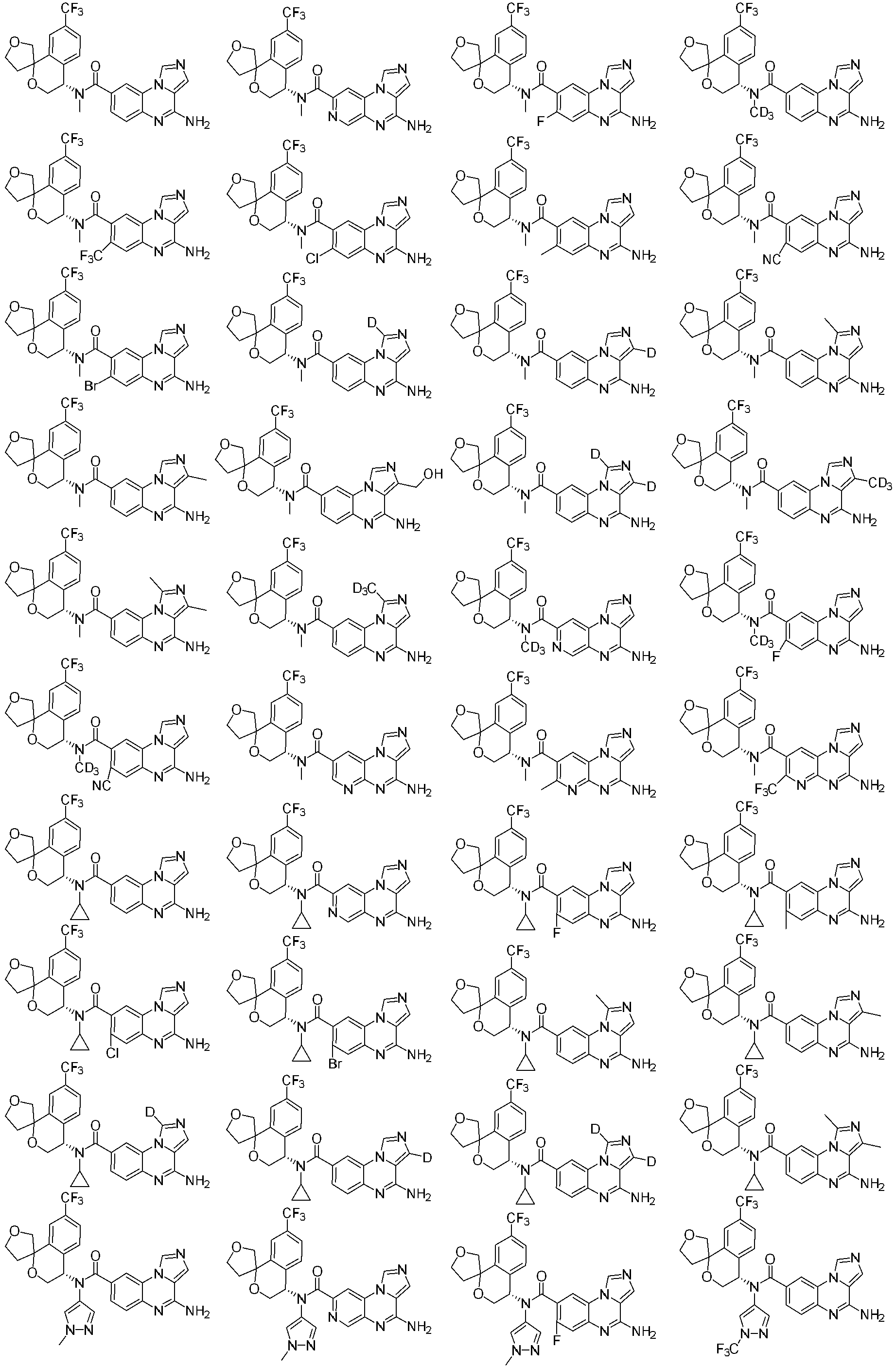

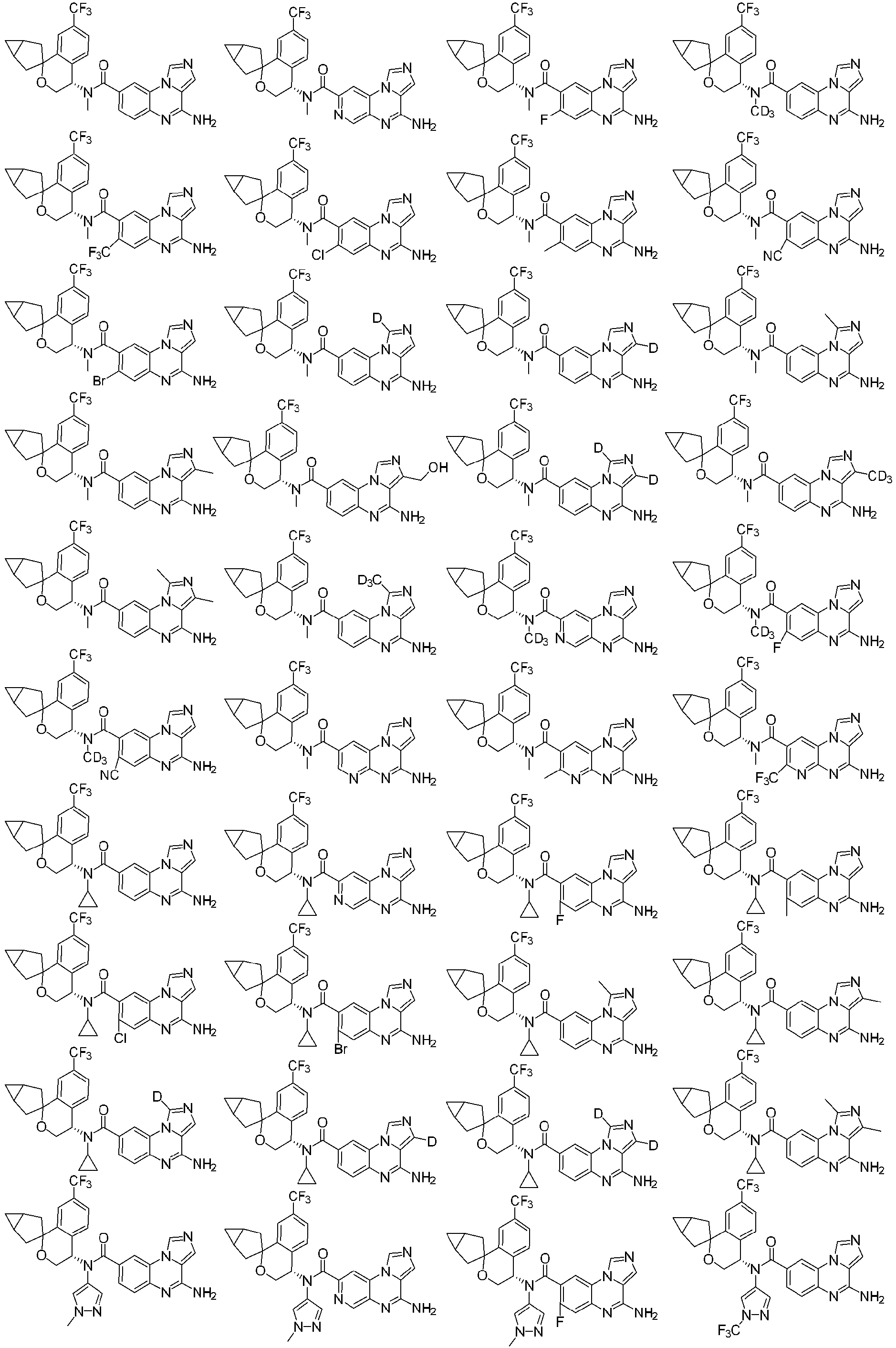

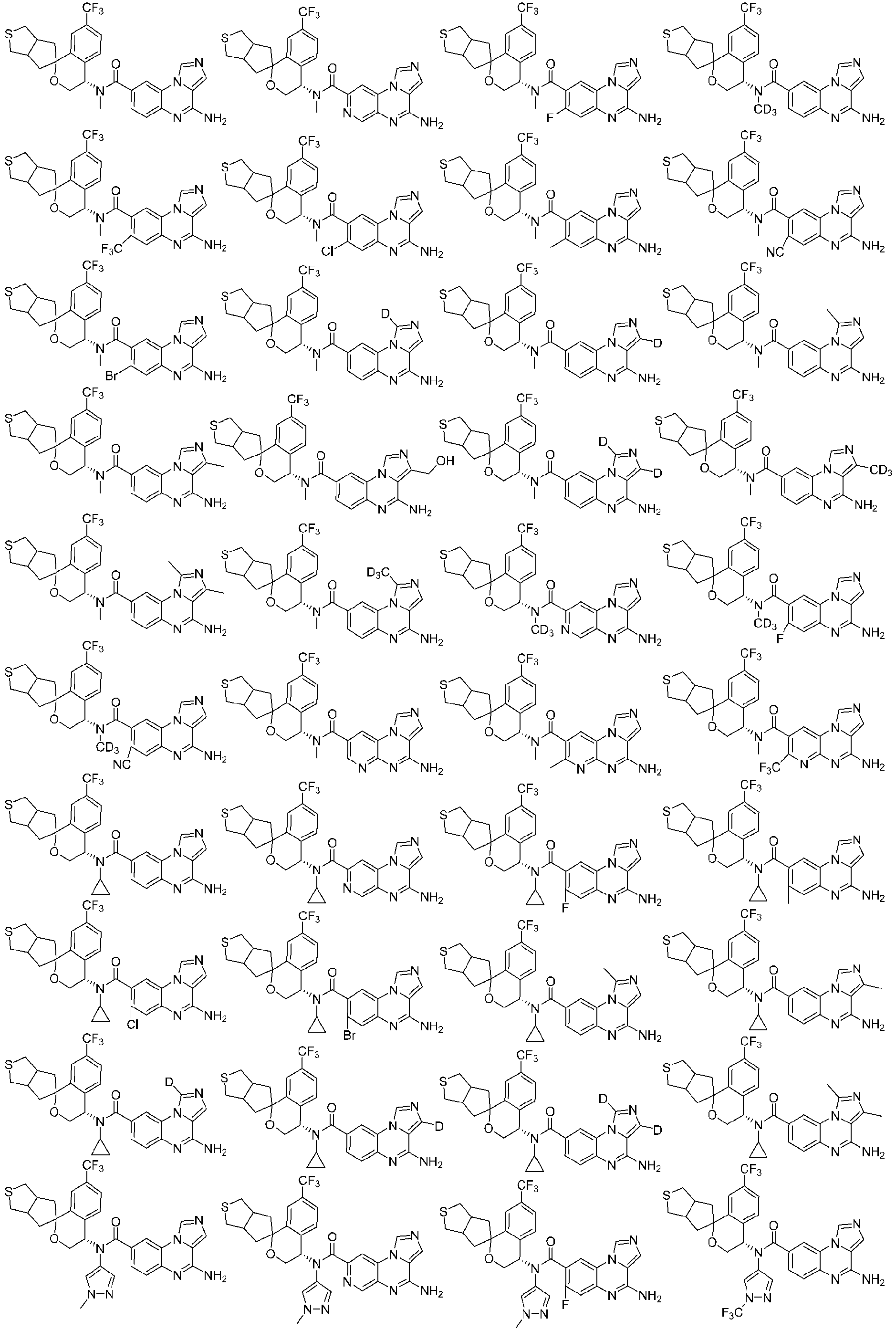

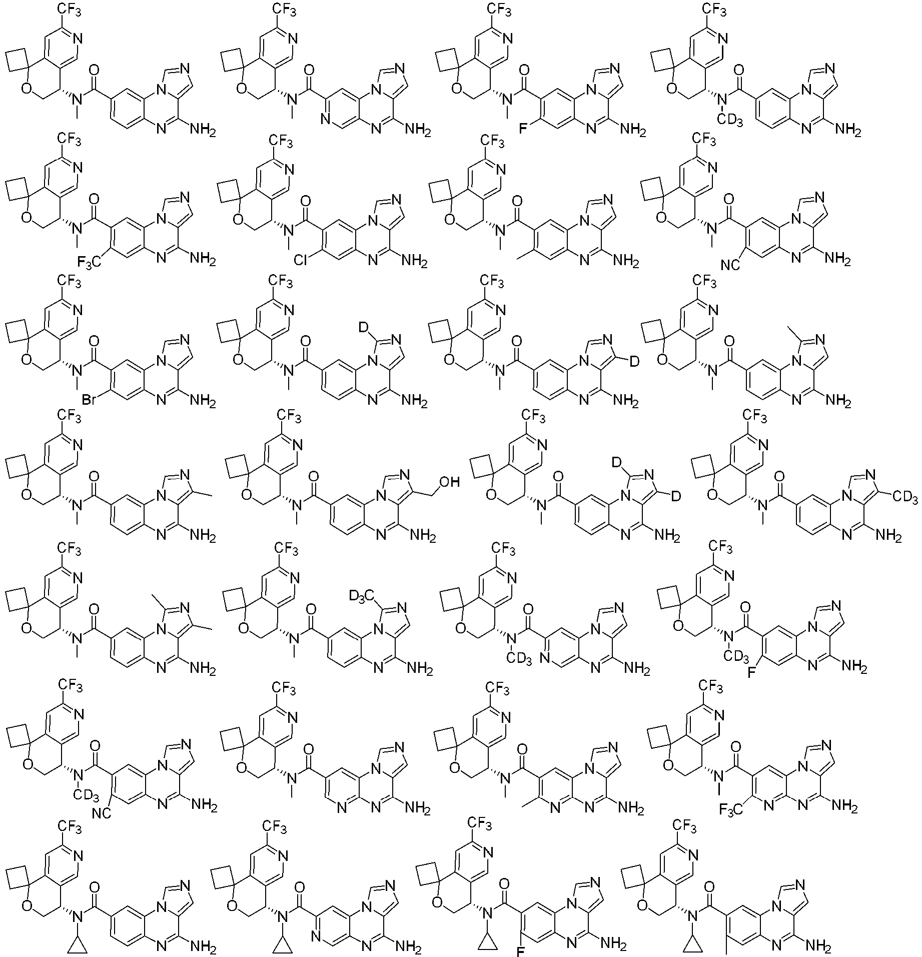

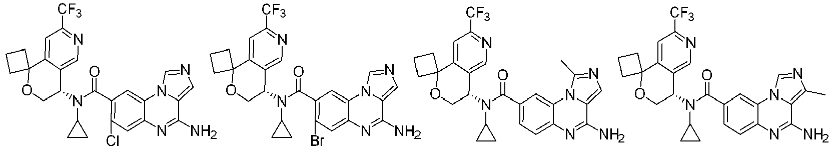

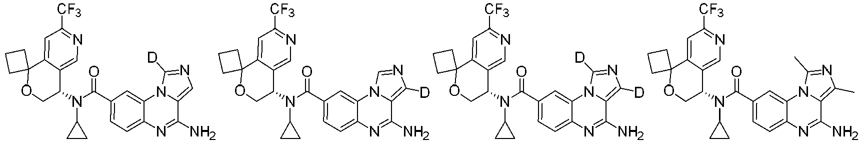

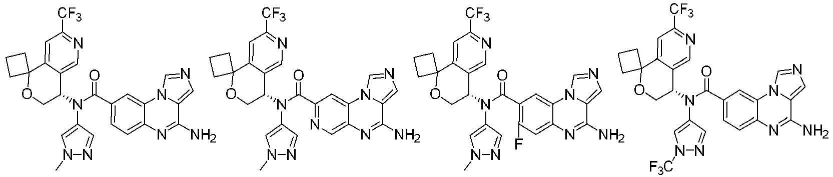

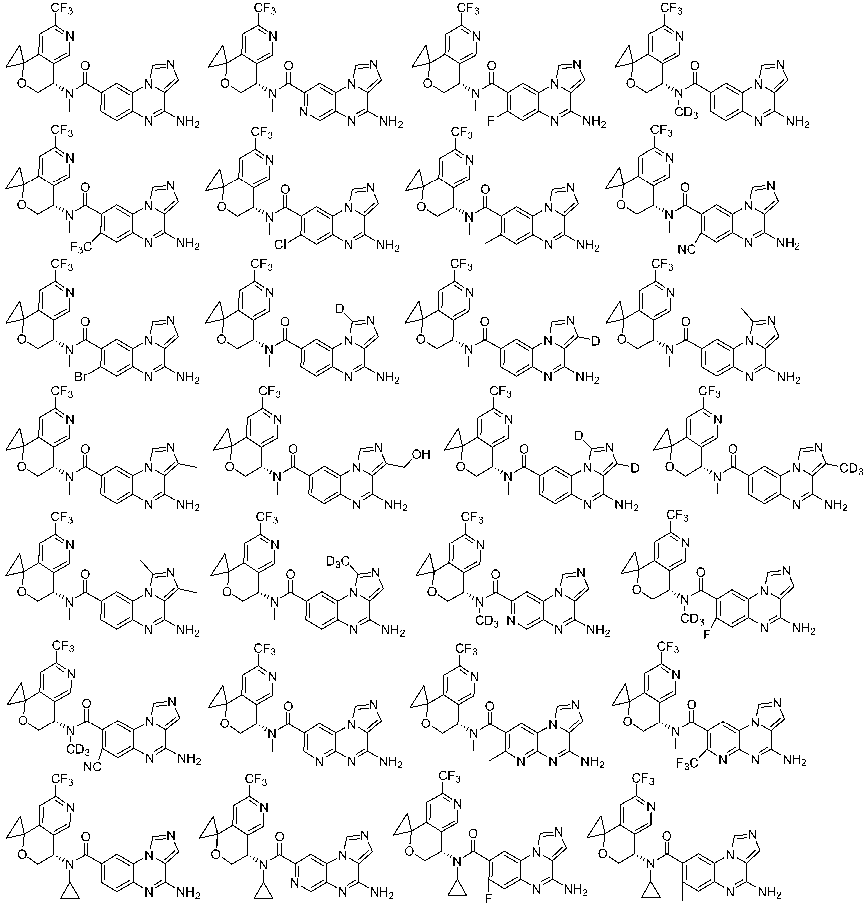

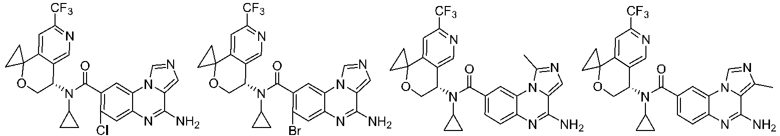

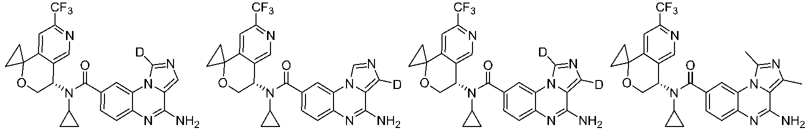

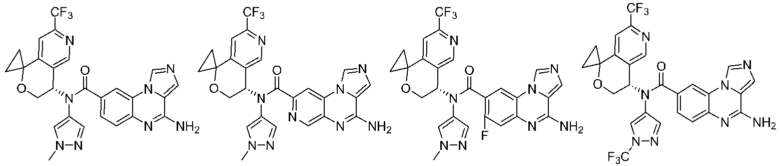

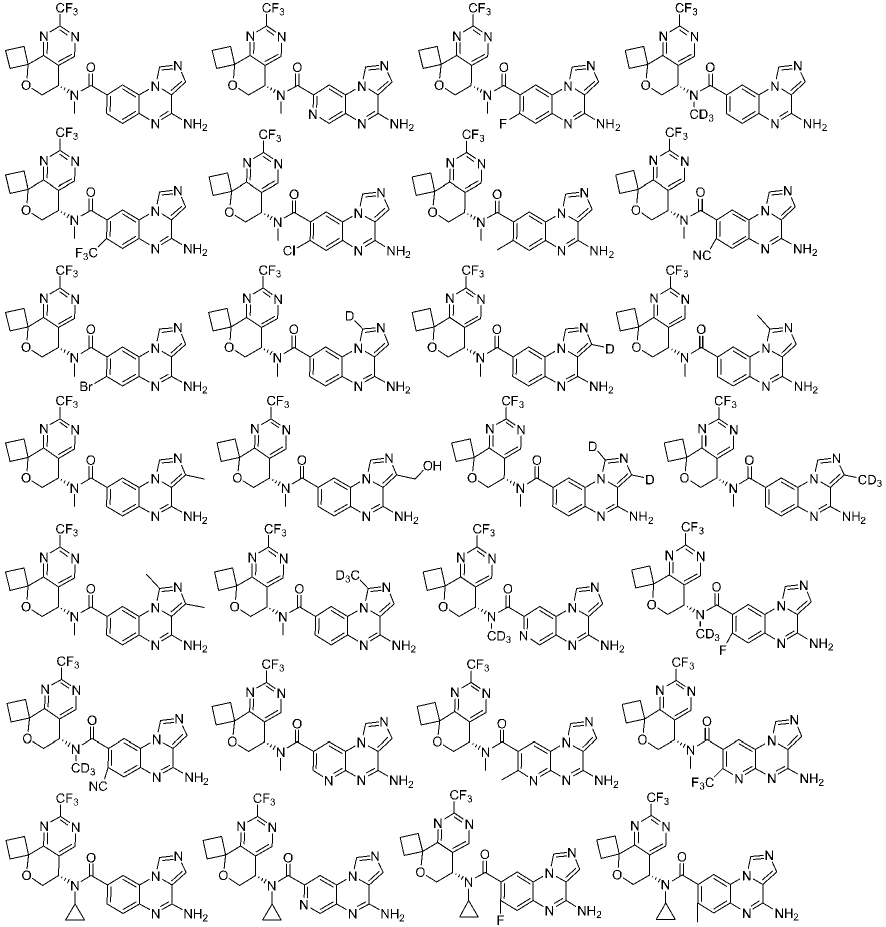

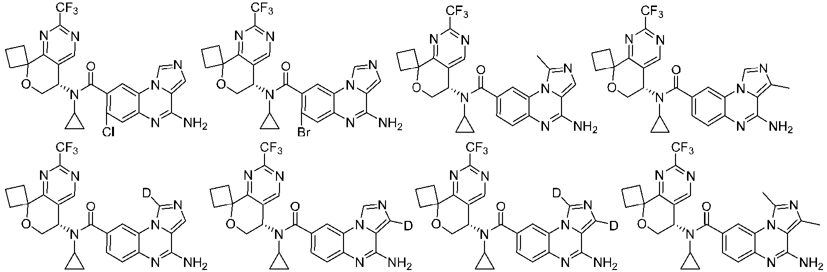

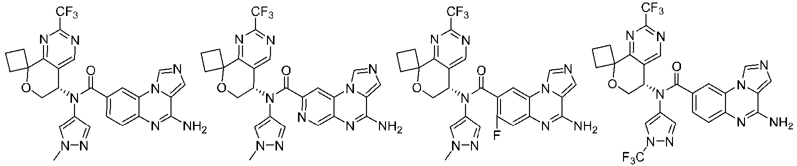

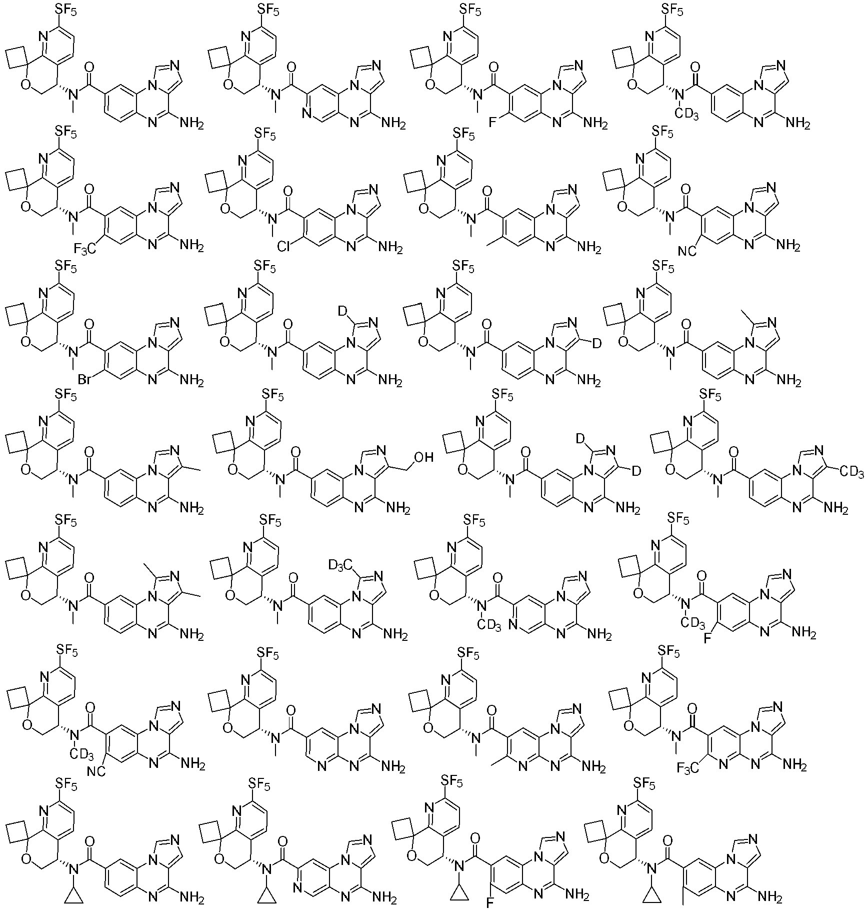

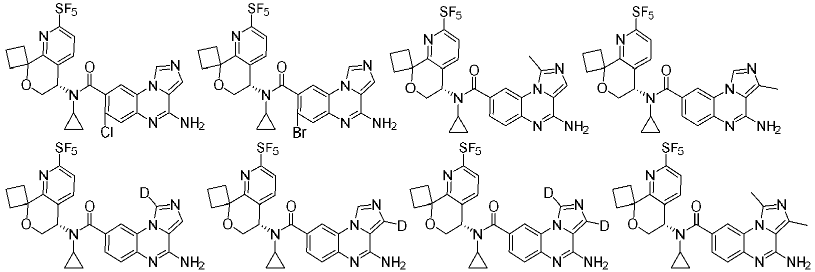

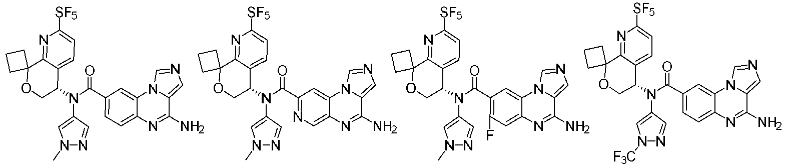

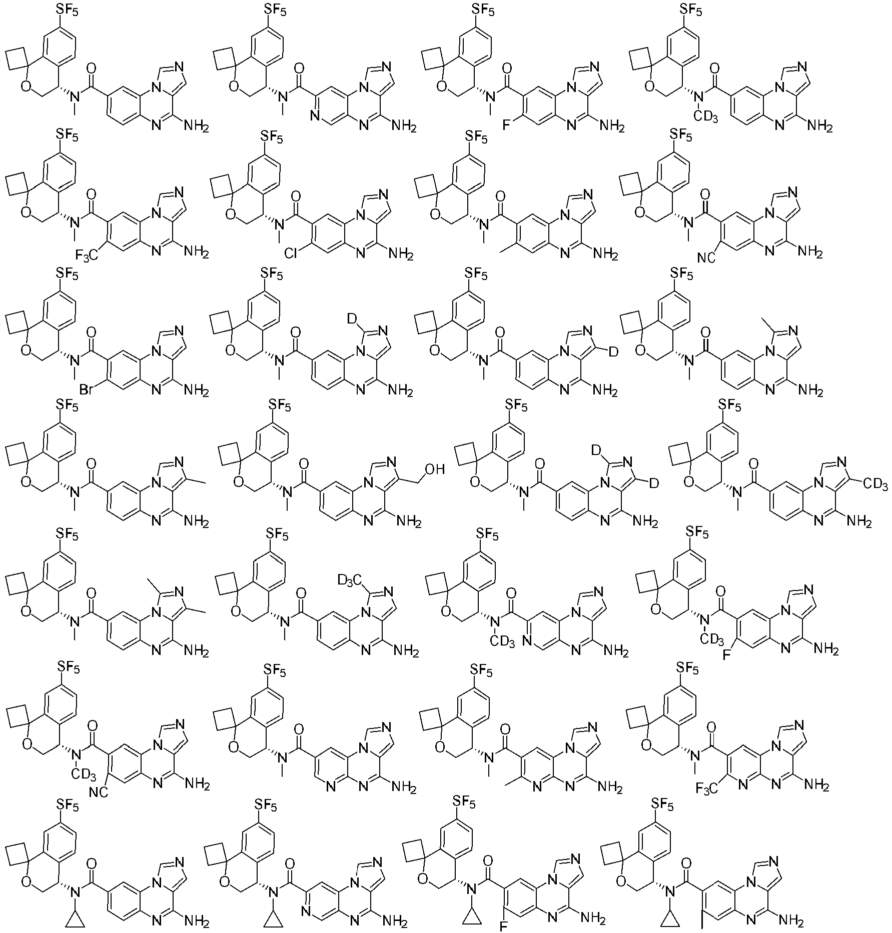

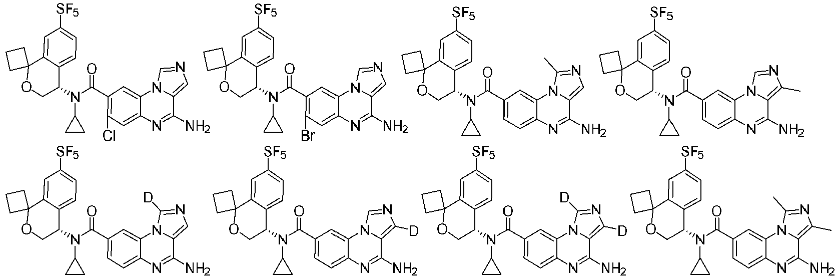

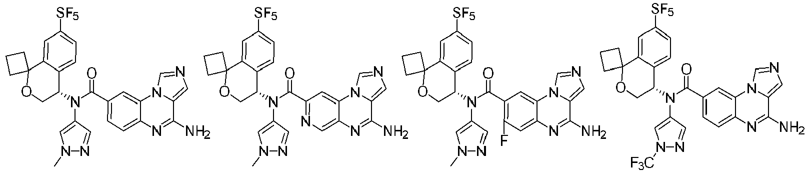

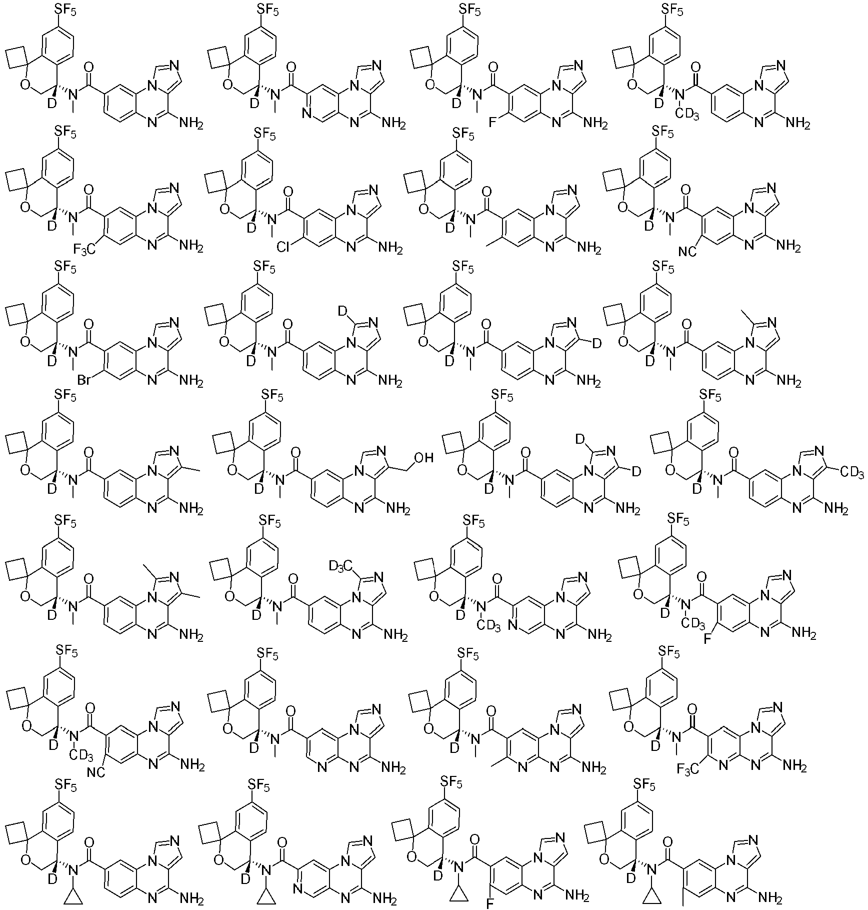

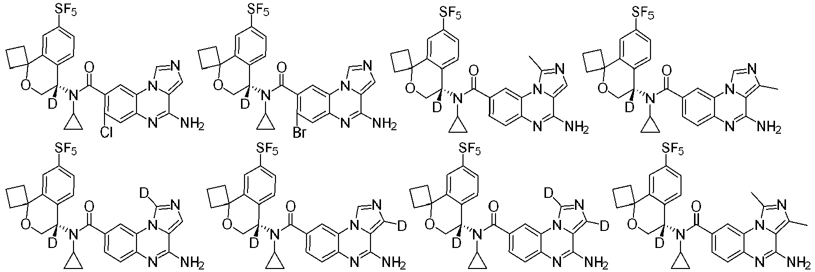

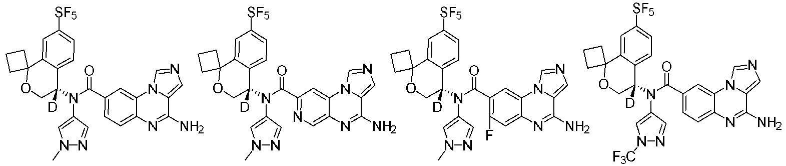

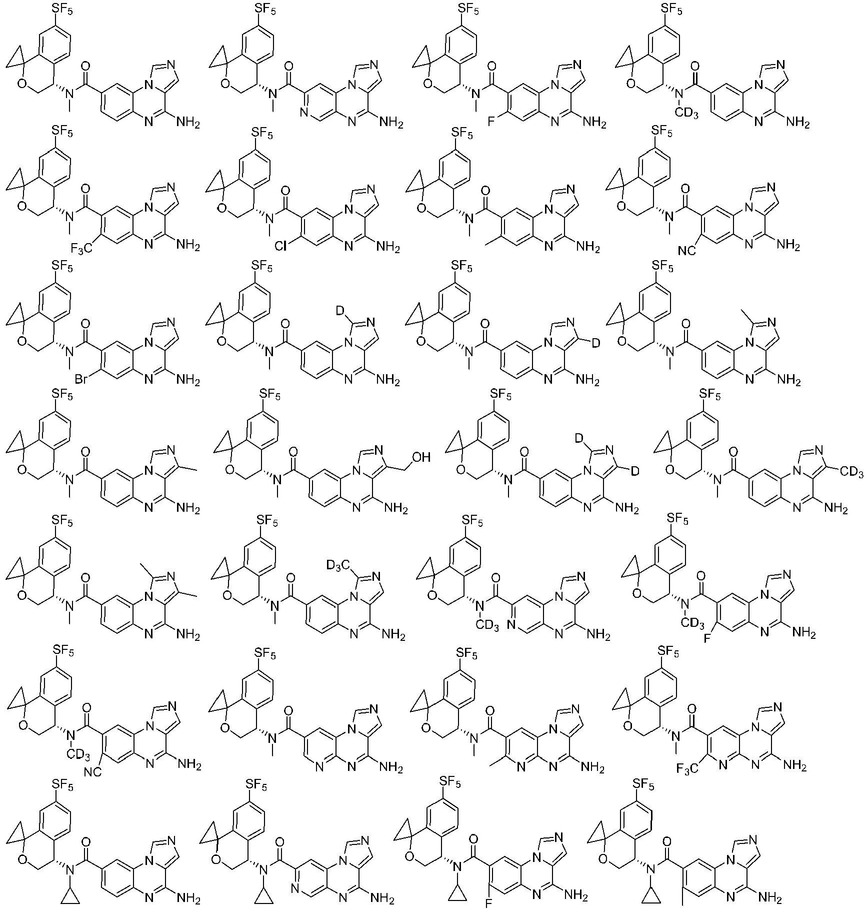

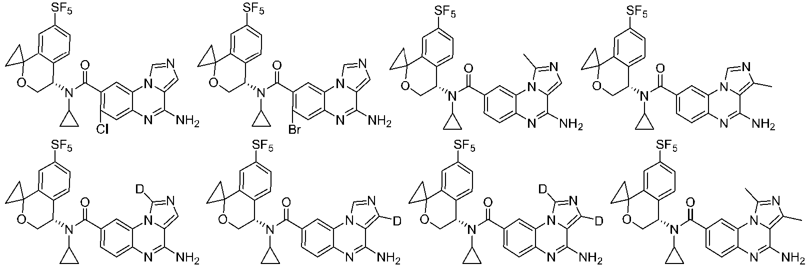

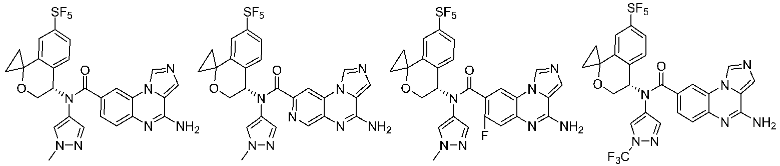

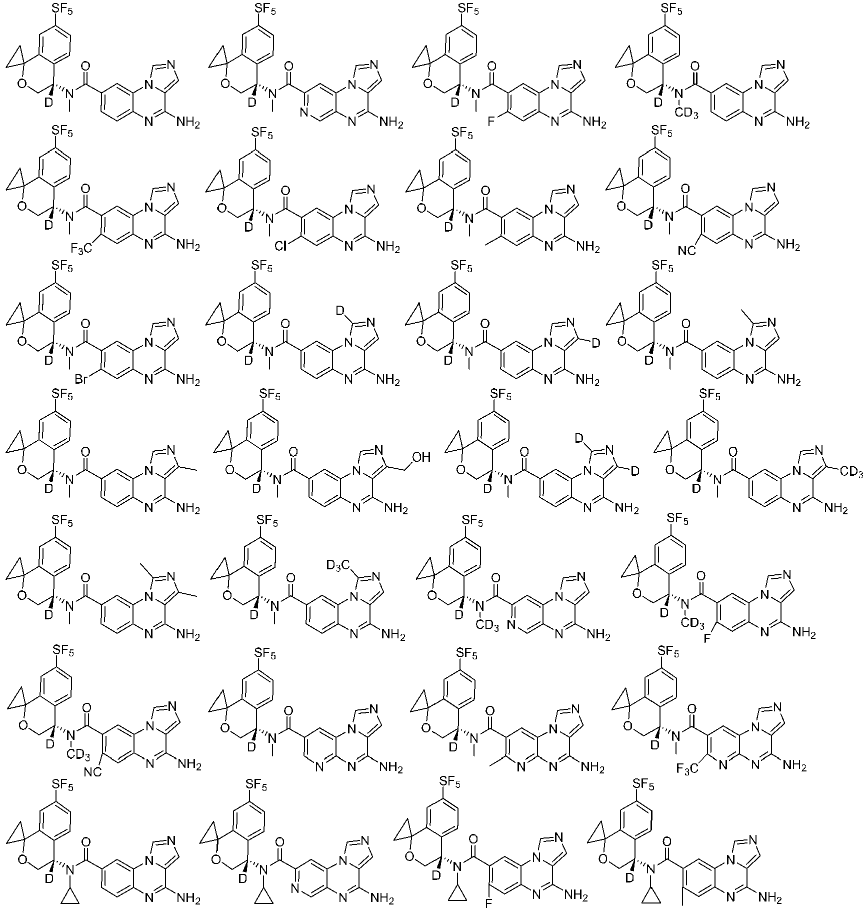

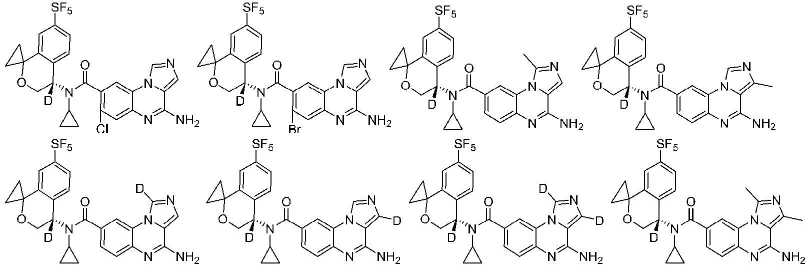

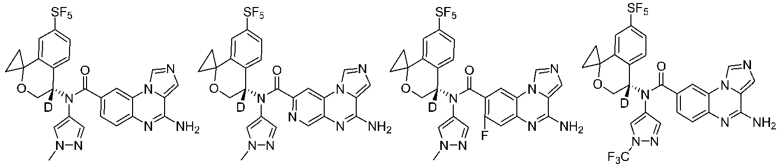

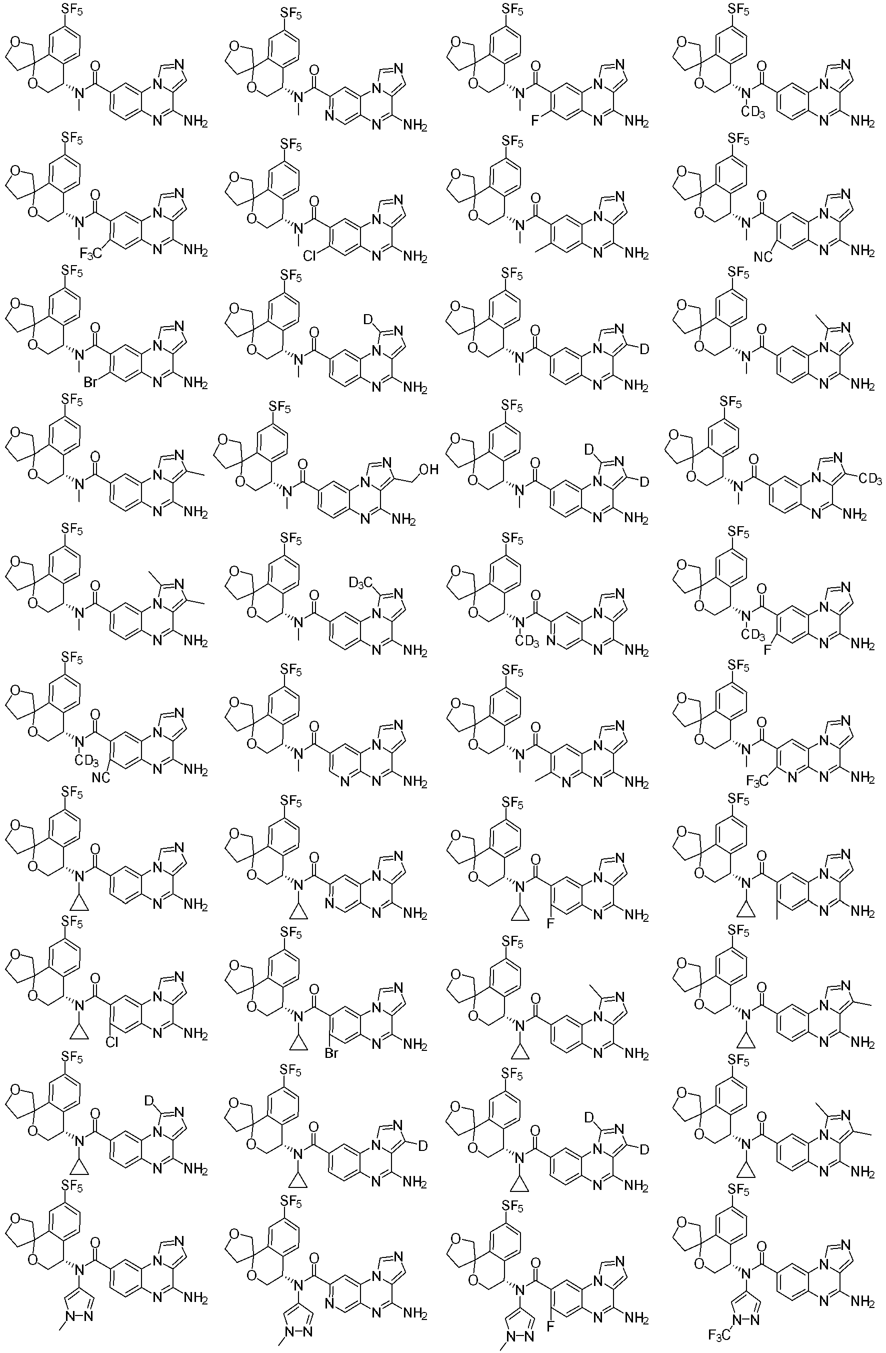

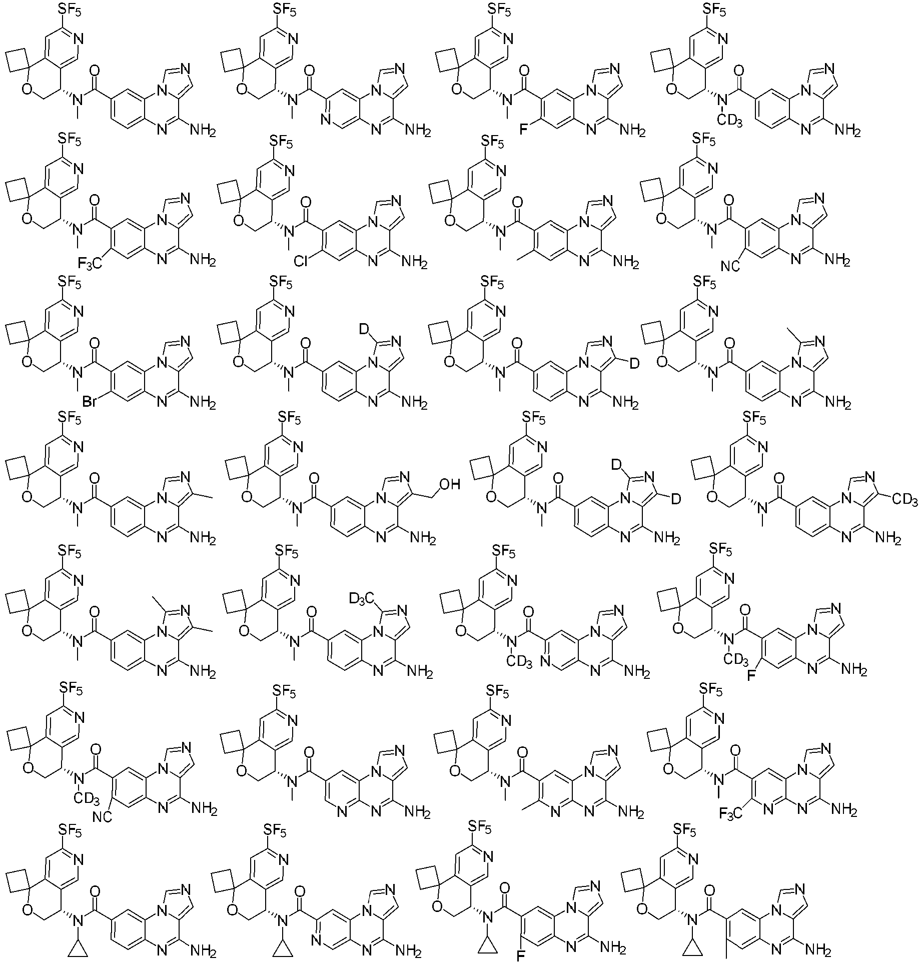

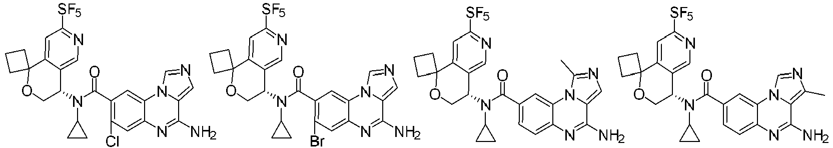

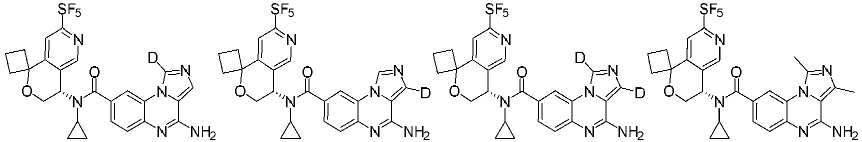

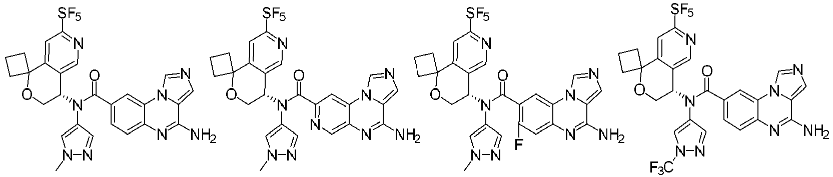

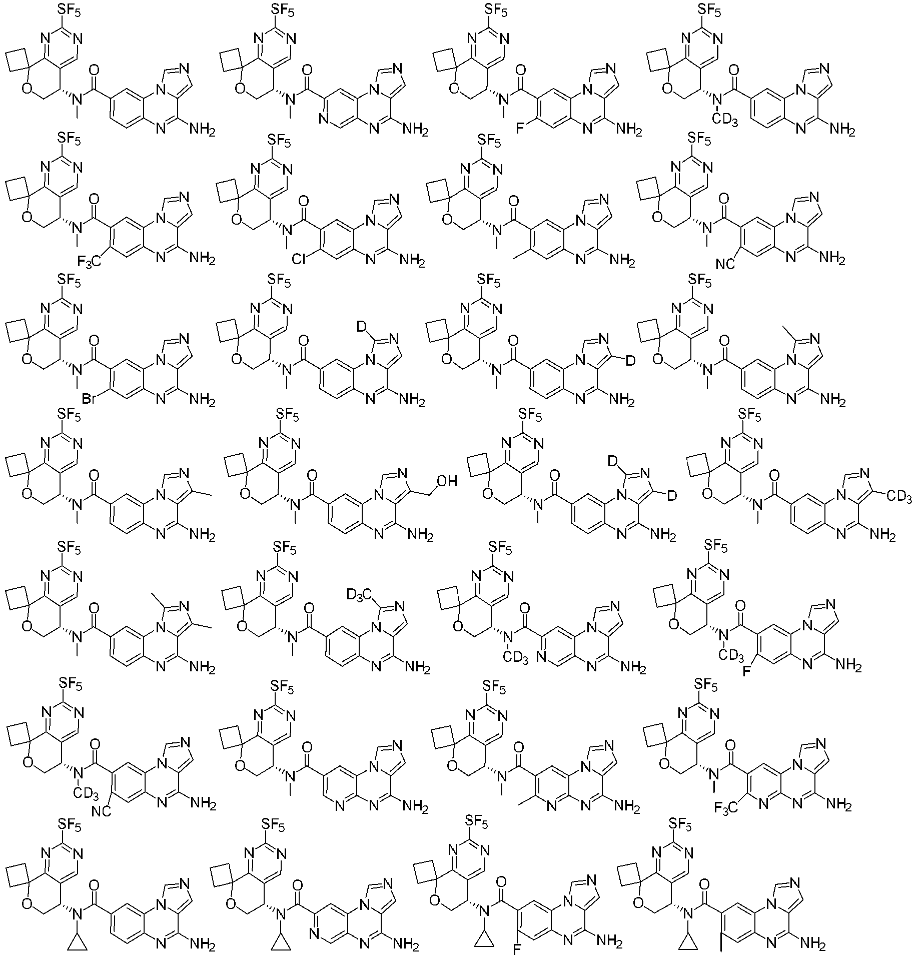

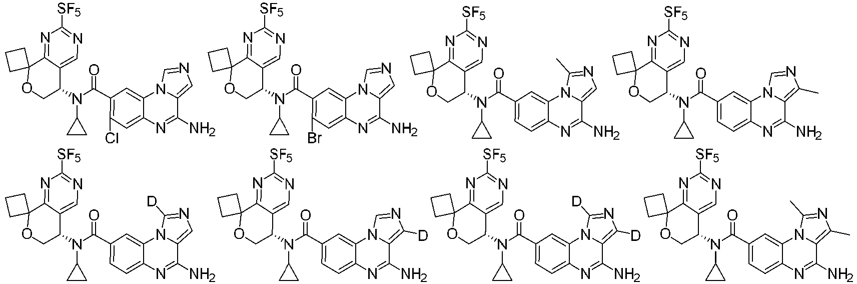

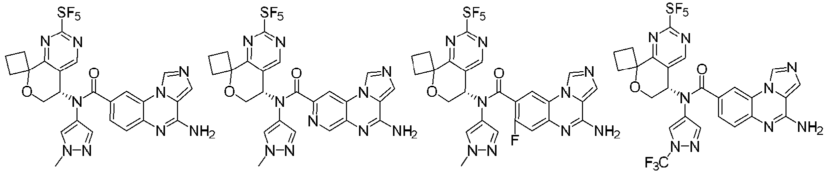

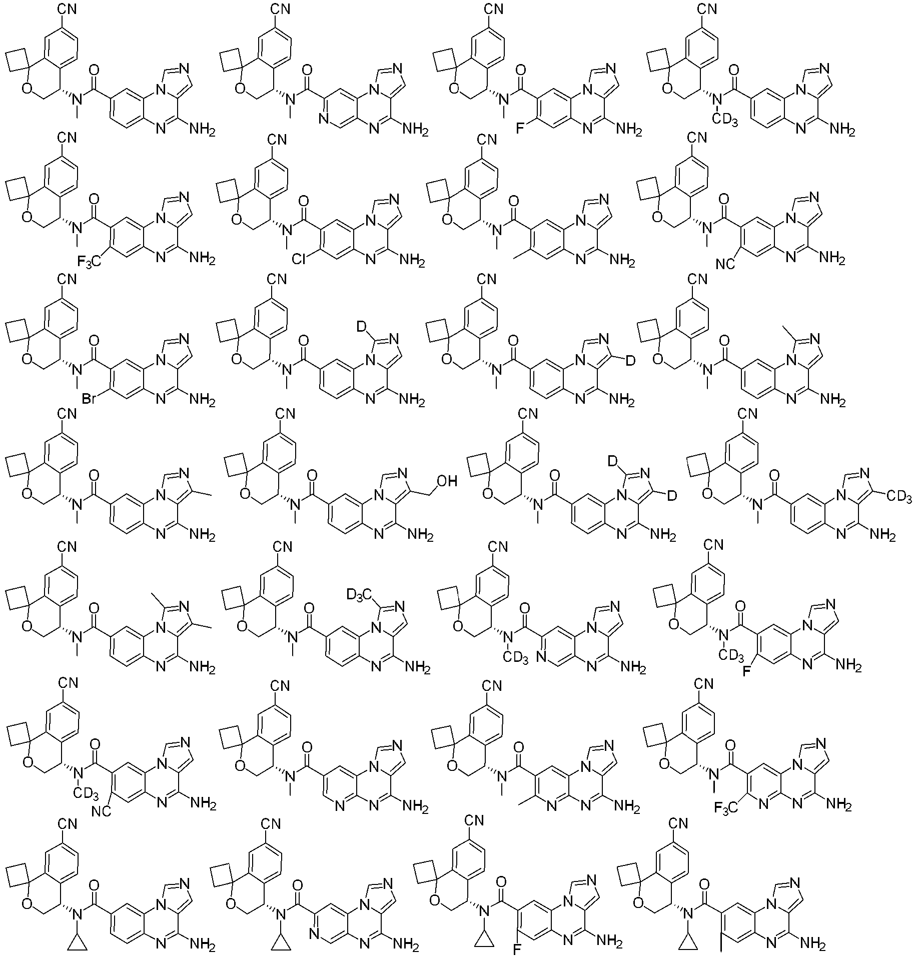

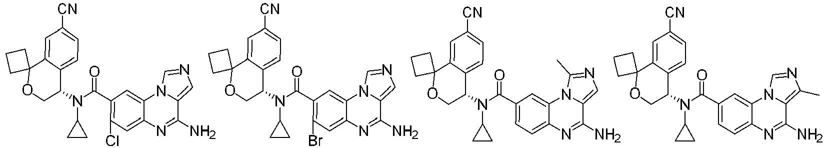

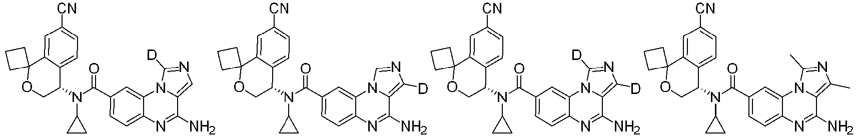

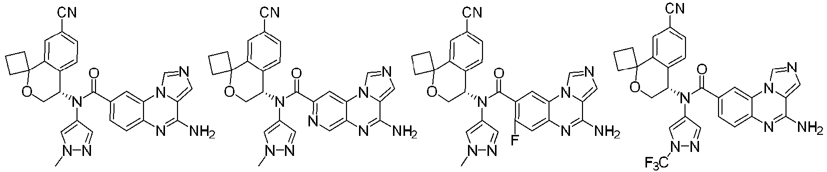

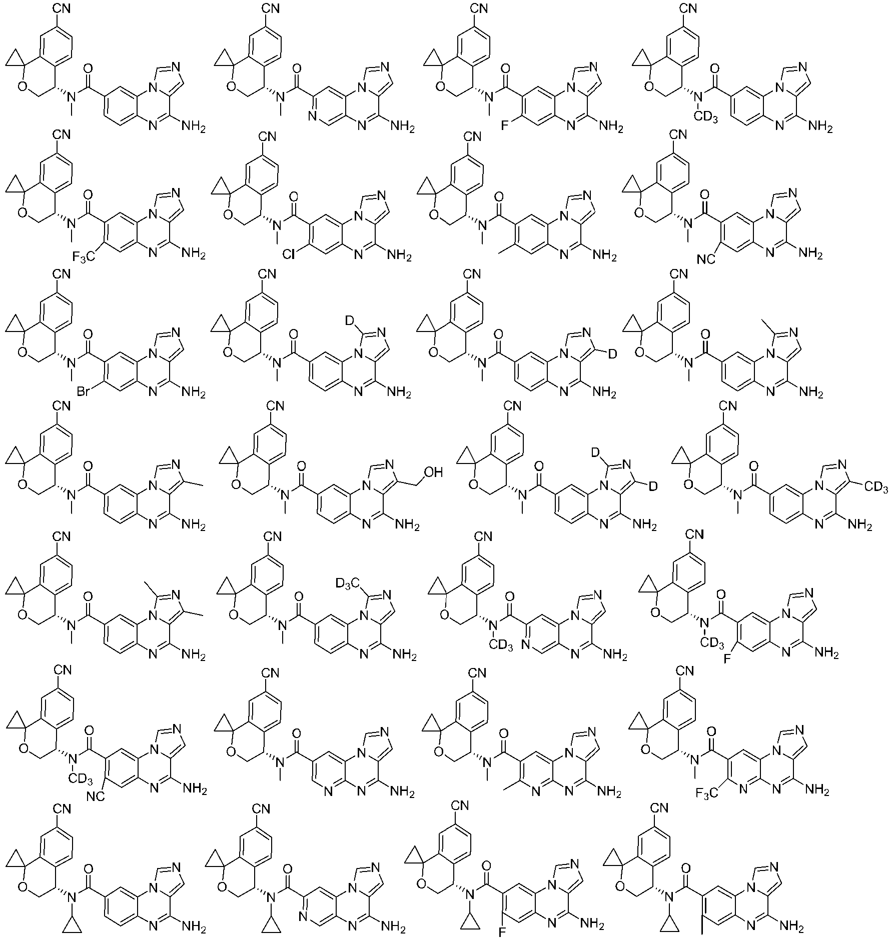

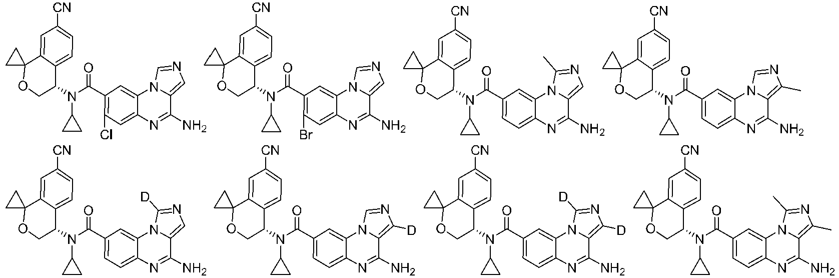

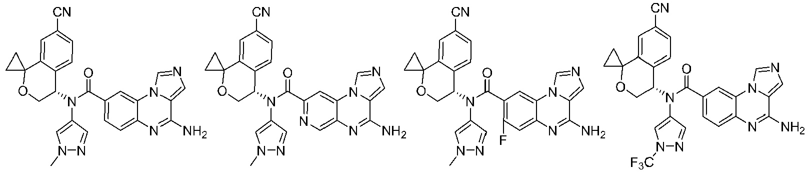

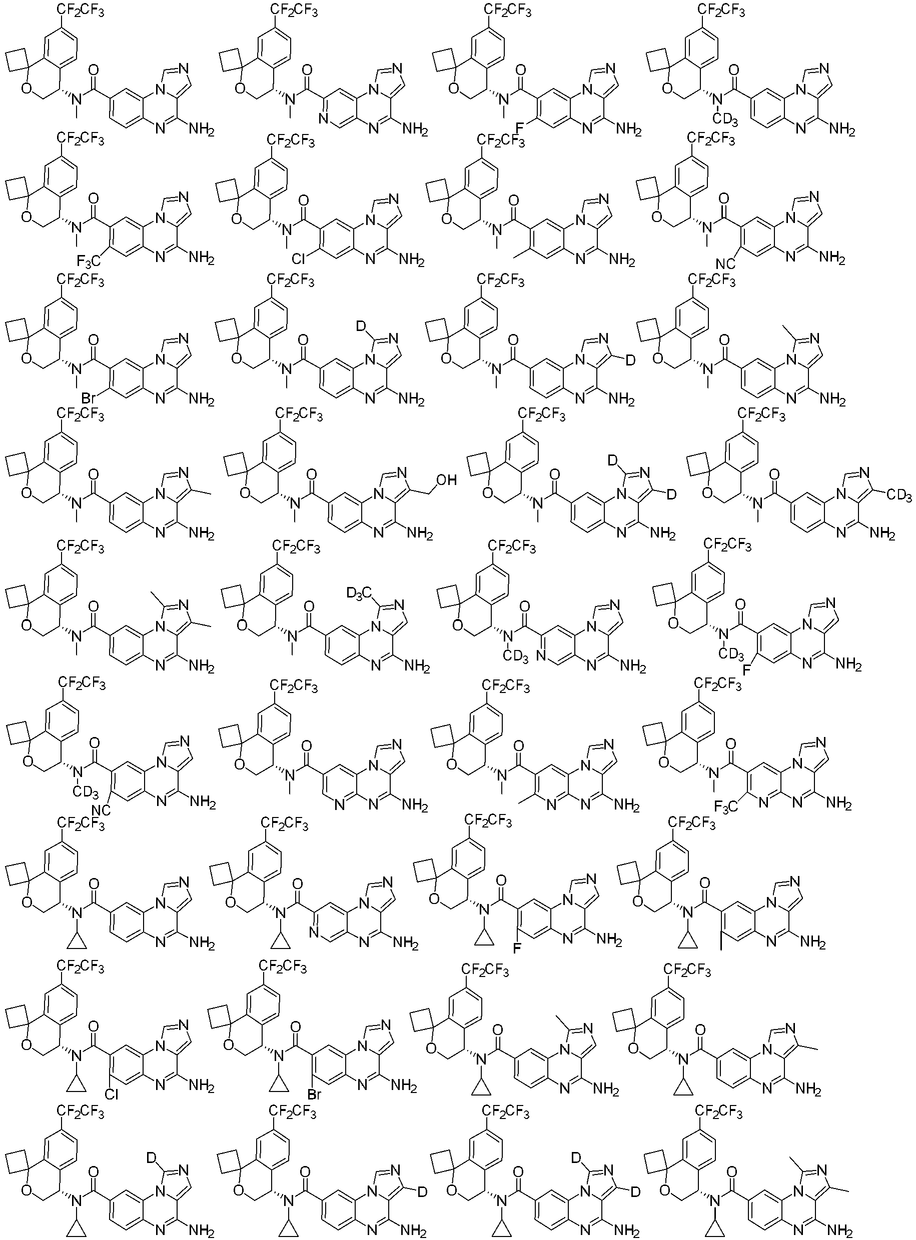

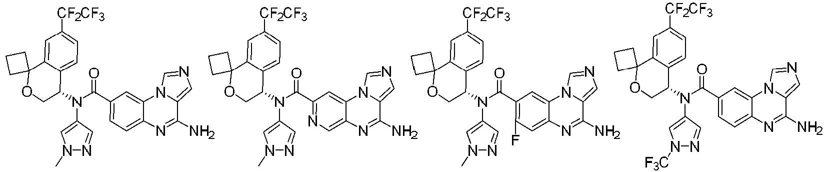

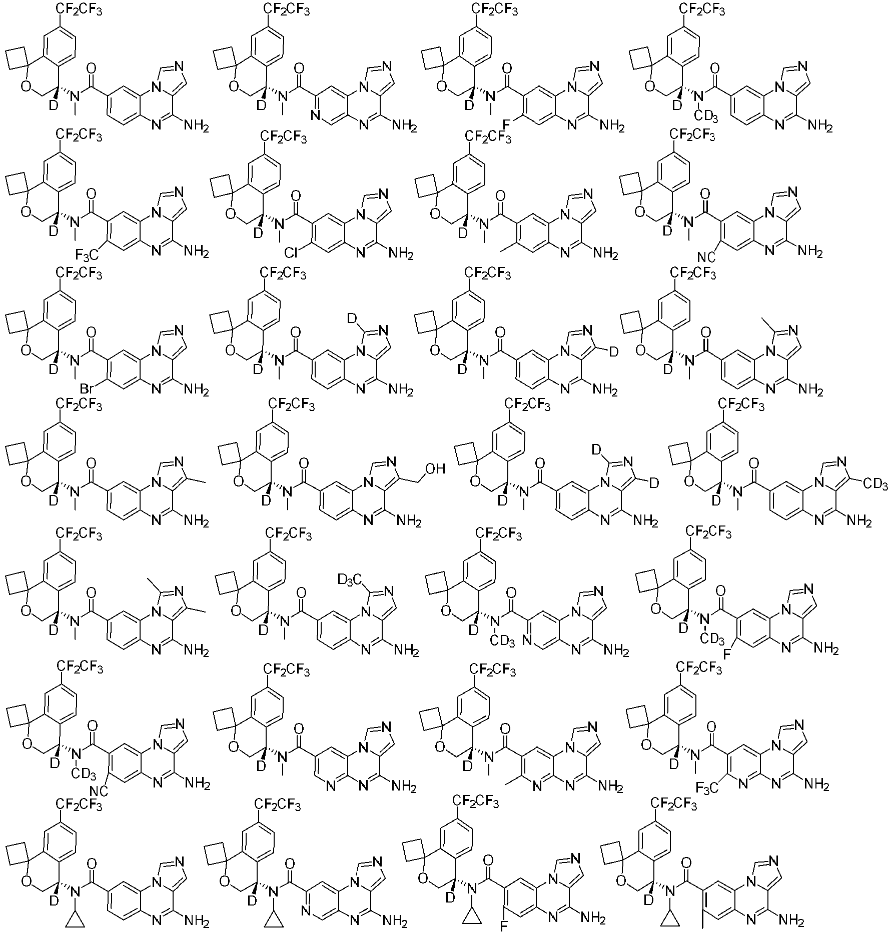

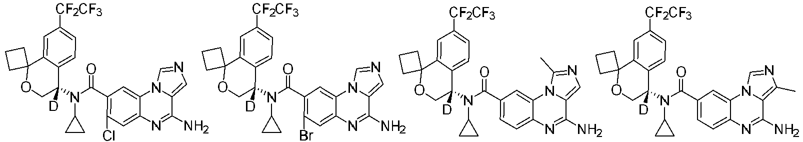

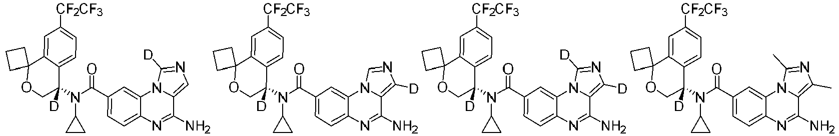

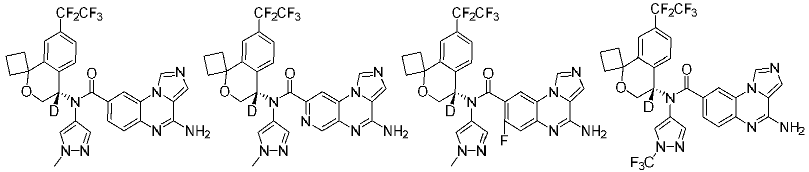

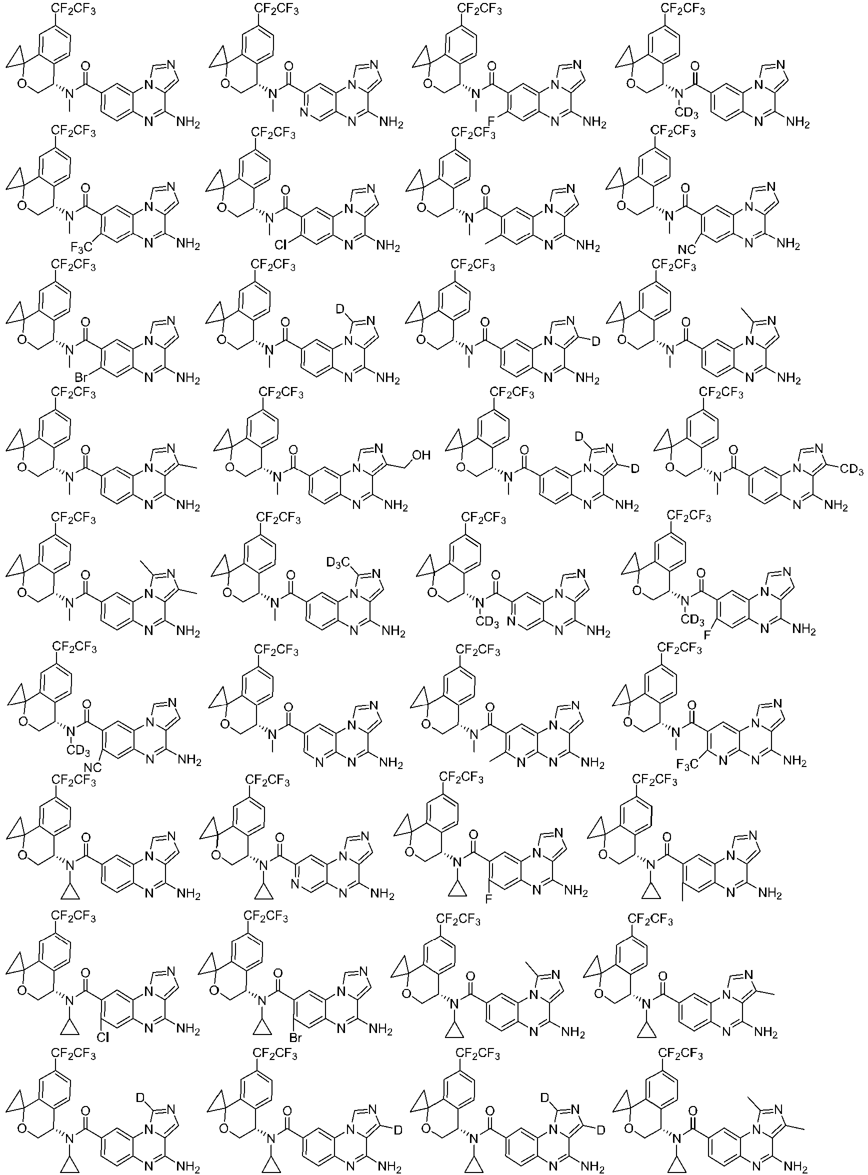

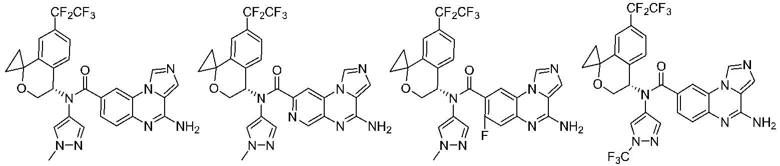

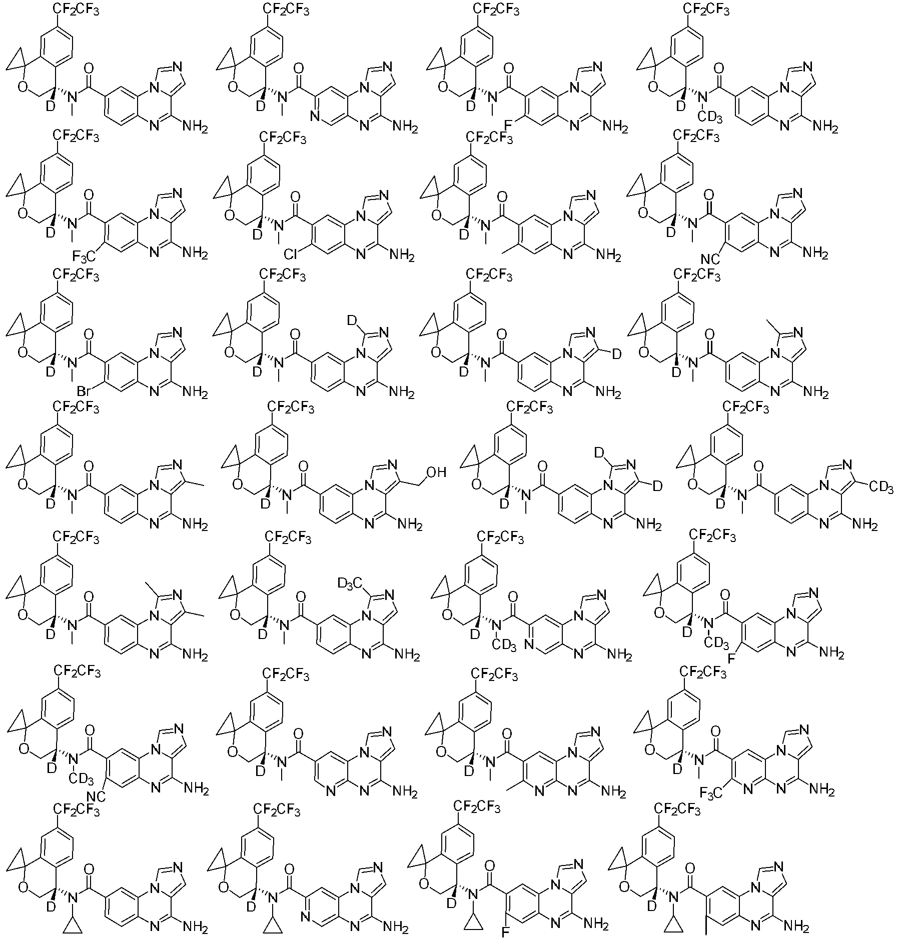

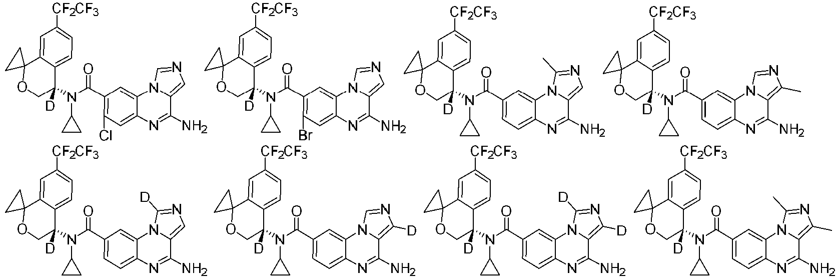

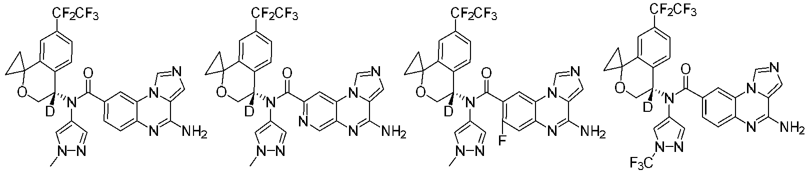

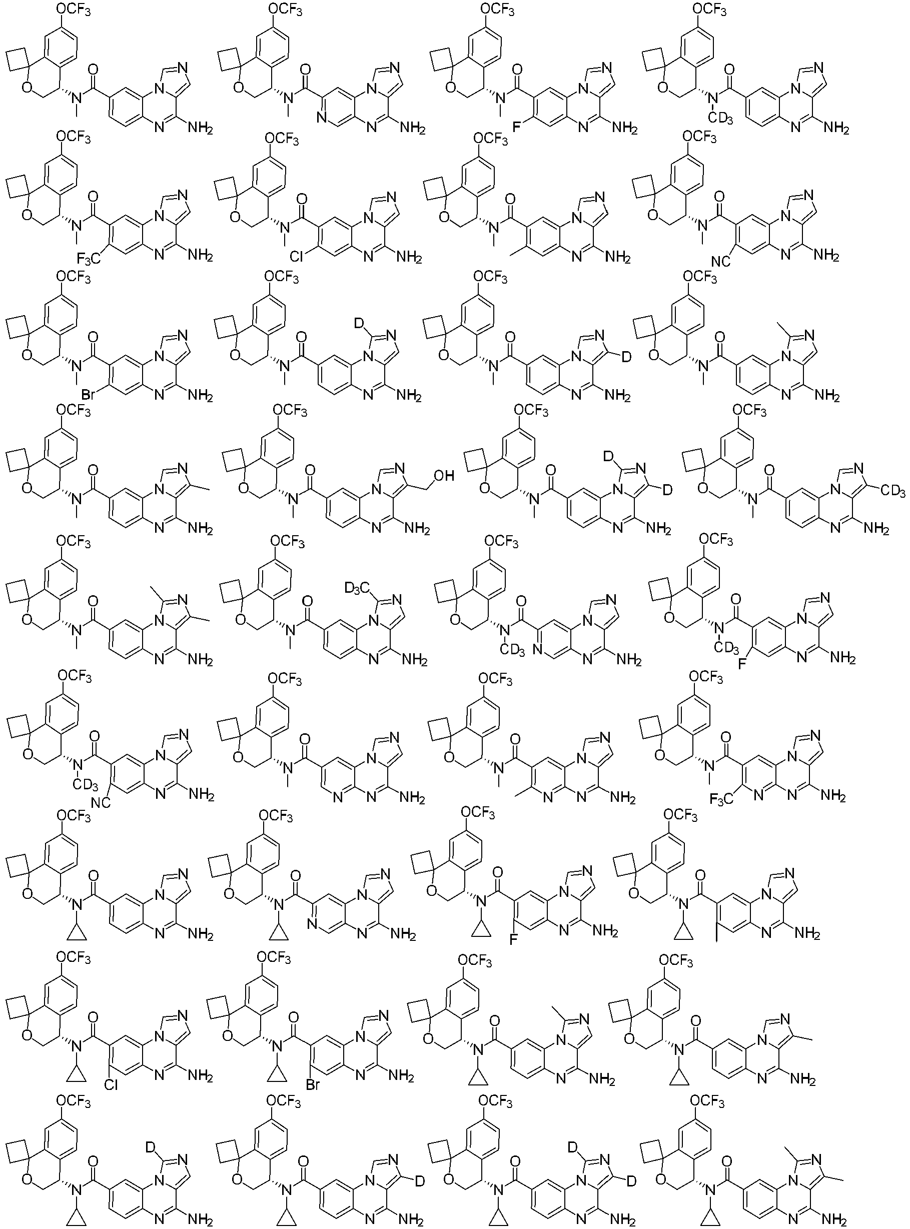

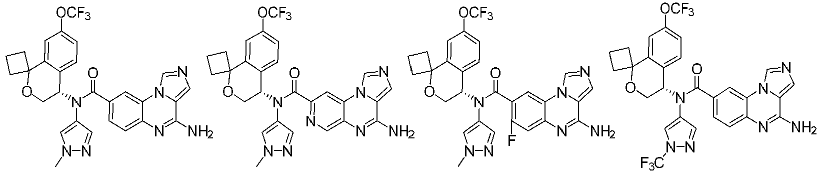

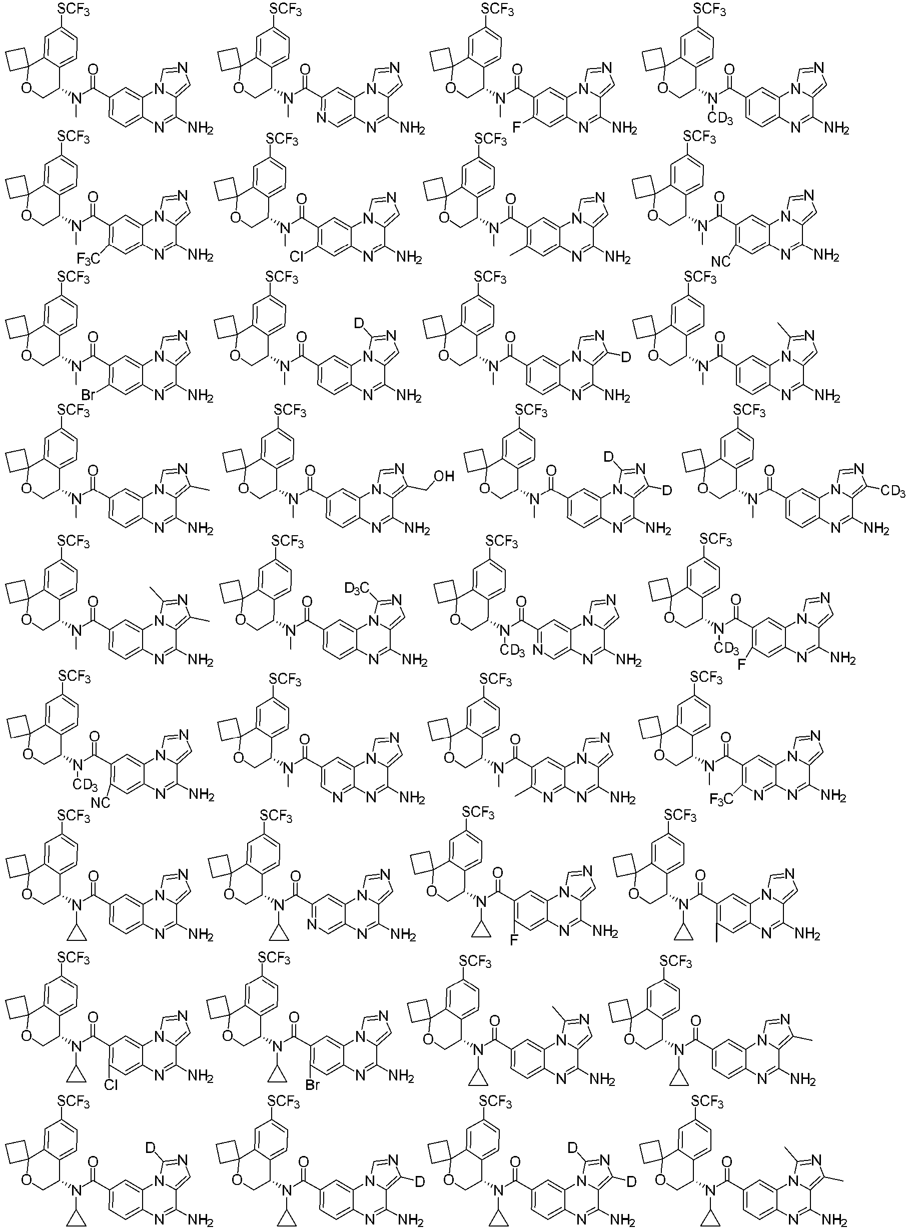

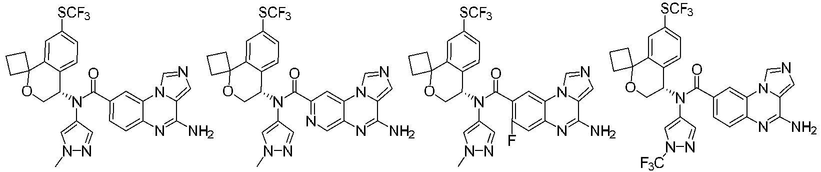

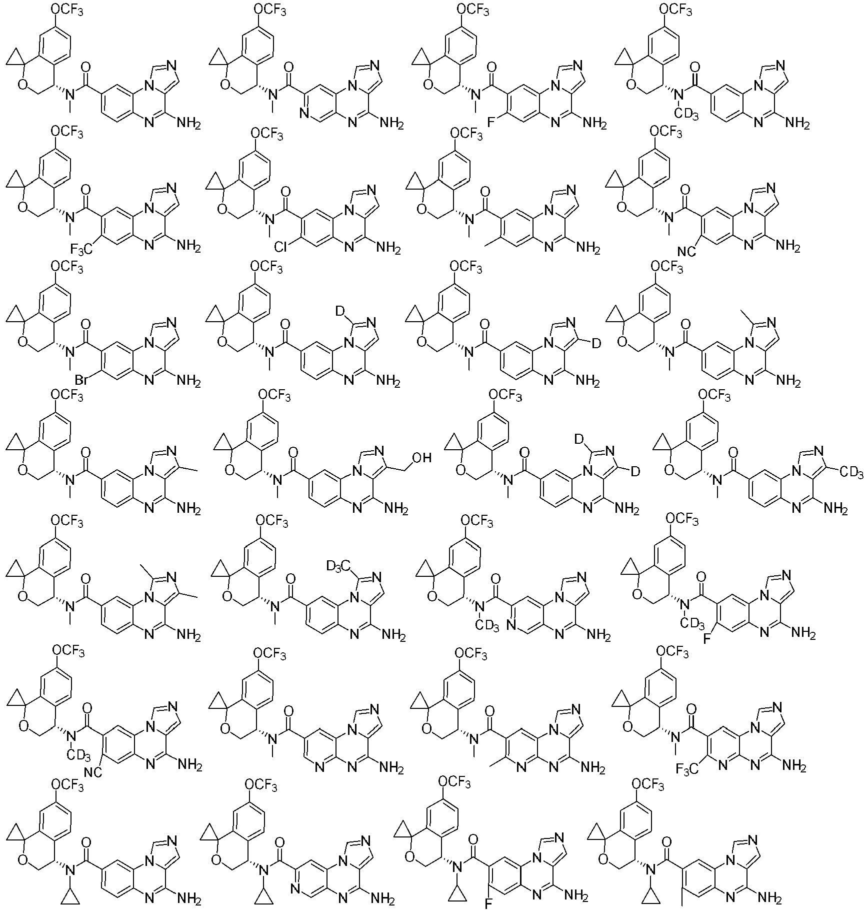

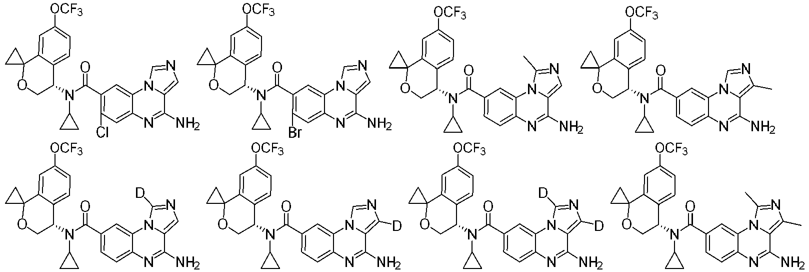

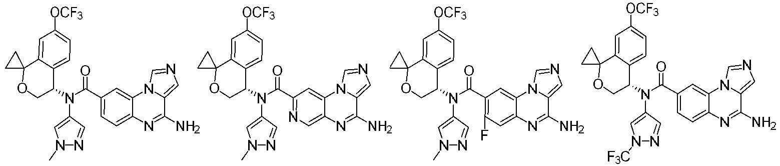

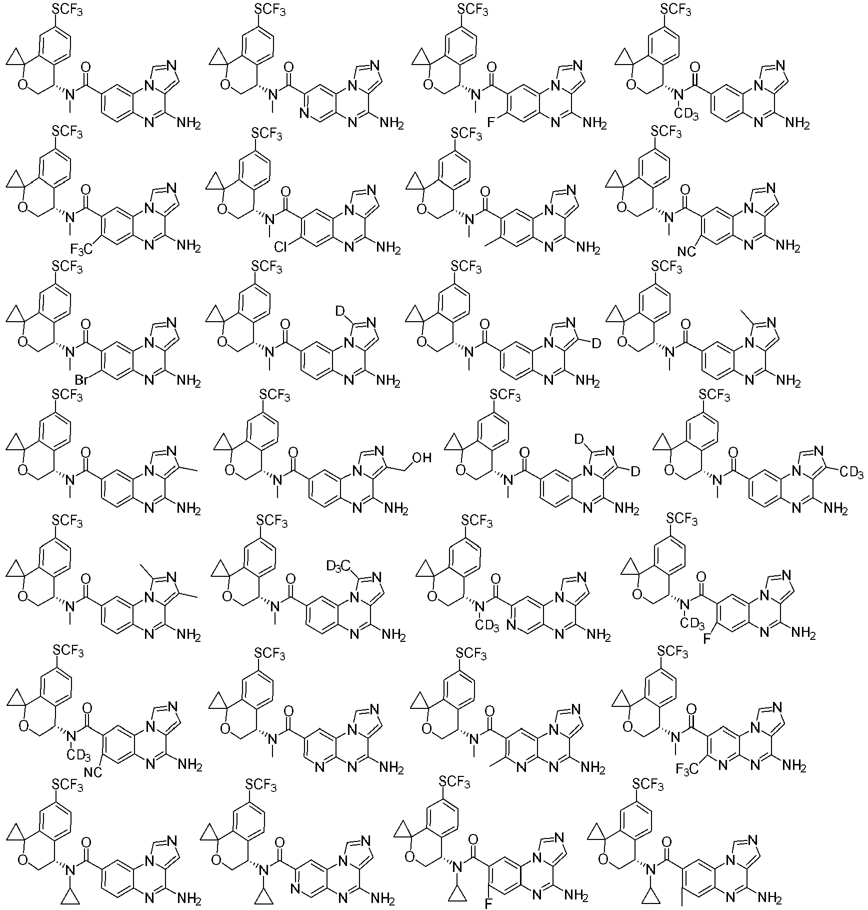

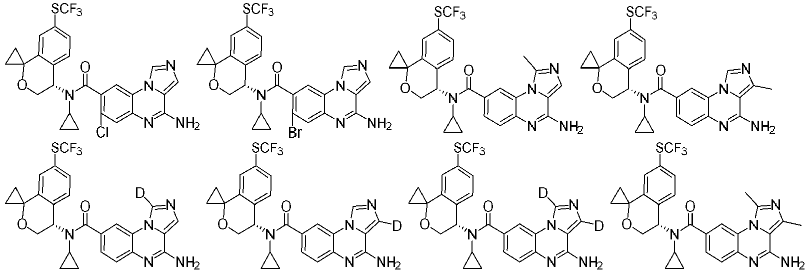

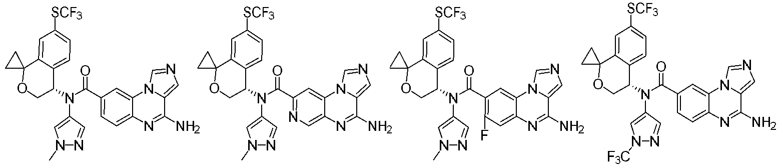

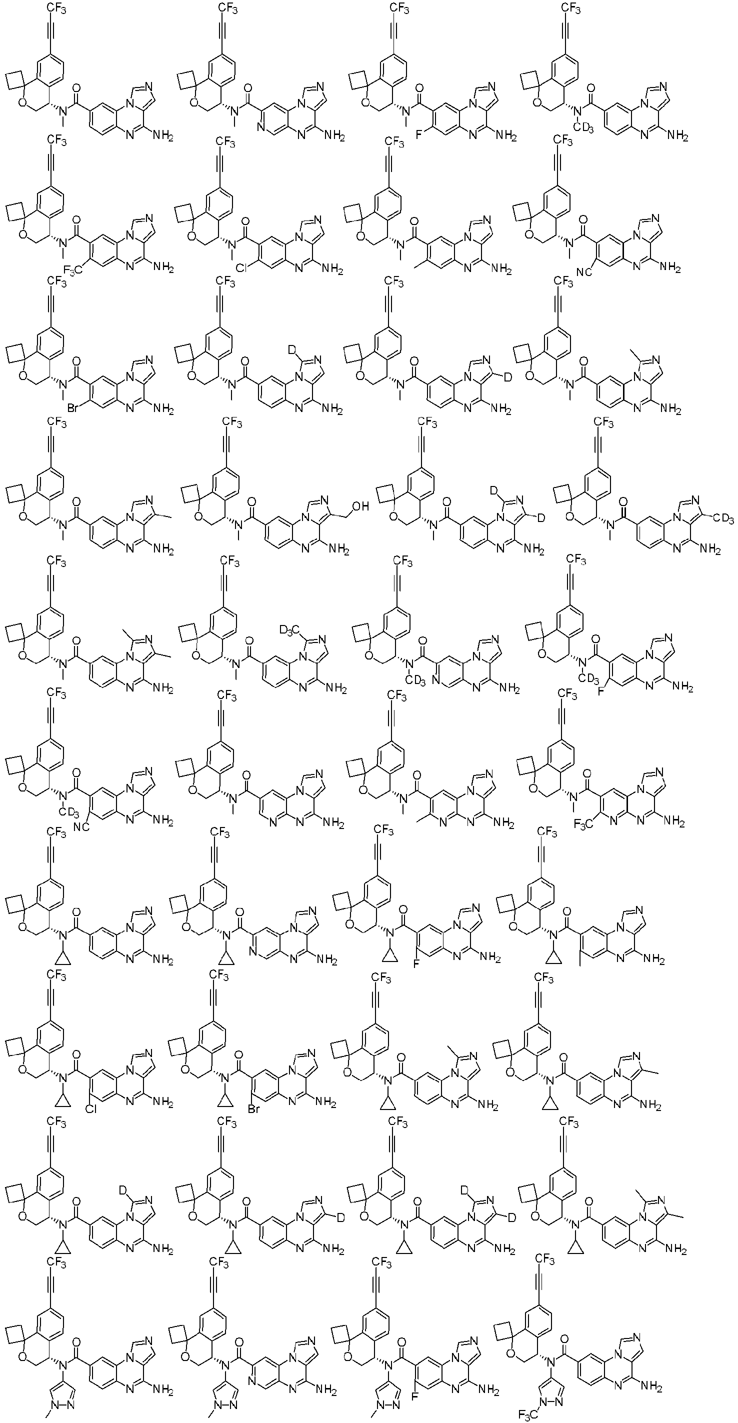

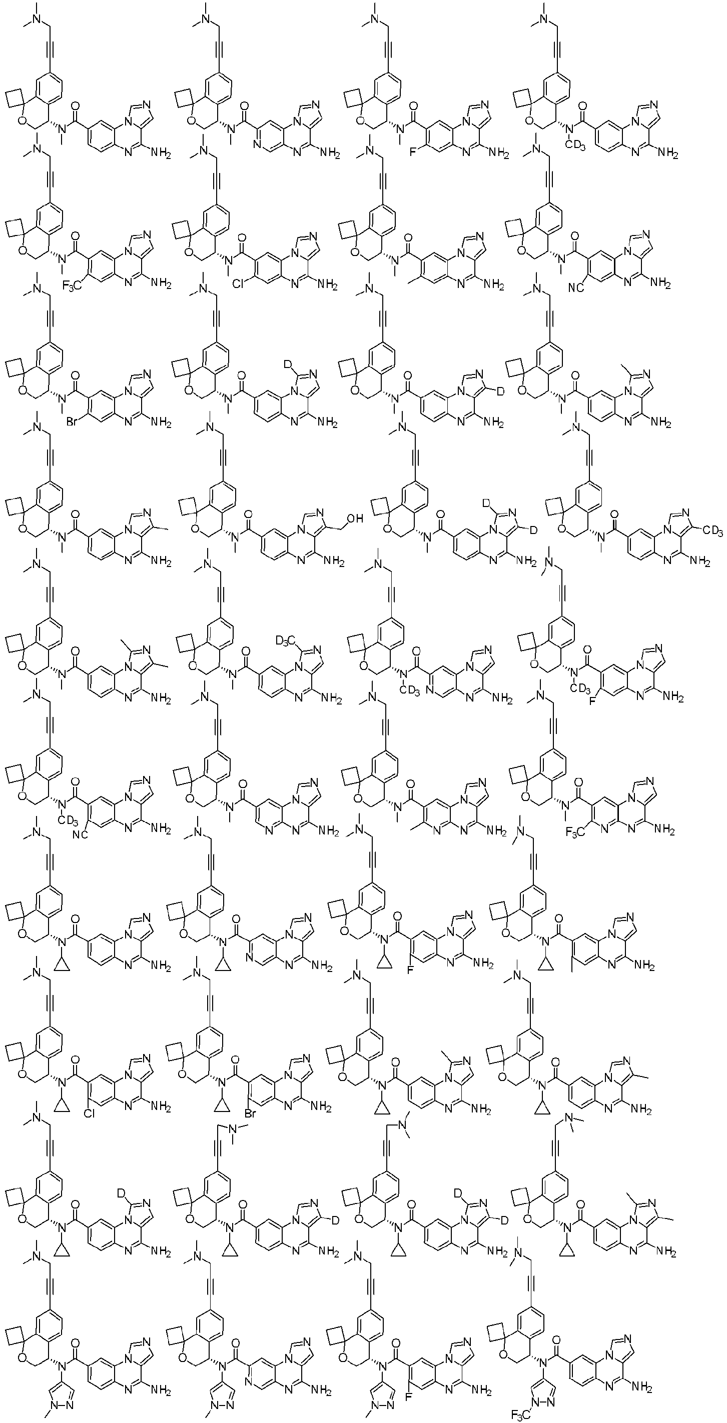

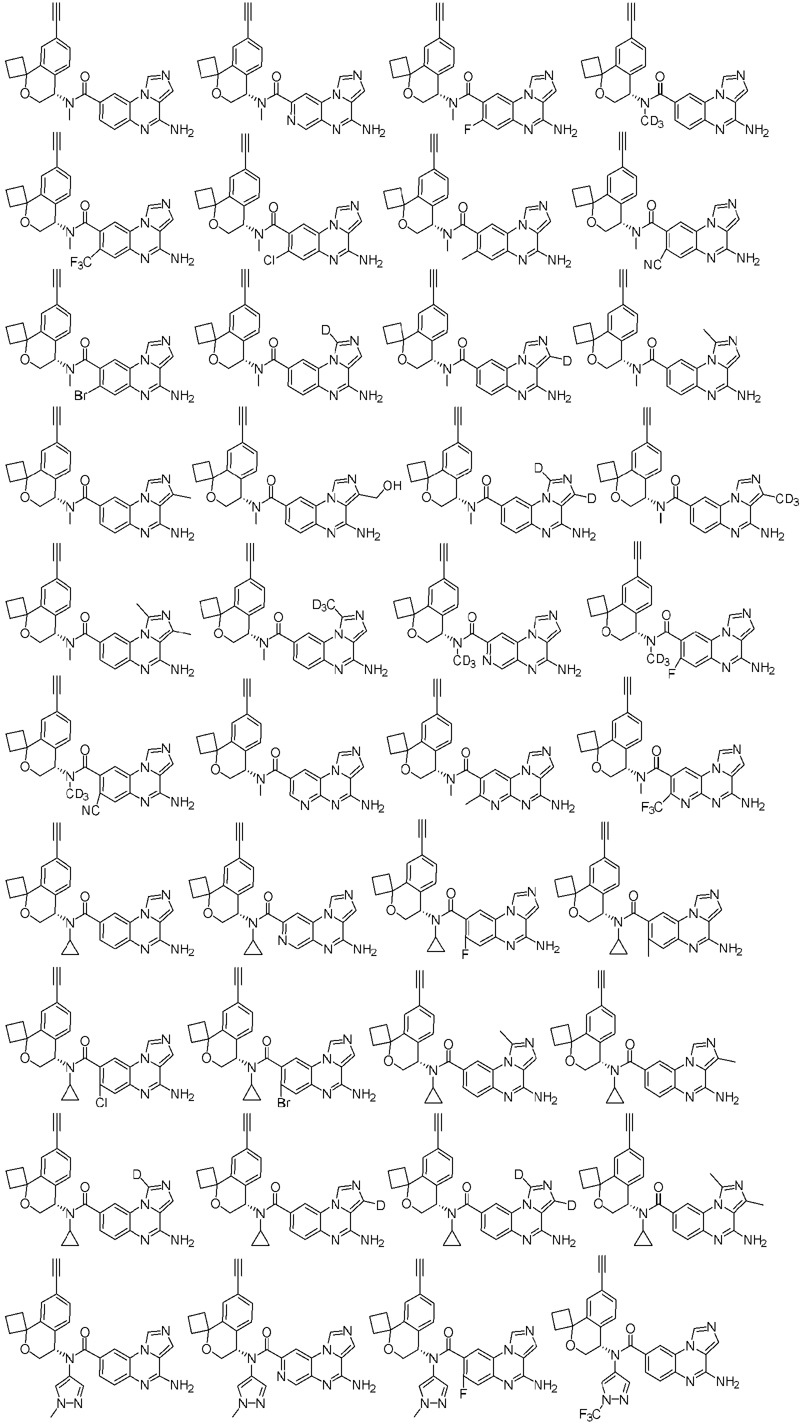

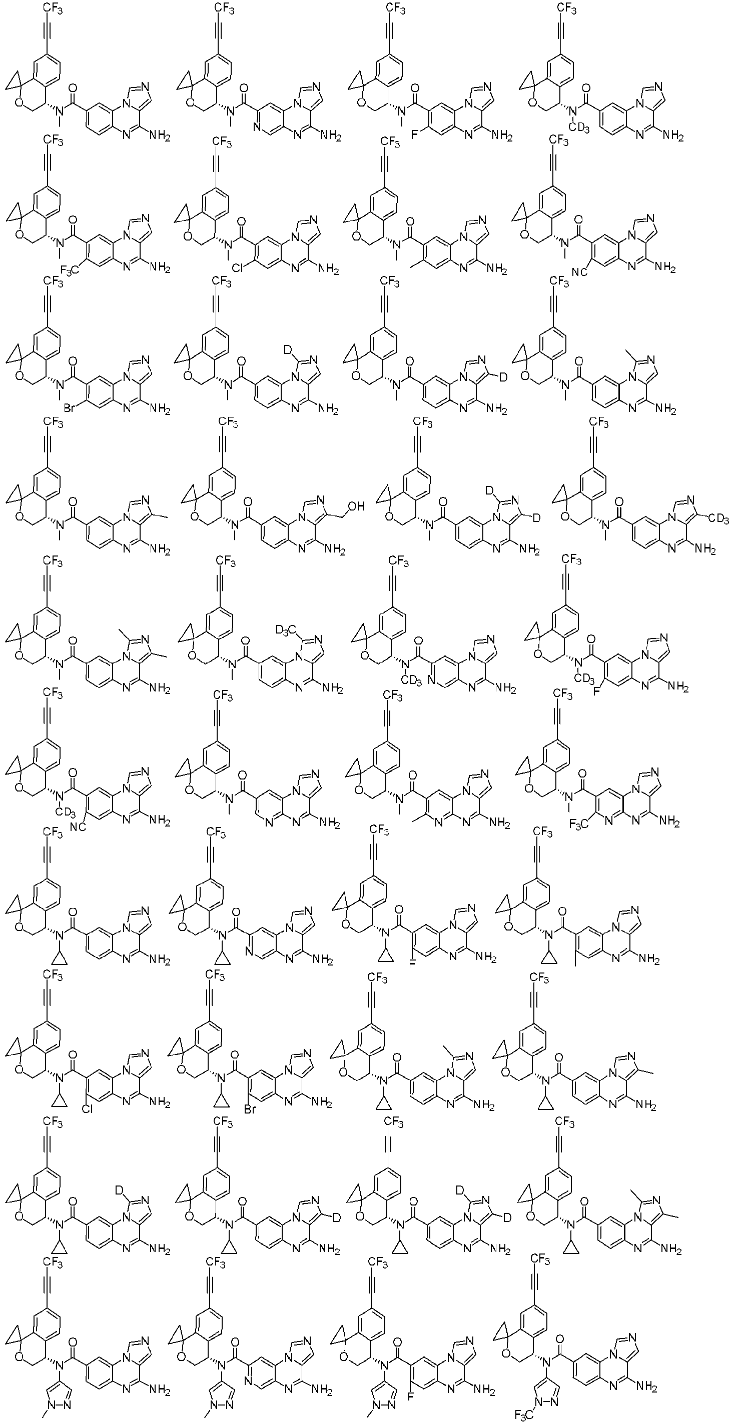

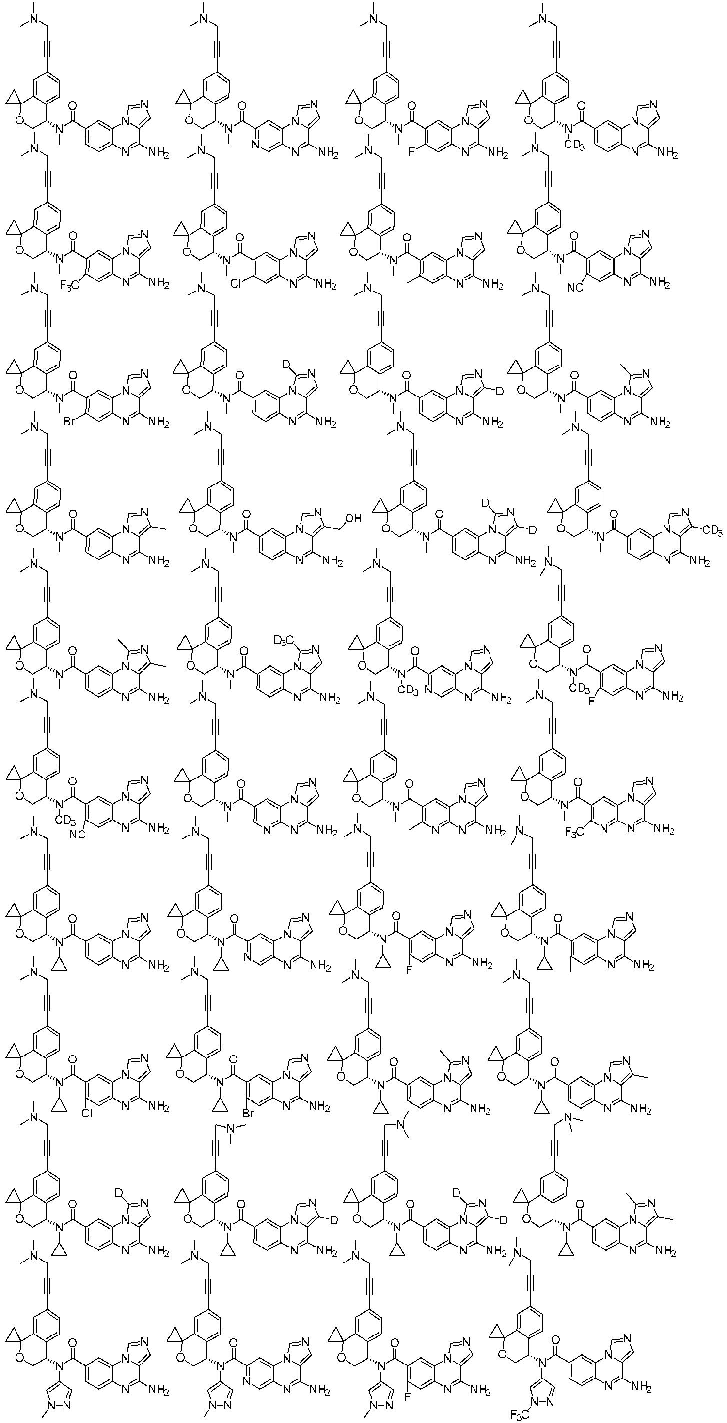

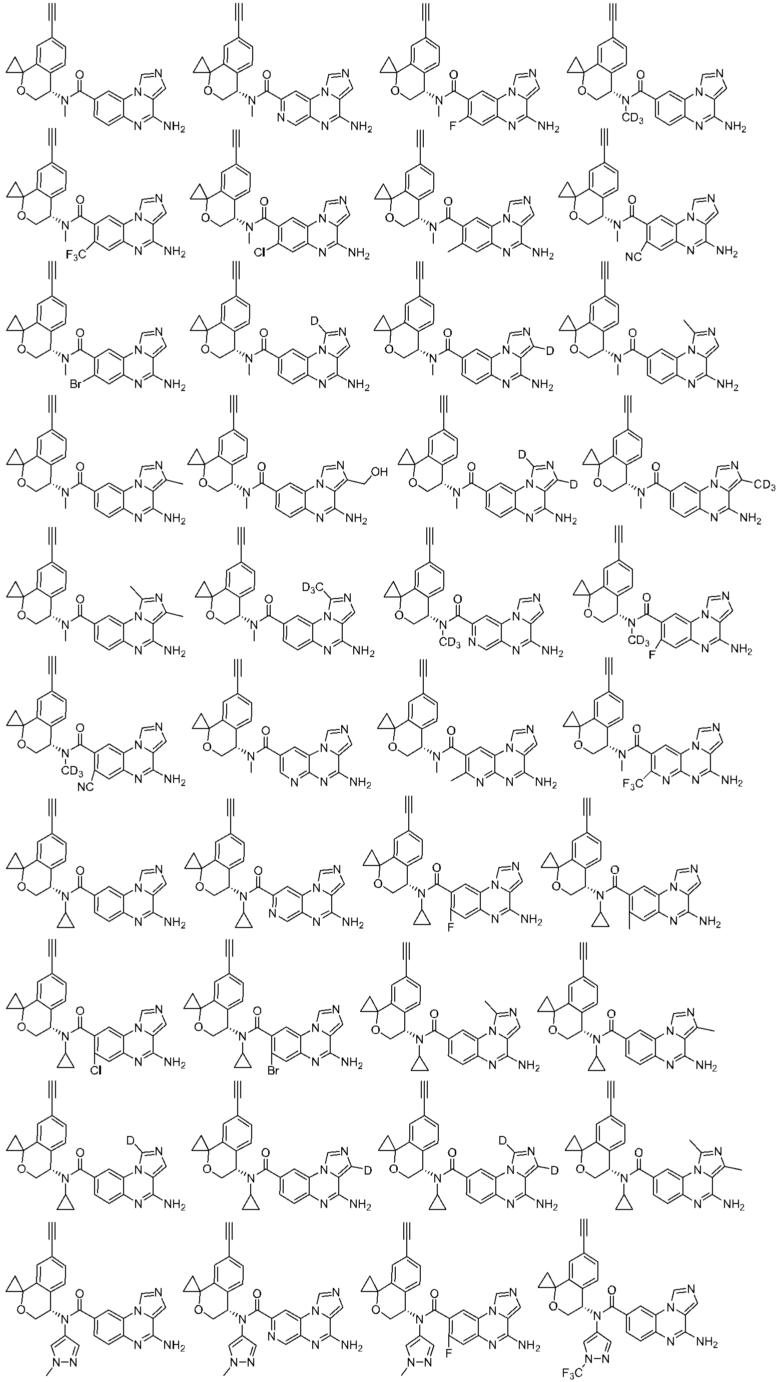

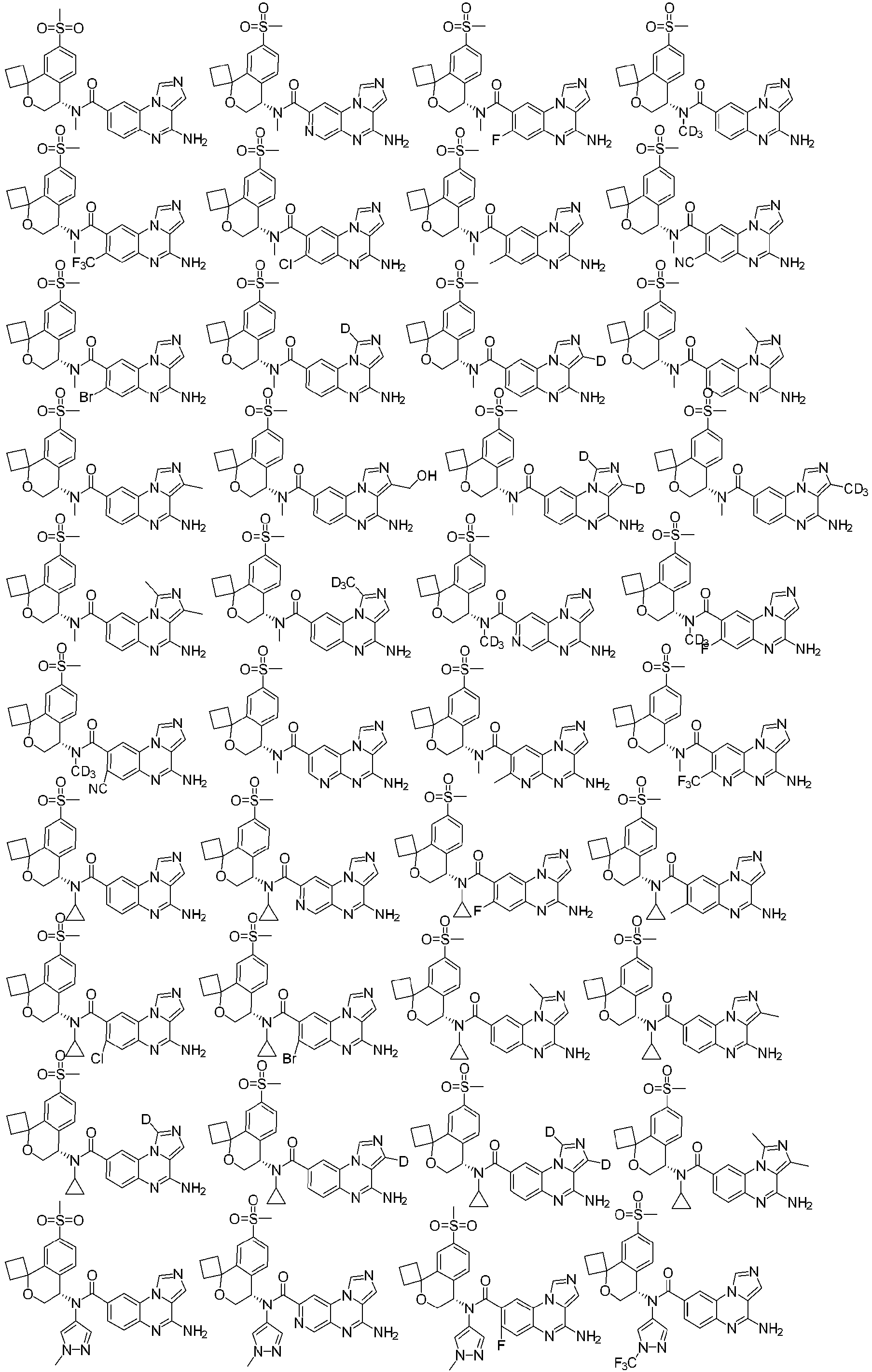

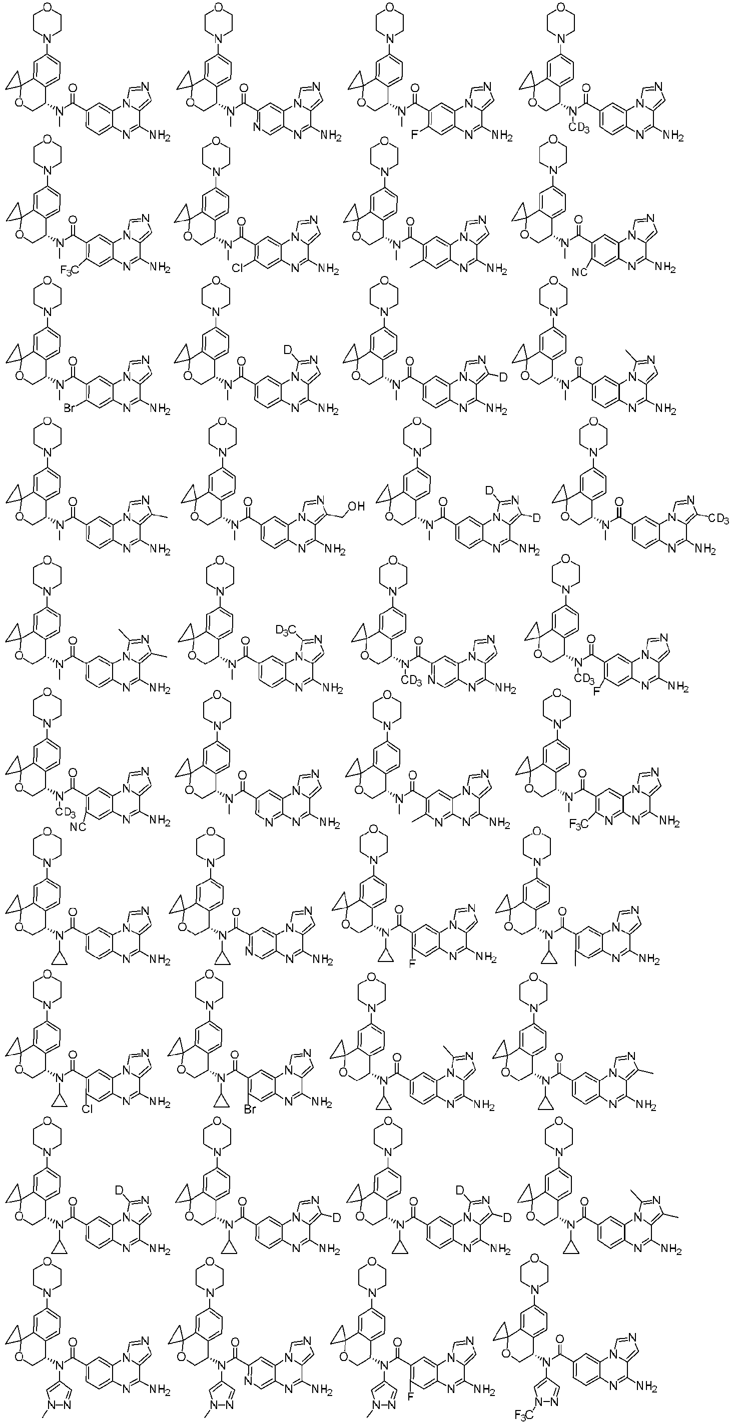

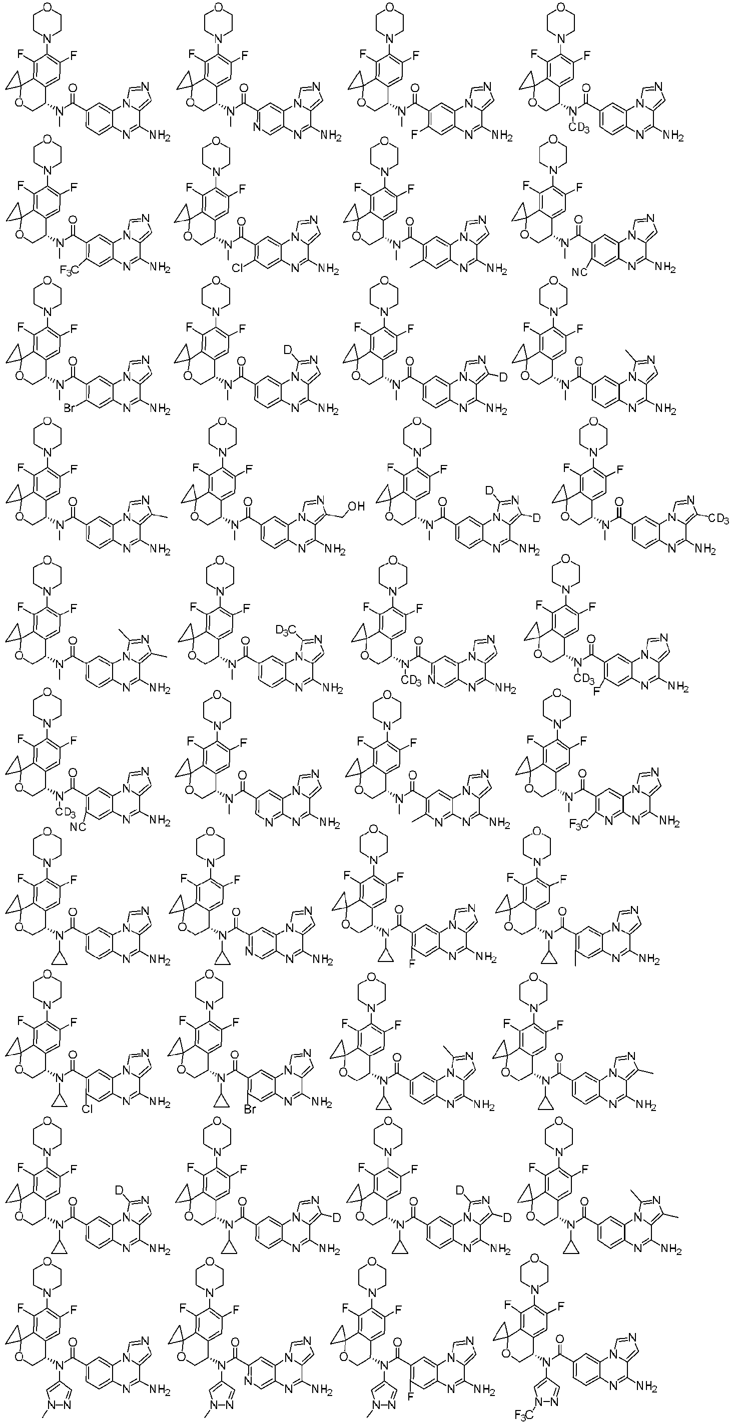

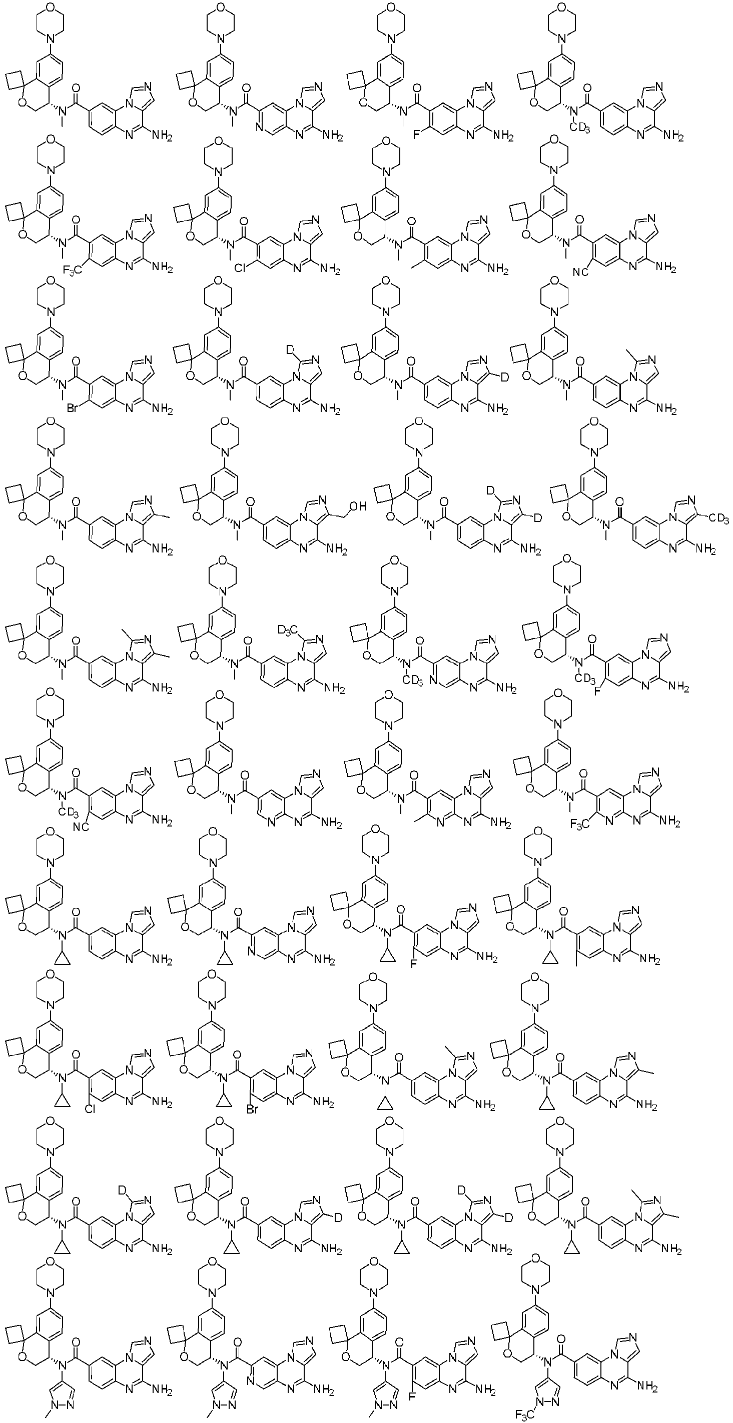

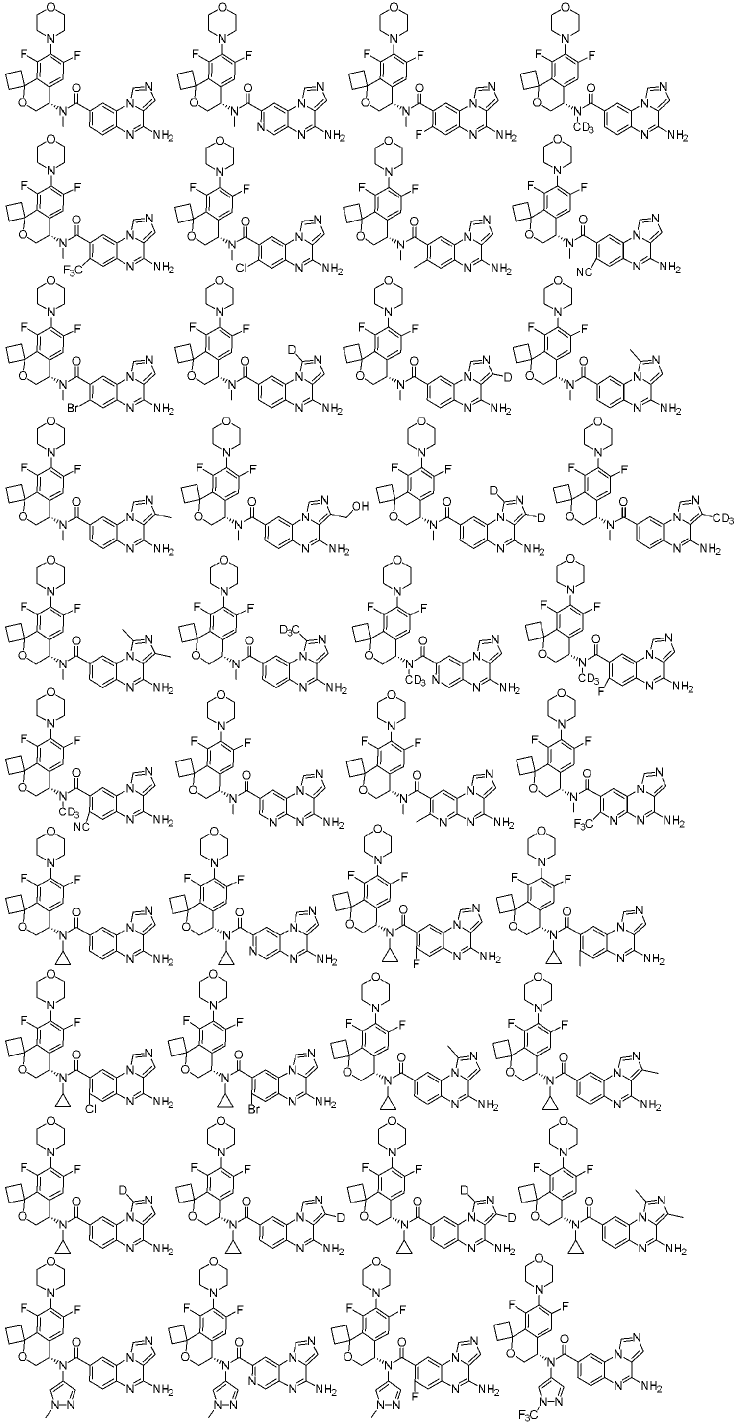

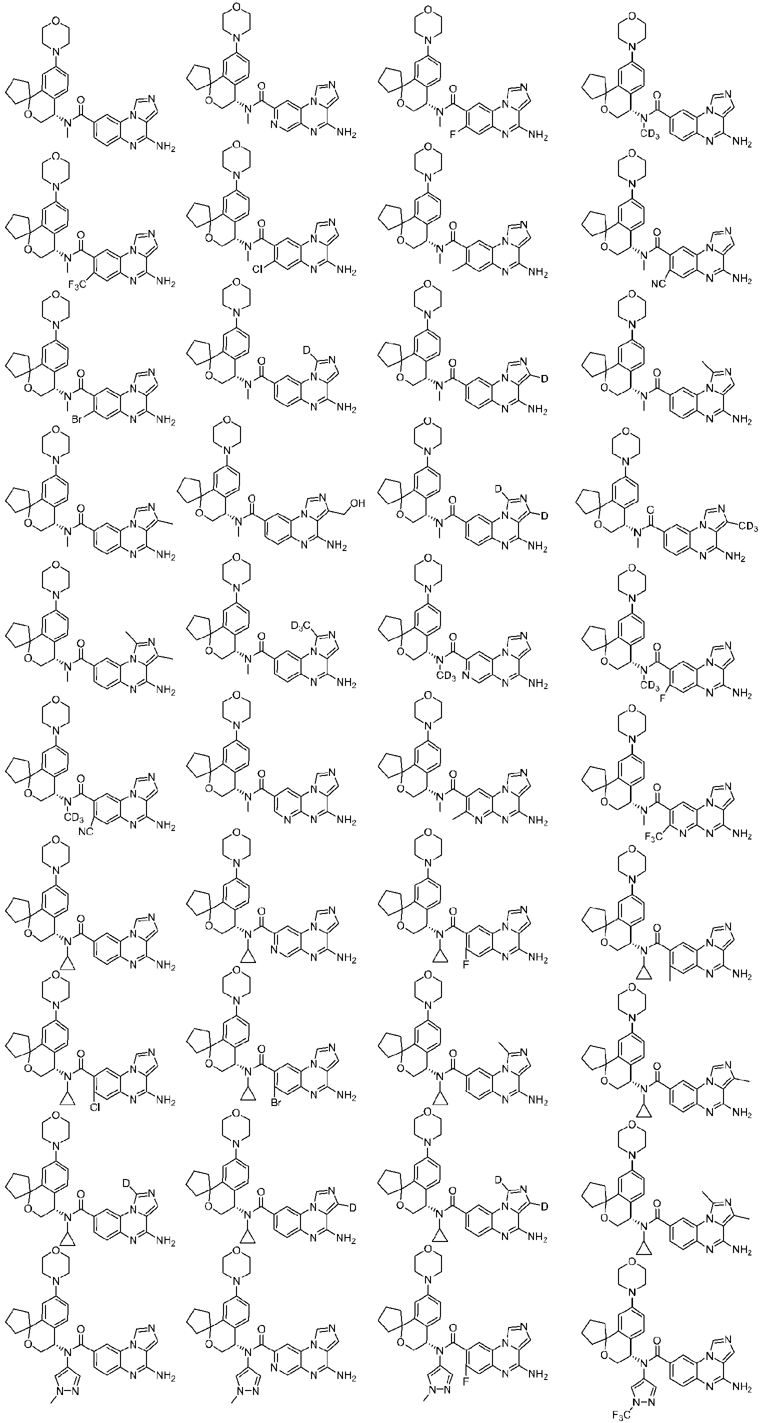

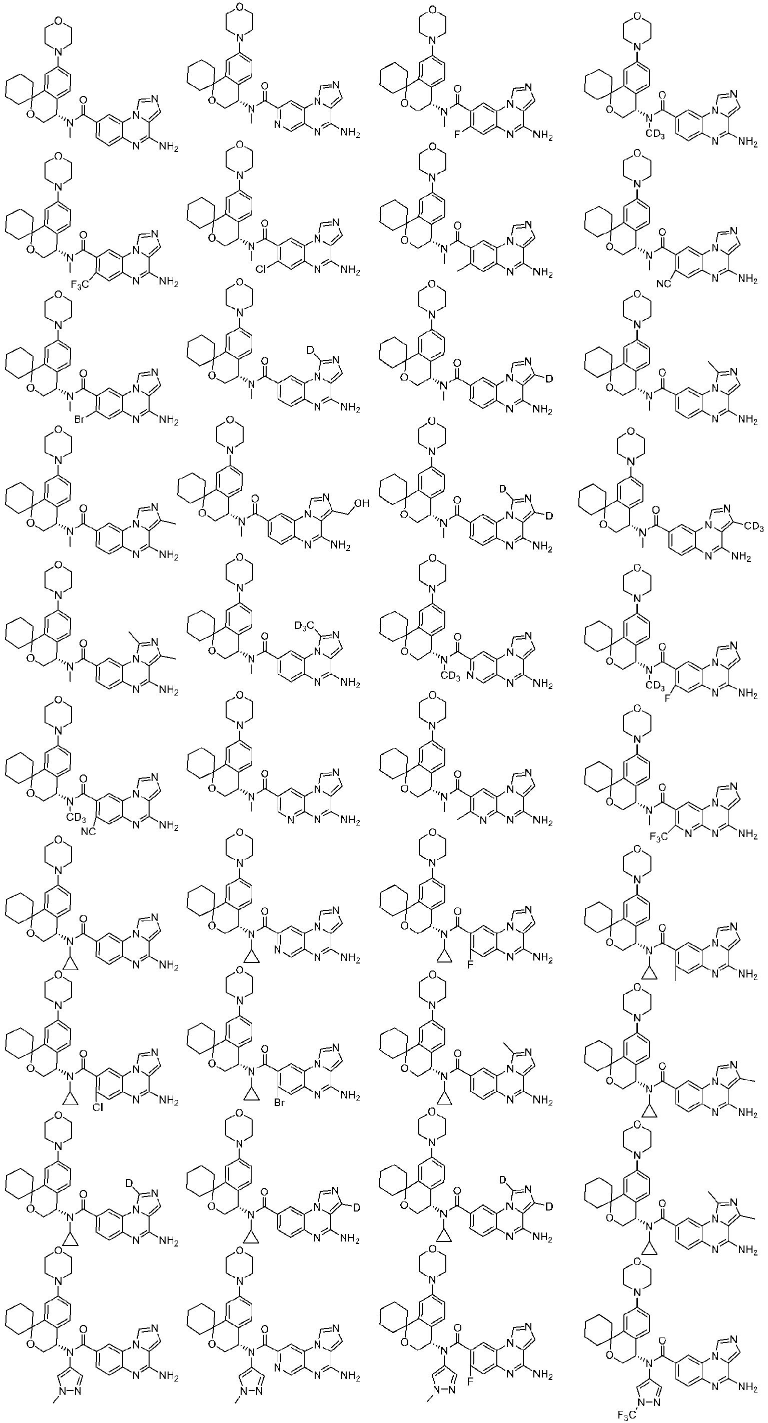

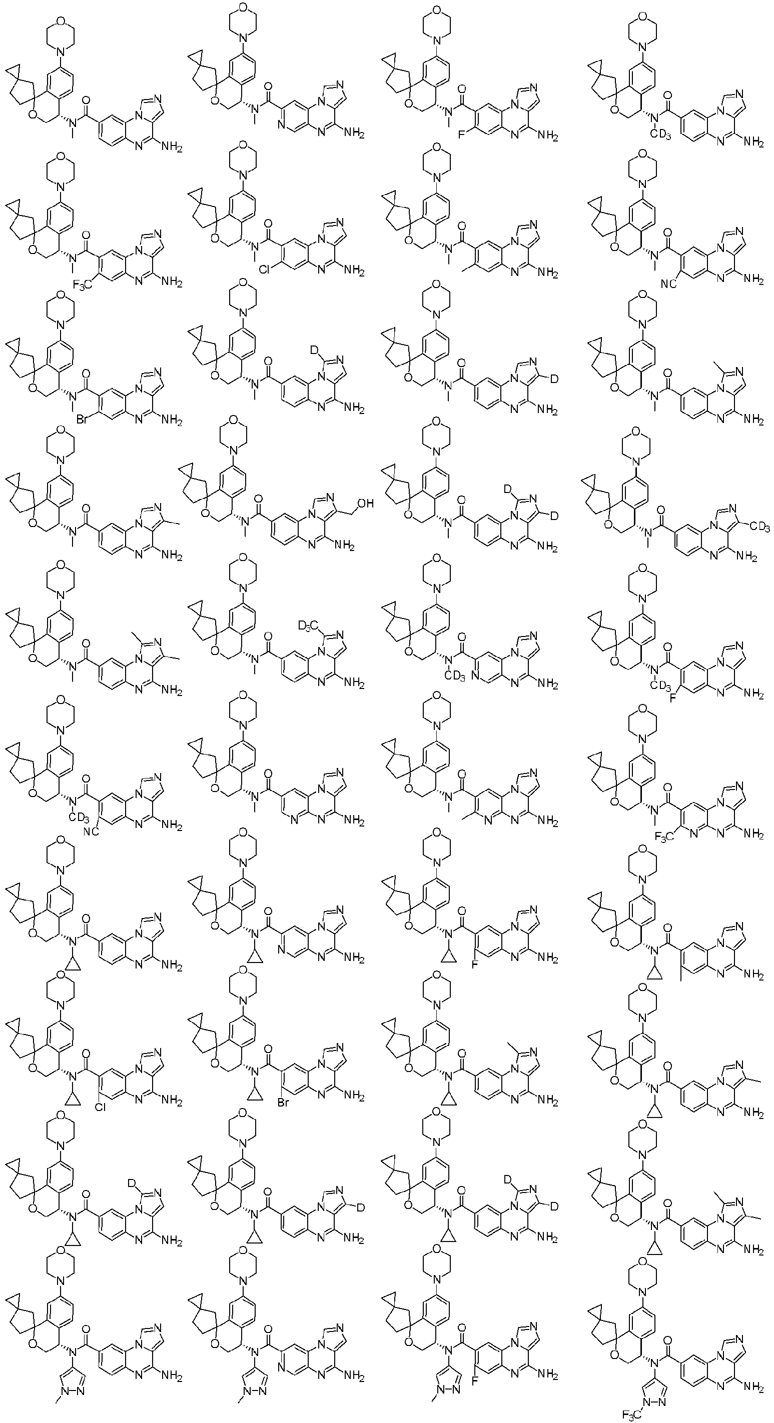

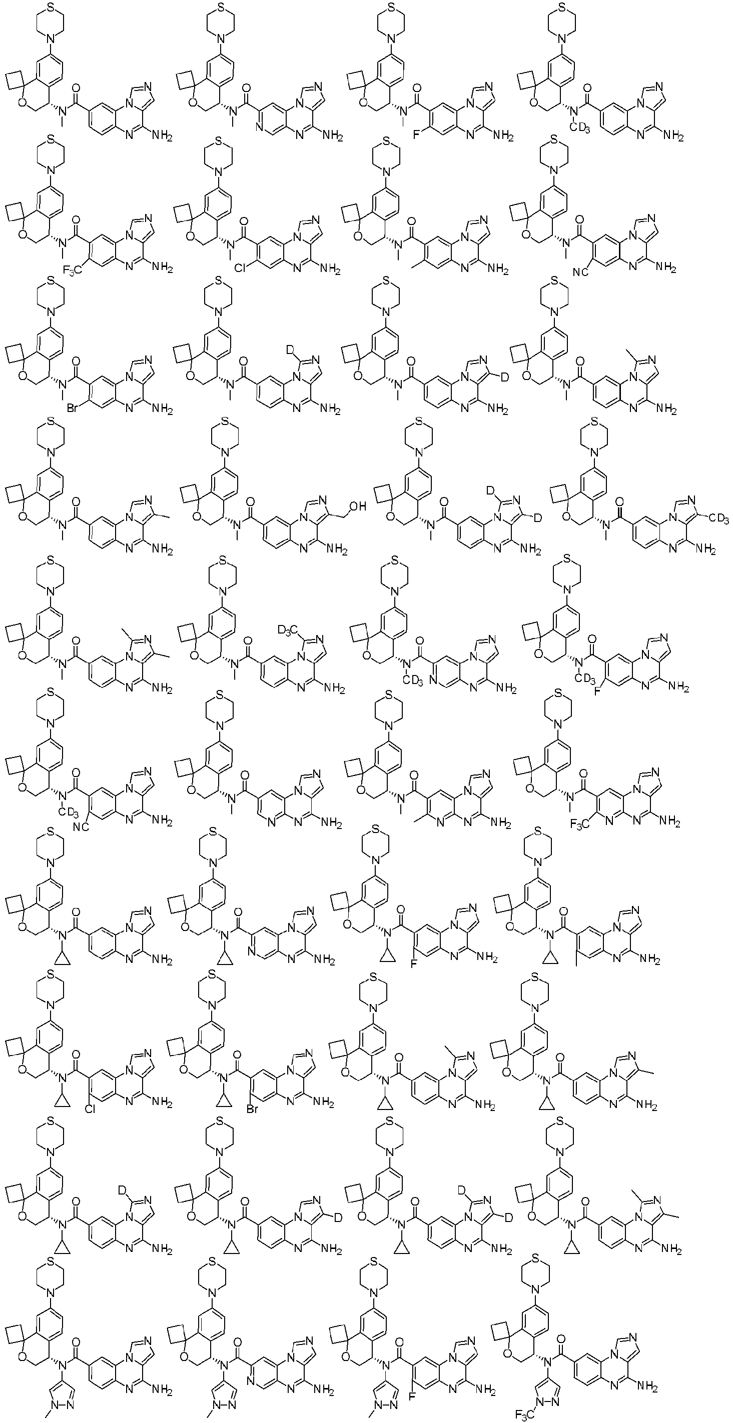

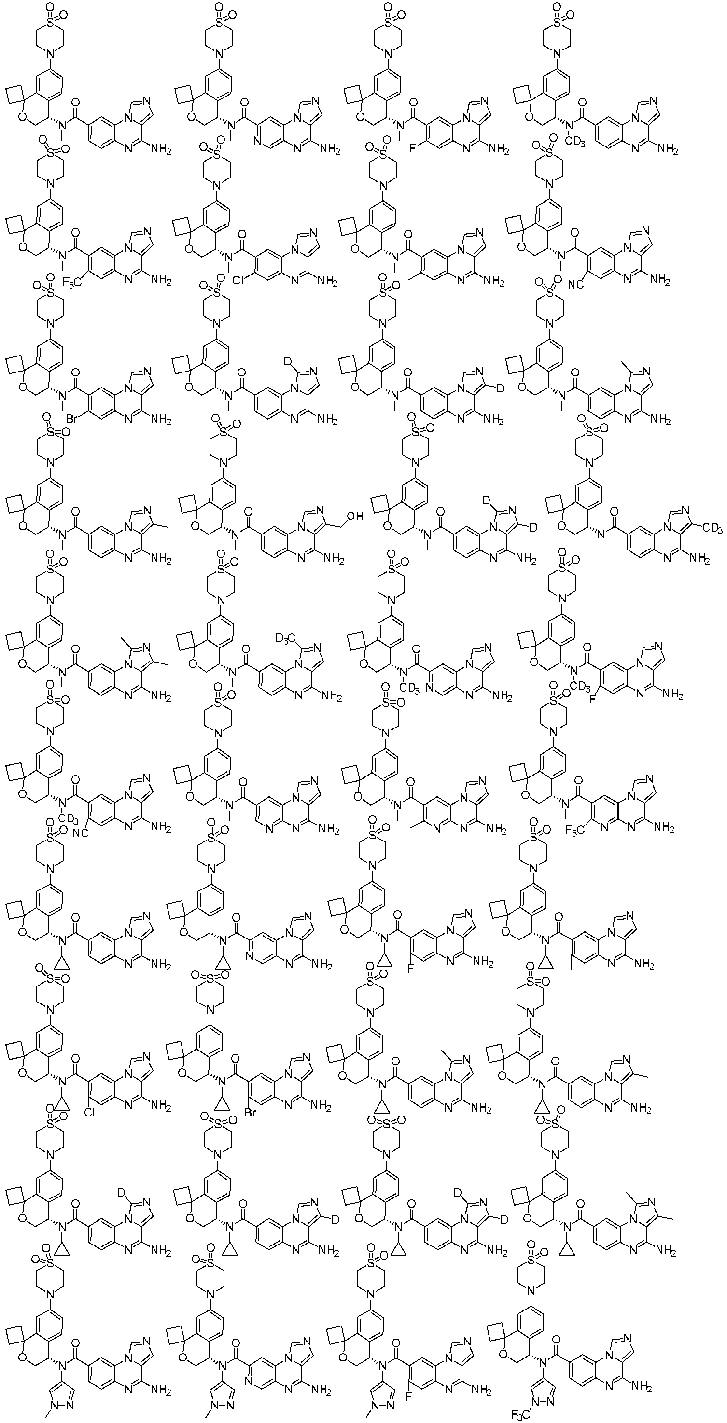

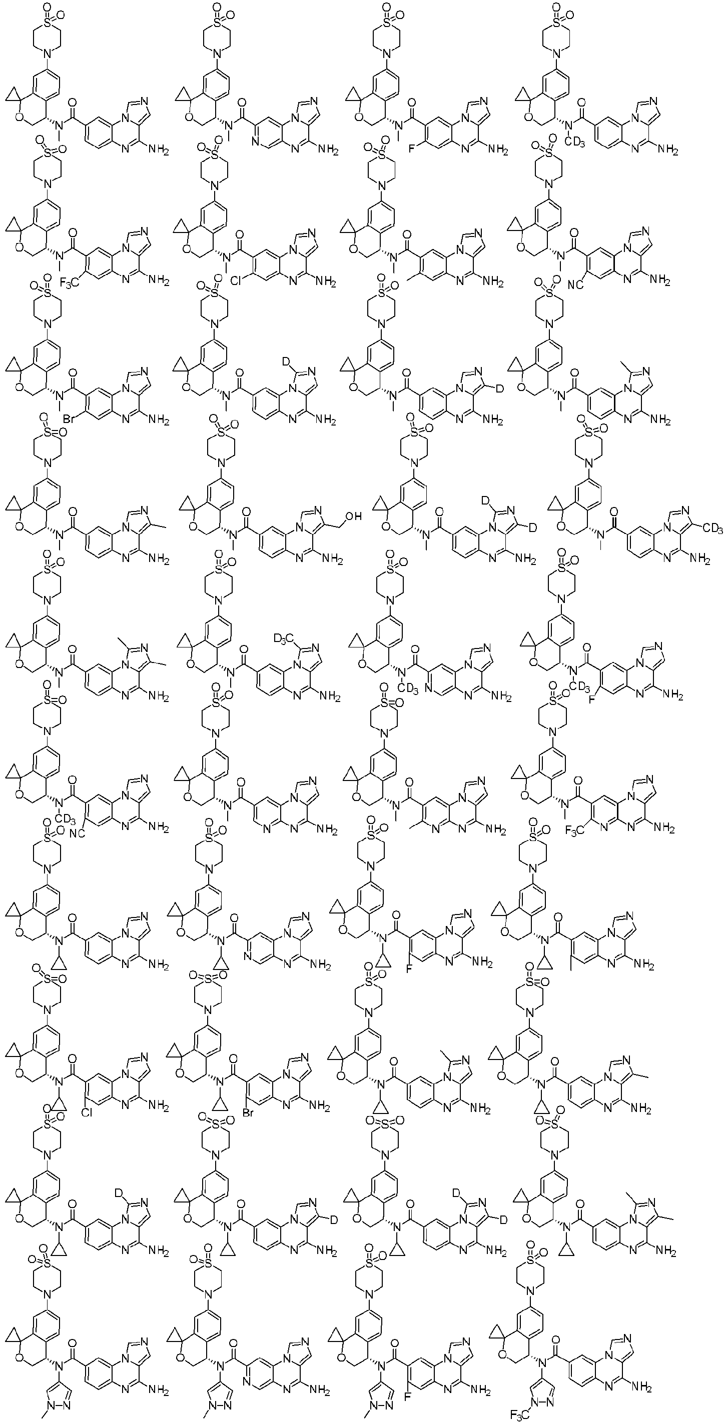

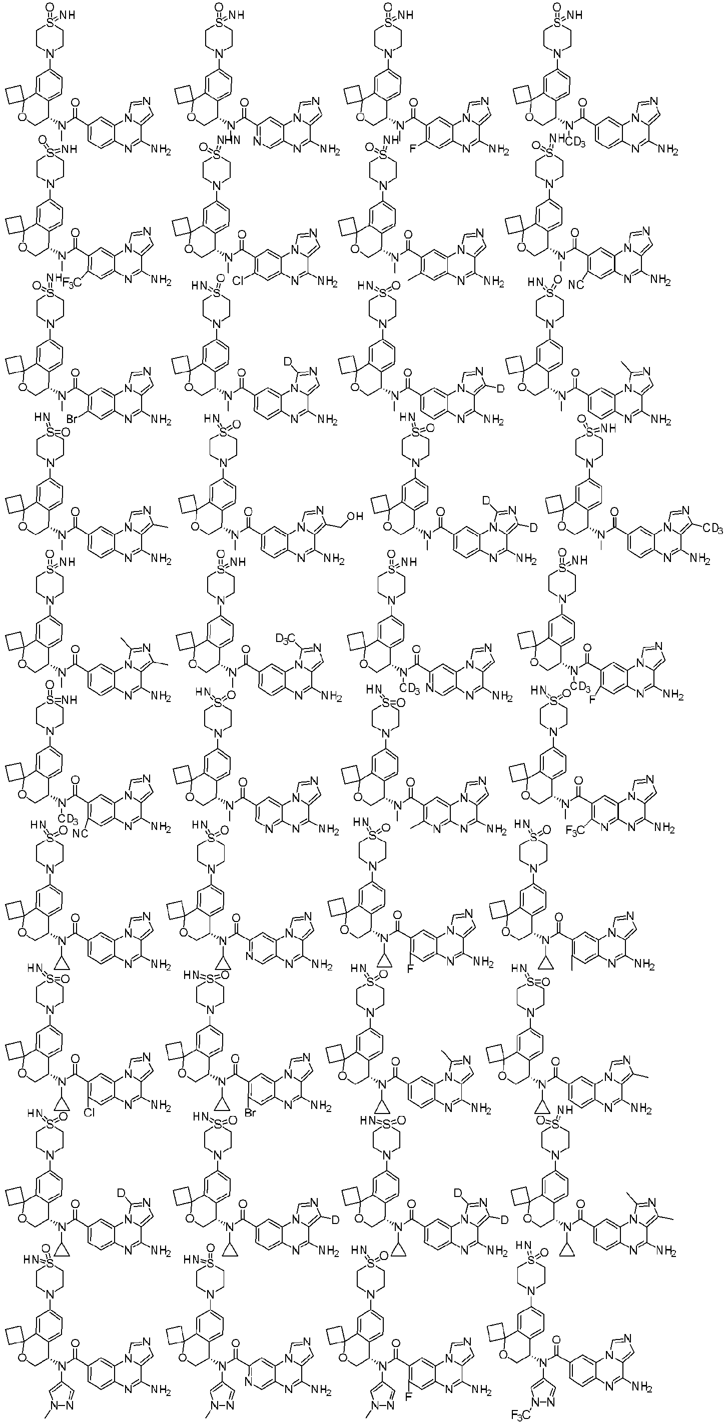

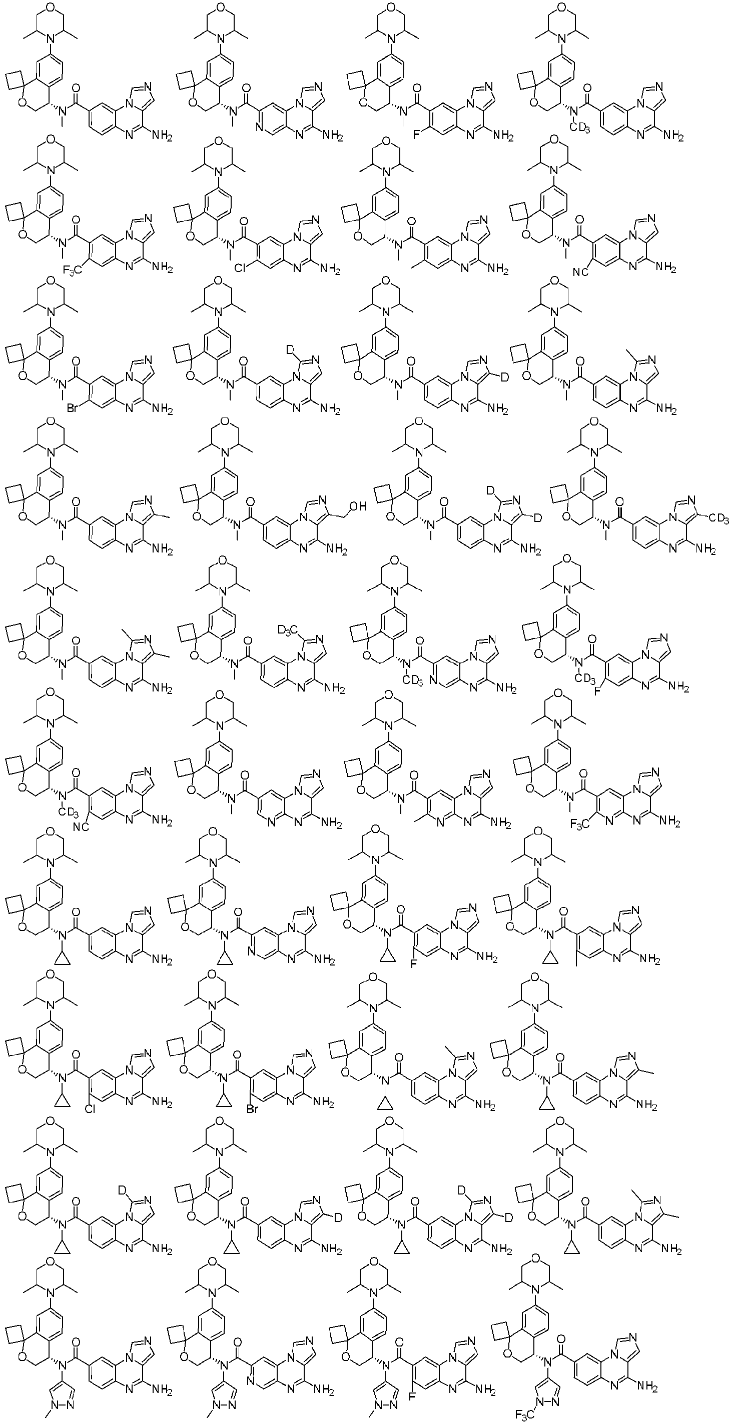

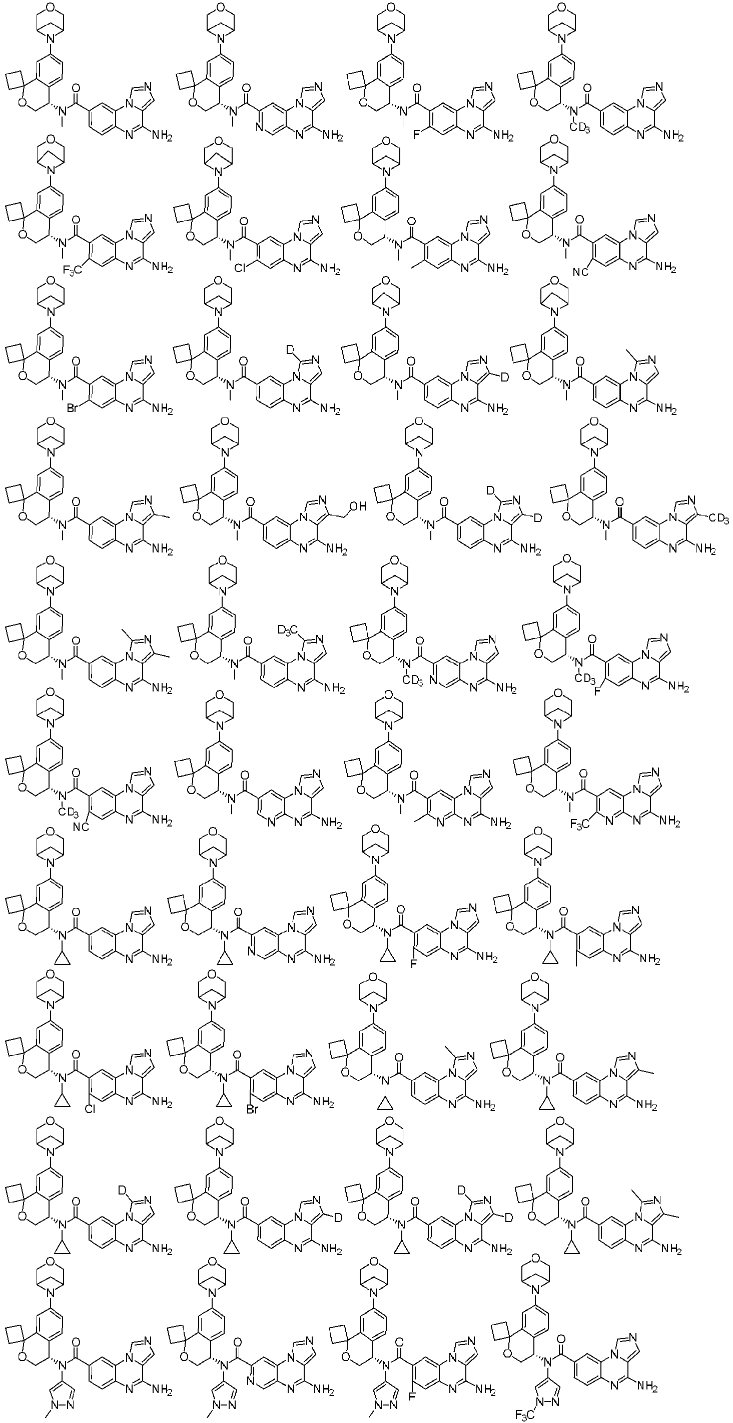

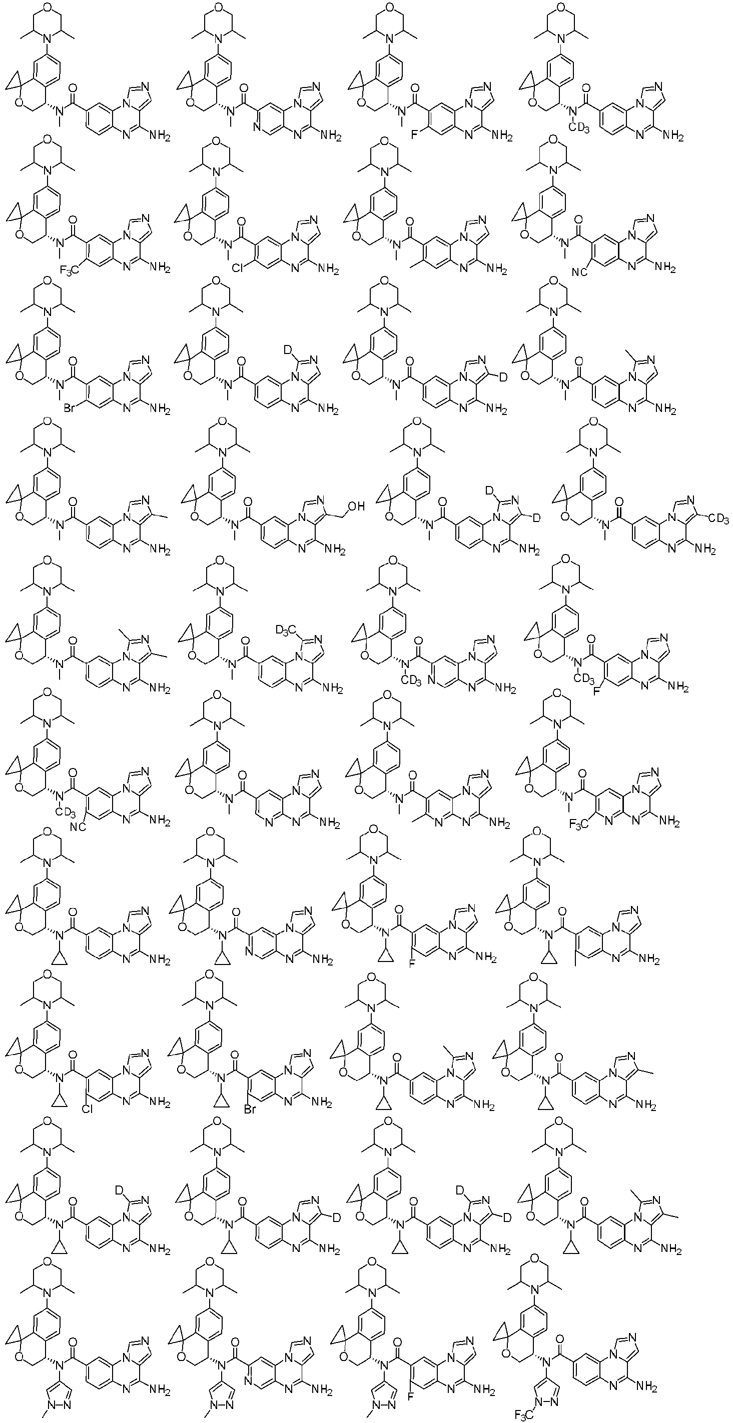

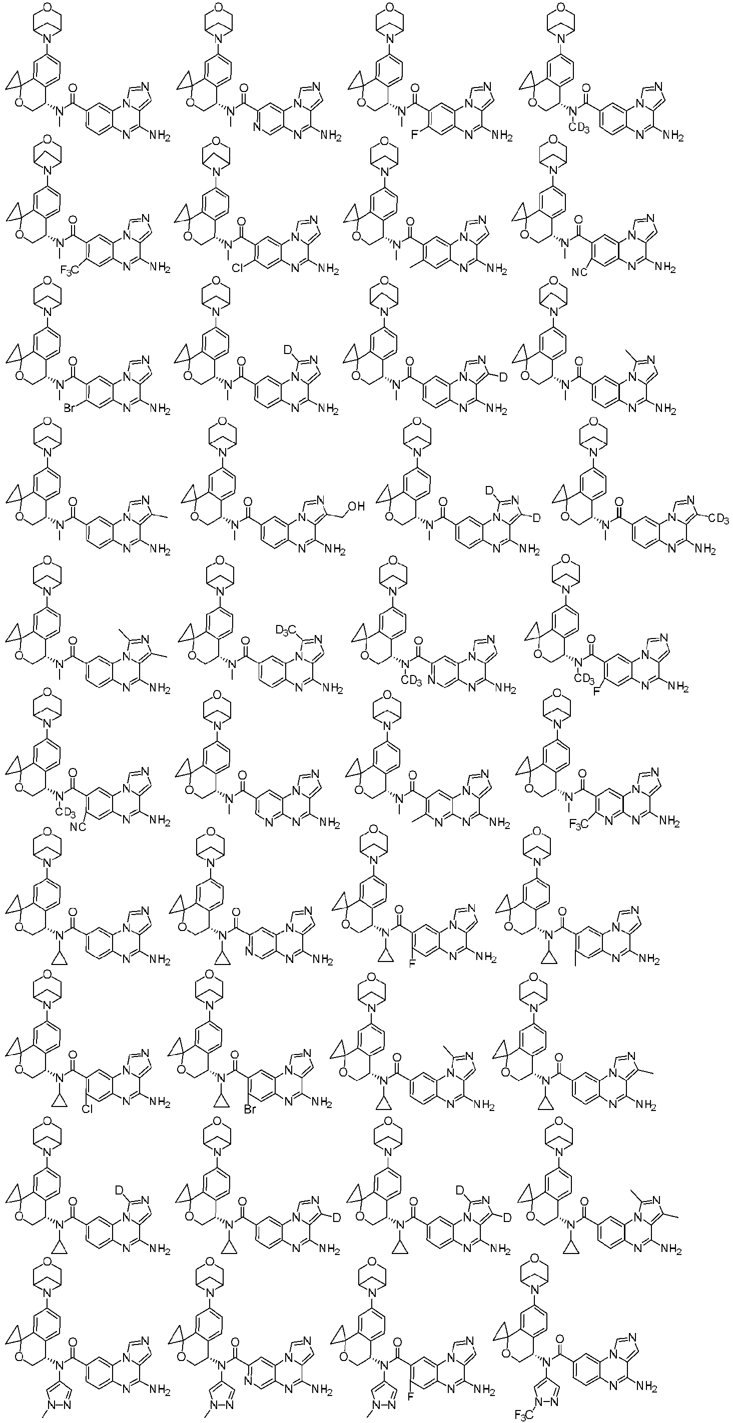

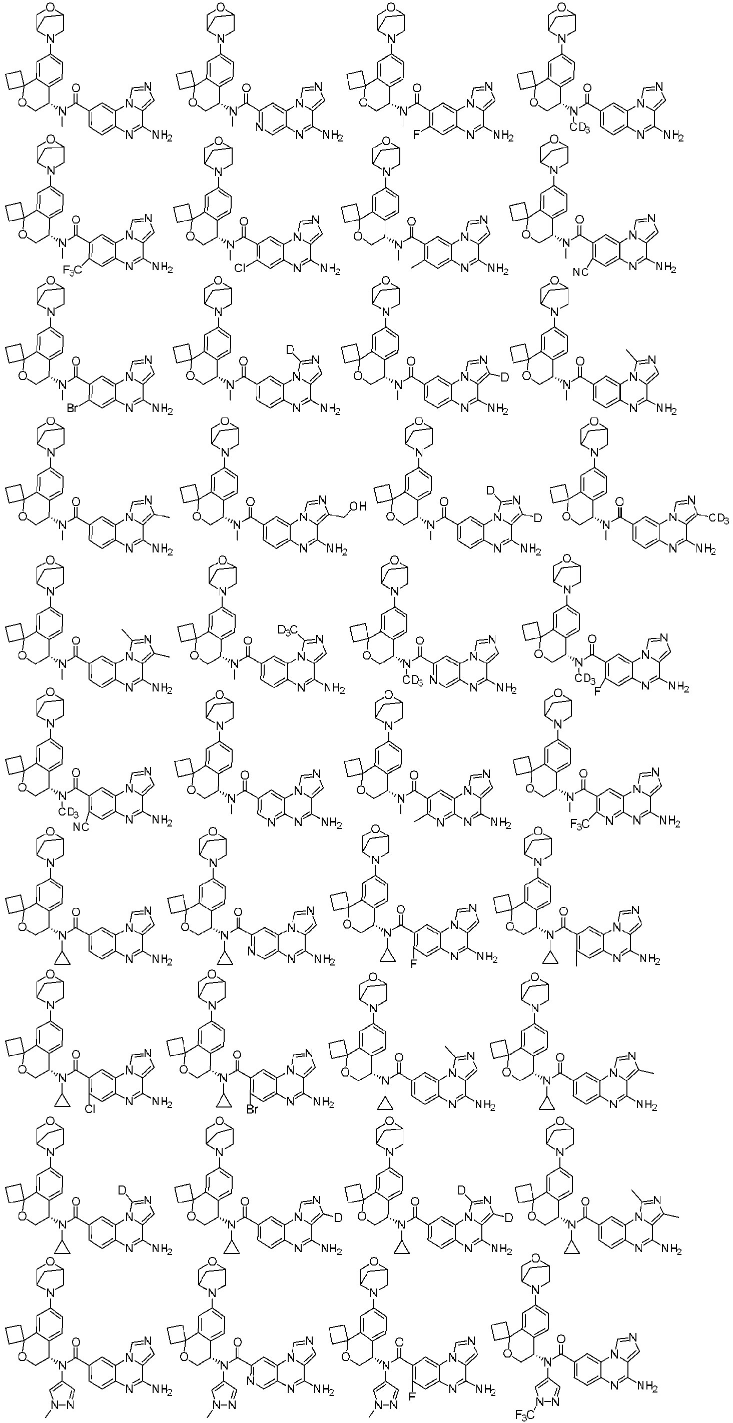

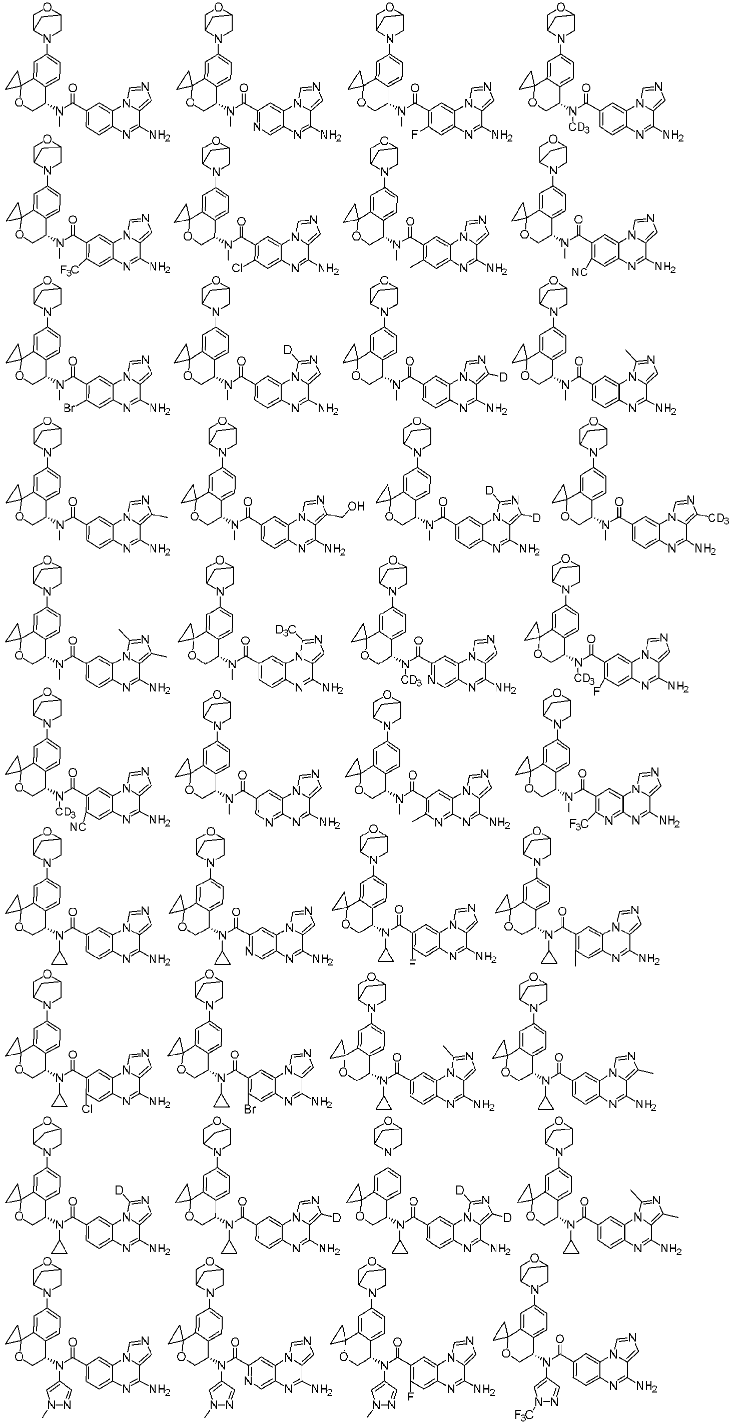

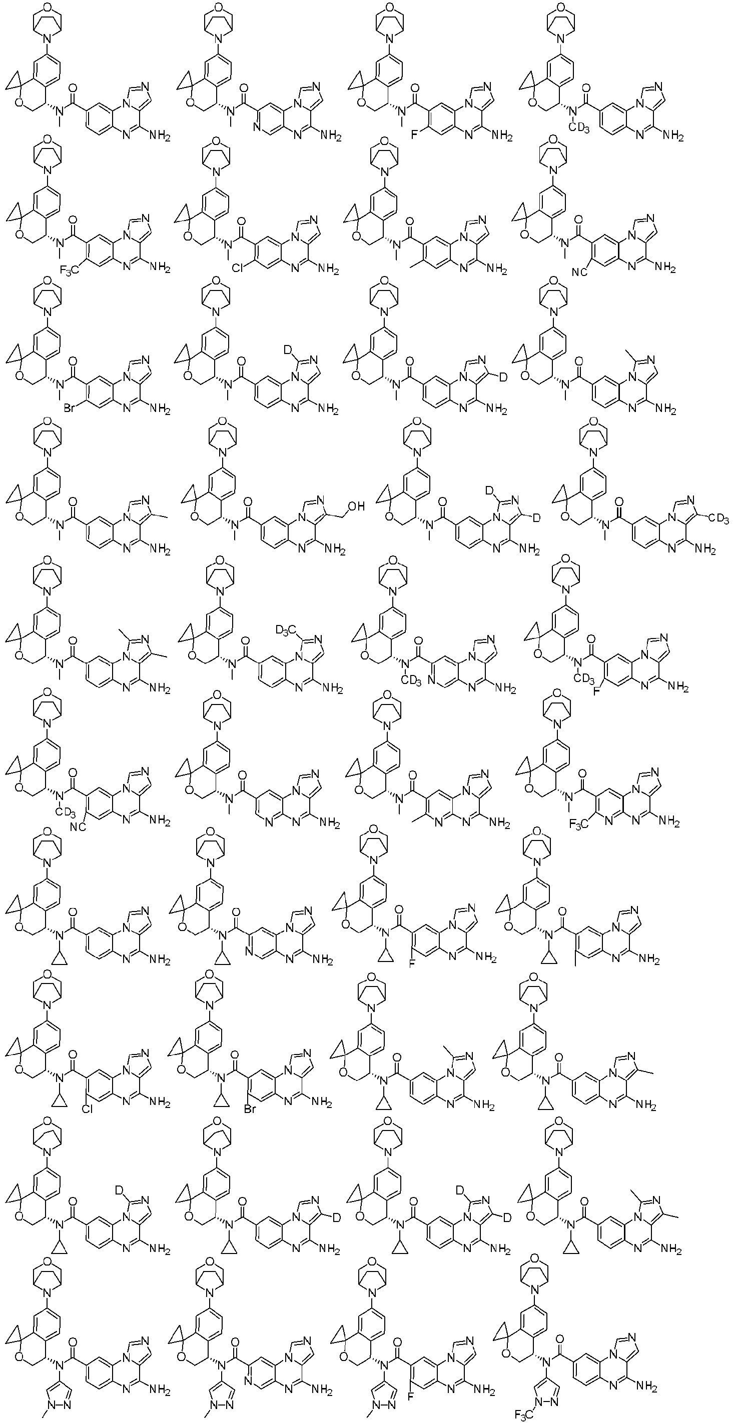

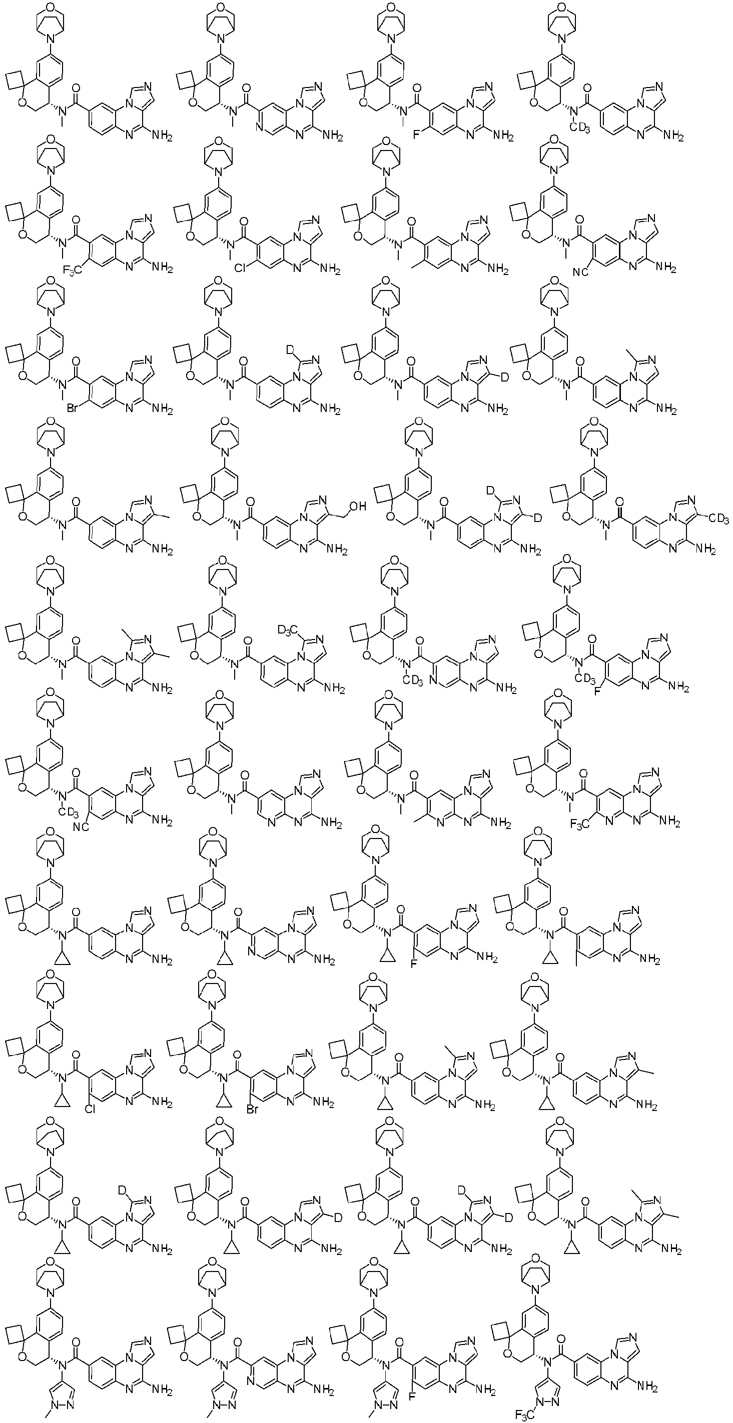

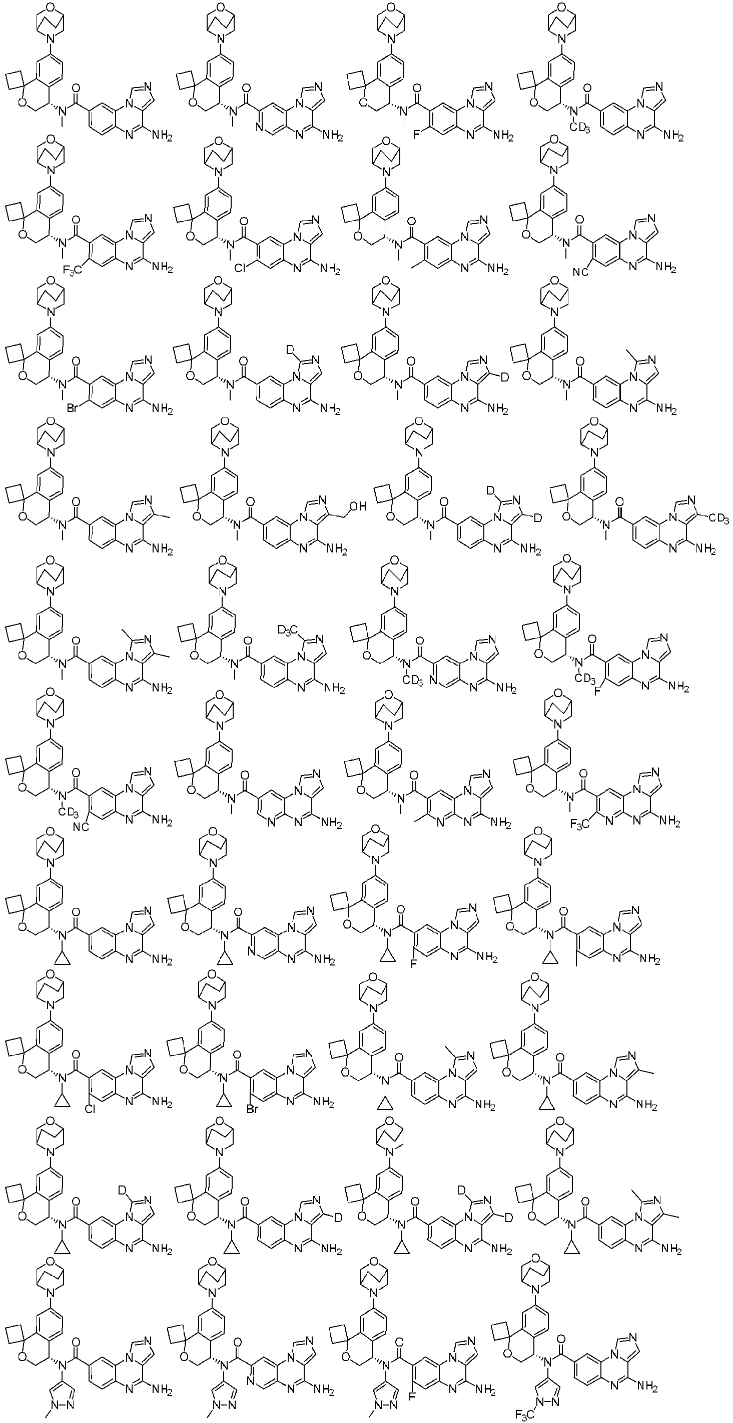

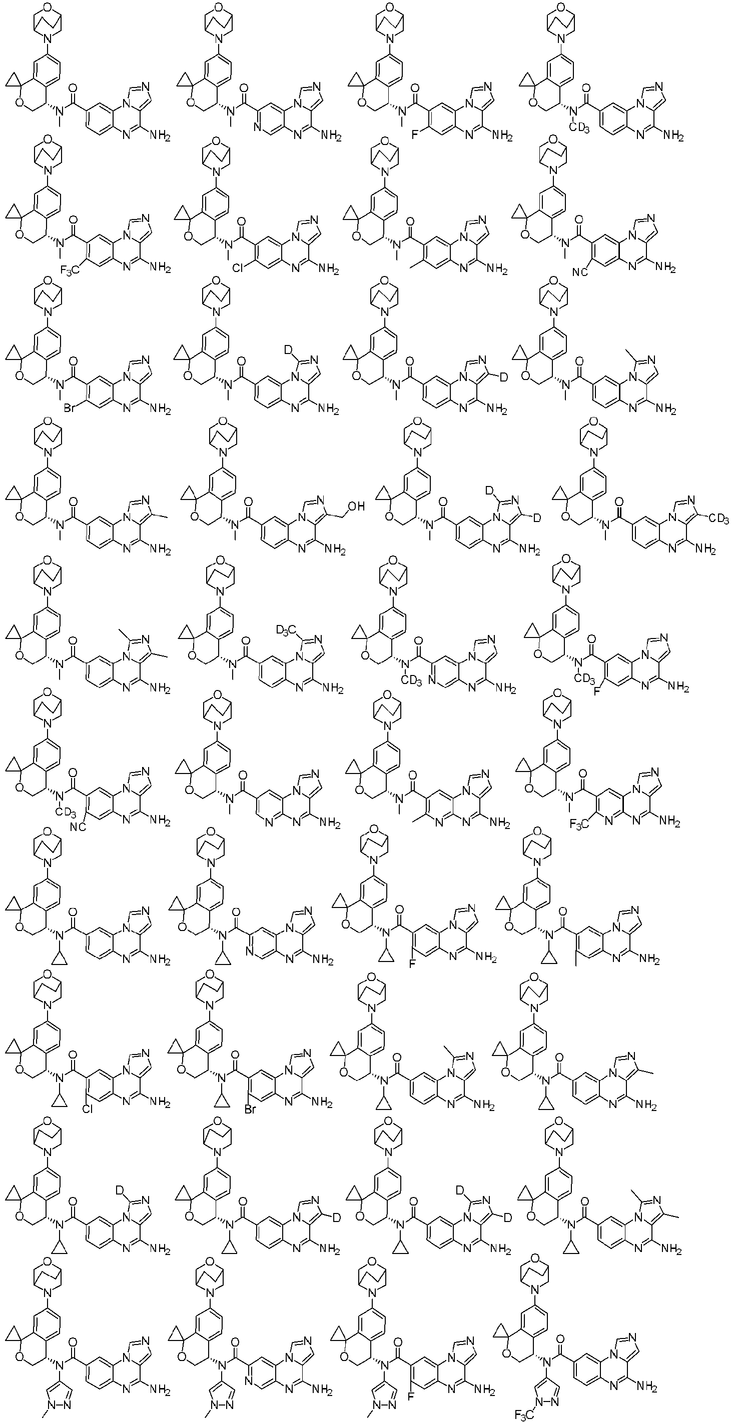

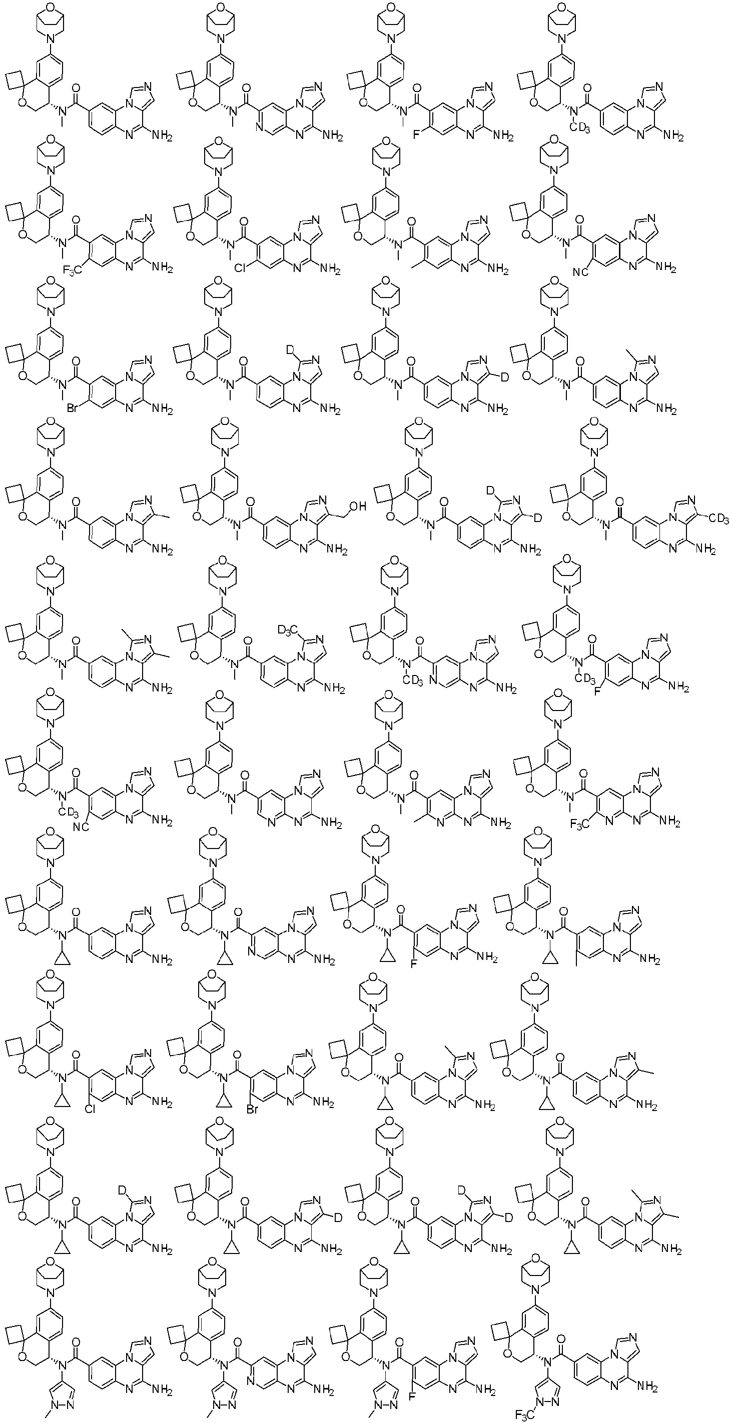

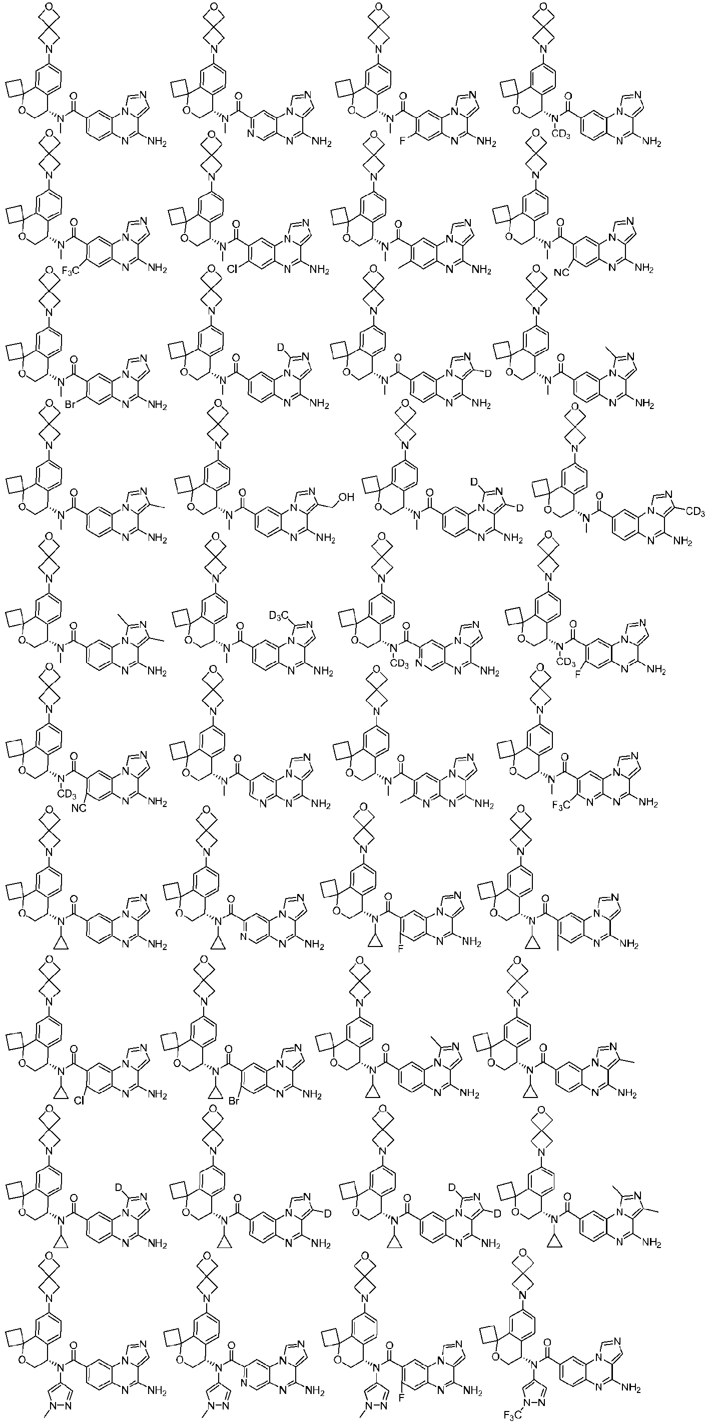

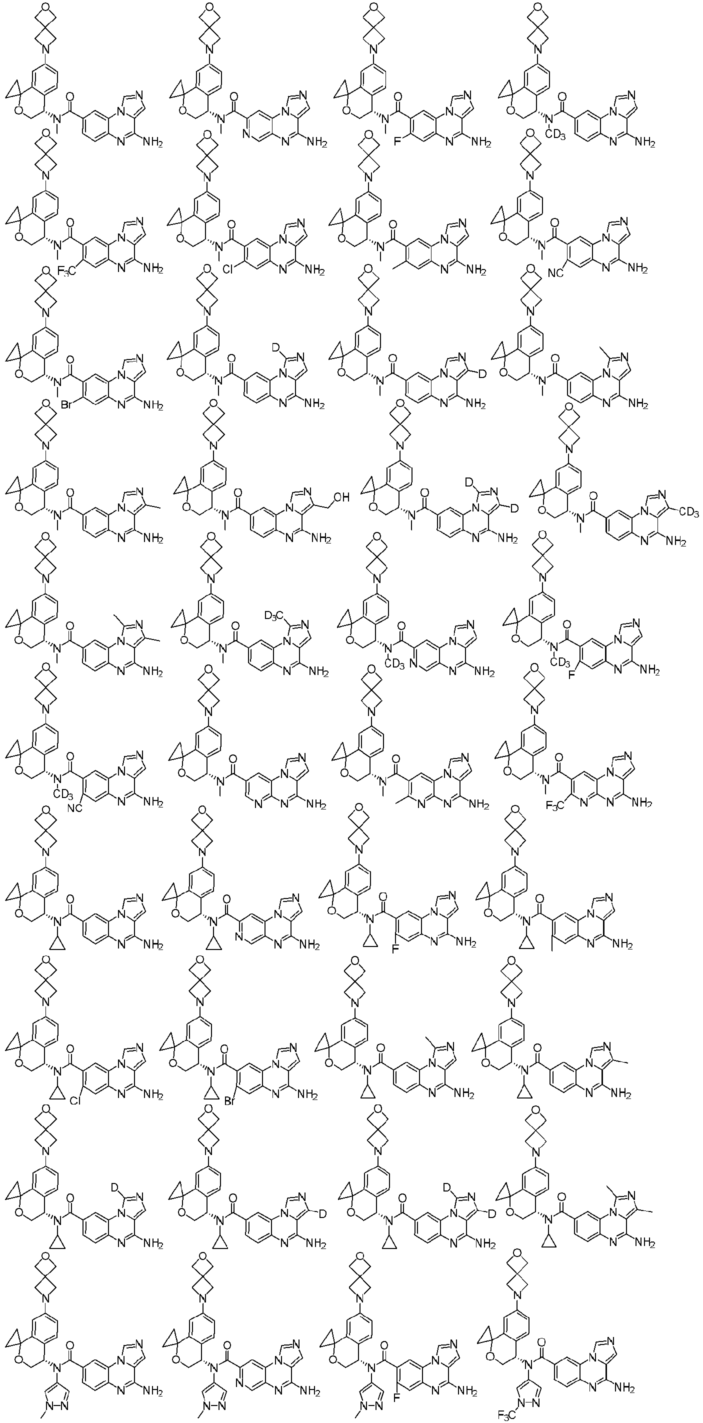

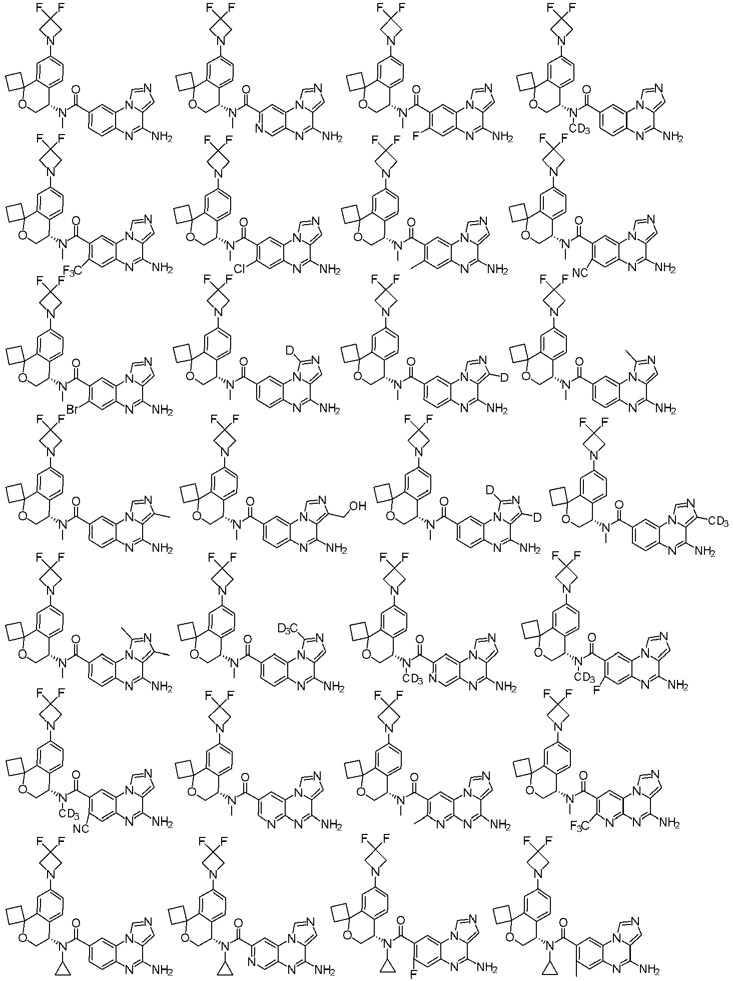

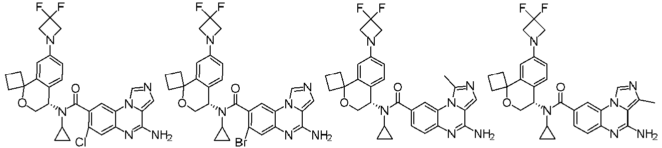

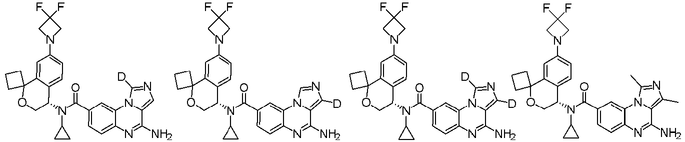

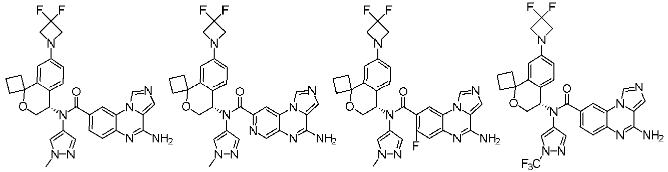

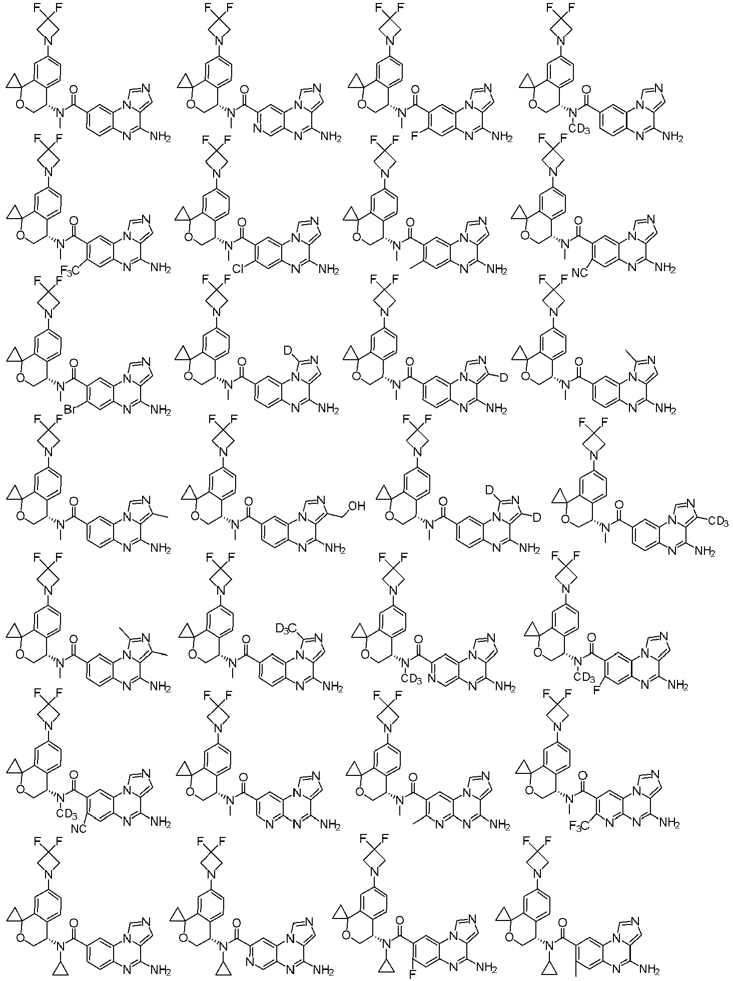

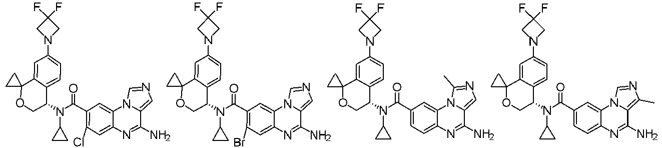

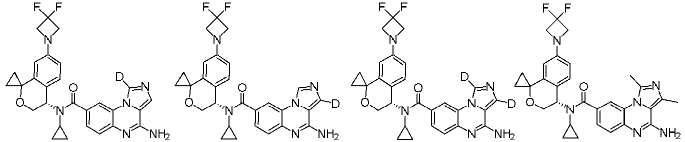

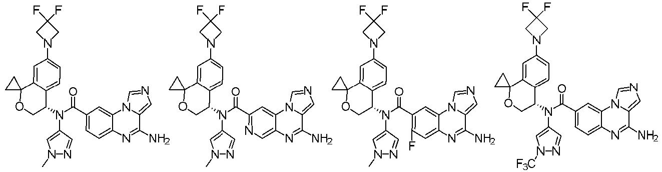

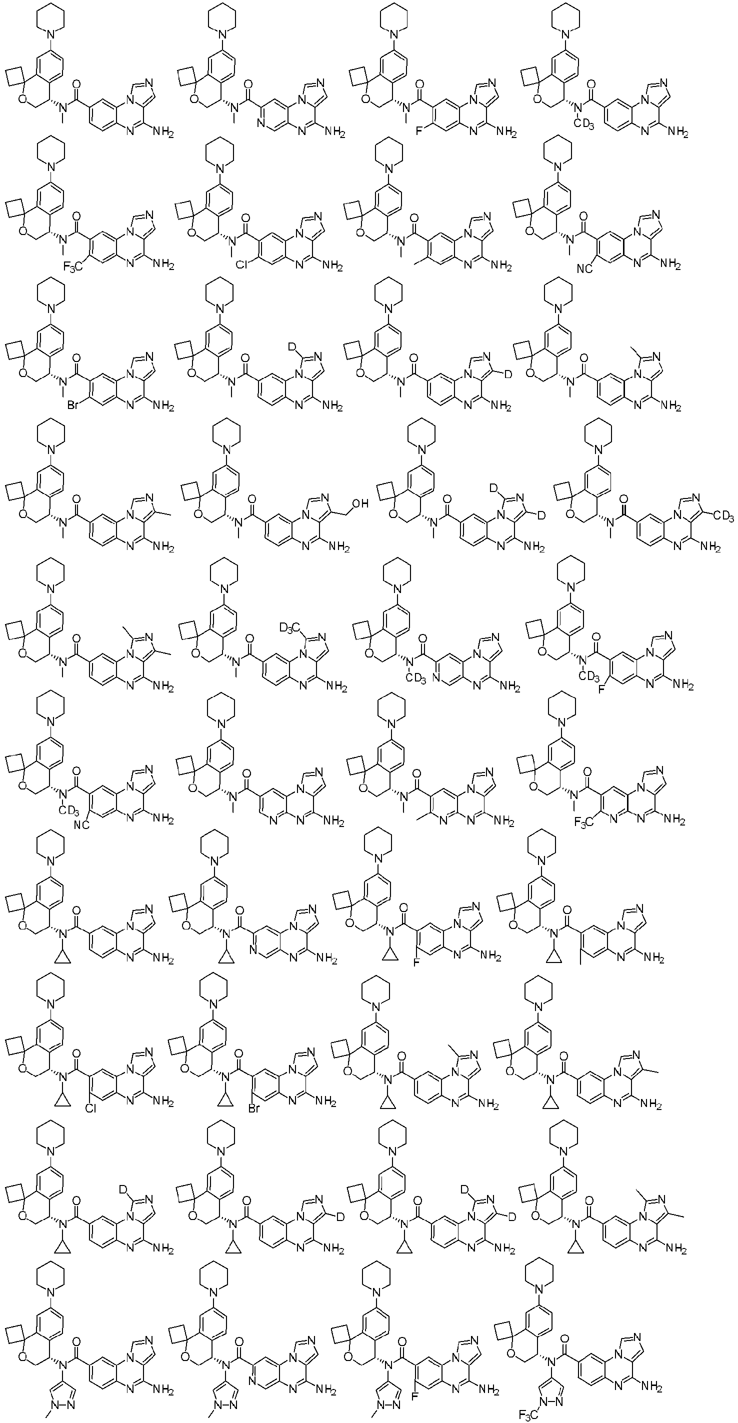

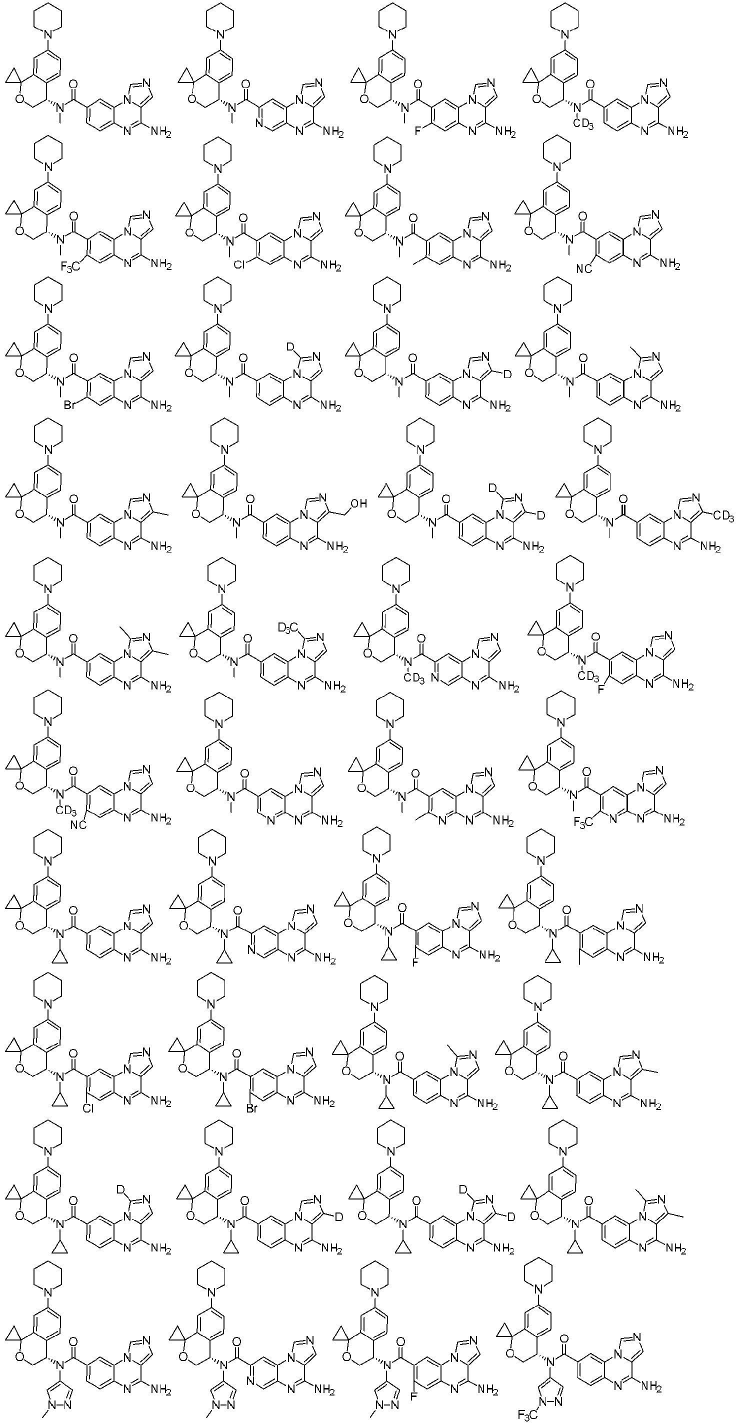

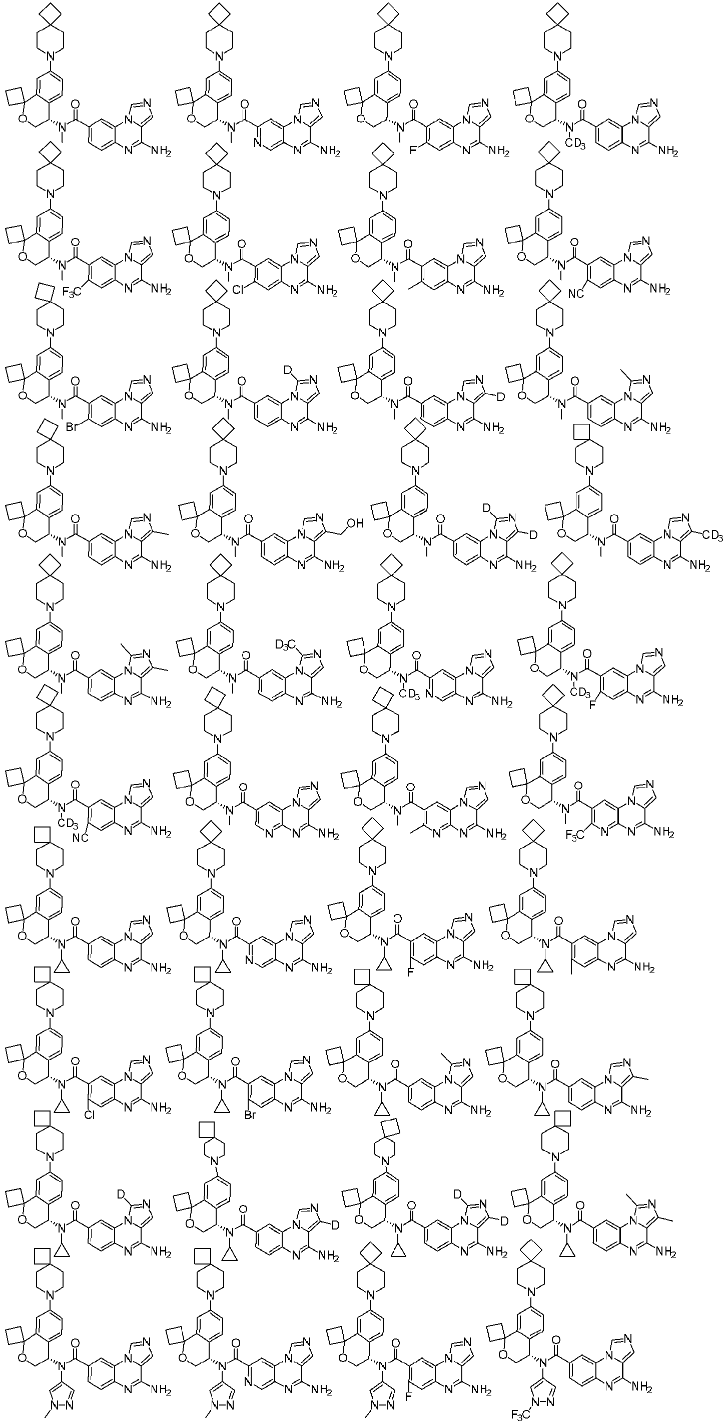

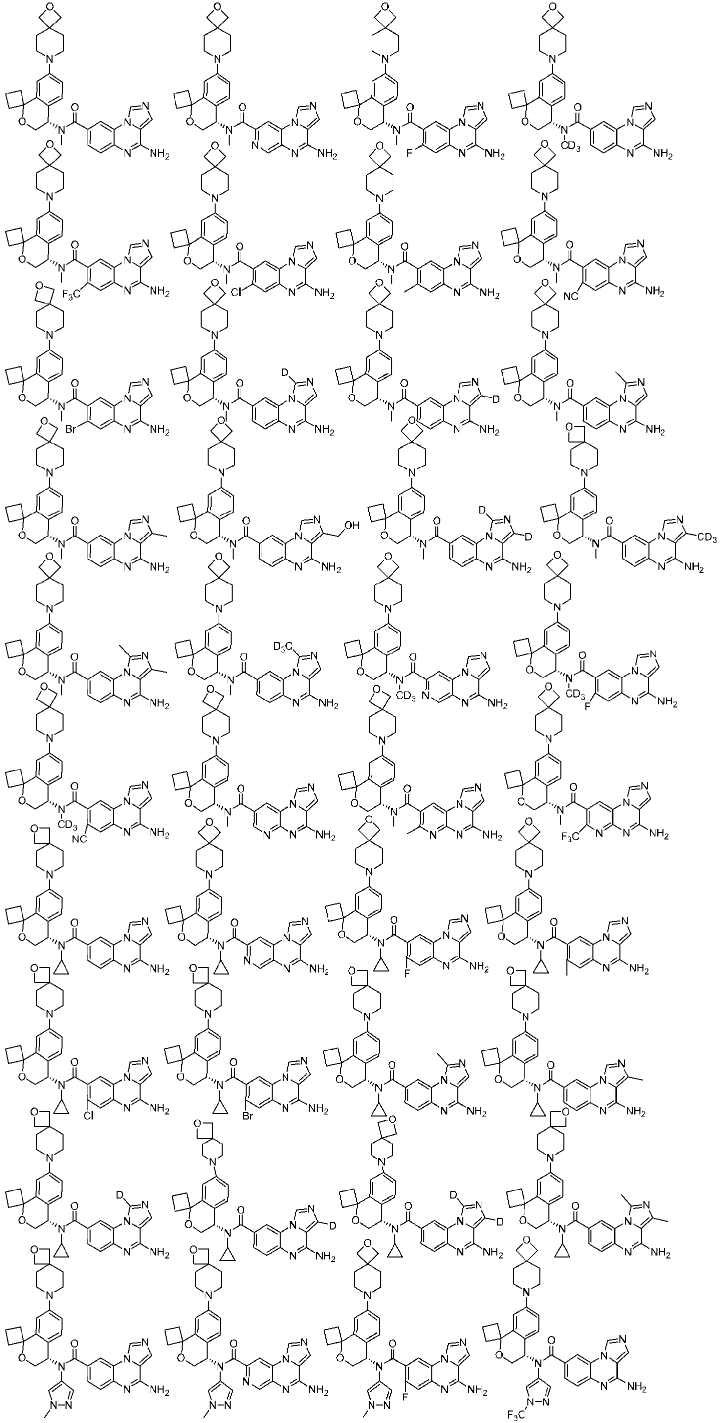

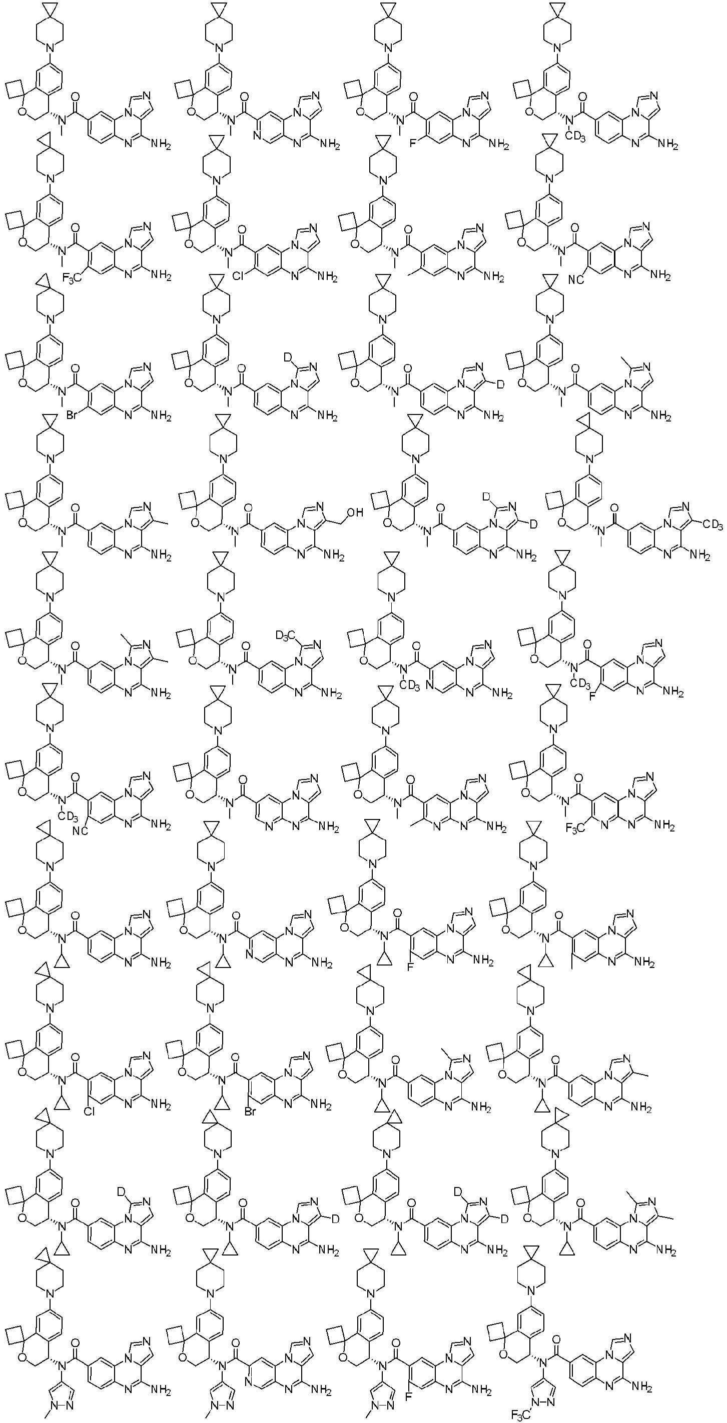

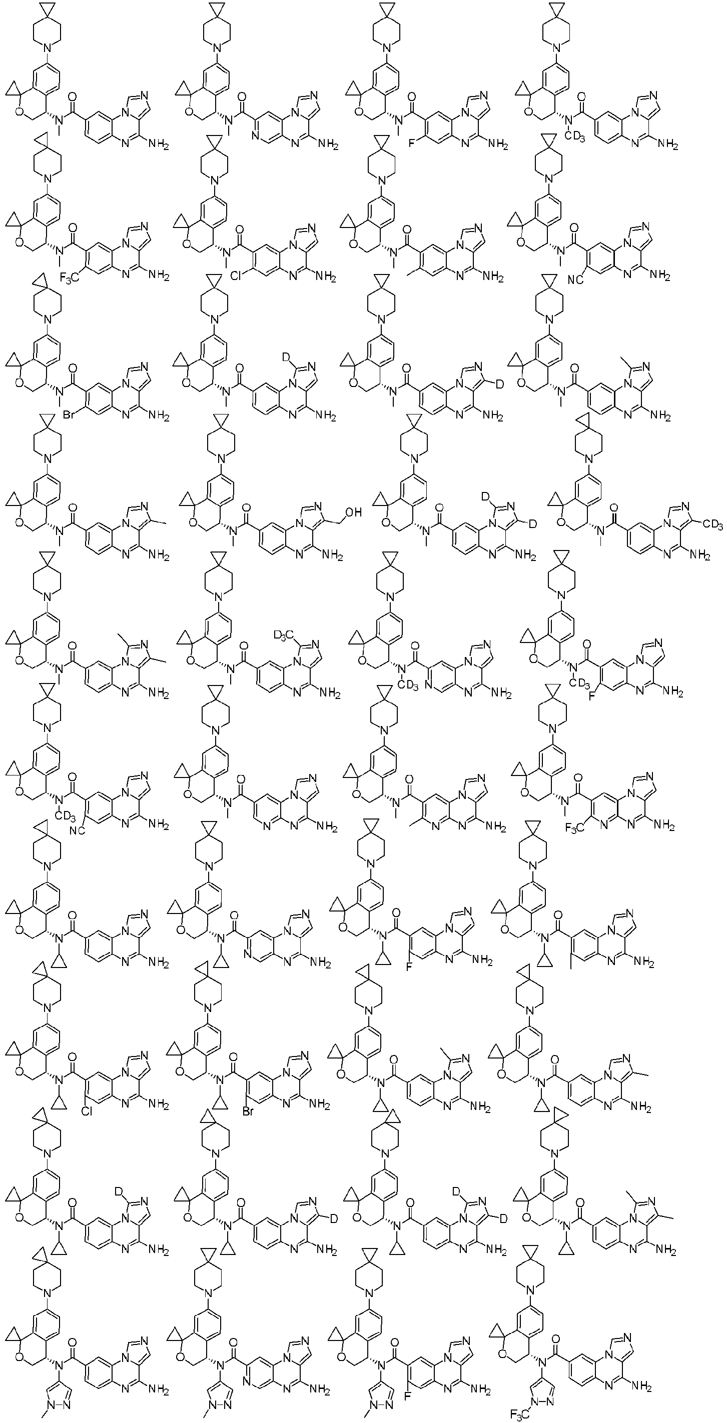

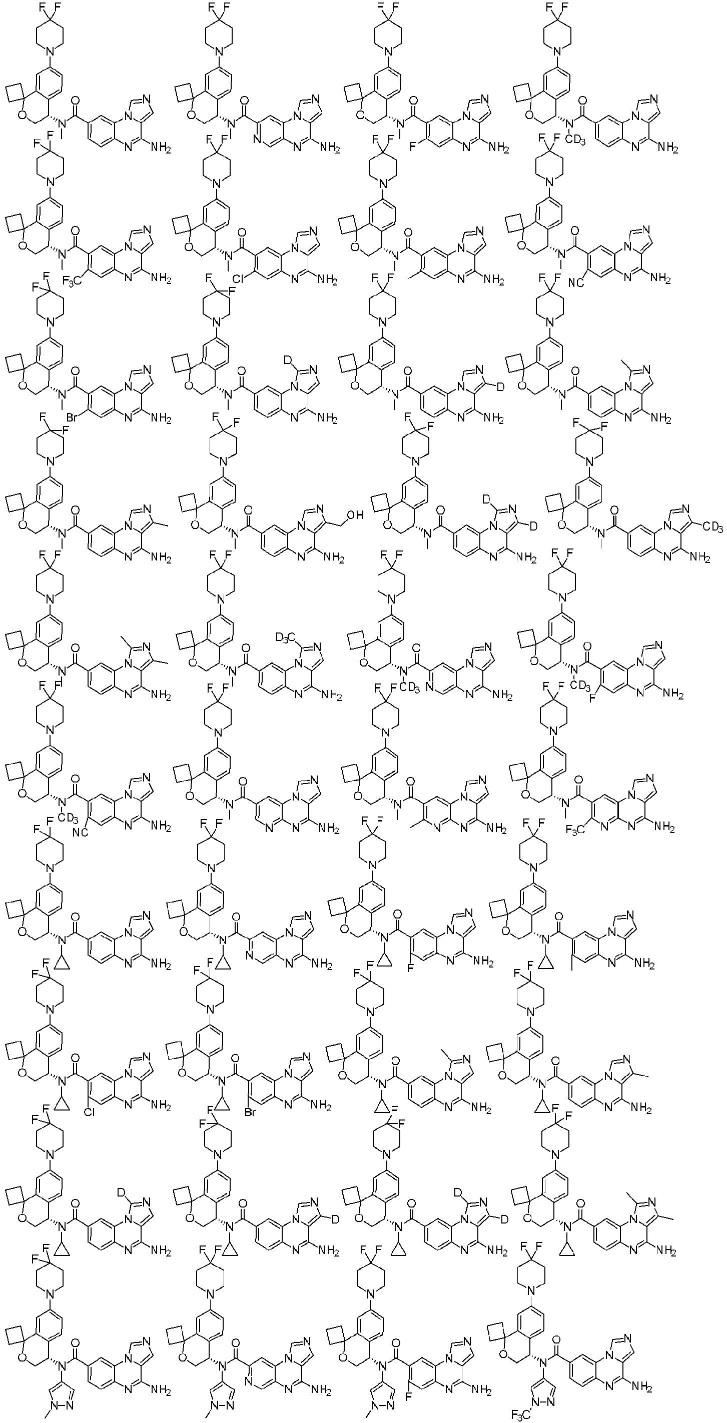

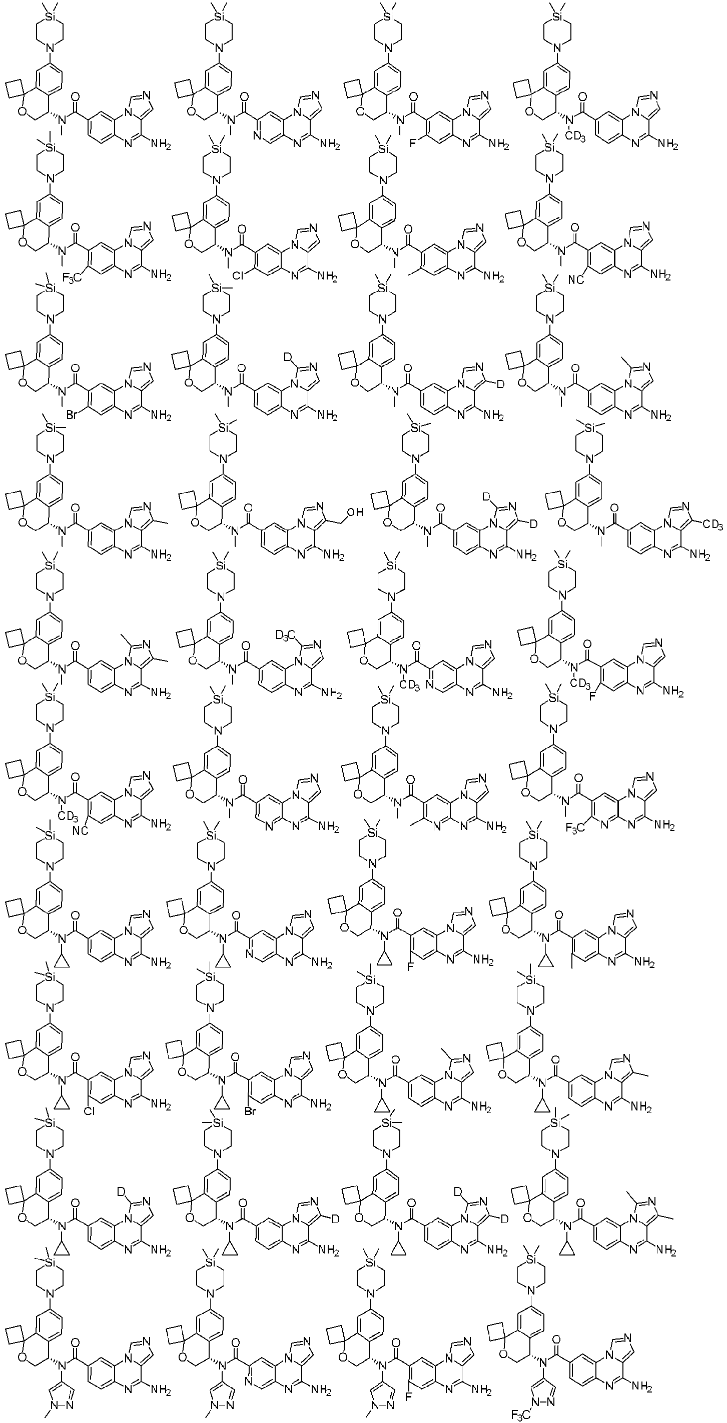

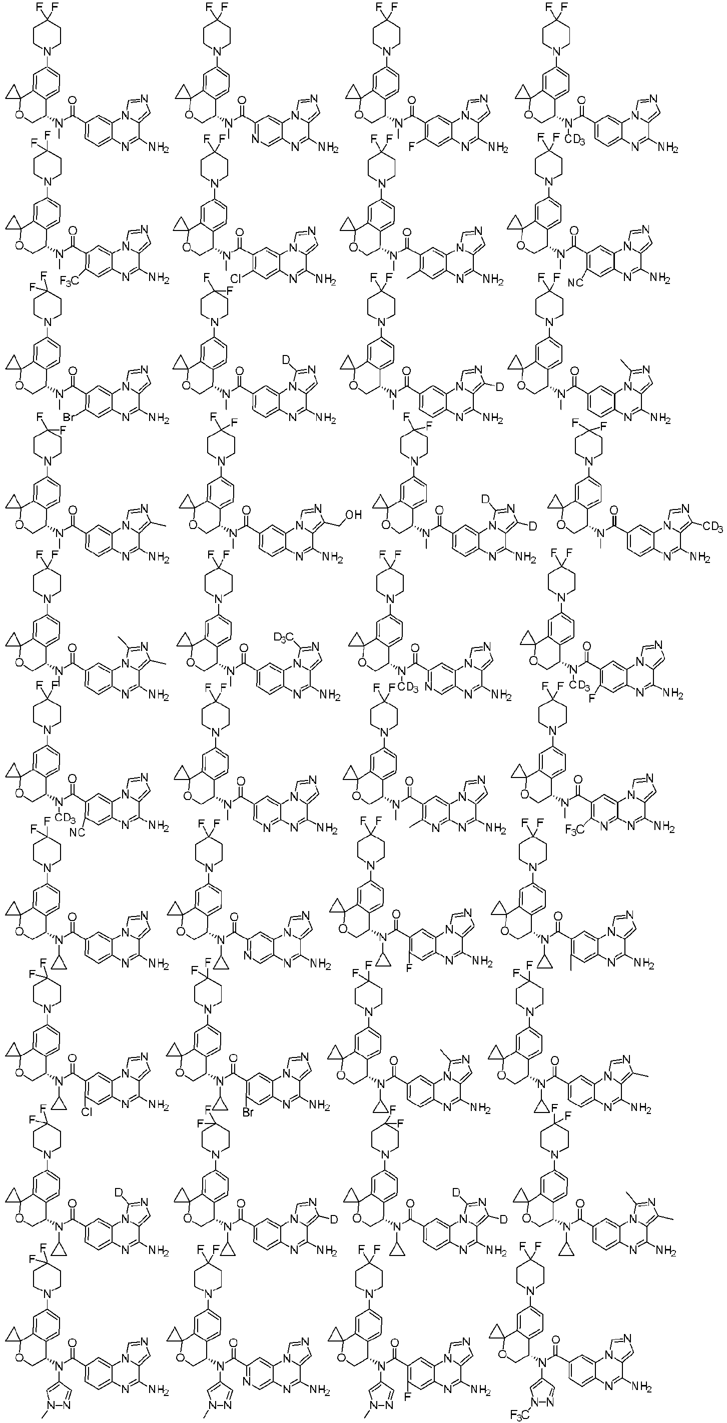

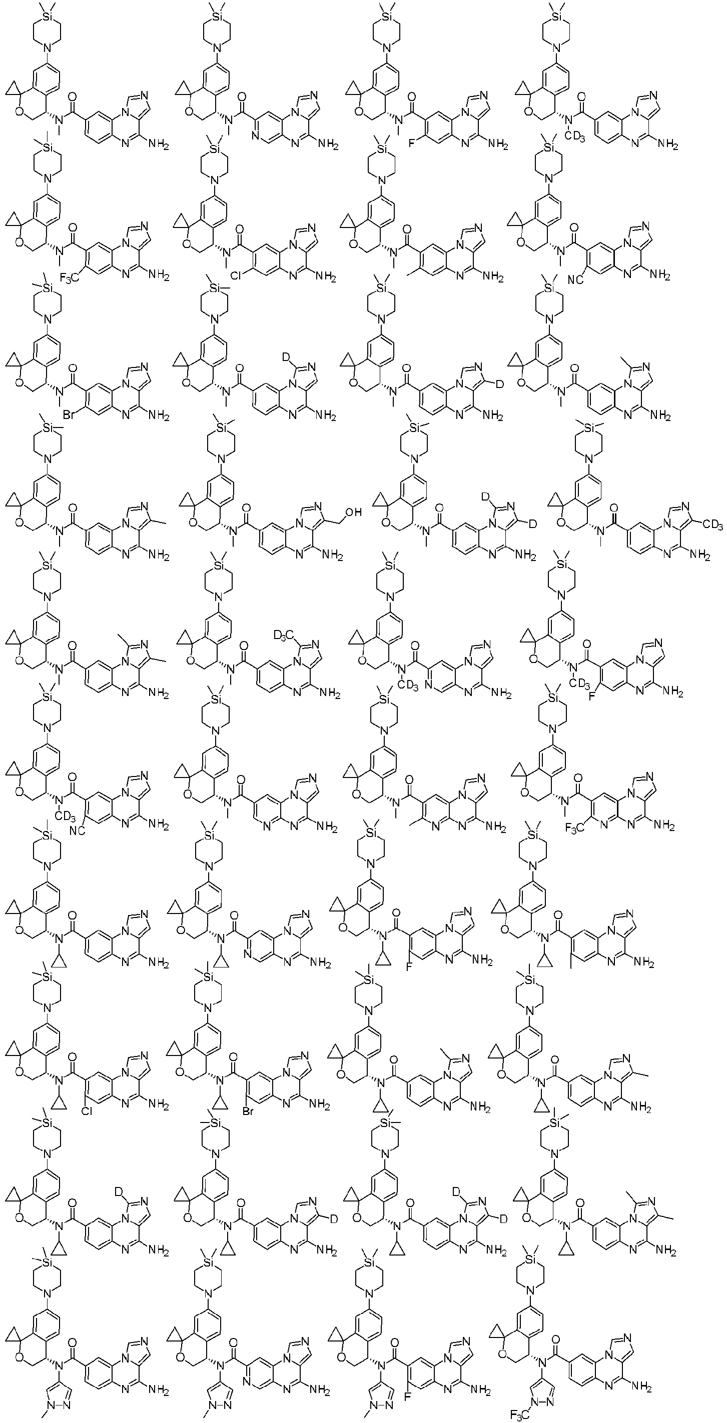

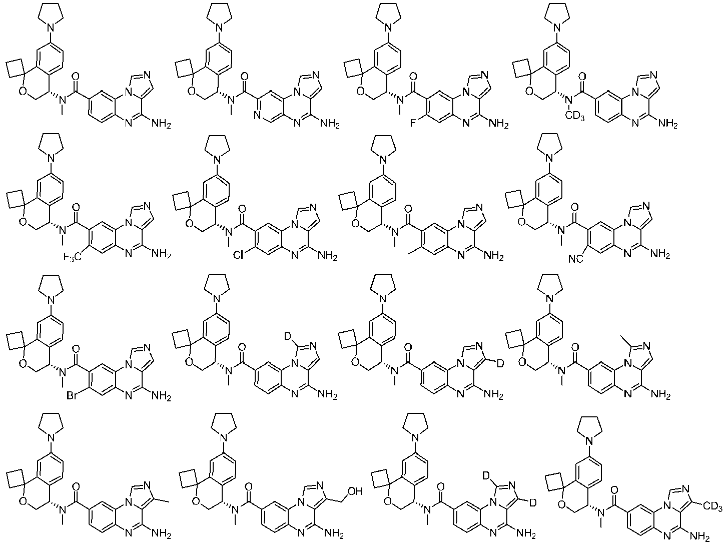

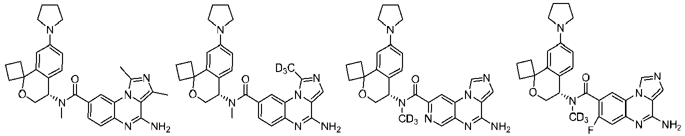

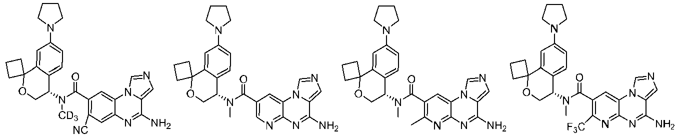

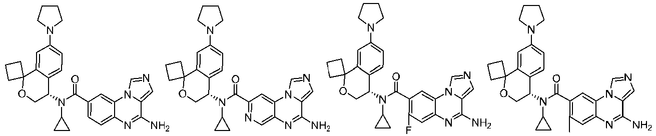

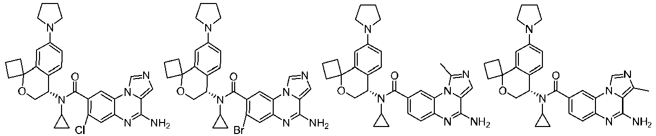

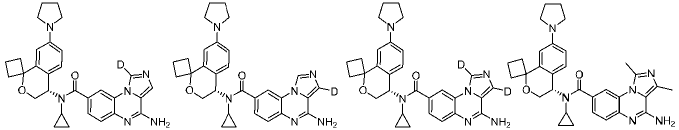

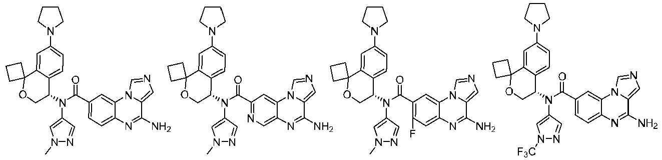

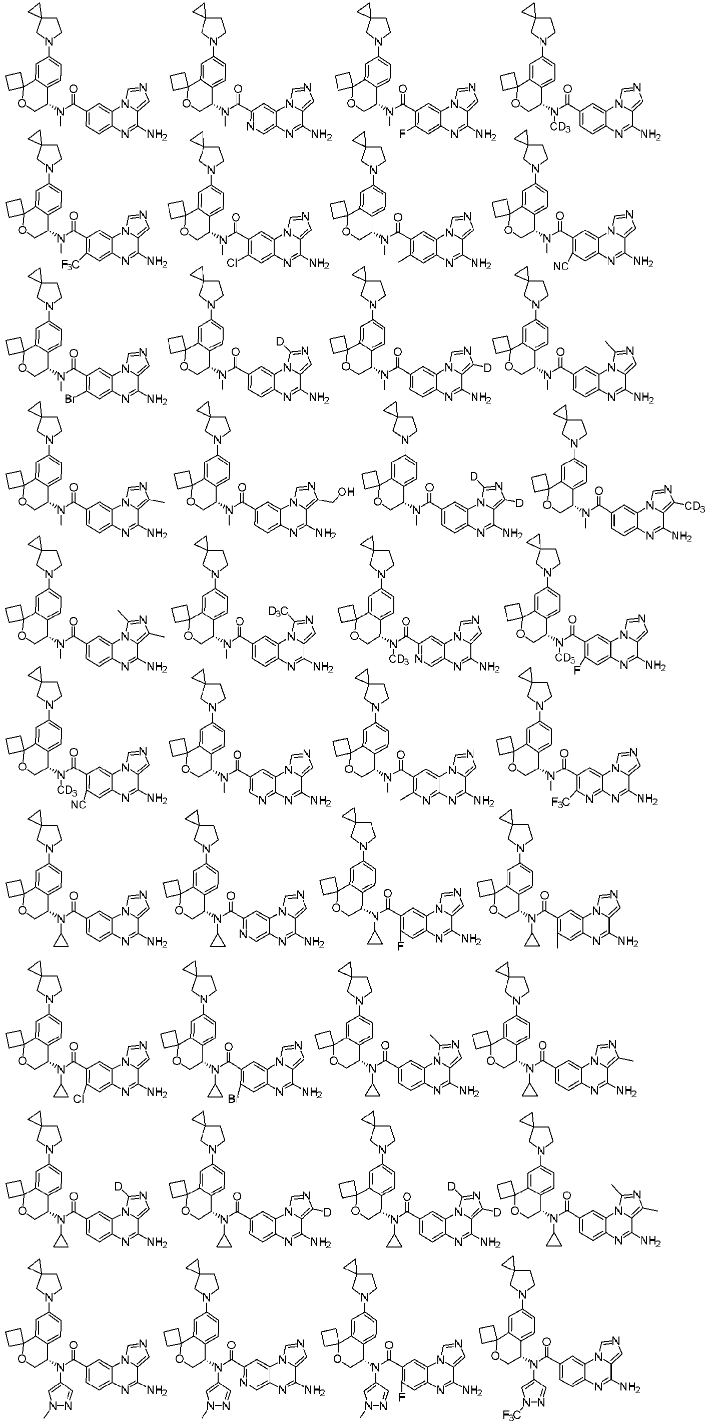

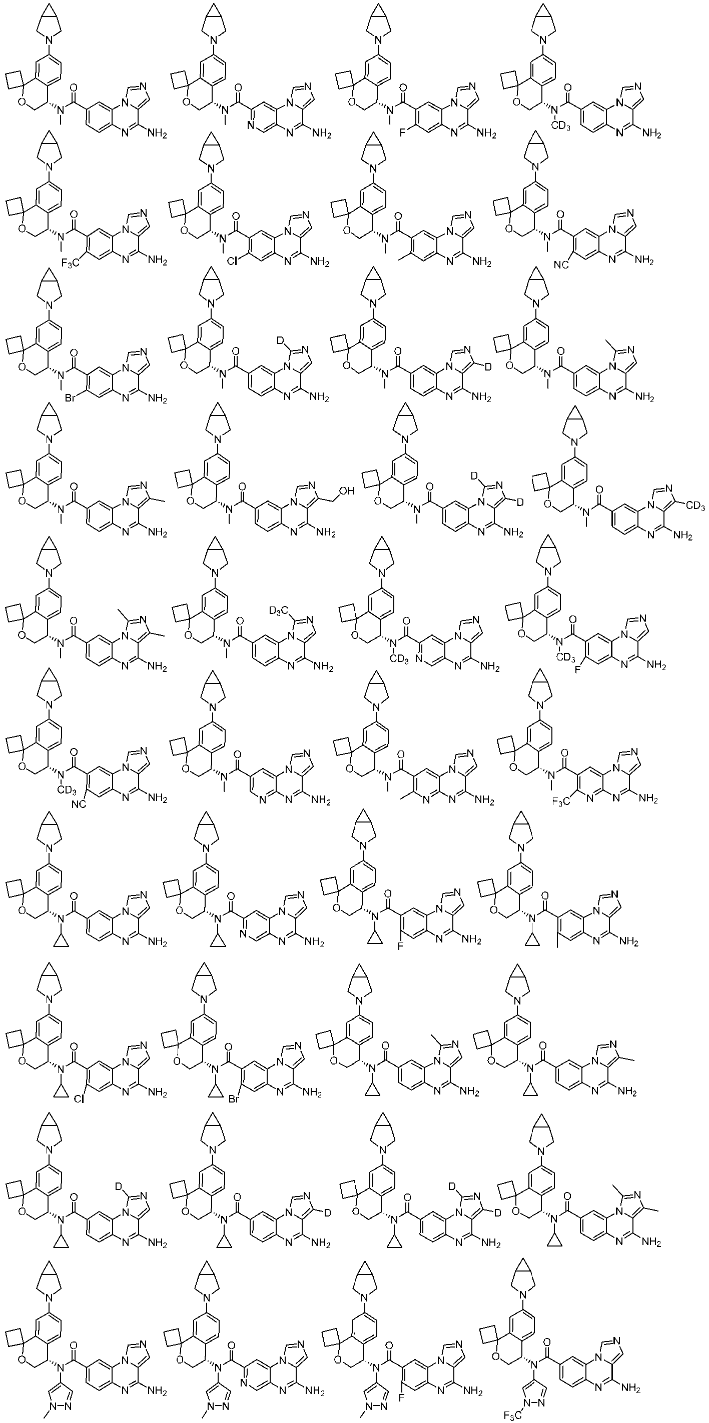

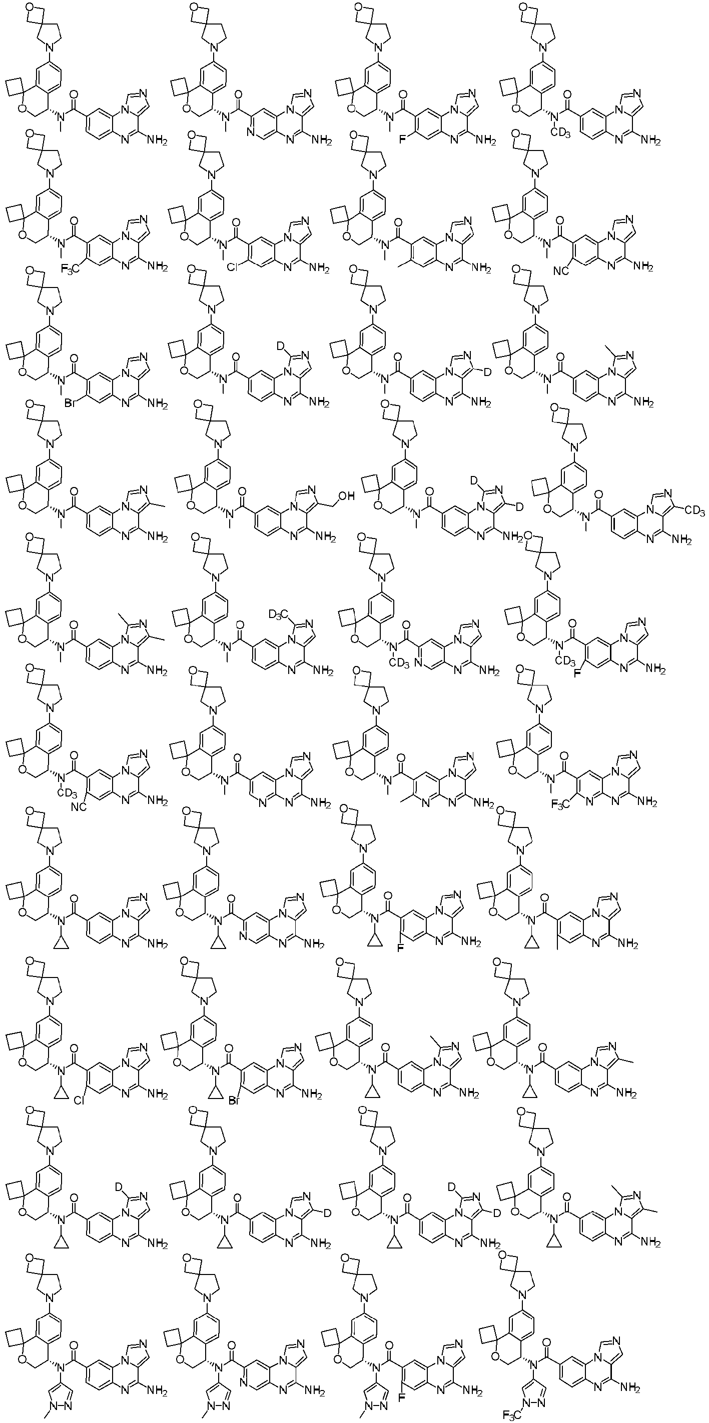

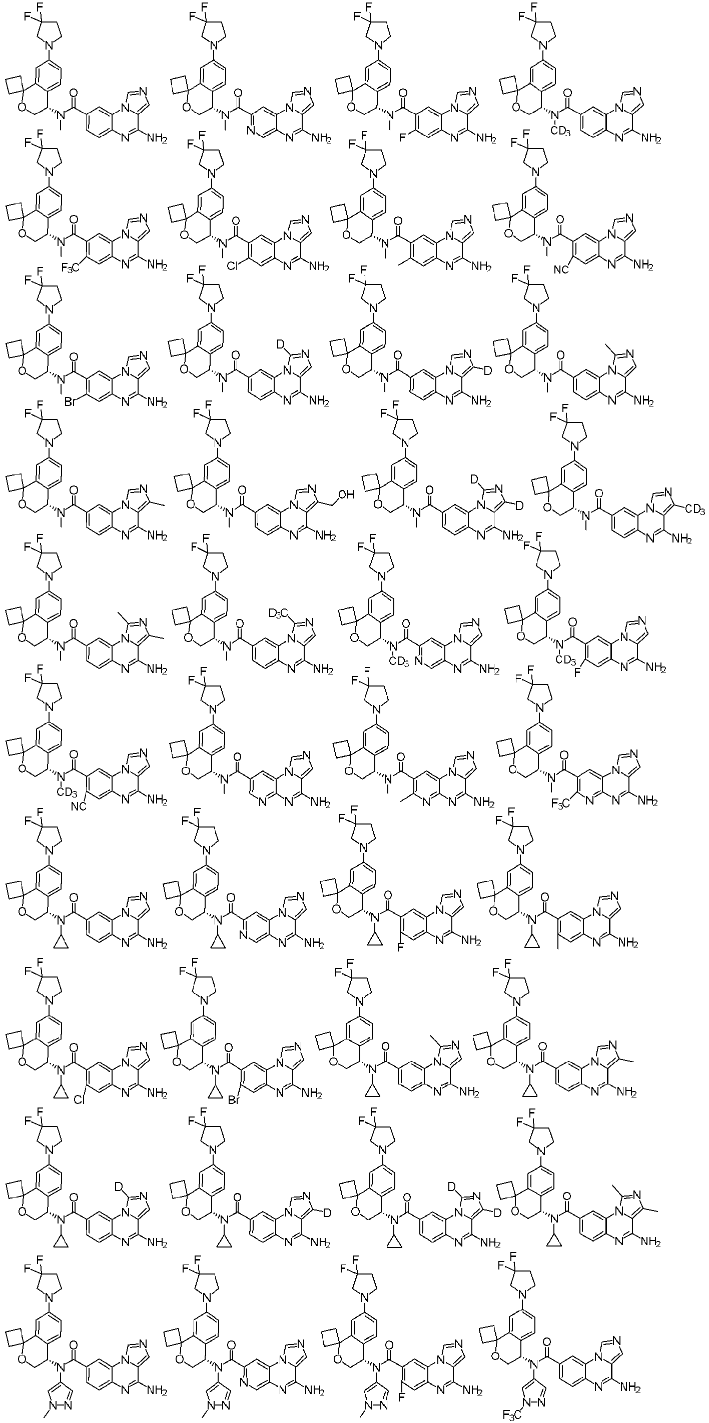

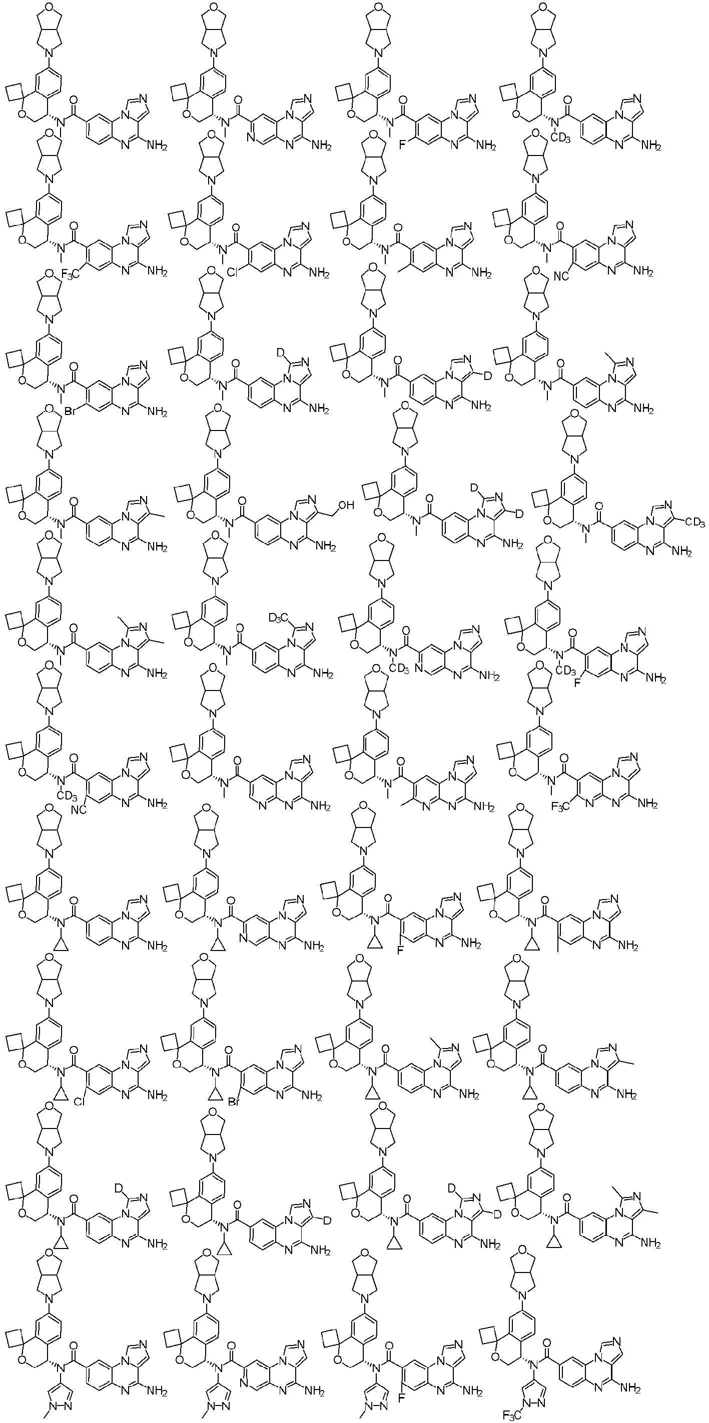

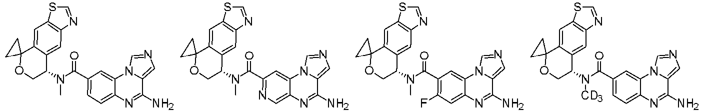

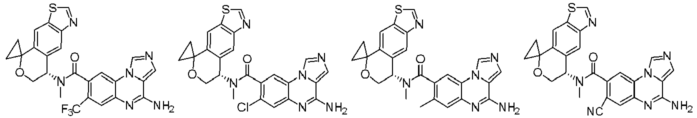

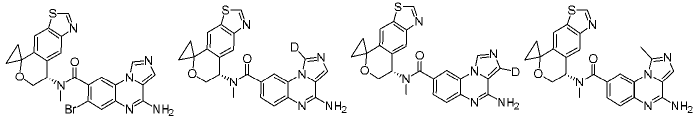

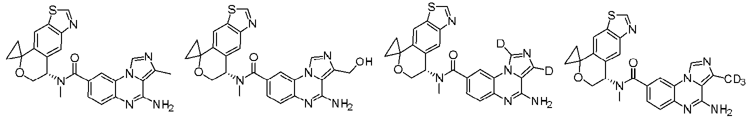

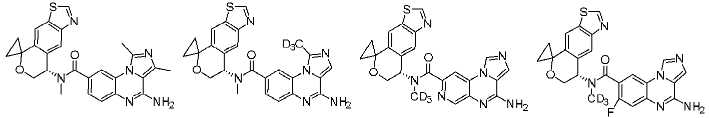

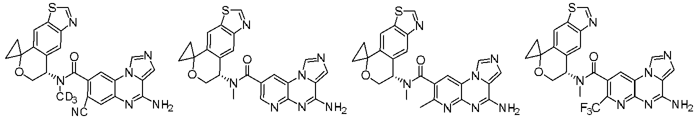

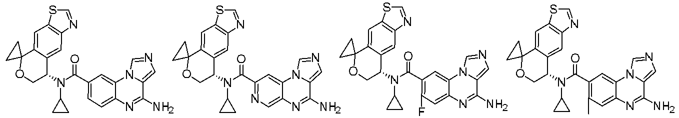

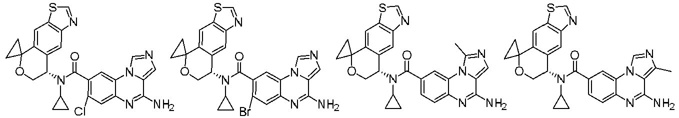

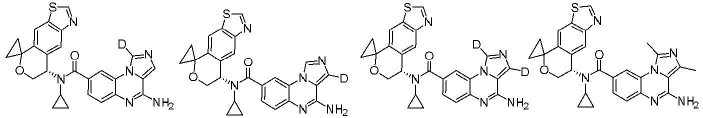

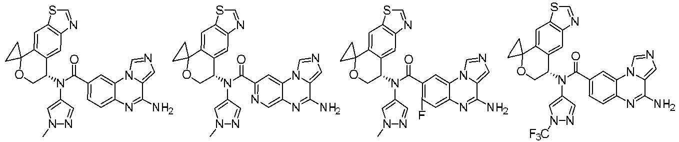

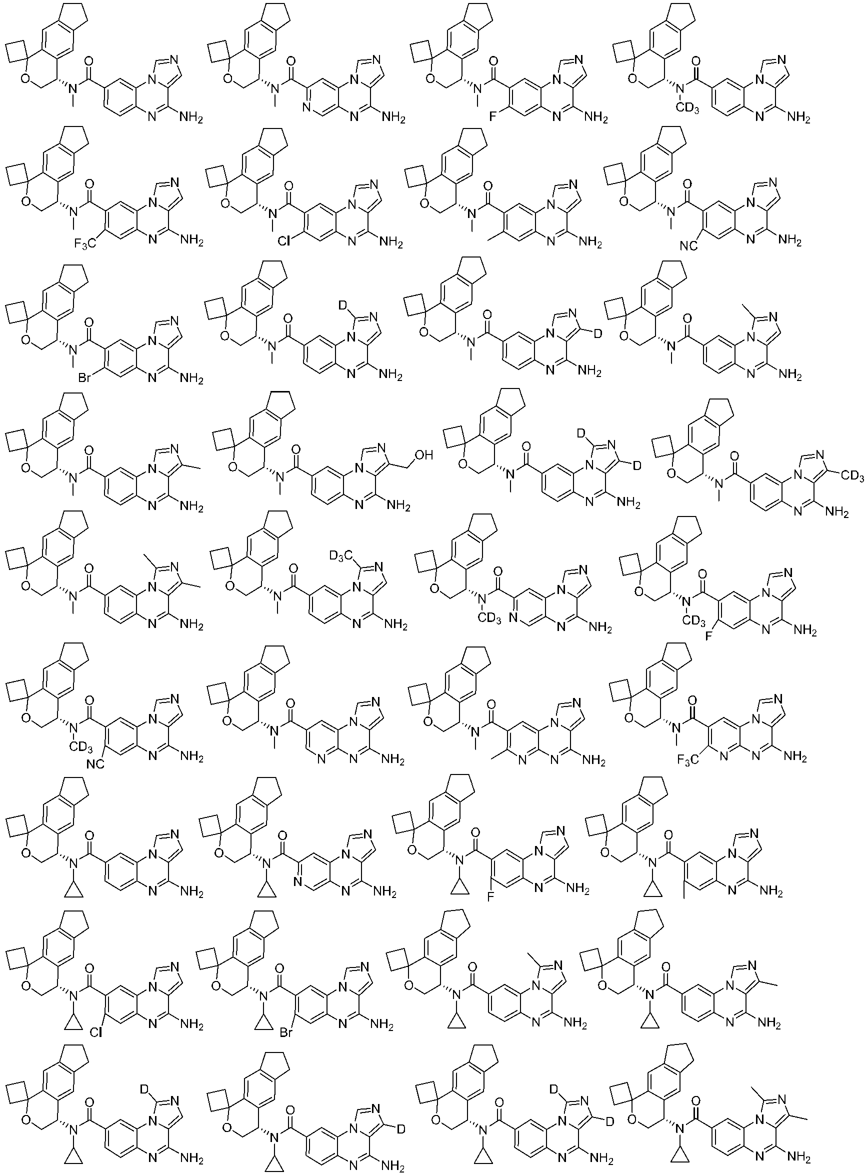

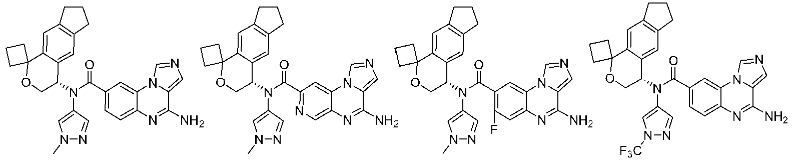

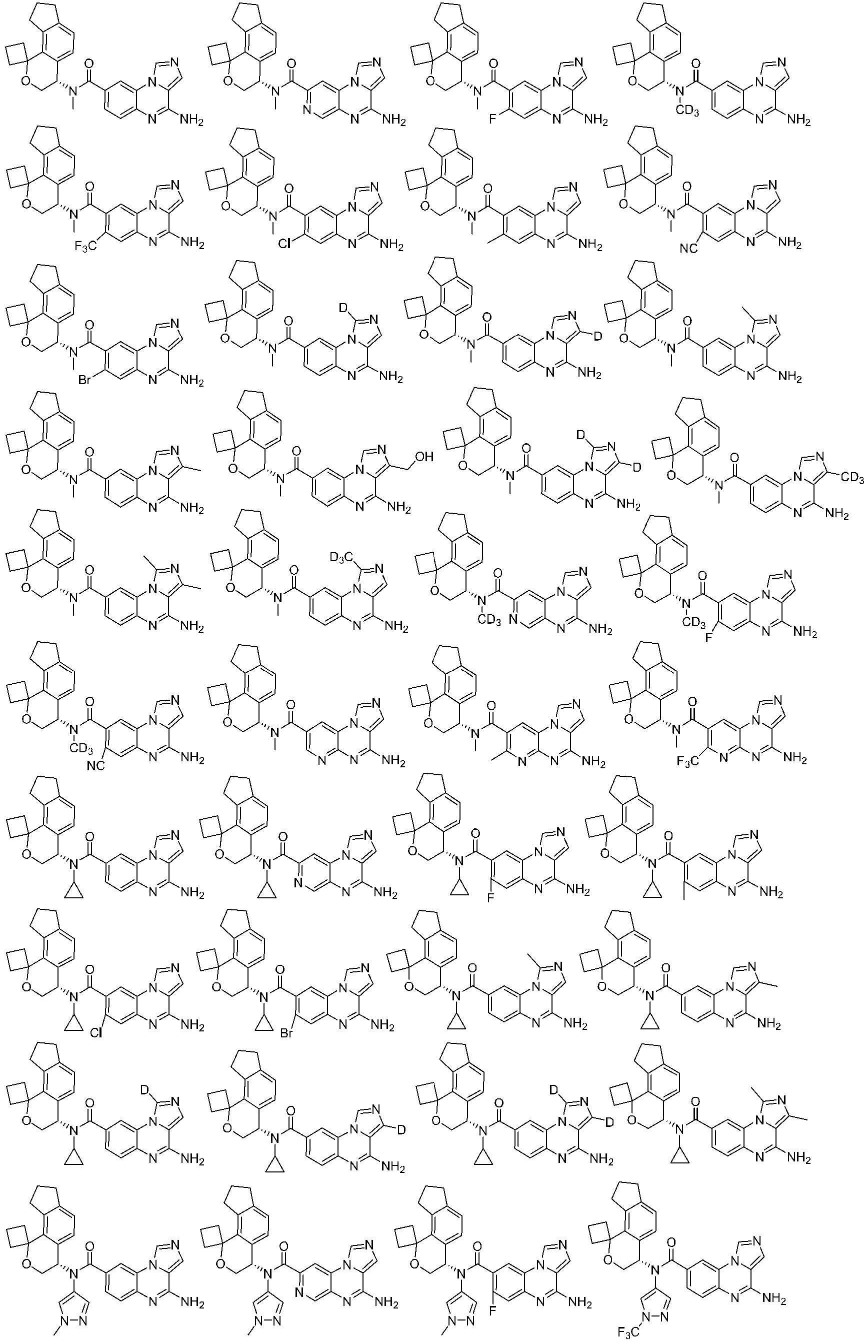

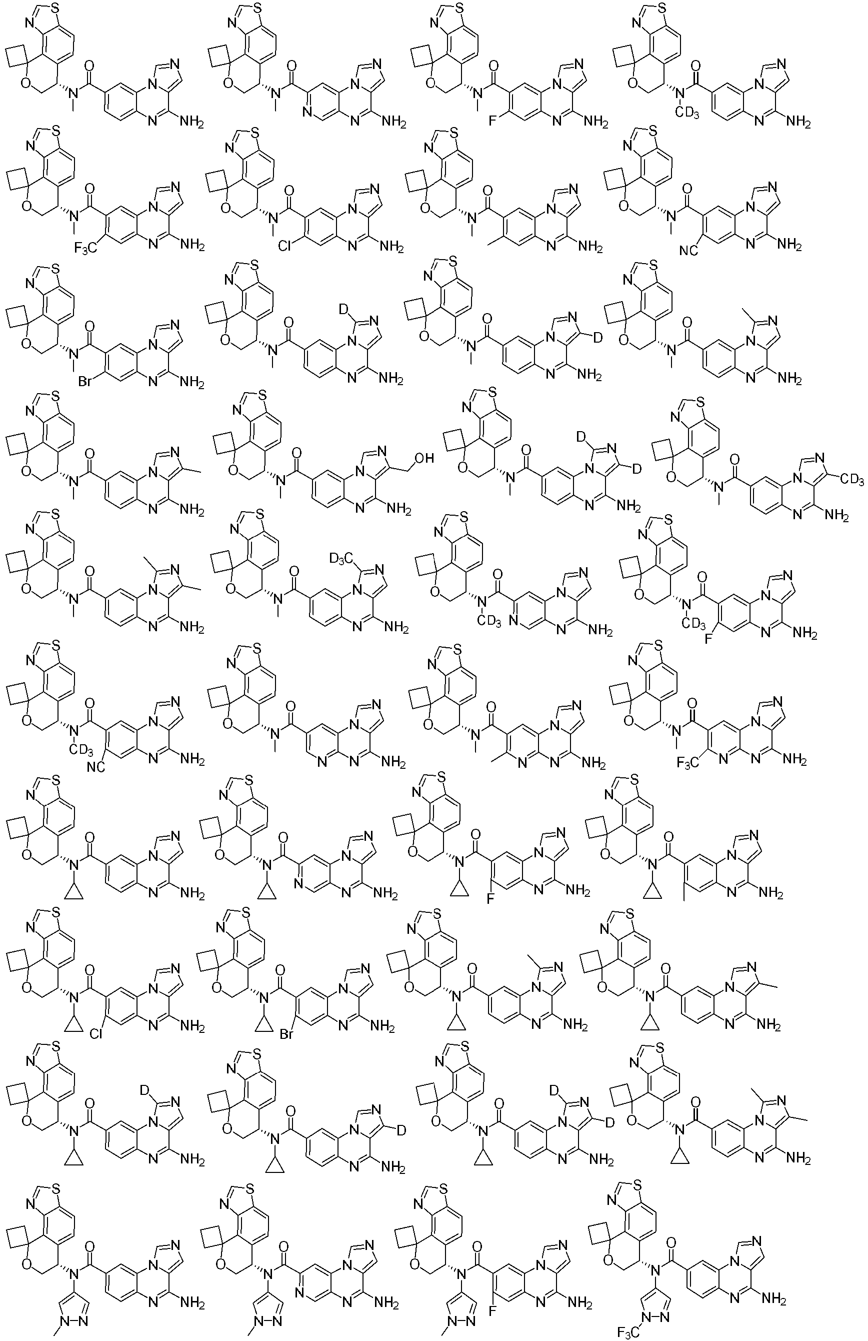

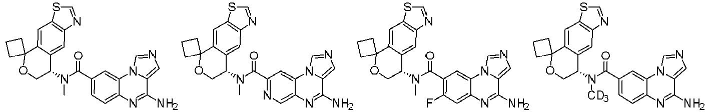

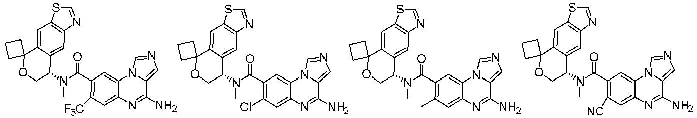

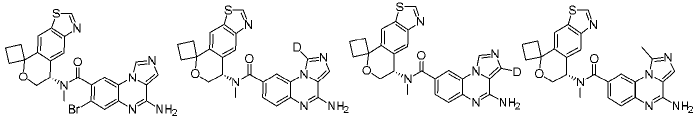

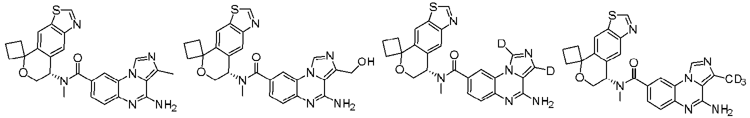

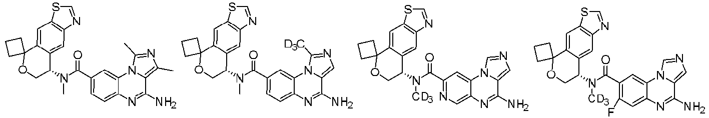

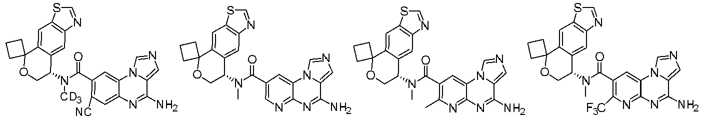

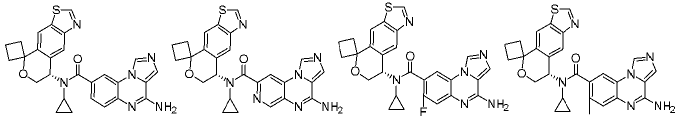

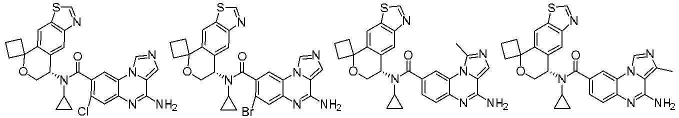

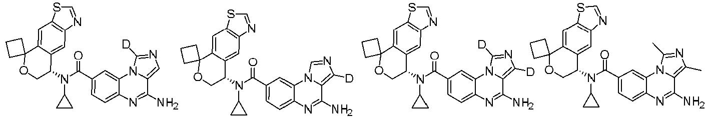

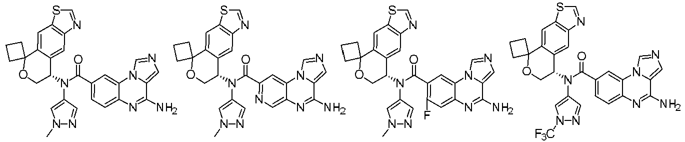

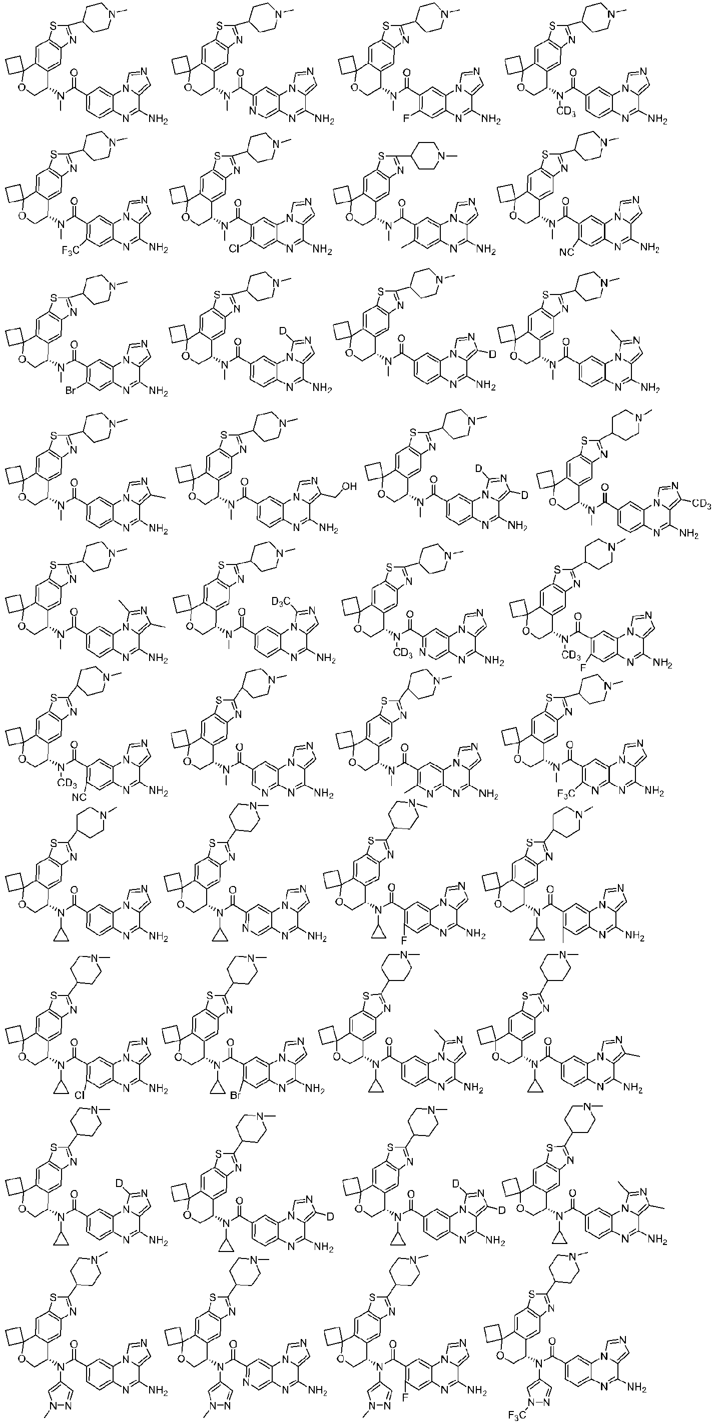

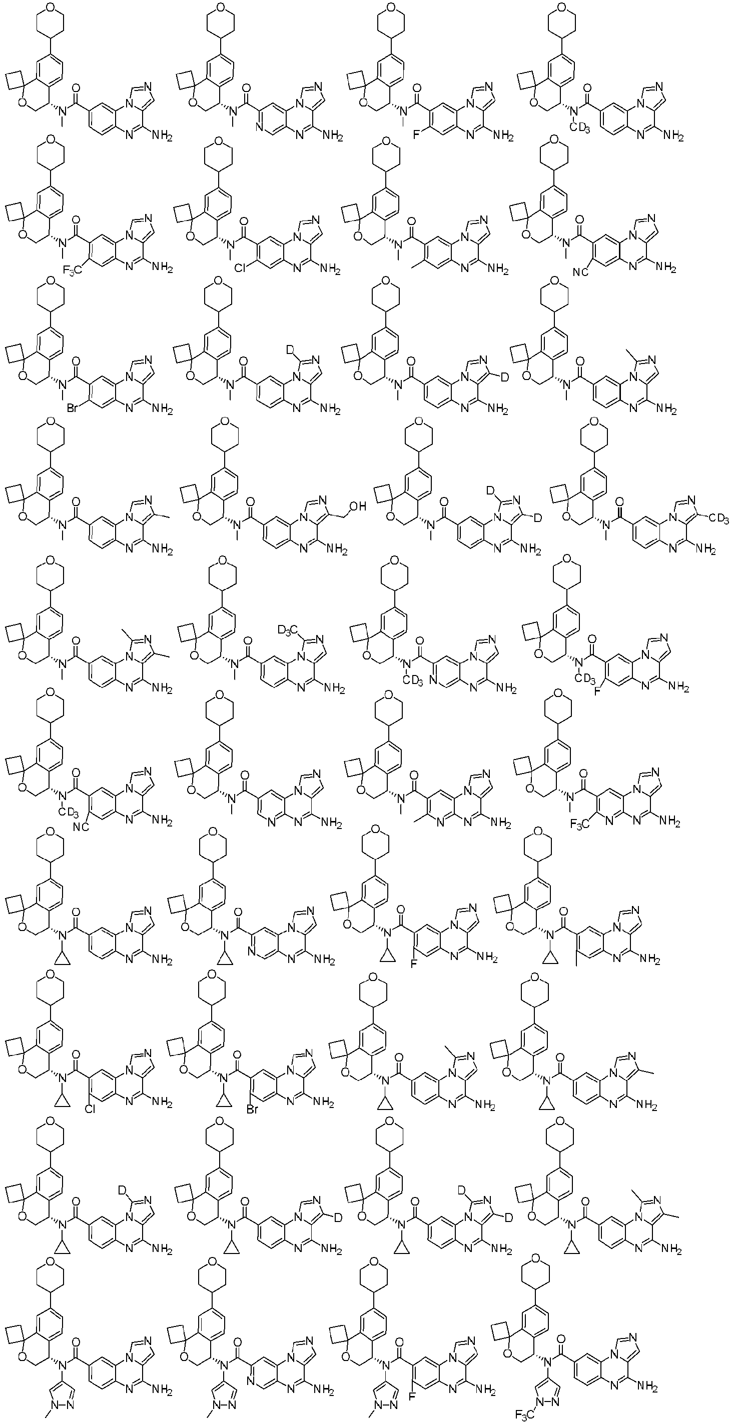

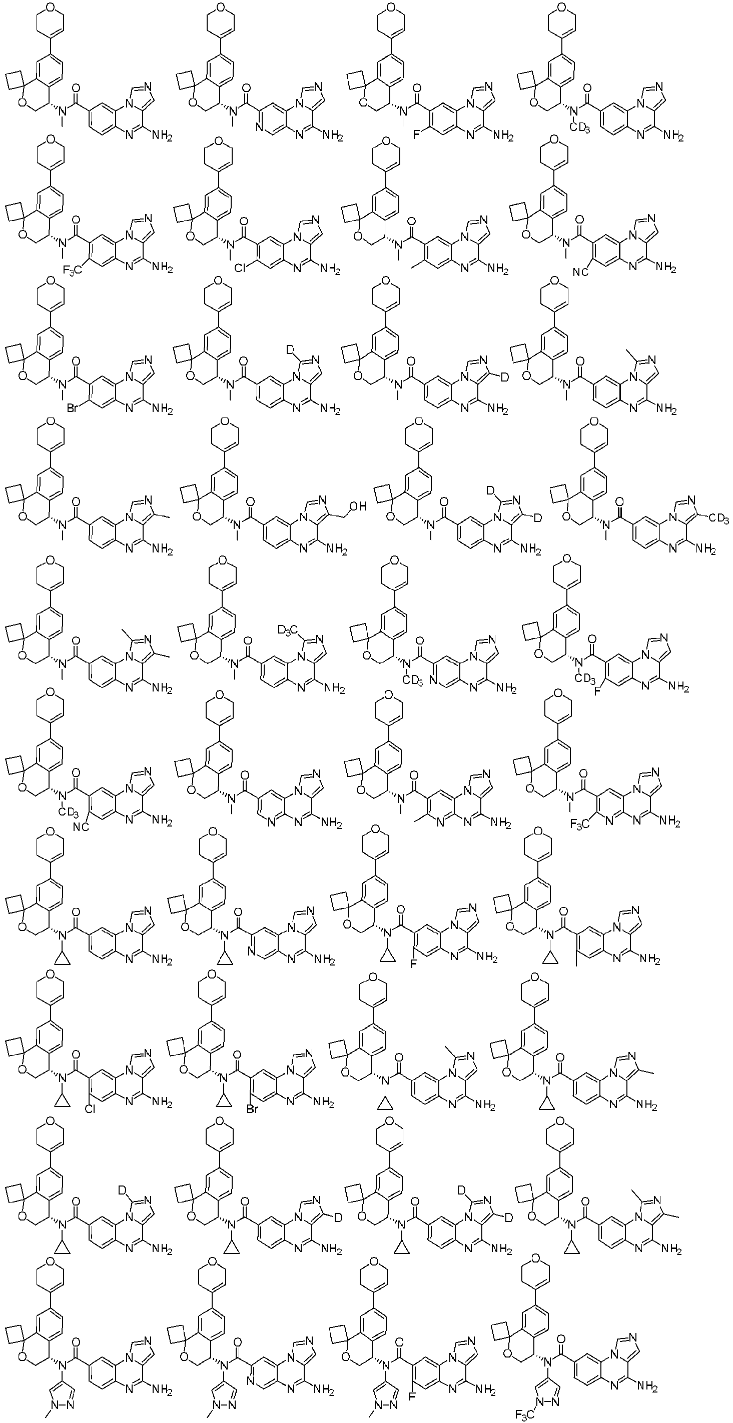

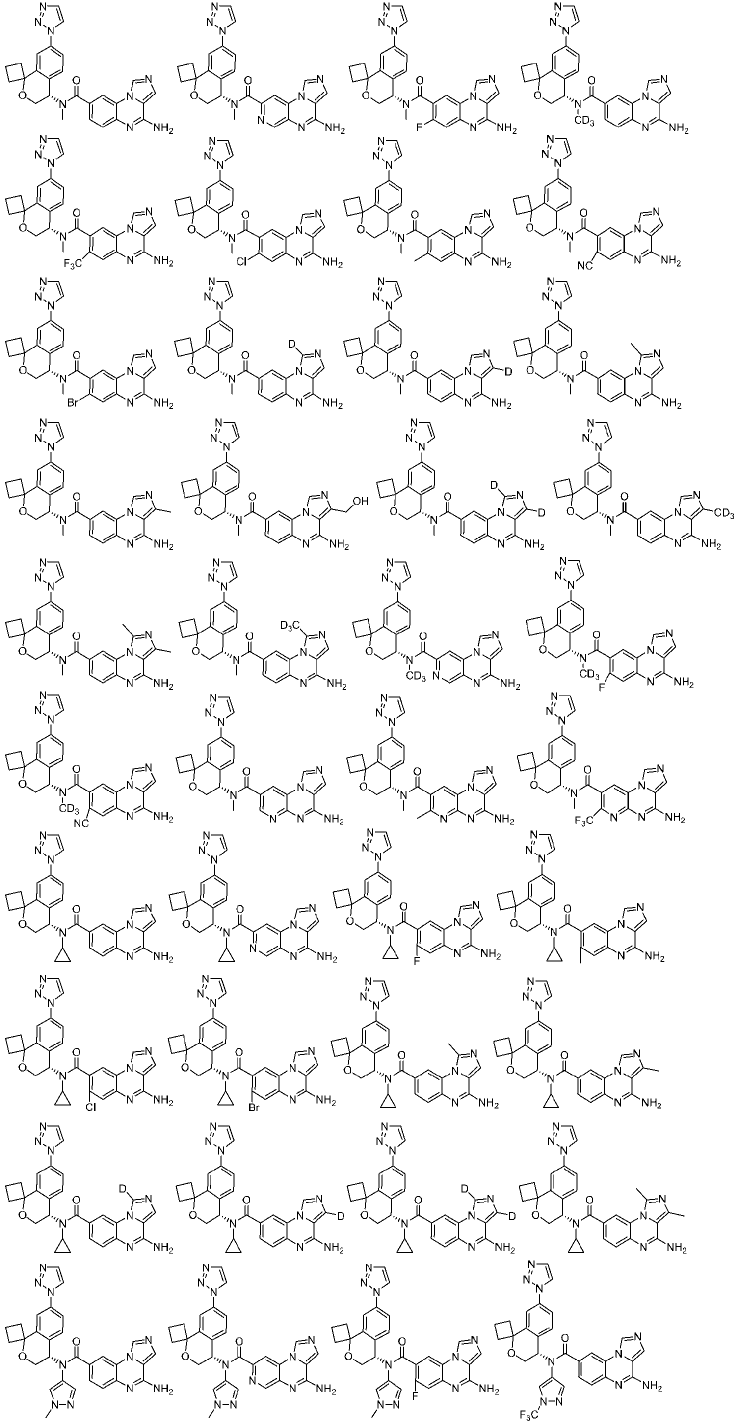

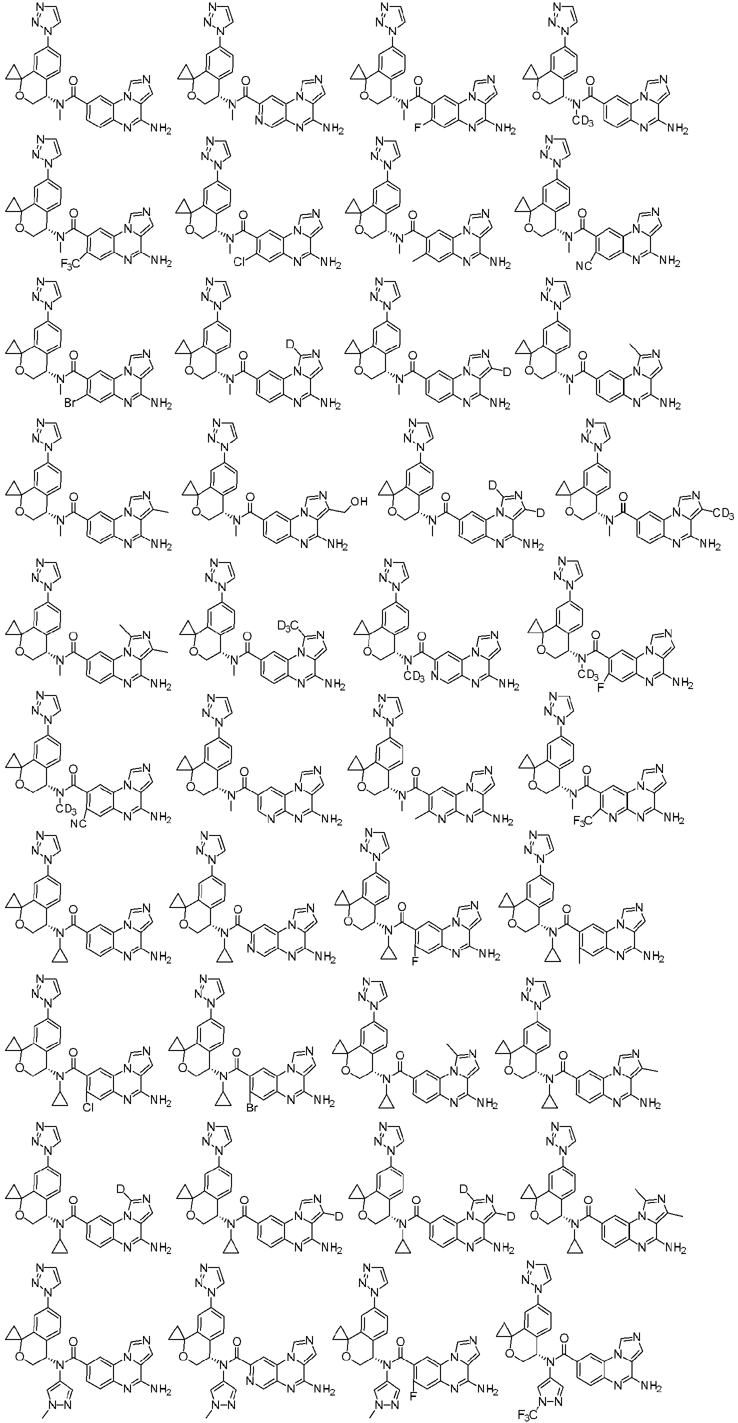

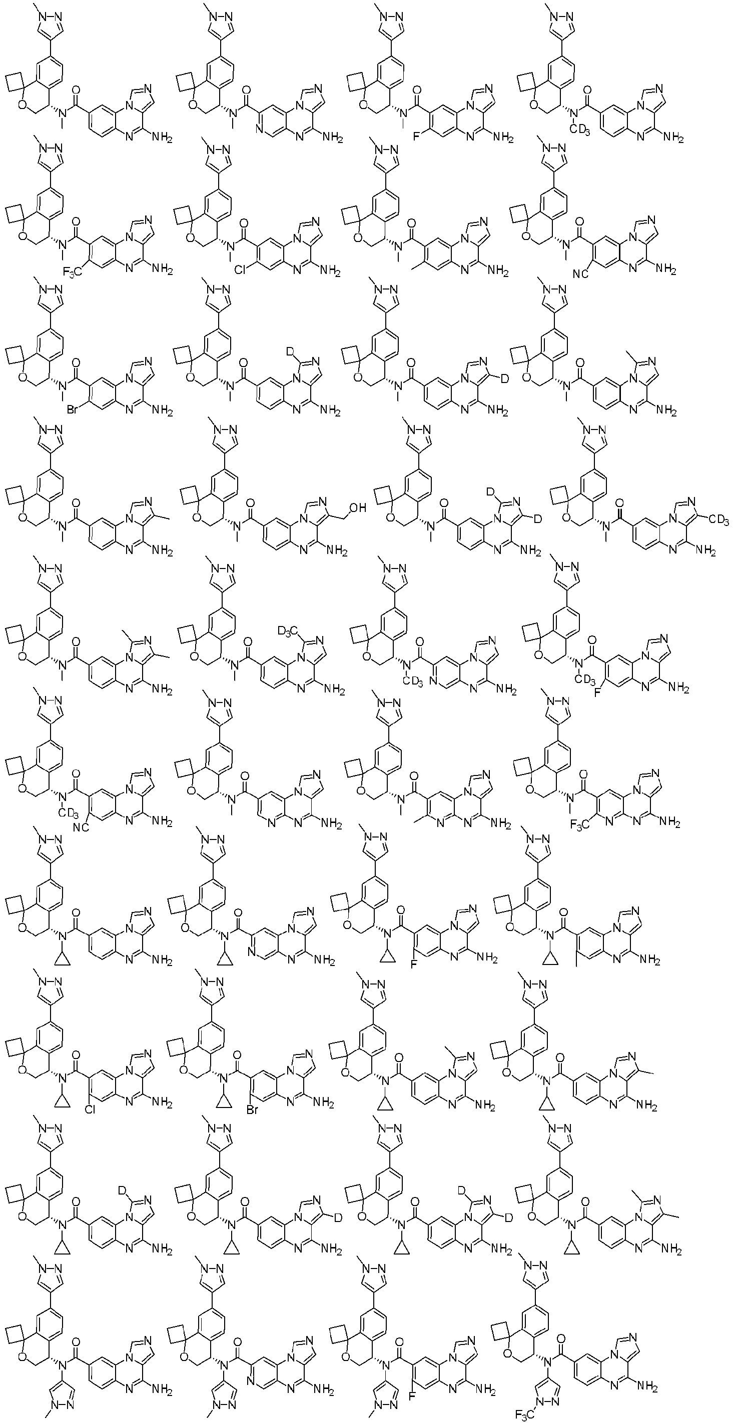

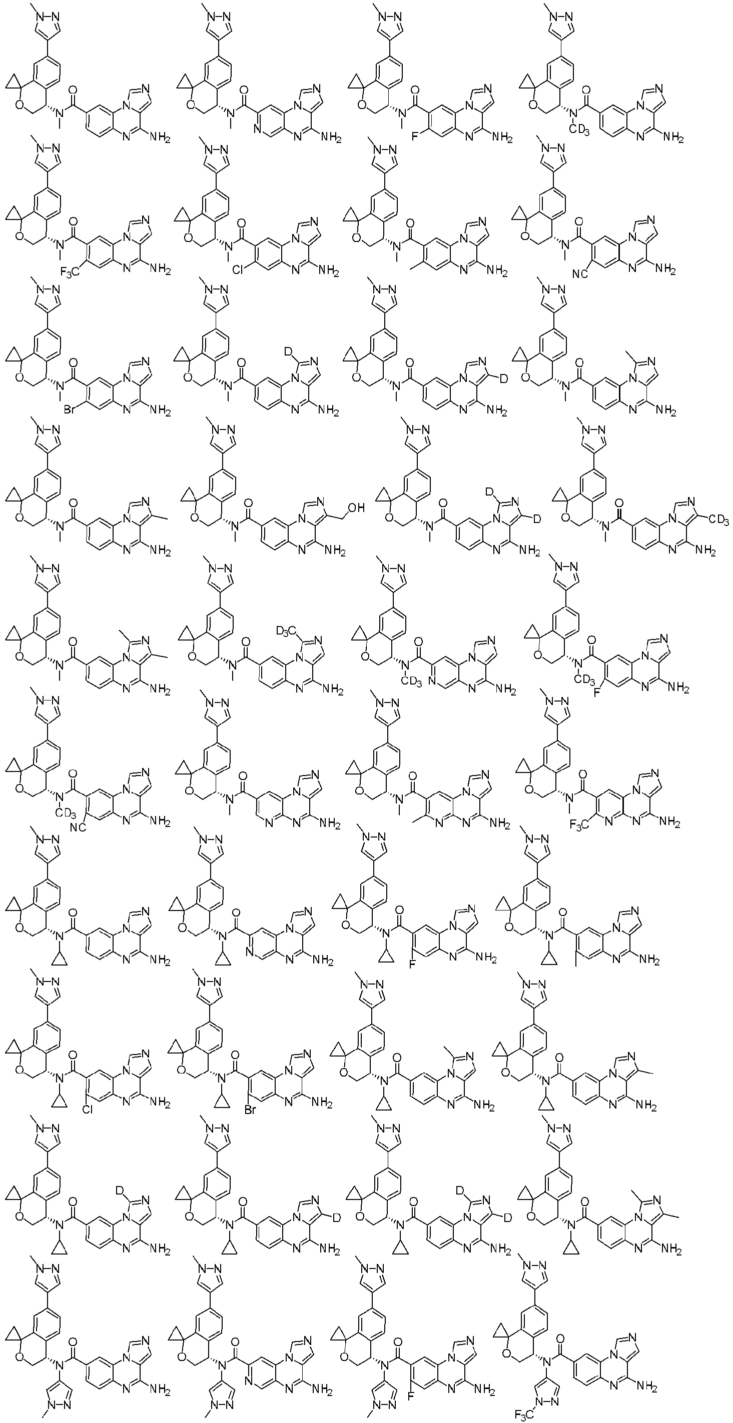

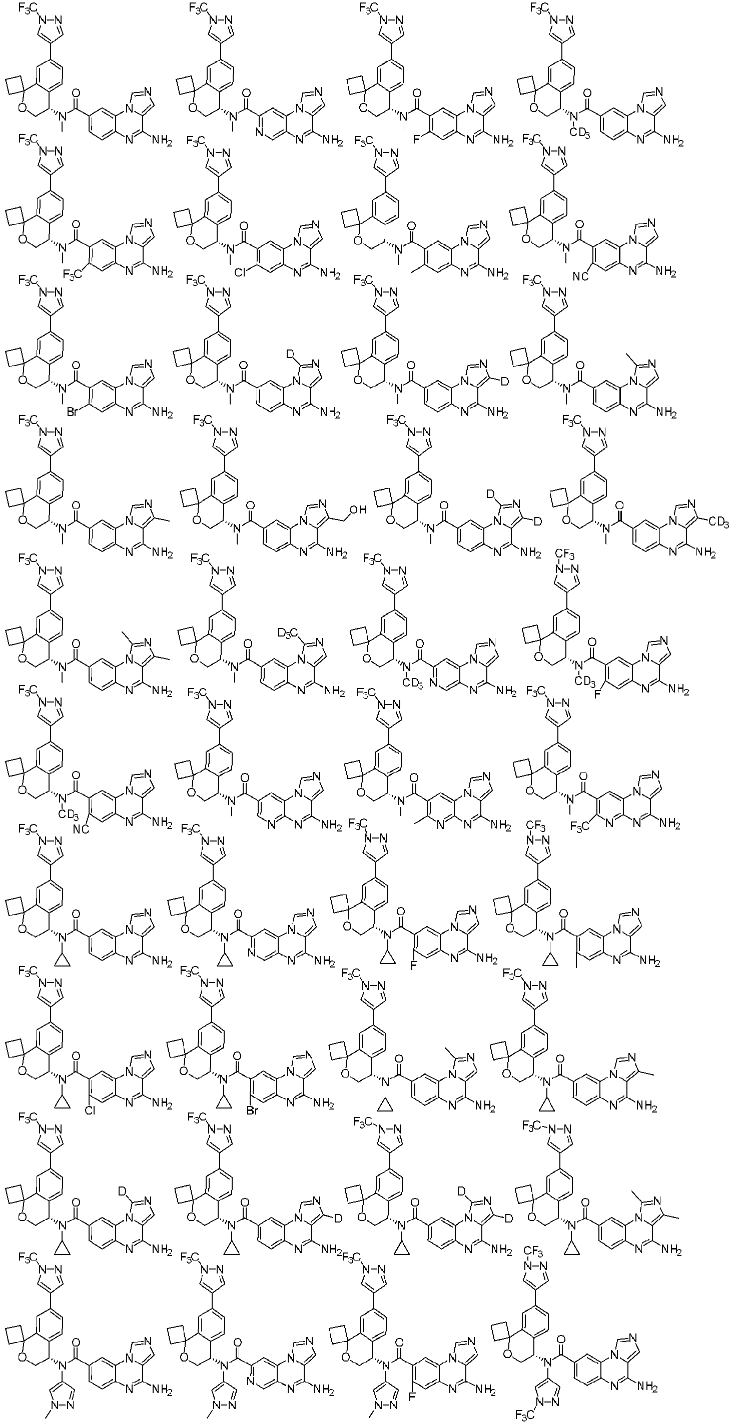

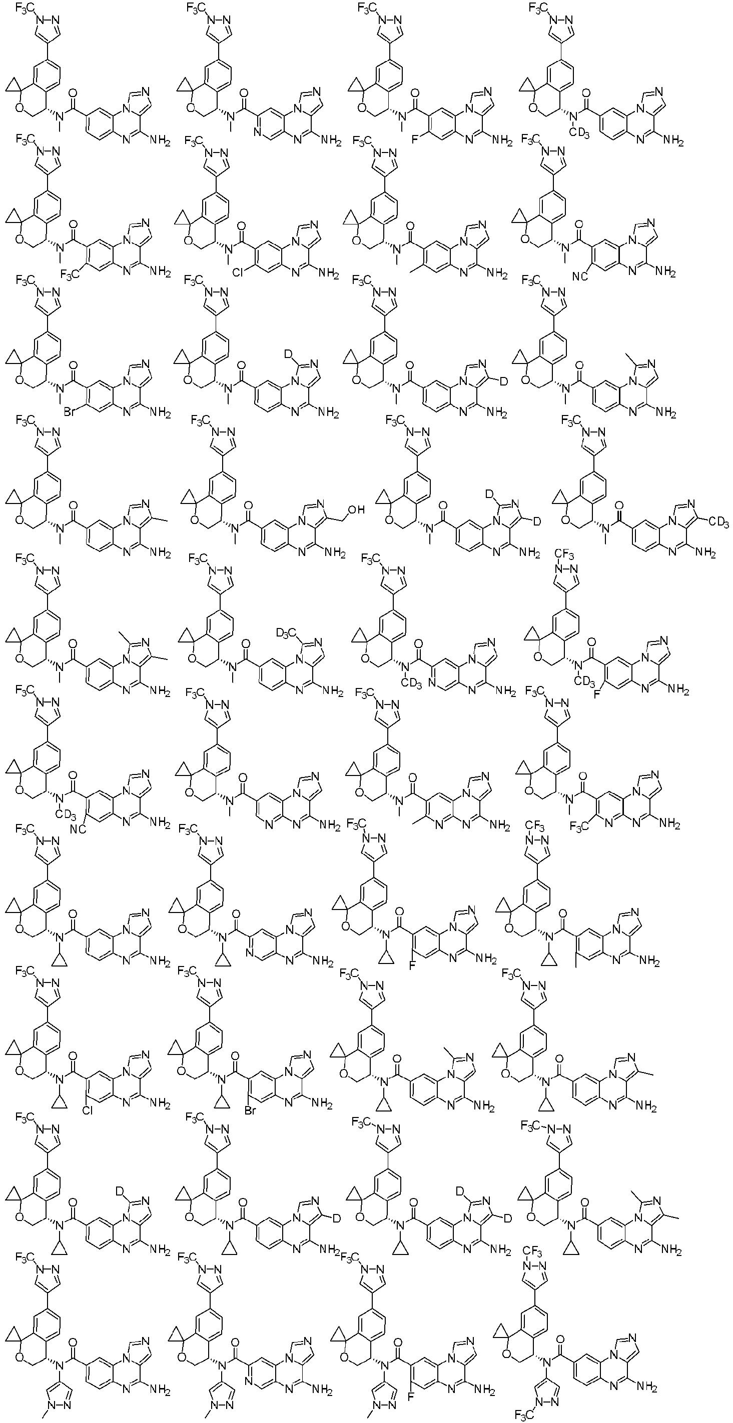

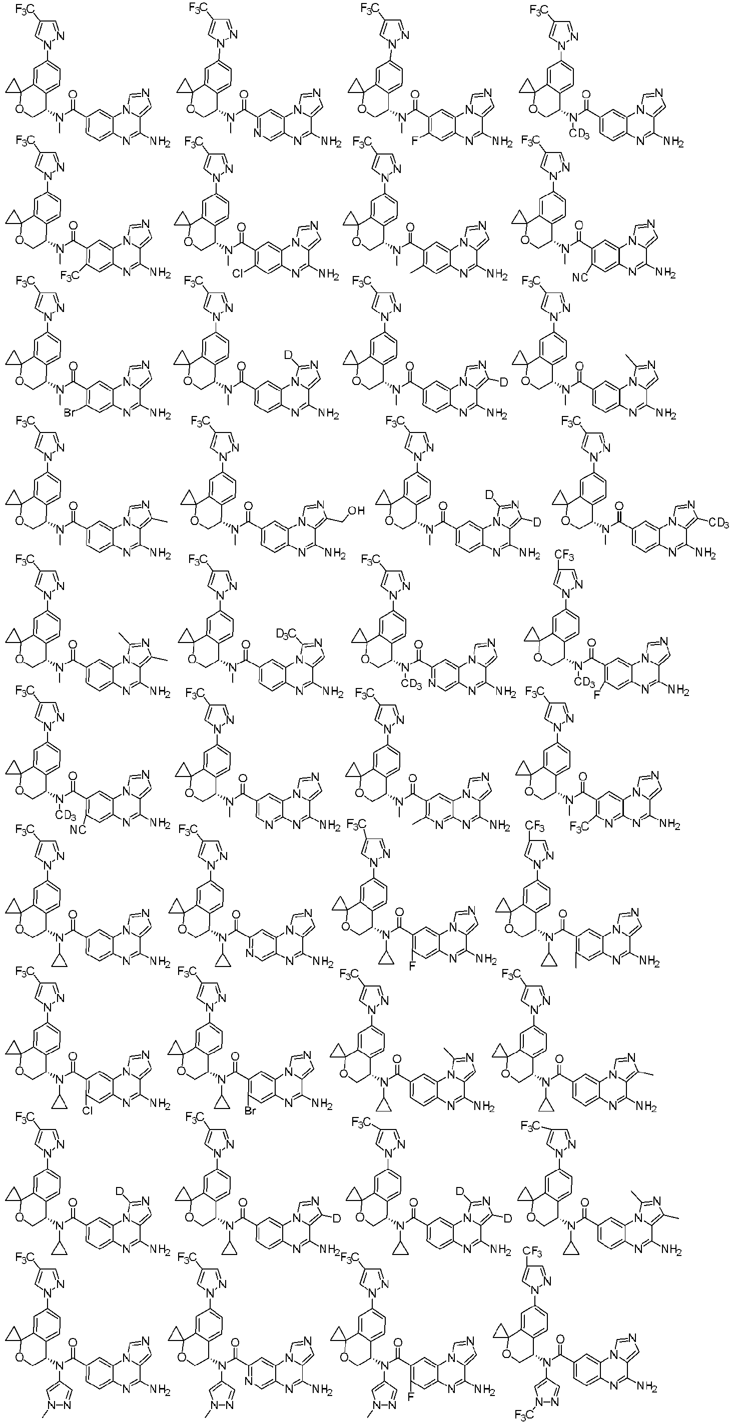

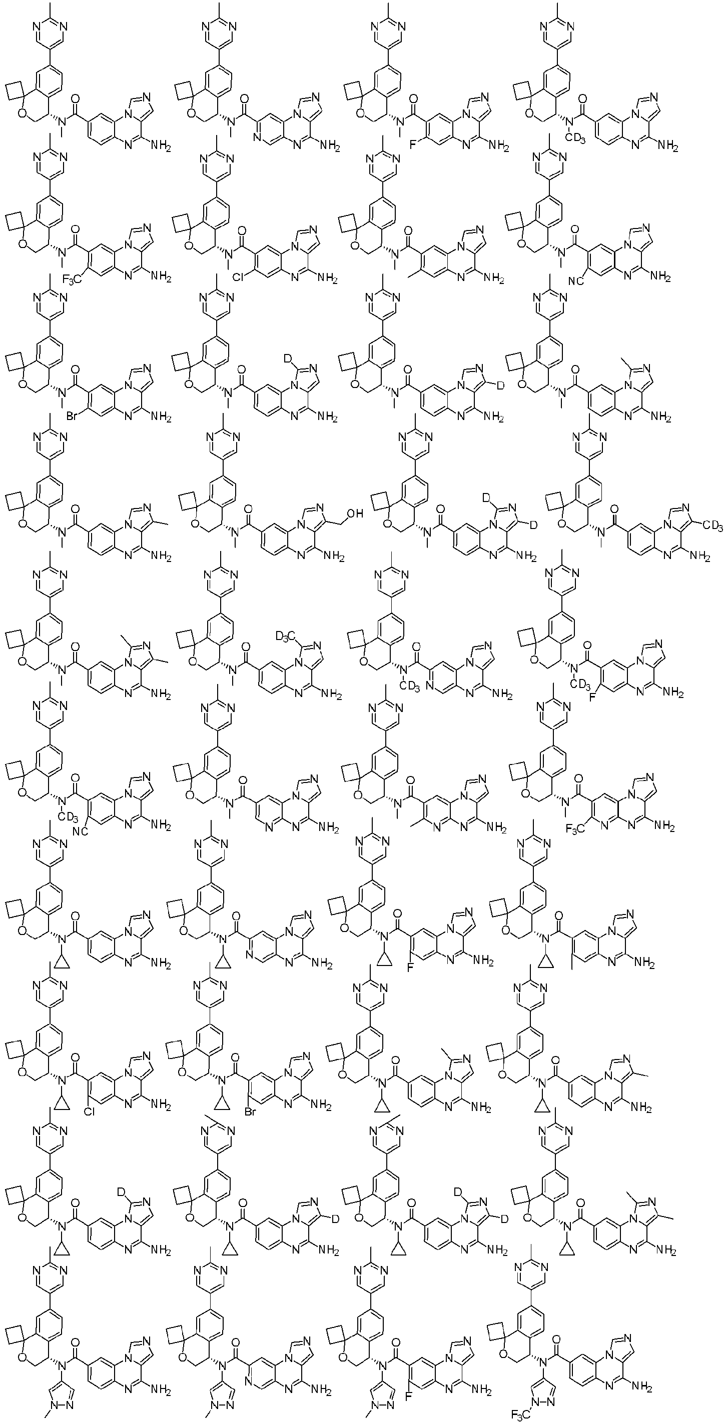

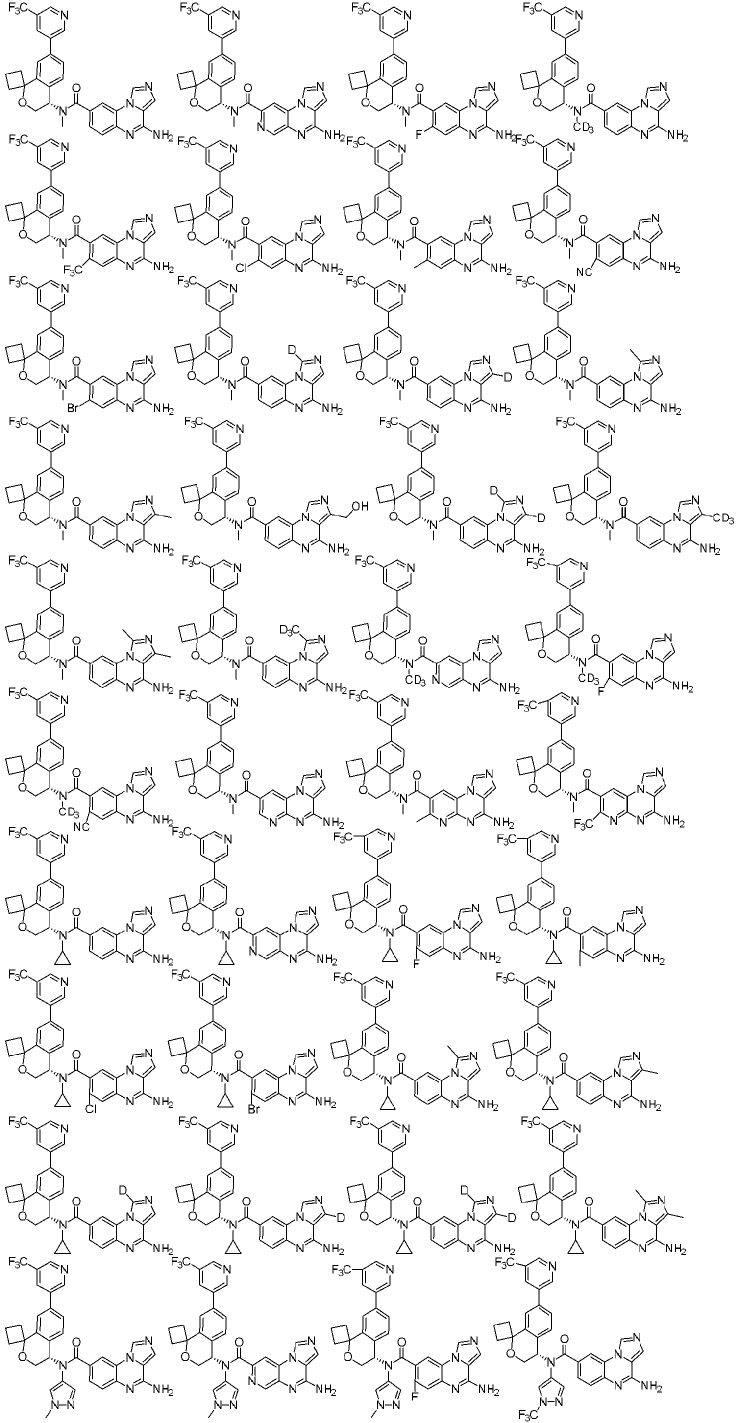

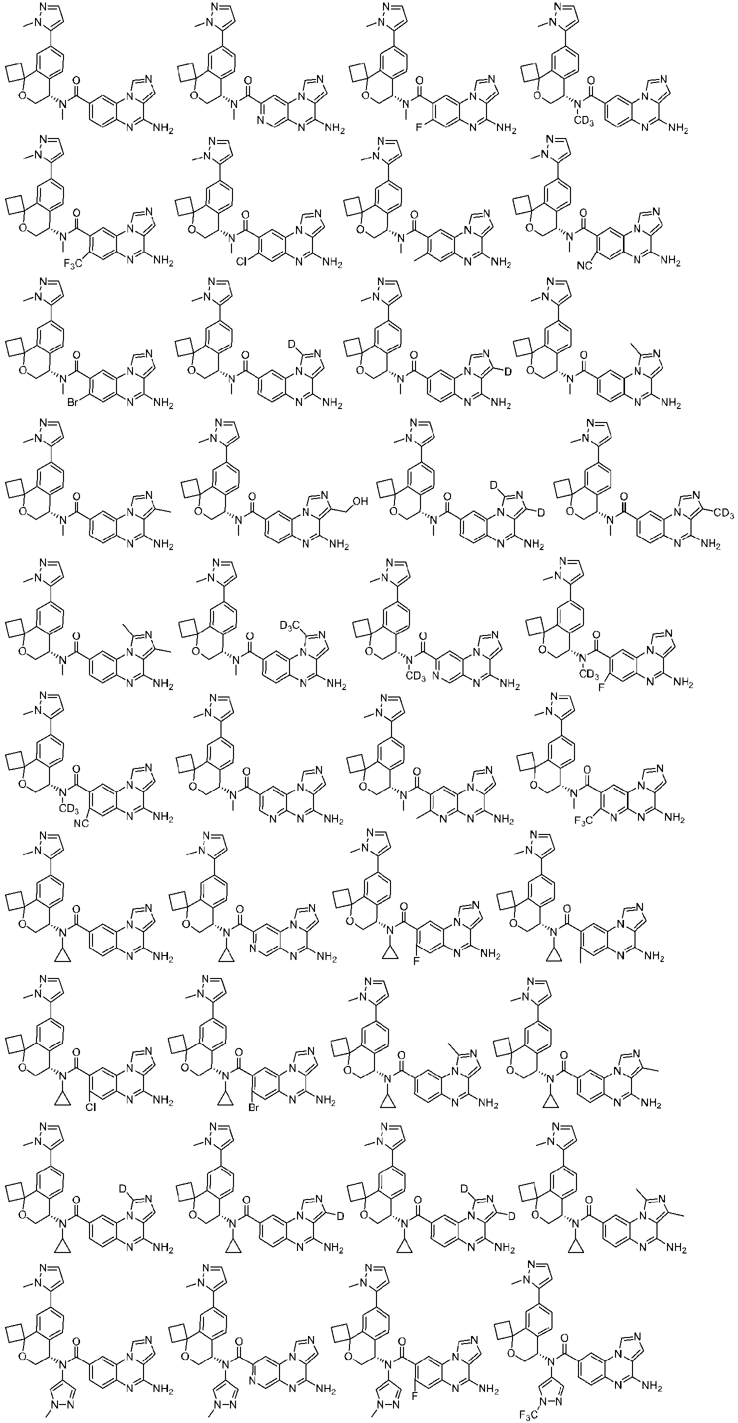

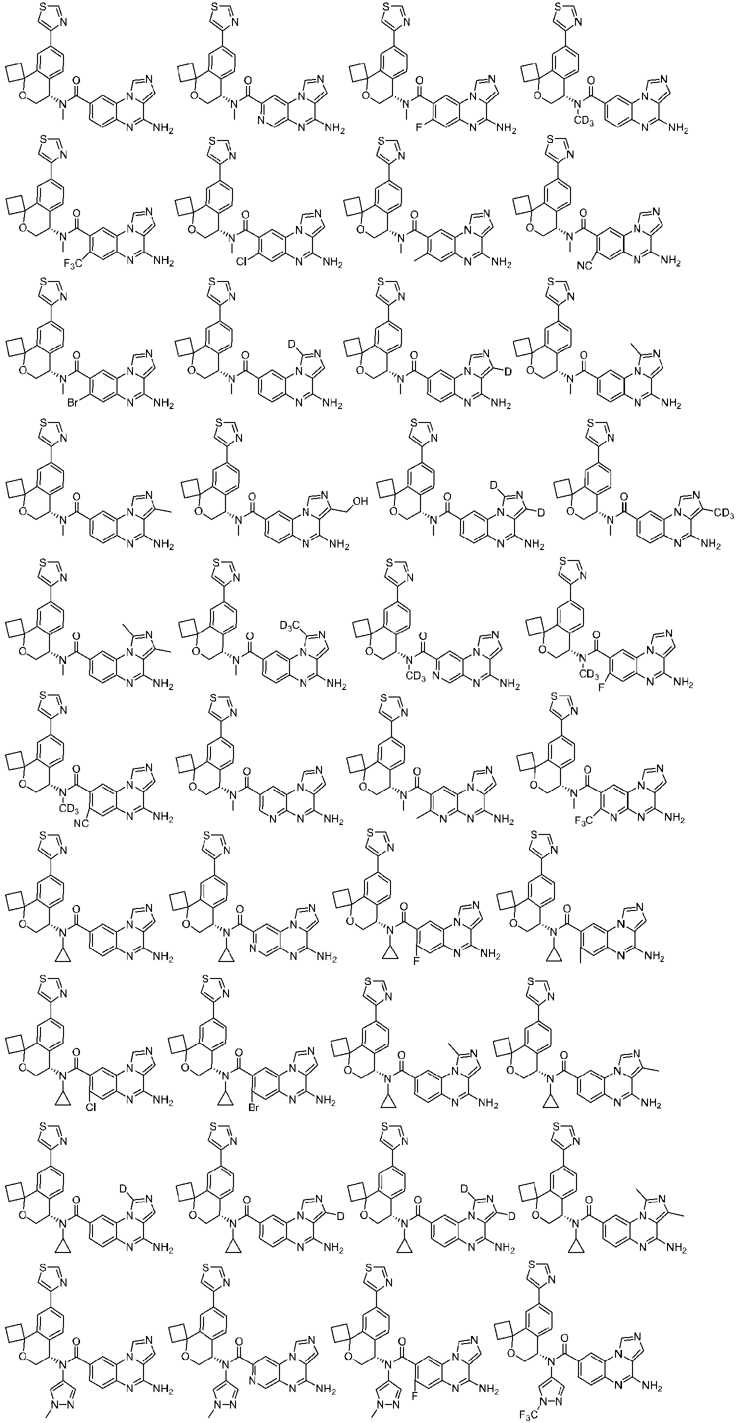

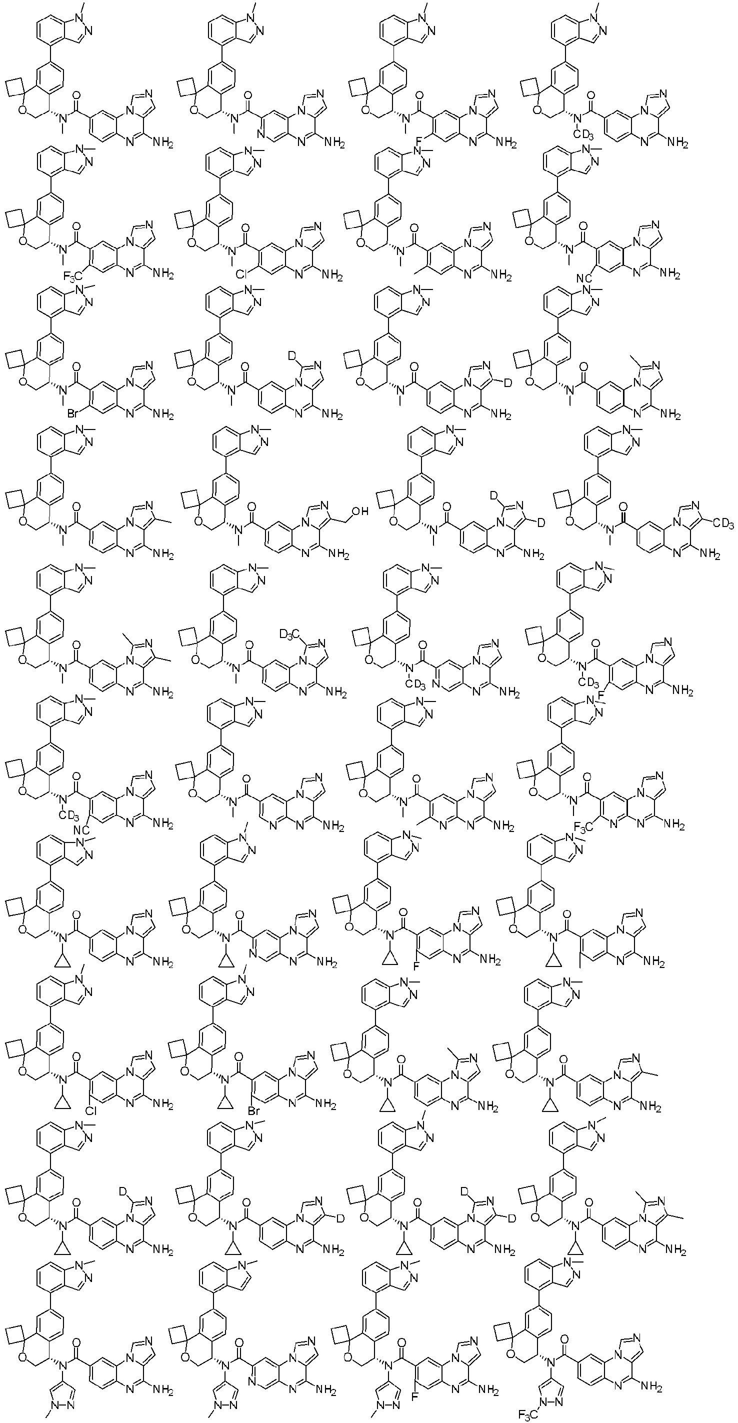

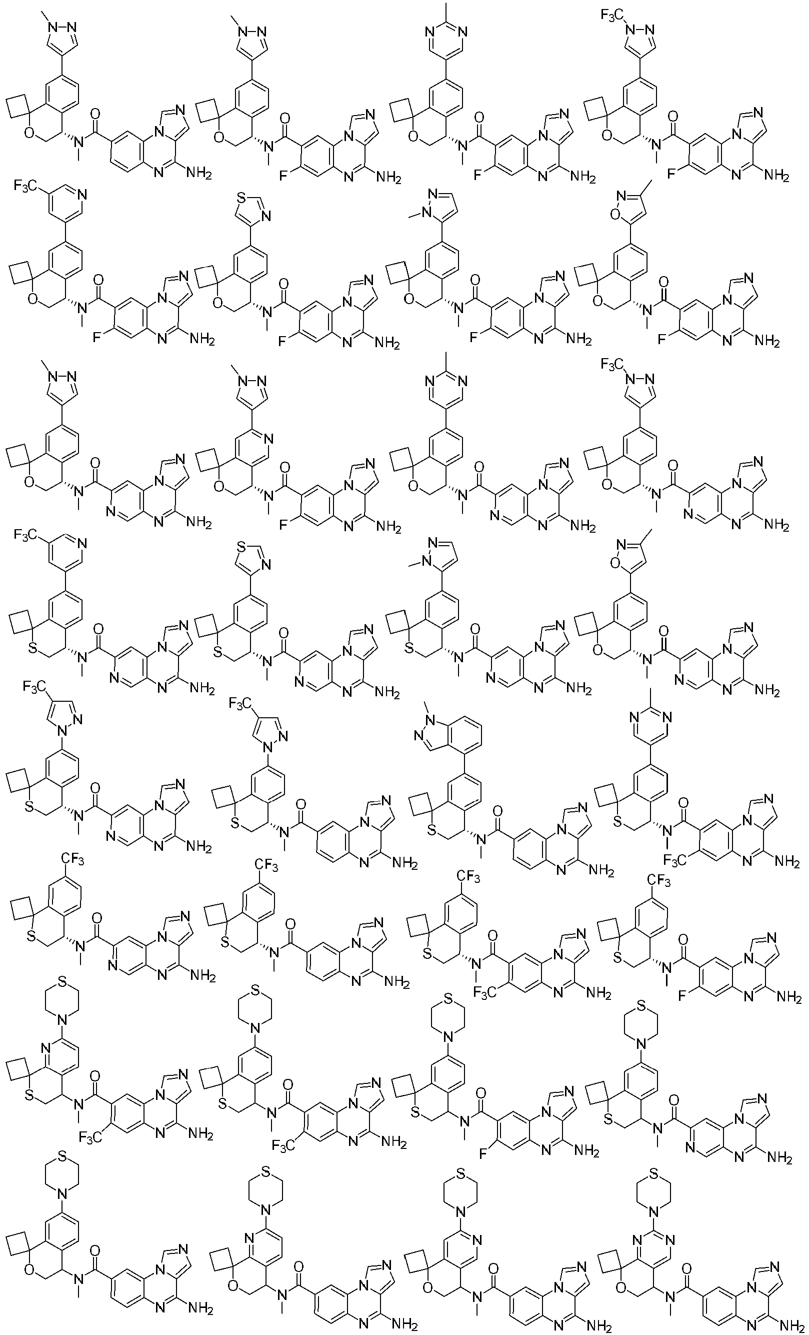

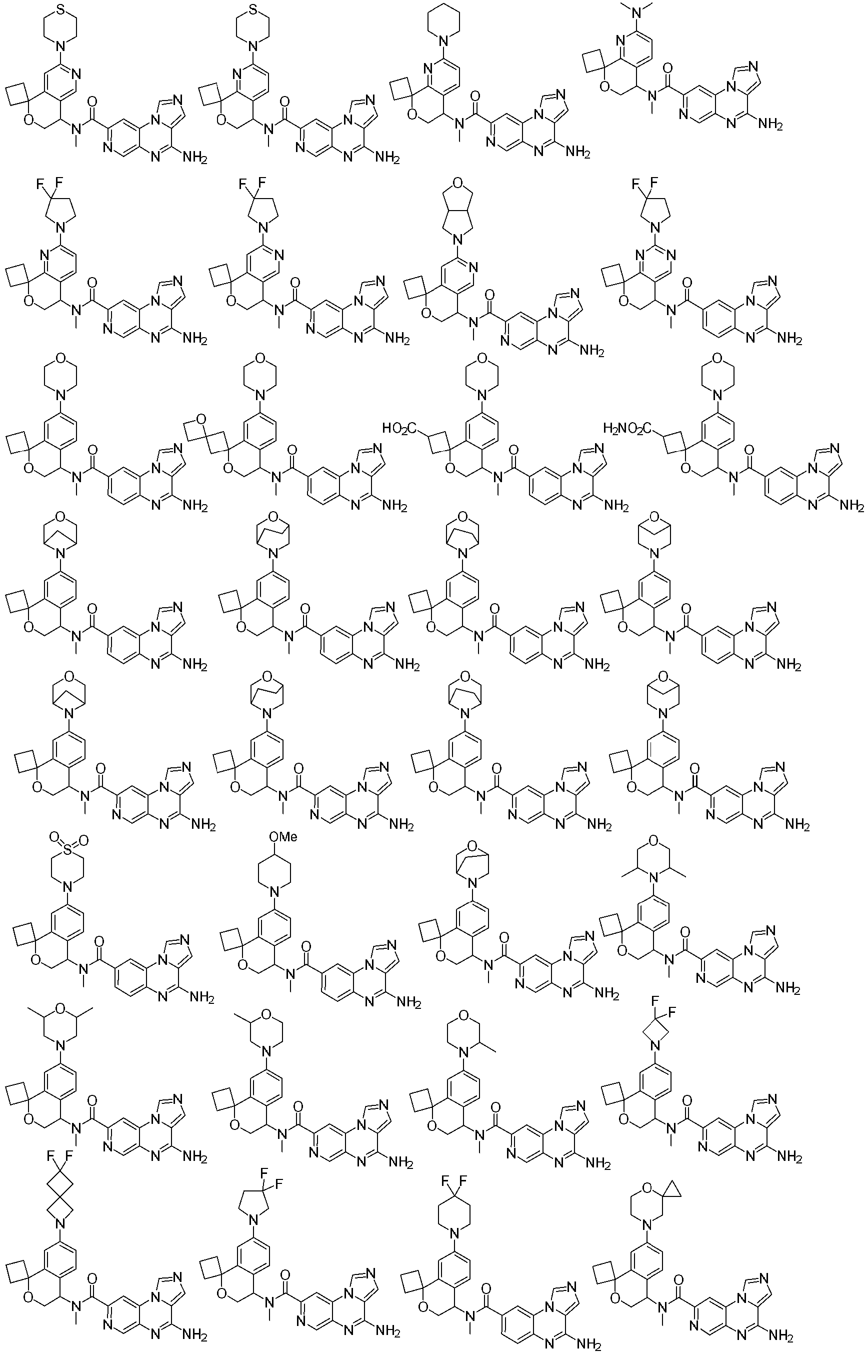

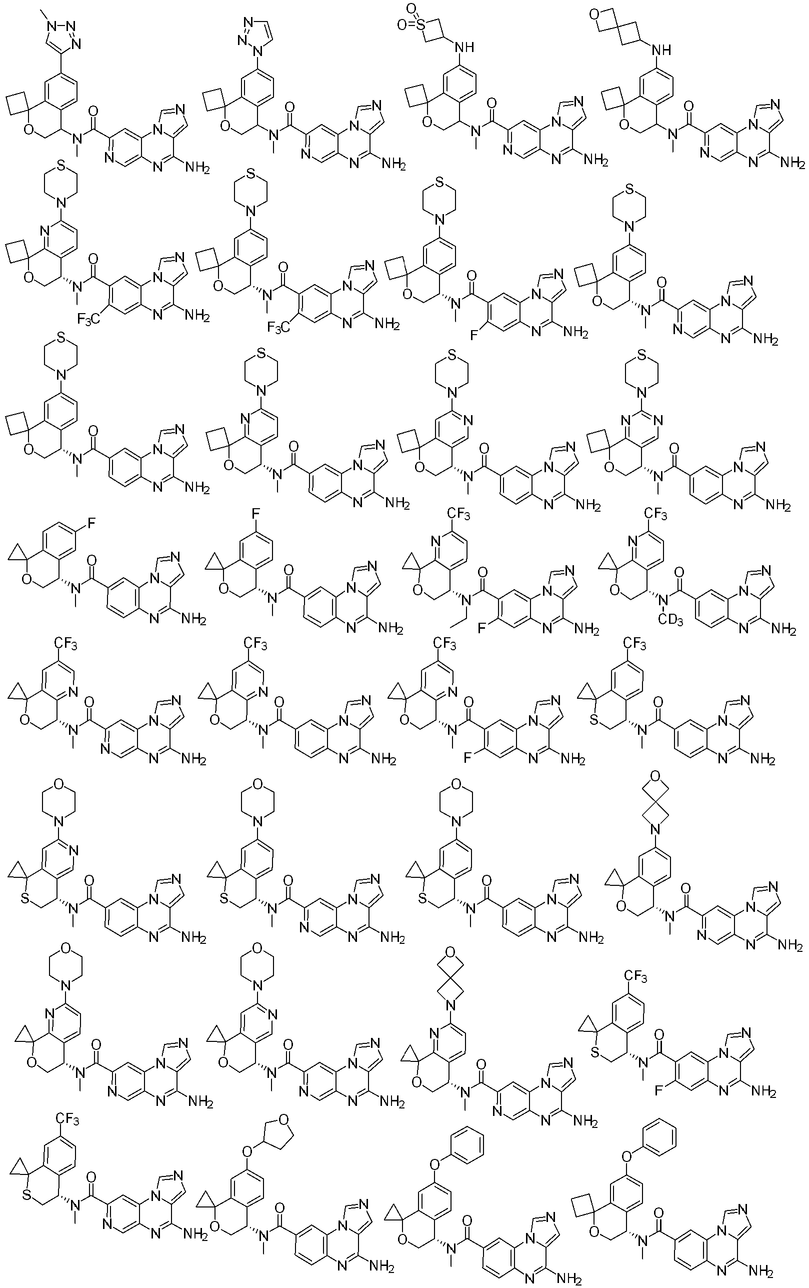

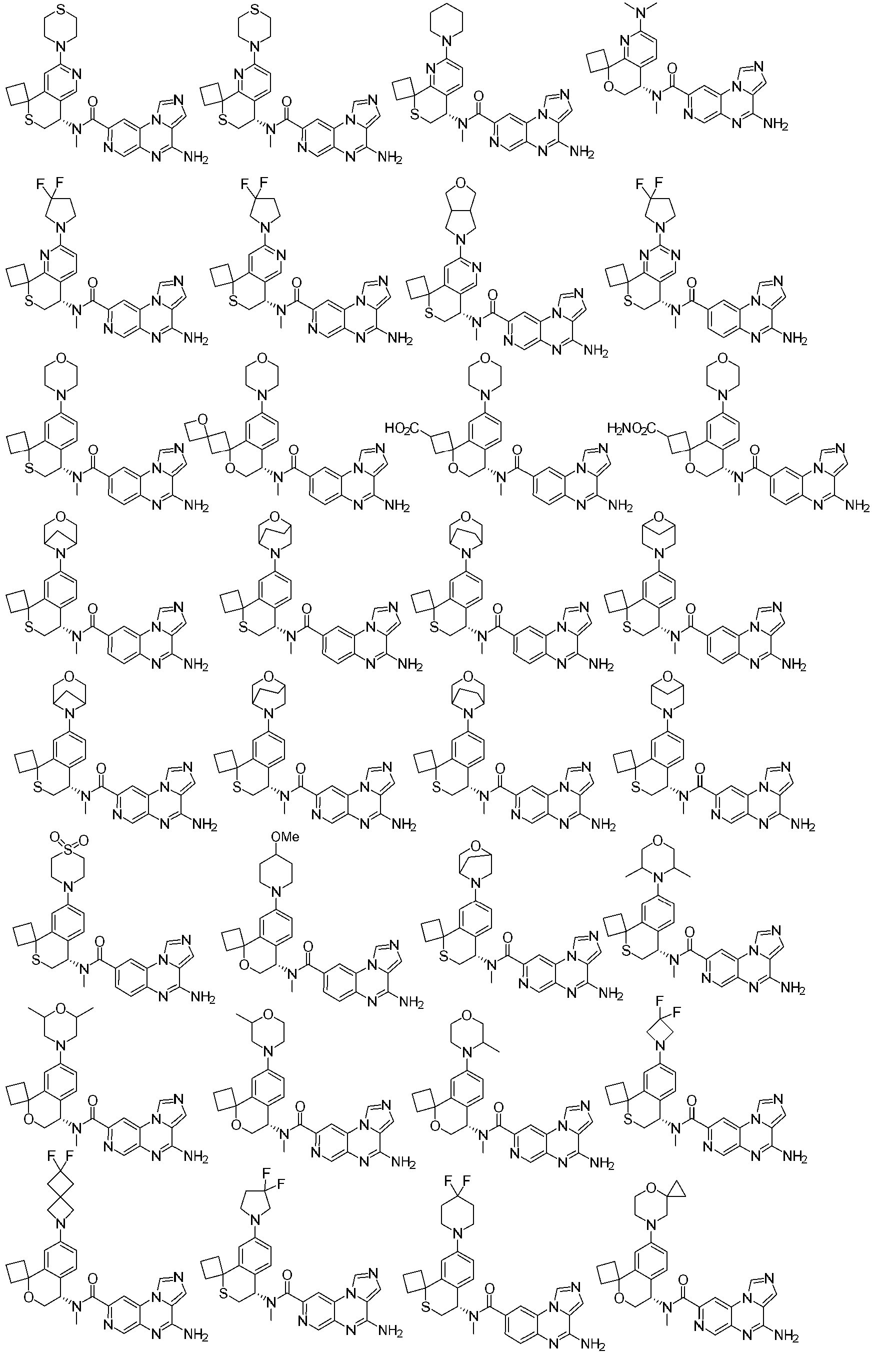

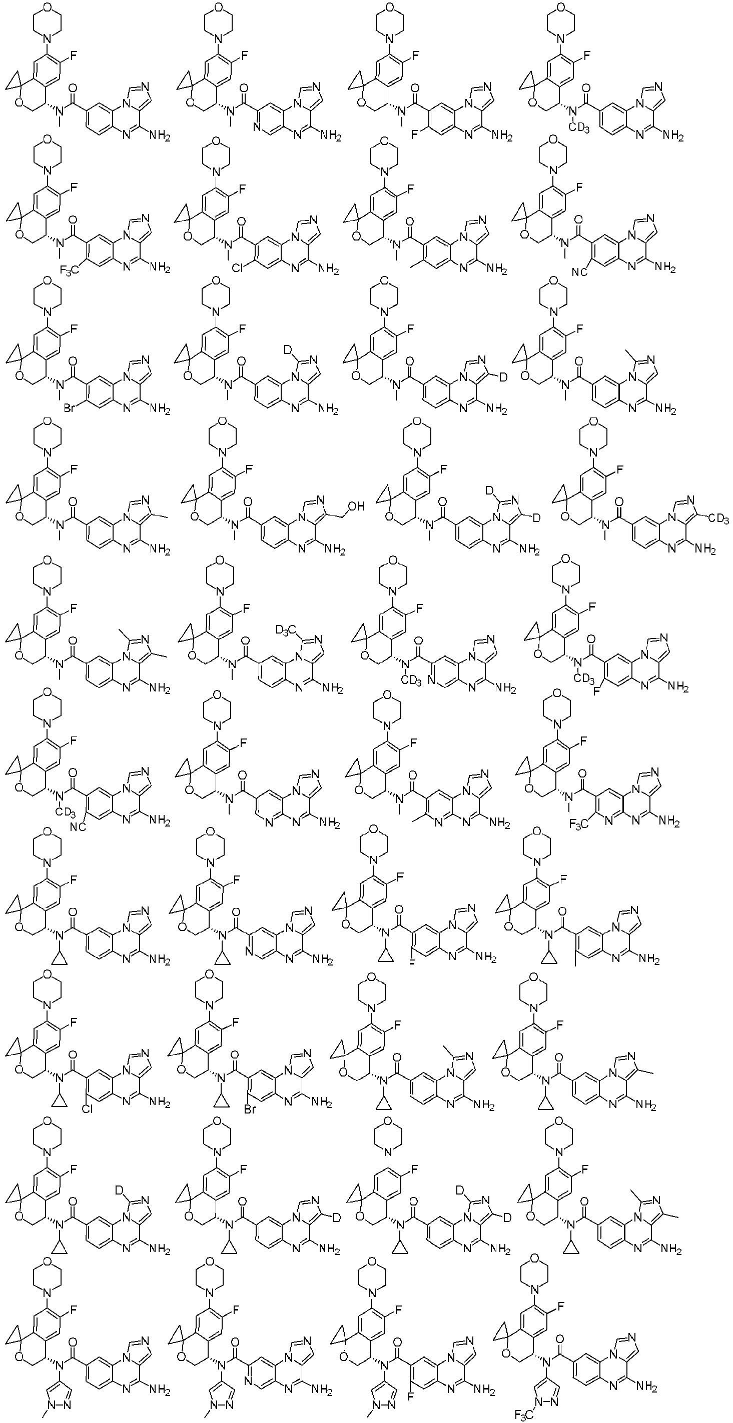

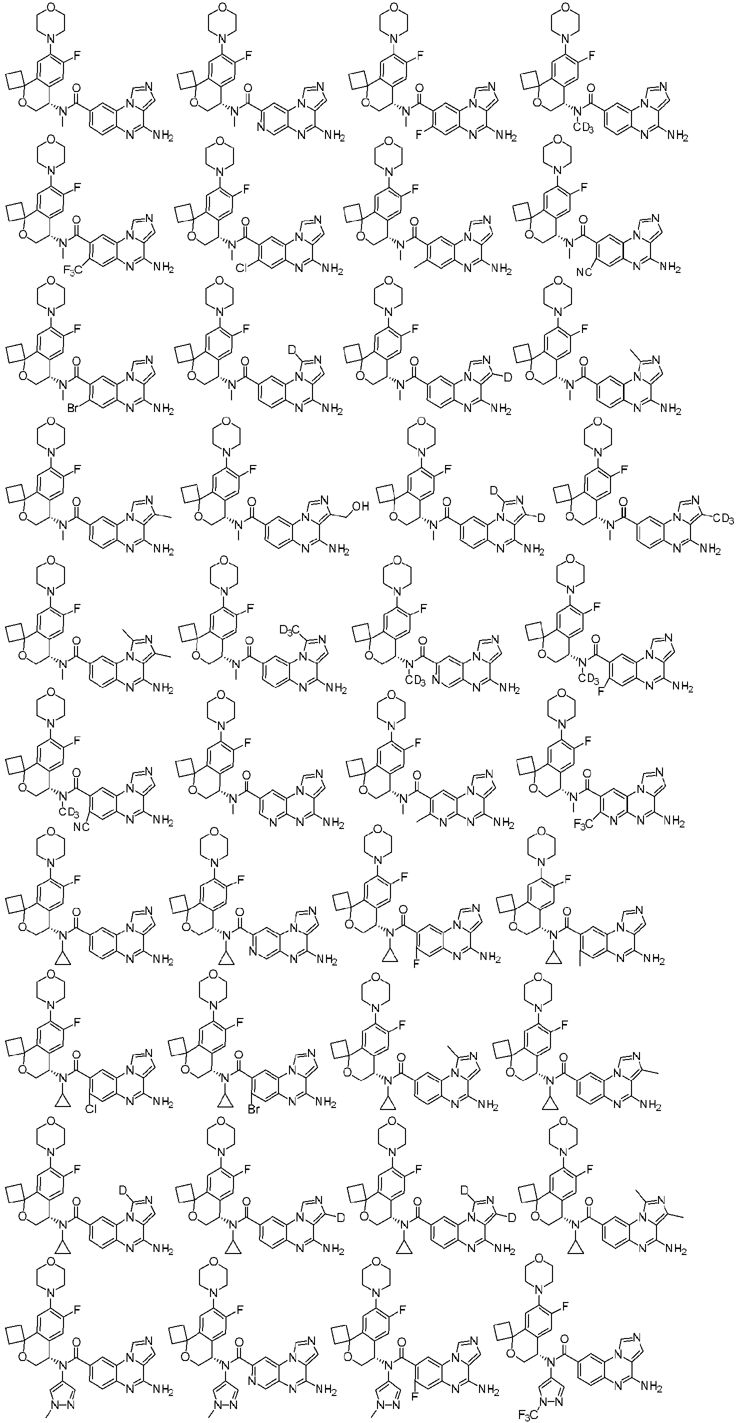

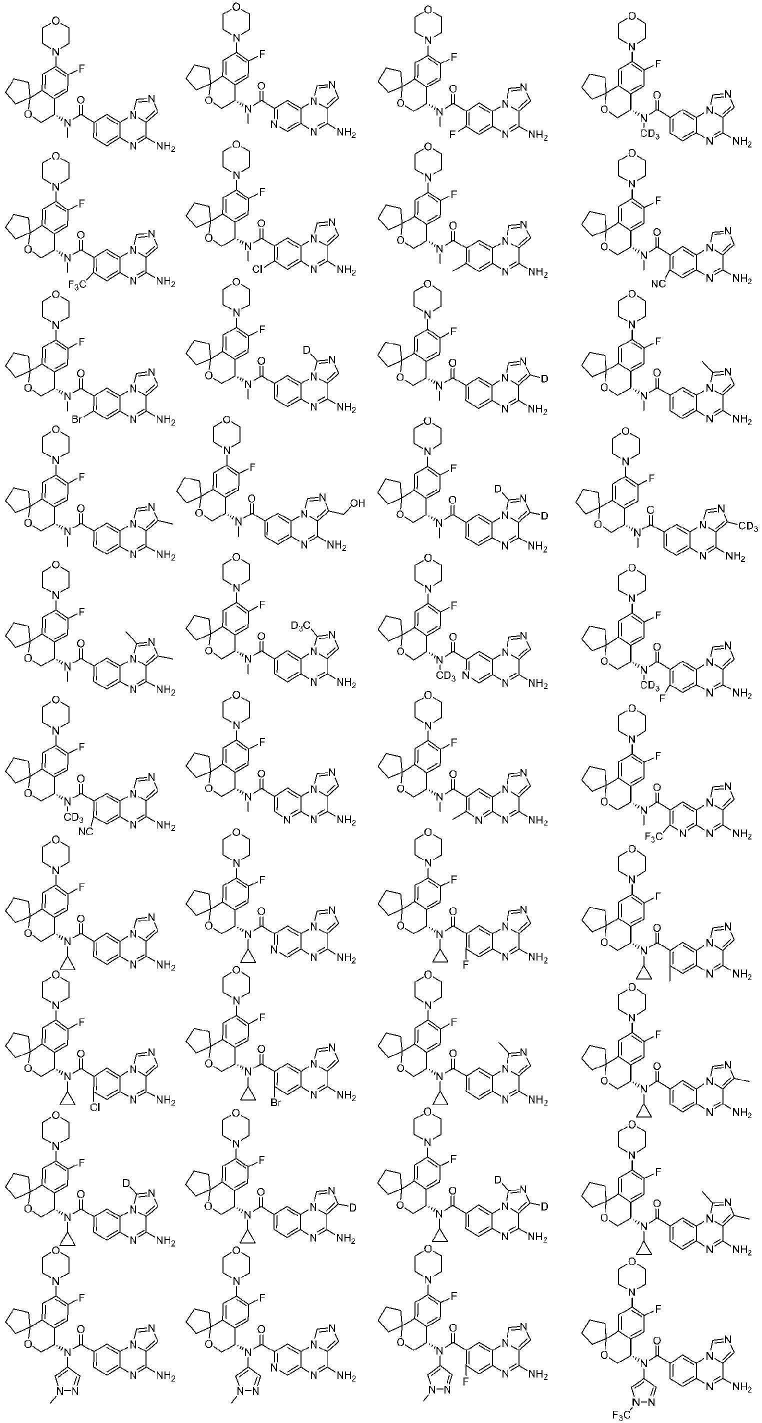

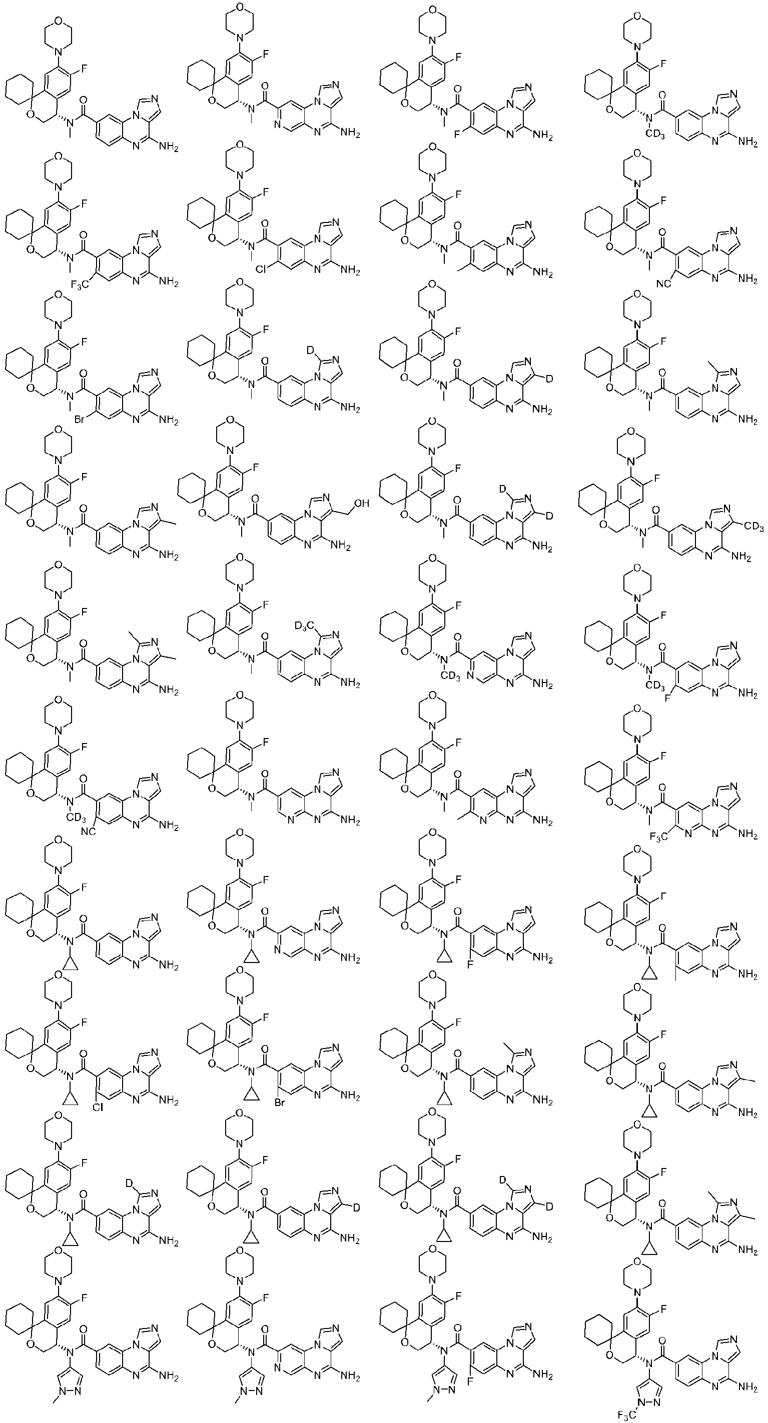

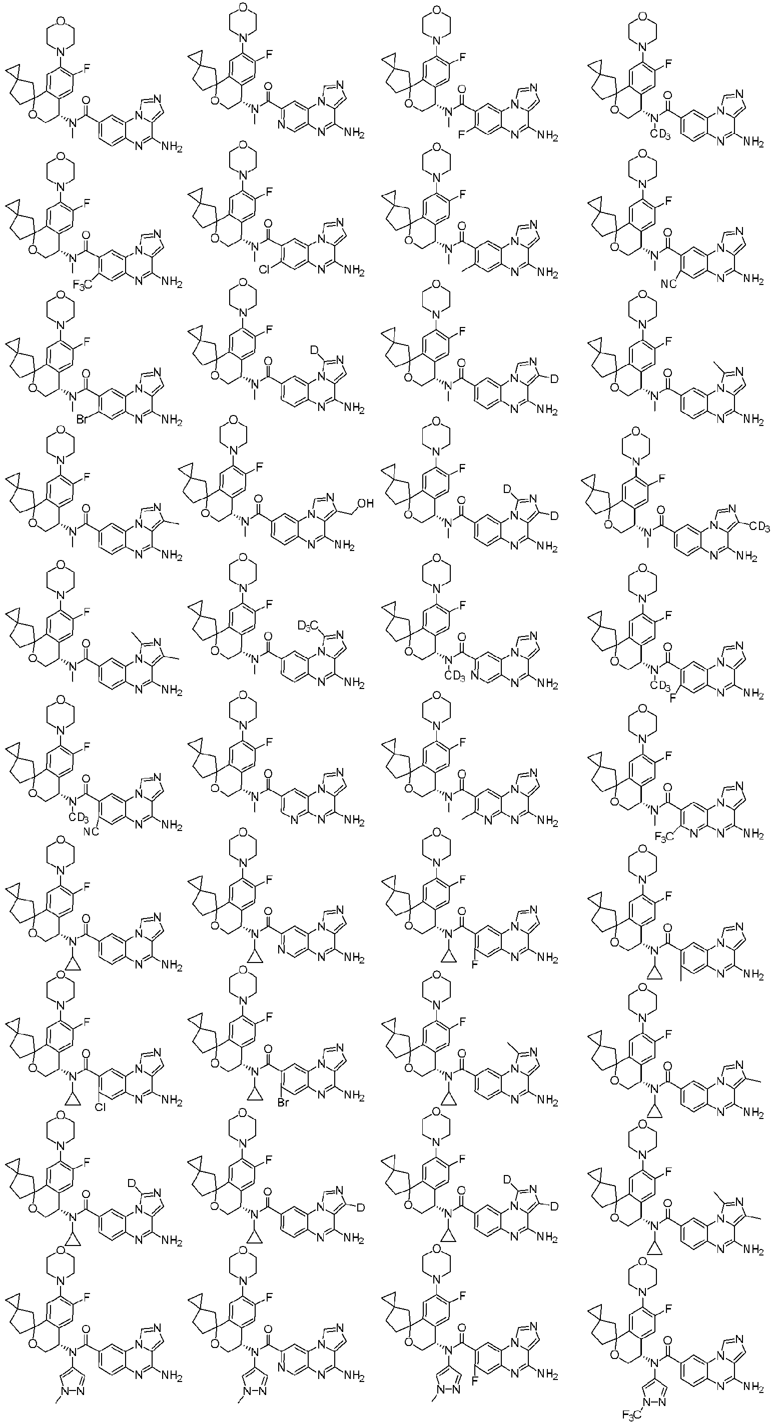

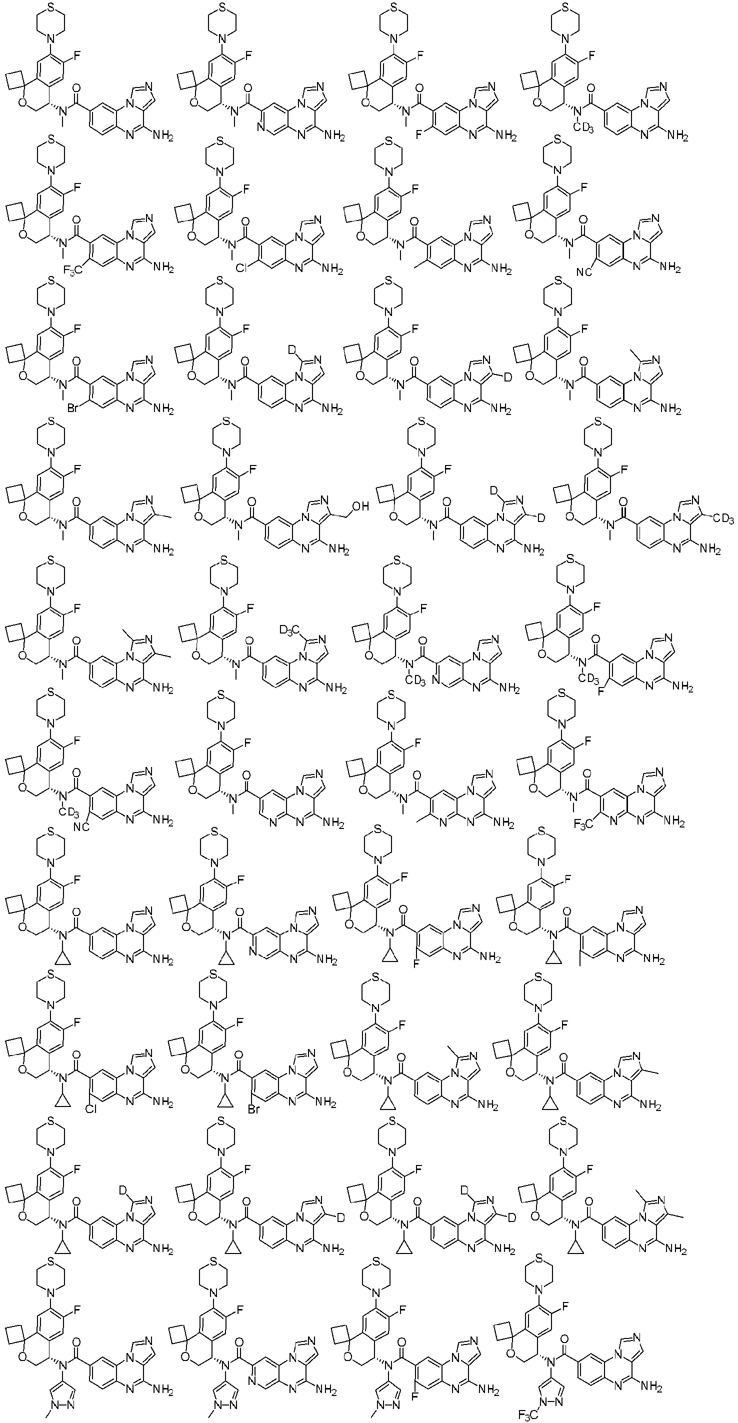

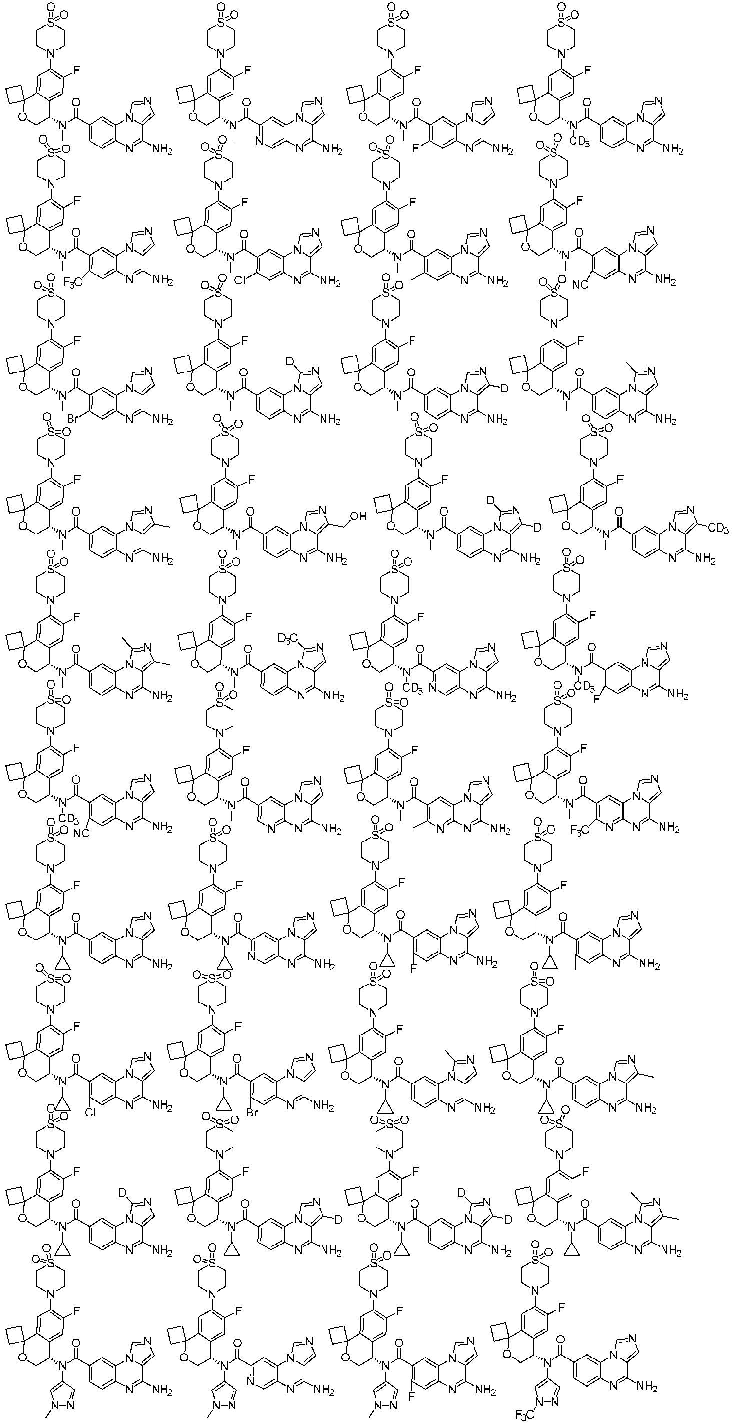

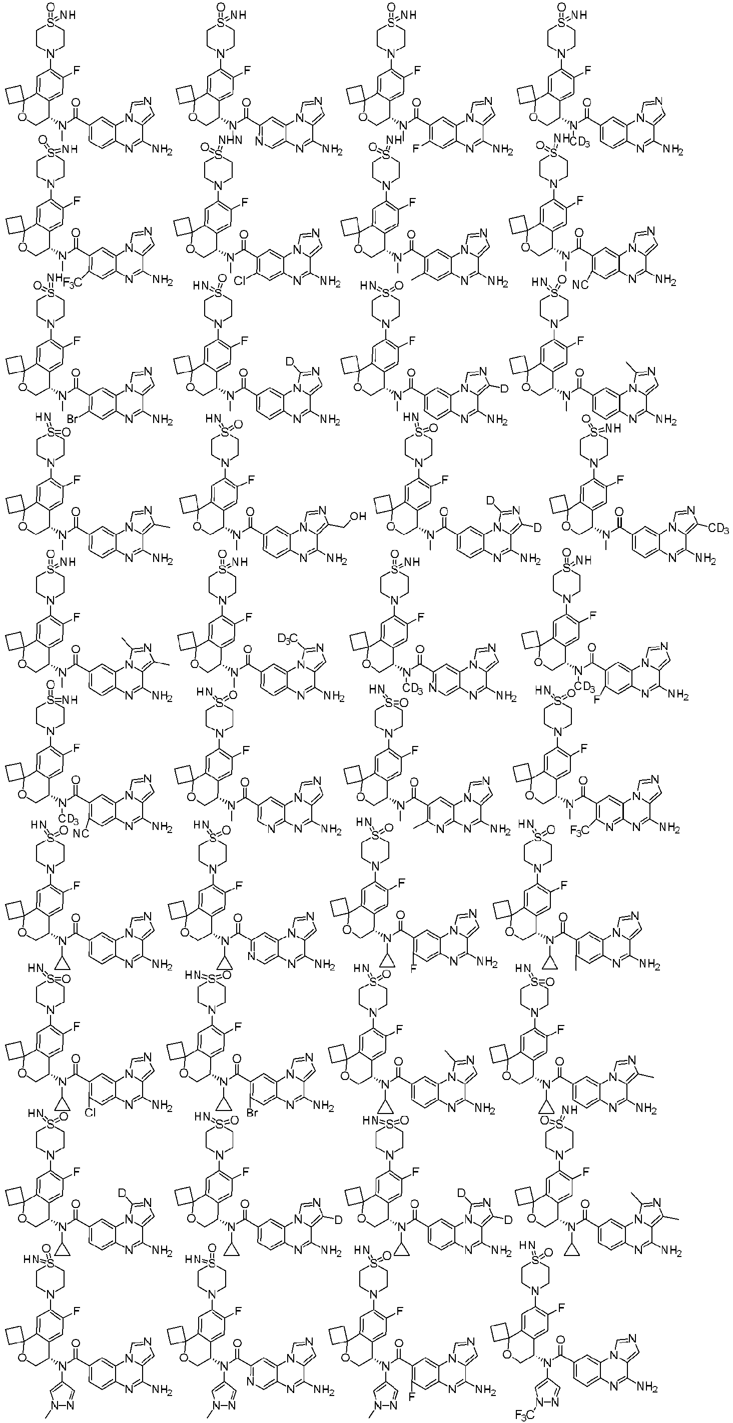

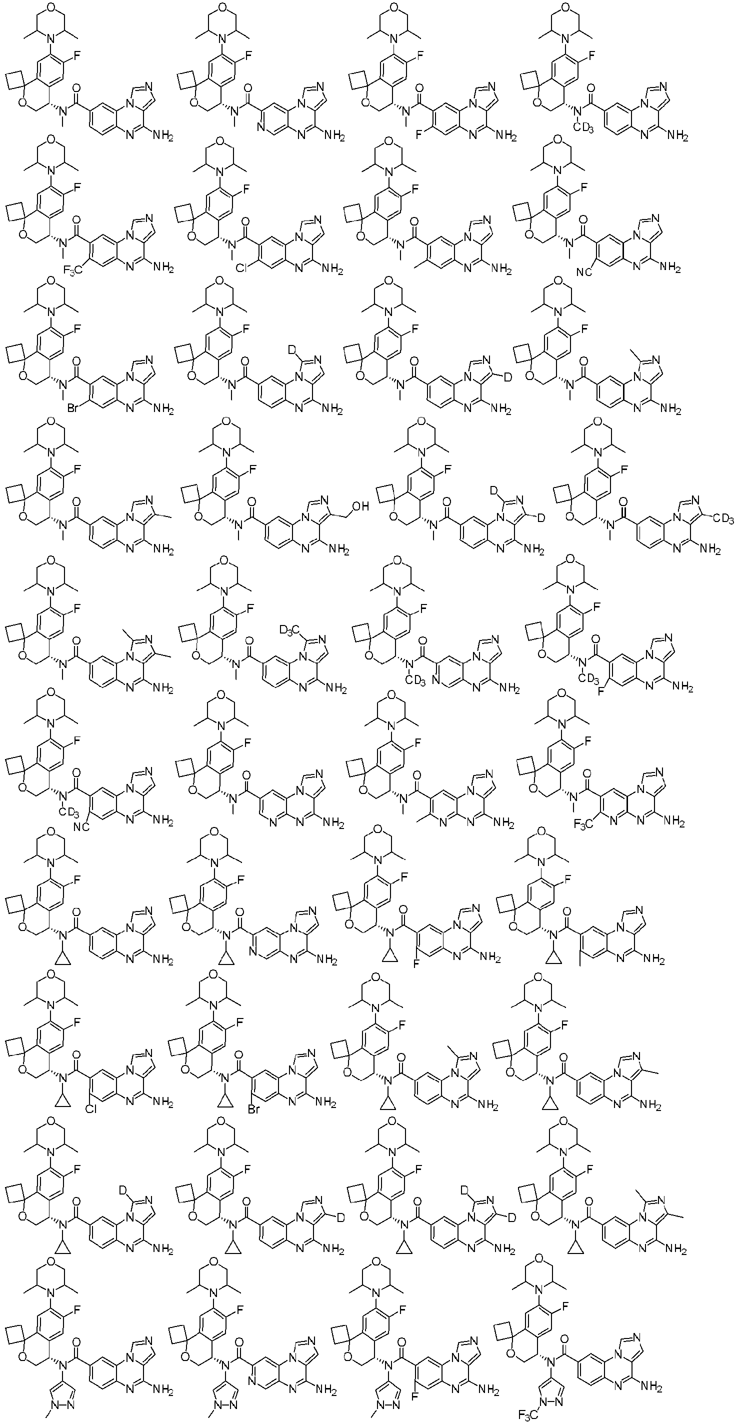

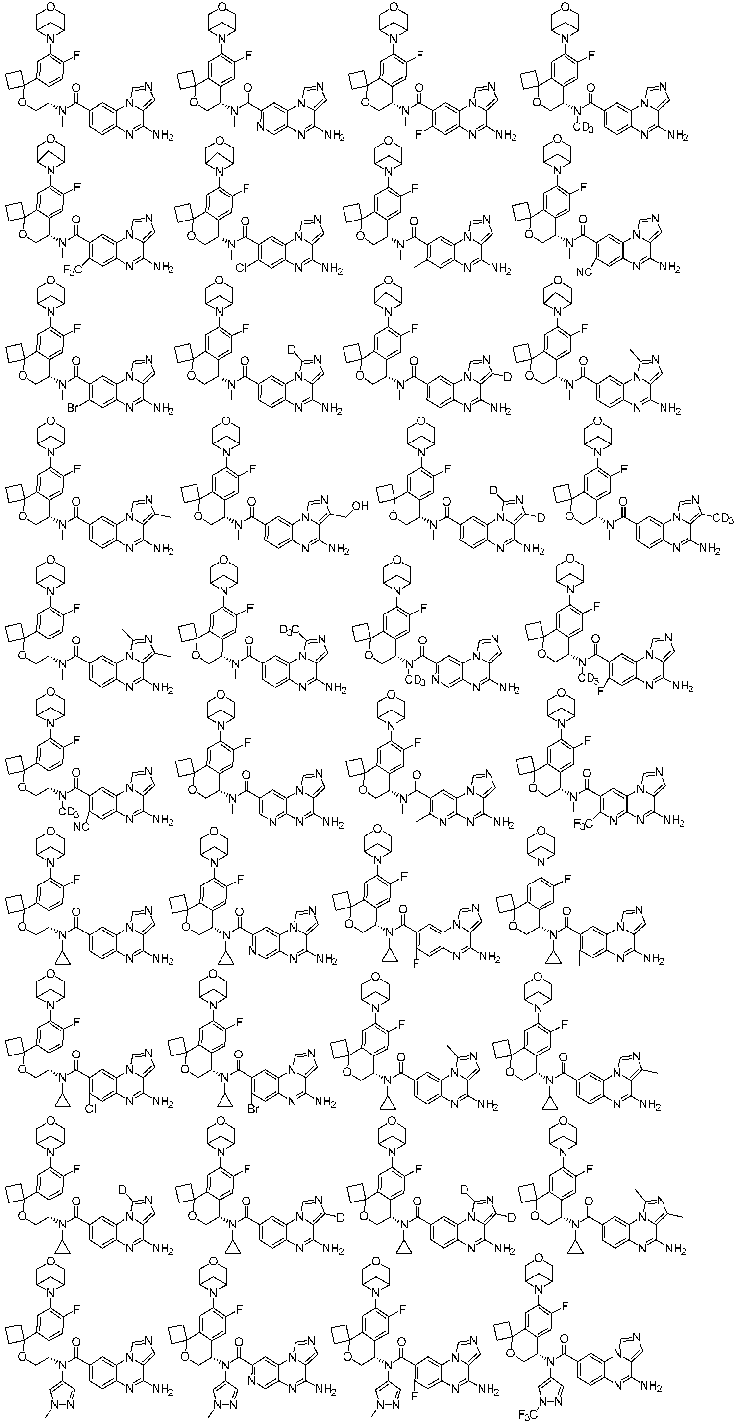

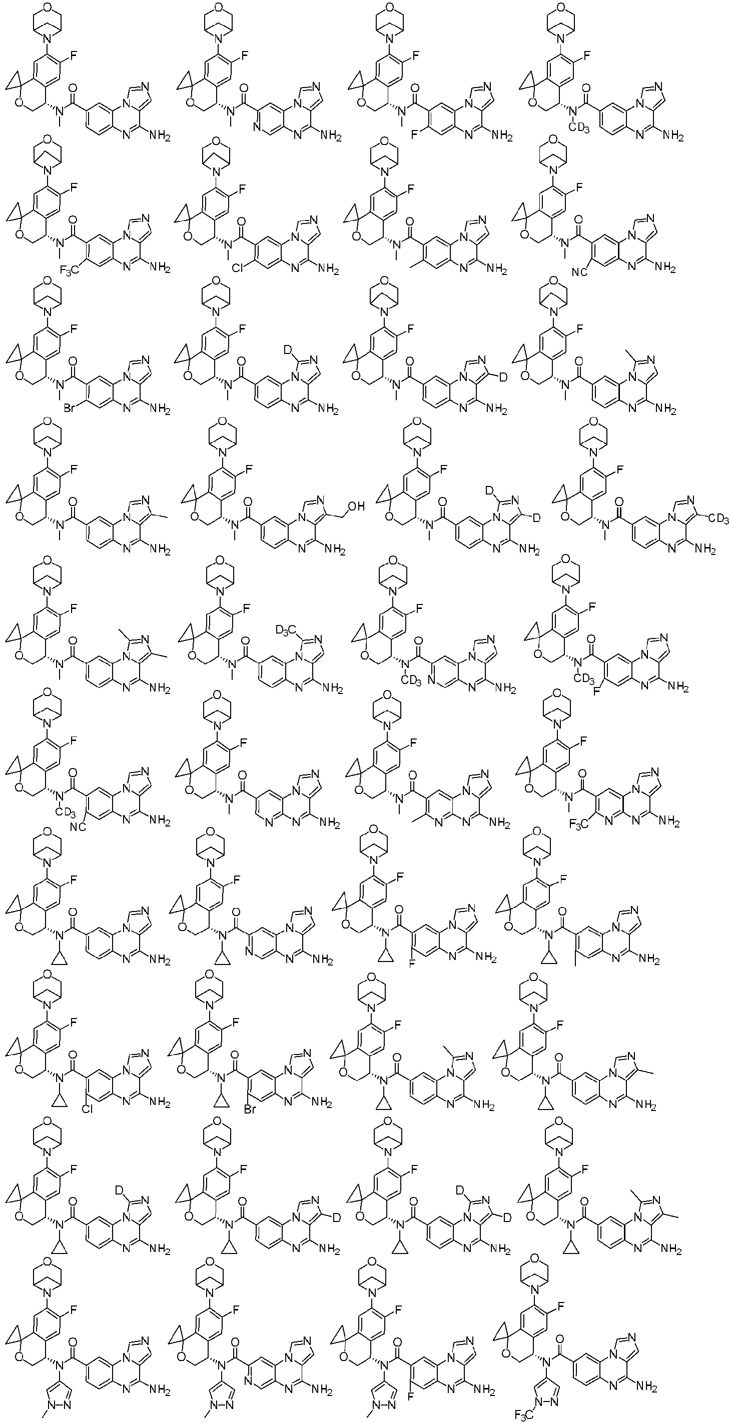

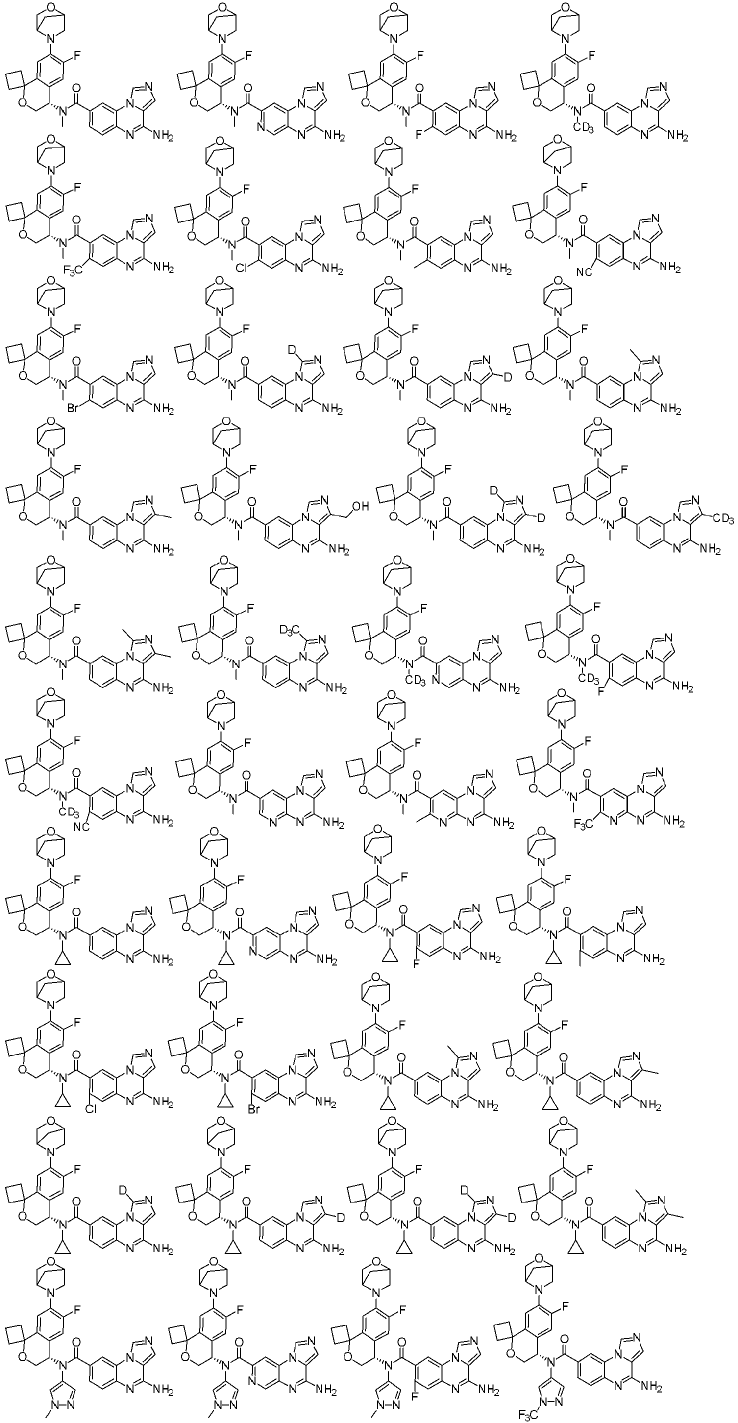

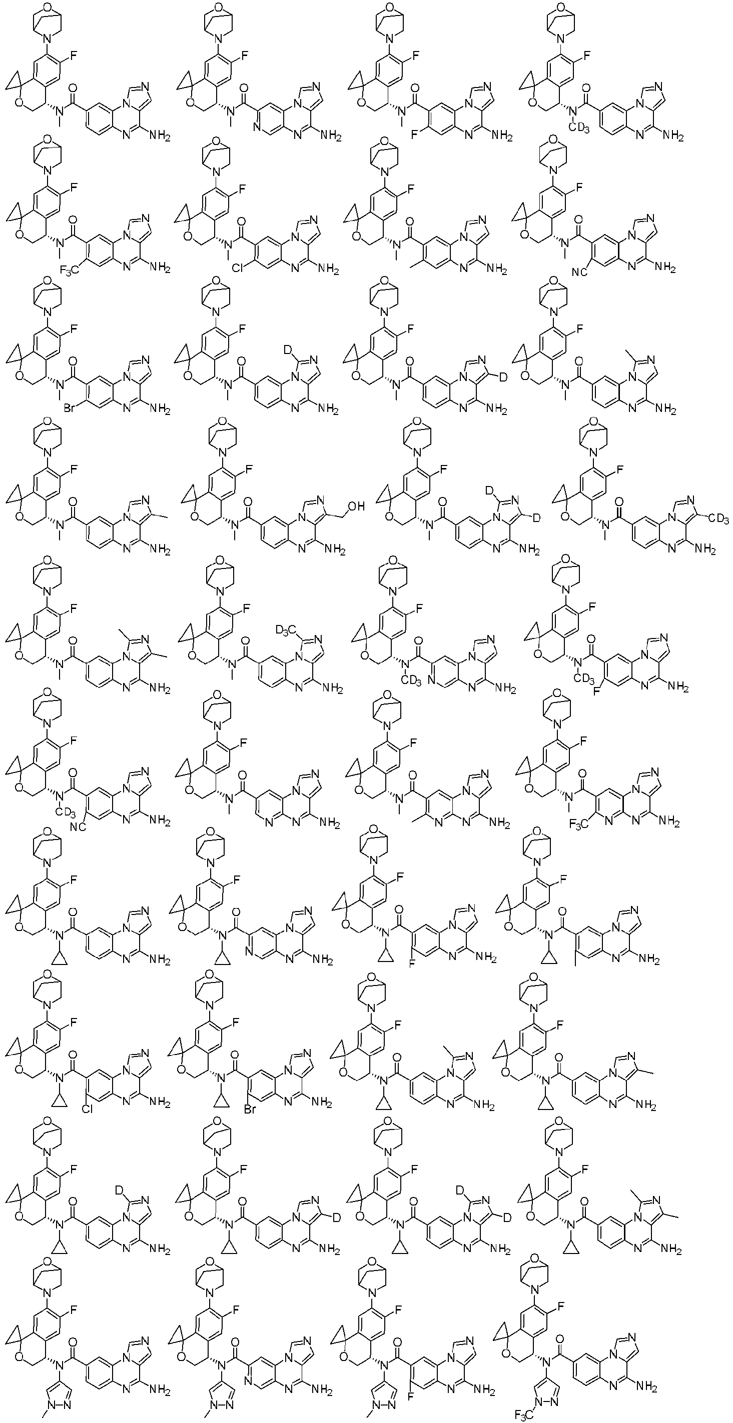

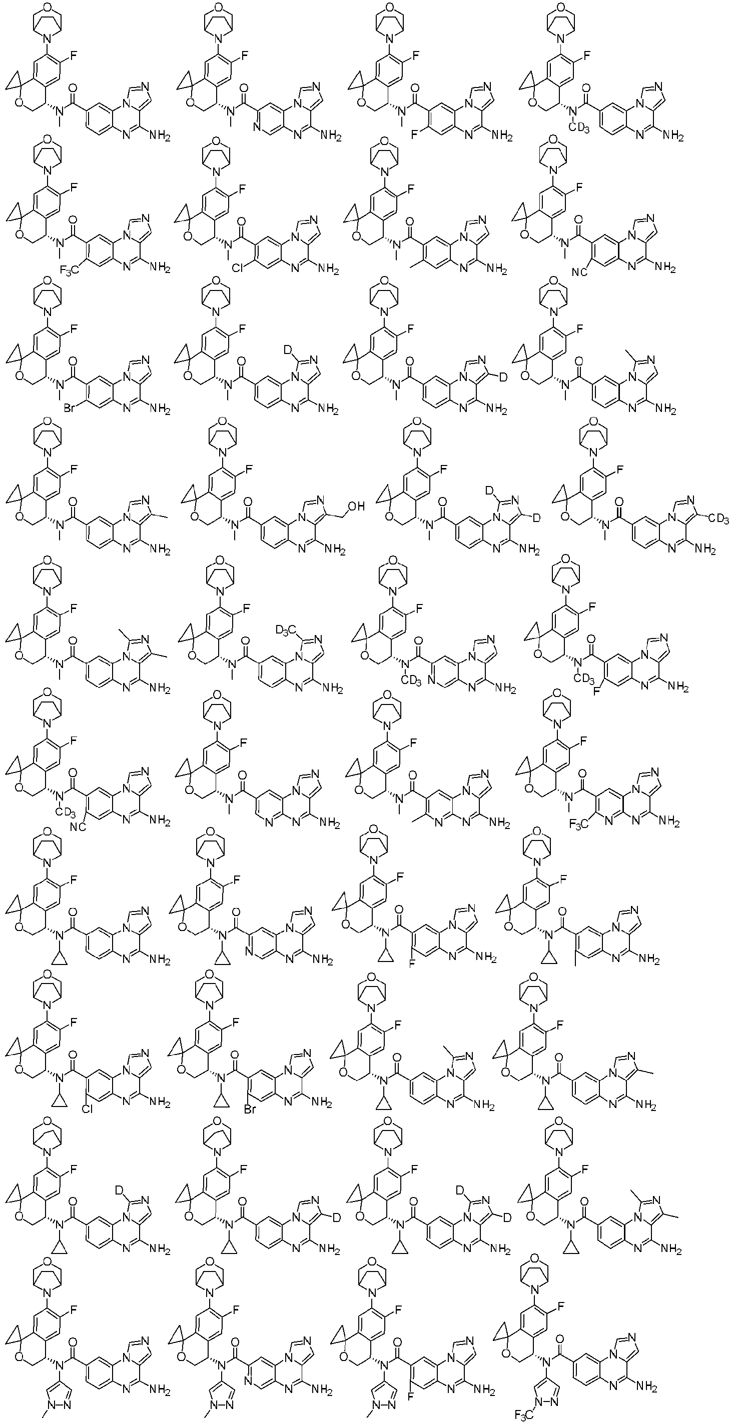

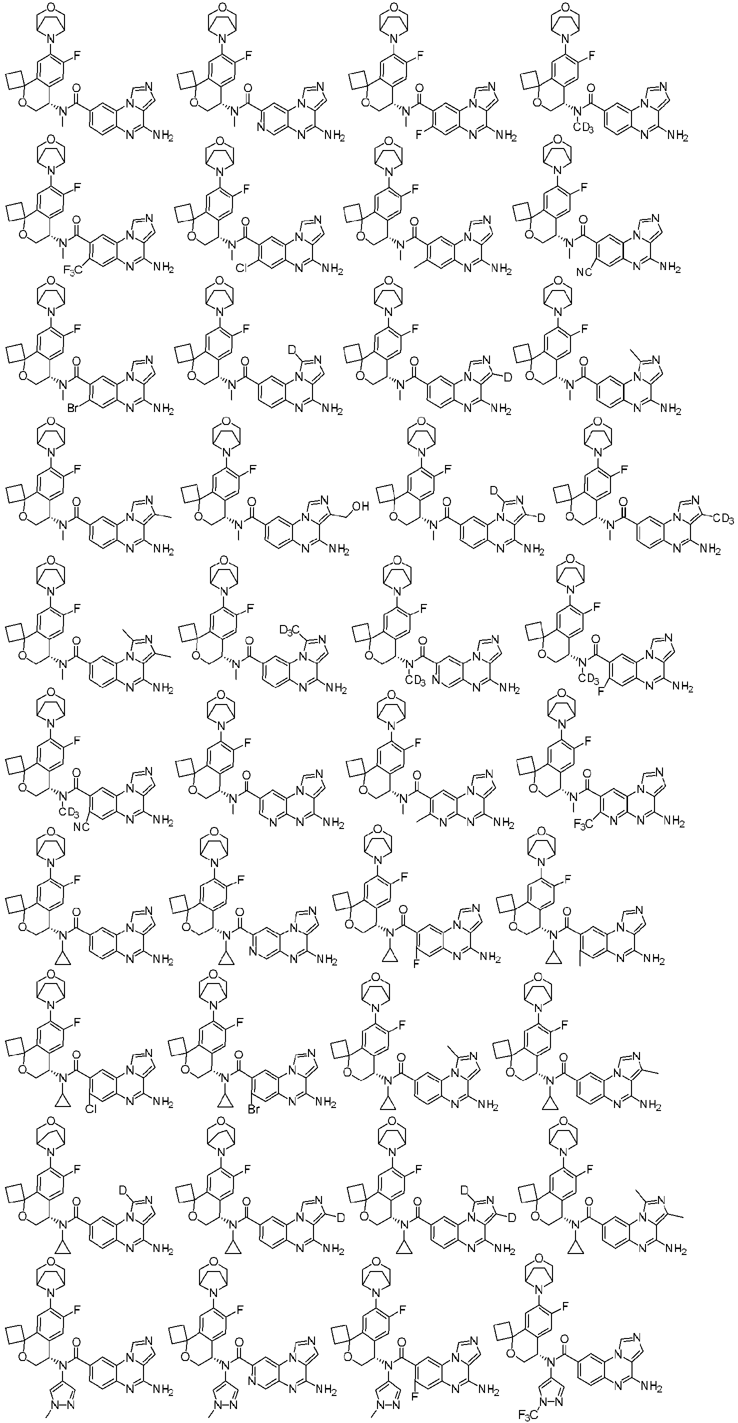

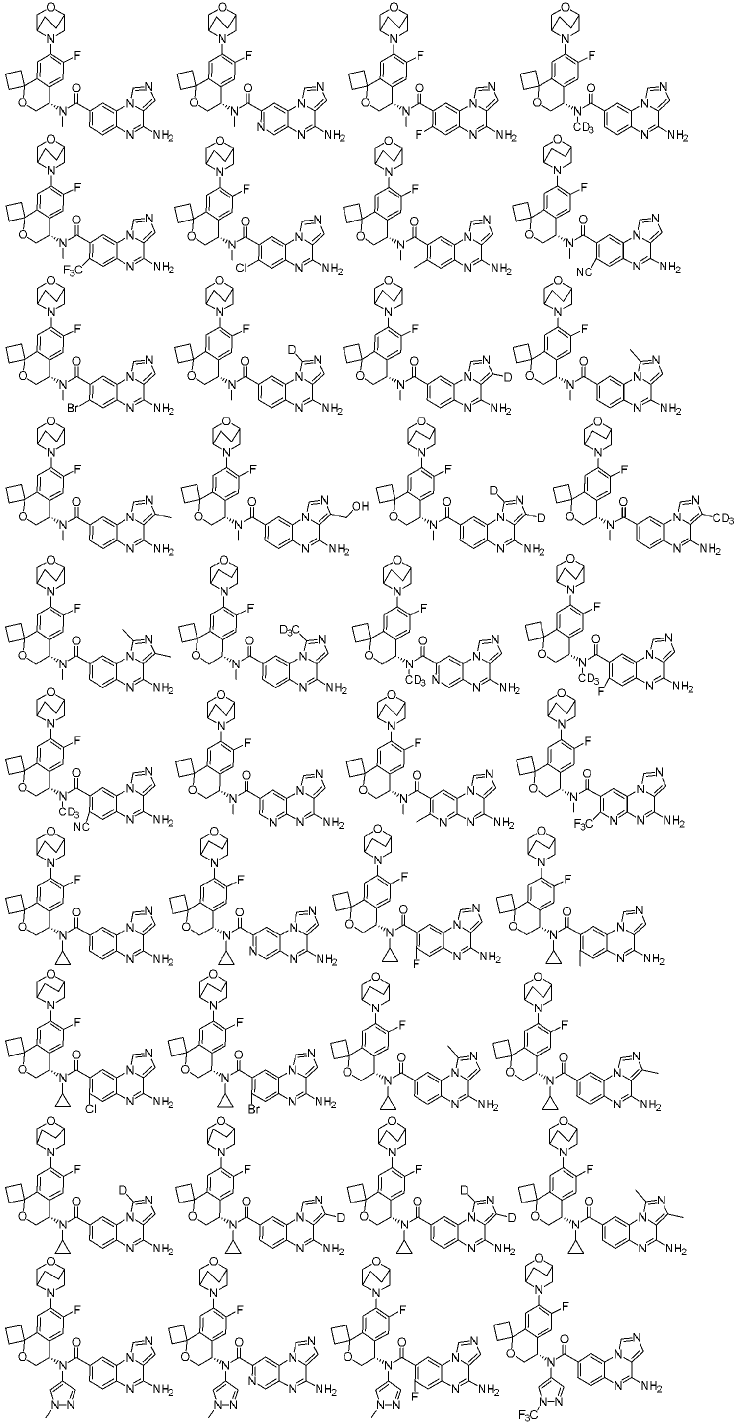

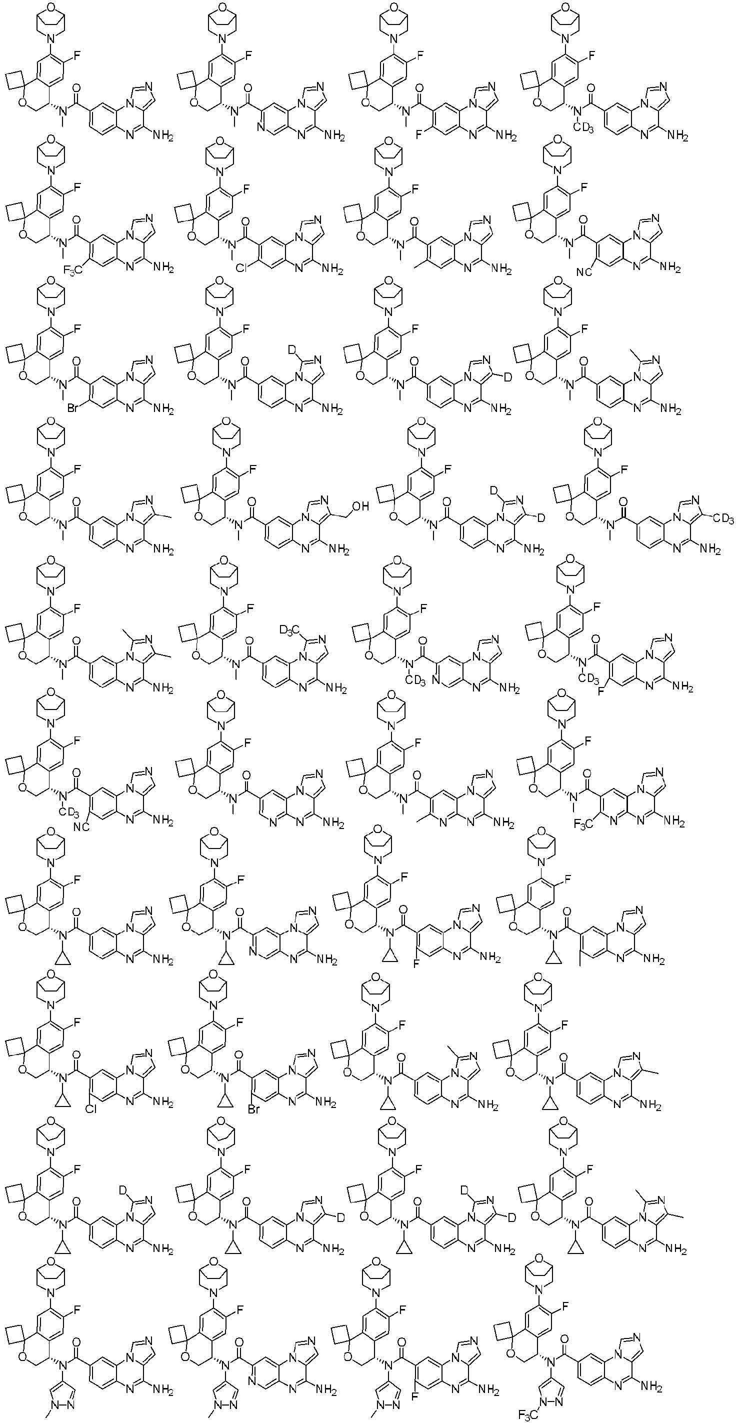

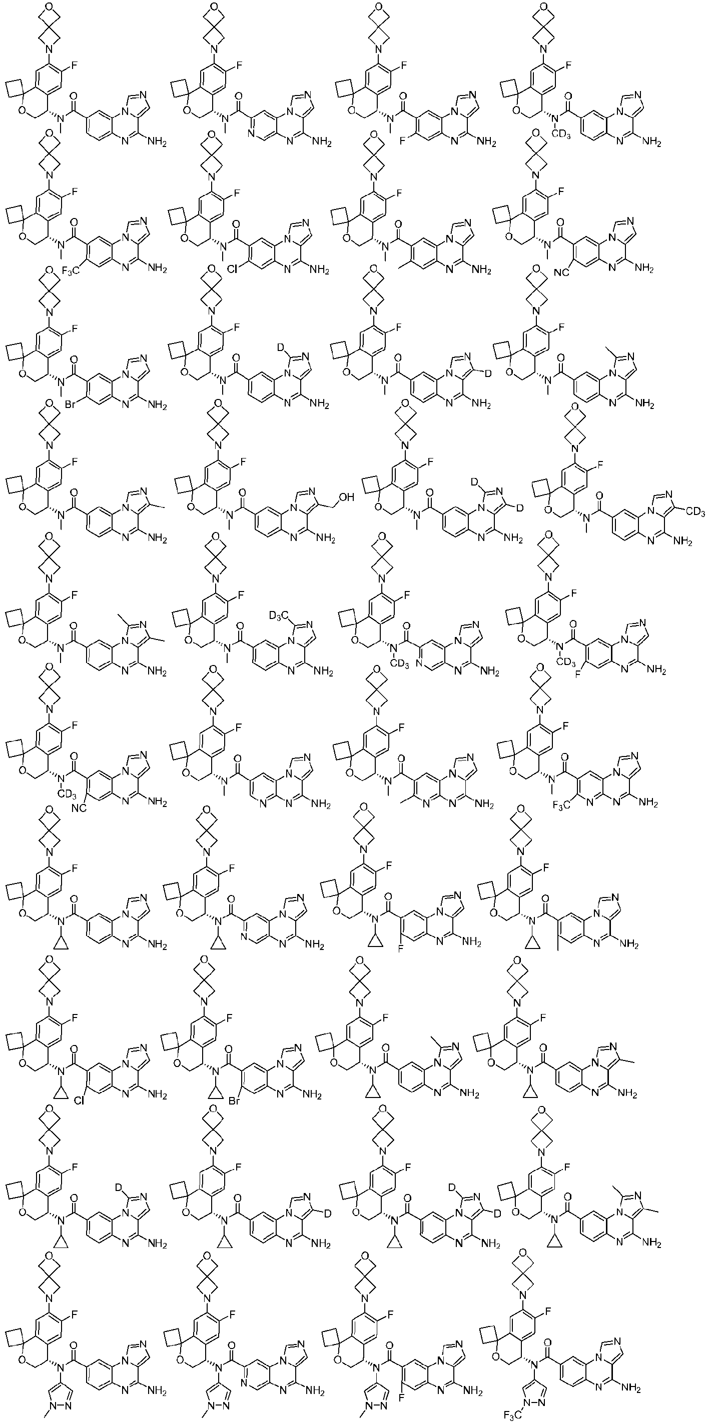

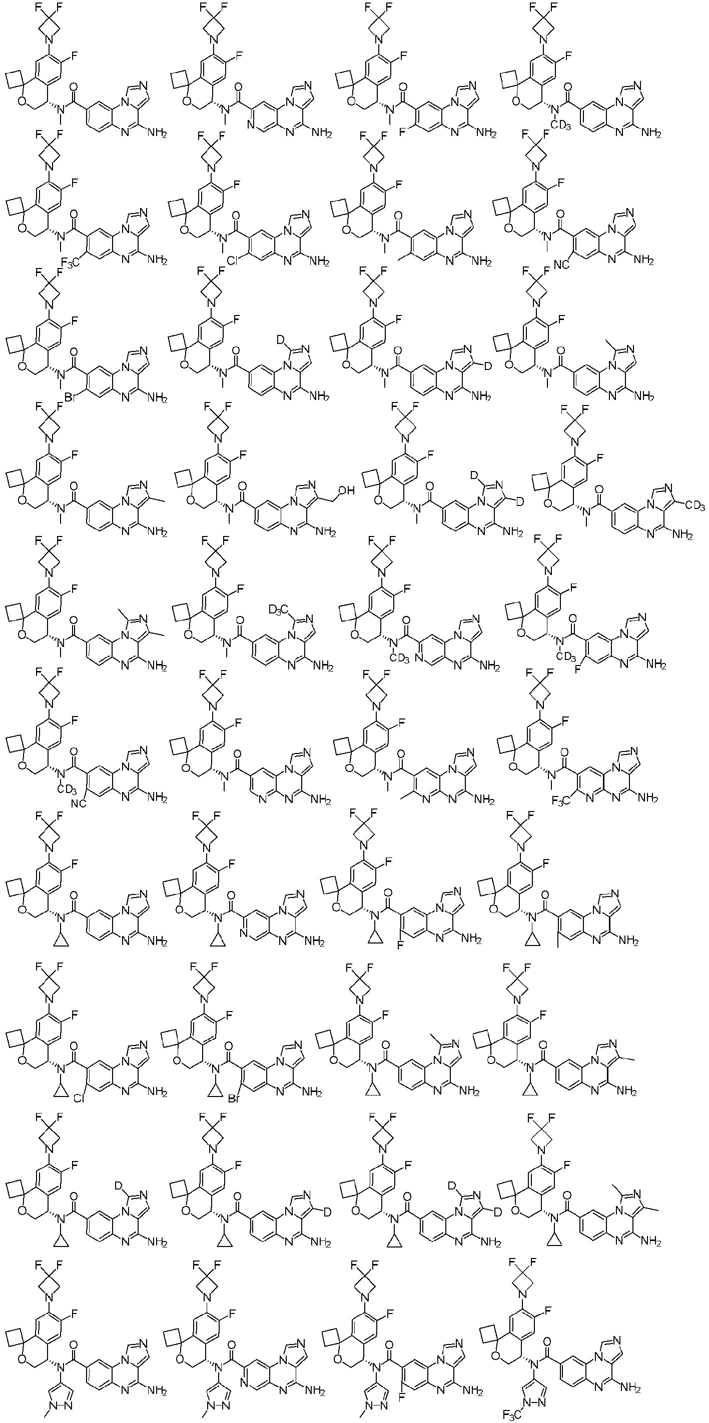

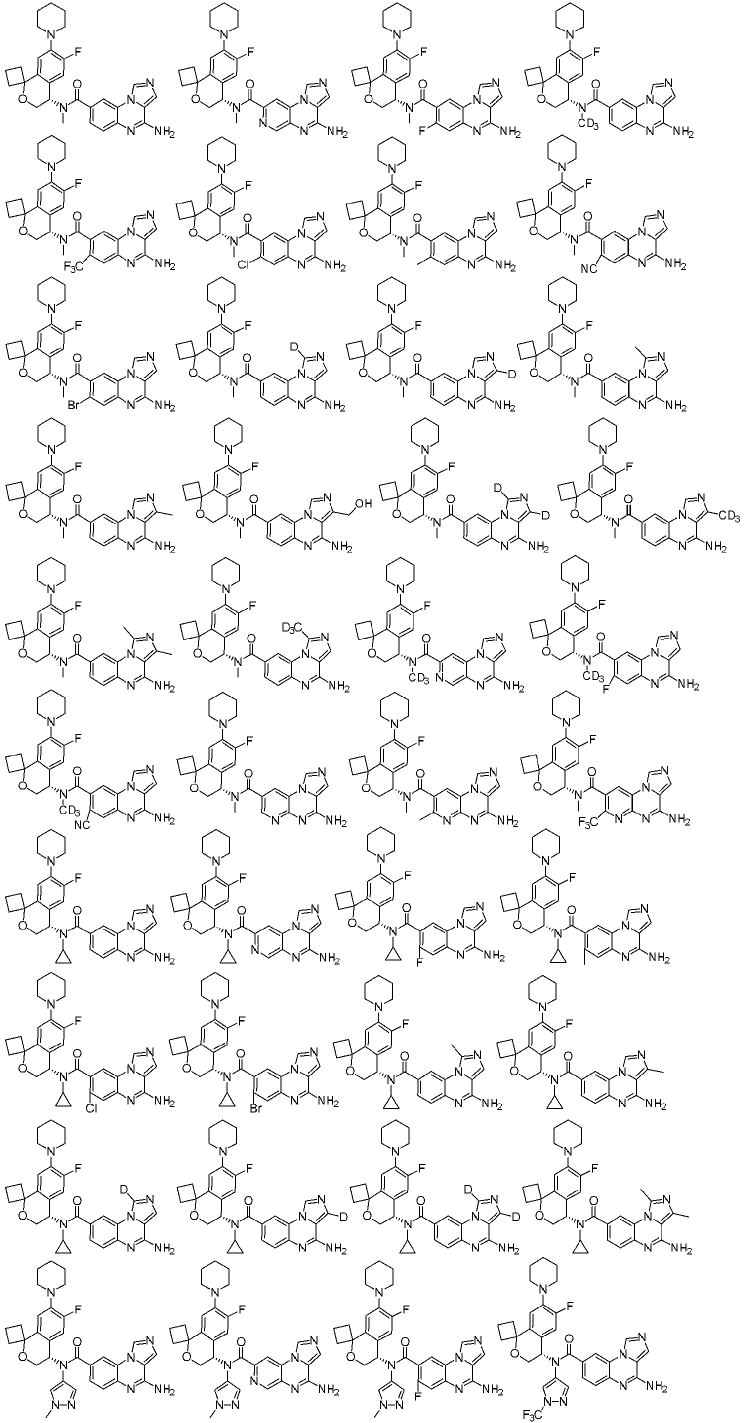

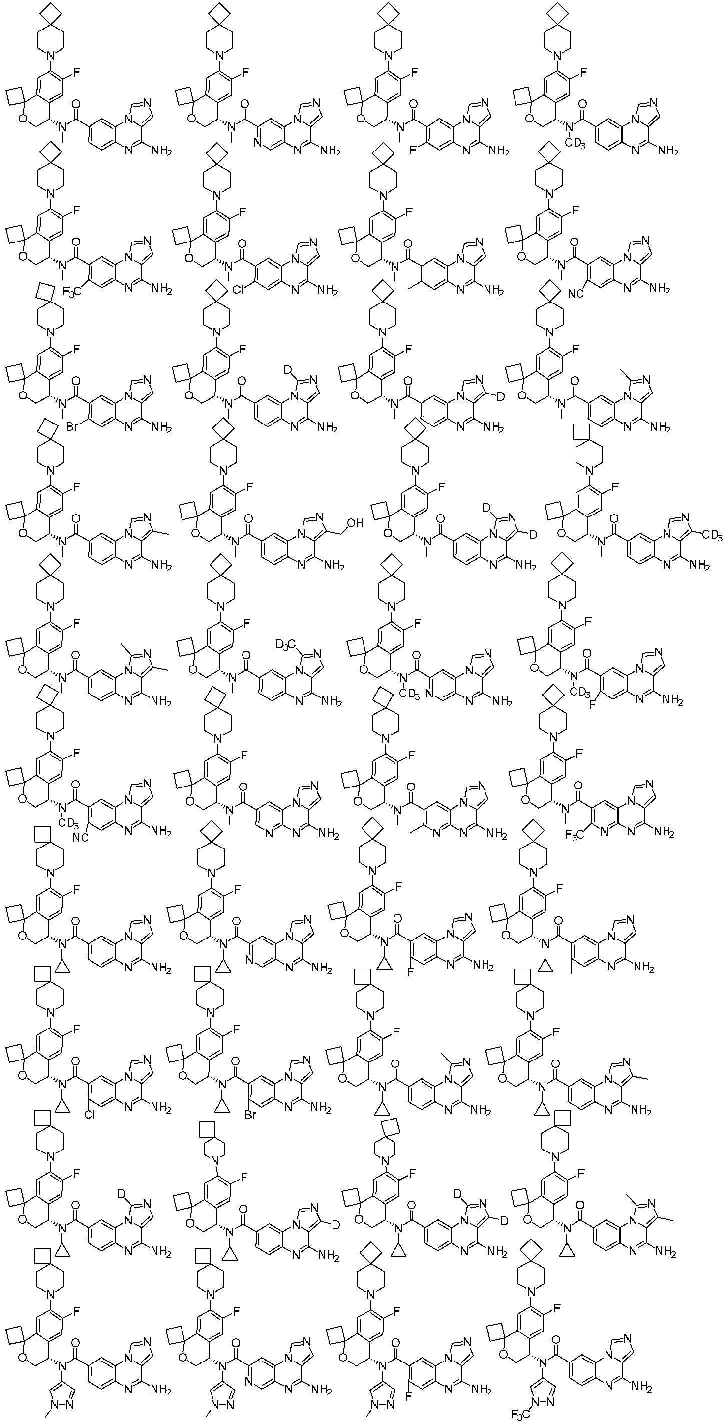

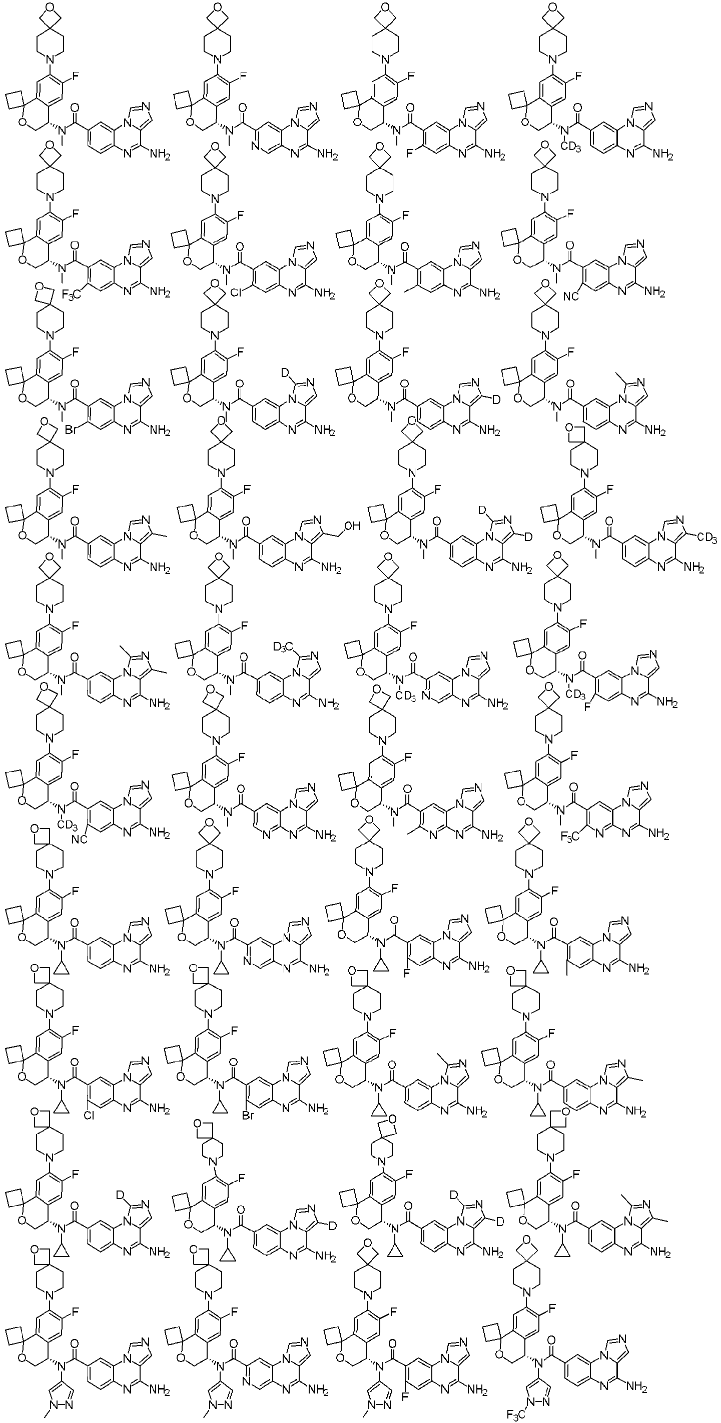

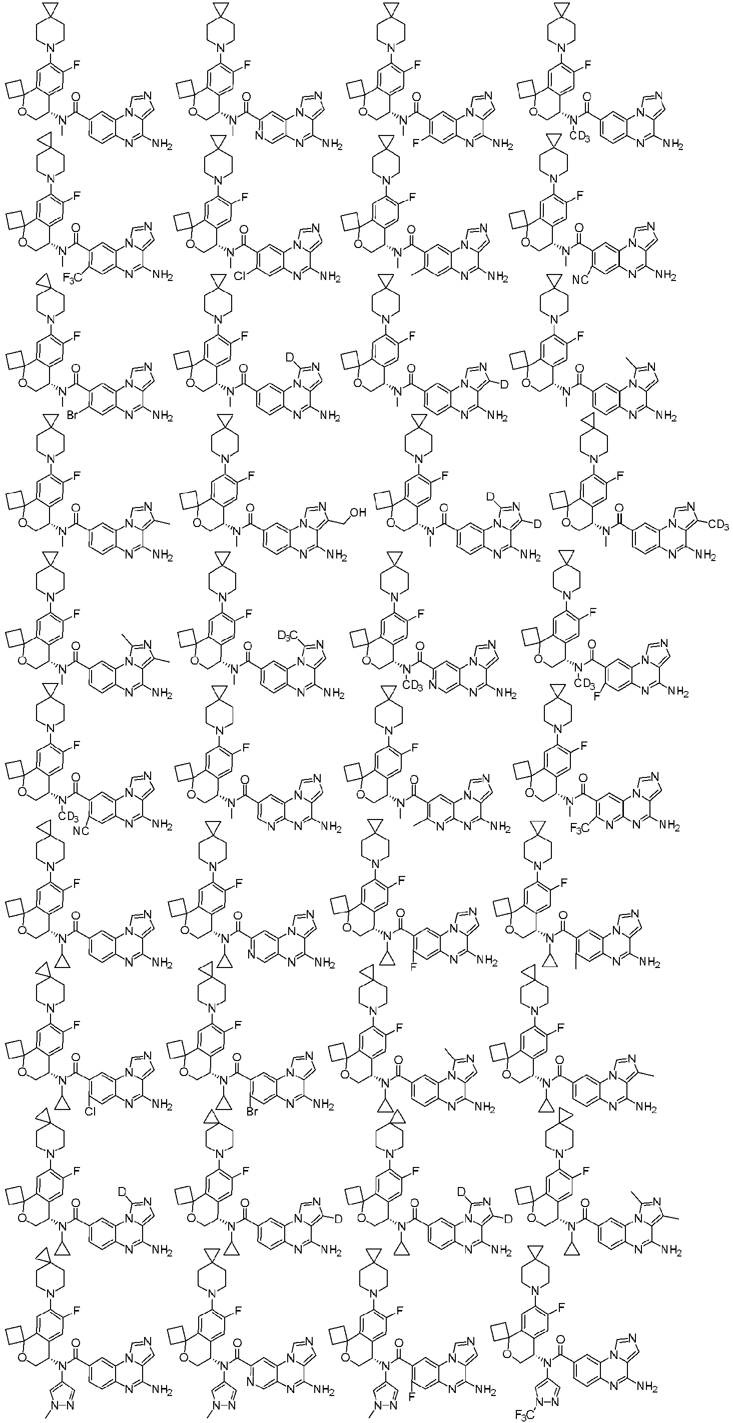

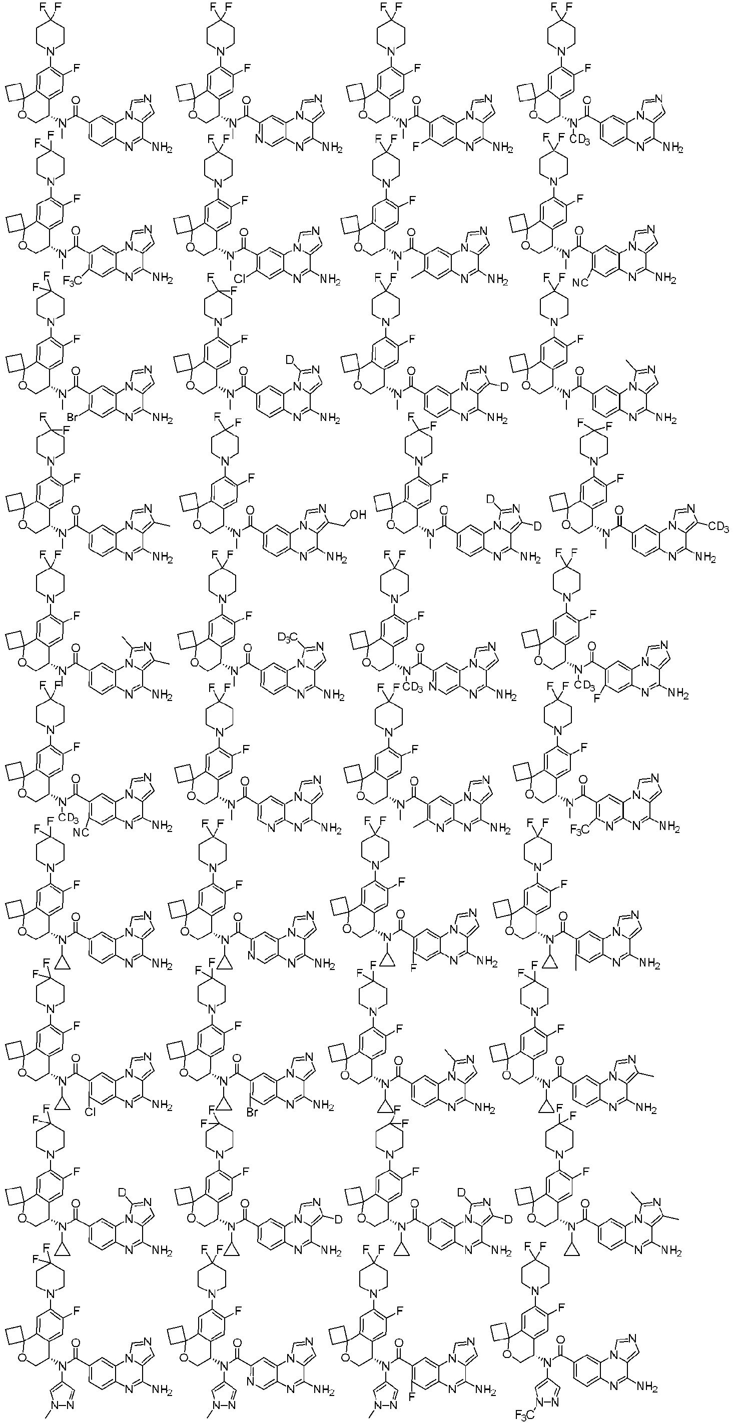

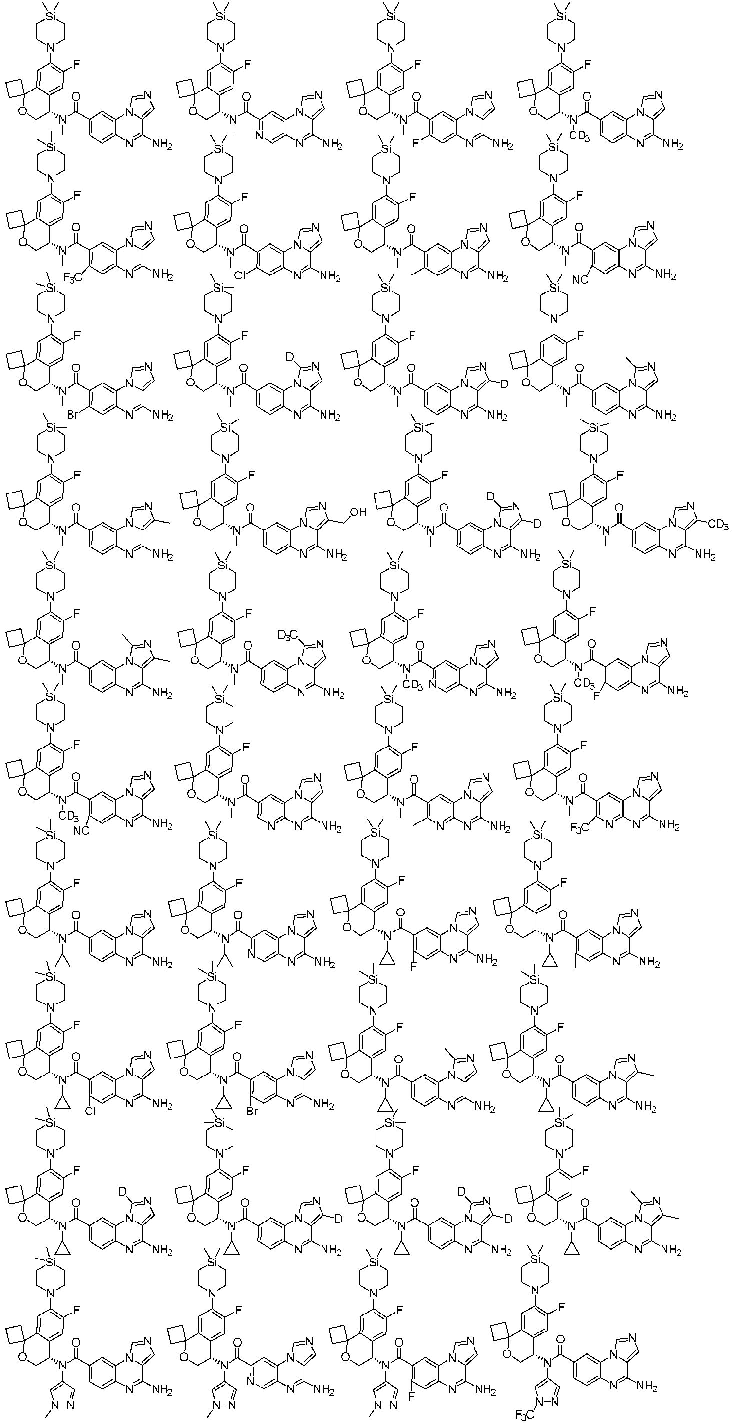

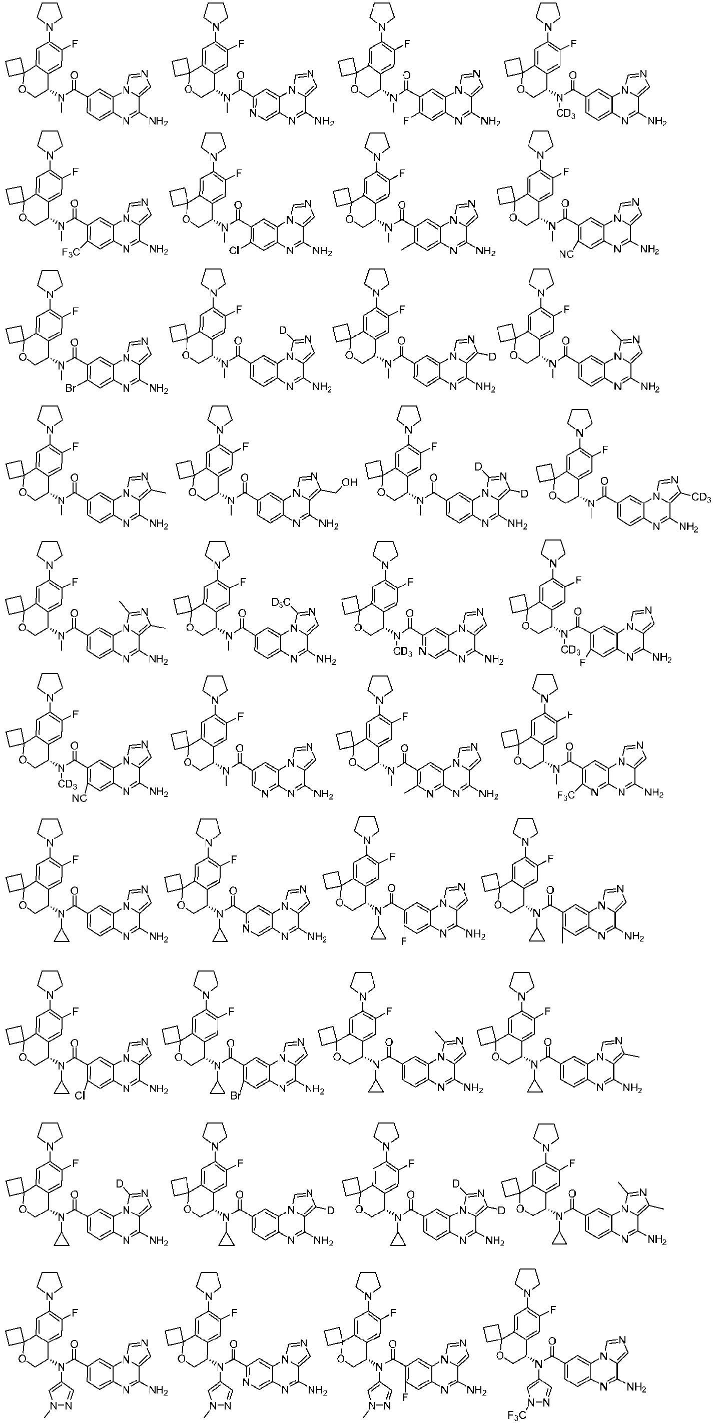

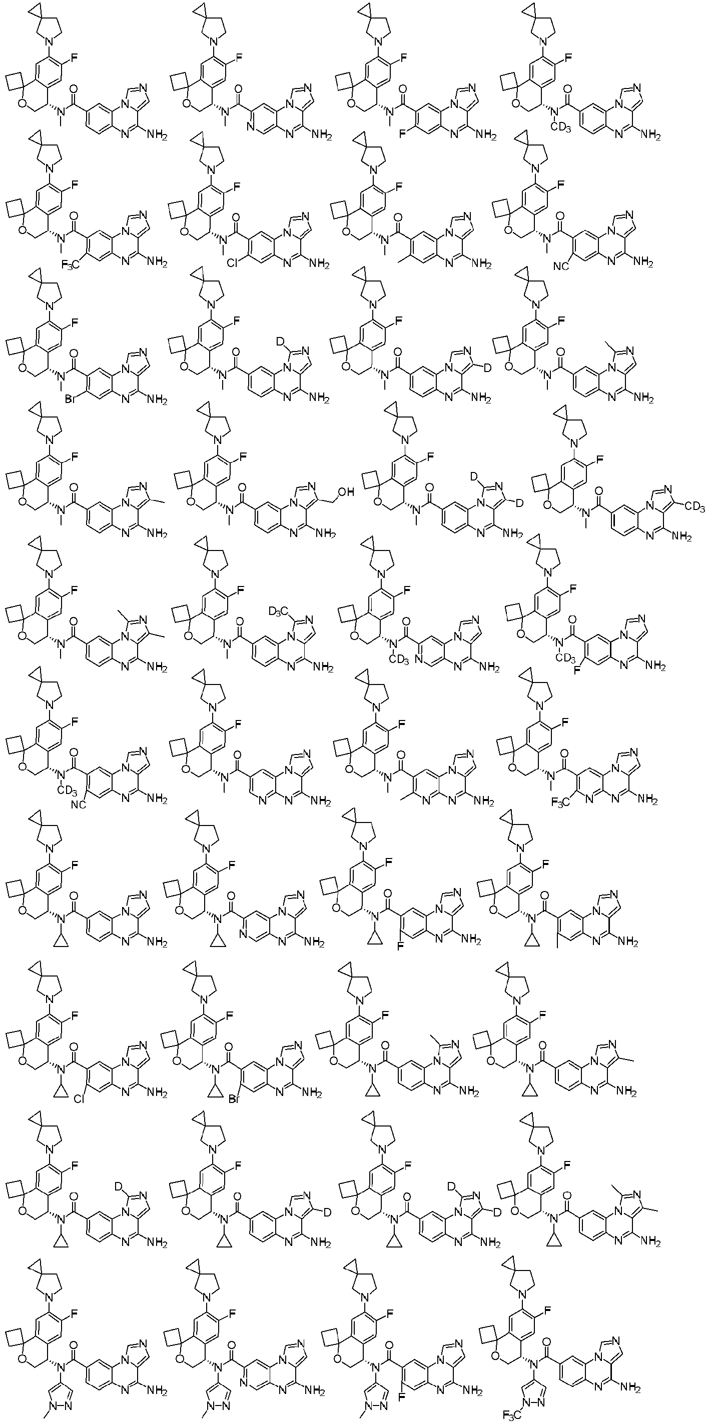

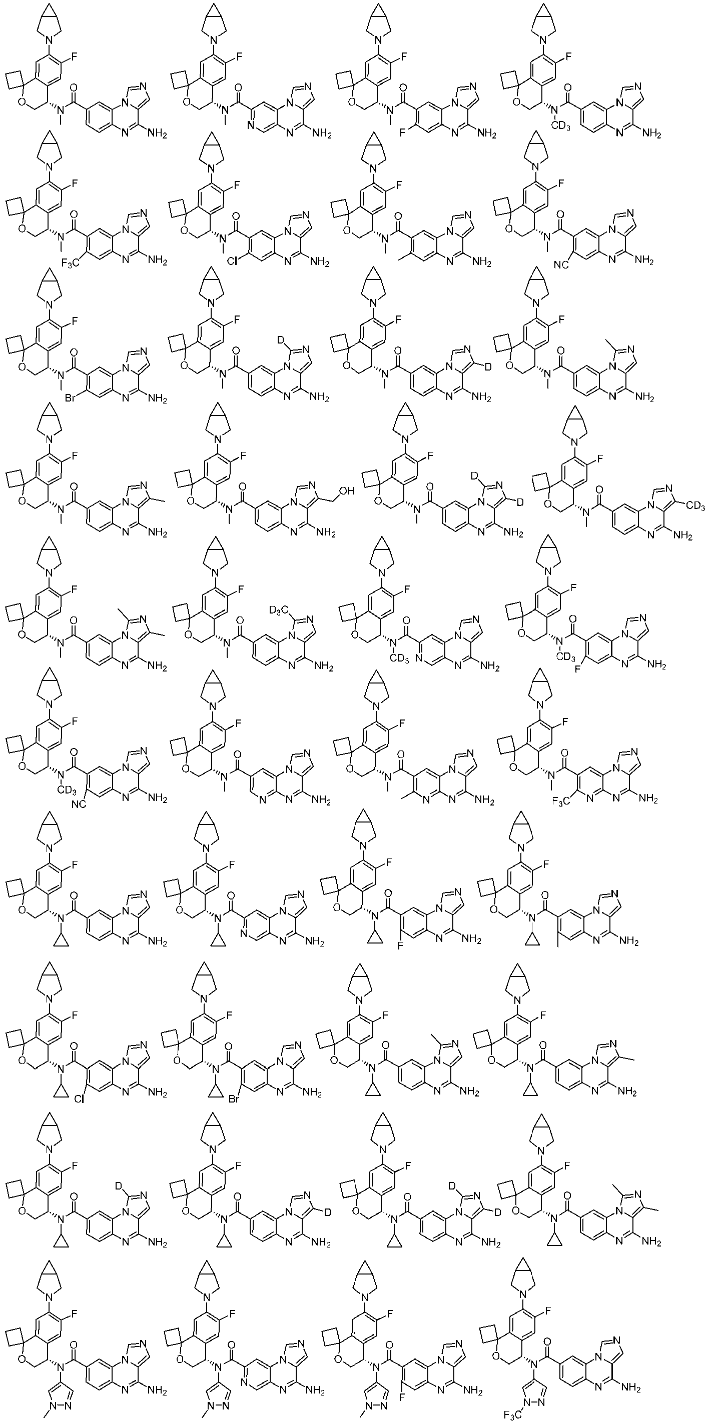

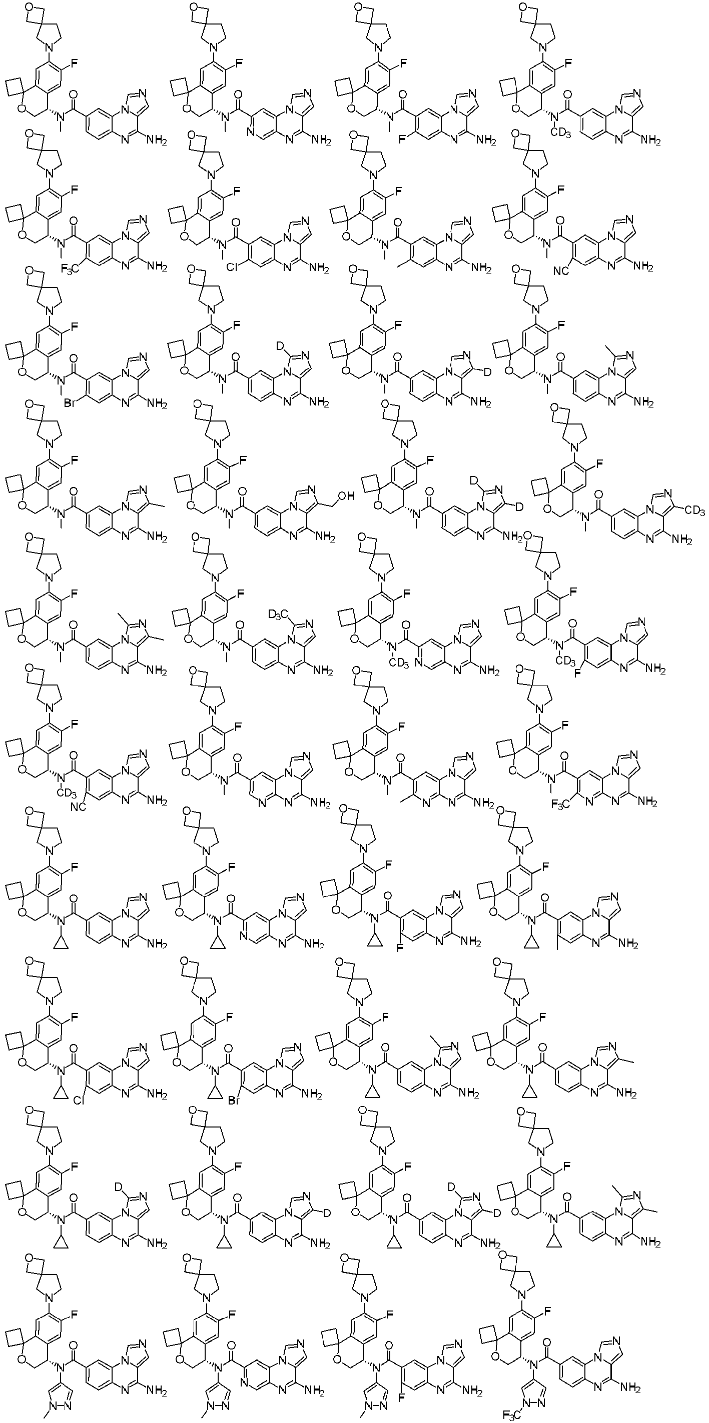

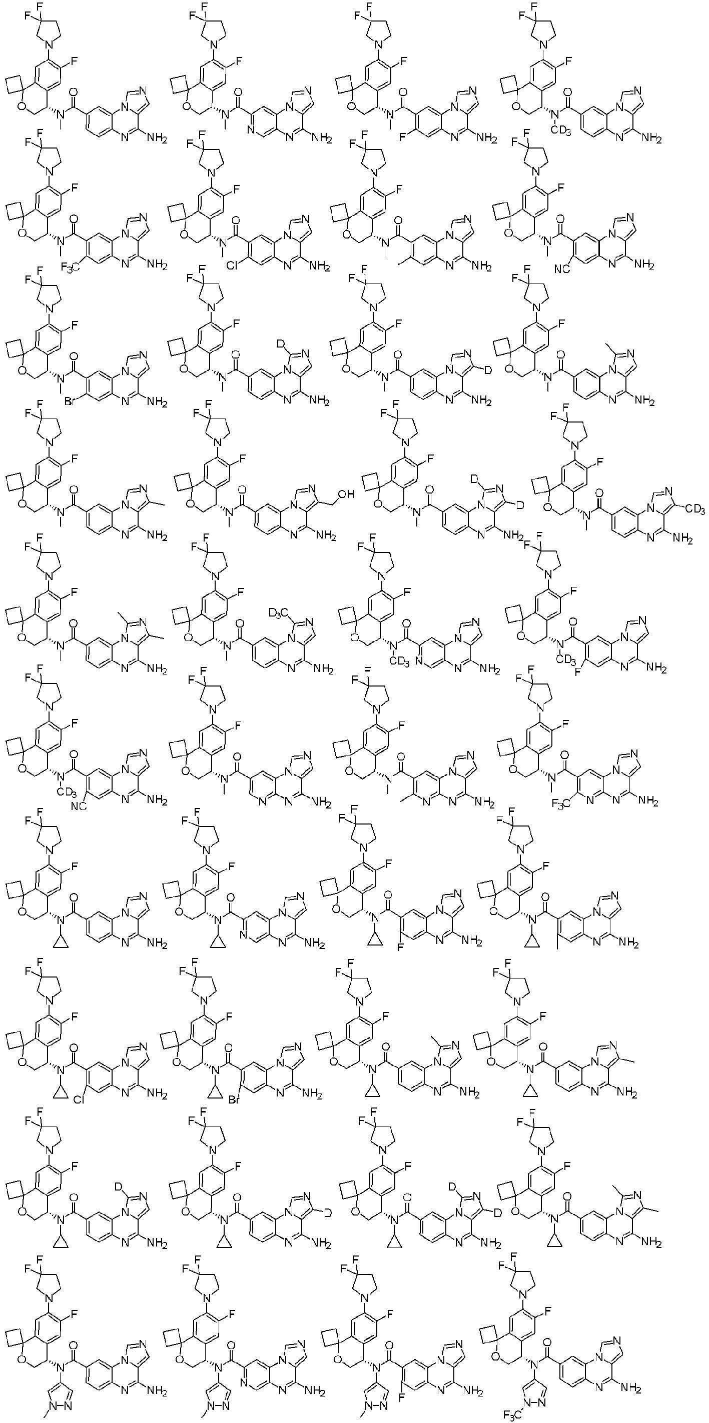

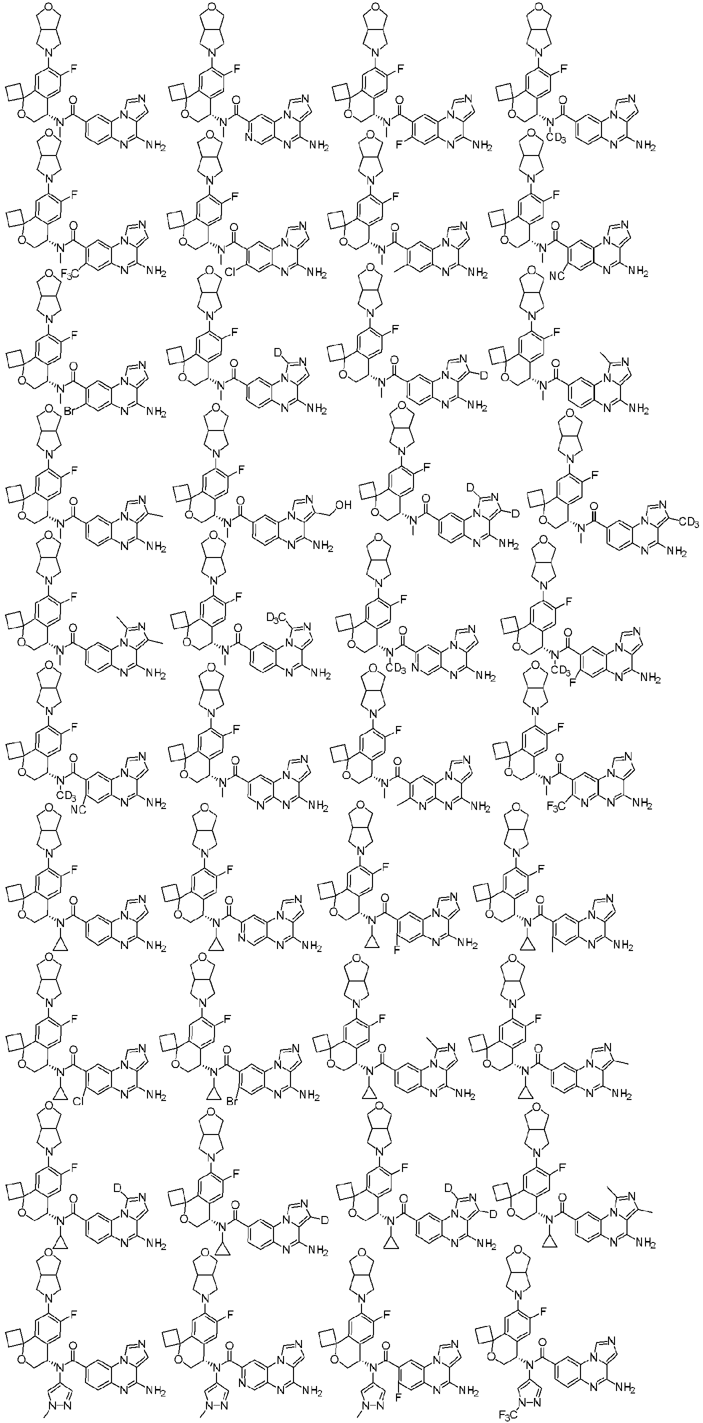

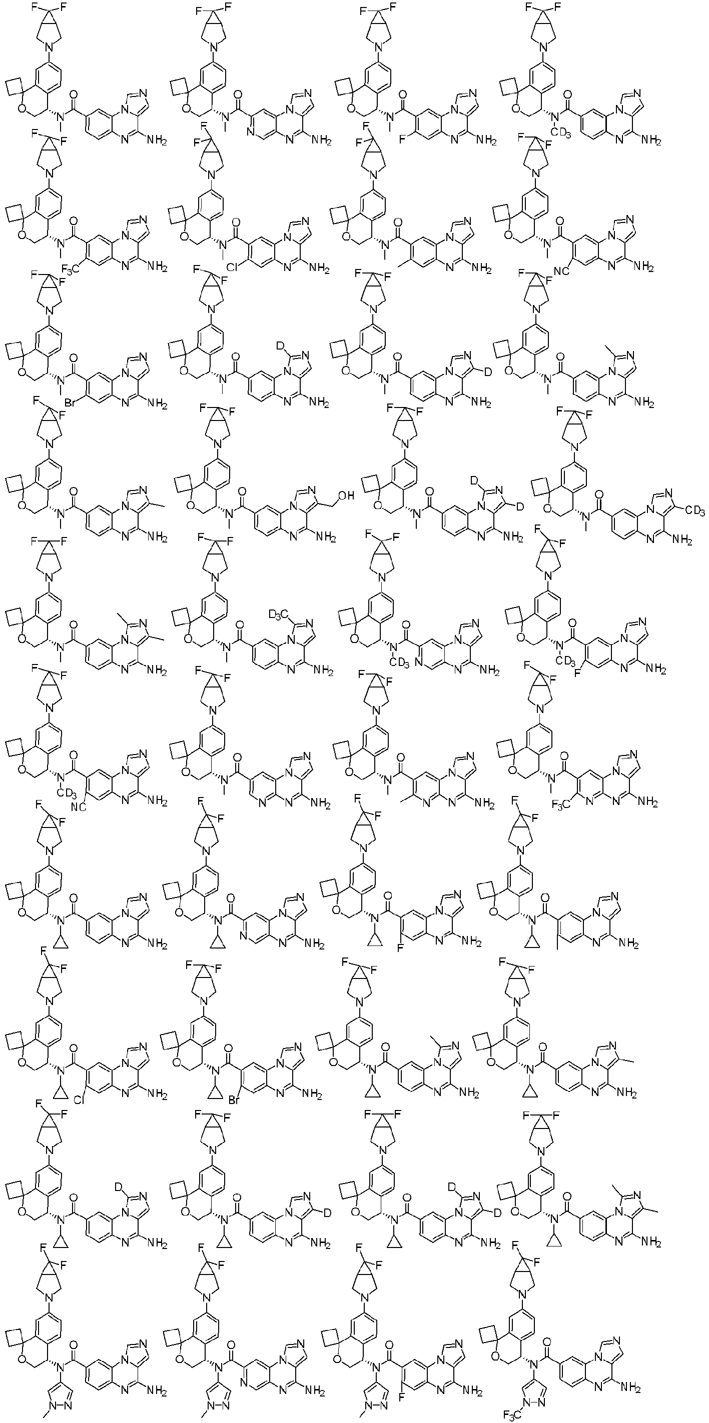

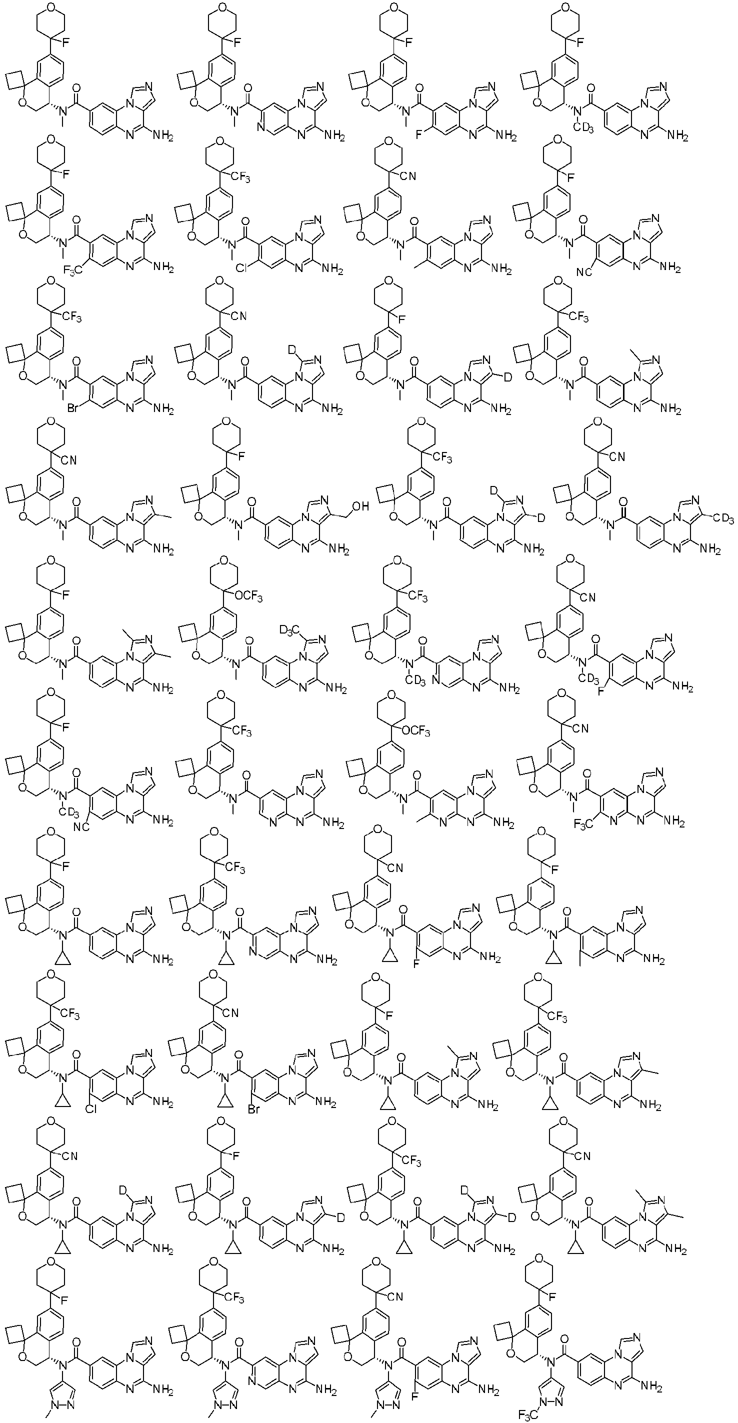

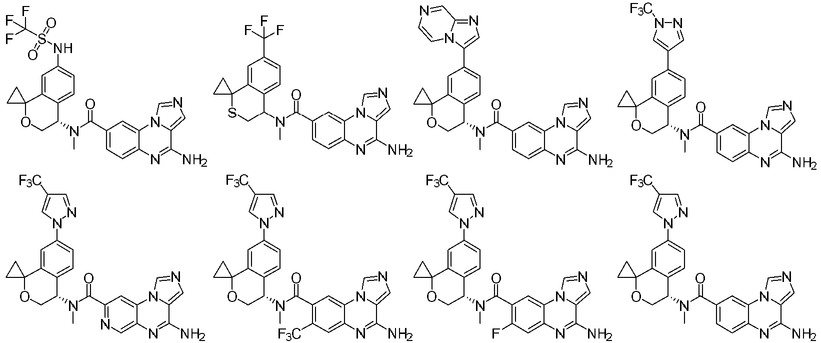

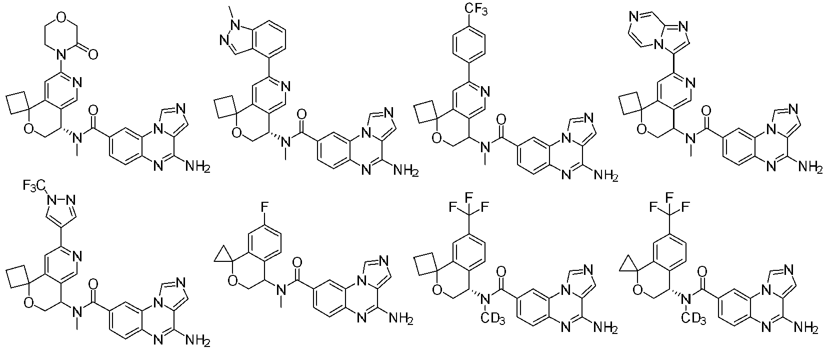

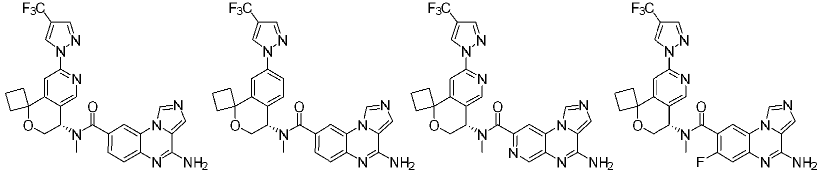

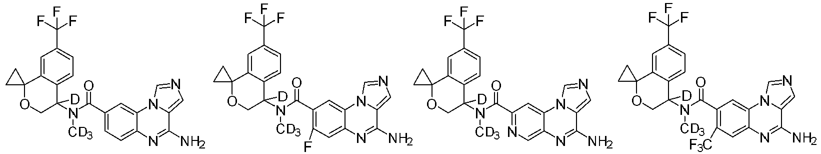

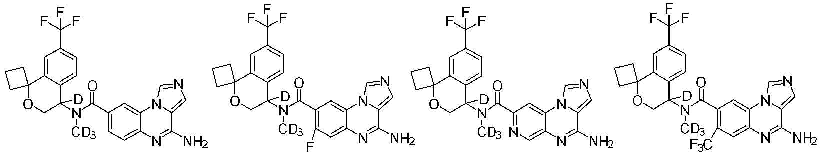

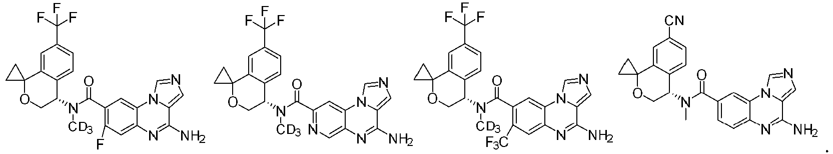

45. A method for treating and/or preventing a disease associated with abnormal expression of PRMT5, which comprises administering to an individual in need thereof a therapeutically and/or prophylactically effective amount of the compound according to any one of claims 1-44, its tautomeric form, or a pharmaceutically acceptable salt form thereof.

46. The method according to claim 45, the disease associated with abnormal expression of PRMTS is a tumor or cancer.

47. The method according to claim 46, wherein the tumor or cancer is any one selected from the following: Heart cancer: Sarcomas (angiosarcoma, fibrosarcoma, rhabdomyosarcoma, liposarcoma), myxoma, rhabdomyosarcoma, fibroma, lipoma and teratoma; Lung cancer: Bronchial cancer (squamous cell carcinoma, undifferentiated small cell carcinoma, undifferentiated large cell carcinoma, adenocarcinoma), alveolar (bronchiole) carcinoma, bronchial adenoma, sarcoma, lymphoma, chondromatous hamartoma, mesothelioma; Gastrointestinal cancer: Esophageal cancer (squamous cell carcinoma, adenocarcinoma, leiomyosarcoma, lymphoma), gastric cancer (cancer, lymphoma, leiomyosarcoma), pancreatic cancer (ductal adenocarcinoma, islet tumor, glucagonoma, gastrinoma, carcinoid tumor, vasoactive intestinal peptide secreting tumor), small intestinal cancer (adenocarcinoma, lymphoma, carcinoid tumor, Kaposi's sarcoma), leiomyoma, hemangioma, lipoma, Neurofibroma, fibroma), colorectal cancer (adenocarcinoma, tubular adenoma, villous adenoma, hamartoma, leiomyoma); Urogenital tract cancer: Renal cancer (adenocarcinoma, Wilms tumor (nephroblastoma), lymphoma, leukemia), bladder and urethral cancer (squamous cell carcinoma, transitional cell carcinoma, adenocarcinoma), prostate cancer (adenocarcinoma, sarcoma), testicular cancer (seminoma, teratoma, embryonal carcinoma, teratocarcinoma, chorionic carcinoma, sarcoma, interstitial cell carcinoma, fibroma, fibroadenomas, adenoma-like adenocarcinoma, lipoma); Liver cancer: Liver cancer (hepatocellular carcinoma), cholangiocarcinoma, hepatoblastoma, angiosarcoma, hepatocellular adenoma and hemangioma; Biliary tract cancer: Gallbladder cancer, ampullary cancer, bile duct cancer; Bone cancer: Osteogenic sarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, osteochondroma (osteocartilaginous exostosis), benign chondroma, chondroblastoma, chondromyxoid fibroma, osteoid osteoma and giant cell tumor; Nervous system cancer: Skull cancer (osteoma, hemangioma, granuloma, xanthoma, osteitis deformans), meningeal carcinoma (meningioma, meningosarcoma, gliomatosis), brain cancer (astrocytoma, medulloblastoma, glioma, ependymoma, germ cell tumor (pineal tumor), glioblastoma multiforme, oligodendroglioma, schwannoma, retinoblastoma, congenital tumor), spinal neurofibroma, meningioma, glioma, sarcoma); Gynecological cancer: Uterine cancer (endometrial carcinoma (serous cystadenocarcinoma, mucinous cystadenocarcinoma, unclassified carcinoma), granulosa cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma), fallopian tube carcinoma (cancer); Blood cancer: Blood cancers (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin cancer: Malignant melanoma, basal cell carcinoma, squamous cell carcinoma, Kaposi's sarcoma, dysplastic nevus, lipoma, hemangioma, dermatofibroma, keloid tumor; adrenal cancer and neuroblastoma.
